# EUROPEAN PATENT APPLICATION

(11) **EP 1 389 463 A1**
(43) Date of publication of application: **18.02.2004**
(21) Application number: 02722752.9
(22) Date of filing: 24.04.2002
(51) Int. Cl.: A61K 31/549, A61K 45/00, A61K 31/5377, A61K 31/55, A61K 31/4709, A61P 31/04, A61P 31/00, A61P 43/00, C12Q 1/18, C12Q 1/02, G01N 33/15, C07D 471/04

(54) **MEDICINE FOR INHIBITING DRUG ELIMINATION PUMP**

(30) Priority: 26.04.2001 US 842234; 08.02.2002 JP 2002033133
(71) Applicant: DAIICHI PHARMACEUTICAL CO., LTD., Chuo-ku, Tokyo 103-8234 (JP); Essential Therapeutics, Inc., Waltham, MA 02451 (US)
(72) Inventor: NAKAYAMA, K., c/o Daiichi Pharmaceutical Co., Ltd, Edogawa-ku, Tokyo 134-8630 (JP); OHTSUKA, M., c/o Daiichi Pharmaceutical Co., Ltd., Edogawa-ku, Tokyo 134-8630 (JP); KAWATO, H., c/o Daiichi Pharmaceutical Co., Ltd, Edogawa-ku, Tokyo 134-8630 (JP); OKUMURA, R., c/o Daiichi Pharmaceutical Co., Ltd., Edogawa-ku, Tokyo 134-8630 (JP); HOSHINO, K., c/o Daiichi Pharmaceutical Co., Ltd., Edogawa-ku, Tokyo 134-8630 (JP); WATKINS, W., c/o Essential Therapeutics, Inc., Waltham, MA 02451 (US); ZHANG, Jason, c/o Essential Therapeutics, Inc., Waltham, MA 02451 (US); PALME, Monica, c/o Essential Therapeutics, Inc., Waltham, MA 02451 (US); CHO, Aesop, c/o Essential Therapeutics, Inc., Waltham, MA 02451 (US)
(74) Representative: Sternagel, Fleischer, Godemeyer & Partner Patentanwälte
(86) International application number: PCT/JP2002/004087
(87) International publication number: WO 2002/087589

(57) **Abstract**

A medicament for preventive and/or therapeutic treatment of a microbial infection which comprises as an active ingredient a compound represented by the following general formula (I): 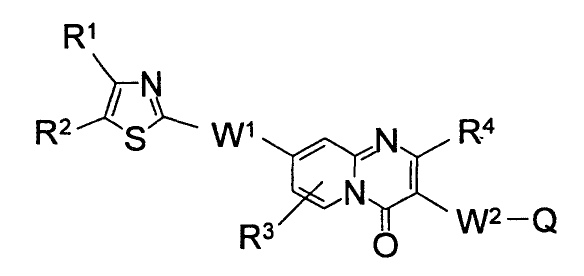 wherein, R¹ and R² represent hydrogen atom, a halogen atom, hydroxyl group or the like, W¹ represents -CH=CH-, -CH₂O-, -CH₂CH₂- or the like; R³ represents hydrogen atom, a halogen atom, hydroxyl group or an amino group; R⁴ represents hydrogen atom, a group of -OZ₀₋₄R⁵ (Z₀₋₄ represents an alkylene group, a fluorine-substituted alkylene group or a single bond, and R⁵ represents a cyclic alkyl group, an aryl group or the like); W² represents a single bond or -C(R⁸)=C(R⁹)- (R⁸ and R⁹ represent hydrogen atom, a halogen atom, a lower alkyl group or the like, Q represents an acidic group, but W² and Q may together form vinylidenethiazolidinedione or an equivalent heterocyclic ring; m and n represent an integer of 0 to 2, and q represents an integer of 0 to 3.

## Description

### Technical Field

The present invention relates to a medicament useful for preventive and therapeutic treatment of microbial infectious diseases.

### Background Art

For preventive or therapeutic treatment of infectious diseases caused by microorganisms, various antibacterial agents have so far been developed, and drugs such as *β* -lactam antibiotics (penicillins, cephems, monobactams, carbapenems, and penems), aminoglycosides, quinolones, macrolides, tetracyclines, rifamycins, chloramphenicols, and phosphomycins have been practically used. However, with the increase of clinically used amount of antibacterial agents, remarkable numbers of resistant bacterial strains to these antibacterial agents have emerged, which becomes a serious problem in the treatment of infectious diseases.

Examples of problematic bacteria, which cause particularly intractable or serious infectious diseases among those caused by resistant bacteria, include *Pseudomonas aeruginosa* and methicillin-resistant *Staphylococcus aureus* (MRSA). Antibacterial agents effective against these bacteria have been limited so far, and it is not certain whether or not therapeutic efficacy of the currently available drugs will be expected in the future. In particular, no drug is available at present by which specifically high efficacy against resistant *Pseudomonas aeruginosa* can be achieved. With the increase of aged population and the popularization of sophisticated medical technologies including human organ transplantation and anti-cancer treatments, infections frequently occurring particularly in patients with reduced immunity, i.e., so-called opportunistic infections, have become an extremely serious problem in the clinical field, and under the circumstances, early developments of measures against the resistant bacteria are desired.

Recently, the presence of drug efflux pumps has recognized as a bacterial excretion mechanism of drugs through researches on resistance acquiring mechanisms of resistant bacteria. In earlier researches, a pump that specifically excretes a tetracycline antibacterial agent from bacterial cells was identified in 1980 by the group of Levy, and the discovery was noted as a major factor of the resistance to tetracycline (L. McMurry, Proc. Natl. Acad. Sci. U.S.A., 77, 3974, 1980). Furthermore, based on recent researches, the presence of multidrug-excreting drug efflux pumps was reported in *Escherichia coli, Pseudomonas aeruginosa, Bacillus subtilis, Staphylococcus* bacteria, *Diplococcus pneumoniae,* and *Neisseria gonorrhoeae.* Four multidrug efflux pumps have so far been reported as homological drug efflux pumps deriving from *Pseudomonas aeruginosa,* and they have been considered as a cause of low drug. sensitivity inherent to *Pseudomonas aeruginosa* (K. Poole et al., J. Bacteriol., 175, 7363, 1993; K. Poole et al., M. Microbiol., 21, 713, 1996; T. Kohler et al., M. Microbiol., 23, 345, 1997; T. Mine et al., Antimicrob. Agents Chemother., 43, 415, 1999).

The drug efflux pumps of *Pseudomonas aeruginosa* excrete various drugs including *β*-lactams, tetracyclines, chloramphenicols, and quinolones, to which the drug resistance of *Pseudomonas aeruginosa* is attributable.

In order to overcome the problem, it will be effective to invent an antibacterial agent that has a novel structure, by which resistance acquisition due to a drug efflux pump, one of factors of resistance acquisition, can be avoided, or develop an agent for a combinational use with currently available antibacterial agents that can restore their efficacy by inhibiting functions of drug efflux pumps. As one of the latter means, drug efflux pump inhibitors have been known (WO 01/30757).

Under the circumstances, if information as to whether or not an etiologic bacterium expresses a drug efflux pump or information as to what kind of drug efflux' pump is expressed by the microorganism can be obtained in a convenient manner, it is believed that more effective chemotherapy will become possible. For example, when a drug efflux pump inhibitor is used in combination which restores efficacy of an existing antibacterial agent by inhibiting the function of drug efflux pump, it is expected that judgment of appropriateness of application of a drug efflux pump inhibitor as well as selection of combinational therapy utilizing an inhibitor effective to a particular drug efflux pump and an antibacterial agent can be made by obtaining the aforementioned information. However, any method to conveniently obtain the aforementioned information has not been known so far. In particular, any means to conveniently identify a kind of drug efflux pump expressed in a microorganism or any means to conveniently know whether or not two or more kinds of drug efflux pumps are expressed in a microorganism has not yet been known to date.

### Disclosure of the Invention

Therefore, an object of the present invention is to provide a novel medicament for the treatment of infectious diseases that improves therapeutic efficacy of an agent against pathogenic microorganisms, in particular, a medicament that acts on a microorganism with acquired resistance to an antimicrobial agent, and eliminates the resistance of the bacteria by inhibiting a drug efflux pump so as to improve preventive and/or therapeutic effect of the antimicrobial agent.

Another object of the present invention is to provide a method for judging effectiveness of a drug efflux pump inhibitor against a microorganism. A still further object of the present invention is to provide a method for conveniently obtaining information on what kind of drug efflux pump is expressed by a microorganism. More specifically, the object is to provide a method for identifying a drug efflux pump expressed in a microorganism, and a method for conveniently verifying expression of two or more kinds of drug efflux pumps in a microorganism.

In order to achieve the aforementioned first object, the inventors of the present invention conducted various researches to search for compounds that eliminate resistance to an antimicrobial drug of *Pseudomonas aeruginosa* that has acquired the resistance. As a result, they found that the compounds represented by the following general formula (I) or (II) had the desired activity, and thus achieved the present invention.

The present invention thus provides a medicament for preventive and/or therapeutic treatment of microbial infections, which comprises as an active ingredient a compound represented by the following general formula (I) or a physiologically acceptable salt thereof, or a hydrate thereof: 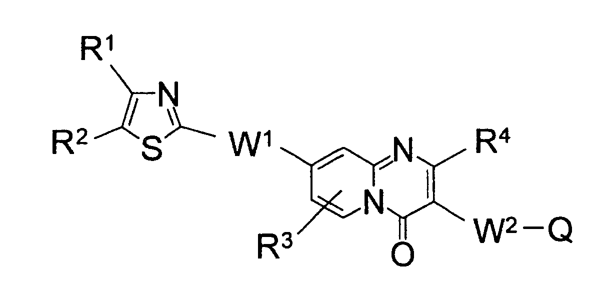 wherein, R¹ and R² each independently represent hydrogen atom, a halogen atom, hydroxyl group, a group of OZ₁₋₆ (the group of OZ₁₋₆ represents an alkyl group having 1-6 carbon atoms or a fluoroalkyl group having 1-6 carbon atoms, which bonds via the oxygen atom), a group of S(O)ₙZ₁₋₄ (Z₁₋₄ represents an alkyl group having 1-4 carbon atoms or a fluoroalkyl group having 1-4 carbon atoms or an alkylene group derived therefrom), a group of N(R¹²)(R¹³) (R¹² and R¹³ each independently represent hydrogen atom, an alkyl group having 1-4 carbon atoms or a fluoroalkyl group having 1-4 carbon atoms), a group of Z₁₋₈ which may be substituted (Z₁₋₈ represents an alkyl group having 1-8 carbon atoms or a fluoroalkyl group having 1-8 carbon atoms), a 5- to 7-membered cyclic alkyl group, an aryl group, a heteroaryl group, or a 4- to 7-membered saturated or partially saturated heterocyclic group (the cyclic alkyl group, aryl group, heteroaryl group and heterocyclic group may have one to three substituents selected from the group consisting of a halogen atom, hydroxyl group, a group of OZ₁₋₄, a group of S(O)ₙZ₁₋₄, a group of N(R¹²)(R¹³), a group of Z₁₋₄, carboxyl group, a group of CO₂Z₁₋₄, group of CONH₂, a group of CONH(Z₁₋₄) and a group of CON(Z₁₋₄)(Z₁₋₄));
W¹ represents a group selected from the group consisting of -CH=CH-, -N(R¹²)CO-, -CON(R¹²)-, -CH₂O- and -CH₂CH₂- (each of the aforementioned groups binds to the thiazole ring at the left end);
R³ represents hydrogen atom, a halogen atom, hydroxyl group or an amino group;
R⁴ represents a group selected from the group consisting of hydrogen atom, a group of -OZ₀₋₄R⁵ (Z₀₋₄ represents an alkylene group having 1-4 carbon atoms, a fluorine-substituted alkylene group having 1-4 carbon atoms or a single bond, and R⁵ represents a 5- to 7-membered cyclic alkyl group, an aryl group, a heteroaryl group or a 4- to 7-membered saturated or partially saturated heterocyclic group (the cyclic alkyl group, aryl group, heteroaryl group and heterocyclic group may have one to three substituents selected from the group consisting of a halogen atom, hydroxyl group, a group of OZ₁₋₄, a group of S(O)ₙZ₁₋₄, a group of N(R¹²)(R¹³), a group of Z₁₋₄, carboxyl group, a group of CO₂Z₁₋₄, group of CONH₂, a group of CONH(Z₁₋₄) and a group of CON(Z₁₋₄)(Z₁₋₄)), a group of -S(O)ₙZ₁₋₄R⁵, a group of -N(R⁶)(R⁷) {R⁶ and R⁷ each independently represent hydrogen atom or Z₁₋₄, or they may bind to each other to form a saturated or unsaturated 5- to 7-membered ring (the ring may contain one or two hetero atoms as ring constituting atoms), and R⁶ and R⁷ may have one to three substituents selected from the group consisting of a halogen atom, hydroxyl group, a group of OCON(R¹⁵)(R¹⁶), a group of CON(R¹⁵)(R¹⁶), a group of N(R¹²)CON(R¹⁵)(R¹⁶), a group of Z₁₋₄, a group of OZ₁₋₄, a group S(O)ₙZ₁₋₄, group of CH₂OH, a group of (CH₂)ₘN(R¹²)(R¹³), a group of Z₁₋₄CON(R¹⁵)(R¹⁶), a group of SO₂N(R¹²)(R¹³), a group of OSO₂N(R¹²)(R¹³), a group of OSO₂R¹², a group of NCOZ₁₋₄R¹⁵ (in the formula, R¹⁵ and R¹⁶ independently represent hydrogen atom, a group of Z₁₋₆R¹¹, a group of Z₁₋₄N(R¹²)(R¹³), a group of Z₁₋₄OH, and a group of Z₁₋₄OZ₁₋₄), carboxyl group, cyano group, a group of COZ₁₋₄R¹⁰, a group of CO-Z₁₋₄(R¹⁰)-N(R¹²)(R¹³) (R¹⁰ is a substituent corresponding to a side chain on an amino acid carbon or a group of -Z₁₋₄-R¹¹ (R¹¹ represents a substituent which forms a quaternary salt) and a group of 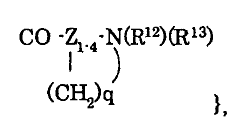 a 5- or 6-membered aryl group which may be substituted and a 5-or 6-membered unsaturated heterocyclic group which may be substituted;
W² represents a single bond or -C(R⁸)=C(R⁹)- (R⁸ and R⁹ each independently represent hydrogen atom, a halogen atom, a lower alkyl group, an alkoxy group, cyano group, carboxyl group, hydroxymethyl group, cyanomethyl group, vinyl group or a group of N(R¹²)(R¹³)), Q represents an acidic group, and W² and Q may bind together to form vinylidenethiazolidinedione in *E*- or *Z*-configuration or an equivalent heterocyclic ring; m and n each independently represent an integer of 0 to 2, and q represents an integer of 0 to 3.

As other aspects of the present invention, provided are a medicament for eliminating resistance of a microorganism with acquired drug resistance, which comprises a compound represented by the aforementioned general formula (I) or a physiologically acceptable salt thereof as an active ingredient; and a medicament for enhancing effect of an antimicrobial agent, which comprises a compound represented by the aforementioned general formula (I) or a physiologically acceptable salt thereof as an active ingredient. The aforementioned medicaments wherein the microorganism is *Pseudomonas aeruginosa* are preferred embodiments of the present invention. The present invention also provides a pharmaceutical composition for preventive and/or therapeutic treatment of microbial infections, which comprises a compound represented by the aforementioned general formula (I) or a physiologically acceptable salt thereof together with an antimicrobial agent.

As further aspects of the present invention, provided are a method for preventive and/or therapeutic treatment of microbial infections, which comprises the step of administering to a mammal including human a preventively and/or therapeutically effective amount of a compound represented by the aforementioned general formula (I) or a physiologically acceptable salt thereof; a method for eliminating resistance of a microorganism with acquired resistance to an antimicrobial agent, which comprises the step of contacting with the microorganism an effective amount of a compound represented by the aforementioned general formula (I) or a physiologically acceptable salt thereof; a method for inhibiting acquisition of resistance to an antimicrobial agent by a microorganism, which comprises the step of contacting with a microorganism an effective amount of a compound represented by the aforementioned general formula (I) or a physiologically acceptable salt thereof; a method for enhancing sensitivity of a microorganism to an antimicrobial agent, which comprises the step of contacting with a microorganism an effective amount of a compound represented by the aforementioned general formula (I) or a physiologically acceptable salt thereof; and a method for improving effect of an antimicrobial agent, which comprises the step of administering to a mammal including human an effective amount of a compound represented by the aforementioned general formula (I) or a physiologically acceptable salt thereof. The compounds represented by the aforementioned general formula (I) or physiologically, acceptable salts thereof are usually administered with one or more of antimicrobial agents simultaneously or separately, or successively. The present invention also provides a use of the compounds represented by the aforementioned general formula (I) or physiologically acceptable salts thereof for the manufacture of the aforementioned medicaments.

In addition to the aforementioned inventions, the present invention also provides a medicament for preventive and/or therapeutic treatment of microbial infections, which comprises as an active ingredient a compound represented by the following general formula (I), a physiologically acceptable salt thereof, or hydrates thereof

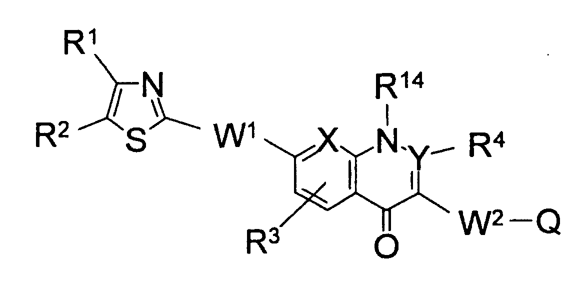

In the formula, R¹, R², R³, R⁴, W¹, W² and Q have the same meanings as those defined above; R¹⁴ represents hydrogen atom, Z₁₋₄, Z₁₋₄R⁵ or Z₁₋₄OR⁵; and X and Y each independently represent C-H or nitrogen atom.

In order to achieve the aforementioned second object, the inventors of the present invention conducted various researches. As a result, they found that judgment of effectiveness of a combination of a drug efflux pump inhibitor and an antibacterial agent was quickly and conveniently made by applying a method of antibiotic susceptibility test utilizing agar medium in which inhibition zones formed on the surface of the medium are observed, and that a type of drug efflux pump expressed in an etiologic bacterium was identifiable by using a combination of a particular antibacterial agent and a drug efflux pump inhibitor, and existence of two or more kinds of drug efflux pumps expressed in a microorganism was verified in a simple manner. The present invention was achieved on the basis of these findings.

The present invention thus provides a method for judging effectiveness of a drug efflux pump inhibitor-against a microorganism, which comprises the steps of:
(A1) spreading a microorganism to be tested on a surface of an agar medium, then providing an antibacterial agent as a spot on the surface of the agar medium and culturing the microorganism;
(A2) determining a growth degree of the microorganism in a region of the agar medium into which the antibacterial agent has diffused during the culture period;
(A3) determining a growth degree of the microorganism in a region of the agar medium in which the antibacterial agent that has diffused during the culture period and a drug efflux pump inhibitor contained in the agar medium coexist; and
(A4) judging that the drug efflux pump inhibitor is effective against the microorganism when the growth degree of the microorganism determined in the step (A2) is significantly higher than the growth degree of the microorganism determined in the step (A3).

As preferred embodiments of the aforementioned method, provided are the aforementioned method wherein the antibacterial agent is provided as a spot on the agar medium surface by means of a disk; the aforementioned method wherein the drug efflux pump inhibitor contained in the agar medium is the drug efflux pump inhibitor diffused from a disk provided as a spot on the agar medium surface; the aforementioned method wherein the drug efflux pump inhibitor contained in the agar medium is the drug efflux pump inhibitor added beforehand to the agar medium during preparation of the agar medium; and the aforementioned method wherein the microorganism is *Pseudomonas aeruginosa.*

In another preferred embodiment, the aforementioned method further comprises steps of (A5) providing two or more kinds of antibacterial agents each as a spot with a space between said spots in the step (A1); (A6) determining a growth degree of the microorganism in a region of the agar medium in which the two or more kinds of antibacterial agents that have diffused into the agar medium during culture period coexist; (A7) determining a growth degree of the microorganism in a region of the agar medium in which the two or more kinds of antibacterial agents that have diffused during the culture period and a drug efflux pump inhibitor contained in the agar medium coexist; and (A8) judging that the drug efflux pump inhibitor is effective against the microorganism when the growth degree of the microorganism determined in the step (A6) is significantly higher than the growth degree of the microorganism determined in the step (A7).

As preferred embodiments of the aforementioned method, provided are the aforementioned method wherein the antibacterial agents are provided each as a spot on the agar medium surface each by using a disk; the aforementioned method wherein the drug efflux pump inhibitor contained in the agar medium is the drug efflux pump inhibitor diffused from a disk provided as a spot on the agar medium surface; the aforementioned method wherein the drug efflux pump inhibitor contained in the agar medium is the drug efflux pump inhibitor added beforehand to the agar medium during preparation of the agar medium; and the aforementioned method wherein the microorganism is *Pseudomonas aeruginosa.*

As another aspect, the present invention provides a method for identifying a drug efflux pump expressed in a microorganism, which comprises the steps of:
(B1) spreading a microorganism to be tested on a surface of an agar medium, then providing an antibacterial agent that can be excreted by a particular drug efflux pump as a spot on the surface of the agar medium and culturing the microorganism;
(B2) determining a growth degree of the microorganism in a region of the agar medium into which the antibacterial agent has diffused during culture period;
(B3) determining a growth degree of the microorganism in a region of the agar medium in which the antibacterial agent that has diffused during the culture period and a drug efflux pump inhibitor contained in the agar medium coexist (provided that said drug efflux pump inhibitor is a specific inhibiter for the particular drug efflux pump); and
(B4) judging that the microorganism expresses the drug efflux pump of the particular type when the growth degree of the microorganism measured in the step (B2) is significantly higher than the growth degree of the microorganism determined in the step (B3).

As preferred embodiments of the aforementioned method, provided are the aforementioned method wherein the antibacterial agent is provided as a spot on the agar medium surface by using a disk; the aforementioned method wherein the drug efflux pump inhibitor contained in the agar medium is the drug efflux pump inhibitor diffused from a disk provided as a spot on the agar medium surface; the aforementioned method wherein the drug efflux pump inhibitor contained in the agar medium is the drug efflux pump inhibitor added beforehand to the agar medium during preparation of the agar medium; and the aforementioned method wherein the microorganism is *Pseudomonas aeruginosa.*

As a further aspect, the present invention provides a method for verifying expression of two or more kinds of drug efflux pumps in a microorganism, which comprises the steps of:
(C1) spreading a microorganism to be tested on a surface of an agar medium, then providing two or more kinds of antibacterial agents (provided that each of the two or more kinds of the antibacterial agents has different effluxing specificity by the two or more kinds of drug efflux pumps, and one of the two or more kinds of the antibacterial agents (hereinafter referred to as "Antibacterial agent (1)") has a property of being excreted by only one of the two or more kinds of the drug efflux pumps (hereinafter referred to as "Drug efflux pump (1)"), whilst the other antibacterial agent or agents have a property of being excreted by Drug efflux pump (1) and the other drug efflux pump or pumps);
(C2) determining a growth degree of the microorganism in a region of the agar medium into which each antibacterial agent has solely diffused during culture period;
(C3) determining a growth degree of the microorganism in a region of the agar medium in which each antibacterial agent that has solely diffused during the culture period and a drug efflux pump inhibitor contained in the agar medium coexist (provided that the drug efflux pump inhibitor is a specific inhibiter for Drug efflux pump (1)); and
(C4) judging that the microorganism expresses Drug efflux pump (1) and one or more kinds of other drug efflux pumps when the growth degree of the microorganism determined in the step (C2) is significantly higher than the growth degree of the microorganism determined in the step (C3) for Antibacterial agent (1) and the growth degree of the microorganism determined in the step (C2) is significantly lower than the growth degree of the microorganism determined in the step (C3) for the other antibacterial agent or agents.

As preferred embodiments of the aforementioned method, provided are the aforementioned method wherein the two or more kinds of antibacterial agents include a combination of a *β*-lactam antibiotic and a quinolone antibacterial agent; the aforementioned method wherein one of the two or more kinds of drug efflux pumps is a MexAB-OprM pump; and the aforementioned method wherein the microorganism is *Pseudomonas aeruginosa.* Also provided are the aforementioned method wherein each of the antibacterial agents is provided as a spot on the agar medium surface each by using a disk; the aforementioned method wherein the drug efflux pump inhibitor contained in the agar medium is the drug efflux pump inhibitor diffused from a disk provided as a spot on the agar medium surface; and the aforementioned method wherein the drug efflux pump inhibitor contained in the agar medium is the drug efflux pump inhibitor added beforehand to the agar medium during preparation of the agar medium.

### Brief Explanation of the Drawings

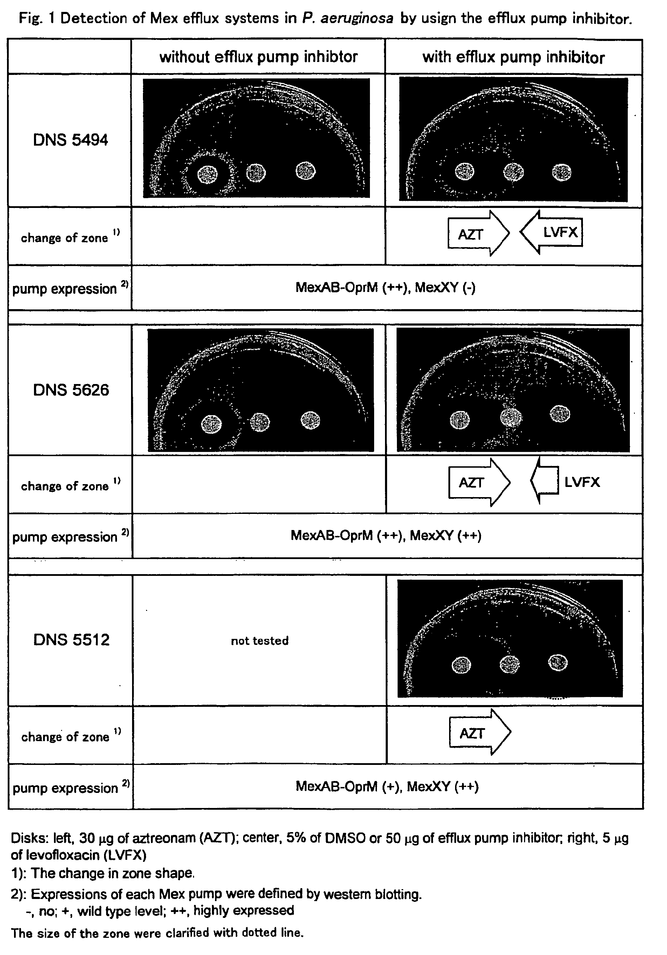

Fig. 1 shows the results of determination performed by using disks according to the method of the present invention. In the figure, the disk on the left side contained Aztreonam (AZT: 30 µ g), the disk at the center contained 5% DMSO solution alone (negative control) in the case of without efflux pump inhibitor or Compound A (50 µ g) in the case of with efflux pump inhibitor, and the disk on the right side contained Levofloxacin (LVFX: 5 µ g). Change of zone represents the change in zone shape on the agar medium, and WB represents the result of the protein analysis by Western blotting method.

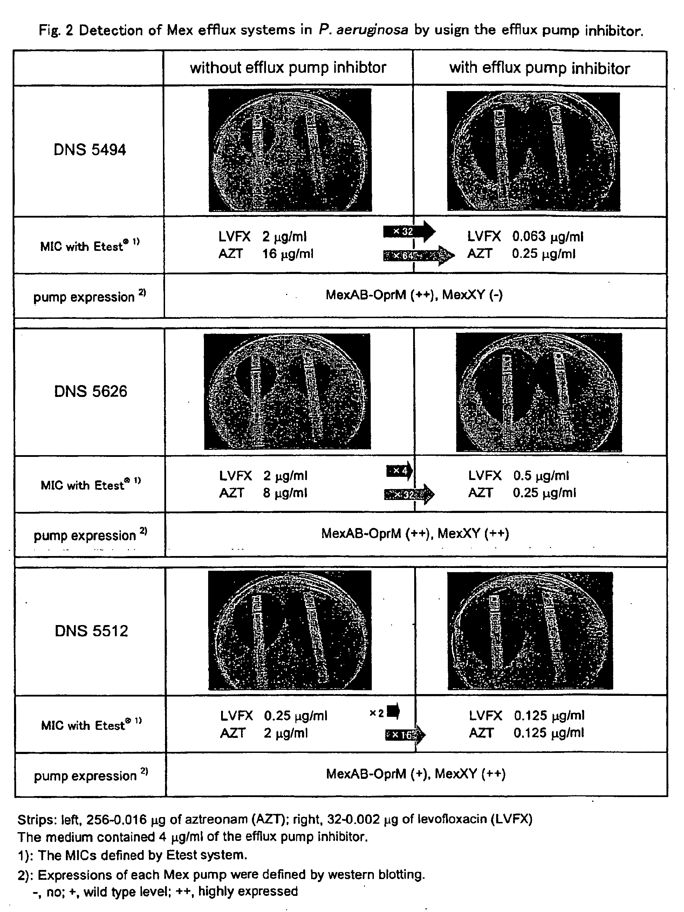

Fig. 2 shows the results of the measurement performed by using Etest instead of disks. In the figure, Etest on the left side contained Aztreonam (AZT: 256-0.016 µ g/mL) and Etest on the right side contained Levofloxacin (LVFX: 32-0.002 µ g/mL). MIC with Etest represents the minimum inhibitory concentration defined by Etest system, and WB represents the result of the protein analysis by Western blotting method.

### Best Mode for Carrying out the Invention

In the specification, the "alkyl group" means a linear, branched or cyclic alkyl group or an alkyl group consisting of a combination thereof. The same is applied to an alkyl moiety of a substituent having the alkyl moiety (for example, fluoroalkyl group).

In the general formula (I), R¹ is preferably an alkyl group. Examples of the alkyl group include a linear or branched alkyl group having 1-8 carbon atoms (for example, methyl group, ethyl group, n-propyl group, isopropyl group, n-butyl group, isobutyl group, sec-butyl group, tert-butyl group, n-pentyl group and the like), and a cyclic alkyl group having 3-8 carbon atoms, preferably 3-6 carbon atoms (for example, cyclopropyl group, cyclobutyl group, cyclopentyl group, cyclohexyl group and the like). The cyclic alkyl group may have another alkyl group or a halogen atom, hydroxyl group or the like on the ring. R¹ is also preferably an aryl group, a heteroaryl group or a heterocyclic group. The aryl group may preferably has a 5- or 6-membered ring. As the heterocyclic group, a heterocyclic group having 3-8, preferably 3-6, of ring constituting atoms can be used, and the ring may be saturated or partially saturated. As a substituent that binds to the heterocyclic group, for example, an alkyl group or a halogen atom is preferred.

R² is preferably hydrogen atom or a halogen atom.

W¹ is preferably a linking group having a length of two atoms, and -CH=CH-, -N(R¹²)CO-, -CON(R¹²)-, -CH₂O- and -CH₂CH₂- are more preferred.

R³ is preferably hydrogen atom, a halogen atom, amino group or hydroxyl group.

R⁴ is preferably hydrogen atom, -OZ₀₋₄R⁵ or -N(R⁶)(R⁷).

R⁵ is preferably a 5- or 6-membered aryl group, 5- to 7-membered alicyclic group, 4- to 7-membered saturated heterocyclic group, or 5- or 6-membered unsaturated heterocyclic group, which may be substituted, and more preferably a 4- to 7-membered saturated heterocyclic group or a 5- or 6-membered unsaturated heterocyclic group.

R⁶ and R⁷ represent hydrogen atom, an alkyl group having 1-4 carbon atoms or a fluoroalkyl group having 1-4 carbon atoms, or they bind to each other to form a saturated or unsaturated 5- to 7-membered ring. The ring may contain one or two hetero atoms as atoms constituting the ring. R⁶ and R⁷ may have one to three substituents selected from the group consisting of a halogen atom, hydroxyl group, a group of OCON(R¹⁵)(R¹⁶), a group of CON(R¹⁵)(R¹⁶), a group of N(R¹²)CON(R¹⁵)(R¹⁶), a group of Z₁₋₄, a group of OZ₁₋₄, a group S(O)ₙZ₁₋₄, group of CH₂OH, a group of (CH₂)ₘN(R¹²)(R¹³), a group of Z₁₋₄CON(R¹⁵)(R¹⁶), a group of SO₂N(R¹²)(R¹³), a group of OSO₂N(R¹²)(R¹³), a group of OSO₂R¹², a group of NCOZ₁₋₄R¹⁵ (in the formula, R¹⁵ and R¹⁶ independently represent hydrogen atom, a group of Z₁₋₆R¹¹, a group of Z₁₋₄N(R¹²)(R¹³), a group of Z₁₋₄OH, and a group of Z₁₋₄OZ₁₋₄), carboxyl group, cyano group, a group of COZ₁₋₄R¹⁰, a group, of CO-Z₁₋₄(R¹⁰)-N(R¹²)(R¹³) (R¹⁰ is a substituent corresponding to a side chain on an amino acid carbon or a group of -Z₁₋₄-R¹¹ (R¹¹ represents a substituent which forms a quaternary salt) and a group of 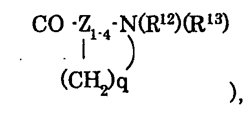 wherein R¹⁰ is a substituent on α -carbon atom of an amino acid, or a group comprising an alkyl group or a fluoroalkyl group having 1-4 carbon atoms and a quaternary salt such as 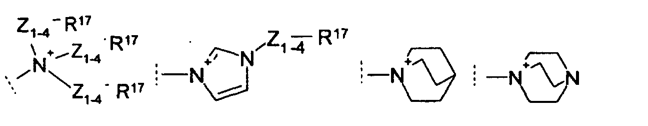 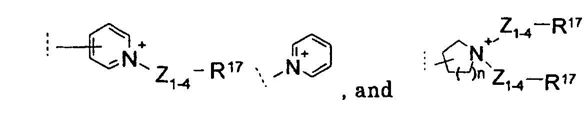 substituted at the terminal of said alkyl or fluoroalkyl group (in the definitions of the aforementioned substituents, R¹² and R¹³ represent hydrogen atom, an alkyl group or a fluoroalkyl group having 1-4 carbon atoms, n independently represents an integer of 0 to 2, and q represents an integer of 0 to 3). Preferred examples of R¹¹ also include a quaternary salt such as 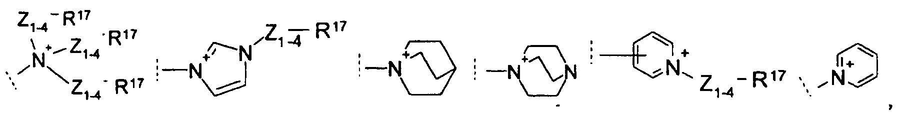 and 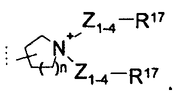 R¹⁷ represents hydrogen atom, halogen atom, hydroxyl group, carboxyl group, carbamoyl group, N-alkylcarbamoyl group, N,N-dialkylcarbamoyl group, dihalogenomethyl group, or trihalogenomethyl group.

More preferably, R⁶ and R⁷ form a piperidine ring or the like and said ring has a substituent such as fluorine atom, hydroxyl group, a group of OCON(R¹⁵)(R1⁶) and a group of CON(R¹⁵)(R¹⁶) and a group of Z₁₋₄CON(R¹⁵)(R¹⁶) on the ring. As the ring formed by R⁶ and R⁷ bound to each other, a piperazine ring is also preferred, and preferred examples include a piperazine ring of which 4-position (nitrogen atom not bound to the pyridopyrimidine ring) is substituted with an alkyl group or a fluoroalkyl group having 1-4 carbon atoms, or with other substituent such as COZ1₋₄R¹⁰, CO-Z₁₋₄(R¹⁰)-N(R¹²)(R¹³) and

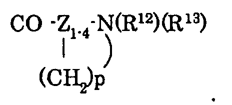

W² is preferably a single bond or a group represented by -C(R⁸)=C(R⁹)-. R⁸ and R⁹ each independently represent hydrogen atom, a halogen atom, a lower alkyl group, an alkoxy group, cyano group, carboxyl group, hydroxymethyl group, cyanomethyl group, vinyl group or a group of N(R¹²)(R¹³), more preferably hydrogen atom, a halogen atom or a lower alkyl group.

Q represents an acidic group, preferably an acidic group which is carboxyl group, 1,2,3,4-tetrazol-5-yl group or an equivalent thereof. However, type of the acidic group is not particularly limited, and said group may be any one of cyclic or non-cyclic, or a combination thereof. Examples include a lower alkoxy group, hydroxyl group, carboxyl group, *N*-cyanocarboxamido group, a methanesulfonylamido group having 1-3 fluorine atoms, -CONH-(5-tetrazolyl) group, 5-tetrazolyl group which may be substituted, 1,2,3-triazolyl group which may be substituted, 2,4-dioxothiazolidin-5-ylidenyl group which may be substituted, 4-oxo-2-thioxothiazolidin-5-ylidenyl group which may be substituted, 5-oxo-4-tetrazolyl group which may be substituted, 3-(5-oxo)-[1.2.4]oxadiazolidinyl group which may be substituted, 2-(3,5-dioxo)-[1.2.4]oxadiazolidinyl group which may be substituted, 5-(3-oxo)-[1.2.4]oxadiazolidinyl group which may be substituted, 3-(5-oxo)-[1.2.4]isoxazolidyl group which may be substituted or the like. More preferred examples include carboxyl group, 5-tetrazolyl group which may be substituted, N-cyanocarboxamido group, a methanesulfonylamido group that has 1-3 fluorine atoms, -CONH-(5-tetrazolyl) group or the like.

For R¹, R², R³, R⁴, W¹, W² and Q of the compounds represented by the general formula (II), those explained as for R¹, R², R³, R⁴, W¹, W² and Q in the aforementioned general formula (I) can be preferably used, respectively.

R¹⁴ is preferably an alkyl group having 1-4 carbon atoms or a fluoroalkyl group having 1-4 carbon atoms.

The compounds represented by the aforementioned general formula (I) can be produced by the following methods.

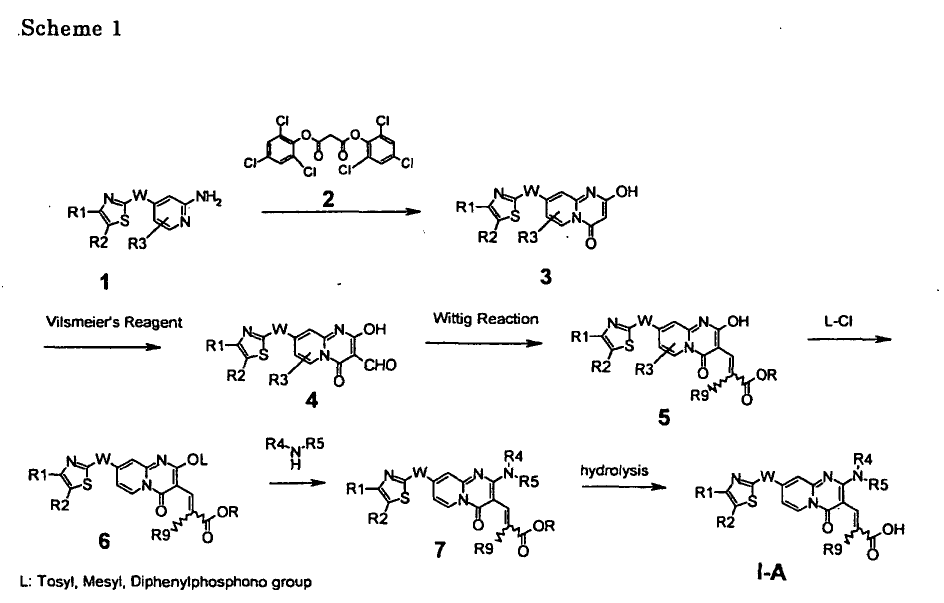

As shown in Scheme 1, Compound 3 can be obtained by heating Aminopyridine derivative 1 and malonic acid or an ester of malonic acid in a solvent such as toluene and xylene. As the ester of malonic acid, Ester 2 as a phenol ester substituted with an electron-withdrawing group such as a halogen atom can be used. An alkyl ester can also be used. By formylating Compound 3 with Vilsmeier's reagent prepared from phosphorus oxychloride or oxalyl chloride and dimethylformamide (DMF), Aldehyde 4 can be obtained. By subjecting Compound 4 to the Wittig reaction or Horner-Emons reaction, Acrylic acid derivative 5 can be synthesized. Where the Wittig reaction is employed, Compound 5 can be obtained by reacting an alkyloxycarbomethylenetriphenylphosphorane which may be substituted with the aldehyde compound in an inert solvent such as tetrahydrofuran, DMF or methylene chloride. Where the Horner-Emons reaction is employed, the target compound can be synthesized by reacting the aldehyde compound with a dialkylphosphonoacetic acid ester which may be substituted at the 2-position in the presence of a base in an inert solvent such as THF and DMF. Compound 7 having amino group at the 2-position as a linker can be obtained by converting the hydroxyl group at the 2-position of Compound 5 into tosyl group, mesyl group, diphenylphosphoric acid ester or the like, and then replacing the resulting group with an amine. The ester group of the acrylic acid moiety can be hydrolyzed in the final step to obtain Compound I-A. As the ester group, a lower alkyl ester such as those of methyl group and ethyl group, tert-butyl ester, benzyl ester, allyl ester and so forth can be used, and they can be hydrolyzed by hydrolysis under an alkaline or acidic condition, catalytic reduction or a method by using a metal catalyst such as palladium.

Further, Compound I-A can also be synthesized by the method shown in Scheme 2. Compound 9 can be obtained by converting the hydroxyl group at the 2-position of Compound 3 into tosyl group, mesyl group, diphenylphosphoric acid ester or the like and then replacing the resulting group with an amine. The resulting product can be formylated with Vilsmeier's reagent prepared from phosphorus oxychloride or oxalyl chloride and dimethylformamide to obtain Aldehyde 10. By subjecting Compound 10 to the Wittig reaction or Horner-Emons reaction, Acrylic acid derivative 7 can be synthesized. Where the Wittig reaction is employed, Compound 7 can be obtained by reacting an alkyloxycarbomethylenetriphenylphosphorane which may be substituted with the aldehyde compound in an inert solvent such as THF, DMF, toluene or methylene chloride. Where the Horner-Emons reaction is employed, the target compound can be synthesized by reacting the aldehyde compound with a dialkylphosphonoacetic acid ester which may be substituted at the 2-position in the presence of a base in an inert solvent such as THF and DMF.

According to this synthetic scheme, various primary or secondary amines can be reacted with Compound 6 to synthesize variety of 2-substituted derivatives by the multiple parallel synthesis method in a liquid phase.

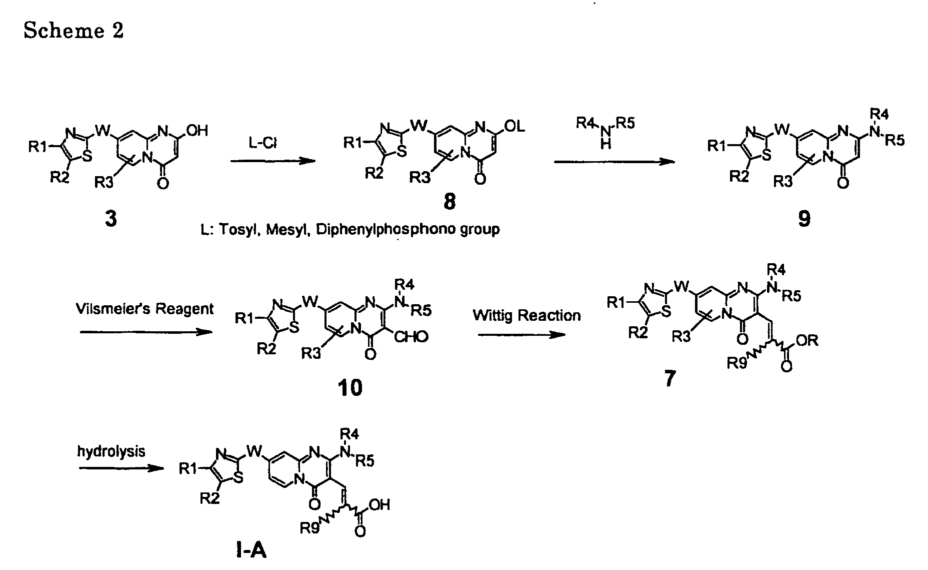

A compound in which a substituent is introduced at the 2-position of the pyridopyrimidine ring as a linker can be synthesized by the method shown in Scheme 3.

Compound 11 can be synthesized by reacting Compound 5 with an alkylating agent in the presence of a base. The ester portion of the resulting Compound 11 can be hydrolyzed to obtain Compound I-B in a manner similar to that for the aforementioned compound having a nitrogen atom as a linker.

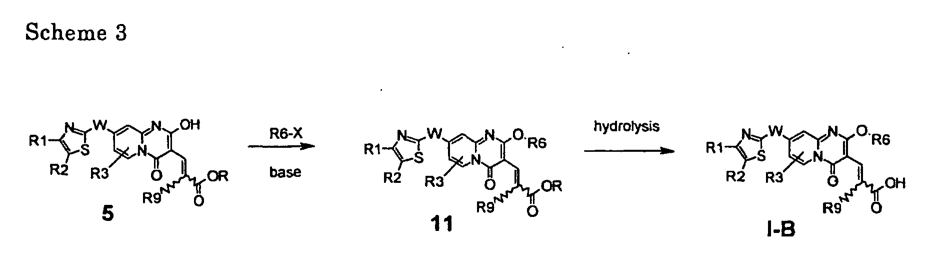

Compound I-B can also be synthesized by the method shown in Scheme 4, i.e., by alkylation of Compound 3 with an alkylating agent and subsequent formylation, olefination and deprotection of the ester portion.

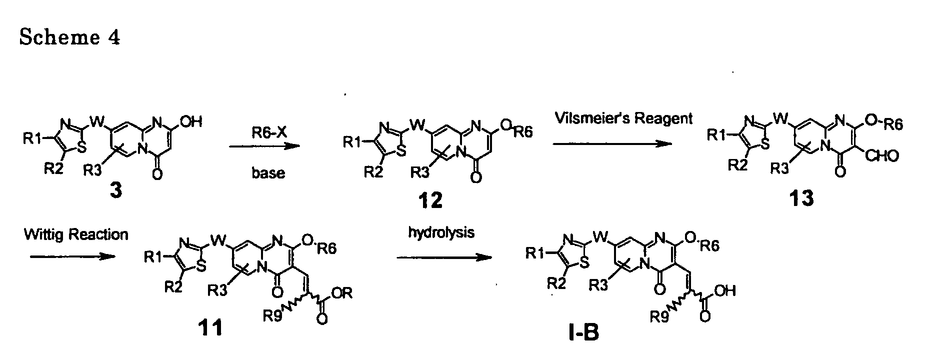

Compound 1 used for the aforementioned synthesis can be synthesized as follows.

For example, Compound 1-A of which W portion is an amide bond can be synthesized by condensing an aminothiazole derivative, which is a known compound or can be synthesized by a known method, and a 2-aminopyridine-4-carboxylic acid derivative of which amino group is protected by a usual method used for a reaction of forming a peptide bond, and deprotecting the protective group of the amino group.

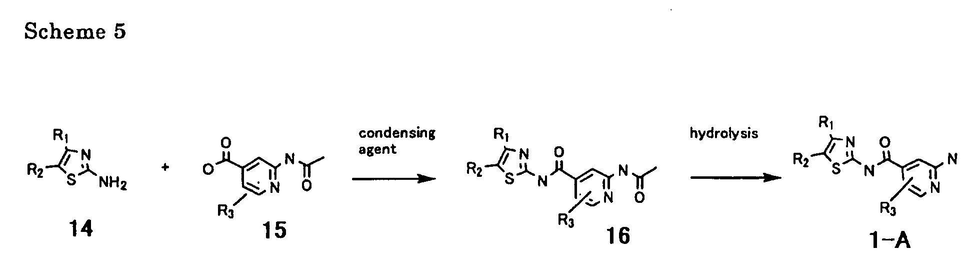

Compound 1-B of which W portion is a double bond can be synthesized by condensing 2-Methylthiazole derivative 17, which is a known compound or can be synthesized by a known method, and 2-Aminopyridine-4-carbaldehyde derivative 18 of which amino group is protected under a condition for the Knoevenagel reaction, and removing the protective group of the amino group. As for the condition of the Knoevenagel reaction, the reaction can also be performed by heating in acetic anhydride or in the presence of a base such as piperidine and piperazine and in the co-presence of an acid such as acetic acid.

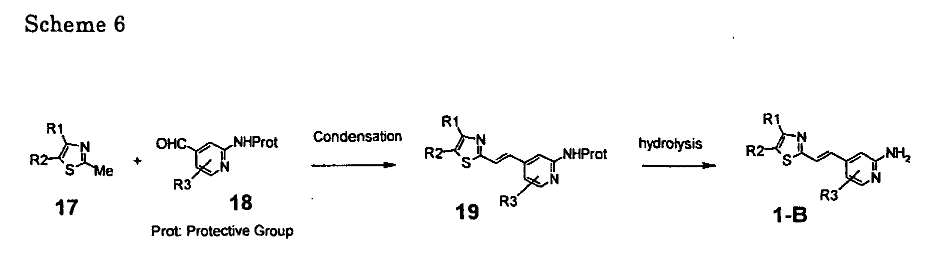

An anion that can be obtained by treating 2-Amino-4-methylpyridine derivative 20 of which amino group is protected with a strong base such as n-BuLi and an aldehyde are reacted, and then the resulting hydroxyl group can be subjected to tosylation, mesylation or the like, or the resulting product is converted into a halogenated compound such as by chlorination or bromination. The resulting product is then subjected to elimination reaction using a base such as DBU to obtain Double bond-containing derivative 23. By removing its amino protective group, Compound 1-C can be synthesized.

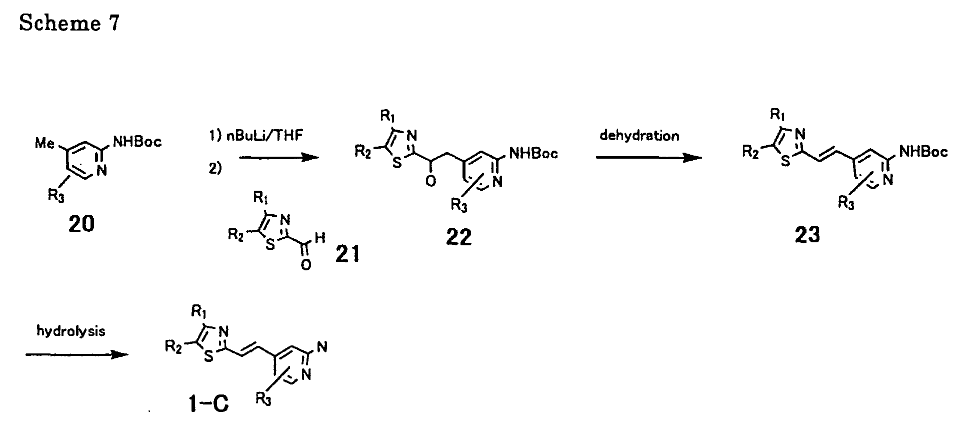

Compound 1-D of which W portion is an ether bond can be synthesized by condensing Thiazolemethyl halide 24, which is a known compound or can be synthesized by a known method, and 4-Hydroxypyridine-2-carboxylic acid ester 25 in the presence of a base, hydrolyzing the ester portion of the resulting Compound 26, and then converting the produced carboxylic acid into Amine derivative 28 by employing the Curtius rearrangement or the like. Amino compound 1-D can also be obtained by first converting the carboxylic acid into a condensation product with hydrazine, then converting the resulting product into an acid azide with a nitrous acid salt or a derivative thereof, further subjecting the resulting product to a rearrangement reaction and, if required, removing the protective group of the amino group.

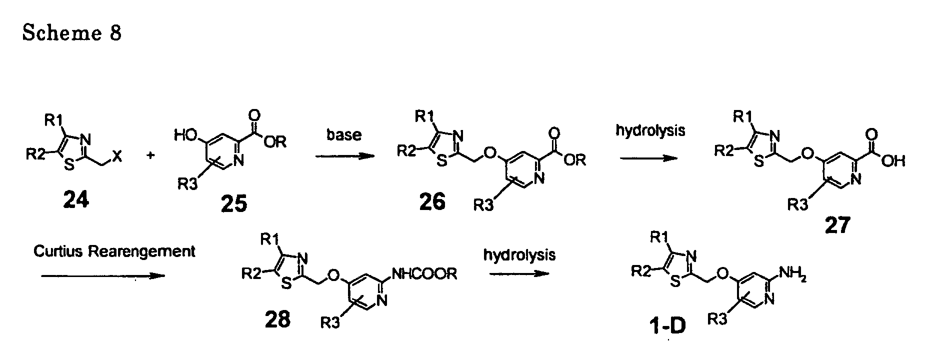

Compound 1-E, where W portion is ethylene, can be synthesized by reacting an anion, obtained by treating 2-Amino-4-methylpyridine derivative 20 of which amino group is protected with a strong base such as n-BuLi, with Thiazolemethyl halide 24 to obtain a condensation product and then removing the protective group of the amino group.

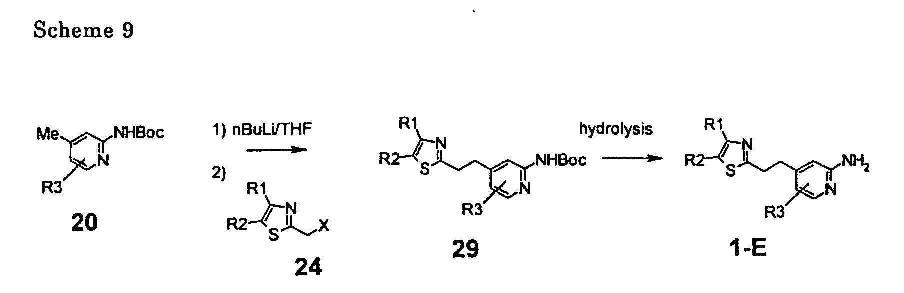

As shown in Scheme 10, Compound 1-E can also be synthesized by reacting an anion, obtained by treating 2-Amino-4-methylpyridine derivative 20 of which amino group is protected with a strong base such as n-BuLi, with Halogenoacetic acid derivative 30 to obtain a condensation product, converting the ester portion into an amide, then converting the resulting product into a thioamide compound by using Lawesson's reagent, diphosphorous pentasulfide or the like and condensing the product with a haloketone compound.

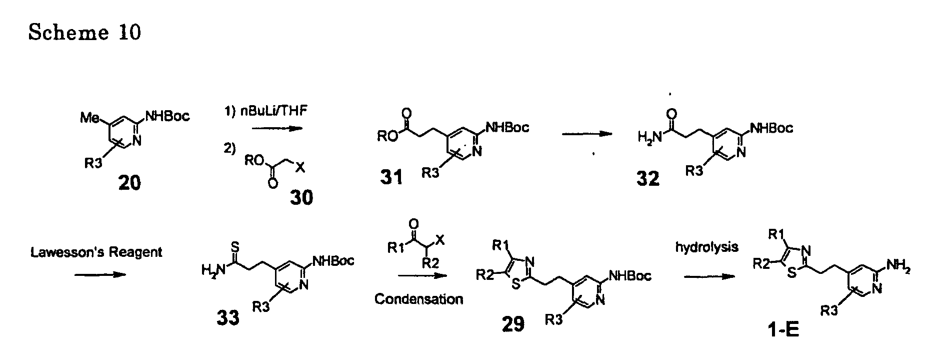

A compound of which W portion is a triple bond can be synthesized by the synthetic methods described in WO9633181 and WO961024.

The compound represented by the general formula (II) can be synthesized by the method shown in Scheme 11.

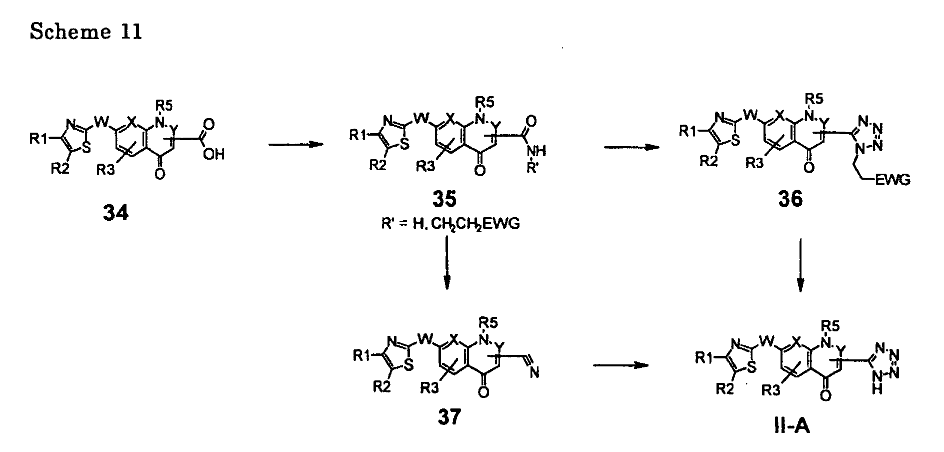

Amide derivative 35 can be obtained by converting N-Alkyl-quinolone-carboxylic acid derivative 34 described in PCT/JP00/07565 into a mixed acid anhydride, and then reacting the resulting product with ammonia or an ethylamine having an electron-withdrawing group (EWG) such as cyanoethylamine and 3-aminopropanoic acid ester. The alkylamide derivative can be converted into Compound 36 by treatment with sodium azide and trifluoromethanesulfonic acid anhydride in an acetonitrile solvent to form a tetrazole ring. Then, the product can be converted into Tetrazole compound II-A by treatment with DUB in an inert solvent such as methylene chloride or treatment with a base such as sodium methoxide in an alcohol.

Further, Tetrazole compound II-A can similarly be obtained by subjecting Carbamoyl derivative 35 to dehydration reaction to obtain Cyano derivative 37 and treating the resulting compound with sodium azide and aluminum chloride in dimethylformamide. Similarly, Tetrazole compound II-A can be produced by subjecting Carboxylic acid 34 having a cinnolin-4-one or naphthylidin-4-one structure to the same treatment.

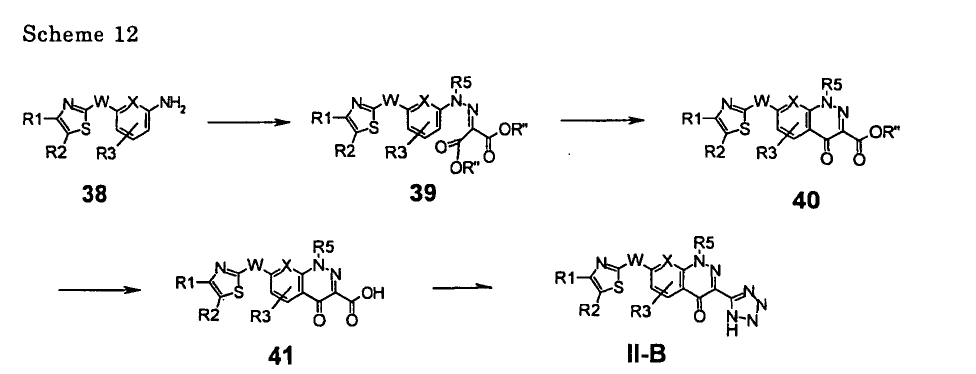

As shown in Scheme 12, where a derivative having a cinnolin-4-one structure is desired, Compound 38, which is a known compound or can be easily derived from a known compound, can be converted into a diazo compound by treating the amino group with sodium nitrite, and the resulting product can be reacted with a malonic acid ester to obtain Compound 39 (R⁵ = H). An electrophilic regent such as an alkyl halide can be reacted with the nitrogen atom of the resulting Compound 39 (R⁵ = H) by using a base such as sodium hydride and potassium carbonate in a solvent such as DMF and THF, and then the product can be heated in a solvent such as Dowtherm A and PPA and treated under an ordinary hydrolysis condition to obtain Carboxylic acid 41. The Carboxylic acid 41 obtained can be subjected to a treatment similar to the aforementioned treatment (Scheme 11) to produce Tetrazole compound II-B.

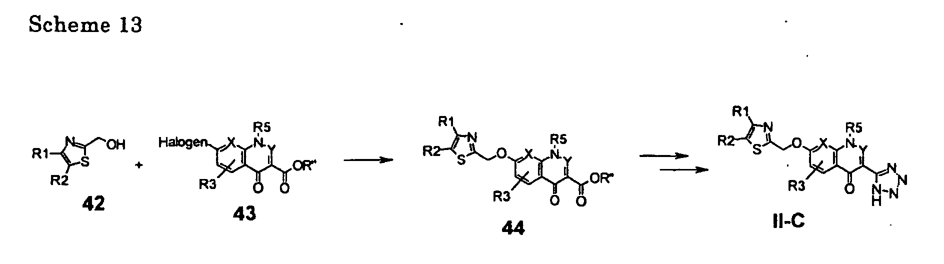

As shown in Scheme 13, when W portion is -CH₂O- Compound 42 can be converted into an alkoxide by treatment with a strong base such as sodium hydride in DMF or THF, and the alkoxide can be reacted with Compound 43, which is a known compound or can be synthesized by a known method (Japanese Patent Un-examined Publication (Kokai) No. 57-144264 or 60-197686), to obtain Compound 44.
Subsequently, Compound 44 can be converted into a carboxylic acid by hydrolysis and subjected to a treatment similar to the aforementioned treatment (Scheme 11) to produce Tetrazole compound II-C.

As for the aforementioned production of cinnolin-4-one derivative, a method wherein 3-chloro-4-fluoroaniline is used as a starting material is compared with a method wherein 3,4-difluoroaniline is used, 3,4-difluoroaniline will give much higher yield of cinnolin-4-one-3-carboxylic acid as for the thermal cyclization reaction performed in a solvent such as Dowtherm A and PPA in the production of the known Compound 43 and selectivity of the substitution of the alkoxide shown in Scheme 13.

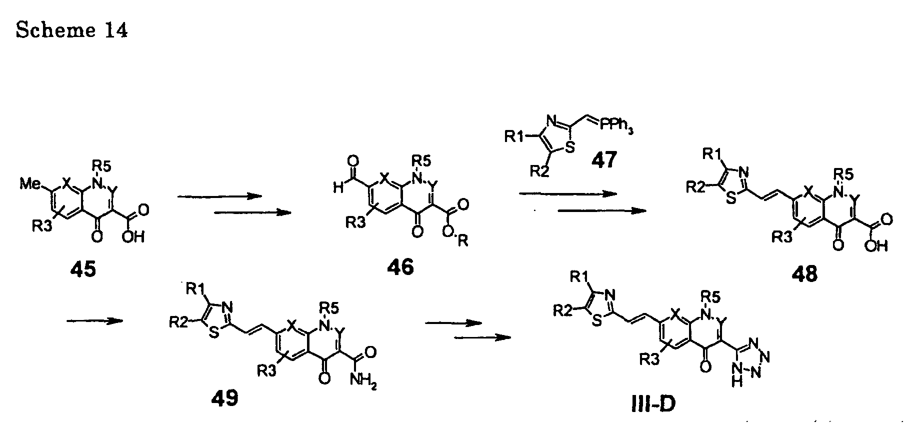

As shown in Scheme 14, where the compound in which W is an olefin is desired, Compound 45, which is a known compound or can be synthesized by a known method, can be esterified and then subjected to treatment with an oxidizing agent such as selenium dioxide, and the resulting Aldehyde derivative 46 and Compound 47 can be subjected to the Wittig reaction and further subjecting the adduct to addition of ammonia to obtain Carbamoyl compound 49. The resulting Carbamoyl derivative 49 can be converted into Tetrazole compound II-D by using the same method as in Scheme 11.

The synthetic intermediates and the target compounds in the aforementioned preparations can be isolated and purified by using methods for isolation and purification ordinarily used in the field of organic synthetic chemistry, for example, neutralization, filtration, extraction, drying, concentration, recrystallization, various chromatographic techniques and the like. The synthetic intermediates may be used in subsequent reactions without purification. When a salt of compound of the general formula (I) or (II) is desired, a product obtained in the form of a salt may be purified without any treatment. When a product is obtained in a free form, a salt can be formed by dissolving or suspending the product in a suitable organic solvent, and then adding an acid or base. It is also possible to convert a compound represented by the general formula (I) or (II) obtained in the form of a salt into a compound in a free form, and then convert the result into an appropriate salt.

Although it is not intended to be bound by any specific theory, the compounds represented by the general formula (I) have an activity for inhibiting drug efflux pumps of microorganisms. More specifically, the compounds represented by the general formula (I) can act on a microorganism with acquired resistance to an antimicrobial agent to inhibit its drug efflux pump, and eliminate the resistance of the microorganism. In addition, the compounds represented by the general formula (I) can act on a microorganism to inhibit a drug efflux pump, thereby suppress the acquisition of resistance to an antimicrobial agent by a microorganism. Therefore, the medicament of the present invention that comprises a compound represented by the general formula (I) as an active ingredient is useful for preventive and/or therapeutic treatment of microbial infections, generally by a combinational administration with an antimicrobial agent. The medicament of the present invention is extremely useful as a medicament for preventive and/or therapeutic treatment of, in particular, infectious diseases caused by a microorganism with acquired resistance to one or more antimicrobial agents.

Methods for using the medicament of the present invention are not particularly limited. Examples include a method of administering one or more antimicrobial agents, and also administering the medicament of the present invention simultaneously, separately, or successively to enhance the activity of the antimicrobial agent(s); and a method of preparing a pharmaceutical composition comprising one or more antimicrobial agents and the medicament of the present invention (so-called a compound drug) and the administering the composition.

Kinds of microbial infections that are applicable by the medicament of the present invention are not particularly limited. Bacteria are suitable as target microorganisms. The medicament of the present invention can be used for various infections by microorganisms including Gram-positive or Gram-negative bacteria, aerobic or anaerobic bacteria and the like. The medicament of the present invention can most suitably be used for infections by *Pseudomonas aeruginosa* with acquired resistance to one or more antimicrobial agents, or infections by *Pseudomonas aeruginosa* with low sensitivity to antimicrobial agents. The medicament of the present invention can be used for microbial infections of mammals including human.

Drugs having variety of structures have been known as antimicrobial agents, and various drugs are clinically used. Kinds of antimicrobial agents that can be administered in combination with the medicament of the present invention are not particularly limited, and examples include, for example, penicillin (penam) antibiotics, cephalosporin (cephem) antibiotics, oxacephem antibiotics, penem antibiotics, carbapenem antibiotics, monobactam antibiotics, aminoglycoside antibiotics, macrolide antibiotics, chloramphenicol antibiotics, tetracycline antibiotics, glycopeptide antibiotics, phosphomycin antibiotics, lincomycin antibiotics, sulfonamide preparations, p-aminosalicylic acid preparations, isonicotinic acid hydrazide preparations, quinolone synthetic antimicrobial agents and the like. However, antimicrobial agents are not limited to these examples. When a pharmaceutical composition comprising one or more antimicrobial agents together with the medicament of the present invention is manufactured, the antimicrobial agents exemplified above can also be used.

As the active ingredient of the medicament of the present invention, a substance selected from the group consisting of the compounds represented by the formula (I) and pharmaceutically acceptable salts thereof, and hydrates thereof and solvates thereof can be used. Two or more of the substances may be used in combination. The aforementioned substance, per se, may be administered as the medicament of the present invention. Generally, however, it is desirable that the substance is administered in the form of a pharmaceutical composition comprising one or more of the aforementioned substances as the active ingredient together with one or more pharmaceutical additives. The pharmaceutical composition may optionally be added with one or more of other pharmaceutically active ingredients such as the aforementioned antimicrobial agents and *β*-lactamase inhibitors.

A pharmaceutical composition for the use of administration *in vivo* can be readily prepared by mixing one or more of the aforementioned substances as the active ingredient and one or more pharmaceutically acceptable additives for pharmaceutical preparations according to methods for formulation ordinarily used in the field of manufacturing pharmacy. The route of administration of the medicament of the present invention is not particularly limited; however, it is desirable to appropriately chose the most effective administration route for preventive and/or therapeutic treatment of a target infectious disease. Examples of pharmaceutical compositions suitable for oral administration include, for example, capsules, powders, tablets, granules, subtilized granules, emulsions, syrups, solutions, suspensions and the like. Examples of pharmaceutical compositions suitable for parenteral administration include, for example, inhalants, sprays, intrarectal preparations, injections, drip infusions, ointments, creams, transdermal preparations, transmucosal preparations, eye drops, nasal drops, ear drops, tape preparations, patches and the like. However, the forms of the medicament of the present invention are not limited to these examples.

Among the pharmaceutical compositions suitable for oral administration, liquid preparations such as emulsions and syrups can be prepared by using pharmaceutical additives including water; saccharides such as sucrose, sorbitol, fructose; glycols such as polyethylene glycol and propylene glycol; oils such as sesame oil, olive oil and soybean oil; antiseptics such as p-hydroxybenzoic acid esters; flavors such as strawberry flavor and peppermint and the like. Solid preparations such as capsules, tablets, powders and granules can be prepared by using excipients such as lactose, glucose, sucrose and mannitol; disintegrating agents such as starch and sodium alginate; lubricants such as magnesium stearate and talc; binders such as polyvinyl alcohol, hydroxypropylcellulose and gelatin; surfactants such as fatty acid esters; plasticizers such as glycerin and the like.

Among the pharmaceutical compositions suitable for parenteral administration, liquid preparations such as injections, drip infusions and eye drops can preferably be prepared as sterilized isotonic liquid preparations. For example, injections can be prepared by using an aqueous medium such as a solution of sodium chloride, a solution of glucose, or a mixture of saline and glucose solution. The intrarectal preparations can be prepared generally in the form of suppositories by using carriers such as cacao butter, hydrogenated fat and hydrogenated carboxylic acid. For the preparation of sprays, a non-irritable carrier can be used that enables fine dispersion and enhances absorption of the aforementioned substances as the active ingredient. Examples of such a carrier include lactose, glycerin and the like. Aerosols, dry powders or the like can also be chosen as the form of preparation. However, pharmaceutical additives used for the manufacture of the medicament of the present invention are not limited to those mentioned above, and any additives available for those skilled in the art can be used.

A dose and frequency of administration of the medicament of the present invention are not particularly limited, and a suitable dose can be chosen depending on the type and severity of a microbial infection, the presence or absence of an underlying disease, the age and body weight of a patient and the like.

The first embodiment of the present invention provided from another aspect is a method for judging effectiveness of a drug efflux pump inhibitor against a microorganism, which comprises the steps of:
(A1) spreading a microorganism to be tested on a surface of an agar medium, then providing an antibacterial agent as a spot on the surface of the agar medium and culturing the microorganism;
(A2) determining a growth degree of the microorganism in a region of the agar medium into which the antibacterial agent has diffused during culture period;
(A3) determining a growth degree of the microorganism in a region of the agar medium in which the antibacterial agent that has diffused during the culture period and a drug efflux pump inhibitor contained in the agar medium coexist; and
(A4) judging that the drug efflux pump inhibitor is effective against the microorganism when the growth degree of the microorganism measured in the step (A2) is significantly higher than the growth degree of the microorganism measured in the step (A3).

Type of the agar medium used for the method of the present invention is not particularly limited, and any agar media commonly used for usual antibiotic susceptibility tests may be used. Examples of the agar medium include, for example, an agar medium containing nutrients including a carbon source and a nitrogen source, and specific examples thereof include Mueller-Hinton agar medium (Difco) and the like.

As the microbial strain, strains isolated from biosamples collected from patients with infectious diseases and cultured in an ordinary manner, as well as type strains may be used. As the microorganism, *Pseudomonas aeruginosa* is preferred. Method and conditions for spreading the bacterial strain on a surface of the agar medium is not particularly limited, and those used for antibiotic susceptibility tests and the like, per se, may be used. For example, a bacterial strain may be spread on a surface by using the standard method of the Japanese Society of Chemotherapy or the agar plate dilution method defined in NCCLS (National Committee for Clinical Laboratory Standards), preferably, Mueller-Hinton agar medium or the like.

Although means for providing an antibacterial agent as a spot on the agar medium is not particularly limited, a disk usually used for antibiotic susceptibility tests and the like (circular filter paper impregnated with a medicament) can generally be used. Disks containing various antibacterial agents such as β -lactam antibiotics and quinolone antibacterial agents are commercially available, and medicament-containing disks as commercial products, per se, can be used for the method of the present invention. Further, antibacterial agent containing strips marketed as Etest (registered trademark, AB BIODISK, Sweden) are also preferred. The term "providing as a spot" used in the specification means that an antibacterial agent is provided in an area sufficiently smaller than the surface area of the agar medium. The antibacterial agent is preferably provided as a single spot on the agar medium, or may also be provided as spots at two or more different positions. Further, two or more kinds of antibacterial agents may be provided as spots on the same agar medium. When antibiotic-containing disks are not commercially available, disks may be easily prepared by impregnating an antibiotic solution prepared at a given concentration into filter paper having a suitable size and shape.

As the drug efflux pump inhibitor, the compounds disclosed in International Patent Publication WO 01/30757 can be used. The entire disclosure of International Patent Publication WO 01/30757 is incorporated herein by reference. Besides the aforementioned compounds, the compounds mentioned in the examples of the specification can also be preferably used. The "drug efflux pump inhibitor" referred to in the present specification means a medicament having an action of inhibiting the function of a drug efflux pump of microorganism, and any medicaments may be used for the method of the present invention so long as they enhance effectiveness of an antibacterial agent on the basis of the aforementioned action.

When *Pseudomonas aeruginosa* is used as an object of the measurement, it is desirable to select as the drug efflux pump inhibitor an inhibitor for the MexAB-OprM pump, which is constitutively expressed by *Pseudomonas aeruginosa.* As the medicament having an inhibitory activity against excretion activity of the MexAB-OprM pump, the following compound (hereinafter referred to as "Compound A" in the specification) or a derivative thereof is preferably used from viewpoints of potent inhibitory activity and diffusibility on the agar medium surface. This Compound A acts as a specific inhibitor against the MexAB-OprM pump.

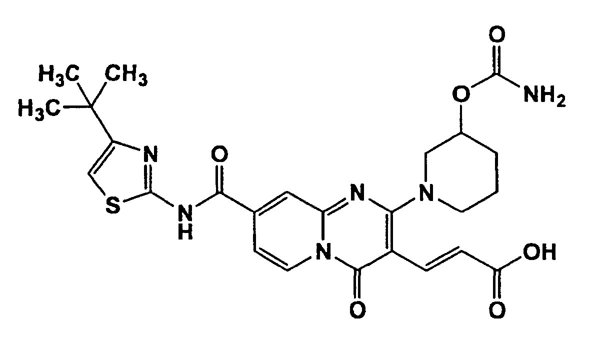

In the method of the present invention, it is necessary to form a region in which the antibacterial agent that has diffused from a disk or the like into the agar medium and the drug efflux pump inhibitor contained in the agar medium coexist. This coexistence state may generally be attained by (i) providing the drug efflux pump inhibitor on the agar medium surface as a spot by means of a disk or the like so that the drug efflux pump inhibitor can diffuse therefrom into the agar medium to form a region in which it exist together with the antibacterial agent that has diffused into the agar medium (region where the diffusions overlap), or (ii) preparing an agar medium added beforehand with the drug efflux pump inhibitor and allowing the antibacterial agent to diffuse into the agar medium from a disk or the like to form a region where the both agents coexist. In the latter embodiment, the disk can be prepared in the same manner as those described above.

The growth degree of the microorganism in the aforementioned coexistence region can be usually determined by visual inspection of the surface of the agar medium, and an area where no microorganism grows is generally observed as a clear portion. As criteria for the judgment, those adopted in antibiotic susceptibility tests according to the standard methods of the Japanese Society of Chemotherapy and the like can be used without any modification. When the growth degree of the microorganism measured in the step (A2) is significantly higher than the growth degree of the microorganism measured in the step (A3), the area of the inhibition zone observed in the step (A3) generally becomes larger than the area of the inhibition zone observed in the step (A2) (inhibition zone may sometimes not exist). Therefore, those skilled in the art can easily observe the aforementioned state to conduct the judgment of the step (A4).

Type of the antibacterial agent used in the aforementioned method is not particularly limited, and any antibacterial agent may be used. Examples include, for example, *β*-lactam antibiotics (penicillins, cephems, monobactams, carbapenems, penems), aminoglycoside antibiotics, quinolone antibacterial agents, macrolide antibiotics, tetracycline antibiotics, rifamycin antibiotics, chloramphenicol, phosphomycin and the like. However, the antibacterial agents are not limited to these examples. In the aforementioned method, effectiveness of combinational use of two or more kinds of antibacterial agents and drug efflux pump inhibitor can be judged by providing two or more kinds of antibacterial agents each as a spot with a space between the spots, determining a growth degree of the microorganism in a region of the agar medium in which the two or more kinds of antibacterial agents that have diffused during the culture period coexist, similarly determining a growth degree of the microorganism in a region of the agar medium in which the two or more kinds of antibacterial agents and the drug efflux pump inhibitor coexist, and comparing the results obtained.

The second embodiment of the present invention is a method for identifying a drug efflux pump expressed in a microorganism, which comprises the steps of:
(B1) spreading a microorganism to be tested on a surface of an agar medium, then providing an antibacterial agent that can be excreted by a particular drug efflux pump as a spot on the surface of the agar medium and culturing the microorganism;
(B2) determining a growth degree of the microorganism in a region of the agar medium into which the antibacterial agent has diffused during culture period;
(B3) determining a growth degree of the microorganism in a region of the agar medium in which the antibacterial agent that has diffused during the culture period and a drug efflux pump inhibitor contained in the agar medium coexist (provided that the drug efflux pump inhibitor is a specific inhibiter for the particular drug efflux pump); and
(B4) judging that the microorganism expresses the drug efflux pump of the particular type when the growth degree of the microorganism measured in the step (B2) is significantly higher than the growth degree of the microorganism measured in the step (B3).

The aforementioned method can be generally used for identification where the microorganism expresses one kind of drug efflux pump. For example, it is known that Aztreonam, which is a *β*-lactam antibiotic, is excreted by the MexAB-OprM pump constitutively expressed in *Pseudomonas aeruginosa.* When aztreonam is used as an antibacterial agent in the aforementioned method for identification where the microorganism to be tested expresses the MexAB-OprM pump, the growth degree of the microorganism measured in the step (B2) will significantly exceed the growth degree of the microorganism measured in the step (B3), and hence it can be judged that the microorganism expresses the MexAB-OprM pump. Method for preparation of agar medium, method for providing an antibacterial agent as a spot, method for achieving coexistence with the drug efflux pump inhibitor and the like used in the aforementioned method are the same as those explained above.

In the aforementioned method, *Pseudomonas aeruginosa* is preferred as the microorganism to be tested. As the antibacterial agent, for example, a β-lactam antibiotic or the like can be used. The β-lactam antibiotic can be suitably selected from those commercially available as therapeutic agents, and β-lactam antibiotics such as penicillin antibiotics, cephem antibiotics, monobactam antibiotics and carbapenem antibiotics can be used. More specifically, penicillin antibiotics, cephem antibiotics, monobactam antibiotics and carbapenem antibiotics such as piperacillin, piperacillin/tazobactam, carbenicillin, ceftazidime, cefepime, aztreonam and meropenem and the like can be used. When *Pseudomonas aeruginosa* is used as a microorganism to be tested, it is preferable to use a *β*-lactam antibiotic as an antibacterial agent which is more specifically excreted by the MexAB-OprM pump compared with other drug efflux pumps. Aztreonam is a particularly preferred antibacterial agent.

The third embodiment of the present invention is a method for verifying expression of two or more kinds of drug efflux pumps in a microorganism, which comprises the steps of:
(C1) spreading a microorganism to be tested on a surface of an agar medium, then providing two or more kinds of antibacterial agents (provided that each of the two or more kinds of the antibacterial agents has different effluxing properties by the two or more kinds of drug efflux pumps, and one of the two or more kinds of the antibacterial agents (hereinafter referred to as "Antibacterial agent (1)") has a property of being excreted by only one of the two or more kinds of the drug efflux pumps (hereinafter referred to as "Drug efflux pump (1)"), whilst the other antibacterial agent or agents have a property of being excreted by Drug efflux pump (1) and the other drug efflux pump or pumps);
(C2) determining a growth degree of the microorganism in a region of the agar medium into which each antibacterial agent has solely diffused during culture period;
(C3) determining a growth degree of the microorganism in a region of the agar medium in which each antibacterial agent that has solely diffused during the culture period and a drug efflux pump inhibitor contained in the agar medium coexist (provided that the drug efflux pump inhibitor is a specific inhibiter for Drug efflux pump (1)); and
(C4) judging that the microorganism expresses Drug efflux pump (1) and one or more kinds of other drug efflux pumps when the growth degree of the microorganism determined in the step (C2) is significantly higher than the growth degree of the microorganism determined in the step (C3) for Antibacterial agent (1) and the growth degree of the microorganism determined in the step (C2) is significantly lower than the growth degree of the microorganism determined in the step (C3) for the other antibacterial agent or agents.

As the combination of antibacterial agents used in the aforementioned method, a combination of two kinds of antibacterial agents is preferred, and for example, combination of a *β*-lactam antibiotic and a quinolone antibacterial agent is preferred. β-Lactam antibiotics as those exemplified above can be used as the β-lactam antibiotic. Among them, Aztreonam can be particularly preferably used for the aforementioned method, which is an antibacterial agent specifically excreted only by a MexAB-OprM pump. Further, the quinolone antibacterial agent can be suitably selected from those commercially available as therapeutic agents. For example, Levofloxacin, Ofloxacin, Ciprofloxacin, Norfloxacin and the like can be used. It is known that those quinolone antibiotics are similarly excreted by the MexAB-OprM pump as well as by other Mex type drug efflux pumps (MexCD-OprJ, MexEF-OprN, MexXY and the like), and they can be most preferably used for the aforementioned method. As the drug efflux pump inhibitor, the aforementioned Compound A is most preferably used, which is a specific inhibitor of a MexAB-OprM pump. Method for preparation of agar medium, method for providing an antibacterial agent as a spot, method for achieving coexistence with the drug efflux pump inhibitor and the like used in the aforementioned method are the same as those explained above.

As an embodiment of the aforementioned method, a method will be specifically explained wherein three kinds of agents including a drug efflux pump inhibitor, *β* -lactam antibiotic and quinolone antibacterial agent are provided each as a spot on a surface of an agar medium spread with a bacterial strain that is an object of the judgment by using disks, for example, as a means for provision as spots. However, the method of the present invention is not limited to the specific embodiment.

Amounts of the *β*-lactam antibiotic, quinolone antibacterial agent, and drug efflux pump inhibitor provided each as a spot are not particularly limited, and the amounts can be suitably selected considering diffusibility of each agent on a surface of agar medium and culture time (diffusion time) as well as strength of the inhibitory effect against the drug efflux pump. For example, when Aztreonam is used as the *β* -lactam antibiotic, its amount provided as a spot is suitably in the range of 10-30 µg, and when Levofloxacin is used as the quinolone antibiotic, its amount provided as a spot is suitably in the range of 1-10 µg. When the aforementioned Compound A is used as the drug efflux pump inhibitor, its amount provided as a spot is suitably in the range of 50-100 µg. However, the amounts to be provided as spots may optionally be increased or decreased depending on the type of microbial strain, which is an object of the measurement, shapes and sizes of inhibition zones formed around the *β*-lactam antibiotic and quinolone antibacterial agent and the like.

Positions of the *β*-lactam antibiotic and quinolone antibacterial agent provided as spots are not particularly limited. Preferred positions may give overlap of an area where either of the *β*-lactam antibiotic or the quinolone antibacterial agent diffuses in the agar medium during the culture period with an area where the drug efflux pump inhibitor diffuses in the agar medium, and also give no overlap of an area where the *β*-lactam antibiotic diffuses during the culture period with an area of the agar medium where the quinolone antibiotic diffuses during the culture period. It is generally preferred that the two antibacterial agents are bilaterally provided between which the drug efflux pump inhibitor is provided.

Diffusion areas of the *β*-lactam antibiotic, quinolone antibacterial agent and drug efflux pump inhibitor generally change depending on factors including type of each antibacterial agent, amount of agent provided as a spot, type of medium, culture conditions (mainly culture time) and the like. Accordingly, the diffusion areas can suitably be controlled by appropriately controlling these factors. For example, the distance between the *β*-lactam antibiotic and the drug efflux pump inhibitor and the distance between the quinolone antibacterial agent and the drug efflux pump inhibitor are preferably each in the range of about 1-3 cm. Inhibition zone may be formed by each of the *β*-lactam antibiotic and quinolone antibacterial agent alone as a control. For that purpose, they can be independently provided each as a spot at such positions that the agents diffused from the disks of the β -lactam antibiotic and quinolone antibacterial agent give no overlap.

Conditions of the culture on the agar medium on which surface the *β*-lactam antibiotic, quinolone antibacterial agent and drug efflux pump inhibitor are placed are not particularly limited, and they can be suitably selected depending on the conditions including type of the microorganism, kind of the agar medium, diffusion area of each antibacterial agents and the like. For example, the culture can be performed at a temperature of 35-37°C for 12-36 hours. During the culture, the *β*-lactam antibiotic, quinolone antibacterial agent and drug efflux pump inhibitor placed on the surface of agar medium diffuse in the surface and inside the agar medium.

As an example of the method of the present invention, standards for judging properties of a microbial strain based on information obtained after culture are explained below as for a test where *Pseudomonas aeruginosa* constitutively expressing an MexAB-OprM pump is used as an object of the measurement, Aztreonam (specifically excreted only by the MexAB-OprM pump) as a *β*-lactam antibiotic and Levofloxacin (excreted by a MexAB-OprM pump and other Mex type drug efflux pumps) as a quinolone drug are used as antibacterial agents, Compound A that is a specific inhibitor to a MexAB-OprM pump is used as the drug efflux pump inhibitor, and disks containing each agent are provided on an agar medium. However, the method of the present invention is not limited to the aforementioned specific embodiment.

### (1) Where bacterial strain is Pseudomonas aeruginosa that expresses MexAB-OprM pump

This bacterial strain has resistance to both of Aztreonam as a *β*-lactam antibiotic and Levofloxacin as a quinolone antibiotic on the basis of excretion of the agents by a MexAB-OprM pump. Therefore, at a position where the diffusion area of Aztreonam or Levofloxacin and that of Compound A as a specific inhibitor for a MexAB-OprM pump give no overlap (only either of Aztreonam or Levofloxacin exists at this position), any inhibition zone is not observed or only a small concentric inhibition zone proximate to the disk is observed. This is because almost no inhibition or absolutely no inhibition of the growth of the cells is achieved solely by Aztreonam or Levofloxacin due to excretion of the antibacterial agents by the action of the MexAB-OprM pump.

Whilst, around Aztreonam or Levofloxacin at a position where the diffusion areas of Aztreonam or Levofloxacin and the diffusion area of Compound A give overlap, a large inhibition zone is observed. This is because the drug excretion action of the MexAB-OprM pump is inhibited by Compound A as a MexAB-OprM pump inhibitor, and as a result, Aztreonam or Levofloxacin penetrates into the cells to enable inhibition of cell growth by the antibacterial activity of each antibacterial agent.

Shape of the inhibition zone observed may change depending on the degree of overlap of the diffusion area of Aztreonam or Levofloxacin and that of Compound A. Further, size of the inhibition zone may also change depending on the expression amount of the MexAB-OprM pump. When a bacterial strain expresses a larger amount of the MexAB-OprM pump, the formation of the inhibition zone is enhanced. However, in each any case, the resulting inhibition zone can be clearly distinguished, based on the difference in diameter of the inhibition zones, from the inhibition zone formed around Aztreonam or Levofloxacin (inhibition zone may sometimes not be formed) at a position where diffusion area thereof gives no overlap with that of Compound A.

### (2) Where bacterial strain is Pseudomonas aeruginosa coexpressing another drug efflux pump in addition to MexAB-OprM pump

In addition to the MexAB-OprM pump constitutively expressed in *Pseudomonas aeruginosa,* MexCD-OprJ pump, MexEF-OprN pump, MexXY pump and the like are know, for example, which are Mex drug efflux pumps. Further, bacterial strains expressing two or more kinds of pumps among the Mex pumps are also known. For strains with said pumps, an antibiotic susceptibility test in which only a β-lactam antibiotic or a quinolone antibacterial agent is used and no MexAB-OprM pump inhibitor is used in combination fails to clarify expression amount of the MexAB-OprM pump or existence of Mex pump expressed in addition to the MexAB-OprM pump. Whilst the method of the present invention will provide distinctive results.

When the objective *Pseudomonas aeruginosa* expresses another Mex pump in addition to the MexAB-OprM pump, such a strain has resistance to both ofAztreonam and Levofloxacin by the synergistic excretion action of the MexAB-OprM pump and the other Mex pump. Therefore, at a position where the diffusion area of Aztreonam or Levofloxacin and that of Compound A give no overlap (only Aztreonam or Levofloxacin exists at this position), no inhibition zone is observed or only a small concentric inhibition zone proximate to the disk is formed.

Further, at a position where the diffusion area of Levofloxacin and that of Compound A give overlap, no formation of inhibition zone is observed or only a small inhibition zone is observed around Levofloxacin. This is because Levofloxacin cannot sufficiently penetrates into the cells due to excretion by the Mex pump other than the MexAB-OprM pump, and as a result, the agent becomes impossible to inhibit the growth of the bacterium.

Whilst, at a position where the diffusion area of Aztreonam and that of Compound A give overlap, a large inhibition zone is formed around the β-lactam antibiotic. The reason can be explained as follows. Compound A as a MexAB-OprM pump inhibitor specifically inhibits only the MexAB-OprM pump, and the agent cannot inhibit another Mex pump if it is expressed. However, Aztreonam as a β-lactam antibiotic that is excreted only by the MexAB-OprM pump is not excreted by the Mex pump other than the MexAB-OprM pump. Therefore, when the MexAB-OprM pump is inhibited by Compound A, Aztreonam penetrates into the cells and inhibits the growth of the cells on the basis of its antibacterial activity.

The present invention was generally explained above, and the method of the present invention will be more specifically explained in the examples of the present specification. Therefore, those skilled in the art can practice the method of the present invention by adding suitable modifications or alterations to the method as required based on the above general descriptions and specific explanations in the examples. It should be understood that those methods added with such modifications or alterations also fall within the scope of the present invention.

### Examples

The present invention will be explained more specifically with reference to the examples. However, the scope of the present invention is not limited to the following examples.

### Example 1: (E)-3-[8-[2-(4-Cyclobutyl-1,3-thiazol-2-yl)ethyl]-2-(3-hydroxypiperidino)-4-oxo-4H-pyrido[1,2-a]pyrimidin-3-yl]-2-propenoic acid

### (A) Methyl 3-{2-[(tert-butoxycarbonyl)amino]-4-pyridyl}propanoate

tert-Butyl N-(4-methyl-2-pyridyl)carbamate (5 g, 24.0 mmol) was dissolved in tetrahydrofuran (120 ml), cooled to -78°C, and then added dropwise with n-butyl lithium (40 ml, 60 mmol). Then, the reaction solution was warmed and stirred at room temperature. The reaction mixture was stirred for 1 hour, then again cooled to -78°C, and added dropwise with methyl bromoacetate (3.4 ml) dissolved in tetrahydrofuran (10 ml). After the reaction mixture was stirred for 30 minutes, the reaction was stopped with saturated brine and the reaction mixture was extracted with ethyl acetate. The resulting organic layer was dried over sodium sulfate and concentrated under reduced pressure, and the residue was purified by silica gel column chromatography (chloroform:ethyl acetate = 3:1 → 5:1) to obtain 3.95 g (59%) of the title compound as pale yellow crystals.
¹H-NMR (CDCl₃) δ : 1.53 (9H, s), 2.67 (2H, t, J=7.81Hz), 2.95 (2H, t, J=7.81Hz), 3.68 (3H, s), 6.82 (1H, dd, J=1.22, 5.13Hz), 7.84 (1H, s), 8.13 (1H, d, J=5.13Hz)

### (B) 3-{2-[(tert-Butoxycarbonyl)amino]-4-pyridyl}propanoic acid

Methyl 3-{2-[(tert-butoxycarbonyl)amino]-4-pyridyl}propanoate (30.65 g, 0.11 mol) dissolved in methanol (200 ml) was added with 1 N aqueous sodium hydroxide (164 ml) and stirred at room temperature for 21 hours. After the solvent was evaporated under reduced pressure and the residue was washed with diethyl ether, the resulting aqueous layer was added with concentrated hydrochloric acid until pH of the layer became 1. The solution was washed with ethyl acetate, and the resulting aqueous layer was neutralized by further adding sodium hydroxide. The solution was extracted with chloroform: methanol = 10:1, and the resulting organic layer was dried over sodium sulfate and concentrated under reduced pressure to obtain 11.16 g (38%) of the title compound as yellow crystals without purification.
¹H-NMR (CD₃OD) δ : 1.54 (9H, s), 2.67 (2H, t, J=7.59Hz), 2.95 (2H, t, J=7.59Hz), 6.90 (1H, d, J=5.14Hz), 7.80 (1H, s), 8.08 (1H, d, J=5.14Hz)
EI/MS; m/z: 267 (M⁺+1)

### (C) tert-Butyl N-[4-(3-amino-3-oxopropyl)-2-pyridyl]carbamate

3-{2-[(tert-Butoxycarbonyl)amino]-4-pyridyl}propanoic acid (11.16 g, 41.92 mmol) dissolved in tetrahydrofuran (200 ml) was added with triethylamine (9 ml, 62.89 mmol), cooled with ice, and then added dropwise with ethyl chloroformate (6 ml, 62.89 mmol). The reaction mixture was stirred for 10 minutes, and then added with aqueous ammonia (50 ml) dissolved in tetrahydrofuran (50 ml) at 0°C. The reaction mixture was stirred for 20 minutes under ice cooling, and then the solvent was evaporated under reduced pressure. The resulting residue was washed with water and extracted with chloroform. The resulting organic layer was washed with saturated brine, and the organic layer was dried over sodium sulfate and concentrated under reduced pressure to obtain 11.794 g (100%) of the title compound as brown crystals without purification.
¹H-NMR (CD₃OD) δ : 1.53 (9H, s), 2.57 (2H, t, J=8.05Hz), 2.97 (2H, t, J=8.05Hz), 5.40 (2H, br), 6.84 (1H, dd, J=1.46, 5.13Hz), 7.83 (1H, s), 8.13 (1H, d, J=5.13Hz)
EI/MS; m/z: 266 (M⁺+1)

### (D) tert-Butyl N-[4-(3-amino-3-thioxopropyl)-2-pyridyl]carbamate

Under argon atmosphere, tert-butyl N-[4-(3-amino-3-oxopropyl)-2-pyridyl]-carbamate (11.79 g, 44.44 mmol) dissolved in tetrahydrofuran (100 ml) was added with Lawesson's reagent (9 g, 22.22 mmol) and stirred at 70-80°C for 30 minutes. After the reaction mixture was returned to room temperature, the solvent was evaporated under reduced pressure and the resulting residue was purified by silica gel column chromatography (chloroform → chloroform:methanol = 20:1) to obtain 9.544 g (76%) of the title compound as pale yellow crystals.
¹H-NMR (CD₃OD) δ : 1.53 (9H, s), 2.89 (2H, t, J=8.30Hz), 3.09 (2H, t, J=8.30Hz), 6.94 (1H, d, J=5.13Hz), 7.75 (1H, s), 8.08 (1H, d, J=5.13Hz)
EI/MS; m/z: 282 (M⁺+1)

### (E) 2-Bromo-1-cyclobutyl-1-ethanone

1-Cyclobutyl-1-ethanone (500 mg, 5.1 mmol) dissolved in methanol (5 ml) was added with bromine (0.3 ml, 5.6 mmol) and stirred at room temperature for 1 hour. The resulting ocher reaction mixture was added with water (3 ml) under ice cooling, and then gradually added with potassium carbonate (350 mg). The mixture was extracted with dichloromethane, and the resulting organic layer was neutralized with saturated aqueous sodium hydrogencarbonate and washed with saturated brine. The resulting organic layer was dried over magnesium sulfate and concentrated under reduced pressure to obtain 867 mg (96%) of the title compound without purification. ¹H-NMR (CDCl₃) δ :1.88 (1H,m), 2.00 (1H,m), 2.20-2.37 (4H,m), 3.60 (1H, qu, J=8.53Hz), 3.88 (2H, s)

### (F) 8-[2-(4-Cyclobutyl-1,3-thiazol-2-yl)ethyl]-2-hydroxy-4H-pyrido[1,2-a]pyrimidin-4-one

tert-Butyl N-[4-(3-amino-3-thioxopropyl)-2-pyridyl]carbamate (1.38 g, 4.9 mmol) dissolved in ethanol was added with 2-bromo-1-cyclobutyl-1-ethanone (867 mg, 4.9 mmol) and refluxed at 100°C for 1 hour. The resulting reaction solution was cooled to room temperature, then neutralized with saturated aqueous sodium hydrogencarbonate and extracted with chloroform. The resulting organic layer was washed with saturated brine, dried over magnesium sulfate and concentrated under reduced pressure.

The resulting oily compound was added with dichloromethane (50 ml) and slowly added dropwise with trifluoroacetic acid (50 ml) under ice cooling. Then, the reaction solution was warmed to room temperature and stirred 1 hour. The resulting solution was neutralized with saturated sodium hydrogencarbonate and extracted with chloroform. The organic layer was washed with saturated brine, and the resulting organic layer was dried over magnesium sulfate and concentrated under reduced pressure.

The brown oily residue (1.37 g) was added with xylene (7 ml) and trichlorophenyl malonate (2.7 g, 5.83 mmol) and refluxed by heating at 140°C for 1 hour, and the solvent was evaporated under reduced pressure. The resulting oily compound was purified by silica gel chromatography (ethyl acetate → chloroform:methanol = 30:1 → 10:1 → 5:1) to obtain 657 mg (41% for the three steps) of the title compound as pale yellow crystals.
¹H-NMR (CDCl₃) δ : 1.91 (1H, m), 2.01 (1H, qu, J=8.79Hz), 2.22 (2H, d qu, J=2.44, 8.79Hz), 2.34 (2H, tq, J=2.44, 8.79Hz), 3.39 (4H, dd, J=6.84, 20.75Hz), 3.63 (1H, qu, J=8.79Hz), 5.33 (1H, s), 6.76 (1H, s), 7.11 (1H, dd, J=1.71, 7.08Hz), 7.40 (1H, s), 9.02 (1H, d, J=7.08Hz)

### (G) 8-[2-(4-Cyclobutyl-1,3-thiazol-2-yl)ethyl]-2-(3-hydroxypiperidino)-4H-pyrido[1,2-a]pyrimidin-4-one

8-[2-(4-Cyclobutyl-1,3-thiazol-2:yl)ethyl]-2-hydroxy-4H-pyrido[1,2-a]pyrimidin -4-one (120 mg, 0.366 mmol) dissolved in tetrahydrofuran (4 ml) and dimethylformamide (1 ml) was added with 4-dimethylaminopyridine (60 mg, 0.475 mmol) and p-tolunenesulfonyl chloride (77 mg, 0.402 mmol) at room temperature and stirred for 30 minutes. The solvent was evaporated under reduced pressure, and the residue was washed with water and extracted with chloroform. The resulting organic layer was dried over magnesium sulfate and concentrated under reduced pressure.

The resulting oily compound was added with dimethylformamide (3 ml) and 3-hydroxypiperidine (45 mg, 0.44 mmol), and stirred at 60°C for 40 hours. The solvent was evaporated under reduced pressure, and then the residue was purified by silica gel column chromatography (chloroform → chloroform:methanol = 20:1) to obtain 103 mg (68% for the two steps) of the title compound as oil.
¹H-NMR (CDCl₃) δ : 1.56 (1H, m), 1.66 (1H, m), 1.62-2.09 (4H, m), 2.23 (2H, d qu, J=2.20, 9.03Hz), 2.35 (2H, m), 3.15 (2H, t, J=7.32Hz), 3.35 (2H, t, J=7.32Hz), 3.37 (1H, m), 3.48 (1H, m), 3.63 (1H, qu, J=9.03Hz), 3.68 (1H, m), 3.84 (1H, m), 3.99 (1H, dd, J=2.93, 13.18Hz), 5.64 (1H, s), 6.71 (1H, dd, J=1.71, 7.32Hz), 6.76 (1H, s), 7.10 (1H, s), 8.76 (1H, d, J=7.32Hz)
EI/MS; m/z: 411 (M⁺+1)

### (H) 2-(3-{[1-(tert-Butyl)-1,1-dimethylsilyl]oxy}piperidino)-8-[2-(4-cyclobutyl-1,3-thiazol-2-yl)ethyl]-4H-pyrido[1,2-a]pyrimidin-4-one

8-[2-(4-Cyclobutyl-1,3-thiazol-2-yl)ethyl]-2-(3-hydroxypiperidino)-4H-pyrido[1, 2-a]pyrimidin-4-one (103 mg, 0.251 mmol) dissolved in dichloromethane (3 ml) was added with imidazole (51 mg, 0.753 mmol) and tert-butyldimethylsilyl chloride (57 mg, 0.376 mmol) under ice cooling and stirred for 1 hour. Then, as the reaction did not progress, the reaction mixture was warmed to room temperature and further added with imidazole and tert-butyldimethylsilyl chloride so that the reaction was completed. The reaction solution was diluted with chloroform, washed with water, dried over magnesium sulfate and then concentrated under reduced pressure. The resulting residue was purified by silica gel column chromatography (chloroform → chloroform:methanol = 20:1) to obtain 128 mg (97%) of the title compound as an orange oily substance.
¹H-NMR (CDCl₃) δ : 0.87 (9H, s), 0.92 (6H, s), 1.53 (2H, m), 1.80-2.09 (4H, m), 2.23 (2H, m), 2.34 (2H, m), 3.02 (2H, m), 3.14 (2H, t, J=8.04Hz), 3.35 (2H, t, J=8.04Hz), 3.64 (2H, m), 4.00 (1H, brd), 4.20 (1H, brd), 5.59 (1H, s), 6.69 (1H, dd, J=1.95, 7.31Hz), 6.75 (1H, s), 7.06 (1H, s), 8.76 (1H, d, J=7.31Hz)
EI/MS; m/z: 525 (M⁺+1)

### (I) 1-{8-[2-(4-Cyclobutyl-1,3-thiazol-2-yl)ethyl]-3-formyl-4-oxo-4H-pyrido[1,2-a]-pyrimidin-2-yl}-3-piperidyl formate

Under ice cooling, dimethylformamide (2 ml) was added with phosphorus oxychloride (34 µl, 0.366 mmol), and then the reaction solution was added dropwise with 2-(3-{[1-(tert-butyl)-1,1-dimethylsilyl]oxy}piperidino)-8-[2-(4-cyclobuty11,3-thiazol-2-yl)ethyl]-4H-pyrido[1,2-a]pyrimidin-4-one (128 mg, 0.244 mmol) dissolved in dimethylformamide under ice cooling. Then the reaction solution was warmed to room temperature. After 2 hours, the reaction solution was further added with phosphorus oxychloride (34 µl) at room temperature and stirred at room temperature for 20 minutes. The solvent was evaporated under reduced pressure, and the resulting residue was neutralized by adding saturated aqueous sodium hydrogencarbonate and extracted with ethyl acetate and chloroform. The organic layer collected was washed with saturated brine, dried over magnesium sulfate and concentrated under reduced pressure. The residue was purified by silica gel column chromatography (chloroform → chloroform:methanol = 20:1) to obtain 69 mg (61%) of the title compound as a yellow oily substance.
¹H-NMR (CDCl₃) δ: 1.25-2.09 (6H, m), 2.22 (2H, dqu, J=2.45, 9.06Hz), 2.34 (2H, m), 3.18 (2H, t, J=7.83Hz), 3.36 (2H, t, J=7.83Hz), 3.65 (3H, m), 3.82 (1H, dd, J=6.37, 13.47Hz), 3.91 (1H, dd, J=3.18, 13.47Hz), 5.07 (1H, m), 6.77 (1H, dd, J=1.71, 7.34Hz), 6.77 (1H, s), 7.07 (1H, s), 7.98 (1H, s), 8.72 (1H, d, J=7.34Hz), 10.08 (1H, s)
EI/MS; m/z: 467 (M⁺+1)

### (J) tert-Butyl (E)-3-[8-[2-(4-cyclobutyl-1,3-thiazol-2-yl)ethyl]-2-(3-formyloxy-piperidino)-4-oxo-4H-pyrido[1,2-a]pyrimidin-3-yl]-2-propenoate

1-{8-[2-(4-Cyclobutyl-1,3-thiazol-2-yl)ethyl]-3-formyl-4-oxo-4H-pyrido[1,2-a]pyrimidin-2-yl}-3-piperidylformate (69 mg, 0.148 mmol) dissolved in tetrahydrofuran (3 ml) was added with (tert-butoxycarbonylmethylene)triphenylphosphorane (84 mg, 0.222 mmol) and refluxed at 100°C. After 7 hours, the reaction solution was further added with the phosphorane (90 mg) and further refluxed for 11 hours. Then the phosphorane (90 mg) was further added, and after 5 hours, the phosphorane (89 mg) was further added. The reaction solution was refluxed for 10 hours, then added with the phosphorane (90 mg) and refluxed for 5 hours. The reaction solution was returned to room temperature, and the solvent was evaporated under reduced pressure. The residue was purified by thin layer silica gel chromatography (chloroform:methanol = 30:1) to obtain 94 mg of the title compound as yellow crystals in a mixture with triphenylphosphine oxide.
¹H-NMR (CDCl₃) δ : 1.51 (9H, s), 1.89-2.04 (6H, m), 2.22 (2H, m), 2.34 (2H, m), 3.19 (2H, t, J=7.81Hz), 3.36 (2H, t, J=7.81Hz), 3.53-3.68 (4H, m), 3.79 (1H, dd, J=3.42, 13.67Hz), 5.13 (1H, m), 6.76 (1H, s), 6.84 (1H, dd, J=1.95, 7.32Hz), 7.06 (1H, d, J=15.63Hz), 7.20 (1H, s), 7.65 (1H, d, J=15.63Hz), 8.08 (1H, s), 8.85 (1H, d, J=7.32Hz)

### (K) tert-Butyl (E)-3-[8-[2-(4-cyclobutyl-1,3-thiazol-2-yl)ethyl]-2-(3-hydroxypiperidino)-4-oxo-4H-pyrido[1,2-a]pyrimidin-3-yl]-2-propenoate

tert-Butyl (E)-3-[8-[2-(4-cyclobutyl-1,3-thiazol-2-yl)ethyl]-2-(3-formyloxy-piperidino)-4-oxo-4H-pyrido[1,2-a]pyrimidin-3-yl]-2-propenoate (95 mg, 0.168 mmol) dissolved in methanol (4 ml) was added with sodium methoxide (4 mg, 0.074 mmol) under ice cooling and stirred for 45 minutes under ice cooling. Then the reaction solution was further added with sodium methoxide (4 mg), and after 5 minutes, further added with sodium methoxide (10 mg). After the reaction solution was stirred for 15 minutes, the reaction was stopped with saturated brine and added with chloroform for extraction. The organic layer was dried over magnesium sulfate and concentrated under reduced pressure, and the residue was purified by thin layer silica gel chromatography (chloroform:methanol = 20:1) to obtain 76 mg (84%) of the title compound as yellow crystals in a mixture with triphenylphosphine oxide.
¹H-NMR (CDCl₃) δ : 1.51 (9H, s), 1.82-2.08 (6H, m), 2.17-2.25 (2H, m), 2.32-2.36 (2H, m), 3.19 (2H, t, J=7.32Hz), 3.36 (2H, t, J=7.32Hz), 3.53-3.65 (4H, m), 3.92 (1H, d, J=13.92Hz), 4.01 (1H, s), 6.77 (1H, s), 6.86 (1H, dd, J=1.95, 7.32Hz), 7.03 (1H, d, J=15.63Hz), 7.19 (1H, s), 7.62 (1H, d, J=15.63Hz), 8.85 (1H, d, J=7.32Hz)
EI/MS; m/z: 537 (M⁺+1)

### (L) (E)-3-[8-[2-(4-Cyclobutyl-1,3-thiazol-2-yl)ethyl]-2-(3-hydroxypiperidino)-4-oxo-4H-pyrido[1,2-a]pyrimidin-3-yl]-2-propenoic acid

tert-Butyl (E)-3-[8-[2-(4-Cyclobutyl-1,3-thiazol-2-yl)ethyl]-2-(3-hydroxy-piperidino)-4-oxo-4H-pyrido[1,2-a]pyrimidin-3-yl]-2-propenoate (76 mg, 0.142 mmol) was added with formic acid (3 ml) and stirred at room temperature for 2 hours and 30 minutes. After the formic acid was evaporated under reduced pressure, the residue was purified by thin layer silica gel chromatography (chloroform:methanol = 15:1) to obtain 39 mg (55% for the three steps) of the title compound as yellow crystals.
¹H-NMR (CDCl₃) δ : 1.25 (1H, m), 1.52 (1H, m), 1.83-2.08 (4H, m), 2.21 (2H, d qu, J=2.44, 8.30Hz), 2.36 (2H, tq, J=2.93, 8.30Hz), 3.19 (2H, t, J=7.32Hz), 3.36 (2H, t, J=7.32Hz), 3.63 (4H, m), 3.88 (1H, d, J=13.43Hz), 4.02 (1H, brd), 6.76 (1H, s), 6.86 (1H, dd, J=2.71, 7.32Hz), 7.06 (1H, d, J=15.63Hz), 7.19 (1H, s), 7.65 (1H, d, J=15.63Hz), 8.84 (1H, d, J=7.32Hz)
EI/MS; m/z: 481 (M⁺+1)

### Example 2: (E)-3-{8-[2-(4-Cyclobutyl-1,3-thiazol-2-yl)etbyl]-2-morpholino-4-oxo-4H-pyrido[1,2-a]pyrimidin-3-yl}-2-propenoic acid

### (A) 8-[2-(4-Cyclobutyl-1,3-thiazol-2-yl)ethyl]-2-morpholino-4H-pyrido[1,2-a]pyrimidin-4-one

8-[2-(4-Cyclobutyl-1,3-thiazol-2-yl)ethyl]-2-hydroxy-4H-pyrido[1,2-a]-pyrimidin-4-one (302 mg, 0.925 mmol) dissolved in tetrahydrofuran (14 ml) and dimethylformamide (6 ml) was added with 4-dimethylaminopyridine (150 mg, 1.20 mmol) and p-tolunenesulfonyl chloride (194 mg, 1.02 mmol) and stirred at room temperature for 20 minutes.

After the solvent was evaporated under reduced pressure, the residue was diluted with chloroform and washed with water. The resulting organic layer was dried over magnesium sulfate and concentrated under reduced pressure.

The residue was dissolved in dimethylformamide (2 ml), added with morpholine (1 ml, 11.5 mmol) and stirred at 70°C for 3 hours. After the reaction solution was returned to room temperature, the solvent was evaporated under reduced pressure and the residue was purified by silica gel column chromatography (chloroform → chloroform:methanol = 20:1) to obtain 351 mg of the title compound as pale yellow crystals as a substance containing dimethylformamide.
¹H-NMR (CDCl₃) δ: 1.92 (1H, m), 2.02 (1H, m), 2.23 (2H, m), 2.35 (2H, m), 3.16 (2H, t, J=8.32Hz), 3.35 (2H, t, J=8.32Hz), 3.66 (4H, t, J=5.38Hz), 3.70 (1H, m), 3.78 (4H, t, J=5.38Hz), 5.57 (1H, s), 6.74 (1H, dd, J=2.94, 7.09Hz), 6.76 (1H, s), 7.13 (1H, s), 8.78 (1H, d, J=7.09Hz)
EI/MS; m/z: 397 (M⁺+1)

### (B) 8-[2-(4-Cyclobutyl-1,3-thiazol-2-yl)ethyl]-2-morpholino-4-oxo-4H-pyrido[1,2-a]-pyrimidine-3-carbaldehyde

Reactions were performed in the same manner as in Example 1, (I) by using 8-[2-(4-cyclobutyl-1,3-thiazol-2-yl)ethyl]-2-morpholino-4H-pyrido[1,2-a]pyrimidin-4-one (346 mg, 0.8726 mmol) to obtain 305 mg (82%) of the title compound as ocher solid. ¹H-NMR (CDCl₃) δ: 1.92-2.96 (6H, m), 3.19 (2H, t, J=7.34Hz), 3.36 (2H, t, J=7.34Hz), 3.68 (1H, m)3.73 (4H, t, J=5.14Hz), 3.82 (4H, t, J=5.14Hz), 6.77 (1H, s), 6.78 (1H, dd, J=1.96, 7.34Hz), 7.08 (1H, s), 8.74 (1H, d, J=7.34Hz), 10.01 (1H, s) EI/MS; m/z: 425 (M⁺+1)

### (C) tert-Butyl (E)-3-{8-[2-(4-cyclobutyl-1,3-thiazol-2-yl)ethyl]-2-morpholino-4-oxo-4H-pyrido[1,2-a]pyrimidin-3-yl}-2-propenoate

Reactions were performed in the same manner as in Example 1, (J) by using 8-[2-(4-cyclobutyl-1,3-thiazol-2-yl)ethyl]-2-morpholino-4-oxo-4H-pyrido[1,2-a]-pyrimidin-3-carbaldehyde (100 mg, 0.2356 mmol) to obtain 127 mg (100%) of the title compound as a yellow oily substance in a mixture with triphenylphosphine oxide.
¹H-NMR (CDCl₃) δ: 2.02 (1H, m), 2.20 (1H, m), 2.23 (2H, m), 2.34 (2H, m), 3.20 (2H, t, J=7.08Hz), 3.37 (2H, t, J=7.08Hz), 3.60 (4H, t, J=4.39Hz), 3.63 (1H, m), 3.83 (4H, t, J=4.39Hz), 6.76 (1H, s), 6.85 (1H, dd, J=1.71, 7.32Hz), 7.05 (1H, d, J=15.63Hz), 7.21 (1H, s), 7.64 (1H, d, J=15.63Hz), 8.86 (1H, d, J=7.32Hz)
EI/MS; m/z: 523 (M⁺+1)

### (D) (E)-3-{8-[2-(4-Cyclobutyl-1,3-thiazol-2-yl)ethyl]-2-morpholino-4-oxo-4H-pyrido-[1,2-a]pyrimidin-3-yl}-2-propenoic acid

Reactions were performed in the same manner as in Example 1, (L) by using tert-butyl (E)-3-{8-[2-(4-cyclobutyl-1,3-thiazol-2-yl)ethyl]-2-morpholino-4-oxo-4H-pyrido[1,2-a]pyrimidin-3-yl}-2-propenoate (127 mg, 0.2430 mmol) to obtain 82.7 mg (73%) of the title compound as yellow crystals.
¹H-NMR (CDCl₃) δ : 1.92 (1H, m), 2.01 (1H, m), 2.22 (2H, m), 2.34 (2H, m), 3.19 (2H, t, J=8.06Hz), 3.37 (2H, t, J=8.06Hz), 3.61 (4H, s), 3.64 (1H, m), 3.81 (4H, s), 6.76 (1H, s), 6.86 (1H, dd, J=1.47, 7.32Hz), 7.08 (1H, d, J=15.63Hz), 7.19 (1H, s), 7.64 (1H, d, J=15.63Hz), 8.87 (1H, d, J=7.32Hz)
EI/MS; m/z: 467 (M⁺+1).
IR (cm⁻¹): 2962, 2850, 1680, 1647, 1516, 1444

### Example 3: (E)-3-{8-[2-(4-Cyclobutyl-1,3-thiazol-2-yl)ethyl]-2-morpholino-4-oxo-4H-pyrido[1,2-a]pyrimidin-3-yl}-2-methyl-2-propenoic acid

### (A) Ethyl (E)-3-{8-[2-(4-cyclobutyl-1,3-thiazol-2-yl)ethyl]-2-morpholino-4-oxo-4H-pyrido[1,2-a]pyrimidin-3-yl}-2-methyl-2-propenoate

8-[2-(4-Cyclobutyl-1,3-thiazol-2-yl)ethyl]-2-morpholino-4-oxo-4H-pyrido[1,2-a]pyrimidin-3-carbaldehyde (100 mg, 0.236 mmol) dissolved in toluene (2 ml) was added with (carbethoxyethylidene)triphenylphosphorane (102 mg, 0.283 mmol) and refluxed by heating at 130°C. Then the regent was added until the reaction was completed, and 7 equivalents of the regent was finally added. After the reaction mixture was stirred for 4 days, the solvent was evaporated under reduced pressure, and the resulting residue was purified by thin layer silica gel chromatography (chloroform:methanol = 40:1) to obtain 443 mg of yellow crystals in a mixture with triphenylphosphine oxide.
¹H-NMR (CDCl₃) δ : 1.33 (3H, t, J=7.08Hz), 1.88 (3H, s), 1.90 (1H, m), 2.05 (1H, m), 2.23 (2H, m), 2.35 (2H, m), 3.20 (2H, t, J=7.81Hz), 3.37 (2H, t, J=7.81Hz), 3.57 (4H, t, J=4.88Hz), 3.64 (1H, m), 3.73 (4H, t, J=4.88Hz), 4.25 (2H, q, J=7.08Hz), 6.77 (1H, s), 6.82 (1H, d, J=7.33Hz), 7.21 (1H, s), 7.57 (1H, s), 8.84 (1H, d, J=7.33Hz)

### (B) (E)-3-{8-[2-(4-Cyclobutyl-1,3-thiazol-2-yl)ethyl]-2-morpholino-4-oxo-4H-pyrido-[1,2-a]pyrimidin-3-yl}-2-methyl-2-propenoic acid

Ethyl (E)-3-{8-[2-(4-cyclobutyl-1,3-thiazol-2-yl)ethyl]-2-morpholino-4-oxo-4H-pyrido[1,2-a]pyrimidin-3-yl}-2-methyl-2-propenoate (120 mg, 0.236 mmol) dissolved in methanol (5 ml) was added dropwise with 1 N aqueous sodium hydroxide (2 ml) under ice cooling. The reaction solution was stirred for 10 minutes and then warmed to room temperature because the reaction did not progress. The reaction solution was stirred for 45 minutes, then further added with 1 N aqueous sodium hydroxide (2 ml), and after 1 hour, further added with 1 N aqueous sodium hydroxide (2 ml). The solvent was evaporated under reduced pressure, and the resulting sodium salt of the title compound was dissolved in 1,4-dioxane, added with 4 N hydrochloric acid (6 ml) and stirred. Then the reaction solution was concentrated under reduced pressure, and the residue was neutralized with saturated aqueous sodium hydrogencarbonate and extracted with chloroform and methanol. The organic layer was dried over magnesium sulfate and concentrated under reduced pressure. The resulting residue was purified by thin layer silica gel chromatography (chloroform:methanol = 30:1) to obtain 75 mg (66%) of the title compound as yellow crystals.
¹H-NMR (CDCl₃) δ: 1.84 (3H, s), 1.91 (1H, m), 2.02 (1H, hex, J=9.06Hz), 2.22 (2H, d qu, J=2.21, 9.06Hz), 2.34 (2H, q, J=8.57Hz), 3.16 (2H, t, J=8.08Hz), 3.36 (2H, t, J=8.08Hz), 3.56 (4H, s), 3.64 (1H, qu, J=8.57Hz), 3.70 (4H, s), 6.76 (1H, s), 6.80 (1H, dd, J=1.22, 7.10Hz), 7.16 (1H, s), 7.57 (1H, s), 8.83 (1H, d, J=7.10Hz)
EI/MS; m/z: 481 (M⁺+1)
IR (cm⁻¹): 2958, 2919, 2850, 1666, 1641, 1440, 1251, 1115

### Example 4: (E)-3-{8-[2-(4-Ethyl-1,3-thiazol-2-yl)ethyl]-2-morpholino-4-oxo-4H-pyrido-[1,2-a]pyrimidin-3-yl}-2-propenoic acid

### (A) 4-[2-(4-Ethyl-1,3-thiazol-2-yl)ethyl]-2-pyridinamine

tert-Butyl N-[4-(3-amino-3-thioxopropyl)-2-pyridyl]carbamate (590 mg, 2.10 mmol) dissolved in ethanol (20 ml) was added with 1-bromo-2-butanone (0.23 ml, 2.10 mmol) and refluxed by heating at 100°C. The reaction solution was stirred for 1 hour and then returned to room temperature. The solvent was evaporated under reduced pressure, and the residue was diluted with chloroform. The mixture was washed with saturated aqueous sodium hydrogencarbonate and dried over sodium sulfate, and concentrated under reduced pressure.

The resulting residue was added with dichloromethane (20 ml) and added dropwise with trifluoroacetic acid (20 ml) under ice cooling. Then the reaction solution was warmed to room temperature and stirred for 15 hours. The trifluoroacetic acid was evaporated under reduced pressure, and the residue was neutralized with saturated sodium hydrogencarbonate and extracted with chloroform. The organic layer was washed with saturated brine and the collected organic layer was dried over magnesium sulfate and concentrated under reduced pressure. The resulting residue without purification gave 477 mg (98%) of the title compound as an orange oily substance.
¹H-NMR (CDCl₃) δ : 1.29 (3H, t, J=7.59Hz), 2.78 (2H, dq, J=0.98, 7.59Hz), 2.98 (2H, t, J=8.33Hz), 3.25 (2H, t, J=8.33Hz), 6.37 (1H, s), 6.52 (1H, dd, J=1.23, 5.39Hz), 6.72 (1H, s), 7.93 (1H, d, J=5.39Hz)

### (B) 8-[2-(4-Ethyl-1,3-thiazol-2-yl)ethyl]-2-hydroxy-4H-pyrido[1,2-a]pyrimidin-4-one

4-[2-(4-Ethyl-1,3-thiazol-2-yl)ethyl]-2-pyridinamine (480 mg, 2.044 mmol) dissolved in xylene (10 ml) was added with trichlorophenyl malonate (1 g, 2.160 mmol) and refluxed by heating at 140°C. The reaction solution was stirred for 3 hours and returned to room temperature. The solvent was evaporated under reduced pressure and the resulting residue was purified by silica gel column chromatography (chloroform → chloroform:methanol = 80:1 → 50:1 → 5:1) to obtain 135 mg (22%) of orange crystals.
¹H-NMR (CDCl₃) δ : 1.28 (3H, t, J=7.57Hz), 2.78 (2H, q, J=7.57Hz), 3.36 (4H, dd, J=6.35, 17.82Hz), 5.33 (1H, s), 6.76 (1H, s), 7.09 (1H, d, J=7.08Hz), 7.39 (1H, s), 9.02 (1H, d, J=7.08Hz)
EI/MS; m/z: 302 (M⁺+1)

### (C) 8-[2-(4-Ethyl-1,3-thiazol-2-yl)ethyl]-2-morpholino-4H-pyrido[1,2-a]pyrimidin-4-one

Reactions were performed in the same manner as in Example 2, (A) by using 8-[2-(4-ethyl-1,3-thiazol-2-yl)ethyl]-2-hydroxy-4H-pyrido[1,2-a]pyrimidin-4-one (135 mg, 0.4480 mmol) as a starting material to obtain 120 mg (72%) of the title compound as pale yellow crystals.
¹H-NMR(CDCl₃) δ : 1.29 (3H, t, J=7.57Hz), 2.78 (2H, dq, J=0.977, 7.57Hz), 3.16 (2H, t, J=8.06Hz), 3.34 (2H, t, J=8.06Hz), 3.64 (4H, t, J=5.13Hz), 3.77 (4H, t, J=5.13Hz), 5.57 (1H, s), 6.74 (1H, d, J=0.977Hz), 6.74 (1H, dd, J=1.953, 7.08Hz), 7.12 (1H, d, J=1.953Hz), 8.79 (1H, d, J=7.08Hz)
EI/MS; m/z: 371 (M⁺+1)

### (D) 8-[2-(4-Ethyl-1,3-thiazol-2-yl)ethyl]-2-morpholino-4-oxo-4H-pyrido[1,2-a]-pyrimidine-3-carbaldehyde

Reactions were performed in the same manner as in Example 1, (I) by using 8-[2-(4-ethyl-1,3-thiazol-2-yl)ethyl]-2-morpholino-4H-pyrido[1,2-a]pyrimidin-4-one (120 mg, 0.324 mmol) to obtain 116 mg (90%) of the title compound.
¹H-NMR (CDCl₃) δ : 1.29 (3H, t, J=7.57Hz), 2.78 (2H, q, J=7.57Hz), 3.19 (2H, t, J=7.08Hz), 3.35 (2H, t, J=7.08Hz), 3.73 (4H, t, J=4.15Hz), 3.82 (4H, t, J=4.15Hz), 6.76 (1H, s), 6.78 (1H, dd, J=1.71, 7.32Hz), 7.07 (1H, d, J=1.71Hz), 8.74 (1H, d, J=7.32Hz), 10.11 (1H, s)
EI/MS; m/z: 399 (M⁺+1)

### (E) tert-Butyl (E)-3-{8-[2-(4-ethyl-1,3-thiazol-2-yl)ethyl]-2-morpholino-4-oxo-4H-pyrido[1,2-a]pyrimidin-3-yl}-2-propenoate

Reactions were performed in the same manner as in Example 1, (J) by using 8-[2-(4-ethyl-1,3-thiazol-2-yl)ethyl]-2-marpholino-4-oxo-4H-pyrido[1,2-a]pyrimidine-3-carbaldehyde (116 mg, 0.2911 mmol)to obtain the title compound as yellow crystals in a mixture with triphenylphosphine oxide.
¹H-NMR (CDCl₃) δ : 1.28 (3H, t, J=7.57Hz), 1.51 (9H, s), 2.78 (2H, q, J=7.57Hz), 3.20 (2H, t, J=7.08Hz), 3.36 (2H, t, J=7.08Hz), 3.60 (4H, t, J=4.88Hz), 3.83 (4H, t, J=4.88Hz), 6.74 (1H, s), 6.85 (1H, dd, J=1.71, 7.32Hz), 7.05 (1H, d, J=15.87Hz), 7.20 (1H, s), 7.68 (1H, d, J=15.87Hz), 8.87 (1H, d, J=7.32Hz)

### (F) (E)-3-{8-[2-(4-Ethyl-1,3-thiazol-2-yl)ethyl]-2-morpholino-4-oxo-4H-pyrido[1,2-a]-pyrimidin-3-yl}-2-propenoic acid

Reactions were performed in the same manner as in Example 1, (L) by using tert-butyl (E)-3-{8-[2-(4-ethyl-1,3-thiazol-2-yl)ethyl]-2-morpholino-4-oxo-4H-pyrido-[1,2-a]pyrimidin-3-yl}-2-propenoate (144 mg, 0.290 mmol) to obtain 93 mg (73%) of the title compound as yellow crystals.
¹H-NMR (CDCl₃) δ: 1.29 (3H, t, J=7.57Hz), 2.79 (2H, q, J=7.57Hz), 3.21 (2H, t, J=7.08Hz), 3.38 (2H, t, J=7.08Hz), 3.63 (4H, t, J=6.15Hz), 3.83 (4H, t, J=6.15Hz), 6.75 (1H, s), 6.87 (1H, dd, J=1.47, 7.32Hz), 7.09 (1H, d, J=15.63Hz), 7.21 (1H, s), 7.68 (1H, d, J=15.63Hz), 8.87 (1H, d, J=7.32Hz)
EI/MS; m/z: 441 (M⁺+1)
IR (cm⁻¹): 2964, 2919, 2850, 1681,1517, 1444

### Example 5: (E)-3-{2-(3-Hydroxypiperidino)-8-[(4-isopropyl-1,3-thiazol-2-yl)methoxy]-4-oxo-4H-pyrido[1,2-a]pyrimidin-3-yl}-2-propenoic acid

### (A) 4-Methoxy-2-pyridinecarbonitrile

4-Methoxypyridine-N-oxide hydrate (1 g, 6.99 mmol) dissolved in dichloromethane (4 ml) was added with trimethylsilyl cyanide (1 ml, 7.69 mmol) and subsequently added dropwise with N,N-dimethylcarbamoyl chloride (0.8 ml, 9.09 mmol) under ice cooling. The reaction solution was warmed to room temperature, stirred for 1 hour, and then added further with trimethylsilyl cyanide (0.2 ml, 1.40 mmol). The reaction solution was stirred for 19 hours, then added with 10% potassium carbonate (2 ml). The mixture was diluted with ethyl acetate, washed with saturated brine and dried over magnesium sulfate, and then concentrated under reduced pressure. The resulting crystals were washed with hexane to obtain 560 mg (60%) of the title compound as pink crystals without purification.
¹H-NMR (CDCl₃) δ : 3.92 (3H, s), 7.02 (1H, dd, J=2.44, 5.86Hz), 7.22 (1H, d, J=2.44Hz), 8.51 (1H, d, J=5.86Hz)

### (B) 4-Hydroxy-2-pyridinecarbonitrile

4-Methoxy-2-pyridinecarbonitrile (544 mg, 4.055 mmol) was added with 47% hydrobromic acid (6 ml) and refluxed by heating at 130°C. The reaction solution was stirred for 22 hours, returned to room temperature, and then concentrated under reduced pressure. The residue was subjected to azeotropy with toluene and diethyl ether to remove excessive hydrobromic acid. The resulting white crystals were washed with diethyl ether to obtain 1.47 g of the title compound containing hydrogen bromide without purification.
¹H-NMR (CD₃OD) δ : 7.48 (1H, dd, J=2.44, 6.83Hz), 7.81 (1H, d, J=2.44Hz), 8.63 (1H, d, J=6.83Hz)

### (C) Ethyl 4-hydroxy-2-pyridinecarboxylate

4-Hydroxy-2-pyridinecarbonitrile (25.48 g, 0.2121 mol) dissolved in ethanol (400 ml) was added with concentrated hydrochloric acid (40 ml) and refluxed by heating at 110°C for 6 days. The reaction solution was returned to room temperature, evaporated under reduced pressure and added with chloroform:methanol = 20:1. After insoluble crystals were removed by filtration, the resulting filtrate was concentrated under reduced pressure. The resulting residue was purified by silica gel column chromatography (chloroform:methanol = 20:1 → 10:1) to obtain 22.4 g (63% for the two steps) of the title compound as yellow crystals.
¹H-NMR (CD₃OD) δ : 1.32 (3H, t, J=7.32Hz), 4.52 (2H, q, J=7.32Hz), 7.18 (1H, dd, J=2.69, 6.84Hz), 7.58 (1H, d, J=2.69Hz), 8.36 (1H, d, J=6.84Hz)
EI/MS; m/z: 168 (M⁺+1)

### (D) 2-(Chloromethyl)-4-isopropyl-1,3-thiazole

(4-Isopropyl-1,3-thiazol-2-yl)methanol (10 g, 63.6011 mmol) dissolved in dichloromethane (60 ml) was added with thionyl chloride (7 ml, 95.40 mmol) under ice cooling, and then the reaction solution was returned to room temperature and stirred for 20 minutes. The reaction solution was concentrated under reduced pressure, and the residue was subjected to azeotropy with toluene and then diluted with diethyl ether, and further neutralized with saturated aqueous sodium hydrogencarbonate. The organic layer was washed with saturated brine, then dried over magnesium sulfate and concentrated under reduced pressure. The title compound was obtained as the residue without purification (11 g, 100%).
¹H-NMR (CDCl₃) δ : 1.30 (6H, d, J=7.08Hz), 3.09 (1H, qu, J=7.08Hz), 4.83 (2H, s), 6.91 (1H, s)
EI/MS; m/z: 176 (M⁺+1)

### (E) Ethyl 4-[(4-isopropyl-1,3-thiazol-2-yl)methoxy]-2-pyridinecarboxylate

Ethyl 4-hydroxy-2-pyridinecarboxylate (19.5 g, 0.057 mol) dissolved in dimethylformamide (200 ml) was added with a solution of 2-(chloromethyl)-4-isopropyl-1,3-thiazole (11 g, 0.063 mol) in dimethylformamide (150 ml). The mixture was added with potassium iodide (9.5 g) and potassium carbonate (12 g) at room temperature, then heated to 110°C and stirred for 1 hour. The reaction solution was returned to room temperature, and the solvent was evaporated under reduced pressure. The residue was diluted with chloroform and washed with water. The resulting organic layer was washed with saturated brine, and the organic layer was dried over magnesium sulfate and concentrated under reduced pressure. The residue was purified by silica gel chromatography (chloroform) to obtain 8.049 g (46%) of the title compound.
¹H-NMR (CDCl₃) δ : 1.32 (6H, d, J=6.86Hz), 1.44 (3H, t, J=7.10Hz), 3.12 (1H, qu, J=6.86Hz), 4.47 (2H, q, J=7.10Hz), 5.44 (2H, s), 6.94 (1H, s), 7.09 (1H, dd, J=2.694, 5.63Hz), 7.80 (1H, d, J=2.694Hz), 8.58 (1H, d, J=5.63Hz)
EI/MS; m/z: 307 (M⁺+1)

### (F) 4-[(4-Isopropyl-1,3-thiazol-2-yl)methoxy]-2-pyridinecarboxylic acid

Ethyl 4-[(4-isopropyl-1,3-thiazol-2-yl)methoxy]-2-pyridinecarboxylate (7.44 g, 24.28 mmol) dissolved in ethanol (50 ml) was added dropwise with 1 N sodium hydroxide (27 ml, 26.70 mmol) at room temperature, and stirred at room temperature for 1 hour and 30 minutes. The solvent was evaporated under reduced pressure, and the resulting Na salt crystals were washed with ethyl acetate.

The crystals were added with 4 N hydrochloric acid (27 ml) and 1,4-dioxane (40 ml), stirred and concentrated under reduced pressure. The residue was subjected to azeotropy with toluene, and the solvent was completely evaporated. The resulting crystals were filtered with chloroform:methanol = 10:1, and the filtrate was concentrated under reduced pressure. The resulting pale yellow crystals were purified by silica gel column chromatography (chloroform:methanol = 20:1 → 10:1 → 5:1) to obtain 6.7 g (100%) of the title compound as white crystals.
¹H-NMR (CD₃OD) δ : 1.37 (6H, d, J=6.83Hz), 3.22 (1H, qu, J=6.83Hz), 5.96 (2H, s), 7.54 (1H, s), 7.92 (1H, dd, J=2.68, 6.83Hz), 8.22 (1H, d, J=2.68Hz), 8.80 (1H, d, J=6.83Hz)
EI/MS; m/z: 279 (M⁺+1).

### (G) tert-Butyl N-{4-[(4-isopropyl-1,3-thiazol-2-yl)methoxy]-2-pyridyl}carbamate

4-[(4-Isopropyl-1,3-thiazol-2-yl)methoxy]-2-pyridinecarboxylic acid (50 mg, 0.18 mmol) was added with toluene (12 ml), triethylamine (63 µl, 0.45 mmol) and diphenyl phosphorylazide (78 µl, 0.36 mmol) and refluxed by heating at 140°C for 7 hours. The reaction solution was returned to room temperature, added with tert-butanol (12 ml) and refluxed again by heating at 140°C. The reaction solution was stirred for 18 hours and then returned to room temperature, and the solvent was evaporated under reduced pressure. The residue was purified by thin layer silica gel chromatography (chloroform:methanol = 40:1) to obtain 33 mg (53%) of the title compound.
¹H-NMR (CDCl₃) δ : 1.32 (6H, d, J=6.86Hz), 1.53 (9H, s), 3.11 (1H, qu, J=6.86Hz), 5.40 (2H, s), 6.60 (1H, dd, J=2.45, 5.88Hz), 6.91 (1H, s), 7.73 (1H, s), 8.15 (1H, dd, J=2.45, 5.88Hz)
EI/MS; m/z: 350 (M⁺+1)

### (H) 4-[(4-Isopropyl-1,3-thiazol-2-yl)methoxy]-2-pyridinamine

tert-Butyl N-{4-[(4-isopropyl-1,3-thiazol-2-yl)methoxy]-2-pyridyl}carbamate (706 mg, 2.202 mmol) dissolved in dichloromethane (20 ml) was added dropwise with trifluoroacetic acid (20 ml) under ice cooling, then warmed to room temperature and stirred for 2 hours. The reaction solution was concentrated under reduced pressure, diluted with chloroform, then neutralized with saturated aqueous sodium hydrogencarbonate and extracted with chloroform. The organic layer was dried over magnesium sulfate and concentrated under reduced pressure. The residue was purified by silica gel column chromatography (chloroform → chloroform:methanol = 50:1 → 30:1 → 20:1) to obtain 331 mg (66%) of the title compound.
¹H-NMR (CDCl₃) δ : 1.32 (6H, d, J=6.83Hz), 3.11 (1H, qu, J=6.83Hz), 5.31 (2H, s), 6.08 (1H, d, J=1.95Hz), 6.35 (1H, dd, J=1.95, 5.85Hz), 6.91 (1H, s), 7.91 (1H, d, J=5.85Hz)

### (I) 2-Hydroxy-8-[(4-isopropyl-1,3-thiazol-2-yl)methoxy]-4H-pyrido[1,2-a]pyrimidin-4-one

4-[(4-Isopropyl-l,3-thiazol-2-yl)methoxy]-2-pyridinamine (378 mg, 1.52 mmol) dissolved in xylene (15 ml) was added with trichlorophenyl malonate (750 mg, 1.62 mmol) and refluxed by heating at 140°C for 1 hour and 30 minutes. The reaction solution was returned to room temperature and concentrated under reduced pressure, and the residue was purified by silica gel column chromatography (chloroform → chloroform:methanol = 100:1 → 80:1 → 50:1) to obtain 307 mg (64%) of the title compound.
¹H-NMR (CDCl₃) δ: 1.33 (6H, d, J=6.84Hz), 3.15 (1H, qu, J=6.84Hz), 5.23 (1H, s), 5.60 (2H, s), 6.95 (1H, dd, J=2.44, 7.57Hz), 7.01 (1H, s), 7.06 (1H, d, J=2.44Hz), 8.99 (1H, d, J=7.57Hz)
EI/MS; m/z: 318 (M⁺+1)

### (J) 2-Hydroxy-8-[(4-isopropyl-1,3-thiazol-2-yl)methoxy]-4-oxo-4H-pyrido[1,2-a]-pyrimidine-3-carbaldehyde

Dimethylformamide (3 ml) was added with phosphorus oxychloride (130 µl, 1.42 mmol) under ice cooling, and further added dropwise with 2-hydroxy-8-[(4-isopropyl-1,3-thiazol-2-yl)methoxy]-4H-pyrido[1,2-a]pyrimidin-4-one (300 mg, 0.945 mmol) dissolved in dimethylformamide (6 ml) under ice cooling. Then, the reaction solution was returned to room temperature and stirred for 1 hour. The reaction was stopped with saturated aqueous sodium hydrogencarbonate, and the reaction solution was extracted with chloroform. The resulting organic layer was washed with saturated brine, dried over sodium sulfate and concentrated under reduced pressure. The residue was purified by silica gel column chromatography (chloroform → chloroform:methanol = 50:1 → 30:1 → 10:1) to obtain 45 mg (14%) of the title compound.
¹H-NMR (CD-Cl₃) δ : 1.32 (6H, d, J=6.84Hz), 3.13 (1H, qu, J=6.84Hz), 5.50 (2H, s), 6.94 (1H, d, J=6.85Hz), 6.99 (2H, s), 8.92 (1H, d, J=6.85Hz), 10.13 (1H, s)
EI/MS; m/z: 346 (M⁺+1)

### (K) tert-Butyl (E)-3-{2-hydroxy-8-[(4-isopropyl-1,3-thiazol-2-yl)methoxy]-4-oxo-4H-pyrido[1,2-a]pyrimidin-3-yl}-2-propenoate

2-Hydroxy-8-[(4-isopropyl-1,3-thiazol-2-yl)methoxy]-4-oxo-4H-pyrido[1,2-a]pyr imidin-3-carbaldehyde (45 mg, 0.13 mmol) dissolved in tetrahydrofuran (2 ml) and dimethylformamide (1 ml) was added with (tert-butoxycarbonylmethylene)triphenylphosphorane (60 mg, 0.16 mmol) and refluxed by heating at 100°C for 2 hours. The reaction solution was returned to room temperature and concentrated under reduced pressure, and the residue was purified by thin layer silica gel chromatography (chloroform:methanol = 20:1) to obtain 36 mg (62%) of the title compound.
¹H-NMR (CDCl₃) δ : 1.26 (6H, d, J=6.86Hz), 1.44 (9H, s), 3.08 (1H, qu, J=6.86Hz), 5.57 (2H, s), 6.82 (1H, d, J=15.92Hz), 6.90 (1H, d, J=7.83Hz), 6.96 (1H, s), 7.01 (1H, s), 7.69 (1H, d, J=15.92Hz), 8.95 (1H, d, J=7.83Hz)
EI/MS; m/z: 444 (M⁺+1)

### (L) tert-Butyl (E)-3-{2-(3-hydroxypiperidino)-8-[(4-isopropyl-1,3-thiazol-2-yl)methoxy]-4-oxo-4H-pyrido[1,2-a]pyrimidin-3-yl}-2-propenoate

Reactions were performed in the same manner as in Example 1, (G) by using tert-butyl (E)-3-{2-hydroxy-8-[(4-isopropyl-1,3-thiazol-2-yl)methoxy]-4-oxo-4H-pyrido[1,2-a]pyrimidin-3-yl}-2-propenoate (26 mg, 0.059 mmol) to obtain 24 mg (78%) of the title compound as yellow oily compound.
¹H-NMR (CDCl₃) δ : 1.33 (6H, d, J=7.08Hz), 1.51 (9H, s), 1.60 (2H, m), 1.83 (2H, m), 3.14 (1H, qu, J=7.08Hz), 3.56 (3H, m), 3.92 (1H, dd, J=3.91,13.67Hz), 4.02 (1H, m), 5.44 (2H, s), 6.75 (1H, dd, J=2.68, 7.32Hz), 6.76 (1H, s), 6.97 (1H, s), 6.98 (1H, d, J=15.62Hz), 7.48 (1H, d, J=15.62Hz), 8.86 (1H, d, J=7.32Hz)
EI/MS; m/z: 527 (M⁺+1)

### (M) (E)-3-{2-(3-Hydroxypiperidino)-8-[(4-isopropyl-1,3-thiazol-2-yl)methoxy]-4-oxo-4H-pyrido[1,2-a]pyrimidin-3-yl}-2-propenoic acid

tert-Butyl (E)-3-{2-(3-hydroxypiperidino)-8-[(4-isopropyl-1,3-thiazol-2-yl)methoxy]-4-oxo-4H-pyrido[1,2-a]pyrimidin-3-yl}-2-propenoate (24 mg, 0.046 mmol) was added with a mixture of 4 N hydrochloric acid and dioxane (1 ml) and stirred at room temperature for 3 hours. The reaction solution was concentrated under reduced pressure, and the residue was purified by thin layer silica gel chromatography (chloroform:methanol = 15:1) to obtain 24 mg (100%) of the title compound as yellow crystals.
¹H-NMR (CD₃OD) δ : 1.33 (6H, d, J=7.08Hz), 1.57 (1H, m), 1.69 (1H, m), 1.89 (1H, m), 2.07 (1H, m), 3.14 (1H, qu, J=7.08Hz), 3.16 (1H, m), 3.17 (1H, m), 3.82 (2H, m), 4.04 (1H, d, J=9.52Hz), 5.54 (2H, s), 6.87 (1H, d, J=7.81Hz), 6.92 (1H, d, J=15.38Hz), 6.95 (1H, s), 7.18 (1H, s), 7.61 (1H, d, J=15.38Hz), 8.81 (1H, d, J=7.81Hz)
EI/MS; m/z: 471 (M⁺+1)

### Example 6: (E)-3-{8-[2-(4-Ethyl-2-thienyl)ethyl]-2-morpholino-4-oxo-4H-pyrido[1,2-a]-pyrimidin-3-yl}-2-propenoic acid

### (A) 4-Ethyl-2-thiophenecarbaldehyde

3-Ethylthiophene (2 g, 17.8 mmol) dissolved in diethyl ether (18 ml) was added with a solution of n-butyl lithium in hexane (1.5 M, 14 ml, 21.4 mmol) at room temperature and refluxed by heating at 60°C for 15 minutes. The reaction solution was returned to room temperature and added dropwise with dimethylformamide (2 ml, 23.2 mmol) dissolved in diethyl ether. After the reaction solution was stirred for 2 hours at room temperature, the reaction was stopped with saturated aqueous ammonium chloride, and the reaction solution was extracted with chloroform. The collected organic layer was washed with saturated brine, then dried over magnesium sulfate and concentrated under reduced pressure.

The residue was purified by silica gel column chromatography (hexane:ethyl acetate = 20:1 → 10:1) to obtain 2 g (80%) of the title compound.
¹H-NMR (CDCl₃) δ : 1.27 (3H, t, J=7.59Hz), 2.68 (2H, q, J=7.59Hz), 7.39 (1H, s), 7.63 (1H, s), 9.87 (1H, s)

### (B) (4-Ethyl-2-thienyl)methanol

4-Ethyl-2-thiophenecarbaldehyde (1 g, 7.13 mmol) dissolved in methanol (7 ml) was added with sodium borohydride (135 mg, 3.57 mmol) under ice cooling, stirred for 10 minutes and then further added with sodium borohydride (150 mg, 3.96 mmol) at 0°C. After the reaction solution was stirred under ice cooling for 30 minutes, the reaction was stopped with saturated aqueous ammonium chloride and the reaction solution was extracted with chloroform. The organic layer collected was dried over magnesium sulfate and concentrated under reduced pressure to obtain 1 g (100%) of the title compound without purification.
¹H-NMR (CDCl₃) δ: 1.22 (3H, t, J=7.56Hz), 2.59 (2H, q, J=7.56Hz), 4.75 (2H, s), 6.86 (2H, s)

### (C) tert-Butyl N-{4-[2-(3-bromo-4-ethyl-2-thienyl)ethyl]-2-pyridyl}carbamate

(4-Ethyl-2-thienyl)methanol (1 g, 7.03 mmol) dissolved in dichloromethane (7 ml) was added with thionyl bromide (0.8 ml, 10.55 mmol) under ice cooling and then warmed to room temperature. The reaction solution was stirred for 20 minutes and then concentrated under reduced pressure, and the residue was neutralized by adding saturated aqueous sodium hydrogencarbonate and extracted with diethyl ether. The organic layer was washed with saturated brine, dried over magnesium sulfate and concentrated under reduced pressure. The residue was used in the subsequent reaction without purification.

tert-Butyl N-(4-methyl-2-pyridyl)carbamate (800 mg, 3.84 mmol) dissolved in tetrahydrofuran (15 ml) was cooled to -78°C and then added dropwise with a solution of n-butyl lithium in hexane (1.5 M, 6.4 ml, 9.6 mmol). The reaction solution was stirred at room temperature for 1 hour, then cooled to -78°C again and added dropwise with a solution of the above obtained 3-bromo-2-(bromomethyl)-4-ethylthiophene in tetrahydrofuran (14 ml). After the reaction solution was stirred at -78°C for 1 hour and the reaction was stopped with saturated brine, the reaction solution was extracted with ethyl acetate. The organic layer collected was dried over magnesium sulfate and concentrated under reduced pressure. The resulting residue was purified by silica gel column chromatography (chloroform) to obtain 1.3 g (85%) of the title compound.
¹H-NMR (CDCl₃) δ: 1.15 (3H, t, J=7.57Hz), 1.55 (9H, s), 2.52 (2H, q, J=7.57Hz), 2.92 (2H, t, J=8.55Hz), 3.04 (2H, t, J=8.55Hz), 6.50 (1H, s), 6.77 (1H, d, J=5.13Hz), 7.87 (1H, s), 8.18 (1H, d, J=5.13Hz)
EI/MS; m/z: 411 (M⁺)

### (D) 4-[2-(3-Bromo-4-ethyl-2-thienyl)ethyl]-2-pyridylamine

tert-Butyl N-{4-[2-(3-bromo-4-ethyl-2-thienyl)ethyl]-2-pyridyl}carbamate (1.34 g, 4.04 mmol) dissolved in dichloromethane (40 ml) was added with trifluoroacetic acid (40 ml) at 0°C and stirred at 0°C for 3 hours. The reaction solution was concentrated under reduced pressure, neutralized with saturated sodium hydrogencarbonate and extracted with chloroform, and then the organic layer was dried over magnesium sulfate and concentrated under reduced pressure. The residue was purified by silica gel column chromatography (chloroform:ethyl acetate = 3:1 → 1:1 → ethyl acetate only) to obtain 524 mg (56%) of the title compound.
¹H-NMR (CD₃OD) δ : 1.12 (3H, t, J=7.56Hz), 2.48 (2H, q, J=7.56Hz), 2.79 (2H, t, J=7.56Hz), 3.01 (2H, t, J=7.56Hz), 6.40 (1H, s), 6.46 (1H, d, J=5.36Hz), 6.53 (1H, s), 7.76 (1H, d, J=5.36Hz)
EI/MS; m/z: 310 (M⁺-1)

### (E) 8-[2-(3-Bromo-4-ethyl-2-thienyl)ethyl]-2-hydroxy-4H-pyrido[1,2-a]pyrimidin-4-one

Reactions were performed in the same manner as in Example 4, (B) by using 4-[2-(3-bromo-4-ethyl-2-thienyl)ethyl]-2-pyridylamine as a starting material. After the reaction was completed, the reaction solution was concentrated under reduced pressure and the crystals in the resulting turbid solution was removed by filtration. The filtrate was concentrated under reduced pressure, and the resulting residue was purified by silica gel column chromatography (chloroform → chloroform:ethyl acetate = 1:1 → ethyl acetate → ethyl acetate :methanol = 50:1 → 30:1 → 10:1) to obtain 180 mg (38%) of the title compound.
¹H-NMR (CDCl₃) δ : 1.14 (3H, t, J=7.56Hz), 2.49 (2H, q, J=7.56Hz), 3.15 (4H, s), 5.31 (1H, s), 6.53 (1H, s), 7.12 (1H, d, J=7.07Hz), 7.24 (1H, s), 8.98 (1H, d, J=7.07Hz)

### (F) 8-[2-(4-Ethyl-2-thienyl)ethyl]-2-hydroxy-4H-pyrido[1,2-a]pyrimidin-4-one

8-[2-(3-Bromo-4-ethyl-2-thienyl)ethyl]-2-hydroxy-4H-pyrido[1,2-a]pyrimidin-4-one (96 mg, 0.25 mmol) dissolved in toluene (6 ml) was added with tributyltin hydride (75 µl) and 2,2-azobisisobutyronitrile (4 mg, 0.025 mmol) and refluxed by heating at 140°C. Then 2,2-azobisisobutyronitrile and tributyltin hydride were further added until the reaction was completed while the progress of the reaction was monitored by LC-MS. 34 mg (0.22 mmol) of 2,2-azobisisobutyronitrile and 350 µl (1.30 mmol) of tributyltin hydride were used. The reaction solution was returned to room temperature and the reaction was stopped with an aqueous solution of potassium fluoride. The reaction solution was extracted with ethyl acetate, and the organic layer was washed with saturated brine. Then, the aqueous layer was extracted with ethyl acetate. The organic layer collected was dried over magnesium sulfate and concentrated under reduced pressure. The residue was purified by thin layer silica gel chromatography (chloroform:methanol =20:1) to obtain 119 mg of the title compound with contained impurities.
¹H-NMR (CDCl₃) δ : 1.28 (3H, t, J=7.32Hz), 2.56 (2H, q, J=7.32Hz), 3.04 (2H, t, J=8.06Hz), 3.17 (2H, t, J=8.06Hz), 5.34 (1H, s), 5.63 (1H, s), 6.74 (1H, d, J=4.88Hz), 7.04 (1H, d, J=7.08Hz), 7.35 (1H, s), 9.02 (1H, d, J=7.08Hz)
ES-MS: 301 (M⁺+1)

### (G) 8-[2-(4-Ethyl-2-thienyl)ethyl]-2-morpholino-4H-pyrido[1,2-a]pyrimidin-4-one

Reactions were performed in the same manner as in Example 2, (A) by using 8-[2-(4-ethyl-2-thienyl)ethyl]-2-hydroxy-4H-pyrido[1,2-a]pyrimidin-4-one (122 mg, 0.406 mmol) as a starting material. As a result, 122 mg of the title compound was obtained as a yellow oily substance in a mixture with impurities.
¹H-NMR (CDCl₃) δ : 1.20 (3H, t, J=7.57Hz), 2.56 (2H, q, J=7.57Hz), 3.01 (2H, t, J=7.32Hz), 3.16 (2H, t, J=7.32Hz), 3.66 (4H, t, J=4.88Hz), 3.78 (4H, t, J=4.88Hz), 5.62 (1H, s), 6.64 (1H, s), 6.73 (2H, m), 7.11 (1H, s), 8.79 (1H, d, J=7.08Hz)
ES-MS; m/z: 370 (M⁺+1)

### (H) 8-[2-(4-Ethyl-2-thienyl)ethyl]-2-morpholino-4-oxo-4H-pyrido[1,2-a]pyrimidin-3-carbaldehyde

Reactions were performed in the same manner as in Example 1, (I) by using 8-[2-(4-ethyl-2-thienyl)ethyl]-2-morpholino-4H-pyrido[1,2-a]pyrimidin-4-one (122 mg, 0.33 mmol) as a starting material. As a result, 52 mg (40% for the three steps) of the title compound in orange color was obtained.
¹H-NMR (CDCl₃) δ : 1.20 (3H, t, J=7.56Hz), 2.56 (2H, q, J=7.56Hz), 3.02 (2H, t, J=8.04Hz), 3.17 (2H, t, J=8.04Hz), 3.73 (4H, d, J=4.88Hz), 3.82 (4H, d, J=4.88Hz), 6.64 (1H, s), 6.74 (1H, s), 6.76 (1H, dd, J=1.95, 7.07Hz), 7.03 (1H, s), 8.74 (1H, d, J=7.07Hz), 10.11 (1H, s)
EI/MS; m/z: 398 (M⁺+1)

### (I) tert-Butyl (E)-3-{8-[2-(4-ethyl-2-thienyl)ethyl]-2-morpholino-4-oxo-4H-pyrido-[1,2-a]pyrimidin-3-yl}-2-propenoate

Reactions were performed in the same manner as in Example 1, (J) by using 8-[2-(4-ethyl-2-thienyl)ethyl]-2-morpholino-4-oxo-4H-pyrido[1,2-a]pyrimidine-3-carbaldehyde (52 mg, 0.13 mmol) as a starting material. As a result, 65 mg of the title compound was obtained as an orange oily substance in a mixture with triphenylphosphine oxide.
¹H-NMR (CDCl₃) δ : 1.20 (3H, t, J=7.59Hz), 1.51 (9H, s), 2.56 (2H, q, J=7.59Hz), 3.04 (2H, t, J=8.08Hz), 3.17 (2H, t, J=8.08Hz), 3.60 (4H, t, J=4.41Hz), 3.83 (4H, t, J=4.41Hz), 6.63 (1H, s), 6.73 (1H, s), 6.82 (1H, dd, J=1.96, 7.35Hz), 7.05 (1H, d, J=15.68Hz), 7.17 (1H, s), 7.69 (1H, d, J=15.68Hz), 8.87 (1H, d, J=7.35Hz)
EI/MS; m/z: 496 (M⁺+1)

### (J) (E)-3-{8-[2-(4-Ethyl-2-thienyl)ethyl]-2-morpholino-4-oxo-4H-pyrido[1,2-a]-pyrimidin-3-yl}-2-propenoic acid

Reactions were performed in the same manner as in Example 1, (L) by using tert-butyl (E)-3-{8-[2-(4-ethyl-2-thienyl)ethyl]-2-morpholino-4-oxo-4H-pyrido[1,2-a]-pyrimidin-3-yl}-2-propenoate (65 mg, 0.13 mmol) as a starting material. As a result, 32 mg (56%) of yellow crystals were obtained.
¹H-NMR (CDCl₃) δ: 1.20 (3H, t, J=7.57Hz), 2.56 (2H, q, J=7.57Hz), 3.05 (2H, t, J=7.81Hz), 3.18 (2H, t, J=7.81Hz), 3.63 (4H, t, J=4.40Hz), 3.84 (4H, t, J=4.40Hz), 6.63 (1H, s), 6.74 (1H, s), 6.84 (1H, dd, J=1.95, 7.32Hz), 7.11 (1H, d, J=15.38Hz), 7.18 (1H, s), 7.69 (1H, d, J=15.38Hz), 8.88 (1H, d, J=7.32Hz)
EI/MS; m/z: 440 (M⁺+1)

### Example 7: (E)-3-[8-({[4-(tert-Butyl)-1,3-thiazol-2-yl]amino}carbonyl)-2-morpholino-4-oxo-4H-pyrido[1,2-a]pyrimidin-3-yl]-2-gropenoic acid

### (A) 2-(Acetylamino)isonicotinic acid

N¹-(4-Methyl-2-pyridyl)acetamide (25 g, 0.168 mol) suspended in water (250 ml) was added with potassium permanganate (76.1 g, 0.50 mol) at 100°C over 1 hour and then stirred for 40 minutes. The reaction solution was returned to room temperature, and black crystals were removed by filtration. The resulting filtrate was added with 12 N hydrochloric acid until pH of the solution became 3 to 4. After the reaction solution was stirred for about 15 minutes, the deposited white crystals were collected by filtration, washed with water, and dried by using a vacuum pump to obtain 7.65 g (25%) of the title compound without purification.
¹H-NMR (CD₃OD) δ : 2.20 (3H, s), 7.59 (1H, dd, J=1.47, 5.13Hz), 8.42 (1H, dd, J=0.73, 5.13Hz), 8.63 (1H, s)
EI/MS; m/z: 179 (M⁺)

### (B) N⁴-[4-(tert-Butyl)-1,3-thiazol-2-yl]-2-(acetylamino)isonicotinamide

2-(Acetylamino)isonicotinic acid (500 mg, 2.8 mmol) was added dropwise with thionyl chloride (15 ml, 68.5 mmol) at room temperature, warmed to 80°C and stirred for 30 minutes. The reaction solution was returned to room temperature, concentrated under reduced pressure, and the residue was subjected to azeotropy with toluene to evaporate excessive thionyl chloride. The resulting yellow crystals was added to a mixed solution of pyridine (0.25 ml), dichloromethane (5 ml) and 4-(tert-butyl)-1,3-thiazol-2-amine (525 mg, 3.35 mmol) under ice cooling. The mixture was stirred at 0°C for 30 minutes, then warmed to room temperature, and further stirred for 2 hours and 30 minutes. The reaction solution was added with water and extracted with chloroform. The organic layer was dried over magnesium sulfate and concentrated under reduced pressure. The resulting residue was purified by silica gel column chromatography (chloroform → chloroform:methanol = 80:1 → 50:1) to obtain 545.8 mg (61%) of the title compound.
¹H-NMR (CDCl₃) δ : 1.31 (9H, s), 2.26 (3H, s), 6.61 (1H, s), 7.63 (1H, dd, J=0.49, 5.14Hz), 8.44 (1H, d, J=5.14Hz), 8.51 (1H, brd), 8.72 (1H, s)
EI/MS; m/z: 319 (M⁺+1)

### (C) N⁴-[4-(tert-Butyl)-1,3-thiazol-2-yl]-2-aminoisonicotinamide

N4-[4-(tert-Butyl)-1,3-thiazol-2-yl]-2-(acetylamino)isonicotinamide (546 mg) dissolved in ethanol (12 ml) was added dropwise with concentrated hydrochloric acid (1.2 ml) at room temperature and stirred at 80-90°C for 1 hour. The reaction solution was returned to room temperature, concentrated under reduced pressure, neutralized with 1 N sodium hydroxide and extracted with chloroform. The organic layer was dried over magnesium sulfate and concentrated under reduced pressure, and the resulting residue was purified by silica gel column chromatography (chloroform → chloroform:methanol = 60:1 → 20:1) to obtain 247.2 mg (52%) of the title compound as white crystals.
¹H-NMR (CD₃OD) δ : 1.33 (9H, s), 6.63 (1H, d, J=0.73Hz), 7.05 (1H, t, J=0.73Hz), 7.09 (1H, ddd, J=0.73, 1.46, 5.36Hz), 8.13 (1H, dd, J=0.73, 5.36Hz)
EI/MS; m/z: 275 (M⁺-1)

### (D) N⁸-[4-(tert-Butyl)-1,3-thiazol-2-yl]-2,4-dioxo-3,4-dihydro-2H-pyrido[1,2-a]-pyrimidine-8-carboxamide

N⁴-[4-(tert-Butyl)-1,3-thiazol-2-yl]-2-aminoisonicotinamide (200 mg, 0.724 mmol) was added with xylene (20 ml) and trichlorophenyl malonate (370 mg, 0.796 mmol) and refluxed by heating at 130°C. The reaction solution was stirred for 1 hour, then returned to room temperature and concentrated under reduced pressure. The resulting orange crystals was taken by filtration, washed with chloroform, dried under reduced pressure to obtain 209 mg (84%) of the title compound as orange crystals without purification.
¹H-NMR (DMSO-d₆) δ: 1.31 (9H, s), 3.45 (2H, m), 6.88 (1H, s), 7.77 (1H, d, J=7.08Hz), 8.02 (1H, s), 8.99 (1H, d, J=7.08Hz)
EI/MS; m/z: 345 (M⁺+1)

### (E) N⁸-[4-(tert-Butyl)-1,3-thiazol-2-yl]-2-morpholino-4-oxo-4H-pyrido[1,2-a]-pyrimidine-8-carboxamide

Reactions were performed in the same manner as in Example 2, (A) by using N⁸-[4-(tert-butyl)-1,3-thiazol-2-yl]-2,4-dioxo-3,4-dihydro-2H-pyrido[1,2-a]pyrimidine-8-carboxamide (209 mg, 0.607 mmol). The resulting crude product was purified by silica gel column chromatography (chloroform:methanol =50:1 → 30:1) to obtain 147 mg of the title compound in a mixture with byproducts.
¹H-NMR (CD₃OD) δ : 1.36 (9H, s), 3.46 (4H, t, J=4.64Hz), 3.58 (4H, t, J=4.64Hz), 5.71 (1H, s), 6.64 (1H, s), 7.51 (1H, d, J=7.57Hz), 7.99 (1H, s), 8.89 (1H, d, J=7.57Hz) EI/MS; m/z: 414 (M⁺+1)

### (F) N⁸-[4-(tert-Butyl)-1,3-thiazol-2-yl]-3-formyl-2-morpholino-4-oxo-4H-pyrido[1,2-a]-pyrimidine-8-carboxamide

Dimethylformamide (1 ml) was added dropwise with phosphorus oxychloride (66 µl, 0.712 mmol) under ice cooling, then warmed to room temperature, and stirred for 15 minutes. The reaction solution was cooled to 0°C with ice, added with N⁸-[4-(tert-butyl)-1,3-thiazol-2-yl]-2-morpholino-4-oxo-4H-pyrido[1,2-a]pyrimidine-8-c arboxamide (147 mg, 0.356 mmol) dissolved in dimethylformamide (4 ml) under ice cooling, and stirred at 0°C for 2 hours and 30 minutes. The reaction solution was neutralized with saturated aqueous sodium hydrogencarbonate and concentrated under reduced pressure. The resulting residue was added with chloroform for extraction, and the organic layer was dried over magnesium sulfate and concentrated under reduced pressure. The resulting residue was purified by thin layer silica gel chromatography (chloroform:methanol = 40:1) to obtain 98.2 mg of the title compound as yellow crystals in a mixture with dimethylformamide.
¹H-NMR (CDCl₃) δ : 1.34 (9H, s), 3.78 (4H, d, J=4.88Hz), 3.82 (4H, d, J=4.88Hz), 6.59 (1H, s), 7.44 (1H, dd, J=1.71, 7.32Hz), 7.86 (1H, s), 8.87 (1H, d, J=7.32Hz), 10.16 (1H, s) EI/MS; m/z: 442 (M⁺+1)

### (G) Methyl (E)-3-[8-({[4-(tert-butyl)-1,3-thiazol-2-yl]amino}carbonyl)-2-morpholino-4-oxo-4H-pyrido[1,2-a]pyrimidin-3-yl]-2-propenoate

N⁸-[4-(tert-Butyl)-1,3-thiazol-2-yl]-3-formyl-2-morpholino-4-oxo-4H-pyrido-[1,2-a]pyrimidine-8-carboxamide (98.2 mg, 0.223 mmol) was added with tetrahydrofuran (6 ml), lithium chloride (30 mg, 0.669 mmol) and bis(2,2,2-trifluoroethyl)(methoxycarbonylmethyl)-phosphonate (142 µl, 0.669 mmol), then added dropwise with 1,8-diazabicyclo[5.4.0]undec-7-ene (92 µl, 0.669 mmol) at room temperature, stirred at room temperature for 1 hour, and further added with bis(2,2,2-trifluoroethyl)(methoxycarbonylmethyl)-phosphonate (75 µl) and 1,8-diazabicyclo[5.4.0]undec-7-ene (45 µl). The reaction solution was further stirred at room temperature for 30 minutes and concentrated under reduced pressure. The resulting residue was purified by thin layer silica gel chromatography (chloroform:methanol = 40:1) to obtain 145.8 mg of the title compound in a mixture with 1,8-diazabicyclo[5.4.0]undec-7-ene.
¹H-NMR (CD₃OD) δ : 1.35 (9H, s), 3.70 (4H, m), 3.78 (4H, m), 3.86 (3H, s), 6.62 (1H, s), 7.12 (1H, d, J=15.63Hz), 7.41 (1H, s), 7.58 (1H, d, J=7.57Hz), 7.59 (1H, d, J=15.63Hz), 8.98 (1H, d, J=7.57Hz)
EI/MS; m/z: 498 (M⁺+1)

### (H) (E)-3-[8-({[4-(tert-Butyl)-1,3-thiazol-2-yl]amino}caxbonyl)-2-morpholino-4-oxo-4H-pyrido[1,2-a]pyrimidin-3-yl]-2-propenoic acid

Methyl (E)-3-[8-({[4-(tert-butyl)-1,3-thiazol-2-yl]amino}carbonyl)-2-morpholino-4-oxo-4H-pyrido[1,2-a]pyrimidin-3-yl]-2-propenoate (145.8 mg, 0.293 mmol) was added with methanol (2 ml), tetrahydrofuran (5 ml) and water (1 ml), and then added dropwise with 1 N sodium hydroxide (1 ml) at room temperature. After the reaction solution was stirred at room temperature for 1 hour, the solution was further added with 1 N sodium hydroxide (2 ml) and further stirred at room temperature for 15 hours. The reaction solution was added with 1 N hydrochloric acid until pH of the solution became 4 and extracted with chloroform. The organic layer was dried over magnesium sulfate and concentrated under reduced pressure. The resulting residue was purified by thin layer silica gel chromatography (chloroform:methanol = 20:1) to obtain 15.4 mg (5% for the four steps) of the title compound as yellow crystals.
¹H-NMR (DMSO-d₆) δ: 1.27 (9H, s), 3.70 (4H, m), 3.78 (4H, m), 6.54 (1H, brd), 6.93 (1H, d, J=15.38Hz), 7.48 (1H, d, J=15.38Hz), 7.78 (1H, d, J=7.08Hz), 8.05 (1H, s), 8.88 (1H, d, J=7.08Hz)
ES-MS: 484 (M⁺+1), 482 (M+-1)

### Example 8: (E)-3-[8-{[(4-Cyclobutyl-1,3-thiazol-2-yl)amino]carbonyl}-2-(3-hydroxypiperidino)-4-oxo-4H-pyrido[1,2-alpyrimidin-3-yl]-2-propenoic acid

### (A) 4-Cyclobutyl-1,3-thiazol-2-amine

Thiourea (2.3 g, 30.0 mmol) was dissolved in ethanol (100 ml), added with 2-bromo-1-cyclobutyl-1-ethanone synthesized in the same manner as in Example 1, (E), and then heated to 100°C. The reaction solution was stirred for 1 hour, then returned to room temperature, neutralized with saturated sodium hydrogencarbonate and extracted with chloroform. The collected organic layer was washed with saturated brine, dried over magnesium sulfate and then concentrated under reduced pressure. The title compound was obtained as the residue without purification as a brown oily substance (5.4 g, 100%).
¹H-NMR (CDCl₃) δ : 1.86 (1H, m), 1.98 (1H, qu, J=9.31Hz), 2.12-2.37 (4H, m), 3.38 (1H, qu, J=8.08Hz), 5.36 (2H, brd), 6.07 (1H, s)

### (B) N⁴-(4-Cyclobutyl-1,3-thiazol-2-yl)-2-(acetylamino)isonicotinamide

Reactions were performed in the same manner as in Example 7, (B) by using 2-(acetylamino)isonicotinic acid (3.3 g, 18.42 mmol) as the starting material and 4-cyclobutyl-1,3-thiazol-2-amine (3 g, 18.42 mmol) to obtain 3.76 g (65%) of the title compound.
¹H-NMR (CDCl₃) δ : 1.91 (1H, m), 2.03 (1H, qu, J=9.03Hz), 2.15-2.42 (4H, m), 2.23 (3H, s), 3.55 (1H, qu, J=8.55Hz), 6.63 (1H, s), 7.56 (1H, d, J=5.13Hz), 8.37 (1H, s), 8.66 (1H, s)
EI/MS; m/z: 317 (M⁺+1)

### (C) N⁴-(4-Cyclobutyl-1,3-thiazol-2-yl)-2-aminoisonicotinamide

Reactions were performed in the same manner as in Example 7, (C) by using N⁴-(4-cyclobutyl-1,3-thiazol-2-yl)-2-(acetylamino)isonicotinamide (3.76 g, 11.9 mmol) to obtain 1.73 g (53%) of the title compound.
¹H-NMR (CDCl₃) δ : 1.94-2.37 (6H, m), 3.58 (1H, qu, J=8.57Hz), 6.67 (1H, s), 7.07 (1H, s), 7.09 (1H, d, J=5.14Hz), 8.10 (1H, d, J=5.14Hz)
EI/MS; m/z: 273 (M⁺-1)

### (D) N⁸-(4-Cyclobutyl-1,3-thiazol-2-yl)-2,4-dioxo-3,4-dihydro-2H-pyrido[1,2-a]-pyrimidine-8-carboxamide

Reactions were performed in the same manner as in Example 7, (D) by using N⁴-(4-cyclobutyl-1,3-thiazol-2-yl)-2-aminoisonicotinamide (1.73 g, 6.3 mmol) to obtain 1.85 g (86%) of the title compound as brown solid.
¹H-NMR (DMSO-d₆) δ : 1.85-1.98 (2H, m), 2.16-2.27 (4H, m), 3.47 (2H, m), 3.57 (1H, m), 6.91 (1H, s), 7.74 (1H, d, J=7.35Hz), 8.27 (1H, s), 8.97 (1H, d, J=7.35Hz)
EI/MS; m/z: 343 (M⁺+1)

### (E) N⁸-(4-Cyclobutyl-1,3-thiazol-2-yl)-2-(3-hydroxypiperidino)-4-oxo-4H-pyrido[1,2-a]pyrimidine-8-carboxamide

N⁸-(4-Cyclobutyl-1,3-thiazol-2-yl)-2,4-dioxo-3,4-dihydro-2H-pyrido[1,2-a]-pyrimidine-8-carboxamide (100 mg, 0.29 mmol) was added with acetonitrile (2 ml) and dimethylformamide (1ml), cooled to -10°C with ice, then added with diphenyl chlorophosphate (0.2 ml, 0.96 mmol), and further added dropwise with diisopropylethylamine (0.3 ml, 1.74 mmol). The reaction solution was stirred at -10°C for 30 minutes, then added with 3-hydroxypiperidine (90 mg, 0.89 mmol), warmed to room temperature, and then further warmed to 80°C. As the reaction was not completed, the reaction solution was further added with 3-hydroxypiperidine (60 mg), stirred for 1 hour, and further added with 3-hydroxypiperidine (70 mg). After the disappear of the starting material was observed, the reaction was stopped with saturated aqueous sodium hydrogencarbonate, and the reaction solution was extracted with chloroform. The organic layer was dried over magnesium sulfate and concentrated under reduced pressure. The resulting residue was purified by thin layer silica gel chromatography (chloroform:methanol = 15:1) to obtain 85.2 mg (69%) of the title compound as yellow crystals.
¹H-NMR (CDCl₃) δ : 1.60-2.06 (6H, m), 2.21-2.35 (4H, m), 3.57 (4H, m), 3.95 (2H, m), 5.75 (1H, s), 6.62 (1H, s), 7.28 (1H, dd, J=1.71, 7.56Hz), 7.74 (1H, s), 8.87 (1H, d, J=7.56Hz)
EI/MS; m/z: 426 (M⁺+1)

### (F) N⁸-(4-Cyclobutyl-1,3-thiazol-2-yl)-3-formyl-2-(3-hydroxypiperidino)-4-oxo-4H-pyrido[1,2-a]pyrimidine-8-carboxamide

Reactions were performed in the same manner as in Example 7, (F) by using N⁸-(4-cyclobutyl-1,3-thiazol-2-yl)-2-(3-hydroxypiperidino)-4-oxo-4H-pyrido[1,2-a]-pyrimidine-8-carboxamide (85.2 mg, 0.20 mmol) to obtain 27.5 mg (30%) of the title compound.
¹H-NMR (CDCl₃) δ : 1.84-2.38 (10H, m), 3.43-3.63 (4H, m), 3.90 (1H, m), 4.08 (1H, m), 6.61 (1H, s), 7.39 (1H, d, J=7.07Hz), 7.82 (1H, s), 8.87 (1H, d.J=7.07Hz), 10.09 (1H, s)
EI/MS; m/z: 454 (M⁺+1)

### (G) Methyl (E)-3-[8-{[(4-cyclobutyl-1,3-thiazol-2-yl)amino]carbonyl}-2-(3-hydroxypiperidino)-4-oxo-4H-pyrido[1,2-a]pyrimidin-3-yl]-2-propenoate

Reactions were performed in the same manner as in Example 7, (G) by using N⁸-(4-cyclobutyl-1,3-thiazol-2-yl)-3-formyl-2-(3-hydroxypiperidino)-4-oxo-4H-pyrido[1,2-a]pyrimidine-8-carboxamide (27.5 mg, 0.061 mmol) to obtain 16.3 mg (53%) of the title compound.
¹H-NMR (CDCl₃) δ : 1.91 (5H, m), 2.06 (1H, m), 2.20 (2H, m), 2.38 (2H, m), 3.61 (4H, m), 3.68 (3H, s), 3.88 (1H, m), 4.04 (1H, m), 6.60 (1H, s), 7.14 (1H, d, J=15.63Hz), 7.47 (1H.dd, J=1.95, 7.57Hz), 7.57 (1H, d, J=15.63Hz), 7.94 (1H, s), 8.99 (1H, d, J=7.57Hz)
EI/MS; m/z: 510 (M⁺+1)

### (H) (E)-3-[8-{[(4-Cyclobutyl-1,3-thiazol-2-yl)amino]carbonyl}-2-(3-hydroxypiperidino)-4-oxo-4H-pyrido[1,2-a]pyrimidin-3-yl]-2-propenoic acid

Reactions were performed in the same manner as in Example 7, (H) by using methyl (E)-3-[8-{[(4-cyclobutyl-1,3-thiazol-2-yl)amino]carbonyl}-2-(3-hydroxypiperidino)-4-oxo-4H-pyrido[1,2-a]pyrimidin-3-yl]-2-propenoate (16.3 mg, 0.032 mmol) to obtain 11 mg (69%) of the title compound as orange crystals.
¹H-NMR (CD₃OD) δ : 1.59-2.08 (6H, m), 2.27-2.37 (4H, m), 3.62 (3H, s), 3.87 (2H, m), 4.08 (1H, d, J=12.21Hz), 6.69 (1H, s), 7.09 (1H, d, J=15.87Hz), 7.48 (1H, d, J=15.87Hz), 7.60 (1H, dd, J=1.95, 7.32Hz), 8.07 (1H, s), 8.94 (1H, d, J=7.32Hz)
EI/MS; m/z: 496 (M⁺+1)

### Example 9: (E)-3-[8-{[(4-Cyclobutyl-1,3-thiazol-2-yl)amino]carbonyl}-2-(4-methylpiperazino)-4-oxo-4H-pyrido[1,2-a]pyrimidin-3-yl]-2-propenoic acid

### (A) N⁸-(4-Cyclobutyl-1,3-thiazol-2-yl)-2-(4-methylpiperazino)-4-oxo-4H-pyrido[1,2-a]-pyrimidine-8-carboxamide

Reactions were performed in the same manner as in Example 8, (E) by using N⁸-(4-cyclobutyl-1,3-thiazol-2-yl)-2,4-dioxo-3,4-dihydro-2H-pyrido[1,2-a]pyrimidine-8-carboxamide (60 mg, 0.174 mmol) and N-methylpiperazine (60 µl, 0.52 mmol) as regents to obtain 51.2 mg (69%) of the title compound.
¹H-NMR (CDCl₃) δ: 1.94 (1H, m), 2.04 (1H, m), 2.23 (2H, m), 2.34 (2H, m), 2.35 (3H, s), 2.50 (4H, t, J=5.13Hz), 3.57 (1H, qu, J=8.30Hz), 3.72 (4H, brd), 5.68 (1H, s), 6.63 (1H, s), 7.30 (1H.dd, J=1.95, 7.32Hz), 7.80 (1H, s), 8.95 (1H, d, J=7.32Hz)
EI/MS; m/z: 425 (M⁺+1)

### (B) Methyl (E)-3-[8-{[(4-cyclobutyl-1,3-thiazol-2-yl)amino]carbonyl}-2-(4-methylpiperazino)-4-oxo-4H-pyrido[1,2-a]pyrimidin-3-yl]-2-propenoate

Reactions were performed in the same manner as in Example 7, (F) by using N⁸-(4-cyclobutyl-1,3-thiazol-2-yl)-2-(4-methylpiperazino)-4-oxo-4H-pyrido[1,2-a]-pyrimidine-8-carboxamide (51.2 mg, 0.1206 mmol), and the product was used without purification to perform reactions in the same manner as in Example 7,(G) to obtain 24.7 mg (40% for the two steps) of the title compound.
¹H-NMR (CDCl₃) δ: 1.94 (1H, m), 2.05 (1H, m), 2.23 (2H, m), 2.34 (3H, s), 2.35 (2H, m), 2.54 (4H, m), 3.58 (1H, qu, J=8.54Hz), 3.68 (4H, m), 3.81 (3H, s), 6.62 (1H, s), 7.16 (1H, d, J=15.63Hz), 7.43 (1H, dd, J=1.95, 7.57Hz), 7.57 (1H, d, J=15.63Hz), 7.88 (1H, s), 8.99 (1H, d, J=7.57Hz)
EI/MS; m/z: 509 (M⁺+1)

### (C) (E)-3-[8-{[(4-Cyclobutyl-1,3-thiazol-2-yl)amino]carbonyl}-2-(4-methylpiperazino)-4-oxo-4H-pyrido[1,2-a]pyrimidin-3-yl]-2-propenoic acid

Reactions were performed in the same manner as in Example 7, (H) by using methyl (E)-3-[8-{[(4-cyclobutyl-1,3-thiazol-2-yl)amino]carbonyl}-2-(4-methylpiperazino)-4-oxo-4H-pyrido[1,2-a]pyrimidin-3-yl]-2-propenoate (24.7 mg, 0.049 mmol) to obtain 14.1 mg (59%) of the title compound in yellow color.
¹H-NMR (CD₃OD) δ : 1.96-2.38 (6H, m), 2.37 (3H, s), 2.61 (4H, m), 3.59 (1H, m), 3.67 (4H, m), 6.66 (1H, s), 7.08 (1H, d, J=15.63Hz), 7.48 (1H, d, J=15.63Hz), 7.61 (1H.dd, J=1.71, 7.57Hz), 8.09 (1H, s), 8.96 (1H, d, J=7.57Hz)
EI/MS; m/z: 495 (M⁺+1)

### Example 10: N⁸-[4-(tert-Butyl)-1,3-thiazol-2-yl]-4-oxo-3-(2H-1,2,3,4-tetrazol-5-yl)-4H-pyrido[1,2-a]pyrimidine-8-carboxamide

### (A) Ethyl 2-[2-(4-methoxybenzyl)-2H-1,2,3,4-tetrazol-5-yl]acetate

Ethyl 1H-tetrazole-5-acetate (30 g, 0.192 mol) was dissolved in dimethylformamide (100 ml), cooled with ice, and added with potassium carbonate (32 g, 0.231 mol). The mixture was added dropwise with 4-methoxybenzochloride (31 ml, 0.231mol) and stirred at room temperature for 21 hours. The reaction solution was concentrated,under reduced pressure and subjected to azeotropy with toluene. The resulting residue was diluted with ethyl acetate and washed with water, and the compound dissolved in the aqueous layer was extracted with ethyl acetate. The organic layer collected was dried over magnesium sulfate. The organic layer was concentrated under reduced pressure, and then the residue was purified by silica gel column chromatography (hexane:ethyl acetate = 2:1) to obtain 24 g (46%) of the title compound as a colorless transparent substance.
¹H-NMR (CDCl₃) δ : 1.25 (3H, t, J=7.07Hz), 3.79 (3H, s), 3.93 (2H, s), 4.18 (2H, qu, J=7.07Hz), 5.68 (2H, s), 6.88 (2H, d, J=8.78Hz), 7.31 (2H, d, J=8.78Hz)
EI/MS; m/z: 275 (M⁺-1)

### (B) Ethyl 3-(dimethylamino)-2-[2-(4-methoxybenzyl)-2H-1,2,3,4-tetrazol-5-yl]-2-propenoate

Dimethylformamide dimethylacetal (13 ml, 97.3 mmol) was dissolved in ethyl 2-[2-(4-methoxybenzyl)-2H-1,2,3,4-tetrazol-5-yl]acetate (24 g, 88.4 mmol) and heated to 100°C for 3 hours with stirring. The reaction solution was purified by silica gel column chromatography (hexane:ethyl acetate = 3:1 → 1:1 → 1:3) to obtain 14 g (48%) of yellow crystals.
¹H-NMR (CDCl₃) δ : 1.26 (3H, t, J=7.07Hz), 2.04 (6H, s), 3.79 (3H, s), 4.11 (2H, qu, J=7.07Hz), 5.70 (2H, s), 6.86 (2H, d, J=9.02Hz), 7.30 (2H, d, J=9.02Hz), 7.73 (1H, s)
EI/MS; m/z: 332 (M⁺+1)

### (C) Ethyl 2-aminoisonicotinate

2-(Acetylamino)isonicotinic acid (13 g, 73.60 mmol) synthesized by the method of Example 7, (A) was added with ethanol (50 ml) and toluene (150 ml) and heated to 100-110°C. The mixture was added dropwise with concentrated sulfuric acid (7 ml) and heated for 11 hour with stirring. The reaction solution was returned to room temperature and poured into saturated aqueous sodium hydrogencarbonate cooled with ice. The mixture was extracted with chloroform and the collected organic layer was dried over magnesium sulfate and concentrated under reduced pressure to obtain 7.6 g (23% for the two steps) of the title compound as pale yellow crystals without purification.
¹H-NMR (CDCl₃) δ : 1.39 (3H, t, J=7.07Hz), 4.37 (2H, qu, J=7.07Hz), 4.63 (2H, brd), 7.07 (1H, s), 7.17 (1H, dd, J=0.98, 5.12Hz), 8.18 (1H, d, J=5.12Hz)
EI/MS; m/z: 165 (M⁺-1)

### (D) Ethyl 3-[2-(4-methoxybenzyl)-2H-1,2,3,4-tetrazol-5-yl]-4-oxo-4H-pyrido[1,2-a]-pyrimidine-8-carboxylate

Ethyl 2-aminoisonicotinate (6.5 g, 39.23 mmol) was added with acetic acid (500 ml) and ethyl 3-(dimethylamino)-2-[2-(4-methoxybenzyl)-2H-1,2,3,4-tetrazol-5-yl]-2-propenoate (13 g, 39.23 mmol) and refluxed by heating at 130°C for 5 hours. The reaction solution was returned to room temperature and poured into water, and this was extracted with chloroform. The resulting organic layer was washed with saturated brine, and the collected organic layer was dried over magnesium sulfate. The organic layer was concentrated under reduced pressure and subjected to azeotropy with toluene, and the resulting residue was purified by silica gel column chromatography (chloroform → chloroform:methanol = 80:1 → 50:1 → 30:1) to obtain 8.6 g of the title compound.
¹H-NMR (CDCl₃) δ : 1.45 (3H, t, J=7.08Hz), 3.79 (3H, s), 4.49 (2H,q, J=7.08Hz), 5.82 (2H, s)6.89 (2H, d, J=8.54Hz), 7.30 (2H, d, J=8.54Hz), 7.74 (1H, d, J=6.59Hz), 8.39 (1H, s), 9.28 (1H, s), 9.29 (1H, d, J=6.59Hz)
EI/MS; m/z: 407 (M⁺+1)

### (E) 3-[2-(4-Methoxybenzyl)-2H-1,2,3,4-tetrazol-5-yl]-4-oxo-4H-pyrido[1,2-a]-pyrimidine-8-carboxylic acid

Ethyl 3-[2-(4-methoxybenzyl)-2H-1,2,3,4-tetrazol-5-yl]-4-oxo-4H-pyrido[1,2-a]-pyrimidine-8-carboxylate (8.6 g, 21.1 mmol) was dissolved in tetrahydrofuran (170 ml) and added dropwise with 0.5 N aqueous sodium hydroxide (65 ml) at room temperature. The reaction solution was stirred for 1 hour and 30 minutes, then added with 1 N hydrochloric acid (55 ml) and water (250 ml) under ice cooling and warmed to room temperature with stirring. The deposited yellow crystals were taken by filtration, washed with water, dissolved in ethanol and concentrated under reduced pressure. The resulting crystals were dried under reduced pressure to obtain 4 g (27% for the two steps) of the title compound without purification.
¹H-NMR (CDCl₃) δ : 3.81 (3H, s), 5.83 (2H, s), 6.92 (2H, d, J=8.55Hz), 7.43 (2H, d, J=8.55Hz), 7.83 (1H, dd, J=1.22, 7.32Hz), 8.40 (1H, s), 9.23 (1H, s), 9.31 (1H, d, J=7.32Hz)
EI/MS; m/z: 379 (M⁺+1)

### (F) N⁸-[4-(tert-Butyl)-1,3-thiazol-2-yl]-4-oxo-3-(2H-1,2,3,4-tetrazol-5-yl)-4H-pyrido[1,2-a]pyrimidine-8-carboxamide

3-[2-(4-Methoxybenzyl)-2H-1,2,3,4-tetrazol-5-yl]-4-oxo-4H-pyrido[1,2-a]-pyrimidine-8-carboxylic acid (40 mg, 0.114 mmol) was added with dimethylformamide (2 ml), 4-(tert-butyl)-1,3-thiazol-2-amine (20 mg, 0.125 mmol), 1-hydroxybenzotriazole (20 mg, 0.136 mmol) and 4-dimethylaminopyridine (21 mg, 0.170 mmol) at room temperature, then added with 1-ethyl-3-(3-dimethylaminopropyl)carbodiimide hydrochloride (35 mg, 0.170 mmol) at room temperature, and stirred at room temperature for 24 hours. The reaction solution was added dropwise with 1 N hydrochloric acid (3 ml) at room temperature, and the deposited yellow crystals were taken by filtration and washed with water. The crystals were dissolved in chloroform and ethanol, concentrated under reduced pressure, and dried to obtain the title compound as yellow crystals without purification. The product was dissolved in anisole (0.2 ml) and trifluoroacetic acid (5 ml), stirred at room temperature for 5 hours, and added with water. The deposited yellow crystals were collected by filtration and washed with water, and the resulting crystals were dissolved in chloroform and ethanol and concentrated under reduced pressure. The crude crystals was added with ethanol, taken by filtration and washed with diethyl ether to obtain 28 mg (67% for the two steps) of the title compound as yellow crystals.
¹H-NMR (CD₃OD) δ : 1.38 (9H, s), 6.66 (1H, s), 8.10 (1H, d, J=8.30Hz), 8.57 (1H, s), 9.38 (1H, d, J=8.30Hz), 9.39 (1H, s)
EI/MS; m/z: 397 (M⁺+1)

### Example 11: N⁸-[4-(4-Pyridyl)-1,3-thiazol-2-yl]-4-oxo-3-(2H-1,2,3,4-tetrazol-5-yl)-4H-pyrido[1,2-a]pyrimidine-8-carboxamide

### (A) 2-Bromo-1-(4-pyridyl)-1-ethanone

4-Acetylpyridine (3.62 g, 29.9 mmol) was added with acetic acid (30 ml) and 47% hydrobromic acid (5.3 ml) at room temperature, subsequently added dropwise with bromine (1.6 ml in total) four times at an interval of 5 minutes, stirred at room temperature for 2 hours and 30 minutes. The mixture was further added with bromine (1.6 ml), and stirred at room temperature for 20 hours. The reaction solution was filtered, and the resulting crystals were washed with diethyl ether and dried to obtain 12.43 g of the title compound as a salt of hydrogen bromide in the form of orange crystals without purification.
¹H-NMR (CD₃OD) δ : 3.73 (2H, dd, J=1.21,28.76Hz), 8.26 (2H, d, J=6.83Hz), 8.89 (2H, d, J=6.83Hz)
EI/MS; m/z: 279 (M⁺-1)

### (B) 4-(4-Pyridyl)-1,3-thiazol-2-amine

Reactions were performed in the same manner as in Example 8, (A) by using 2-bromo-1-(4-pyridyl)-1-ethanone (1 g, 3.56 mmol) to obtain 410 mg (65%) of the title compound.
¹H-NMR (CD₃OD) δ : 7.26 (1H, s), 7.80 (2H, d, J=6.35Hz), 8.50 (2H, d, J=6.35Hz)
EI/MS; m/z: 176 (M⁺-1)

### (C) N⁸-[4-(4-Pyridyl)-1,3-thiazol-2-yl]-4-oxo-3-(2H-1,2,3,4-tetrazol-5-yl)-4H-pyrido[1,2-a]pyrimidine-8-carboxamide

Reactions were performed in the same manner as in Example 10, (F) by using 3-[2-(4-methoxybenzyl)-2H-1,2,3,4-tetrazol-5-yl]-4-oxo-4H-pyrido[1,2-a]pyrimidine-8-c arboxylic acid (120 mg, 0.32 mmol) and 4-(4-pyridyl)-1,3-thiazol-2-amine (62 mg, 0.35 mmol) to obtain 35.7 mg (27% for the two steps) of the title compound as orange crystals.
¹H-NMR (DMSO-d₆) δ : 8.03 (3H, m), 8.29 (1H, s), 8.66 (1H, d, J=0.977Hz), 8.73 (2H, d, J=5.62Hz), 9.29 (1H, s), 9.32 (1H, d, J=6.54Hz)
ES-MS; m/z: 418 (M⁺+1)
IR (cm⁻¹): 1668, 1633, 1567, 1492, 1288

### Example 12: N⁸-(1,3-Benzothiazol-2-yl)-4-oxo-3-(2H-1,2,3,4-tetrazol-5-yl)-4H-pyrido[1,2-a]pyrimidine-8-carboxamide

Reactions were performed in the same manner as in Example 10, (F) by using 3-[2-(4-methoxybenzyl)-2H-1,2,3,4-tetrazol-5-yl]-4-oxo-4H-pyrido[1,2-a]pyrimidine-8-c arboxylic acid (60 mg, 0.16 mmol) and 2-aminobenzothiazole (30 mg, 0.17 mmol) to obtain 2 mg (3% for the two steps) of an orange compound.
¹H-NMR (DMSO-d₆) δ : 7.39 (1H, t, J=7.57Hz), 7.52 (1H, t, J=7.57Hz), 7.78 (1H, d, J=7.57Hz), 8.05 (2H, d, J=7.57Hz), 8.61 (1H, s), 9.28 (1H, s), 9.31 (1H, d, J=7.57Hz)
ES-MS; m/z: 391 (M⁺+1)

### Example 13: N⁸-(4-Cyclobutyl-1,3-thiazol-2-yl)-2-(4-methylpiperazino)-4-oxo-4H-pyrido[1,2-a]pyrimidine-8-carboxamide

Reactions were performed in the same manner as in Example 9, (A) by using N⁸-(4-cyclobutyl-1,3-thiazol-2-yl)-2,4-dioxo-3,4-dihydro-2H-pyrido[1,2-a]pyrimidine-8-carboxamide (40 mg, 0.12 mmol), and the resulting compound was added with 4 N hydrochloric acid solution in dioxane (2 ml) and stirred to obtain 12 mg (24%) of the title compound as hydrochloride.
NMR for free form and Mass:
¹H-NMR (CDCl₃) δ : 1.94 (1H, m), 2.04 (1H, m), 2.23 (2H, m), 2.34 (2H, m), 2.35 (3H, s), 2.50 (4H, t, J=5.13Hz), 3.57 (1H, qu, J=8.30Hz), 3.72 (4H, brd), 5.68 (1H, s), 6.63 (1H, s), 7.30 (1H.dd, J=1.95, 7.32Hz), 7.80 (1H, s), 8.95 (1H, d, J=7.32Hz)
EI/MS; m/z: 425 (M⁺+1)

### Example 14: N⁸-(4-Isopropyl-1,3-thiazol-2-yl)-4-oxo-3-(2H-1,2,3,4-tetrazol-5-yl)-4H-pyrido[1,2-a]pyrimidine-8-carboxamide

### (A) N⁸-(4-Isopropyl-1,3-thiazol-2-yl)-3-[2-(4-methoxybenzyl)-2H-1,2,3,4-tetrazol-5-yl]-4-oxo-4H-pyrido[1,2-a]pyrimidine-8-carboxamide

3-[2-(4-Methoxybenzyl)-2H-1,2,3,4-tetrazol-5-yl]-4-oxo-4H-pyrido[1,2-a]-pyrimidine-8-carboxylic acid (250 mg, 0.66 mmol) was dissolved in dimethylformamide (6 ml), added with N,N-carbonylbis-1H-imidazole (abbreviated as "CDI" hereinafter, 160 mg, 0.991 mmol) and heated to 90°C with stirring. After 1 hour and 30 minutes, the reaction solution was further added with CDI (170 mg), further stirred for 1 hour, and then further added with CDI (170 mg). The reaction solution was stirred further 2 hours, then returned to room temperature, added with 4-isopropyl-1,3-thiazol-2-amine hydrobromide (1.32 mmol) and stirred at room temperature for 2 hours and 30 minutes. The reaction solution was added with 2 N hydrochloric acid (7 ml), and the deposited crystals were collected by filtration and washed with water. The resulting yellow crystals were dissolved in chloroform and washed with saturated brine, and the collected organic layer was dried over magnesium sulfate and concentrated under reduced pressure to obtain the title compound as yellow crystals without purification.
¹H-NMR (CD₃OD) δ : 1.34 (6H, d, J=6.83Hz), 3.00 (1H, qu, J=6.83Hz), 3.80 (3H, s), 5.85 (2H, s), 6.63 (1H, s), 6.92 (2H, d, J=8.78Hz), 7.44 (2H, d, J=8.78Hz), 8.04 (1H, dd, J=1.46, 7.56Hz), 8.50 (1H, s), 9.20 (1H, s), 9.34 (1H, d, J=7.56Hz)
EI/MS; m/z: 503 (M⁺+1)

### (B) N⁸-(4-Isopropyl-1,3-thiazol-2-yl)-4-oxo-3-(2H-1,2,3,4-tetrazol-5-yl)-4H-pyrido[1,2-a]pyrimidine-8-carboxamide

N⁸-(4-Isopropyl-1,3-thiazol-2-yl)-3-[2-(4-methoxybenzyl)-2H-1,2,3,4-tetrazol-5-yl]-4-oxo-4H-pyrido[1,2-a]pyrimidine-8-carboxamide (330 mg, 0.66 mmol) was added with trifluoroacetic acid (10 ml) at room temperature and stirred for 3 days. The reaction solution was poured into ice water, and the deposited yellow crystals were collected by filtration, washed with water, then dissolved in chloroform and ethanol, and concentrated under reduced pressure. The resulting yellow crystals were suspended in a small amount of ethanol, and the crystals were taken by filtration and washed with diethyl ether to obtain 54 mg (21% for the two steps) of the title compound as yellow crystals.
¹H-NMR (DMSO-d₆) δ : 1.27 (6H, d, J=6.84Hz), 2.93 (1H, m), 6.90 (1H, s), 8.02 (1H, d, J=7.08Hz), 8.55 (1H, s), 9.26 (1H, s), 9.29 (1H, d, J=7.08Hz)
ES-MS; m/z: 383 (M⁺+1)

### Example 15: (E)-3-(2-{3-[(Aminocarbonyl)oxy]piperidino}-8-{[(4-cyclobutyl-1,3-thiazol-2-yl)amino]carbonyl}-4-oxo-4H-pyrido[1,2-a]pyrimidin-3-yl)-2-propenoic acid

### (A) 1-(8-{[(4-Cyclobutyl-1,3-thiazol-2-yl)amino]carbonyl}-3-formyl-4-oxo-4H-pyrido[1,2-a]pyrimidin-2-yl)-3-piperidyl formate

Reactions were performed in the same manner as in Example 7, (F) by using N⁸-(4-cyclobutyl-1,3-thiazol-2-yl)-2-(3-hydroxypiperidino)-4-oxo-4H-pyrido[1,2-a]-pyrimidine-8-carboxamide (85 mg, 0.20 mmol) to obtain 21 mg (22%) of the title compound.
¹H-NMR (CDCl₃) δ : 1.92-2.41 (10H, m), 3.58-3.68 (3H, m), 3.93 (2H, m), 5.10 (1H, m), 6.61 (1H, s), 7.39 (1H, dd, J=1.95, 7.31Hz), 7.79 (1H, d, J=1.95Hz), 8.01 (1H, s), 8.89 (1H, d, J=7.31Hz), 10.14 (1H, s)
EI/MS; m/z: 482 (M⁺+1)

### (B) tert-Butyl (E)-3-[8-{[(4-cyclobutyl-1,3-thiazol-2-yl)amino]carbonyl}-2-(3-formyloxypiperidino)-4-oxo-4H-pyrido[1,2-a]pyrimidin-3-yl]-2-propenoate

Reactions were performed in the same manner as in Example 1, (J) by using 1-(8-{[(4-cyclobutyl-1,3-thiazol-2-yl)amino]carbonyl}-3-formyl-4-oxo-4H-pyrido[1,2-a]-pyrimidin-2-yl)-3-piperidyl formate (36 mg, 0.075 mmol), and the resulting reaction solution was purified by thin layer silica gel chromatography (chloroform:methanol = 30:1) to obtain 35 mg of the title compound as orange crystals in a mixture with triphenylphosphine oxide.
¹H-NMR (CDCl₃) δ : 1.52 (9H, s), 1.94-2.00 (6H, m), 2.23-2.34 (4H, m), 3.57-3.83 (5H, m), 5.15 (1H, m), 6.62 (1H, s), 7.09 (1H, d, J=15.63Hz), 7.48 (1H, dd, J=1.71, 7.32Hz), 7.49 (1H, d, J=15.63Hz), 7.91 (1H, s), 8.09 (1H, s), 8.98 (1H, d, J=7.32Hz)

### (C) tert-Butyl (E)-3-[8-{[(4-cyclobutyl-1,3-thiazol-2-yl)amino]carbonyl}-2-(3-hydroxypiperidino)-4-oxo-4H-pyrido[1,2-a]pyrimidin-3-yl]-2-propenoate

Reactions were performed in the same manner as in Example 1, (K) by using tert-butyl (E)-3-[8-{[(4-cyclobutyl-1,3-thiazol-2-yl)amino]carbonyl}-2-(3-formyloxypiperidino)-4-oxo-4H-pyrido[1,2-a]pyrimidin-3-yl]-2-propenoate (35 mg, 0.060 mmol), and the resulting crude crystals were purified by thin layer silica gel chromatography (chloroform:methanol = 40:1) to obtain 16 mg (40% for the two steps) of the title compound as yellow crystals.
¹H-NMR (CDCl₃) δ: 1.52 (9H, s), 1.72-2.07 (6H, m), 2.17-2.38 (4H, m), 3.60 (4H, m), 3.80 (1H, m), 4.02 (1H, m), 6.61 (1H, s), 7.10 (1H, d, J=15.60Hz), 7.48 (1H, dd, J=1.71, 7.31Hz), 7.49 (1H, d, J=15.60Hz), 7.97 (1H, s), 9.00 (1H, d, J=7.31Hz)
EI/MS; m/z: 552 (M⁺+1)

### (D) tert-Butyl (E)-3-(2-{3-[(aminocarbonyl)oxy]piperidino}-8-{[(4-cyclobutyl-1,3-thiazol-2-yl)amino]carbonyl}-4-oxo-4H-pyrido[1,2-a]pyrimidin-3-yl)-2-propenoate

tert-Butyl (E)-3-[8-{[(4-cyclobutyl-1,3-thiazol-2-yl)amino]carbonyl}-2-(3-hydroxypiperidino)-4-oxo-4H-pyrido[1,2-a]pyrimidin-3-yl]-2-propenoate (16 mg, 0.029 mmol) was dissolved in ethyl acetate (3 ml), added with trichloroacetyl isocyanate (10 µl) under ice cooling, stirred at 0°C for 1 hour, then further added with trichloroacetyl isocyanate (10 µl), and further stirred for 30 minutes. Then, the reaction solution was concentrated under reduced pressure, and the resulting residue was added with methanol (2 ml), water (0.2 ml) and sodium formate (12 mg) at room temperature and stirred at room temperature for 7 hours. The reaction solution was concentrated under reduced pressure, and the residue was purified by thin layer silica gel chromatography (chloroform:methanol = 30:1) to obtain 20 mg of the title compound.
¹H-NMR (CDCl₃) δ : 1.51 (9H, s), 1.90-2.36 (10H, m), 3.36-3.77 (4H, m), 4.04 (1H, m), 4.92 (1H, m), 6.62 (1H, s), 7.10 (1H, d, J=15.38Hz), 7.54 (1H, d, J=7.57Hz), 7.60 (1H, d, J=15.38Hz), 8.07 (1H, s), 9.00 (1H, d, J=7.57Hz)
EI/MS; m/z: 595 (M⁺+1)

### (E) (E)-3-(2-{3-[(Aminocarbonyl)oxy]piperidino}-8-{[(4-cyclobutyl-1,3-thiazol-2-yl)-amino]carbonyl}-4-oxo-4H-pyrido[1,2-a]pyrimidin-3-yl)-2-propenoic acid

tert-Butyl (E)-3-(2-{3-[(aminocarbonyl)oxy]piperidino}-8-{[(4-cyclobutyl-1,3-thiazol-2-yl)amino]carbonyl}-4-oxo-4H-pyrido[1,2-a]pyrimidin-3-yl)-2-propenoate (20 mg, 0.034 mmol) was added with a mixed solution of 4 N hydrochloric acid and dioxane (1.5 ml) at room temperature and stirred for 2 hours. The reaction solution was concentrated under reduced pressure, and the resulting residue was purified by thin layer silica gel chromatography (chloroform:methanol = 10:1) to obtain 9 mg (58% for the two steps) of the title compound as yellow crystals.
¹H-NMR (CD₃OD) δ: 1.68 (2H, m), 1.94-2.10 (4H, m), 2.23 (2H, m), 2.38 (2H, m), 3.39-3.72 (4H, m), 3.98 (1H, dd, J=5.86, 11.70Hz), 4.86 (1H, m), 6.64 (1H, s), 7.10 (1H, d, J=15.63Hz), 7.56 (1H, d, J=7.32Hz), 7.68 (1H, d, J=15.63Hz), 8.07 (1H, s), 8.96 (1H, d, J=7.32Hz)
ES-MS; m/z: 539 (M⁺+1)

### Example 16: 2-{8-[2-(4-Isopropyl-1,3-thiazol-2-yl)ethyl]-2-morpholino-4-oxo-4H-pyrido[1,2-a]pyrimidin-3-yl}-1-cyclopropanecarboxylic acid

### (A) 3-[(E)-3-Hydroxy-1-propenyl]-8-[2-(4-isopropyl-1,3-thiazol-2-yl)ethyl]-2-morpholino-4H-pyrido[1,2-a]pyrimidin-4-one

(E)-3-{8-[2-(4-Isopropyl-1,3-thiazol-2-yl)ethyl]-2-morpholino-4-oxo-4H-pyrido[1,2-a]pyrimidin-3-yl}-2-propenoic acid (222.7 mg, 0.49 mmol) dissolved in tetrahydrofuran (8 ml) was added with triethylamine (342 µl) and ethyl chloroformate (141 µl) at -20°C, then stirred at room temperature for 1 hour, and subsequently added with aqueous sodium borohydride (0.8 M, 4 ml) at room temperature. The aqueous sodium borohydride was occasionally added until the reaction was completed, and the completion of the reaction was confirmed by TLC. Then, the reaction solution was added with water and extracted with chloroform and chloroform:methanol = 10:1, and the organic layer collected was dried over magnesium sulfate and concentrated under reduced pressure. The resulting residue was purified by silica gel column chromatography (chloroform → chloroform:methanol = 100:1 → 60:1 → 30:1) to recover 81 mg (37%) of the title compound and 82 mg of the starting material.
¹H-NMR (CDCl₃) δ : 1.29 (6H, d, J=6.83Hz), 3.07 (1H, qu, J=6.83Hz), 3.19 (2H, t, J=7.80Hz), 3.36 (2H, t, J=7.80Hz), 3.53 (4H, t, J=4.88Hz), 3.80 (4H, t, J=4.88Hz), 4.35 (2H, d, J=5.85Hz), 6.44 (1H, d, J=15.60Hz), 6.72 (1H, s), 6.83 (1H, dd, J=1.95, 7.31Hz), 7.00 (1H, dt, J=5.85, 15.60Hz) 7.21 (1H, s), 8.87 (1H, d, J=7.31Hz)
EI/MS; m/s: 441 (M⁺+1)

### (B) 3-[2-(Hydroxymethyl)cyclopropyl]-8-[2-(4-isopropyl-1,3-thiazol-2-yl)ethyl]-2-morpholino-4H-pyrido[1,2-a]pyrimidin-4-one

3-[(E)-3-Hydroxy-1-propenyl]-8-[2-(4-isopropyl-1,3-thiazol-2-yl)ethyl]-2-morpholino-4H-pyrido[1,2-a]pyrimidin-4-one (81 mg, 0.18 mmol) was added with dichloromethane (3 ml) and a solution of diethyl zinc in hexane (1.02 M, 270 µl, 0.275 mmol) and subsequently added dropwise with iodomethane (30 µl, 0.366 mmol) at room temperature. After the reaction solution was stirred for 2 hours, the reaction was stopped with saturated aqueous ammonium chloride, and the reaction solution was extracted with chloroform. The organic layer was dried over magnesium sulfate and concentrated under reduced pressure. The resulting residue was purified by thin layer silica gel chromatography (chloroform:methanol = 15:1) to obtain 61 mg (73%) of the title compound.
¹H-NMR (CDCl₃) δ: 0.90 (1H, m), 1.04 (2H, m), 1.29 (6H, d, J=6.84Hz), 1.52 (1H, dt, J=5.62, 7.57Hz), 3.07 (1H, qu, J=6.84Hz), 3.17 (2H, t, J=7.32Hz), 3.35 (2H, t, J=7.32Hz), 3.56 (2H, m), 3.84 (6H, m), 4.12 (1H, d, J=9.52Hz), 4.68 (1H, brd), 6.73 (1H, s), 6.78 (1H, dd, J=1.71, 7.32Hz), 7.16 (1H, s), 8.80 (1H, d, J=7.32Hz)
EI/MS; m/s: 455 (M⁺+1)

### (C) 2-{8-[2-(4-Isopropyl-1,3-thiazol-2-yl)ethyl]-2-morpholino-4-oxo-4H-pyrido[1,2-a]-pyrimidin-3-yl}-1-cyclopropanecarboxylic acid

3-[2-(Hydroxymethyl)cyclopropyl]-8-[2-(4-isopropyl-1,3-thiazol-2-yl)ethyl]-2-morpholino-4H-pyrido[1,2-a]pyrimidin-4-one (55 mg, 0.121 mmol) dissolved in acetone (10 ml) was added dropwise with Jones' reagent (130 µl, 0.363 mmol) under ice cooling with and stirred for 2 hours. Then, the reaction solution was further added with Jones'reagent (165 µl) and stirred for 2 hours. Then, the reaction was stopped with saturated sodium thiosulfate, and the reaction solution was extracted with chloroform. The organic layer was dried over magnesium sulfate and concentrated under reduced pressure. The resulting residue was purified by thin layer silica gel chromatography (chloroform:methanol = 10:1) to obtain 2.2 mg (4%) of the title compound as yellow crystals.
¹H-NMR (CDCl₃) δ : 1.29 (6H, d, J=7.07Hz), 1.35 (1H, dt, J=4.39, 7.80Hz), 1.63 (1H, dt, J=4.39, 8.53Hz), 1.85 (1H, dt, J=3.90, 8.53Hz), 2.34 (1H, dt, J=3.90, 7.80Hz), 3.07 (1H, qu, J=7.07Hz), 3.18 (2H, t, J=7.07Hz), 3.35 (2H, t, J=7.07Hz), 3.58 (2H, m), 3.77 (6H, m), 6.72 (1H, s), 6.80 (1H, dd, J=1.46, 7.31Hz), 7.17 (1H, s), 8.81 (1H, d, J=7.31Hz)
EI/MS; m/s: 469 (M⁺+1)

### Example 17: (E)-3-{8-({[4-(tert-Butyl)-1,3-thiazol-2-yl]amino}carbonyl)-2-[(3R)-3-hydroxyhexahydro-1-pyridinyl]-4-oxo-4H-pyrido[1,2-a]pyrimidin-3-yl}-2-propenoic acid

### (A) N⁸-[4-(tert-Butyl)-1,3-thiazol-2-yl]-2-[(3R)-3-hydroxyhexahydro-1-pyridinyl]-4-oxo-4H-pyrido[1,2-a]pyrimidine-8-carboxamide

Reactions were performed in the same manner as in Example 8, (E) by using N⁸-[4-(tert-butyl)-1,3-thiazol-2-yl]-2,4-dioxo-3,4-dihydro-2H-pyrido[1,2-a]pyrimidine-8-carboxamide (300 mg, 0.871 mmol) and (R)-(+)-3-hydroxypiperidine (530 mg, 5.226 mmol) to obtain 241.8 mg (65%) of the title compound.
¹H-NMR (CDCl₃) δ : 1.34 (9H, s), 1.57 (1H, m), 1.76 (1H, m), 1.96 (2H, m), 3.61 (3H, m), 3.90 (2H, m), 5.69 (1H, s), 6.60 (1H, s), 7.31 (1H, dd, J=1.71, 7.32Hz), 7.76 (1H, s), 8.86 (1H, d, J=7.32Hz)
EI/MS; m/s: 428 (M⁺+1)

### (B) (3R)-1-[8-({[4-(tert-Butyl)-1,3-thiazol-2-yl]amino}carbonyl)-3-formyl-4-oxo-4H-pyrido[1,2-a]pyrimidin-2-yl]hexahydro-3-pyridinyl formate

Reactions were performed in the same manner as in Example 7, (F) by using N⁸-[4-(tert-butyl)-1,3-thiazol-2-yl]-2-[(3R)-3-hydroxyhexahydro-1-pyridinyl]-4-oxo-4H-pyrido[1,2-a]pyrimidine-8-carboxamide (295 mg, 0.690 mmol) and phosphorus oxychloride (130 µl, 1.38 mmol) to obtain 277 mg (68%) of the title compound.
¹H-NMR (CDCl₃) δ: 1.34 (9H, s), 1.70 (1H, m), 1.87-2.03 (3H, m), 3.66 (2H, m), 3.91 (2H, m), 5.09 (1H, brd), 6.58 (1H, s), 7.52 (1H, dd, J=1.71, 7.32Hz), 7.96 (1H, s), 8.01 (1H, s), 8.87 (1H, d, J=7.32Hz), 10.14 (1H, s)
EI/MS; m/s: 484 (M⁺+1)

### (C) tert-Butyl (E)-3-{8-({[4-(tert-butyl)-1,3-thiazol-2-yl] amino}carbonyl)-2-[(3R)-3-formyloxyhexahydro-1-pyridinyl]-4-oxo-4H-pyrido[1,2-a]pyrimidin-3-yl}-2-propenoate

Reactions were performed in the same manner as in Example 1, (J) by using (3R)-1-[8-({[4-(tert-butyl)-1,3-thiazol-2-yl]amino}carbonyl)-3-formyl-4-oxo-4H-pyrido[1,2-a]pyrimidin-2-yl]hexahydro-3-pyridinyl formate (277 mg, 0.5723 mmol), and the resulting residue was purified by silica gel column chromatography (chloroform → chloroform:methanol = 100:1 → 70:1 → 50:1) to obtain 559 mg (100% or more) of the title compound as a brown oily substance in a mixture with byproducts.
¹H-NMR (CDCl₃) δ : 1.28 (9H, s), 1.53 (9H, s), 1.71-1.97 (4H, m), 3.46-3.73 (4H, m), 5.09 (1H, brd), 6.56 (1H, s), 7.09 (1H, d, J=15.60Hz), 7.64 (1H, dd, J=1.95, 7.31Hz), 7.65 (1H, d, J=15.60Hz), 8.01 (1H, s), 8.05 (1H, s), 8.82 (1H, d, J=7.31Hz)
EI/MS; m/s: 580 (M⁺-1)

### (D) tert-Butyl (E)-3-{8-({[4-(tert-butyl)-1,3-thiazol-2-yl]amino}carbonyl)-2-[(3R)-3-hydroxyhexahydro-1-pyridinyl]-4-oxo-4H-pyrido(1,2-a]pyrimidin-3-yl}-2-propenoate

Reactions were performed in the same manner as in Example 1, (K) by using tert-butyl (E)-3-{8-({[4-(tert-butyl)-1,3-thiazol-2-yl]amino}carbonyl)-2-[(3R)-3-formyloxyhexahydro-1-pyridinyl]-4-oxo-4H-pyrido[1,2-a]pyrimidin-3-yl}-2-propenoate (559 mg, 0.961 mmol) to obtain 368 mg (69%) of the title compound as an orange oily substance.
¹H-NMR (CDCl₃) δ: 1.32 (9H, s), 1.52 (9H, s), 1.77-1.90 (4H, m), 3.54-3.73 (4H, m), 4.01 (1H, brd), 6.54 (1H, s), 7.04 (1H, d, J=15.63Hz), 7.64 (1H, dd, J=1.95, 7.32Hz), 7.65 (1H, d, J=15.63Hz), 8.00 (1H, s), 8.84 (1H, d, J=7.32Hz)

### (E) (E)-3-{8-({[4-(tert-Butyl)-1,3-thiazol-2-yl]amino}carbonyl)-2-[(3R)-3-hydroxyhexahydro-1-pyridinyl]-4-oxo-4H-pyrido[1,2-a]pyrimidin-3-yl}-2-propenoic acid

Reactions were performed in the same manner as in Example 15, (E) by using tert-butyl (E)-3-{8-({[4-(tert-butyl)-1,3-thiazol-2-yl]amino}carbonyl)-2-[(3R)-3-hydroxyhexahydro-1-pyridinyl]-4-oxo-4H-pyrido[1,2-a]pyrimidin-3-yl}-2-propenoate (140 mg, 0.253 mmol) as a mixture containing triphenylphosphine oxide to obtain 42 mg (33%) of the title compound as orange crystals.
¹H-NMR (CD₃OD) δ : 1.36 (9H, s), 1.61 (2H, m), 1.92 (1H, m), 2.06 (1H, m), 3.21 (1H, dd, J=8.55, 12.94Hz), 3.36 (1H, m), 3.86 (2H, m), 4.10 (1H, d, J=12.94Hz), 6.68 (1H, s), 7.05 (1H, d, J=15.63Hz), 7.60 (1H, dd, J=1.95, 7.32Hz), 7.61 (1H, d, J=15.63Hz), 8.05 (1H, s), 8.95 (1H, d, J=7.32Hz)
ES-MS; m/s: 498 (M⁺+1)

### Example 18: (E)-3-[2-{(3R)-3-[(Aminocarbonyl)oxy]hexahydro-1-pyridinyl}-8-({[4-(tert-butyl)-1,3-thiazol-2-yl]amino}carbonyl)-4-oxo-4H-pyrido[1,2-a]pyrimidin-3-yl]-2-propenoic acid

### (A) tert-Butyl (E)-3-[2-{(3R)-3-[(aminocarbonyl)oxy]hexahydro-1-pyridinyl}-8-({[4-(tert-butyl)-1,3-thiazol-2-yl]amino}carbonyl)-4-oxo-4H-pyrido[1,2-a]pyrimidin-3-yl]-2-propenoate

Reactions were performed in the same manner as in Example 15, (D) by using tert-butyl (E)-3-{8-({[4-(tert-butyl)-1,3-thiazol-2-yl]amino}carbonyl)-2-[(3R)-3-hydroxyhexahydro-1-pyridinyl]-4-oxo-4H-pyrido[1,2-a]pyrimidin-3-yl}-2-propenoate (240 mg, 0.436 mmol) to obtain 254 mg (98%) of an orange oily compound.
¹H-NMR (CDCl₃) δ : 1.30 (9H, s), 1.49 (9H, s), 1.69-2.17 (4H, m), 3:34 (1H, m), 3.54 (1H, m), 3.65 (1H, m), 3.87 (1H, m), 4.93 (1H, brd), 6.60 (1H, s), 7.07 (1H, d, J=15.63Hz), 7.56 (1H, dd, J=1.95, 7.33Hz), 7.68 (1H, d, J=15.63Hz), 8.06 (1H, s), 8.96 (1H, d, J=7.33Hz)

### (B) (E)-3-[2-{(3R)-3-[(Aminocarbonyl)oxy]hexahydro-1-pyridinyl}-8-({[4-(tert-butyl)-1,3-thiazol-2-yl]amino}carbonyl)-4-oxo-4H-pyrido[1,2-a]pyrimidin-3-yl]-2-propenoic acid

Reactions were performed in the same manner as in Example 15, (E) by using tert-butyl (E)-3-[2-{(3R)-3-[(aminocarbonyl)oxy]hexahydro-1-pyridinyl}-8-({[4-(tert-butyl)-1,3-thiazol-2-yl]amino}carbonyl)-4-oxo-4H-pyrido[1,2-a]pyrimidin-3-yl]-2-propenoate (254 mg, 0.426 mmol) containing byproducts to obtain 51 mg (22%) of the title compound as orange crystals.
¹H-NMR (CD₃OD) δ : 1.35 (9H, s), 1.68 (1H, m), 1.94 (3H, m), 3.46 (1H, m), 3.66 (3H, m), 3.98 (1H, dd, J=4.88, 13.43Hz), 4.87 (1H, s), 6.63 (1H, s), 7.10 (1H, d, J=15.63Hz), 7.56 (1H, dd, J=1.95; 7.33Hz), 7.68 (1H, d, J=15.63Hz), 8:06 (1H, s), 8.96 (1H, d, J=7.33Hz)
EI/MS; m/s: 541 (M⁺+1)

### Example 19: (E)-3-[2-{(3S)-3-[(Aminocarbonyl)oxy]hexahydro-1-pyridinyl}-8-({[4-(tert-butyl)-1,3-thiazol-2-yl]amino}carbonyl)-4-oxo-4H-pyrido[1,2-a]pyrimidin-3-yl]-2-p ropenoic acid

### (A) N⁸-[4-(tert-Butyl)-1,3-thiazol-2-yl]-2-[(3S)-3-hydroxyhexahydro-1-pyridinyl]-4-oxo-4H-pyrido[1,2-a]pyrimidine-8-carboxamide

Reactions were performed in the same manner as in Example 8, (E) by using N⁸-[4-(tert-butyl)-1,3-thiazol-2-yl]-2,4-dioxo-3,4-dihydro-2H-pyrido[1,2-a]pyrimidine-8-carboxamide (600 mg, 1.74 mmol) and (S)-(-)-3-hydroxypiperidine hydrochloride (360 mg, 2.61 mmol) to obtain 463 mg (62%) of the title compound.
¹H-NMR (CDCl₃) δ : 1.35 (9H, s), 1.59 (1H, brd), 1.73-1.99 (3H, m), 3.61 (3H, m), 3.93 (2H, m), 5.71 (1H, s), 6.61 (1H, s), 7.31 (1H, d, J=7.32Hz), 7.77 (1H, s), 8.89 (1H, d, J=7.32Hz)
EI/MS; m/s: 428 (M⁺+1)

### (B) (3S)-1-[8-({[4-(tert-Butyl)-1,3-thiazol-2-yl]amino}carbonyl)-3-formyl-4-oxo-4H-pyrido[1,2-a]pyrimidin-2-yl]hexahydro-3-pyridinyl formate

Reactions were performed in the same manner as in Example 7, (F) by using N⁸-[4-(tert-butyl)-1,3-thiazol-2-yl]-2-[(3S)-3-hydroxyhexahydro-1-pyridinyl]-4-oxo-4H-pyrido[1,2-a]pyrimidine-8-carboxamide (463 mg, 1.08 mmol) and phosphorus oxychloride (0.3 ml, 3.25 mmol) to obtain 600 mg (100%) of the title compound as a substance containing dimethylformamide without purification.
¹H-NMR (CDCl₃) δ : 1.32 (9H, s), 1.72 (1H, m), 1.89-2.04 (3H, m), 3.68 (2H, m), 3.92 (2H, d, J=3.90Hz), 5.09 (1H, brd), 6.59 (1H, s), 7.47 (1H, dd, J=1.95, 7.31Hz), 7.89 (1H, s), 8.01 (1H, s), 8.88 (1H, d, J=7.31Hz), 10.14 (1H, s)

### (C) tert-Butyl (E)-3-{8-({[4-(tert-butyl)-1,3-thiazol-2-yl]amino}carbonyl)-2-[(3S)-3-formyloxyhexahydro-1-pyridinyl]-4-oxo-4H-pyrido[1,2-a]pyrimidin-3-yl}-2-propenoate

Reactions were performed in the same manner as in Example 1, (J) by using (3S)-1-[8-({[4-(tert-butyl)-1,3-thiazol-2-yl]amino}carbonyl)-3-formyl-4-oxo-4H-pyrido[1,2-a]pyrimidin-2-yl]hexahydro-3-pyridinyl-formate (524 mg, 1.083 mmol), and the resulting residue was purified by silica gel column chromatography (chloroform → chloroform:methanol =100:1 → 70:1 → 50:1) to obtain 742 mg (100% or more) of the title compound as a brown oily substance in a mixture containing byproducts.
¹H-NMR (CDCl₃) δ: 1.28 (9H, s), 1.53 (9H, s), 1.68-2.03 (4H, m), 3.48-3.71.(4H, m), 5.10 (1H, m), 6.57 (1H, s), 7.09 (1H, d, J=15.63Hz), 7.64 (1H, dd, J=1.95, 7.32Hz), 7.65 (1H, d, J=15.63Hz), 7.99 (1H, s), 8.06 (1H, s), 8.85 (1H, d, J=7.32Hz)
EI/MS; m/s: 580 (M⁺-1)

### (D) tert-Butyl (E)-3-{8-({[4-(tert-butyl)-1,3-thiazol-2-yl]amino}carbonyl)-2-[(3S)-3-hydroxyhexahydro-1-pyridinyl]-4-oxo-4H-pyrido[1,2-a]pyrimidin-3-yl}-2-propenoate

Reactions were performed in the same manner as in Example 1, (K) by using tert-butyl (E)-3-{8-({[4-(tert-butyl)-1,3-thiazol-2-yl]amino}carbonyl)-2-[(3S)-3-formyloxyhexahydro-1-pyridinyl]-4-oxo-4H-pyrido[1,2-a]pyrimidin-3-yl}-2-propenoate (630 mg, 1.083 mmol) to obtain 439 mg (73%) of the title compound as an orange oily substance.
¹H-NMR (CDCl₃) δ : 1.34 (9H, s), 1.52 (9H, s), 1.78-1.90 (4H, m), 3.54 (2H, brd), 3.65-3.74 (2H, m), 4.02 (1H, brd), 6.53 (1H, s), 7.03 (1H, d, J=15.60Hz), 7.56 (1H, d, J=7.31Hz), 7.60 (1H, d, J=15.60Hz), 7.99 (1H, s), 8.84 (1H, d, J=7.31Hz)
EI/MS; m/s: 554 (M⁺+1)

### (E) tert-Butyl (E)-3-[2-{(3S)-3-[(aminocarbonyl)oxy]hexahydro-1-pyridinyl}-8-({[4-(tert-butyl)-1,3-thiazol-2-yl]amino}carbonyl)-4-oxo-4H-pyrido[1,2-a]pyrimidin-3-yl]-2-propenoate

Reactions were performed in the same manner as in Example 15, (D) by using tert-butyl (E)-3-{8-({[4-(tert-butyl)-1,3-thiazol-2-yl]amino}carbonyl)-2-[(3S)-3-hydroxyhexahydro-1-pyridinyl]-4-oxo-4H-pyrido[1,2-a]pyrimidin-3-yl}-2-propenoate (140 mg, 0.2529 mmol) to obtain 131 mg (87%) of an orange oily compound.
¹H-NMR (CDCl₃) δ : 1.30 (9H, s), 1.49 (9H, s), 1.70-2.00 (4H, m), 3.35 (1H, m), 3.54 (1H, d, J=12.43Hz), 3.67 (1H, m), 3.88 (1H, m), 4.92 (1H, brd), 6.60 (1H, s), 7.05 (1H, d, J=15.60Hz), 7.55 (1H, dd, J=1.95, 7.31Hz), 7.68 (1H, d, J=15.60Hz), 8.05 (1H, s), 8.97 (1H, d, J=7.31Hz)
EI/MS; m/s: 597 (M⁺+1)

### (F) (E)-3-[2-{(3S)-3-[(Aminocarbonyl)oxy]hexahydro-1-pyridinyl}-8-({[4-(tert-butyl)-1,3-thiazol-2-yl]amino}carbonyl)-4-oxo-4H-pyrido[1,2-a]pyrimidin-3-yl]-2-propenoic acid

Reactions were performed in the same manner as in Example 15, (E) by using tert-butyl (E)-3-[2-{(3S)-3-[(aminocarbonyl)oxy]hexahydro-1-pyridinyl}-8-({[4-(tert-butyl)-1,3-thiazol-2-yl]amino}carbonyl)-4-oxo-4H-pyrido[1,2-a]pyrimidin-3-yl]-2-propenoate (131 mg, 0.219 mmol) containing by products to obtain 40 mg (3.4%) of the title compound as orange crystals.
¹H-NMR (CD₃OD) δ : 1.35 (9H, s), 1.68 (1H, m), 1.93 (3H, m), 3.49 (1H, m), 3.69 (2H, m), 3.96 (1H, dd, J=5.36, 12.92Hz), 4.86 (1H, brd), 6.62 (1H, s), 7.11 (1H, d, J=15.60Hz), 7.55 (1H, dd, J=1.95, 7.56Hz), 7.68 (1H, d, J=15.60Hz), 8.06 (1H, s), 8.96 (1H, d, J=7.56Hz)
EI/MS; m/s: 541 (M⁺+1)

### Example 20: (E)-3-{8-({[4-(tert-butyl)-1,3-thiazol-2-yl]amino}carbonyl)-2-[(3S)-3-hydroxyhexahydro-1-pyridinyl]-4-oxo-4H-pyrido[1,2-a]pyrimidin-3-yl}-2-propenoic acid

Reactions were performed in the same manner as in Example 15, (E) by using tert-butyl(E)-3-{8-({[4-(tert-butyl)-1,3-thiazol-2-yl]amino}carbonyl)-2-[(3S)-3-hydroxyhexahydro-1-pyridinyl]-4-oxo-4H-pyrido[1,2-a]pyrimidin-3-yl}-2-propenoate (50 mg, 0.090 mmol) containing triphenylphosphine oxide as a mixture to obtain 17 mg (38%) of the title compound as orange crystals.
¹H-NMR (CD₃OD) δ : 1.39 (9H, s), 1.65 (2H, m), 1.92 (1H, m), 2.04 (1H, m), 3.82-4.03 (5H, m), 6.62 (1H, s), 7.07 (1H, d, J=15.43Hz), 7.56 (1H, d, J=7.35Hz), 7.60 (1H, d, J=15.43Hz), 8.03 (1H, s), 8.95 (1H, d, J=7.35Hz)
ES-MS; m/s: 498 (M⁺+1)

### Example 21: (E)-3-{2-[(3R)-3-(Dimethylamino)tetrahydro-1H-1-pyrrolyl]-8-[2-(4-isopropyl-1,3-thiazol-2-yl)ethyl]-4-oxo-4H-pyrido[1,2-a]pyrimidin-3-yl}-2-propenoic acid

tert-Butyl (E)-3-{8-[2-(4-isopropyl-1,3-thiazol-2-yl)ethyl]-2-[(4-methylphenyl)-sulfonyl]oxy-4-oxo-4H-pyrido[1,2-a]pyrimidin-3-yl}-2-propenate (31.4 mg, 0.053 mmol) was dissolved in dimethylformamide (1 ml), added with (3R)-(+)-3-(dimethylamino)-pyrrolidine (30.1 mg, 0.26 mmol), and stirred at room temperature for 4 hours. The solvent was evaporated, and the residue was purified by preparative TLC (chloroform:methanol = 10:1, v/v) to obtain 16.8 mg of yellow oil.

The product was added with 4 N hydrochloric acid solution in dioxane (2 ml), and stirred at room temperature for 4 hours. The solvent was evaporated, and the residue was purified by preparative TLC (chloroform:methanol =10:1, v/v). The residue was lyophilized to obtain 11.3 mg (44.5% for the two steps) of the title compound as yellow powder.
¹H-NMR (DMSO-d₆) δ : 1.20 (6H, d, J=6.8Hz), 1.68-1.80 (1H, m), 2.03-2.15 (1H, m), 2.19 (6H, s), 2.62-2.75 (1H, m), 2.92-3.01 (1H, m), 3.14 (2H, t, J=7.7Hz), 3.49-3.58 (1H, m), 3.62-3.78 (3H, m), 6.79 (1H, d, J=15.1Hz), 7.07 (1H, s), 7.08 (1H, dd, J=7.3, 1.7Hz), 7.22 (1H, s), 7.68 (1H, d, J=15.1Hz), 8.72 (1H, d, J=7.3Hz)
ESI/MS; m/z: 482 (MH⁺)
EI/MS; m/z: 481 (M⁺)
FAB/MS; m/z: 482 (MH⁺)
H-R FAB/MS: Calcd. for C₂₅H₃₁N₅O₃S: 482.2226, Found: 482.2230

In a similar manner, the following compounds were synthesized in which the substituent at the 2-position was converted.

### Example 22: (E)-3-{8-[2-(4-Isopropyl-1,3-thiazol-2-yl)ethyl]-2-[3-(oxymethyl)-piperidino]-4-oxo-4H-pyrido[1,2-a]pyrimidin-3-yl}-2-propenoic acid

¹H-NMR (DMSO-d₆) δ : 1.20 (6H, d, J=6.9Hz), 1.15-1.32 (2H, m), 1.52-1.83 (5H, m), 2.85 (1H, t, J=11.4Hz), 2.90-3.10 (2H, m), 3.16 (2H, t, J=7.3Hz), 3.85 (1H, brd, J=11.8Hz), 4.01 (1H, brd, J=12.0Hz), 4.56 (1H, brs), 6.85 (1H, d, J=15.4Hz), 7.07 (1H, s), 7.15 (1H, dd, J=7.3Hz), 7.28 (1H, s), 7.42 (1H, d, J=15.7Hz), 8.75 (1H, d, J=7.1Hz) ESI/MS; m/z: 483 (MH⁺)
FAB/MS; m/z: 483 (MH⁺), 505 (M⁺+Na)
Anal. Calcd. for C₂₅H₃₀N₄O₄Si · 5/4H₂O: C, 50.45; H, 6.49; N, 11.09, Found: C, 50.35; H, 6.16; N, 10.68

### Example 23: (E)-3-{2-[2-(Hydroxymethyl)morpholino]-8-[2-(4-isopropyl-1,3-thiazol-2-yl)ethyl]-4-oxo-4H-pyrido[1,2-a]pyrimidin-3-yl}-2-propenoic acid

¹H-NMR (DMSO-d₆) δ : 1.20 (6H, d, J=7.1Hz), 2.91-3.02 (2H, m), 3.12-3.22 (4H, m), 3.42-3.67 (2H, m), 3.75-3.82 (1H, m), 3.87-3.97 (2H, m), 4.77-4.82 (1H, m), 6.87 (1H, d, J=15.5Hz), 7.07 (1H, s), 7.20 (1H, dd, J=7.3, 2.0Hz), 7.34 (1H, s), 7.46 (1H, d, J=15.5Hz), 8.79 (1H, d, J=7.3Hz), 11.88 (1H, brs)
ESI/MS; m/z: 485 (MH⁺)
FAB/MS; m/z: 485 (MH⁺)
H-RFAB/MS: Calcd. for C₂₄H₂₈N₄O₅S: 485.1859, Found: 485.1862

### Example 24: (E)-3-{2-(3-Hydroxytetrahydro-1H-1-pyrrolyl)-8-[2-(4-isopropyl-1,3-thiazol-2-yl)ethyl]-4-oxo-4H-pyrido[1,2-a]pyrimidin-3-yl}-2-propenoic acid

¹H-NMR (DMSO-d₆) δ : 1.20 (6H, d, J=6.9Hz), 1.78-2.00 (2H, m), 2.92-3.02 (1H, m), 3.10-3.18 (3H, m), 3.55-3.64 (1H, m), 3.78-2.92 (2H, m), 4.39-4.46 (1H, m), 5.00 (1H, brd, J=3.4Hz), 6.79 (1H, d, J=15.2Hz), 7.06 (1H, d, J=1.7Hz), 7.07 (1H, dd, J=7.3, 0.7Hz), 7.18 (1H, d, J=1.7Hz), 7.68 (1H, d, J=15.2Hz), 8.70 (1H, d, J=7.3Hz)
EI/MS; m/z: 436 (M⁺-H₂O)
FAB/MS; m/z: 455 (MH⁺), 477 (M⁺+Na)
H-R FAB/MS: Calcd. for C₂₃H₂₆N₄O₄S: 455.1753, Found: 455.1753

### Example 25: (E)-3-{8-[2-(4-Isopropyl-1,3-thiazol-2-yl)ethyl]-4-oxo-2-[(3-pyridylmethyl)-amino]-4H-pyrido[1,2-a]pyrimidin-3-yl}-2-propenoic acid

¹H-NMR (DMSO-d₆) δ : 1.19 (6H, d, J=6.8Hz), 2.90-3.10 (1H, m), 3.13 (2H, t, J=7.5Hz), 4.69 (2H, d, J=5.9Hz), 7.06 (1H, s), 7.10 (1H, dd, J=7.3, 2.0Hz), 7.13 (1H, d, J=14.9Hz), 7.21 (1H, s), 7.32 (1H, dd, J=7.8, 4.9Hz), 7.74 (1H, d, J=14.9Hz), 7.75 (1H, brs), 8.32-8.39 (1H, m), 8.40-8.47 (1H, m), 8.58-8.62 (1H, m), 8.73 (1H, d, J=7.3Hz), 11.82 (1H, brs)
EI/MS; m/z: 431 (M⁺-CO₂)
FAB/MS; m/z: 476 (MH⁺)
H-R FAB/MS: Calcd. for C₂₅H₂₅N₅O₃S: 476.1756, Found: 476.1757

### Example 26: (E)-3-{8-[2-(4-Isopropyl-1,3-thiazol-2-yl)ethyl]-2-(3-hydroxypiperidino)-4-oxo-4H-pyrido[1,2-a]pyrimidin-3-yl}-2-propenoic acid

¹H-NMR (DMSO-d₆) δ : 1.19 (6H, d, J=6.8Hz), 1.30-1.48 (1H, m), 1.48-1.62 (1H, m), 1.73-1.88 (1H, m), 1.88-1.98 (1H, m), 2.92-3.01 (2H, m), 3.10-3.21 (3H, m), 3.37 (2H, t, J=7.6Hz), 3.54-3.65 (1H; m), 3.65-3.75 (1H, m), 3.84-3.92 (1H, m), 3.85-3.63 (1H, m), 6.85 (1H, d, J=15.5Hz), 7.07 (1H, s), 7.15 (1H, dd, J=7.4, 1.7Hz), 7.28 (1H, brs), 7.42 (1H, d, J=15.5Hz), 8.75 (1H, d, J=7.4Hz)
FAB/MS; m/z: 469 (MH⁺)
H-R FAB/MS: Calcd. for C₂₄H₂₈N₄O₄S: 469.1910, Found: 469.1927

### Example 27: (E)-3-{2-[(3R)-3-Aminotetrahydro-1H-1-pyrrolyl]-8-[2-(4-isopropyl-1,3-thiazol-2-yl)ethyl]-4-oxo-4H-pyrido[1,2-a]pyrimidin-3-yl}-2-propenoic acid

¹H-NMR (DMSO-d₆) δ : 1.21 (6H, d, J=6.8Hz), 2.00-2.10 (1H, m), 2.17-2.27 (1H, m), 2.93-3.03 (1H, m), 3.16 (2H, t, J=7.2Hz), 3.65-3.75 (2H, m), 3.84-3.98 (3H, m), 6.86 (1H, d, J=15.2Hz), 7.09 (1H, s), 7.13 (1H, d, J=7.3Hz), 7.25 (1H, s), 7.70 (1H, d, J=15.2Hz), 8.13-7.28 (2H, br), 8.75 (1H, d, J=7.3Hz)
LC/MS; m/z: 454 (MH⁺)
FAB/MS; m/z: 454 (MH⁺)
H-R FAB/MS: Calcd. for C₂₃H₂₇N₅O₃S: 454.1913, Found: 454.1920

### Example 28: (E)-3-{8-[2-(4-Isopropyl-1,3-thiazol-2-yl)ethyl]-4-oxo-2-piperazino-4H-pyrido[1,2-a]pyrimidin-3-yl}-2-propenoic acid

¹H-NMR (DMSO-d₆) δ : 1.20 (6H, d, J=6.8Hz), 2.92-3.02 (1H, m), 3.13-3.27 (6H, m), 3.21 (2H, t, J=7.2Hz), 6.92 (1H, d, J=15.6Hz), 7.10 (1H, s), 7.27 (1H, d, J=7.1Hz), 7.40 (1H, s), 7.43 (1H, d, J=15.6Hz), 8.85 (1H, d, J=7.3Hz), 9.17 (1H, br)
LC/MS; m/z: 454 (MH⁺)
EI/MS; m/z: 453 (M⁺)
FAB/MS; m/z: 454 (MH⁺), 476 (M⁺+Na)
H-R FAB/MS: Calcd. for C₂₃H₂₇N₅O₃S: 454.1913, Found: 454.1912

### Example 29: (E)-3-{2-(3,5-cis-Dimethylpiperazino)-8-[2-(4-isopropyl-1,3-thiazol-2-yl)-ethyl]-4-oxo-4H-pyrido[1,2-a]pyrimidin-3-yl}-2-propenoic acid

¹H-NMR (DMSO-d₆) δ : 1.21 (6H, d, J=6.9Hz), 1.26 (3H, s), 1.27 (3H, s), 2.92-3.02 (1H, m), 3.12-3.25 (4H, m), 3.90-3.98 (2H, m), 6.90 (1H, d, J=15.7Hz), 7.09 (1H, s), 7.26 (1H, dd, J=7.3, 1.7Hz), 7.42 (1H, d, J=15.7Hz), 7.41 (1H, s), 8.83 (1H, d, J=7.3Hz), 9.03 (1H, d, J=9.6Hz), 9.50 (1H, br)
EI/MS; m/z: 481 (M⁺)
H-R EI/MS: Calcd. for C₂₅H₃₁N₅O₃S: 481.2148, Found: 481.2150

### Example 30: (E)-3-{2-[4-(Dimethylamino)piperidino]-8-[2-(4-isopropyl-1,3-thiazol-2-yl)ethyl]-4-oxo-4H-pyrido[1,2-a]pyrimidin-3-yl}-2-propenoic acid

¹H-NMR (DMSO-d₆) δ: 1.21 (6H, d, J=6.8Hz), 1.64-1.78 (2H, m), 2.10-2.19 (2H, m), 2.73 (3H, s), 2.75 (3H, s), 2.93-3.02 (1H, m), 3.03-3.14 (2H, m), 3.19 (2H, t, J=7.2Hz), 3.38 (2H, t, J=7.2Hz), 4.02-4.11 (1H, m), 6.91 (1H, d, J=15.5Hz), 7.09 (1H, s), 7.22 (1H, dd, J=7.3, 1.7Hz), 7.34 (1H, s), 7.43 (1H, d, J=15.5Hz), 8.81 (1H, d, J=7.3Hz), 10.19-10.27 (1H, m)
FAB/MS; m/z: 496 (MH⁺)
H-R FAB/MS: Calcd. for C₂₆H₃₃N₅O₃S: 496.2382, Found: 496.2386

### Example 31: (E)-3-{2-[2-(Aminomethyl)morpholino]-8-[2-(4-isopropyl-1,3-thiazol-2-yl)-ethyl]-4-oxo-4H-pyrido[1,2-a]pyrimidin-3-yl}-2-propenoic acid

¹H-NMR (DMSO-d₆) δ : 1.20 (6H, d, J=6.9Hz), 2.84-3.14 (5H, m), 3.20 (2H, t, J=7.1Hz), 3.63-3.85 (3H, m), 3.85-3.92 (1H, m), 3.97-4.03 (1H, m), 6.90 (1H, d, J=15.5Hz), 7.08 (1H, s), 7.25 (1H, d, J=7.3Hz), 7.33 (1H, s), 7.45 (1H, d, J=15.5Hz), 7.92 (2H, br), 8.82 (1H, d, J=7.6Hz)
FAB/MS; m/z: 484 (MH⁺)
H-R EI/MS: Calcd. for C₂₄H₂₉N₅O₄S: 484.2019, Found: 484.1999

### Example 32: (E)-3-{8-[2-(4-Isopropyl-1,3-thiazol-2-yl)ethyl]-2-[(3R)-3-(methylamino)-tetrahydro-1H-1-pyrrolyl]-4-oxo-4H-pyrido[1,2-a]pyrimidin-3-yl}-2-propenoic acid

¹H-NMR (DMSO-d₆) δ : 1.20 (6H, d, J=6.8Hz), 2.11-2.31 (2H, m), 2.58-2.64 (3H, m), 2.92-3.02 (1H, m), 3.13-3.21 (2H, m), 3.35-3.42 (2H, m), 3.44-3.52 (1H, m), 3.65-3.75 (1H, m), 3.80-4.05 (3H, m), 6.86 (1H, d, J=15.2Hz), 7.11 (1H, s), 7.14 (1H, dd, J=7.3, 1.7Hz), 7.26 (1H, s), 7.68 (1H, d, J=15.2Hz), 8.76 (1H, d, J=7.3Hz), 9.05-9.30 (1H, br)
FAB/MS; m/z: 468 (MH⁺)
H-R FAB/MS: Calcd. for C₂₄H₂₉N₅O₃S: 468.2069, Found: 468.2085

### Example 33: ((E)-3-{2-[(3S)-3-(Dimethylamino)tetrahydro-1H-1-pyrrolyl]-8-[2-(4-. isopropyl-1,3-thiazol-2-yl)ethyl]-4-oxo-4H-pyrido[1,2-a]pyrimidin-3-yl}-2-propenoic acid

¹H-NMR (DMSO-d₆) δ : 1.20 (6H, d, J=6.8Hz), 1.68-1.80 (1H, m), 2.03-2.12 (1H, m), 2.18 (6H, s), 2.64-2.73 (1H, m), 2.92-3.03 (1H, m), 3.10-3.18 (2H, m), 2.49-2.58 (1H, m), 2.62-2.79 (3H, m), 6.78. (1H, d, J=15.1Hz), 7.05-7.08 (2H, m), 7.22 (1H, s), 7.68 (1H, d, J=15.1Hz), 8.71 (1H, d, J=7.3Hz)
FAB/MS; m/z: 482 (MH⁺), 504 (M⁺+Na).
H-R FAB/MS: Calcd. for C₂₅H₃₁N₅O₃S: 482.2226, Found: 482.2231

### Example 34: (E)-3-{8-[2-(4-Isopropyl-1,3-thiazol-2-yl)ethyl]-2-(4-methyl-1,4-diazepan-1-yl)-4-oxo-4H-pyrido[1,2-a]pyrimidin-3-yl}-2-propenoic acid

¹H-NMR (DMSO-d₆) δ : 1.21 (6H, d, J=6.8Hz), 2.08-2.10 (1H, m), 2.12-2.37 (1H, m), 2.77 (3H, s), 2.92-3.03 (1H, m), 3.12-3.20 (2H, m), 3.50-3.72 (5H, m), 3.75-3.87 (2H, m), 4.08-4.17 (1H, m), 6.80 (1H, d, J=15.4Hz), 7.10 (1H, s), 7.16 (1H, dd, J=7.3, 1.2Hz), 7.28 (1H, s), 7.48 (1H, d, J=15.4Hz), 8.77 (1H, d, J=7.3Hz), 10.38 (1H, br)
FAB/MS; m/z: 482 (MH⁺), 504 (M⁺+Na)
H-R FAB/MS: Calcd. for C₂₅H₃₁N₅O₃S: 482.2226, Found: 482.2234

### Example 35: (E)-3-{2-(4-Aminopiperidino)-8-[2-(4-isopropyl-1,3-thiazol-2-yl)ethyl]-4-oxo-4H-pyrido[1,2-a]pyrimidin-3-yl}-2-propenoic acid

¹H-NMR (DMSO-d₆) δ : 1.21 (6H, d, J=7.1Hz), 1.58-1.72 (2H, m), 1.97-2.08 (2H, m), 2.93-3.03 (1H, m), 3.06-3.17 (2H, m), 3.17-3.23 (2H, m), 3.23-3.55 (3H, m), 3.60-3.75 (2H, m), 6.91 (1H, d, J=15.5Hz), 7.15 (1H, s), 7.21 (1H, d, J=7.1Hz), 7.34 (1H, s), 7.42 (1H, d, J=15.5Hz), 8.19 (2H, br), 8.80 (1H, d, J=7.3Hz)
FAB/MS; m/z: 468 (MH⁺)
H-R FAB/MS: Calcd. for C₂₄H₂₉N₅O₃S: 468.2069, Found: 468.2069

### Example 36: (E)-3-{2-[4-(Hydroxymethyl)piperidino]-8-[2-(4-isopropyl-1,3-thiazol-2-yl)ethyl]-4-oxo-4H-pyrido[1,2-a]pyrimidin-3-yl}-2-propenoic acid

¹H-NMR (DMSO-d₆) 6 : 1.20 (6H, d, J=6.9Hz), 1.12-1.35 (2H, m), 1.68-1.82 (3H, m), 2.92-3.07 (3H, m), 3.12-3.21 (2H, m), 3.95-4.03 (2H, m), 4.52-4.57 (1H, m), 6.87 (1H, d, J=15.5H_{z}), 7.07 (1H, s), 7.16 (1H, d, J=6.6Hz), 7.28 (1H, s), 7.41 (1H, d, J=15.5Hz), 8.75 (1H, d, J=7.1Hz)
FAB/MS; m/z: 483 (MH⁺), 505 (M⁺+Na)
H-R FAB/MS: Calcd. for C₂₅H₃₀N₄O₄S: 483.2066, Found: 483.2064

### Example 37: (E)-3-{2-(3-Aminopiperidino)-8-[2-(4-isopropyl-1,3-thiazol-2-yl)ethyl]-4-oxo-4H-pyrido[1,2-a]pyrimidin-3-yl}-2-propenoic acid

¹H-NMR (DMSO-d₆) 8 : 1.21 (6H, d, J=6.8Hz), 1.58-1.72 (2H, m), 1.77-1.88 (1H, m), 2.00-2.14 (1H, m), 2.92-3.05 (1H, m), 3.03-3.55 (8H, m), 6.91 (1H, d, J=15.5H_{z}), 7.13 (1H, s), 7.24 (1H, d, J=6.8Hz), 7.37 (1H, s), 7.45 (1H, d, J=15.5Hz), 8.25 (3H, br), 8.82 (1H, d, J=6.8Hz)
FAB/MS; m/z: 468 (MH⁺)
H-R FAB/MS: Calcd. for C₂₄H₂₉N₅O₃S: 468.2069, Found: 468.2073

### Example 38: (E)-3-{8-[2-(4-Isopropyl-1,3-thiazol-2-yl)ethyl]-2-[3-(methylamino)-piperidino]-4-oxo-4H-pyrido[1,2-a]pyrimidin-3-yl}-2-propenoic acid

¹H-NMR (DMSO-d₆) δ : 1.21 (6H, d, J=6.8Hz), 1.55-1.78 (2H, m), 1.78-1.89 (1H, m), 2.05-2.20 (1H, m), 2.62 (3H, s), 2.92-3.03 (1H, m), 3.05-3.55 (8H, m), 3.98-4.07 (1H, m), 6.91 (1H, d, J=15.5Hz), 7.11 (1H, s), 7.24 (1H, d, J=7.3Hz), 7.38 (1H, s), 7.44 (1H, d, J=15.5Hz), 8.82 (1H, d, J=7.3Hz), 8.90 (1H, br), 9.07 (1H, br)
EI/MS; m/z: 481 (M⁺)
FAB/MS; m/z: 482 (MH⁺)
H-R FAB/MS: Calcd. for C₂₅H₃₁N₅O₃S: 482.2226, Found: 482.2244

### Example 39: (E)-3-{2-[3-(Dimethylamino)piperidino]-8-[2-(4-isopropyl-1,3-thiazol-2-yl)ethyl]-4-oxo-4H-pyrido[1,2-a]pyrimidin-3-yl}-2-propenoic acid

¹H-NMR (CDCl₃) δ : 1.29 (6H, d, J=6.8Hz), 1.52-1.80 (2H, m), 1.82-1.93 (1H, m), 2.14-2.23 (1H, m), 2.49 (6H, s), 2.80-2.90 (1H, m), 2.90-3.01 (2H, m), 3.01-3.12 (1H, m), 3.17-3.24 (2H, m), 3.32-3.40 (2H, m), 4.02-4.12 (1H, m), 4.27-4.36 (1H, m), 6.74 (1H, s), 6.83 (1H, dd, J=7.3, 1.7Hz), 7.08 (1H, d, J=15.6Hz), 7.19 (1H, s), 7.56 (1H, d, J=15.6Hz), 8.85 (1H, d, J=7.3Hz)
EI/MS; m/z: 495 (M⁺)
FAB/MS; m/z: 496 (MH⁺)
H-R FAB/MS: Calcd. for C₂₆H₃₃N₅O₃S: 496.2382, Found: 496.2383

### Example 40: (E)-3-{2-[3-(Aminocarbonyl)piperidino]-8-[2-(4-isopropyl-1,3-thiazol-2-yl)-ethyl]-4-oxo-4H-pyrido[1,2-a]pyrimidin-3-yl}-2-propenoic acid

¹H-NMR (DMSO-d₆) δ : 1.20 (6H, d, J=6.9Hz), 1.55-1.67 (2H, m), 1.67-1.84 (1H, m), 1.88-2.00 (1H, m), 2.40-2.50 (1H, m), 2.90-3.12 (3H, m), 3.12-3.22 (2H, m), 3.81-3.91 (1H, m), 4.00-4.12 (1H, m), 6.86 (1H, d, J=15.5Hz), 6.89 (1H, s), 7.07 (1H, s), 7.17 (1H, d, J=7.1Hz), 7.29 (1H, br), 7.34 (1H, s), 7.41 (1H, d, J=15.5Hz), 8.76 (1H, d, J=7.1Hz), 11.89 (1H, br)
FAB/MS; m/z: 496 (MH⁺)
H-R FAB/MS: Calcd. for C₂₅H₂₉N₅O₄S: 496.2019, Found: 496.2018

### Example 41: (E)-3-{2-[4-(Aminocarbonyl)piperidino]-8-[2-(4-isopropyl-1,3-thiazol-2-yl)-ethyl]-4-oxo-4H-pyrido[1,2-a]pyrimidin-3-yl}-2-propenoic acid

¹H-NMR (DMSO-d₆) δ : 1.20 (6H, d, J=6.9Hz), 1.60-1.76 (2H, m), 1.76-1.85 (2H, m), 2.35-2.46 (1H, m), 2.92-3.09 (3H, m), 3.12-3.20 (2H, m), 3.92-4.00 (2H, m), 6.82 (1H, s), 6.87 (1H, d, J=15.5Hz), 7.07 (1H, s), 7.17 (1H, dd, J=7.3, 1.7Hz), 7.22-7.33 (2H, m), 7.42 (1H, d, J=15.5Hz), 8.77 (1H, d, J=15.5Hz), 11.88 (1H, br)
FAB/MS; m/z: 496 (MH⁺)
H-R FAB/MS: Calcd. for C₂₅H₂₉N₅O₄S: 496.2019, Found: 496.2015

### Example 42: (E)-3-{2-[(7S)-7-Amino-5-azaspiro[2,4]hept-5-yl]-8-[2-(4-isopropyl-1,3-thiazol-2-yl)ethyl]-4-oxo-4H-pyrido[1,2-a]pyrimidin-3-yl}-2-propenoic acid

¹H-NMR (DMSO-d₆) δ : 1.70-1.90 (3H, m), 1.00-1.09 (1H, m), 1.20 (6H, d, J=6.9Hz), 2.91-3.02 (1H, m), 3.13-3.22 (2H, m), 3.22-3.28 (1H, m), 3.62-3.75 (1H, m), 3.77-3.85 (1H, m), 4.19-4.32 (2H, m), 6.87 (1H, d, J=15.2Hz), 7.08 (1H, s), 7.14 (1H, d, J=6.9, 2.2Hz), 7.25 (1H, s), 7.68 (1H, d, J=15.2Hz), 8.16 (2H, br), 8.75 (1H, d, J=6.9Hz)
FAB/MS; m/z: 480 (MH⁺)
H-R FAB/MS: Calcd. for C₂₅H₂₉N₅O₃S: 480.2069, Found: 480.2062

### Example 43: (E)-3-{2-[(3S,4S)-3-Amino-4-(fluoromethyl)tetrahydro-1H-1-pyrrolyl]-8-[2-(4-isopropyl-1,3-thiazol-2-yl)ethyl]-4-oxo-4H-pyrido[1,2-a]pyrimidin-3-yl}-2-propenoic acid

¹H-NMR (DMSO-d₆) δ : 1.21 (6H, d, J=6.9Hz), 2.83-3.07 (2H, m), 3.12-3.20 (2H, m), 3.34-3.42 (2H, m), 3.65-3.83 (2H, m), 3.87-4.10 (3H, m), 4.62-4.70 (1H, m), 4.72-4.81 (1H, m), 6.88 (1H, d, J=15.3Hz), 7.09 (1H, s), 7.14 (1H, dd, J=7.3, 1.7Hz), 7.27 (1H, s), 7.68 (1H, d, J=15.3Hz), 8.29 (2H, br), 8.75 (1H, d, J=7.3Hz)
FAB/MS; m/z: 486 (MH⁺)
H-R FAB/MS: Calcd. for C₂₄H₂₈FN₅O₃S: 486.1975, Found: 486.1974

### Example 44:

### (E)-3-{2-[(3R)-3-Hydroxypiperidino]-8-[2-(4-isopropyl-1,3-thiazol-2-yl)ethyl]-4-oxo-4H-pyrido[1,2-a]pyrimidin-3-yl}-2-propenoic acid

¹H-NMR (DMSO-d₆) δ : 1.19 (6H, d, J=6.9Hz), 1.30-1.60 (2H, m), 1.73-1.83 (1H, m), 1.83-1.96 (1H, m), 2.90-3.00 (2H, m), 3.08-3.21 (3H, m), 3.52-3.61 (1H, m), 3.63-3.72 (1H, m), 3.82-3.91 (1H, m), 4.85-4.93 (1H, m), 6.84 (1H, d, J=15.4Hz), 7.07 (1H, s), 7.14 (1H, d, J=6.9Hz), 7.27 (1H, s), 7.41 (1H, d, J=15.4Hz), 8.74 (1H, d, J=7.8Hz), 11.85 (1H, br).
FAB/MS; m/z: 469 (MH⁺)
H-R FAB/MS: Calcd. for C₂₄H₂₈N₄O₄S: 469.1910, Found: 469.1901

### Example 45: (E)-3-{2-[(3S)-3-Hydroxypiperidino]-8-[2-(4-isopropyl-1,3-thiazol-2-yl)-ethyl]-4-oxo-4H-pyrido[1,2-a]pyrimidin-3-yl}-2-propenoic acid

¹H-NMR (DMSO-d₆) δ : 1.20 (6H, d, J=6.9Hz), 1.31-1.61 (2H, m), 1.74-1.85 (1H, m), 1.85-1.98 (1H, m), 2.90-3.00 (2H, m), 3.08-3.21 (3H, m), 3.53-3.63 (1H, m), 3.63-3.75 (1H, m), 3.83-3.92 (1H, m), 4.86-4.95 (1H, m), 6.85 (1H, d, J=15.7Hz), 7.07 (1H, s), 7.15 (1H, d, J=6.6Hz), 7.28 (1H, s), 7.42 (1H, d, J=15.7Hz), 8.75 (1H, d, J=7.3Hz), 11.88 (1H, br)
FAB/MS; m/z: 469 (MH⁺)
H-R FAB/MS: Calcd. for C₂₄H₂₈N₄O₄S: 469.1910, Found: 469.1921

### Example 46: (E)-3-{2-(3-Amino-1-azetanyl)-8-[2-(4-isopropyl-1,3-thiazol-2-yl)ethyl]-4-oxo-4H-pyrido[1,2-a]pyrimidin-3-yl}-2-propenoic acid

¹H-NMR (DMSO-d₆) δ : 1.21 (6H, d, J=6.8Hz), 2.93-3.03 (1H, m), 3.13-3.23 (2H, m), 3.43-3.53 (2H, m), 3.65-3.85 (2H, m), 4.25-4.35 (2H, m), 4.50-4.58 (1H, m), 6.92 (1H, d, J=15.2Hz), 7.10 (1H, s), 7.16 (1H, d, J=7.6Hz), 7.28 (1H, s), 7.50 (1H, d, J=15.2Hz), 8.49 (1H, br), 8.77 (1H, d, J=7.1Hz)
FAB/MS; m/z: 440 (MH⁺)
H-R FAB/MS: Calcd. for C₂₂H₂₅N₅O₃S: 440.1756, Found: 440.1768

### Example 47: (E)-3-{2-(4-Fluoropiperidino)-8-[2-(4-isopropyl-1,3-thiazol-2-yl)ethyl]-4-oxo-4H-pyrido[1,2-a]pyrimidin-3-yl}-2-propenoic acid

¹H-NMR (DMSO-d₆) δ : 1.20 (6H, d, J=6.8Hz), 1.75-1.88 (2H, m), 1.93-2.10 (2H, m), 2.91-3.01 (1H, m), 3.14-3.21 (2H, m), 3.32-3.40 (2H, m), 3.40-3.52 (2H, m), 3.52-3.68 (2H, m), 4.83-4.92 (0.5H, m), 4.95-5.04 (0.5H, m), 6.88 (1H, d, J=15.6Hz), 7.07 (1H, s), 7.19 (1H, dd, J=7.1, 1.5Hz), 7.33 (1H, s), 7.44 (1H, d; J=15.6Hz), 8.79 (1H, d, J=7.1Hz), 11.91 (1H, br)
FAB/MS; m/z: 470 (MH⁺)
H-R FAB/MS: Calcd. for C₂₄H₂₇FN₄O₃S: 470.1788, Found: 470.1779

### Example 48: (E)-3-{8-[2-(4-Isopropyl-1,3-thiazol-2-yl)ethyl]-4-oxo-2-(4-oxopiperidino)-4H-pyrido[1,2-a]pyrimidin-3-yl]-2-propenoic acid

¹H-NMR (DMSO-d₆) δ : 1.20 (6H, d, J=6.8Hz), 2.91-3.01 (1H, m), 3.15-3.22 (2H, m), 3.30-3.41 (6H, m), 3.79-3.87 (4H, m), 6.91 (1H, d, J=15.4Hz), 7.07 (1H, s), 7.22 (1H, dd, J=7.3, 1.8Hz), 7.37 (1H, s), 7.52 (1H, d, J=15.4Hz), 8.81 (1H, d, J=7.3Hz), 11.94 (1H, br)
FAB/MS; m/z: 467 (MH⁺)
H-R FAB/MS: Calcd. for C₂₄H₂₆N₄O₄S: 467.1753, Found: 467.1765

### Example 49: (E)-3-(8-[2-(4-Isopropyl-1,3-thiazol-2-yl)ethyl]-4-oxo-2-{4-[(2R,3R,4S,5S,6R)-3,4,5-trihydroxy-6-(hydroxymethyl)tetrahydro-2H-2-pyranyl]-piperazino}-4H-pyrido[1,2-a]pyrimidin-3-yl)-2-propenoic acid

¹H-NMR (CD₃OD) δ: 1.35 (6H, d, J=6.8Hz), 3.12-3.23 (2H, m), 3.25-3.43 (4H, m), 3.53-3.90 (18H, m), 7.10 (1H, d, J=15.7Hz), 7.18 (1H, s), 7.35 (1H, dd, J=7.1,2.4Hz), 7.42 (1H, s), 7.57 (1H, d, J=15.7Hz), 8.94 (1H, d, J=7.1Hz)
LC-MS; m/z: 616 (MH⁺)

### Example 50: (E)-3-[8-[2-(4-Isopropyl-1,3-thiazol-2-yl)ethyl]-4-oxo-2-(4-{[(2R,3R,4S,5R,6R)-3,4,5-trihydroxy-6-(hydroxymethyl)tetrahydro-2H-2-pyranyl]oxy}piperidino)-4H-pyrido[1,2-a]pyrimidin-3-yl]-2-propenoic acid

¹H-NMR (CD₃OD) δ : 1.26 (6H, d, J=6.8Hz), 1.70-1.92 (2H, m), 1.92-2.10 (2H, m), 2.98-3.10 (1H, m), 3.17-3.25 (2H, m), 3.36-3.58 (7H, m), 3.68-3.78 (2H, m), 3.82-3.98 (3H, m), 4.00-4.12 (1H, m), 4.39 (1H, d, J=7.3Hz), 6.95 (1H, d, J=15.6Hz), 6.96 (1H, s), 7.04 (1H, dd, J=7.3, 1.7Hz), 7.21 (1H, s), 7.60 (1H, d, J=15.6Hz), 8.79 (1H, d, J=7.3Hz)
FAB/MS; m/z: 631 (MH⁺)
H-R FAB/MS: Calcd. for C₃₀H₃₈N₄O₉S: 631.2438, Found: 631.2485

### Example 51: (E)-3-{2-[cis-3,4-Dihydroxyhexahydro-1-pyridinyl]-8-[2-(4-isopropyl-1,3-thiazol-2-yl)ethyl]-4-oxo-4H-pyrido[1,2-a]pyrimidin-3-yl}-2-propenoic acid

¹H-NMR (DMSO-d₆) δ: 1.19 (6H, d, J=7.1Hz), 1.60-1.72 (1H, m), 1.77-1.88 (1H, m), 2.90-3.01 (1H, m), 3.12-3.20 (2H, m), 3.35-3.42 (2H, m), 3.45-3.66 (3H, m), 3.72-3.80 (1H, m), 4.57-4.60 (1H, m), 4.60-4.70 (1H, m), 6.83 (1H, d, J=15.5Hz), 7.06 (1H, s), 7.13 (1H, d, J=7.6Hz), 7.26 (1H, s), 7.42 (1H, d, J=15.5Hz), 8.73 (1H, d, J=7.1Hz)
FAB/MS; m/z: 485 (MH⁺)
H-R FAB/MS: Calcd. for C₂₄H₂₈N₄O₅S: 485.1859, Found: 485.1882

### Example 52: 3-{8-[2-(4-Isopropyl-1,3-thiazol-2-yl)ethyl]-2-morpholino-4-oxo-4H-pyrido[1,2-a]pyrimidin-3-yl}-2-propanoic acid

### (A) tert-Butyl 3-{8-[2-(4-isopropyl-1,3-thiazol-2-yl)ethyl]-4-oxo-4H-pyrido[1,2-a]-pyrimidin-3-yl}-2-propanoate

tert-Butyl (E)-3-{8-[2-(4-isoprolyl-1,3-thiazol-2-yl)ethyl]-2-morpholino-4-oxo-4H-pyrido[1,2-a]pyrimidin-3-yl}-2-propenate (29.8 mg, 0.058 mmol) was dissolved in methanol (20 ml), added with 10% palladium/carbon (6.0 mg) and stirred at room temperature under hydrogen flow for 4 hours and 30 minutes. After the catalyst was removed by filtration, the solvent was evaporated and the residue was purified by preparative TLC (chloroform:methanol = 30:1, v/v) to obtain the title compound (8.0 mg, 26.7%) as a pale yellow oily substance.
¹H-NMR (CDCl₃) δ: 1.29 (6H, d, J=6.8Hz), 1.43 (9H, s), 2.62-2.70 (2H, m), 2.87-2.93 (2H, m), 3.02-3.12 (1H, m), 3.16-3.23 (2H, m), 3.32-3.47 (6H, m), 3.80-3.86 (4H, m), 6.73 (1H, s), 6.82 (1H, dd, J=7.3, 1.7Hz), 7.22 (1H, s), 8.81 (1H, d, J=7.3Hz)

### (B) 3-{8-[2-(4-Isopropyl-1,3-thiazol-2-yl)ethyl]-2-morpholino-4-oxo-4H-pyrido[1,2-a]-pyrimidin-3-yl}-2-propanoic acid

The tert-Butyl 3-{8-[2-(4-isopropyl-1,3-thiazol-2-yl)ethyl]-4-oxo-4H-pyrido[1,2-a]pyrimidin-3-yl}-2-propanoate (8.0 mg, 0.016 mmol) obtained in (A) was added with 4 N hydrochloric acid in dioxane (1 ml), stirred at room temperature for 3 hours, further added with 4 N hydrochloric acid in dioxane (1 ml), and stirred for further 4 hours. The solvent was evaporated, and the residue was purified by preparative TLC (chloroform:methanol = 10:1, v/v) and lyophilized from dioxane to obtain the title compound (4.3 mg, 60.4%) as pale yellow powder.
¹H-NMR (CDCl₃) δ: 1.29 (6H, d, J=6.9Hz), 2.76 (2H, t, J=6.9Hz), 2.92 (2H, t, J=6.9Hz), 3.02-3.12 (1H, m), 3.18-3.25 (2H, m), 3.3.4-3.56 (6H, m), 3.78-3.86 (4H, m), 6.73 (1H, s), 6.88 (1H, dd, J=7.3, 1.7Hz), 7.26 (1H, s), 8.82 (1H, d, J=7.1Hz)
LC/MS; m/z: 457 (MH⁺), 455 (M⁺-1)
EI/MS; m/z: 456 (MH⁺)
H-R EI/MS: Calcd. for C₂₃H₂₈N₄O₄S: 456.1831, Found: 456.1848

### Example 53: (E)-3-{2-(4,4-Dimethylhexahydropyrazin-4-ium-1-yl)-8-[2-(4-isopropyl-1,3-thiazol-2-yl)ethyl]-4-oxo-4H-pyrido[1,2-a]pyrimidin-3-yl}-2-propenoic acid

(E)-3-{8-[2-(4-Isopropyl-1,3-thiazol-2-yl)ethyl]-2-(4-methyl)piperazino-4-oxo-4H-pyrido[1,2-a]pyrimidin-3-yl}-2-propenoic acid (19.9 mg, 0.043 mmol) was dissolved in dimethylformamide (1 ml), added dropwise with methyl iodide (0.1 ml, 1.61 mmol), sealed with a stopper and left in a refrigerator for 14 hours. The solvent and excessive reagents were evaporated, and the residue was lyophilized from dioxane/water to obtain the title compound (29.7 mg, quantitative).
¹H-NMR (DMSO-d₆) δ : 1.21 (6H, d, J=6.9Hz), 2.55 (6H, s), 2.91-3.02 (1H, m), 3.15-3.26 (2H, m), 3.45-3.55 (4H, m), 3.82-3.92 (4H, m), 6.93 (1H, d, J=15.5Hz), 7.09 (1H, s), 7.29 (1H, d, J=7.1Hz), 7.41 (1H, s), 7.45 (1H, d, J=15.5Hz), 8.14 (1H, br), 8.85 (1H, d, J=7.3Hz)
FAB/MS; m/z: 482 (M⁺)
H-R FAB/MS: Calcd. for C₂₅H₃₂N₅O₃S: 482.2226, Found: 482.2216

### Example 54: (E)-3-{2-{(3R)-3-[(Aminocarbonyl)oxy]piperidino}-8-[2-(4-isopropyl-1,3-thiazol-2-yl)ethyl]-4-oxo-4H-pyrido[1,2-a]pyrimidin-3-yl}-2-propenoic acid

### (A) tert-Butyl (E)-3-{2-[(3R)-3-hydroxyhexahydro-1-pyridinyl]-8-[2-(4-isopropyl-1,3-thiazol-2-yl)ethyl]-4-oxo-4H-pyrido[1,2-a]pyrimidin-3-yl}-2-propenate

tert-Butyl (E)-3-{8-[2-(4-isopropyl-1,3-thiazol-2-yl)ethyl]-2-[(4-methylphenyl)-sulfonyl]oxy-4-oxo-4H-pyrido[1,2-a]pyrimidin-3-yl}-2-propenate (111.4 mg, 0.19 mmol) was dissolved in dimethylformamide (3 ml), added dropwise with triethylamine (130.4 µl, 0.93 mmol), added with R-(+)-3-hydroxypiperidine hydrochloride (128.7 mg, 0.93 mmol), and stirred at room temperature for 17 hours. The solvent was evaporated, and then the residue was purified by preparative TLC (chloroform:methanol = 30:1, v/v) to obtain the title compound (91.4 mg, 93.2%) as yellow oil.
¹H-NMR (CDCl₃) δ: 1.29 (6H, d, J=6.8Hz), 1.51 (9H, s), 1.72-1.95 (4H, m), 3.01-3.11 (1H, m), 3.16-3.23 (2H, m), 3.32-3.40 (2H, m), 3.49-3.68 (3H, m), 3.88-3.97 (1H, m), 3.97-4.07 (1H, m), 6.74 (1H, s), 6.86 (1H, d, J=7.3Hz), 7.03 (1H, d, J=15.6Hz), 7.19 (1H, s), 7.49 (1H, d, J=15.6Hz), 8.85 (1H, d, J=7.3Hz)

### (B) tert-Butyl (E)-3-{2-(3R)-3-[(aminocarbonyl)oxy]hexahydro-1-pyridinyl-8-[2-(4-isopropyl-1,3-thiazol-2-yl)ethyl]-4-oxo-4H-pyrido[1,2-a]pyrimidin-3-yl}-2-propenate

The tert-Butyl (E)-3-{2-[(3R)-3-hydroxyhexahydro-1-pyridinyl]-8-[2-(4-isopropyl-1,3-thiazol-2-yl)ethyl]-4-oxo-4H-pyrido[1,2-a]pyrimidin-3-yl}-2-propenate (38.9 mg, 0.074 mmol) obtained in (A) was dissolved in ethyl acetate (3 ml), added with trichloroacetyl isocyanate (9.7 µl, 0.082 mmol) under ice cooling, and stirred at the same temperature for 1 hour. The reaction solution was further added with trichloroacetyl isocyanate (9.7 µ l, 0.082 mmol), stirred at the same temperature for further 1 hour, and added with chloroform/methanol (10:1, v/v, 6 ml), and the solvent was evaporated. The residue was dissolved in methanol (1.5 ml), added with water (0.2 ml) and sodium formate (9.6 mg, 0.14 mmol) stirred at room temperature for 2 hours and 30 minutes. The mixture was further added with sodium formate (9.6 mg, 0.14 mmol), and stirred at room temperature for further 20 hours. The solvent was evaporated, and the residue was purified by preparative TLC (chloroform:methanol =30:1, v/v) to obtain the title compound (74.9 mg, quantitative) as yellow solid.
¹H-NMR (CDCl₃) δ: 1.29 (6H, d, J=6.9Hz), 1.50 (9H, s), 1.80-2.10 (4H, m), 3.02-3.12 (1H, m), 3.15-3.23 (2H, m), 3.23-3.40 (3H, m), 3.45-3.63 (1H, m), 3.65-3.77 (2H, m), 4.75-4.85 (1H, m), 6.73 (1H, s), 6.85 (1H, dd, J=7.3, 1.7Hz), 7.08 (1H, d, J=15.7Hz), 7.22 (1H, s), 7.73 (1H, d, J=15.7Hz), 8.86 (1H, d, J=7.1Hz)

### (C) (E)-3-{2-{(3R)-3-[(Aminocarbonyl)oxy]hexahydro-1-pyridinyl}-8-[2-(4-isopropyl-1,3-thiazol-2-yl)ethyl]-4-oxo-4H-pyrido[1,2-a]pyrimidin-3-yl}-2-propenic acid

The tert-Butyl (E)-3-{2-(3R)-3-[(aminocarbonyl)oxy]hexahydro-1-pyridinyl-8-[2-(4-isopropyl-1,3-thiazol-2-yl)ethyl]-4-oxo-4H-pyrido[1,2-a]pyrimidin-3-yl}-2-propenate (74.9 mg, 0.074 mmol) obtained in (B) was dissolved in 4 N hydrochloric acid solution in dioxane and stirred at room temperature for 5 hours. After the solvent was evaporated, the residue was purified by preparative TLC (chloroform:methanol =10:1, v/v) and lyophilized from dioxane to obtain the title compound (17.6 mg, 67.5%) as yellow powder.
¹H-NMR (DMSO-d₆) δ : 1.20 (6H, d, J=6.9Hz), 1.55-1.70 (2H, m), 1.80-1.90 (1H, m), 1.90-2.02 (1H, m), 2.90-3.01 (1H, m), 3.13-3.20 (2H, m), 3.30-3.42 (3H, m), 3.45-3.60 (2H, m), 3.80-3.88 (1H, m), 4.54-4.62 (1H, m), 6.49 (2H, br), 6.87 (1H, d, J=15.4Hz), 7.07 (1H, s,), 7.17 (1H, dd, J=7.3, 1.7Hz), 7.32 (1H, s), 7.44 (1H, d, J=15.4Hz), 8.76 (1H, d, J=7.3Hz), 11.89 (1H, br)
FAB/MS; m/z: 512 (MH⁺)
H-R FAB/MS: Calcd. for C₂₅H₂₉N₅O₅S: 512.1968, Found: 512.1970

### Example 55: (E)-3-{2-{(3S)-3-[(Aminocarbonyl)oxy]piperidino}-8-[2-(4-isopropyl-1,3-thiazol-2-yl)ethyl]-4-oxo-4H-pyrido[1,2-a]pyrimidin-3-yl}-2-propenoic acid

The title compound was synthesized in the same manner as in Example 54.
¹H-NMR (DMSO-d₆) δ: 1.20 (6H, d, J=6.9Hz), 1.53-1.70 (2H, m), 1.80-1.90 (1H, m), 1.90-2.02 (1H, m), 2.91-3.01 (1H, m), 3.13-3.20 (2H, m), 3.30-3.42 (3H, m), 3.45-3.62 (2H, m), 3.80-3.88 (1H, m), 4.53-4.63 (1H, m), 6.49 (2H, br), 6.87 (1H, d, J=15.4Hz), 7.07 (1H, s,), 7.17 (1H, dd, J=7.3, 1.7Hz), 7.32 (1H, s), 7.44 (1H, d, J=15.4Hz), 8.76 (1H, d, J=7.3Hz), 11.89 (1H, br)
FAB/MS; m/z: 512 (MH⁺)
H-R FAB/MS: Calcd. for C₂₅H₂₉N₅O₅S: 512.1968, Found: 512.1968

### Example 56: (E)-3-{2-{4-[(Aminocarbonyl)oxy]hexahydro-1-pyridinyl}-8-[2-(4-isopropyl-1,3-thiazol-2-yl)ethyl]-4-oxo-4H-pyrido[1,2-a]pyrimidin-3-yl}-2-propenoic acid

The title compound was synthesized in the same manner as in Example 54.
¹H-NMR (DMSO-d₆) δ : 1.20 (6H, d, J=6.9Hz), 1.57-1.70 (2H, m), 1.91-2.01 (2H, m), 2.91-3.01 (1H, m), 3.13-3.20 (2H, m), 3.27-3.40 (4H, m), 3.72-3.82 (2H, m), 4.69-4.78 (1H, m), 6.50 (2H, br), 6.86 (1H, d, J=15.4Hz), 7.07 (1H, s), 7.18 (1H, dd, J=7.3, 2.0Hz), 7.32 (1H, s), 7.44 (1H, d, J=15.4Hz), 8.77 (1H.d, J=7.3Hz), 11.90 (1H, br)
FAB/MS; m/z: 512 (MH⁺)
H-R FAB/MS: Calcd. for C₂₅H₂₉N₅O₅S: 512.1968, Found: 512.1964

### Example 57:8-[2-(4-Isopropyl-1,3-thiazol-2-yl)ethyl]-2-morpholino-3-[2-(2H-1,2,3,4-tetrazol-5-yl)acetyl]-4H-pyrido[1,2-a]pyrimidin-4-one

### (A) Ethyl 2-[2-(4-methoxybenzyl)-2H-1,2,3,4-tetrazol-5-yl]acetate

Ethyl 2-(1H-1,2,3,4-tetrazol-5-yl)acetate (5.0 g, 32.0 mmol) was dissolved in dimethylformamide (20 ml), added with potassium carbonate (5.75 g, 41.6 mmol). The mixture was further added dropwise with 4-methoxybenzyl chloride (5.21 ml, 38.4 mmol) under ice cooling and stirred at the same temperature for 1 hour and 30 minutes and at room temperature for 15 hours. The solvent was evaporated, and the residue was diluted with toluene, washed with water and saturated brine, and dried over anhydrous sodium sulfate. Then, the solvent was evaporated and the residue was purified by silica gel column chromatography (hexane:ethyl acetate = 2:1, v/v) to obtain the title compound (3.78 g, 42.7%) as colorless oil.
¹H-NMR(CDCl₃) δ : 1.25 (3H, t, J=7.1Hz), 3.79 (3H, s), 3.94 (2H, s), 4.19 (2H,q, J=7.1H_{z}), 5.68 (2H, s), 6.82-6.92 (2H, m), 7.28-7.38 (2H, m)

### (B) 2-[2-(4-Methoxybenzyl)-2H-1,2,3,4-tetrazol-5-yl]acetic acid

The ethyl 2-[2-(4-methoxybenzyl)-2H-1,2,3,4-tetrazol-5-yl]acetate (2.15 g, 7.78 mmol) obtained in (A) was dissolved in tetrahydrofuran/methanol (3:1, v/v, 60 ml), added dropwise with a solution of lithium hydroxide (359.2 mg, 8.56 mmol) in water (15 ml) under ice cooling and then stirred at room temperature for 2 hours. The solvent was evaporated, and the residue was added with 1 N aqueous hydrochloric acid to obtain pH of about 1. The solution was extracted with ethyl acetate, and the organic layer was washed with saturated brine and dried over anhydrous sodium sulfate. Then, the solvent was evaporated to obtain the title compound (1.91 g, 98.8%).
¹H-NMR (CDCl₃) δ : 3.79 (3H, s), 3.99 (2H, s), 5.68 (2H, s), 6.85-6.95 (2H, m), 7.29-7.39 (2H, m)

### (C) 2-[2-(4-Methoxybenzyl)-2H-1,2,3,4-tetrazol-5-yl]ethanoyl chloride

The 2-[2-(4-methoxybenzyl)-2H-1,2,3,4-tetrazol-5-yl]acetic acid (262.0 mg, 1.05 mmol) obtained in (B) was added dropwise with thionyl chloride (615.9 µl, 8.44 mmol) under ice cooling and stirred at room temperature for 30 minutes. Excessive regents were evaporated to obtain the title compound (0.27 g, quantitative) as pale yellow oil.

### (D) 8-[2-(4-Isopropyl-1,3-thiazol-2-yl)ethyl]-3-{2-[2-(4-methoxybenzyl)-2H-1,2,3,4-tetrazol-5-yl]acetyl}-2-morpholino-4H-pyrido[1,2-a]pyrimidin-4-one

The 2-[2-(4-methoxybenzyl)-2H-1,2,3,4-tetrazol-5-yl]ethanoyl chloride (0.27 g, 1.05 mmol) obtained in (C) was dissolved in methylene chloride (3 ml), added dropwise with pyridine (170.2 µl, 2.10 mmol) under ice cooling, and added dropwise with a solution of 8-[2-(4-isopropyl-1,3-thiazol-2-yl)ethyl]-2-morpholino-4H-pyrido[1,2-a]pyrimidin-4-one (80.9 mg, 0.21 mmol) in methylene chloride (3 ml). The reaction solution was stirred at room temperature for 23 hours, then further added with a solution of 2-[2-(4-methoxybenzyl)-2H-1,2,3,4-tetrazol-5-yl]ethanoyl chloride (0.27 g, 1.05 mmol) in methylene chloride (2 ml) and pyridine (170.2 µl, 2.10 mmol) under ice cooling. Further, the reaction solution was added with the same amounts of the acid chloride and pyridine twice every 24 hours, and stirred at room temperature. The solvent was evaporated, and the residue was added with saturated aqueous sodium hydrogencarbonate and extracted with ethyl acetate. The organic layer was washed with saturated brine and dried over anhydrous sodium sulfate. The solvent was evaporated and the residue was purified by preparative TLC (hexane:ethyl acetate = 1:1, v/v and chloroform:methanol = 30:1, v/v) to obtain the title compound (10.1 mg, 7.8%) as yellow oil.
¹H-NMR (CDCl₃) δ : 1.29 (6H, d, J=6.8Hz), 3.02-3.12 (1H, m), 3.12-3.20 (2H, m), 3.31-3.38 (2H, m), 3.60-3.75 (8H, m), 3.79 (3H, s), 4.68 (2H, s), 5.66 (2H, s), 6.72 (1H, dd, J=7.3, 1.5Hz), 6.73 (1H, s), 6.86 (2H, d, J=8.6Hz), 7.03 (1H, s), 7.29 (2H, d, J=8.6Hz), 8.71 (1H, d, J=7.3Hz)

### (E) 8-[2-(4-Isopropyl-1,3-thiazol-2-yl)ethyl]-2-morpholino-3-[2-(2H-1,2,3,4-tetrazol-5-yl)acetyl]-4H-pyrido[1,2-a]pyrimidin-4-one

The 8-[2-(4-isopropyl-1,3-thiazol-2-yl)ethyl]-3-{2-[2-(4-methoxybenzyl)-2H-1,2,3,4-tetrazol-5-yl]acetyl}-2-morpholino-4H-pyrido[1,2-a]pyrimidin-4-one (8.9 mg, 0.014 mmol) obtained in (D) was dissolved in trifluoroacetic acid (5 ml)and stirred at room temperature for 18 hours. The solvent was evaporated, and the residue was purified by preparative TLC (chloroform:methanol =10:1, v/v) and lyophilized from dioxane to obtain the title compound (4.8 mg, 67.0%) as pale yellow powder.
¹H-NMR (CDCl₃) δ : 1.29 (6H, d, J=6.9Hz), 3.01-3.12 (1H, m), 3.17-3.23 (2H, m), 3.32-3.40 (2H, m), 3.55-3.63 (4H, m), 3.67-3.76 (4H, m), 4.75 (2H, s), 6.74 (1H, s), 6.80 (1H, d, J=7.3Hz), 7.07 (1H, s), 8.73 (1H, d, J=7.3Hz)
FAB/MS; m/z: 495 (MH⁺)
H-R FAB/MS: Calcd. for C₂₃H₂₆N₈O₃S: 495.1927, Found: 495.1955

### Example 58: (E)-3-(8-({[4-(tert-Butyl)-1,3-thiazol-2-yl]amino}carbonyl)-2-{3-[(dimethylamino)carbonyl]piperidino}-4-oxo-4H-pyrido[1,2-a]pyrimidin-3-yl)-2-propenoic acid

### (A) N⁸-[4-(tert-Butyl)-1,3-thiazol-2-yl]-2-{3-[(dimethylamino)carbonyl]piperidino}-4-oxo-4H-pyrido[1,2-a]pyrimidine-8-carboxamide

N⁸-[4-(tert-butyl)-1,3-thiazol-2-yl]-2,4-dioxo-3,4-dihydro-2H-pyrido[1,2-a]-pyrimidine-8-carboxamide (301.9 mg, 0.88 mmol) was suspended in dimethylformamide (6 ml) and acetonitrile (12 ml), added dropwise with diisopropylethylamine (1.83 ml, 10.5 mmol) and diphenyl chlorophosphate (545.1 µl, 2.63 mmol) at -10°C under an argon flow, and stirred at the same temperature for 5 minutes and at room temperature for 15 minutes. The reaction solution was cooled to -10°C again, added dropwise with a solution of 3-[(dimethylamino)carbonyl]piperidine trifluoroacetic acid salt (1.18 g, 4.38 mmol) in dimethylformamide (5 ml), and stirred at room temperature for 1 hour and at about 80°C for 2 hours. The reaction solution was heated to about 100°C, heated for 30 minutes with stirring, then added with diisopropylethylamine (1.83 ml, 10.5 mmol), and further heated at 100°C for 3 hours and 30 minutes with stirring. After cooling, the solution was added with saturated aqueous sodium hydrogencarbonate and extracted with chloroform. The organic layer was washed with saturated brine and dried over anhydrous sodium sulfate and the solvent was evaporated. The residue was purified by silica gel column chromatography (chloroform → chloroform:methanol = 50:1 → 20:1, v/v) and preparative TLC (chloroform:methanol = 20:1, v/v) to obtain the title compound (85.9 mg, 20.3%) as yellow orange oil.
¹H-NMR (CDCl₃) δ : 1.33 (9H, s), 1.42-1.62 (1H, m), 1.70-2.08 (3H, m), 2.57-3.20 (3H, m), 2.99 (3H, s), 3.12 (3H, s), 4.03-4.90 (2H, m), 5.69 (1H, s), 6.60 (1H, s), 7.36-7.22 (1H, m), 7.89 (1H, s), 8.97 (1H, d, J=7.3Hz)
LC-MS; m/z: 483 (MH⁺)

### (B) N⁸-[4-(tert-Butyl)-1,3-thiazol-2-yl]-2-{3-[(dimethylamino)carbonyl]piperidino}-3-formyl-4-oxo-4H-pyrido[1,2-a]pyrimidine-8-carboxamide

Dimethylformamide (2 nil) was added dropwise with phosphorus oxychloride (24.9 µl, 0.27 mmol) under ice cooling, and stirred at room temperature for 30 minutes. The reaction solution was cooled with ice again, added dropwise with a solution of the N⁸-[4-(tert-butyl)-1,3-thiazol-2-yl]-2-{3-[(dimethylamino)carbonyl]-piperidino}-4-oxo-4H-pyrido[1,2-a]pyrimidine-8-carboxamide (85.9 mg, 0.18 mmol) obtained in (A) in dimethylformamide (2 ml) and stirred at the same temperature for 2 hours. The reaction solution was added with saturated aqueous sodium hydrogencarbonate and extracted with chloroform. The organic layer was washed with saturated brine and dried over anhydrous sodium sulfate, and the solvent was evaporated to obtain the title compound (84.2 mg, quantitative) as yellow oil.
¹H-NMR (CDCl₃) δ : 1.34 (9H, s), 1.57-2.10 (4H, m), 2.89 (3H, s), 2.97 (3H, s), 3.00-3.28 (3H, m), 4.10-4.45 (2H, m), 6.59 (1H, s), 7.35-7.45 (1H, m), 7.82 (1H, s), 8.95 (1H, s), 8.86 (1H, d, J=7.3Hz), 10.12 (1H, s)
ESI/MS; m/z: 511 (MH⁺)

### (C) Methyl (E)-3-(8-({[4-(tert-Butyl)-1,3-thiazol-2-yl]amino}carbonyl)-2-{3-[(dimethylamino)carbonyl]piperidino}-4-oxo-4H-pyrido[1,2-a]pyrimidin-3-yl)-2-propenoate

The N⁸-[4-(tert-butyl)-1,3-thiazol-2-yl]-2-{3-[(dimethylamino)carbonyl]-piperidino}-3-formyl-4-oxo-4H-pyrido[1,2-a]pyrimidine-8-carboxamide (84.2 mg, 0.18 mmol) obtained in (B) was dissolved in tetrahydrofuran (10 ml), added with lithium chloride (45.3 mg, 1.07 mmol), and added dropwise with bis(2,2,2-trifluoroethyl)-(methoxycarbonylmethyl)phosphonate (112.9 µl, 0.53 mmol) and 1,8-diazabicyclo[5,4,0]undec-7-ene (73.4 µl, 0.53 mmol). After the reaction solution was stirred at room temperature for 2 hours, the solvent was evaporated, and the residue was purified by preparative TLC (chloroform:methanol = 20:1, v/v) to obtain the title compound (63.7 mg, 68.2%) as a mixture of orange oil and solid.
¹H-NMR (CDCl₃) δ : 1.35 (9H, s), 1.56-1.70 (1H, m), 1.70-2.00 (3H, m), 2.92-3.20 (3H, m), 2.99 (3H, s), 3.19 (3H, s), 3.78 (3H, s), 3.97-4.03 (1H, m), 4.22-4.30 (1H, m), 6.60 (1H, s), 7.10 (1H, d, J=15.6Hz), 7.45 (1H, dd, J=7.3, 1.7Hz), 7.49 (1H, d, J=15.6Hz), 7.85 (1H, d, J=1.7Hz), 8.96 (1H, d, J=7.3Hz)

### (D) (E)-3-(8-({[4-(tert Butyl)-1,3-thiazol-2-yl]amino}carbonyl)-2-{3-[(dimethylamino)-carbonyl]piperidino}-4-oxo-4H-pyrido[1,2-a]pyrimidin-3-yl)-2-propenoic acid

The methyl (E)-3-(8-({[4-(tert-butyl)-1,3-thiazol-2-yl]amino}carbonyl)-2-{3-[(dimethylamino)carbonyl]piperidino}-4-oxo-4H-pyrido[1,2-a]pyrimidin-3-yl)-2-propenoate (63.7 mg, 0.11 mmol) obtained in (C) was dissolved in methanol (10 ml), added dropwise with 1 N aqueous sodium hydroxide (562.0 µ l, 0.56 mmol), and stirred at room temperature for 1 hour. The reaction solution was further added with 1 N aqueous sodium hydroxide (5.62 ml, 5.62 mmol), stirred at room temperature for 2 hours, further added with 1 N aqueous sodium hydroxide (2.81 ml, 2.81 mmol), and stirred at room temperature for 3 hours. The reaction solution was adjusted to about pH 2 with 1 N hydrochloric acid aqueous solution and extracted with chloroform/methanol (10:1, v/v). The organic layer was washed with saturated brine and dried over anhydrous sodium sulfate, and the solvent was evaporated. The residue was purified by preparative TLC (chloroform:methanol = 20:1, v/v) and lyophilized from dioxane to obtain the title compound (37.6 mg, 38.5%, for the three steps) as yellow orange powder.
¹H-NMR (DMSO-d₆) δ : 1.31 (9H, s), 1.60-1.80 (3H, m), 1.85-1.94 (1H, m), 2.84 (3H, s), 2.97-3.05 (1H, m), 3.12 (3H, s), 3.30-3.45 (2H, m), 3.95-4.03 (1H, m), 4.08-4.15 (1H, m), 6.82-6.95 (1H, m), 6.93 (1H, d, J=15.6Hz), 7.44 (1H, d, J=15.6Hz), 7.60-7.67 (1H, m), 8.12-8.18 (1H, m), 8.90 (1H, d, J=7.3Hz)
FAB/MS; m/z: 553 (MH⁺)
H-R FAB/MS: Calcd. for C₂₇H₃₂N₆O₅S: 553.2233, Found: 553.2236

### Example 59: (E)-3-[8-({[4-(tert-Butyl)-1,3-thiazol-2-yl]amino}carbonyl)-4-oxo-2-piperidino-4H-pyrido[1,2-a]pyrimidin-3-yl]-2-propenoic acid

The title compound was synthesized in the same manner as in Example 58.
¹H-NMR (DMSO-d₆) δ : 1.31 (9H, s), 1.62-1.72 (6H, m), 3.53-3.62 (4H, m), 6.82-6.92 (1H, m), 6.93 (1H, d, J=15.6Hz), 7.45 (1H, d, J=15.6Hz), 7.57-7.62 (1H, m), 8.14-8.20 (1H, m), 8.89 (1H, d, J=7.3Hz)
FAB/MS; m/z: 482 (MH⁺)
H-R FAB/MS: Calcd. for C₂₄H₂₇N₅O₄S: 482.1862, Found: 482.1844

### Example 60: (E)-3-(8-({[4-(tert-Butyl)-1,3-thiazol-2-yl]amino}carbonyl)-2-{3-[(methylamino)carbonyl]piperidino}-4-oxo-4H-pyrido[1,2-a]pyrimidin-3-yl)-2-propenoic acid

The title compound was synthesized in the same manner as in Example 58.
¹H-NMR (DMSO-d₆) δ : 1.31 (9H, s), 1.58-1.80 (3H, m), 1.89-1.97 (1H, m), 2.59 (3H, d, J=4.4Hz), 3.18-3.20 (2H, m), 3.30-3.42 (2H, m), 3.90-3.97 (1H, m), 4.11-4.18 (1H, m), 6.79-6.89 (1H, m), 6.93 (1H, d, J=15.6Hz), 7.43 (1H, d, J=15.6Hz), 7.60-7.66 (1H, m), 7.77-7.83 (1H, m), 8.19-8.26 (1H, m), 8.90 (1H, d, J=7.6Hz)
FAB/MS; m/z: 539 (MH⁺)
H-R FAB/MS: Calcd. for C₂₆H₃₀N₆O₅S: 539.2077, Found: 539.2112

### Example 61: (E)-3-{8-[2-(4-Isopropyl-1,3-thiazol-2-yl)ethyl]-2-morpholino-4-oxo-4H-pyrido[1,2-a]pyrimidin-3-yl}-2-propenoic acid

### (A) 2-Hydroxy-8-[2-(4-isopropyl-1,3-thiazol-2-yl)ethyl]-4H-pyrido[1,2-a]pyrimidin-4-one

2-Amino-4-[2-(4-isopropyl-1,3-thiazol-2-yl)ethyl]-2-pyridine (9.8 g) and bis(2,4,6-trichlorophenyl) malonate (18 g) were refluxed by heating in xylene for 30 minutes and left stand for cooling. The reaction mixture was added with ether, and the deposited crystals were collected by filtration, washed with ethyl acetate and dried to obtain the title compound (10.3 g). The reaction solution was combined and the solvent was evaporated. The residue was purified by silica gel column chromatography to further obtain the title compound (1.5 g).
¹H-NMR (CDCl₃): 1.29 (6H, d, J=6.8Hz), 3.06 (1H, m), 3.34 (2H, m), 3.38 (2H, m), 5.34 (1H, s), 6.74 (1H, s), 7.10 (1H, dd, J=7.1,1.7Hz), 7.37 (1H, s), 9.02 (1H, d, J=7.1Hz)

### (B) 8-[2-(4-Isopropyl-1,3-thiazol-2-yl)ethyl]-2-morpholino-4H-pyrido[1,2-a]pyrimidin-4-one

The 2-hydroxy-8-[2-(4-isopropyl-1,3-thiazol-2-yl)ethyl]-4H-pyrido[1,2-a]-pyrimidin-4-one (500 mg) obtained in (A) was dissolved in methylene chloride (20 ml), added with triethylamine (0.26 ml) and p-toluenesulfonyl chloride (360 mg) and stirred for 24 hours under nitrogen atmosphere. The reaction solution was added with morpholine (0.83 ml) and stirred for 12 hours, and the solvent was evaporated. The residue was purified by silica gel column chromatography to obtain the title compound (273 mg).
¹H-NMR (CDCl₃): 1.30 (s, 3H); 1.33 (s, 3H), 3.07 (m, 1H), 3.18 (m, 2H), 3.48 (m, 2H), 3.67 (m, 4H), 3.79 (m, 4H), 5.59 (s, 1H), 6.77 (s, 1H), 6.78 (d, 1H), 7.11 (s, 1H), 8.80 (d, 1H)

### (C) 8-[2-(4-Isopropyl-1,3-thiazol-2-yl)ethyl]-2-morpholino-4-oxo-4H-pyrido[1,2-a]-pyrimidine-3-carbaldehyde

Dimethylformamide (10 ml) was added with phosphorus oxychloride (0.60 ml) under ice cooling, stirred for 30 minutes, then added with the 8-[2-(4-isopropyl-1,3-thiazol-2-yl)ethyl]-2-morpholino-4H-pyrido[1,2-a]pyrimidin-4-one (1.0 g) obtained in (B), stirred for 1 hour, and added with saturated aqueous sodium hydrogencarbonate to adjust the reaction solution to pH of about 8. The reaction solution was extracted with ethyl acetate, and the organic layer was washed with saturated brine and dried over sodium sulfate. The solvent was evaporated under reduced pressure to obtain the title compound (1.07 g).
¹H-NMR (CDCl₃): 1.31 (s, 3H), 1.33 (s, 3H), 3.10 (m, 1H), 3.22 (m, 2H), 3.42 (m, 2H), 3.73 (m, 4H), 3.81 (m, 4H), 6.80 (s, 1H), 6.82 (d, 1H), 7.09 (s, 1H), 8.75 (d, 1H), 10.11 (s, 1H)

### (D) tert-Butyl (E)-3-(8-[2-(4-isopropyl-1,3-thiazol-2-yl)ethyl]-2-morpholino-4-oxo-4H-pyrido[1,2-a]pyrimidin-3-yl)-2-propenoate

The 8-[2-(4-isopropyl-1,3-thiazol-2-yl)ethyl]-2-morpholino-4-oxo-4H-pyrido[1,2-a]pyrimidine-3-carbaldehyde (110 mg) obtained in (C) and (tert-butoxy-carbonylmethylene)triphenylphosphorane (441 mg) were stirred in tetrahydrofuran (5 ml) at 80°C for 15 hours. After the solvent was evaporated, the residue was purified by silica gel column chromatography to obtain the title compound (150 mg) as yellow powder.
¹H-NMR (CDCl₃): 1.29 (6H, d, J=6.8Hz), 1.51 (9H, s), 3.05 (1H, m), 3.20 (2H, m), 3.37 (2H, m), 3.60 (4H, m), 3.83 (4H, m), 6.73 (1H, s), 6.85 (1H, d, J=6.8Hz), 7.05 (1H, d, J=15.4Hz), 7.20 (1H, s), 7.50 (1H, d, J=15.4Hz), 8.87 (1H, d, J=7.1Hz)

### (E) (E)-3-{8-[2-(4-Isopropyl-1,3-thiazol-2-yl)ethyl]-2-morpholino-4-oxo-4H-pyrido[1,2-a]pyrimidin-3-yl}-2-gropenoic acid

The tert-butyl (E)-3-{8-[2-(4-isopropyl-1,3-thiazol-2-yl)ethyl]-2-morpholino-4-oxo-4H-pyrido[1,2-a]pyrimidin-3-yl}-2-propenoate (39 mg) obtained in (D) was stirred in formic acid (1 ml) for 4.5 hours, and the solvent was evaporated under reduced pressure to obtain the title compound (30 mg).
¹H-NMR (CD₃OD): 1.26 (6H, d, J=6.8Hz), 3.03 (1H, m), 3.20 (2H, m), 3.31 (2H, m), 3.56 (4H, m), 3.79 (4H, m), 6.94 (1H, d, J=15.6Hz), 6.96 (1H, s), 7.05 (1H, d, J=6.6Hz), 7.21 (1H, s), 7.55 (1H, d, J=15.6Hz), 8.76 (1H, d, J=7.1Hz)

The compounds of Examples 62 to 77 mentioned below were synthesized in the same manner as in Example 61.

### Example 62: (E)-3-(2-Morpholino-4-oxo-8-{2-[4-(trifluoromethyl)-1,3-thiazol-2-yl]-ethyl}-4H-pyrido[1,2-a]pyrimidin-3-yl)-2-propenoic acid

¹H-NMR (CD₃OD) δ : 3.26 (m, 2H), 3.50 (m, 2H), 3.61 (m, 4H), 3.81 (m, 4H), 6.68 (d, J=15.6Hz, 1H), 7.12 (d, 1H), 7.32 (s, 1H), 7.62 (d, J=16Hz, 1H), 8.03 (s, 1H), 8.84 (d, 1H)
MS (ES+) m/z 481 (M⁺+1)

### Example 63: (E)-3-{8-[2-(4-tert-Butyl-1,3-thiazol-2-yl)ethyl]-2-morpholino-4-oxo-4H-pyrido[1,2-a]pyrimidin-3-yl}-2-propenoic acid

¹H-NMR (CDCl₃) δ : 1.31 (s, 9H), 3.19 (t, 2H), 3.35 (t, 2H), 3.59 (m, 4H), 3.79 (m, 4H), 6.72 (s, 1H), 6.85 (d, 1H), 7.07 (d, J=16Hz, 1H), 7.18 (s, 1H), 7.62 (d, J=16Hz, 1H), 8.84 (d, 1H)
MS (ES+) m/z 469 (M⁺+1); MS (ES-) m/z 467 (M⁺-1)

### Example 64: (E)-3-{8-(2-[4-(1-Methylcyclopropyl)-1,3-thiazol-2-yl]ethyl)-2-morpholino-4-oxo-4H-pyrido[1,2-a]pyrimidin-3-yl}-2-propenoic acid

¹H-NMR (CD₃OD) δ : 0.70 (m, 2H), 1.03 (m, 2H), 1.41 (s, 3H), 3.18 (t, 2H), 3.37 (t, 2H), 3.59 (m, 4H), 3.80 (m, 4H), 6.92 (s, 1H), 6.98 (d, J=16Hz, 1H), 7.08 (s, 1H), 7.25 (s, 1H), 7.60 (d, J=16Hz, 1H), 8.82 (d, 1H)
MS (ES+) m/z 467 (M⁺+1); MS (ES-) m/z 465 (M⁺-1)

### Example 65: (E)-3-{8-[2-(4-Isopropyl-1,3-thiazol-2-yl)ethyl]-2-(3-carboxypiperidino)-4-oxo-4H-pyrido[1,2-a]pyrimidin-3-yl}-2-propenoic acid

¹H-NMR (CDCl₃) δ : 1.26 (d, 6H), 2.38 (m, 1H), 2.55 (m, 1H), 2.67 (m, 1H), 2.82 (m, 1H), 3.08 (m, 1H), 3.12 (m, 2H), 3.39 (m, 3H), 3.56 (m, 2H), 3.82 (m, 2H), 3.92 (m, 1H), 6.75 (s, 1H), 6.84 (d, 1H), 7.07 (d, J=14Hz, 1H), 7.21 (s, 1H), 7.33 (s, 1H), 7.20 (s, 1H), 7.65 (m, 2H), 8.82 (d, 1H)
MS (ES+) m/z 497 (M⁺+1); MS (ES-) m/z 495 (M⁺-1)

### Example 66: (E)-3-{8-[2-(4-Isopropyl-1,3-thiazol-2-yl)ethyl]-2-(4-carboxypiperidino)-4-oxo-4H-pyrido[1,2-a]pyrimidin-3-yl}-2-propenoic acid

¹H-NMR (CDCl₃) δ : 1.29 (d, 6H), 2.00 (m, 2H), 2.13 (m, 2H), 2.62 (m, 1H), 3.10 (m, 3H), 3.20 (m, 2H), 3.39 (m, 2H), 4.08 (m, 2H), 6.75 (s, 1H), 6.83 (d, 1H), 7.08 (d, J=15.6Hz, 1H), 7.22 (s, 1H), 7.69 (d, J=15.6Hz, 1H), 8.86 (d, 1H)
MS (ES+) m/z 497 (M⁺+1); MS (ES-) m/z 495 (M⁺-1)

### Example 67: (E)-3-{8-[2-(4-Isopropyl-1,3-thiazol-2-yl)ethyl]-4-oxo-2-(1,2,3,4-tetrahydro-2-isoquinolinyl)-4H-pyrido[1,2-a]pyrimidin-3-yl}-2-propenoic acid

¹H-NMR (CDCl₃) δ : 1.33 (d, 6H), 2.95 (m, 1H), 3.12 (m, 2H), 3.23 (m, 2H), 3.56 (t, 2H), 3.88 (t, 2H), 4.81 (s, 2H), 6.88 (m, 2H), 7.11 (d, J=15Hz, 1H), 7.19 (m, 6H), 7.79 (d, J=15Hz, 1H), 8.86 (d, 1H)
MS (ES+) m/z 501 (M⁺+1)

### Example 68: (E)-3-{8-[2-(4-Isopropyl-1,3-thiazol-2-yl)ethyl]-4-oxo-2-(3-hydroxy-3-methylpiperidino)-4H-pyrido[1,2-a]pyrimidin-3-yl}-2-propenoic acid

¹H-NMR (CD₃OD) δ : 1.12 (s, 3H), 1.23 (d, 6H), 1.65 (m, 2H), 1.97 (m, 1H), 3.08 (m, 1H), 3.2 (m, 3H), 3.39 (m, 4H), 3.68 (m, 2H), 6.95 (d, J=14Hz, 1H), 6.98 (s, 1H), 7.02 (d, 1H), 7.21 (s, 1H), 7.62 (d, J=14Hz, 1H), 8.78 (d, 1H)
MS (ES+) m/z 483 (M⁺+1); MS (ES-) m/z 481 (M⁺-1)

### Example 69: (E)-3-{8-[2-(4-Isopropyl-1,3-thiazol-2-yl)ethyl]-4-oxo-2-(3-cyano-piperidino)-4H-pyrido[1,2-a]pyrimidin-3-yl}-2-propenoic acid

¹H-NMR (CDCl₃) δ : 1.30 (d, 6H), 1.65 (m, 1H), 1.90 (m, 2H), 2.00 (m, 1H), 2.98 (m, 1H), 3.08 (m, 1H), 3.22 (m, 2H), 3.40 (m, 2H), 3.52 (m, 1H), 3.65 (m, 1H), 3.83 (m, 1H), 4.18 (m, 1H), 6.75 (s, 1H), 6.92 (d, 1H), 7.11 (d, J=15.6Hz, 1H), 7.25 (s, with CDCl₃, 1H), 7.63 (d, J=15.6Hz, 1H), 8.88 (d, 1H)
MS (ES+) m/z 478 (M⁺+1); MS (ES-) m/z 476 (M⁺-1)

### Example 70: (E)-3-{8-[2-(4-Isopropyl-1,3-thiazol-2-yl)ethyl]-4-oxo-2-(4-cyano-piperidino)-4H-pyrido[1,2-a]pyrimidin-3-yl}-2-propenoic acid

¹H-NMR (CDCl₃) δ : 1.33 (d, 6H), 2.04 (m, 4H), 2.95 (m, 1H), 3.21 (m, 3H), 3.55 (m, 4H), 3.79 (m, 2H), 6.92 (m, 2H), 7.08 (d, J=15.6Hz, 1H), 7.26 (1Hs, with CHCl₃, 1H), 7.62 (d, J=15.6Hz, 1H), 8.89 (d, 1H)
MS (ES+) m/z 478 (M⁺+1)

### Example 71: (E)-3-{8-[2-(4-Isopropyl-1,3-thiazol-2-yl)ethyl]-4-oxo-2-(3-cyano-morpholino)-4H-pyrido[1,2-a]pyrimidin-3-yl}-2-propenoic acid

¹H-NMR (CDCl₃) δ : 1.29 (d, 6H), 3.05 (m, 1H), 3.22 (t, 2H), 3.40 (t, 2H), 3.46 (m, 1H), 3.7-4.0 (m, 4H), 4.08 (m, 1H), 4.72 (m, 1H), 6.74 (s, 1H), 6.97 (d, 1H), 7.14 (d, J=15.6Hz, 1H), 7.28 (s, 1H), 7.67 (d, J=15.6Hz, 1H), 8.90 (d, 1H)
MS (ES+) m/z 480 (M⁺+1); MS (ES-) m/z 478 (M⁺-1)

### Example 72: (E)-3-{8-[2-(4-Isopropyl-1,3-thiazol-2-yl)ethyl]-2-(3-aminocarbonylpiperazino)-4-oxo-4H-pyrido[1,2-a]pyrimidin-3-yl}-2-propenoic acid

¹H-NMR (CD₃OD) δ : 1.25 (d, 6H), 3.02 (m, 1H), 3.2-3.4 (m, with CHD₂OD) 4.24 (m, 1H), 6.96 (s, 1H), 7.04 (d, J=16Hz, 1H), 7.14 (m, 1H), 7.37 (s, 1H), 7.58 (d, J=16Hz, 1H), 8.84 (d, 1H)
MS (ES+) m/z 497 (M⁺+1)

### Example 73: (E)-3-{8-[2-(4-Isopropyl-1,3-thiazol-2-yl)ethyl]-2-(3-carboxypiperazino)-4-oxo-4H-pyrido[1,2-a]pyrimidin-3-yl}-2-propenoic acid

¹H-NMR (CD₃OD) δ : 1.23 (d, 6H), 3.02 (m, 1H), 3.15-3.42 (m, with CHD₂OD), 3.62 (m, 1H), 3.95 (m, 1H), 4.75 (m, 1H), 6.9.6 (s, 1H), 7.05 (m, 2H), 7.37 (m, 2H), 8.82 (d, 1H) MS (ES+) m/z 520 (M⁺+Na); MS (ES-) m/z 496 (M⁺-1)

### Example 74: (E)-3-{8-[2-(4-Isopropyl-1,3-thiazol-2-yl)ethyl]-2-(3-cyanopiperazino)-4-oxo-4H-pyrido[1,2-a]pyrimidin-3-yl}-2-propenoic acid

¹H-NMR (300MHz, CD₃OD) δ : 1.23 (d, 6H), 3.02 (m, 1H), 3.15-3.42 (m, with CHD₂OD), 3.71 (m, 1H), 3.92 (m, 1H), 4.12 (m, 1H), 6.81 (s, 1H), 7.00 (m, 2H), 7.25 (m, 1H), 7.52 (d, J=16Hz, 1H), 8.82 (d, 1H)

### Example 75: (E)-3-{8-[2-(4-Isopropyl-1,3-thiazol-2-yl)ethyl]-2-(3-carboxymorpholino)-4-oxo-4H-pyrido[1,2-a]pyrimidin-3-yl}-2-propenoic acid

¹H-NMR (CDCl₃ + CD₃OD) δ : 1.22 (d, 6H), 3.0 (m, 1H), 3.14 (t, 2H), 3.31 (m, 4H), 3.73 (m, 2H), 4.02 (m, 1H), 4.12 (m, 2H), 6.70 (s, 1H), 6.88 (d, 1H), 6.97 (d, J=15.6Hz, 1H), 7.21 (s, 1H), 7.53 (d, J=15.6Hz, 1H), 8.78 (d, 1H).
MS (ES+) m/z 499 (M⁺+1)

### Example 76: (E)-3-{8-[2-(4-Isopropyl-1,3-thiazol-2-yl)ethyl]-2-(3-aminocarbonylmorpholino)-4-oxo-4H-pyrido[1,2-a]pyrimidin-3-yl}-2-propenoic acid

¹H-NMR (CDCl₃) δ : 1.30 (d, 6H), 3.0-3.2 (m, 5H), 3.47 (m, 2H), 3.81 (m, 2H), 4.02 (m, 1H), 4.21 (m, 1H), 4.42 (m, 1H), 6.82 (s, 1H), 6.92 (d, 1H), 7.08 (d, J=15.6Hz, 1H), 7.26 (s, with CHCl₃, 1H), 7.64 (d, J=15.6Hz, 1H), 8.87 (d, 1H)
MS (ES+) m/z 498 (M⁺+1); MS (ES-) m/z 496 (M⁺- 1)

### Example 77: (E)-3-{8-[2-(4-Isopropyl-1,3-thiazol-2-yl)ethyl-4-oxo-2-([(2S,3R,4R,5S,6S)-3,4,5,6-tetrahydroxytetrahydro-2H-2-pyranyl]methylamino)-4H-pyrido[1,2-a]-pyrimidin-3-yl]-2-propenoic acid

¹H-NMR (CD₃OD) δ: 1.23 (d, 6H), 3.02 (m, 1H), 3.18 (m, 2H), 3.4 (m, 3H), 3.75 (m, 1H), 3.98 (m, 1H), 4.5 & 5.12 (2 doublets, mixture of anomers, 1H), 6.98 (s, 1H), 7.02 (m, 2H), 7.18 (s, 1H), 7.73 (d, J=16Hz, 1H), 8.75 (d, 1H)
MS (ES+) m/z 547 (M⁺+1); MS (ES-) m/z 545 (M⁺-1)

### Example 78: (E)-3-{2-[4-((2S)-2-Amino-5-{[amino(imino)methyl]amino}pentanoyl)-piperazino]-8-[(E)-2-(4-isopropyl-1,3-thiazol-2-yl)-1-ethenyl]-4-oxo-4H-pyrido[1,2-a]-pyrimidin-3-yl}-2-propenoic acid

tert-Butyl (E)-3-{8-[(E)-2-(4-isopropyl-1,3-thiazol-2-yl)-1-ethenyl]-4-oxo-2-piperazino-4H-pyrido[1,2-a]pyrimidin-3-yl}-2-propenoate (10 mg, 0.0197 mmol) and N-α , ω-1, ω-2-tri-tert-butoxycarbonyl-L-arginine (BOC-Arg, (BOC)₂OH, 14 mg, 0.0295 mmol) were added with methylene chloride (1 ml). The mixture was further added with 1-ethyl-3-(3-dimethylaminopropyl)carbodiimide hydrochloride (EDC HCl, 11 mg, 0.0591 mmol) and stirred at room temperature overnight. The reaction solution was diluted with methylene chloride, washed with 5% aqueous citric acid, saturated aqueous sodium hydrogencarbonate and saturated brine, and dried over sodium sulfate. The solvent was evaporated under reduced pressure, and then the residue was added with trifluoroacetic acid (1 ml) and stirred in the dark for 1 hour. The reaction solution was added with toluene, and the solvent was evaporated under reduced pressure. The residue was purified by medium pressure column chromatography (Amberkron column, gradient 100% 0.1% TFA in H₂O to 100% CH₃CN over 80 minutes, 3 ml/minute) to obtain the title compound (7.0 mg).
¹H-NMR (CD₃OD): 1.34 (d, 6H), 1.70 (m, 2H), 1.90 (m, 2H), 3.10 (m, 1H), 3.70 (m, 8H), 3.92 (m, 1H), 4.54 (t, 1H), 7.08 (d, 1H), 7.28 (s, 1H), 7.52 (d, 1H), 7.55 (m, 2H), 7.66 (d, 1H), 7.72 (d, 1H), 8.93 (d, 1H)
MS (ES+): 631 (M+Na)

The compounds of Examples 79 to 91 mentioned below were synthesized in the same manner as in Example 78.

### Example 79: (E)-3-{2-[4-((2S)-2-Amino-5-{[amino(imino)methyl]amino}pentanoyl)-piperazino]-8-[(E)-2-(4-isopropyl-1,3-thiazol-2-yl)ethyl]-4-oxo-4H-pyrido[1,2-a]-pyrimidin-3-yl}-2-propenoic acid

¹H-NMR (CD₃OD): 1.26 (d, 6H), 1.70 (m, 2H), 1.90 (m, 2H), 3.03 (m, 1H), 3.20-3.42 (m, 7H), 3.50-3.80 (m, 8H), 3.90 (m, 1H), 4.53 (t, 1H), 6.98 (s, 1H), 7.05 (d, 1H), 7.15 (d, 1H), 7.30 (s, 1H), 7.64 (d, 1H), 8.87 (d, 1H)
MS (ES+): 610

### Example 80: (E)-3-{2-[4-((2S)-2-Amino-5-{[amino(imino)methyl]amino}pentanoyl)-piperazino]-8-[(E)-2-(4-tert-butyl-1,3-thiazol-2-yl)ethyl]-4-oxo-4H-pyrido[1,2-a]-pyrimidin-3-yl}-2-propenoic acid

¹H-NMR (CD₃OD): 1.29 (s, 9H), 1.69 (m, 2H), 1.88 (m, 2H), 3.10-3.50 (m, 5H), 3.50-3.80 (m, 10H), 3.90 (m, 2H), 4.53 (m, 1H), 6.96 (s, 1H), 7.05 (d, 1H), 7.16 (d, 1H), 7.30 (s, 1H), 7.65 (d, 1H), 8.88 (d, 1H)
MS (ES+): 624

### Example 81: (E)-3-{8-[2-(4-Isopropyl-1,3-thiazol-2-yl)ethyl]-4-oxo-2-(3-dimethylaminoethylaminocarbonylpiperidino)-4H-pyrido[1,2-a]pyrimidin-3-yl}-2-propenoic acid

¹H-NMR (CD₃OD) δ : 1.26 (d, J=7Hz, 6H), 1.80 (m, 4H), 2.05 (m, 1H), 2.65 (m, 1H), 2.94 (s, 6H), 3.05 (m, 2H), 3.15-3.25 (m, 4H), 3.42 (t, 2H), 3.58 (t, 2H), 4.10 (m, 2H), 6.98 (d, J=15.6.Hz, 1H), 7.03 (s, 1H), 7.06 (dd, 1H), 7.23 (s, 1H), 7.58 (d, J=15.6Hz, 1H), 8.80 (d, 1H)
MS (ES+) m/z 567 (M⁺+1)

### Example 82: (E)-3-{8-[2-(4-Isopropyl-1,3-thiazol-2-yl)ethyl]-4-oxo-2-(4-dimethylaminoethylaminocarbonylpiperidino)-4H-pyrido[1,2-a]pyrimidin-3-yl}-2-propenoic acid

¹H-NMR (CD₃OD) δ : 1.26 (d, J=7Hz, 6H), 1.88 (m, 4H), 2.53 (m, 1H), 2.94 (s, 6H), 3.02 (m, 1H), 3.0-3.25 (m, 6H), 3.41 (t, 2H), 3.55 (t, 2H), 4.15 (m, 2H), 6.97 (m, 2H), 7.04 (dd, 1H), 7.22 (s, 1H), 7.60 (d, J=15.6Hz, 1H), 8.80 (d, 1H)
MS (ES+) m/z 567 (M⁺+1)

### Example 83: (E)-3-{8-[2-(4-Isopropyl-1,3-thiazol-2-yl)ethyl]-4-oxo-2-(4-dimethylaminoacetylpiperazino)-4H-pyrido[1,2-a]pyrimidin-3-yl}-2-propenoic acid

¹H-NMR (CD₃OD) δ : 1.23 (d, 6H), 2.95 (s, 6H), 3.02 (m, 1H), 3.2-3.35 (m, with CHD₂OD), 3.42 (m, 1H), 3.58 (m, 1H), 3.63 (m, 2H), 3.80 (m, 1H), 4.28 (s, 2H), 6.96 (s, 1H), 7.05 (d, J=13Hz, 1H), 7.14 (d, 1H), 7.29 (s, 1H), 7.62 (d, J=13Hz, 1H), 8.85 (d, 1H)

### Example 84: (E)-3-{8-[2-(4-Isopropyl-1,3-thiazol-2-yl)ethyl]-4-oxo-2-(aminoethylthioethylamino)-4H-pyrido[1,2-a]pyrimidin-3-yl}-2-propenoic acid

¹H-NMR (CD₃OD) δ : 1.26 (d, J=7Hz, 6H), 2.85 (m, 4H), 3.02 (m, 1H), 3.2 (m, 4H), 3.40 (t, 2H), 3.78 (t, 2H), 6.99 (m, 2H), 7.11 (d, J=15.6Hz, 1H), 7.15 (s, 1H), 7.73 (d, J=15.6Hz, 1H), 8.78 (d, 1H)
MS (ES+) m/z 488 (M⁺+1)

### Example 85: (E)-3-{8-[2-(4-Isopropyl-1,3-thiazol-2-yl)ethyl]-4-oxo-2-(3-aminopropylaminocarbonylpiperidino)-4H-pyrido[1,2-a]pyrimidin-3-yl}-2-propenoic acid

¹H-NMR (CD₃OD) δ : 1.26 (d, J=7Hz, 6H), 1.84 (m, 3H), 2.06 (m, 1H), 2.68 (m, 1H), 2.93 (m, 2H), 3.02 (m, 2H), 3.2-3.3 (m, withCD₃OD), 3.42 (m, 3H), 4.08 (m, 2H), 6.9-7.02 (m, 2H), 7.07 (dd, 1H), 7.25 (s, 1H), 7.58 (d, J=15.6Hz, 1H), 8.81 (d, 1H)
MS (ES+) m/z 553 (M⁺+1)

### Example 86: (E)-3-{8-[2-(4-Isopropyl-1,3-thiazol-2-yl)ethyl]-4-oxo-2-(3-aminoethylaminocarbonylpiperidino)-4H-pyrido[1,2-a]pyrimidin-3-yl}-2-propenoic acid

¹H-NMR (CD₃OD) δ : 1.26 (d, J=7Hz, 6H), 1.79 (m, 3H), 2.06 (m, 1H), 2.68 (m, 1H), 3.0-3.1 (m, 4H), 3.15-3.28 (m, 3H), 3.45 (m, 4H), 4.1 (m, 2H), 6.97 (d, J=15.6Hz, 1H), 7.06 (m, 2H), 7.25 (s, 1H), 7.58 (d, J=15.6Hz, 1H), 8.79 (d, 1H)
MS (ES+) m/z 539 (M⁺+1)

### Example 87: (E)-3-{8-[2-(4-Isopropyl-1,3-thiazol-2-yl)ethyl]-4-oxo-2-(3-[(tetrahydro-1H-2-pyrrolylmethyl)amino]carbonylpiperidino)-4H-pyrido[1,2-a]pyrimidin-3-yl}-2-propenoic acid

¹H-NMR (CD₃OD) δ : 1.26 (d, J=7Hz, 6H), 1.79 (m, 4H), 2.04 (m, 4H), 2.68 (m, 1H), 2.9-3.1 (m, 2H), 3.15-3.35 (m, with CD₃OD), 3.35-3.55 (m, 4H), 3.68 (m, 1H), 4.1 (m, 2H), 6.99 (m, 2H), 7.08 (d, 1H), 7.25 (s, 1H), 7.59 (d, J=15.6Hz, 1H), 8.81 (d, 1H)
MS (ES+) m/z 579 (M⁺+1)

### Example 88: (E)-3-{8-[2-(4-Isopropyl-1,3-thiazol-2-yl)ethyl]-4-oxo-2-(4-aminomethylcarbonylpiperazino)-4H-pyrido[1,2-a]pyrimidin-3-yl}-2-propenoic acid

¹H-NMR (CD₃OD) δ : 1.26 (d, J=7Hz, 6H), 3.02 (m, 1H), 3.23 (t, 2H), 3.42 (t, 2H), 3.62 (m, 6H), 3.78 (m, 2H), 3.99 (s, 2H), 6.98 (s, 1H), 7.02 (d, J=15.6Hz, 1H), 7.13 (d, 1H), 7.27 (s, 1H), 7.63 (d, J=15.6Hz, 1H), 8.84 (d, 1H)
MS (ES+) m/z 511 (M⁺+1)

### Example 89: (E)-3-{8-[2-(4-Isopropyl-1,3-thiazol-2-yl)ethyl]-4-oxo-2-(4-prolylpiperazino)-4H-pyrido[1,2-a]pyrimidin-3-yl}-2-propenoic acid

¹H-NMR (CD₃OD) δ : 1.26 (d, J=7Hz, 6H), 1.9-2.1 (m, 3H), 2.52 (m, 1H), 3.02 (m, 1H), 3.25 (t, 2H), 3.42 (t, 2H), 3.5-3.9 (m, 10H), 4.68 (m, 1H), 6.97 (s, 1H), 7.03 (d, J=15.6Hz, 1H), 7.14 (d, 1H), 7.28 (s, 1H), 7.63 (d, J=15.6Hz, 1H), 8.83 (d, 1H, d)
MS (ES+) m/z 551 (M⁺+1)

### Example 90: (E)-3-{8-[2-(4-Isopropyl-1,3-thiazol-2-yl)ethyl]-4-oxo-2-(4-lysylpiperazino)-4H-pyrido[1,2-a]pyrimidin-3-yl}-2-propenoic acid

¹H-NMR (CD₃OD) δ : 1.26 (d, J=7Hz, 6H), 1.51 (m, 2H), 1.72 (m, 2H), 1.90 (m, 2H), 2.95 (m, 2H), 3.02 (m, 1H), 3.25 (t, 2H), 3.42 (t, 2H), 3.5-3.8 (m, 7H), 3.95 (m, 1H), 4.50 (m, 1H), 6.98 (s, 1H), 7.04 (d, J=15.6Hz, 1H), 7.14 (d, 1H), 7.28 (s, 1H), 7.64 (d, J=15.6Hz, 1H), 8.85 (d, 1H)
MS (ES+) m/z 582 (M⁺+1)

### Example 91: (E)-3-{8-[2-(4-Isopropyl-1,3-thiazol-2-yl)ethyl]-4-oxo-2-(4-ornithylpiperazino)-4H-pyrido[1,2-a]pyrimidin-3-yl}-2-propenoic acid

¹H-NMR (CD₃OD) δ: 1.26 (d, J=7Hz, 6H), 1.80 (m, 2H), 1.92 (m, 2H), 2.99 (m, 3H), 3.22 (t, 2H), 3.42 (t, 2H), 3.5-3.8 (m, 7H), 3.94 (m, 1H), 4.58 (m, 1H), 6.99 (s, 1H), 7.04 (d, J=15.6Hz, 1H), 7.14 (d, 1H), 7.28 (s, 1H), 7.64 (d, J=15.6Hz, 1H), 8.85 (d, 1H)
MS (ES+) m/z 568 (M⁺+1)

### Example 92: (E)-3-{2-(4-[2-(4-Aza-1-azoniabicyclo[2.2.2]oct-1-yl)acetyl]piperazino)-8-[2-(4-isopropyl-1,3-thiazol-2-yl)ethyl]-4-oxo-4H-pyrido[1,2-a]pyrimidin-3-yl}-2-propenoic acid

### (A) tert-Butyl (E)-3-{2-[4-(2-chloroacetyl)piperazino]-8-[2-(4-isopropyl-1,3-thiazol-2-yl)ethyl]-4-oxo-4H-pyrido[1,2-a]pyrimidin-3-yl}-2-propenoate

tert-Butyl (E)-3-{8-[(E)-2-(4-isopropyl-1,3-thiazol-2-yl)ethyl]-4-oxo-2-piperazino-4H-pyrido[1,2-a]pyrimidin-3-yl]-2-propenoate (206 mg, 0.405 mmol) was dissolved in methylene chloride (8 ml), added with triethylamine (170 ml) and chloroacetyl chloride (64 ml) at -78°C, and then stirred overnight at room temperature in the dark. The reaction solution was diluted with methylene chloride, washed with water, 5% aqueous citric acid, saturated aqueous sodium hydrogencarbonate and then with saturated brine, and the solution was dried over magnesium sulfate. Then, the solvent was evaporated under reduced pressure. The residue was purified by silica gel column chromatography to obtain the title compound (260 mg).
¹H-NMR (CDCl₃): 1.28 (d, 6H), 1.51 (s, 9H), 3.06 (m, 1H), 3.21 (m, 2H), 3.37 (m, 2H), 3.62 (brm, 6H), 3.78 (brm, 2H), 4.10 (s, 2H), 6.72 (s, 1H), 6.89 (d, 1H), 7.07 (d, 1H), 7.24 (d, 1H), 7.49 (d, 1H), 8.88 (d, 1H)

### (B) tert-Butyl (E)-3-{2-[4-(2-iodoacetyl)piperazino]-8-[2-(4-isopropyl-1,3-thiazol-2-yl)-ethyl]-4-oxo-4H-pyrido[1,2-a]pyrimidin-3-yl}-2-propenoate

The tert-butyl (E)-3-{2-[4-(2-chloroacetyl)piperazino]-8-[2-(4-isopropyl-1,3-thiazol-2-yl)ethyl]-4-oxo-4H-pyrido[1,2-a]pyrimidin-3-yl}-2-propenoate (240 mg, 0.405 mmol) obtained in (A) and NaI (300 mg, 2.03 mmol) were refluxed by heating in acetone (8 ml) for 3 hours in the dark. The reaction solution was cooled, then diluted with methylene chloride, washed with water and saturated brine, and dried over sodium sulfate. Then, the solvent was evaporated under reduced pressure to obtain the title compound (226 mg) as a yellow amorphous substance.
¹H-NMR (CDCl₃): 1.28 (d, 6H), 1.52 (s, 9H), 3.10 (m, 1H), 3.22 (m, 2H), 3.38 (m, 2H), 3.60 (m, 4H), 3.79 (s, 2H), 6.73 (s, 1H), 6.90 (d, 1H), 7.08 (d, 1H), 7.50 (d, 1H), 8.88 (d, 1H)

### (C) (E)-3-{2-(4-[2-(4-Aza-1-azoniabicyclo[2.2.2]oct-1-yl)acetyl]piperazino)-8-[2-(4-isopropyl-1,3-thiazol-2-yl)ethyl]-4-oxo-4H-pyrido[1,2-a]pyrimidin-3-yl}-2-propenoic acid

The tert-butyl (E)-3-{2-[4-(2-iodoacetyl)piperazino]-8-[2-(4-isopropyl-1,3-thiazol-2-yl)ethyl]-4-oxo-4H-pyrido[1,2-a]pyrimidin-3-yl}-2-propenoate (226 mg, 0.33 mmol) obtained in (B) and 1,4-diazabicyclo[2.2.2]octane (187 mg, 1.67 mmol) were stirred overnight in tetrahydrofuran (9 ml) under nitrogen atmosphere, then the solvent was evaporated, and the residue was stirred in trifluoroacetic acid (TFA, 4 ml) for 1 hour. The solvent was evaporated under reduced pressure, and the residue was purified by medium pressure reverse phase chromatography (Amberkron column, gradient 100% 0.1% TFA in H₂O to 100% CH₃CN over 80 minutes, 3 ml/minute) to obtain the title compound (150 mg, 75%, for the two steps).
¹H-NMR (CD₃OD) δ : 1.27 (d, 6H), 3.02 (m, 1H), 3.26 (t, 2H), 3.43 (t, 2H), 3.62 (m, 5H), 3.78 (m, 6H)., 4.43 (s, 2H), 7.00 (s, 1H), 7.02 (d, J=15.6Hz, 1H), 7.13 (d, J=7Hz, 1H), 7.29 (s, 1H), 7.63 (d, J=15.6Hz, 1H), 8.85 (d, J=7Hz, 1H)
MS (ES+) m/z 606 (M⁺+1)

The compounds of Examples 93 to 97 mentioned below were synthesized in the same manner as in Example 92.

### Example 93: (E)-3-{8-[2-(4-Isopropyl-1,3-thiazol-2-yl)ethyl]-2-(4-[2-(1-methyl-1H-imidazol-3-ium-3-yl)acetyl]piperazino)-4-oxo-4H-pyrido[1,2-a]pyrimidin-3-yl}-2-propenoic acid

¹H-NMR (CDCl₃ + CD₃OD) δ : 1.23 (d, J=7Hz, 6H), 3.02 (m, 1H); 3.17 (t, 2H), 3.34 (t, 2H), 3.57 (m, 2H), 3.6-3.8 (m, 6H), 3.92 (s, 3H), 5.40 (s, 2H), 6.72 (s, 1H), 6.88 (d, J=7.2Hz, 1H), 7.00 (d, J=15.6Hz, 1H), 7.20 (m, 2H), 7.37 (s, 1H), 7.55 (d, J=15.6Hz, 1H), 8.81 (d, J=7.2Hz, 1H), 9.24 (s, 1H)
MS (ES+) m/z 576 (M⁺+1)

### Example 94: (E)-3-{2-4-[2-(1-Azabicyclo[2.2.2]oct-1-yl)acetyl]piperazino-8-[2-(4-isopropyl-1,3-thiazol-2-yl)ethyl]-4-oxo-4H-pyrido[1,2-a]pyrimidin-3-yl}-2-propenoic acid

¹H-NMR (CD₃OD): 1.26 (d, 6H), 2.05 (brm, 7H), 2.19 (m, 1H), 3.03 (m, 1H), 3.22 (m, 2H), 3.39 (m, 2H), 3.61 (brm, 6H), 3.67 (m, 8H), 4.26 (s, 2H), 6.97 (s, 1H), 6.99 (d, 1H), 7.12 (d, 1H), 7.28 (s, 1H), 7.62 (d, 1H), 8.85 (d, 1H)
MS (ES+): 605

### Example 95: (E)-3-{8-[2-(4-Isopropyl-1,3-thiazol-2-yl)ethyl]-4-oxo-2-(4-[2-(1-pyridinium)acetyl]piperazino)-4H-pyrido[1,2-a]pyrimidin-3-yl]-2-propenoic acid

¹H-NMR (CD₃OD): 1.27 (d, 6H), 3.02 (m, 1H), 3.25 (m, 2H), 3.40 (m, 2H), 5.80 (s, 2H), 6.98 (s, 1H), 7.02 (d, 1H), 7.17 (d, 1H), 7.31 (s, 1H), 7.65 (d, 1H), 8.20 (m, 2H), 8.65 (m, 1H), 8.88 (m, 2H)
MS (ES+): 573

### Example 96: (E)-3-{2-(4-[2-(1-Azabicyclo[2.2.2]oct-1-yl)butanoyl]piperazino)-8-[2-(4-isopropyl-1,3-thiazol-2-yl)ethyl]-4-oxo-4H-pyrido[1,2-a]pyrimidin-3-yl}-2-propenoic acid

¹H-NMR (CD₃OD): 1.25 (d, 6H), 2.07 (m, 1H), 2.58 (m, 1H), 3.02 (m, 1H), 3.20-3.80 (m, 24H), 6.99 (s, 1H), 7.02 (d, 1H), 7.12 (d, 1H), 7.28 (s, 1H), 7.64 (d, 1H), 8.86 (d, 1H)

### Example 97: (E)-3-{2-(4-[2-(4-Aza-1-azoniabicyclo[2.2.2]oct-1-yl)acetyl]piperazino-8-(2-[4-(tert-butyl)-1,3-thiazol-2-yl]ethyl)-4-oxo-4H-pyrido[1,2-a]pyrimidin-3-yl)-2-propenoic acid

¹H-NMR (CD₃OD): 1.29 (s, 9H), 3.20-3.41 (m, 5H), 3.50-3.79 (m, 20H), 4.39 (s, 2H), 6.96 (s, 1H), 7.02 (d, 1H), 7.13 (d, 1H), 7.29 (s, 1H), 7.64 (d, 1H), 8.86 (d, 1H)
MS (ES+): 620

### Example 98: N-((E)-3-{8-[2-(4-Isopropyl-1,3-thiazol-2-yl)ethyl]-2-morpholino-4-oxo-4H-pyrido[1,2-a]pyrimidin-3-yl}-2-propenoyl)methanesulfonamide

(E)-3-{8-[2-(4-Isopropyl-1,3-thiazol-2-yl)ethyl]-2-morpholino-4-oxo-4H-pyrido[1,2-a]pyrimidin-3-yl}-2-propenoic acid (24 mg) was dissolved in dimethylformamide (2 ml), added with methanesulfonamide (15 mg), dimethylaminopyridine (20 mg) and 1-ethyl-3-(3-dimethylaminopropyl)carbodiimide hydrochloride (31 mg), and then the mixture was stirred at room temperature for 24 hours. The reaction solution was added with ethyl acetate and hexane, washed with 0.2 M hydrochloric acid, and dried over sodium over sodium sulfate, and the solvent was evaporated under reduced pressure. The residue was purified by silica gel column chromatography to obtain the title compound (14 mg) as yellow powder.
¹H-NMR (CDCl₃) δ : 1.21 (d, 6H), 3.02 (m, 1H), 3.15 (m, 2H), 3.19 (s, 3H), 3.36 (m, 2H), 3.53 (m, 4H), 3.70 (m, 4H), 6.78 (s, 1H), 6.86 (d, 1H), 6.92 (d, J=16Hz, 1H), 7.17 (s, 1H), 7.53 (d, J=16Hz, 1H), 8.67 (d, 1H)
MS (ES+) m/z 532 (M⁺+1)

### Example 99: N-((E)-3-{8-[2-(4-Isopropyl-1,3-thiazol-2-yl)ethyl]-4-oxo-4H-pyrido[1,2-a]-pyrimidin-3-yl}-2-propenoyl)methanesulfonamide

¹H-NMR (CDCl₃) δ : 1.28 (d, 6H), 3.04 (m, 1H), 3.11 (s, 3H), 3.37 (m, 4H), 6.73 (s, 1H), 7.19 (d, J=7Hz, 1H), 7.32 (d, J=14Hz, 1H), 7.59 (s, 1H), 7.72 (d, J=14Hz, 1H), 8.48 (s, 1H), 9.08 (d, J=7Hz, 1H)
MS (ES+) m/z 447 (M⁺+ 1); MS (ES-) m/z 445 (M⁺- 1)

### Example 100: N-((E)-3-{8-[2-(4-Isopropyl-1,3-thiazol-2-yl)ethyl]-2-morpholino-4-oxo-4H-pyrido[1,2-a]pyrimidin-3-yl}-2-propenoyl)-3-amino-1-propanesulfonamide

### (A) N-((E)-3-{8-[2-(4-Isopropyl-1,3-thiazol-2-yl)ethyl]-2-morpholino-4-oxo-4H-pyrido[1,2-a]pyrimidin-3-yl}-2-propenoyl)-3-(tert-butoxycarbonylamino)-1-propanesulfonamide

(E)-3-{8-[2-(4-Isopropyl-1,3-thiazol-2-yl)ethyl]-2-morpholino-4-oxo-4H-pyrido[1,2-a]pyrimidin-3-yl}-2-propenoic acid (53 mg) was dissolved in dimethylformamide (2 ml), added with 3-(N-tert-butoxycarbonylamino)propanesulfonamide (55 mg) and 1-ethyl-3-(3-dimethylaminopropyl)carbodiimide hydrochloride (67 mg), and the mixture was stirred at room temperature for 24 hours. The reaction solution was added with ethyl acetate and hexane, washed with 0.2 M hydrochloric acid, and dried over sodium sulfate, and the solvent was evaporated under reduced pressure. The residue was purified by silica gel column chromatography to obtain the title compound (32 mg) as yellow powder.
¹H-NMR (300MHz) δ : 1.22 (d, J=7.2Hz, 6H), 1.40 (s, 9H), 1.90-2.05 (m, 2H), 3.00-3.80 (m, 17H), 6.70 (s, 1H), 6.85 (d, J=7.5Hz, 1H), 7.20 (s, 1H), 7.35 (d, J=15.4Hz, 1H), 7.65 (d, J=15.4Hz, 1H), 9.00-9.10 (m, 2H)

### (B) N-((E)-3-{8-[2-(4-Isopropyl-1,3-thiazol-2-yl)ethyl]-2-morpholino-4-oxo-4H-pyrido[1,2-a]pyrimidin-3-yl}-2-propenoyl)-3-amino-1-propanesulfonamide

The N-((E)-3-{8-[2-(4-isopropyl-1,3-thiazol-2-yl)ethyl]-2-morpholino-4-oxo-4H-pyrido[1,2-a]pyrimidin-3-yl}-2-propenoyl)-3-(tert-butoxycarbonylamino)-1-propanesulfonamide (32 mg) obtained in (A) was dissolved in trifluoroacetic acid (2 ml) and stirred for 40 minutes. The solvent was evaporated under reduced pressure, and the residue was purified by medium pressure reverse phase column chromatography to obtain the title compound (quantitative).
¹H-NMR (CD₃OD) δ : 1.25 (d, 6H), 2.18 (m, 2H), 3.02 (m, 1H), 3.1-3.45 (m, with CHD₂OD), 3.60 (m, 4H), 3.80 (m, 4H), 6.98 (s, 1H), 7.12 (s, 1H), 7.16 (d, J=16Hz, 1H), 7.26 (s, 1H), 7.75 (d, J=16Hz, 1H), 8.82 (d, 1H)
MS (ES+) m/z 575 (M⁺+1); MS (ES-) m/z 573 (M⁺-1)

### Example 101: (E)-3-{8-[(E)-2-(4-Isopropyl-1,3-thiazol-2-yl)-1-ethenyl]-2-morpholino-4-oxo-4H-pyrido[1,2-a]pyrimidin-3-yl}-2-propenoic acid

### (A) tert-Butyl (E)-3-{8-[(E)-2-(4-isopropyl-1,3-thiazol-2-yl)-1-ethenyl]-2-{[(4-methylphenyl)sulfonyl]oxy}-4-oxo-4H-pyrido[1,2-a]pyrimidin-3-yl}-2-propenoate

4-[(E)-2-(4-Isopropyl-1,3-thiazol-2-yl)-1-ethenyl]-2-pyridinamine (100 mg, 0.408 mmol) was dissolved in xylene (1.2 ml), added with trichlorophenyl malonate (208 mg, 0.448 mmol), and then the mixture was refluxed by heating for 1.3 hours. The reaction mixture was added with n-hexane, and the deposited solid was collected. by filtration. Dimethylformamide (110 µl) was added with phosphorus oxychloride (190 µl, 2.04 mmol) under ice cooling and stirred at room temperature for 30 minutes. The mixture was added with the solid dissolved in dichloromethane (3.0 ml) under ice cooling, and stirred at room temperature for 1.5 hours. The reaction mixture was added with saturated aqueous sodium hydrogencarbonate and extracted with chloroform. The organic layer was dried over magnesium sulfate, and the solvent was evaporated under reduced pressure. The residue was suspended in anhydrous tetrahydrofuran (4.0 ml), added with (tert-butoxycarbonylmethylene)triphenylphosphorane (460 mg, 1.22 mmol), and stirred at 80°C for 5 days. The reaction solution was concentrated, and then the residue was purified by silica gel column chromatography (chloroform:methanol = 100:0 → 100:10, v/v) and thin layer chromatography (chloroform:methanol = 10:1, v/v). The resulting compound was dissolved in anhydrous tetrahydrofuran (600 µl) and dimethylformamide (600 µl), added with 4-dimethylaminopyridine (10.2 mg, 0.0831 mmol) and p-toluenesulfonyl chloride (13.4 mg, 0.0703 mmol), and stirred at room temperature for 2 hours. The reaction mixture was added with water and extracted with chloroform. The organic layer was dried over magnesium sulfate, and the solvent was evaporated under reduced pressure. The residue was purified by thin layer chromatography (n-hexane:ethyl acetate = 2:1, v/v) to obtain the title compound (9.0 mg, 3.7%).
¹H-NMR (CDCl₃) δ : 1.37 (6H, d, J=6.8Hz), 1.51 (9H, s), 2.48 (3H, s), 3.14-3.21 (1H, m), 7.03 (1H, s), 7.18 (1H, d, J=15.9Hz), 7.38-7.42 (4H, m), 7.52 (1H, d, J=1.5Hz), 7.56 (1H, d, J=16.1Hz), 7.67 (1H, d, J=15.9Hz), 8.05 (2H, d, J=8.3Hz), 9.02 (1H, d, J=7.6Hz)

### (B) tert-Butyl (E)-3-{8-[(E)-2-(4-isopropyl-1,3-thiazol-2-yl)-1-ethenyl]-2-morpholino-4-oxo-4H-pyrido [1,2-a]pyrimidin-3-yl}-2-propenoate

The tert-butyl (E)-3-{8-[(E)-2-(4-isopropyl-1,3-thiazol-2-yl)-1-ethenyl]-2-{[(4-methylphenyl)sulfonyl]oxy}-4-oxo-4H-pyrido[1,2-a]pyrimidin-3-yl}-2-properioate (9.0 mg, 0.0152 mmol) obtained in (A) was dissolved in dimethylformamide (0.75 ml), added with morpholine (13.2 µl, 0.152 mmol), and stirred overnight at room temperature. The reaction mixture was concentrated, and then the residue was purified by thin layer chromatography (n-hexane:ethyl acetate = 1:1, v/v) to obtain the title compound (6.7 mg, 87%).
¹H-NMR (CDCl₃) δ : 1.33 (6H, d, J=6.8Hz), 1.52 (9H, s), 3.12-3.19 (1H, m), 3.62-3.64 (4H, m), 3.73-3.75 (4H, m), 6.97 (1H, s), 7.08 (1H, d, J=15.9Hz), 7.16 (1H, dd, J=1.5, 7.6Hz), 7.34-7.38 (2H, m), 7.50 (2H, d, J=15.9Hz), 8.91 (1H, d, J=7.6Hz)

### (C) (E)-3-{8-[(E)-2-(4-Isopropyl-1,3-thiazol-2-yl)-1-ethenyl]-2-morpholino-4-oxo-4H-pyrido[1,2-a]pyrimidin-3-yl}-2-propenoic acid

The tert-butyl (E)-3-{8-[(E)-2-(4-isopropyl-1,3-thiazol-2-yl)-1-ethenyl]-2-morpholino-4-oxo-4H-pyrido[1,2-a]pyrimidin-3-yl}-2-propenoate (6.7 mg, 0.0132 mmol) obtained in (B) was dissolved in 1,4-dioxane (260 µl), added with 4 N hydrochloric acid solution in 1,4-dioxane (260 µl), and stirred at room temperature for 7 hours. The reaction solution was concentrated, and the residue was purified by thin layer chromatography (chloroform:methanol = 10:1, v/v) to obtain the title compound (4.8 mg, 81%).
¹H-NMR (CDCl₃ + CD₃OD) δ : 1.36 (6H, d, J=6.8Hz), 3.13-3.20 (1H, m), 3.67 (4H, t, J=4.4Hz), 3.86 (4H, t, J=4.4Hz), 7.04 (1H, d, J=15.6Hz), 7.07 (1H, s), 7.33 (1H, dd, J=1.6, 7.5Hz), 7.40 (1H, d, J=16.2Hz), 7.47 (1H, d, J=6.6Hz), 7.58 (1H, d, J=16.2Hz), 7.62 (1H, d, J=15.6Hz), 8.89 (1H, d, J=7.5Hz)
FAB-MS; m/z: 453 (M⁺+1)
FAB-HRMS; Calcd. for C₂₃H₂₅O₄N₄S+H⁺: 453.1597, Found: 453.1602

### Example 102: (E)-3-{2-(3-Hydroxypiperidino)-8-[(E)-2-(4-isopropyl-1,3-thiazol-2-yl)-1-ethenyl]-4-oxo-4H-pyrido[1,2-a]pyrimidin-3-yl}-2-propenoic acid

### (A) 4-Isopropyl-1,3-thiazole-2-carbaldehyde

(4-Isopropyl-1,3-thiazol-2-yl)methanol (5.20 g, 33.0 mmol) was dissolved in methylene chloride (100 ml), added with pyridinium dichromate (14.9 g, 39.7 mmol), and stirred at room temperature for 19 hours stirred. After insoluble solids were removed, the reaction solution was evaporated, and the residue was purified by silica gel column chromatography (chloroform) to obtain the title compound (3.32 g, 65%). ¹H-NMR (CDCl₃) δ : 1.37 (6H, d, J=6.8Hz), 3.18-3.25 (1H, m), 7.34 (1H, s), 9.98 (1H, s)

### (B) tert-Butyl N-4-[2-hydroxy-2-(4-isopropyl-1,3-thiazol-2-yl)ethyl]-2-pyridylcarbamate

tert-Butyl N-(4-methyl-2-pyridyl)carbamate (6.24 g, 30.0 mmol) was dissolved in anhydrous tetrahydrofuran (100 ml), added with n-butyl lithium (1.59 M in n-hexane, 47.1 ml, 74.9 mmol) at -78°C under argon atmosphere, and then stirred at room temperature for 1.5 hours. The reaction solution was cooled to -78°C again, then added with a solution of the 4-isopropyl-1,3-thiazole-2-carbaldehyde (4.65 g, 30.0 mmol) obtained in (A) in anhydrous tetrahydrofuran (50.0 ml), and then the mixture was stirred for 3 hours. The reaction mixture was added with saturated aqueous ammonium chloride, warmed to room temperature, and then extracted with ethyl acetate. The organic layer was dried over magnesium sulfate, and the solvent was evaporated under reduced pressure. The residue was purified by silica gel column chromatography (n-hexane:ethyl acetate = 3:1 → 1:1, v/v) to obtain the title compound (5.42 g, 50%).
¹H-NMR (CDCl₃) δ : 1.29 (6H, d, J=6.8Hz), 1.53 (9H, s), 3.03-3.11 (2H, m), 3.28-3.32 (2H, m), 5.24 (1H, dd, J=4.2, 8.5Hz), 6.82 (1H, d, J=0.7Hz), 6.85 (1H, dd, J=1.6, 5.2Hz), 7.90 (1H, s), 8.10 (1H, s), 8.14 (1H, d, J=5.2Hz)
ESI-MS; m/z: 364 (M⁺+1)

### (C) tert-Butyl N-4-[(E)-2-(4-isopropyl-1,3-thiazol-2-yl)-1-ethenyl]-2-pyridylcarbamate

The tert-butyl N-4-[2-hydroxy-2-(4-isopropyl-1,3-thiazol-2-yl)ethyl]-2-pyridylcarbamate (5.30 g, 14.6 mmol) obtained in (B) was dissolved in anhydrous tetrahydrofuran (100 ml), added with triethylamine (5.08 ml, 36.5 mmol) and methanesulfonyl chloride (1.35 ml, 17.5 mmol), and then the mixture was stirred at room temperature for 1 hour. After insoluble solids were removed, the reaction solution was washed with tetrahydrofuran, and the solvent was evaporated under reduced pressure. The residue was dissolved in toluene (100 ml), added with 1,8-diazabicyclo[5.4.0]undec-7-ene (10.9 ml, 72.9 mmol), and refluxed by heating for 30 minutes. The reaction solution was concentrated, then added with 1 N hydrochloric acid and extracted with chloroform. The organic layer was washed with saturated aqueous sodium hydrogencarbonate and dried over magnesium sulfate, and the solvent was evaporated under reduced pressure. The residue was purified by silica gel column chromatography (n-hexane:ethyl acetate = 5:1 → 3:1, v/v) to obtain the title compound (3.76 g, 75%).
¹H-NMR (CDCl₃) δ : 1.34 (6H, d, J=6.8Hz), 1.56 (9H, s), 3.07-3.18 (1H, m), 6.87 (1H, s), 7.07 (1H, dd, J=1.2, 5.1Hz), 7.31 (1H, d, J=16.1Hz), 7.50 (1H, d, J=16.1Hz), 8.12 (1H, s), 8.25 (1H, s), 8.26 (1H, s)

### (D) 4-[(E)-2-(4-Isopropyl-1,3-thiazol-2-yl)-1-ethenyl]-2-pyridinamine

The tert-butyl N-4-[(E)-2-(4-isopropyl-1,3-thiazol-2-yl)-1-ethenyl]-2-pyridylcarbamate (3.75 g, 10.9 mmol) obtained in (C) was dissolved in dichloromethane (50.0 ml), added with trifluoroacetic acid (50.0 ml), and then the mixture was stirred at room temperature for 30 minutes. The reaction solution was concentrated, then neutralized with saturated aqueous sodium hydrogencarbonate, and extracted with chloroform. The organic layer was dried over magnesium sulfate, and then the solvent was evaporated under reduced pressure to obtain the title compound (2.65 g, 100%).
¹H-NMR (CDCl₃) δ : 1.34 (6H, d, J=6.8Hz), 3.09-3.16 (1H, m), 4.67 (1H, brs), 6.57 (1H, s), 6.80 (1H, dd, J=1.3, 5.5Hz), 6.87 (1H, s), 7.18 (1H, d, J=16.1Hz), 7.37 (1H, d, J=16.1Hz), 8.04 (1H, d, J=5.5Hz)

### (E) 2-Hydroxy-8-[(E)-2-(4-isopropyl-1,3-thiazol-2-yl)-1-ethenyl]-4H-pyrido[1,2-a]-pyrimidin-4-one

The 4-[(E)-2-(4-isopropyl-1,3-thiazol-2-yl)-1-ethenyl]-2-pyridinamine (2.65 g, 10.8 mmol) obtained in (D) was dissolved in toluene (100 ml), added with trichlorophenyl malonate (5.50 g, 11.9 mmol), and refluxed by heating for 1.5 hours. After the reaction mixture was concentrated, the deposited solid was collected by filtration and washed with, n-hexane and ether to obtain the title compound (3.14 g, 93%) as yellow solid.
¹H-NMR (CDCl₃) δ: 1.37 (6H, d, J=6.8Hz), 3.14-3.21 (1H, m), 5.41 (1H, s), 7.05 (1H, s), 7.40 (1H, dd, J=1.5, 7.4Hz), 7.46 (1H, d, J=16.1Hz), 7.51 (1H, d, J=1.5Hz), 7.66 (1H, d, J=16.1Hz), 9.09 (1H, d, J=7.4Hz)

### (F) 2-(3-Hydroxypiperidino)-8-[(E)-2-(4-isopropyl-1-,3-thiazol-2-yl)-1-ethenyl]-4H-pyrido[1,2-a]pyrimidin-4-one

The 2-hydroxy-8-[(E)-2-(4-isopropyl-1,3-thiazol-2-yl)-1-ethenyl]-4H-pyrido[1,2-a]-pyrimidin-4-one (1.00 g, 3.19 mmol) obtained in (E) was dissolved in anhydrous tetrahydrofuran (15.0 ml) and anhydrous dimethylformamide (15.0 ml), added with 4-dimethylaminopyridine (585 mg, 4.79 mmol) and p-toluenesulfonyl chloride (730 mg, 3.83 mmol), and then the mixture was stirred at room temperature for 50 minutes. Subsequently, the reaction solution was added with 3-hydroxypiperidine (646 mg, 6.38 mmol) and stirred at room temperature for 16 hours, and then added with 3-hydroxypiperidine (968 mg, 9.57 mmol) and stirred at 60°C for 1.5 hours. After the reaction mixture was concentrated, the residue was purified by silica gel column chromatography (chloroform:methanol = 100:0 → 100:1 → 100:2 → 100:5 → 100:10, v/v) to obtain the title compound (865 mg, 68%).
¹H-NMR (CDCl₃) δ : 1.35 (6H, d, J=7.1Hz), 1.54-1.72 (1H, m), 1.86-2.01 (3H, m), 3.11-3.21 (1H, m), 3.39-3.45 (1H, m), 3.55 (1H, dd, J=7.1,13.2Hz), 3.73-3.78 (1H, m), 3.85-3.89 (1H, m), 4.00 (1H, dd, J=3.1,12.9Hz), 5.65 (1H, m), 6.94 (1H, m), 7.03 (1H, dd, J=2.0, 7.6Hz), 7.32 (1H, d, J=16.1Hz), 7.45 (1H, d, J=16.1Hz), 8.82 (1H, d, J=7.3Hz) ESI-MS; m/z: 397 (M⁺+1)

### (G) tert-Butyl (E)-3-{2-(3-formyloxypiperidino)-8-[(E)-2-(4-isopropyl-1,3-thiazol-2-yl)-1-ethenyI]-4-oxo-4H-pyrido[1,2-a]pyrimidin-3-yl}-2-propenoate

Dimethylformamide (15.0 ml) was added with phosphorus oxychloride (608 µ 1, 6.52 mmol) under ice cooling and stirred at room temperature for 40 minutes. The mixture was added to a solution of the 2-(3-hydroxypiperidino)-8-[(E)-2-(4-isopropyl-1,3-thiazol-2-yl)-1-ethenyl]-4H-pyrido[1,2-a]pyrimidin-4-one (862 mg, 2.17 mmol) obtained in (F) in dimethylformamide (15.0 ml) under ice cooling, and then the mixture was stirred at room temperature for 1.5 hours. The reaction mixture was added with saturated aqueous sodium hydrogencarbonate and extracted with chloroform. The organic layer was dried over magnesium sulfate, and the solvent was evaporated under reduced pressure. The residue was suspended in anhydrous tetrahydrofuran (20 ml) and anhydrous N,N-dimethylformamide (10 ml), added with (tert-butoxycarbonylmethylene)triphenylphosphorane (1.64 g, 4.35 mmol), and stirred at 50°C for 16 hours. The reaction mixture was concentrated, and the residue was purified by silica gel column chromatography (n-hexane:ethyl acetate = 10:1 → 5:1 → 3:1 → 2:1, v/v) to obtain the title compound (488 mg, 41%).
¹H-NMR (CDCl₃) δ : 1.35 (6H, d, J=6.8Hz), 1.52 (9H, s), 1.72-1.75 (1H, m), 1.81-1.85 (1H, m), 1.94-2.03 (2H, m), 3.12-3.18 (1H, m), 3.51-3.60 (2H, m), 3.71 (1H, dd, J=6.3, 13.4Hz), 3.82 (1H, dd, J=3.1,13.4Hz), 5.13-5.16 (1H, m), 6.97 (1H, s), 7.07 (1H, d, J=15.6Hz), 7.15 (1H, dd, J=1.7, 7.6Hz), 7.34-7.37 (2H, m), 7.49 (1H, d, J=16.1Hz), 7.51 (1H, d, J=15.6Hz), 8.10 (1H, s), 8.89 (1H, d, J=7.6Hz)
ESI-MS; m/z: 551 (M⁺+1)

### (H) tert-Butyl (E)-3-{2-(3-hydroxypiperidino)-8-[(E)-2-(4-isopropyl-1,3-thiazol-2-yl)-1-ethenyl)-4-oxo-4H-pyrido[1,2-a]pyrimidin-3-yl}-2-propenoate

The tert-butyl (E)-3-{2-(3-formyloxypiperidino)-8-[(E)-2-(4-isopropyl-1,3-thiazol-2-yl)-1-ethenyl]-4-oxo-4H-pyrido[1,2-a]pyrimidin-3-yl}-2-propenoate (184 mg, 0.334 mmol) obtained in (G) was dissolved in methanol (3.0 ml), added with 1 N aqueous sodium hydroxide (1.0 ml), and then the mixture was stirred at room temperature for 10 minutes. The reaction mixture was added with 1 N hydrochloric acid (1.0 ml) and extracted with chloroform. The organic layer was dried over magnesium sulfate and the solvent was evaporated under reduced pressure. The residue was purified by silica gel column chromatography (n-hexane:ethyl acetate = 2:1 → 2:1 → 1:2, v/v) to obtain the title compound (174 mg, 100%).
¹H-NMR (CDCl₃) δ : 1.35 (6H, d, J=6.8Hz), 1.52 (9H, s), 1.68-1.89 (4H, m), 3.12-3.18 (1H, m), 3.55-3.66 (3H, m), 3.93-3.96 (1H, m), 4.03 (1H, br s), 6.97 (1H, s), 7.06 (1H, d, J=15.6Hz), 7.16 (1H, dd, J=1.6, 7.5Hz), 7.34 (1H, d, J=16.1Hz), 7.34 (1H, d, J=1.6Hz), 7.49 (1H, d, J=16.1Hz), 7.50 (1H, d, J=15.6Hz), 8.89 (1H, d, J=7.5Hz)
ESI-MS; m/z: 523 (M⁺+1)

### (I) (E)-3-{2-(3-Hydroxypiperidino)-8-[(E)-2-(4-isopropyl-1,3-thiazol-2-yl)-1-ethenyl]-4-oxo-4H-pyrido[1,2-a]pyrimidin-3-yl}-2-propenoic acid

The tert-Butyl (E)-3-{2-(3-hydroxypiperidino)-8-[(E)-2-(4-isopropyl-1,3-thiazol-2-yl)-1-ethenyl]-4-oxo-4H-pyrido[1,2-a]pyrimidin-3-yl}-2-propenoate (43.4 mg, 0.0830 mmol) obtained in (H) was dissolved in 1,4-dioxane (200 µl), added with 4 N hydrochloric acid solution in 1,4-dioxane, and stirred at room temperature for 3.5 hours. The reaction solution was concentrated, and the residue was purified by thin layer chromatography (chloroform:methanol:water = 10:1:0 → 8:3:0.1 → 7:3:1, v/v) to obtain the title compound (18.9 mg, 49%).
¹H-NMR (DMSO-d₆) δ : 1.29 (6H, d, J=6.6Hz), 1.42-1.47 (1H, m), 1.55-1.59 (1H, m), 1.81 (1H, m), 1.90-1.95 (1H, m), 2.97-3.74 (4H, m), 3.89-3.92 (1H, m), 4.95 (1H, br s), 6.89 (1H, d, J=15.1Hz), 7.42-7.46 (2H, m), 7.54-7.62 (3H, m), 7.87 (1H, d, J=16.6Hz), 8.76 (1H, d, 7.8Hz)
ESI-MS; m/z: 467 (M⁺+1)

### Example 103: (E)-3-(2-{3-[(Aminocarbonyl)oxy]piperidino}-8-[(E)-2-(4-isopropyl-1,3-thiazol-2-yl)-1-ethenyl]-4-oxo-4H-pyrido[1,2-a]pyrimidin-3-yl)-2-propenoic acid

### (A) tert-Butyl (E)-3-(2-{3-[(aminocarbonyl)oxy]piperidino}-8-[(E)-2-(4-isopropyl-1,3-thiazol-2-yl)-1-ethenyl]-4-oxo-4H-pyrido[1,2-a]pyrimidin-3-yl)-2-propenoate

tert-Butyl (E)-3-{2-(3-hydroxypiperidino)-8-[(E)-2-(4-isopropyl-1,3-thiazol-2-yl)-1-ethenyl]-4-oxo-4H-pyrido[1,2-a]pyrimidin-3-yl}-2-propenoate (138 mg, 0.264 mmol) was dissolved in ethyl acetate (2.6 ml), added with trichloroacetyl isocyanate (62.6 µl, 0.528 mmol) under ice cooling, and then the mixture was stirred at room temperature for 15 minutes. The reaction mixture was concentrated, dissolved in methanol (5.0 ml) and water (0.5 ml), and then added with sodium formate (71.8 mg, 1.06 mmol), and stirred overnight at room temperature. The reaction mixture was further added with chloroform (3.0 ml) and sodium formate (71.8 mg, 1.06 mmol) and stirred overnight. The reaction mixture was concentrated, and the residue was purified by silica gel column chromatography (chloroform:methanol = 100:0 → 100:1 → 100:2, v/v) to obtain the title compound (150 mg, 100%).
¹H-NMR (CDCl₃) δ : 1.35 (6H, d, J=6.8Hz), 1.50 (9H, s), 1.70-2.04 (4H, m), 3.11-3.18 (1H, m), 3.27-3.33 (1H, m), 3.52-3.56 (1H, m), 3.68-3.74 (2H, m), 4.85 (1H, br s), 5.15 (2H, br s), 6.97 (1H, s), 7.11 (1H, d, J=15.6Hz), 7.15 (1H, dd, J=1.8, 7.6Hz), 7.34 (1H, d, J=16.5Hz), 7.37 (1H, s), 7.49 (1H, d, J=16.5Hz), 7.72 (1H, d, J=15.6Hz), 8.89 (1H, d, J=7.6Hz)
ESI-MS; m/z: 566 (M⁺+1)

### (B) (E)-3-(2-{3-[(Aminocarbonyl)oxy]piperidino}-8-[(E)-2-(4-isopropyl-1,3-thiazol-2-yl)-1-ethenyl]-4-oxo-4H-pyrido[1,2-a]pyrimidin-3-yl)-2-propenoic acid

The tert-butyl (E)-3-(2-{3-[(aminocarbonyl)oxy]piperidino}-8-[(E)-2-(4-isopropyl-1,3-thiazol-2-yl)-1-ethenyl]-4-oxo-4H-pyrido[1,2-a]pyrimidin-3-yl)-2-propenoate obtained in (B) was dissolved in 1,4-dioxane (100 µl), added with 4 N hydrochloric acid solution in 1,4-dioxane (400 µl), and stirred at room temperature for 15 hours. The reaction solution was further added with 4 N hydrochloric acid solution in 1,4-dioxane (400 µl) and stirred at room temperature for 7 hours. The reaction solution was concentrated, and the residue was subjected to azeotropy with toluene. The product was added with saturated aqueous sodium hydrogencarbonate, neutralized with phosphate buffer (pH 7-8), and extracted with a mixture of chloroform/methanol (10:1, v/v). The organic layer was dried over magnesium sulfate, and the solvent was evaporated under reduced pressure. The residue was purified by thin layer chromatography (chloroform:methanol = 20:1, v/v) to obtain the title compound (8.1 mg, 36%).
¹H-NMR (CDCl₃ + CD₃OD) δ : 1.35 (6H, d, J=7.1Hz), 1.71 (1H, m), 1.90-1.96 (3H, m), 3.12-3.19 (1H, m), 3.38-3.40 (1H, m), 3.58-3.81 (3H, m), 4.87 (1H, br s), 6.99 (1H, s), 7.11 (1H, d, J=15.6Hz), 7.21 (1H, d, J=7.7Hz), 7.35 (1H, d, J=16.1Hz), 7.40 (1H, s), 7.52 (1H, d, J=16.1Hz), 7.76 (1H, d, J=15.6Hz), 8.88 (1H, d, J=7.7Hz)
ESI-MS; m/z: 510 (M⁺+1)

### Example 104: 2-(3-Hydroxypiperidino)-8-[(E)-2-(4-isopropyl-1,3-thiazol-2-yl)-1-ethenyl]-3-[(E)-2-(1H-1,2,3,4-tetrazol-5-yl)-1-ethenyl]-4H-pyrido[1,2-a]pyrimidin-4-one

### (A) tert-Butyl (E)-3-{2-[(3-acetyloxy)piperidino]-8-[(E)-2-(4-isopropyl-1,3-thiazol-2-yl)-1-ethenyl]-4-oxo-4H-pyrido[1,2-a]pyrimidin-3-yl}-2-propenoate

tert-Butyl (E)-3-{2-(3-formyloxypiperidino)-8-[(E)-2-(4-isopropyl-1,3-thiazol-2-yl)-1-ethenyl]-4-oxo-4H-pyrido[1,2-a]pyrimidin-3-yl}-2-propenoate (115 mg, 0.209 mmol) was dissolved in methanol (2.0 ml), added with 1 N aqueous sodium hydroxide (627 µl), and stirred at room temperature for 10 minutes. The reaction solution was added with 1 N hydrochloric acid (627 µl) and extracted with chloroform. The organic layer was dried over magnesium sulfate, and the solvent was evaporated under reduced pressure. The residue was dissolved in dichloromethane (2.0 ml), added with 4-dimethylaminopyridine (51.0 mg, 0.418 mmol) and acetic anhydride (29.6 µl), and then the mixture was stirred at room temperature for 10 minutes. The reaction mixture was added with 1 N hydrochloric acid and extracted with chloroform. The organic layer was dried over magnesium sulfate, and the solvent was evaporated under reduced pressure. The residue was purified by silica gel column chromatography (n-hexane:ethyl acetate = 5:1 → 3:1 → 2:1, v/v) to obtain the title compound (121 mg, quantitative).
¹H-NMR (CDCl₃) δ : 1.35 (6H, d, J=6.8Hz), 1.52 (9H, s), 1.70-1.78 (2H, m), 1.90-2.02 (2H, m), 2.06 (3H, s), 3.12-3.19 (1H, m), 3.47-3.52 (1H, m), 3.61 (2H, dd, J=7.1, 13.2Hz), 3.88 (1H, dd, J=3.3, 13.2Hz), 4.96-5.00 (1H, m), 6.96 (1H, s), 7.06 (1H, d, J=15.6Hz), 7.13 (1H, dd, J=2.0, 7.6Hz), 7.33-7.37 (2H, m), 7.48 (1H, d, J=16.1Hz), 7.50 (1H, d, J=15.6Hz), 7.50 (1H, d, J=15.6Hz), 8.89 (1H, d, J=7.6Hz)
ESI-MS; m/z: 565 (M⁺+1)

### (B) 1-(3-{(E)-3-[(2-Cyanoethyl)amino]-3-oxo-1-propenyl}-8-[(E)-2-(4-isopropyl-1,3-thiazol-2-yl)-1-ethenyl]-4-oxo-4H-pyrido[1,2-a]pyrimidin-2-yl)-3-piperidyl acetate

The tert-Butyl (E)-3-{2- [(3-acetyloxy)piperidino]-8-[(E)-2-(4-isopropyl-1,3-thiazol-2-yl)-1-ethenyl]-4-oxo-4H-pyrido[1,2-a]pyrimidin-3-yl}-2-propenoate (121 mg, 0.214 mmol) obtained in (A) was dissolved in 1,4-dioxane (1.0 ml), added with 4 N hydrochloric acid solution in 1,4-dioxane, and then the mixture was stirred overnight at room temperature. The reaction mixture was concentrated, and the residue was suspended in dichloromethane (2.0 ml), added with 2-cyanoethylamine (79 µl, 1.07 mmol), BOPCl (109 mg, 0.429 mmol) and diisopropylethylamine (187 µl, 1.07 mmol), and stirred for 1 hour. The reaction solution was further added with 2-cyanoethylamine (79 µl, 1.07 mmol), BOPCl (109 mg, 0.429 mmol) and diisopropylethylamine (187 µl, 1.07 mmol) and stirred overnight. The reaction mixture was added with saturated aqueous sodium hydrogencarbonate and extracted with a mixture of chloroform/methanol (10:1, v/v). The organic layer was dried over magnesium sulfate, and the solvent was evaporated under reduced pressure. The residue was purified by column chromatography (chloroform:methanol , 100:0 → 100:1 → 100:2, v/v) to obtain the title compound (53.3 mg, 48%).
¹H-NMR (CDCl₃) δ : 1.35 (6H, d, J=6.8Hz), 1.70-1.79 (2H, m), 1.90-1.93 (1H, m), 2.01-2.04 (1H, m), 2.06 (3H, s), 2.71 (2H, t, J=6.5Hz), 3.11-3.18 (1H, m), 3.46-3.51 (1H, m), 3.56-3.68 (4H, m), 3.92 (1H, dd, J=3.1,13.1Hz), 4.95-5.00 (1H, m), 6.58-6.61 (1H, m), 6.96 (1H, s), 7.13 (1H, d, J=7.6Hz), 7.25 (1H, d, J=15.1Hz), 7.31-7.34 (2H, m), 7.47 (1H, d, J=16.4Hz), 7.47 (1H, d, J=15.1Hz), 7.52 (1H, d, J=15.1Hz), 8.83 (1H, d, J=7.6Hz)
ESI-MS; m/z: 561 (M⁺+1)

### (C) 1-(3-{(E)-2-[1-(2-Cyanoethyl)-1H-1,2,3,4-tetrazol-5-yl]-1-ethenyl}-8-[(E)-2-(4-isopropyl-1,3-thiazol-2-yl)-1-ethenyl]-4-oxo-4H-pyrido[1,2-a]pyrimidin-2-yl)-3-piperidyl acetate

The 1-(3-{(E)-3-[(2-cyanoethyl)amino]-3-oxo-1-propenyl}-8-[(E)-2-(4-isopropyl-1,3-thiazol-2-yl)-1-ethenyl]-4-oxo-4H-pyrido[1,2-a]pyrimidin-2-yl)-3-piperidyl acetate (57.3 mg, 0.102 mmol) obtained in (B) was suspended in acetonitrile (3.0 ml), added with sodium azide (13.3 mg, 0.204 mmol) and trifluoromethanesulfonic acid anhydride (25.8 µl, 0.153 mmol) under ice cooling, and then the mixture was stirred at room temperature for 1 hour. The reaction mixture was further added with sodium azide (13.3 mg, 0.204 mmol) and trifluoromethanesulfonic acid anhydride (25.8 µl, 0.153 mmol), and stirred for 3 hours. The reaction mixture was added with saturated aqueous sodium hydrogencarbonate and extracted with chloroform, and the organic layer was dried over magnesium sulfate. The solvent was evaporated under reduced pressure, and the residue was purified by thin layer chromatography (hexane:ethyl acetate = 1:2 → 1:3 → 0:1, v/v) to obtain the title compound (26.8 mg, 45%).
¹H-NMR (CDCl₃) δ : 1.36 (6H, d, J=6.9Hz), 1.73-1.80 (4H, m), 2.04 (3H, s), 3.10 (2H, t, J=7.0Hz), 3.13-3.19 (1H, m), 3.57-3.73 (3H, m), 3.91 (1H, dd, J=2.8, 13.1Hz), 4.69 (2H, t, J=7.0Hz), 4.95-4.98 (1H, m), 6.97 (1H, s), 7.17 (1H, dd, J=1.5, 7.5Hz), 7.36 (1H, d, J=1.5Hz), 7.36 (1H, d, J=16.0Hz), 7.50 (1H, d, J=16.0Hz), 7.75 (1H, d, J=15.3Hz), 7.83 (1H, d, J=15.3Hz), 8.86 (1H, d, J=7.5Hz)

### (D) 2-(3-Hydroxypiperidino)-8-[(E)-2-(4-isopropyl-1,3-thiazol-2-yl)-1-ethenyl]-3-[(E)-2-(1H-1,2,3,4-tetraazol-5-yl)-1-ethenyl]-4H-pyrido[1,2-a]pyrimidin-4-one

The 1-(3-{(E)-2-[1-(2-cyanoethyl)-1H-1,2,3,4-tetraazol-5-yl]-1-ethenyl}-8-[(E)-2-(4-isopropyl-1,3-thiazol-2-yl)-1-ethenyl]-4-oxo-4H-pyrido[1,2-a]pyrimidin-2-yl)-3-piperidyl acetate (25.9 mg, 0.0442 mmol) obtained in (C) was suspended in methanol (0.5 ml) and anhydrous tetrahydrofuran (1.0 ml), added with sodium methoxide (4.8 mg, 0.0884 mmol) under ice cooling, and then stirred for 4 hours. The reaction solution was further added with sodium methoxide (4.8 mg, 0.0884 mmol) and stirred for 1 hour. The reaction mixture was added with saturated aqueous ammonium chloride and extracted with chloroform, and the organic layer was dried over magnesium sulfate. Then, the solvent was evaporated under reduced pressure, and the residue was purified by thin layer chromatography (chloroform:methanol = 10:1, v/v) to obtain the title compound (12.7 mg, 59%).
¹H-NMR (CD₃OD) δ : 1.32 (6H, d, J=7.1Hz), 1.56-1.61 (1H, m), 1.67-1.71 (1H, m), 1.89-1.92 (1H, m), 2.03-2.07 (1H, m), 3.08-3.19 (2H, m), 3.45 (1H, s), 3.76-3.80 (1H, m), 3.84-3.88 (1H, m), 4.00 (1H, dd, J=2.9, 12.9Hz), 7.19 (1H, s), 7.34-7.36 (2H, m), 7.39 (1H, d, J=16.4Hz), 7.51 (1H, d, J=16.1Hz), 7.57 (1H, d, J=16.4Hz), 7.74 (1H, d, J=16.1Hz), 8.76 (1H, d, J=7.6Hz)
ESI-MS; m/z: 491 (M⁺+1)

### Example 105: (E)-3-{2-[(3R)-3-Hydroxyhexahydro-1-pyridinyl]-8-[(E)-2-(4-isopropyl-1,3-thiazol-2-yl)-1-ethenyl]-4-oxo-4H-pyrido[1,2-a]pyrimidin-3-yl}-2-propenoic acid

### (A) tert-Butyl (E)-3-{2-[(3R)-3-Formyloxyhexahydro-1-pyridinyl)-8-[(E)-2-(4-isopropyl-1,3-thiazol-2-yl)-1-ethenyl]-4-oxo-4H-pyrido[1,2-a]pyrimidin-3-yl}-2-propenoate

2-Hydroxy-8-[(E)-2-(4-isopropyl-1,3-thiazol-2-yl)-1-ethenyl]-4H-pyrido[1,2-a]-pyrimidin-4-one (500 mg, 1.60 mmol) was treated in the same manner as in Example 102, (F) and (G) to obtain the title compound (488 mg, 56%).
¹H-NMR (CDCl₃) δ : 1.35 (6H, d, J=6.8Hz), 1.52 (9H, s), 1.73-1.75 (1H, m), 1.83-1.85 (1H, m), 1.95-2.03 (2H, m), 3.13-3.17 (1H, m), 3.54-3.60 (2H, m), 3.71 (1H, dd, J=6.2, 13.5Hz), 3.82 (1H, dd, J=3.2, 13.5Hz), 5.14-5.16 (1H, m), 6.97 (1H, s), 7.07 (1H, d, J=15.6Hz), 7.14 (1H, dd, J=1.6, 7.6Hz), 7.34-7.37 (2H, m), 7.49 (1H, d, J=16.0Hz), 7.51 (1H, d, J=15.6Hz), 8.10 (1H, s), 8.89 (1H, d, J=7.6Hz)

### (B) tert-Butyl (E)-3-{2-[(3R)-3-Hydroxyhexahydro-1-pyridinyl]-8-[(E)-2-(4-isopropyl-1,3-thiazol-2-yl)-1-ethenyl]-4-oxo-4H-pyrido[1,2-a]pyrimidin-3-yl}-2-propenoate

The tert-butyl (E)-3-{2-[(3R)-3-formyloxyhexahydro-1-pyridinyl]-8-[(E)-2-(4-isopropyl-1,3-thiazol-2-yl)-1-ethenyl]-4-oxo-4H-pyrido[1,2-a]pyrimidin-3-yl}-2-propenoate (488 mg, 0.886 mmol) obtained in (A) was treated in the same manner as in Example 102, (H) to obtain the title compound (373 mg, 81%).
¹H-NMR (CDCl₃) δ: 1.35 (6H, d, J=6.8Hz), 1.52 (9H, s), 1.82-1.88 (4H, m), 3.11-3.18 (1H, m), 3.54-3.67 (3H, m), 3.95-3.98 (1H, m), 4.03 (1H, br s), 6.97 (1H, s), 7.05 (1H, d, J=15.7Hz), 7.16 (1H, dd, J=1.8, 7.6Hz), 7.34 (1H, d, J=16.1Hz), 7.34 (1H, s), 7.49 (1H, d, J=16.1Hz), 7.50 (1H, d, J=15.7Hz), 8.89 (1H, d, J=7.6Hz)

### (C) (E)-3-{2-[(3R)-3-Hydroxyhexahydro-1-pyridinyl]-8-[(E)-2-(4-isopropyl-1,3-thiazol-2-yl)-1-ethenyl]-4-oxo-4H-pyrido[1,2-a]pyrimidin-3-yl}-2-propenoic acid

tert-Butyl (E)-3-{2-[(3R)-3-hydroxyhexahydro-1-pyridinyl]-8-[(E)-2-(4-isopropyl-1,3-thiazol-2-yl)-1-ethenyl]-4-oxo-4H-pyrido[1,2-a]pyrimidin-3-yl}-2-propenoate (105 mg, 0.201 mmol) obtained in (B) was treated in the same manner as in Example 102, (I) to obtain the title compound (64.0 mg, 68%).
¹H-NMR (DMSO-d₆) δ : 1.27 (6H, d, J=6.8Hz), 1.40-1.45 (1H, m), 1.54-1.57 (1H, m), 1.80-1.83 (1H, m), 1.89-1.93 (1H, m), 2.96-3.19 (2H, m), 3.60 (1H, m), 3.88-3.90 (1H, m), 4.92 (1H, br s), 6.87 (1H, d, J=15.5Hz), 7.40 (1H,S), 7.43 (1H, d, J=15.5Hz), 7.54 (1H, d, J=16.1Hz), 7:58-7.59 (2H, m), 7.85 (1H, d, J=16.1Hz), 8.74 (1H, d, 8.1Hz)

### Example 106: (E)-3-{2-[(3S)-3-Hydroxyhexahydro-1-pyridinyl]-8-[(E)-2-(4-isopropyl-1,3-thiazol-2-yl)-1-ethenyl]-4-oxo-4H-pyrido[1,2-a]pyrimidin-3-yl}-2-propenoic acid

### (A) tert-Butyl (E)-3-{2-[(3S)-3-formyloxyhexahydro-1-pyridinyl]-8-[(E)-2-(4-isopropyl-1,3-thiazol-2-yl)-1-ethenyl]-4-oxo-4H-pyrido[1,2-a]pyrimidin-3-yl}-2-propenoate

2-Hydroxy-8-[(E)-2-(4-isopropyl-1,3-thiazol-2-yl)-1-ethenyl]-4H-pyrido[1,2-a]-pyrimidin-4-one(500 mg, 1.60 mmol) was treated in the same manner as in Example 102, (F) and (G) to obtain the title compound (164 mg, 19%).
¹H-NMR (CDCl₃) δ : 1.35 (6H, d, J=6.9Hz), 1.52 (9H, s), 1.73-1.75 (1H, m), 1.81-1.85 (1H, m), 1.96-2.05 (2H, m), 3.11-3.18 (1H, m), 3.54-3.57 (2H, m), 3.70 (1H, dd, J=6.5, 13.1Hz), 3.82 (1H, d, J=13.5Hz), 5.15 (1H, m), 6.96 (1H, s), 7.06 (1H, d, J=15.4Hz), 7.14 (1H, dd, J=1.6, 7.4Hz), 7.32-7.36 (2H, m), 7.48 (1H, d, J=15.9Hz), 7.51 (1H, d, J=15.4Hz), 8.10 (1H, s), 8.87 (1H, d, J=7.4Hz)

### (B) tert-Butyl (E)-3-{2-[(3S)-3-hydroxyhexahydro-1-pyridinyl]-8-[(E)-2-(4-isopropyl-1,3-thiazol-2-yl)-1-ethenyl]-4-oxo-4H-pyrido[1,2-a]pyrimidin-3-yl}-2-propenoate

The tert-butyl (E)-3-{2-[(3S)-3-formyloxyhexahydro-1-pyridinyl]-8-[(E)-2-(4-isopropyl-1,3-thiazol-2-yl)-1-ethenyl]-4-oxo-4H-pyrido[1,2-a]pyrimidin-3-yl}-2-propenoate (164 mg, 0.298 mmol) obtained in (A) was treated in the same manner as in Example 102, (H) to obtain the title compound (156 mg, 100%).
¹H-NMR (CDCl₃) δ : 1.35 (6H, d, J=6.8Hz), 1.52 (9H, s), 1.81-1.91 (4H, m), 3.11-3.18 (1H, m), 3.60-3.63 (3H, m), 3.86-3.89 (1H, m), 4.02 (1H, brs), 6.97 (1H, s), 7.04 (1H, d, J=15.6Hz), 7.13 (1H, dd, J=1.7, 7.6Hz), 7.31 (1H, d, J=1.7Hz), 7.32 (1H, d, J=16.0Hz), 7.48 (1H, d, J=16.0Hz), 7.49 (1H, d, J=15.6Hz), 8.87 (1H, d, J=7.6Hz)

### (C) (E)-3-{2-[(3S)-3-Hydroxyhexahydro-1-pyridinyl]-8-[(E)-2-(4-isopropyl-1,3-thiazol-2-yl)-1-ethenyl]-4-oxo-4H-pyrido[1,2-a]pyrimidin-3-yl}-2-propenoic acid

The tert-butyl (E)-3-{2-[(3S)-3-hydroxyhexahydro-1-pyridinyl]-8-[(E)-2-(4-isopropyl-1,3-thiazol-2-yl)-1-ethenyl]-4-oxo-4H-pyrido[1,2-a]pyrimidin-3-yl}-2-propenoate (72.7 mg, 0.139 mmol) obtained in (B) was treated in the same manner as in Example 102, (I) to obtain the title compound (35.5 mg, 55%).
¹H-NMR (DMSO-d₆) δ: 1.29 (6H, d, J=6.8Hz), 1.42-1.45 (1H, m), 1.56-1.59 (1H, m), 1.81-1:85 (1H, m), 1.93-1.95 (1H, m), 2:97-3.20 (3H, m), 3.62 (1H, m), 3.69-3.74 (1H, m), 3.89-3.92 (1H, m), 4.90 (1H, br s), 6.89 (1H, d, J=15.4Hz), 7.42-7.46 (1H, m), 7.55 (1H, d, J=16.2Hz), 7.61-7.62 (2H, m), 7.87 (1H, d, J=16.2Hz), 8.76 (1H, d, 7.6Hz), 11.84 (1H, br s)

### Example 107: (E)-3-(2-{(3R)-3-[(Aminocarbonyl)oxy)hexahydro-1-pyridinyl}-8-[(E)-2-(4-isopropyl-1,3-thiazol-2-yl)-1-ethenyl]-4-oxo-4H-pyrido[1,2-a]pyrimidin-3-yl)-2-propenoic acid

The tert-butyl (E)-3-{2-[(3R)-3-hydroxyhexahydro-1-pyridinyl]-8-[(E)-2-(4-isopropyl-1,3-thiazol-2-yl)-1-ethenyl]-4-oxo-4H-pyrido[1,2-a]pyrimidin-3-yl}-2-propenoate (109 mg, 0.209 mmol) was treated in the same manner as in Example 103, (A) and (B) to obtain the title compound (89.7 mg, 84%).
¹H-NMR (DMSO-d₆) δ : 1.29 (6H, d, J=6.9Hz), 1.66 (2H, m), 1.88-1.98 (2H, m), 3.05-3.12 (1H, m), 3.36-3.54 (3H, m), 3.86 (1H, d, J=10.0Hz), 4.60 (1H, m), 6.47 (2H, br s), 6.90 (1H, d, J=15.2Hz), 7.42 (1H, s), 7.45 (1H, d, J=15.2Hz), 7.55 (1H, d, J=16.2Hz), 7.62-7.64 (2H, m), 7.88 (1H, d, J=16.2Hz), 8.77 (1H, d, J=7.3Hz), 11.90 (1H, br s)

### Example 108: (E)-3-(2-{(3S)-3-[(Aminocarbonyl)oxy]hexahydro-1-pyridinyl}-8-[(E)-2-(4-isopropyl-1,3-thiazol-2-yl)-1-ethenyl]-4-oxo-4H-pyrido[1,2-a]pyrimidin-3-yl)-2-propenoic acid

The tert-butyl (E)-3-{2-[(3S)-3-hydroxyhexahydro-1-pyridinyl]-8-[(E)-2-(4-isopropyl-1,3-thiazol-2-yl)-1-ethenyl)-4-oxo-4H-pyrido[1,2-a]pyrimidin-3-yl}-2-propenoate (83.6 mg, 0.160 mmol) was treated in the same manner as in Example 103 (A) and (B) to obtain the title compound (59.4 mg, 73%).
¹H-NMR (DMSO-d₆) δ : 1.29 (6H, d, J=6.6Hz), 1.66 (2H, m), 1.88 (1H, m), 1.98 (1H, m), 3.06-3.13 (1H, m), 3.43-3.51 (3H, m), 3.86 (1H, d, J=11.7Hz), 4.61 (1H, m), 6.47 (2H, brs), 6.90 (1H, d, J=15.6Hz), 7.42 (1H, s), 7.46 (1H, d, J=15.6Hz), 7.55 (1H, d, J=16.1Hz), 7.62-7.64 (2H, m), 7.88 (1H, d, J=16.1Hz), 8.77 (1H, d, J=7.3Hz), 11.90 (1H, brs)

### Example 109: (E)-3-(2-{3-[(Dimethylamino)carbonyl]piperidino}-8-[(E)-2-(4-isopropyl-1,3-thiazol-2-yl)-1-ethenyl]-4-oxo-4H-pyrido[1,2-a]pyrimidin-3-yl)-2-propenoic acid (A) N³,N³-Dimethyl-1-{8-[(E)-2-(4-isopropyl-1,3-thiazol-2-yl)-1-ethenyl]-4-oxo-4H-pyrido[1,2-a]pyrimidin-2-yl}-3-piperidinecarboxamide

The 2-hydroxy-8-[(E)-2-(4-isopropyl-1,3-thiazol-2-yl)-1-ethenyl]-4H-pyrido[1,2-a]pyrimidin-4-one (164 mg, 0.523 mmol) obtained in Example 101, (E) was dissolved in anhydrous tetrahydrofuran (2.5 ml) and anhydrous dimethylformamide (2.5 ml), added with 4-dimethylaminopyridine (83.0 mg, 0.680 mmol) and p-toluenesulfonyl chloride (110 mg, 0.576 mmol), and then the mixture was stirred at 0°C for 3 hours. Subsequently, the reaction solution was added with triethylamine (729 µl, 5.23 mmol) and N³,N³-dimethyl-3-piperidinecarboxamide trifluoroacetic acid salt (707 mg, 2.62 mmol) and stirred at 0°C for 1 hour, at room temperature for 30 minutes and at 60°C for 19 hours. Then, the reaction solution was added with triethylamine (40.0 µl, 0.287 mmol) and N³,N³-dimethyl-3-piperidinecarboxamide trifluoroacetic acid salt (400 mg, 1.48 mmol) and stirred at 60°C for 5 hours. The reaction mixture was concentrated, added with saturated aqueous ammonium chloride, and then extracted with chloroform. The organic layer was dried over sodium sulfate, and the solvent was evaporated under reduced pressure. The residue was purified by silica gel column chromatography (chloroform:methanol = 100:1 → 100:3, v/v) to obtain the title compound (168 mg, 71%).
¹H-NMR (CDCl₃) δ : 1.36 (6H, d, J=6.4Hz), 1.52-1.62 (1H, m), 1.73 (1H, s), 1.81-1.97 (3H, m), 2.70-2.76 (1H, m), 2.99 (3H, s), 3.14 (3H, s), 3.04-3.18 (2H, m), 4.35 (1H, m), 4.65 (1H, m), 5.63 (1H, s), 6.95 (1H, s), 7.03 (1H, d, J=7.3Hz), 7.23 (1H, s), 7.32 (1H, d, J=16.1Hz), 7.45 (1H, d, J=16.1Hz), 8.82 (1H, d, J=7.3Hz)

### (B) tert-Butyl (E)-3-(2-{3-[(dimethylamino)carbonyl]piperidino}-8-[(E)-2-(4-isopropyl-1,3-thiazol-2-yl)-1-ethenyl]-4-oxo-4H-pyrido[1,2-a]pyrimidin-3-yl)-2-propenoate

Dimethylformamide (3:0 ml) was added with phosphorus oxychloride (104 µl, 1.12 mmol) under ice cooling and stirred at room temperature for 30 minutes. This was added to the N³,N³-dimethyl-1-{8-[(E)-2-(4-isopropyl-1,3-thiazol-2-yl)-1-ethenyl]-4-oxo-4H-pyrido[1,2-a]pyrimidin-2-yl}-3-piperidinecarboxamide (168 mg, 0.372 mmol) obtained in (A) and dissolved in dimethylformamide (4.0 ml) under ice cooling, and stirred at room temperature for 2 hours. The reaction mixture was concentrated, added with saturated aqueous sodium hydrogencarbonate, and then extracted with chloroform. The organic layer was dried over sodium sulfate, and the solvent was evaporated under reduced pressure. The residue was suspended in anhydrous tetrahydrofuran (3 ml) and anhydrous N,N-dimethylformamide (3 ml), added with (tert-butoxycarbonylmethylene)triphenylphosphorane (280 mg, 0.744 mmol), and stirred at 50°C for 37 hours. The reaction mixture was further added with (tert-butoxycarbonylmethylene)triphenylphosphorane (280 mg, 0.744 mmol), stirred at 80°C for 5 hours, and then concentrated, and the residue was purified by silica gel column chromatography (chloroform:methanol = 100:1 → 100:2, v/v) to obtain the title compound (167 mg, 78%).
¹H-NMR (CDCl₃) δ : 1.36 (6H, d, J=6.8Hz), 1.51 (9H, s), 1.65-1.68 (1H, m), 1.81-1.84 (1H, m), 1.89-1.94 (2H, m), 2.98 (3H, s), 3.04-3.17 (4H, m), 3.22 (3H, s), 4.05 (1H, m), 4.24 (1H, m), 6.96 (1H, s), 7.06 (1H, d, J=15.6Hz), 7.25 (1H, d, J=7.6), 7.34 (1H, d, J=16.1Hz), 7.46 (1H, s), 7.47 (1H, d, J=16.1Hz), 7.52 (1H, d, J=15.6), 8.89 (1H, d, J=7.6Hz)

### (C) (E)-3-(2-{3-[(Dimethylamino)carbonyl]piperidino}-8-[(E)-2-(4-isopropyl-1,3-thiazol-2-yl)-1-ethenyl]-4-oxo-4H-pyrido[1,2-a]pyrimidin-3-yl)-2-propenoic acid

The tert-butyl (E)-3-(2-{3-[(dimethylamino)carbonyl]piperidino}-8-[(E)-2-(4-isopropyl-1,3-thiazol-2-yl)-1-ethenyl]-4-oxo-4H-pyrido[1,2-a]pyrimidin-3-yl)-2-propenoate (71.0 mg, 0.123 mmol) obtained in (B) was added with trifluoroacetic acid (3.0 ml) and stirred at room temperature for 2 hours. The trifluoroacetic acid in the reaction mixture was evaporated under reduced pressure, and the residue was neutralized with 0.1 N sodium hydroxide and phosphate buffer (pH 7-8) and extracted with chloroform. The organic layer was dried over sodium sulfate, and the solvent was evaporated under reduced pressure. The residue was purified by thin layer chromatography (chloroform:methanol and chloroform:methanol:water, 8:3:1, v/v, lower layer) to obtain the title compound (39.0 mg, 61%).
¹H-NMR (DMSO-d₆) δ : 1.29 (6H, d, J=6.8Hz), 1.64 (1H, m), 1.72 (2H, m), 1.87 (1H, m), 2.84 (3H, s), 3.04 (2H, m), 3.07-3.11 (2H, m), 3.15 (3H, s), 3.92 (1H, m), 4.08 (1H, m), 6.89 (1H, d, J=15.4), 7.42 (1H, s), 7.43 (1H, d, J=15.4Hz), 7.56 (1H, d, J=16.1Hz), 7.60 (1H, s), 7.62 (1H, d, J=7.8Hz), 7.87 (1H, d, J=16.1Hz), 8.77 (1H, d, J=7.8Hz)
EI-MS; m/z: 522 (M⁺+1)

### Example 110: (E)-3-(8-[(E)-2-(4-Isopropyl-1,3-thiazol-2-yl)-1-ethenyl]-2-{3-[(methylamino)carbonyl]piperidino}-4-oxo-4H-pyrido[1,2-a]pyrimidin-3-yl)-2-propenoic acid

### (A) N³-Methyl-1-{8-[(E)-2-(4-isopropyl-1,3-thiazol-2-yl)-1-ethenyl]-4-oxo-4H-pyrido[1,2-a]pyrimidin-2-yl}-3-piperidinecarboxamide

2-Hydroxy-8-((E)-2-(4-isopropyl-1,3-thiazol-2-yl)-1-ethenyl]-4H-pyrido[1,2-a]-pyrimidin-4-one (157 mg, 0.501 mmol) was dissolved in anhydrous tetrahydrofuran (2.5 ml) and anhydrous dimethylformamide (2.5 ml), added with 4-dimethylaminopyridine (80.0 mg, 0.651 mmol) and p-toluenesulfonyl chloride (105 mg, 0.551 mmol), and stirred at 0°C for 1.5 hours. Subsequently, the reaction mixture was added with triethylamine (698 µ l, 5.01 mmol) and N3-methyl-3-piperidinecarboxamide hydrochloride (448 mg, 2.50 mmol), and then the mixture was stirred at 60°C for 5.5 hours. The reaction mixture was concentrated, added with saturated aqueous ammonium chloride, and then extracted with chloroform. The organic layer was dried over sodium sulfate, and the solvent was evaporated under reduced pressure. The residue was purified by silica gel column chromatography (chloroform:methanol = 100:2 → 100:3, v/v) to obtain the title compound (174 mg, 79%).
¹H-NMR (CDCl₃) δ: 1.35 (6H, d, J=6.8Hz), 1.60 (1H, m), 1.73 (1H, m), 1.94 (1H, m), 2.02-2.05 (1H, m), 2.36-2.38 (1H, m), 2.83 (3H, d, J=4.9), 3.13-3.20 (2H, m), 3.59 (1H, dd, J=8.8, 13.7), 4.00 (1H, m), 4.24 (1H, m), 5.62 (1H, s), 6.95 (1H, s), 7.05 (1H, d, J=7.8Hz), 7.27 (1H, s), 7.33 (1H, d, J=16.1Hz), 7.46 (1H, d, J=16.1Hz), 8.83 (1H, d, J=7.8Hz)

### (B) tert-Butyl (E)-3-(8-[(E)-2-(4-isopropyl-1,3-thiazol-2-yl)-1-ethenyl]-2-{3-[(methylamino)carbonyl]piperidino}-4-oxo-4H-pyrido[1,2-a]pyrimidin-3-yl)-2-propenoate

Dimethylformamide (3.0 ml) was added with phosphorus oxychloride (111 µl, 1:19 mmol) under ice cooling and stirred at room temperature for 30 minutes. The mixture was added to the N³-methyl-1-{8-[(E)-2-(4-isopropyl-1,3-thiazol-2-yl)-1-ethenyl]-4-oxo-4H-pyrido[1,2-a)pyrimidin-2-yl}-3-piperidinecarboxamide (174 mg, 0.398 mmol) obtained in (A) and dissolved in dimethylformamide (4.0 ml) under ice cooling, and then the mixture was stirred at room temperature for 2 hours. The reaction mixture was concentrated, added with saturated aqueous sodium hydrogencarbonate, and then extracted with chloroform. The organic layer was dried over sodium sulfate, and the solvent was evaporated under reduced pressure. The residue was suspended in anhydrous tetrahydrofuran (3 ml) and dimethylformamide (3 ml), added with (tert-butoxycarbonylmethylene)triphenylphosphorane (449 mg, 1.19 mmol) and stirred at 75°C for 24 hours. The reaction mixture was concentrated, and the residue was purified by silica gel column chromatography (chloroform:methanol = 100:0 → 100:3, v/v) to obtain the title compound (192 mg, 86%).
¹H-NMR (CDCl₃) δ : 1.35 (6H, d, J=7.1Hz), 1.54 (9H, m), 1.65-1.68 (1H, m), 1.85-1.90 (1H, m), 1.95-2.00 (2H, m), 2.77 (3H, d, J=4.6), 2.82 (1H, m), 2.92 (1H, m), 3.11-3.17 (2H, m), 3.90-4.00 (1H, m), 4.45-4.50 (1H, m), 6.97 (1H, s), 7.09 (1H, d, J=15.6Hz), 7.12 (1H, d, J=1.7), 7.30 (1H, d, J=1.7Hz), 7.34 (1H, d, J=16.1Hz), 7.40-7.70 (2H, m), 8.86 (1H, d, J=7.3Hz)

### (C) (E)-3-(8-[(E)-2-(4-Isopropyl-1,3-thiazol-2-yl)-1-ethenyl]-2-{3-[(methylamino)-carbonyl]piperidino}-4-oxo-4H-pyrido[1,2-a]pyrimidin-3-yl)-2-propenoic acid

The tert-butyl (E)-3-(8-[(E)-2-(4-isopropyl-1,3-thiazol-2-yl)-1-ethenyl]-2-{3-[(methylamino)carbonyl]piperidino}-4-oxo-4H-pyrido[1,2-a]pyrimidin-3-yl)-2-propenoate (192 mg, 0.341 mmol) obtained in (B) was added with trifluoroacetic acid (5.0 ml) and stirred at room temperature for 1.5 hours. After the reaction was completed, the trifluoroacetic acid was evaporated under reduced pressure, and the residue was neutralized with 0.1 N sodium hydroxide and phosphate buffer (pH 7-8) and extracted with chloroform. The organic layer was dried over sodium sulfate, and the solvent was evaporated under reduced pressure. The residue was purified by thin layer chromatography (chloroform:methanol and chloroform:methanol:water = 8:3:1, v/v, lower layer) to obtain the title compound (49.0 mg, 28%).
¹H-NMR (DMSO-d₆) δ : 1.29 (6H, d, J=6.8Hz), 1.62-1.71 (2H, m), 1.77 (1H, m), 1.90 (1H, m), 2.58 (3H, d, J=4.6Hz), 3.05-3.13 (3H, m), 3.27 (1H, m), 3.89 (1H, m), 4.08 (1H, m), 6.89 (1H, d, J=15.6Hz), 7.42 (1H, s), 7.42 (1H, d, J=15.6Hz), 7.54 (1H, d, J=16.1Hz), 7.62 (1H, d, J=7.6Hz), 7.67 (1H, s), 7.79 (1H, s), 7.88 (1H, d, J=16.1Hz), 8.77 (1H, d, J=7.6Hz)
EI-MS; m/z: 508 (M⁺+1)

### Example 111: 8-(E)-2-[4-(tert-Butyl)-1,3-thiazol-2-yl]-1-ethenyl-3-(2H-1,2,3,4-tetrazol-5-yl)-4H-pyrido[1,2-a]pyrimidin-4-one

### (A) [4-(tert-Butyl)-1,3-thiazol-2-yl]methanol

Ethyl thiooxamate (4.94 g, 37.1 mmol) was dissolved in anhydrous ethanol (250 ml), added with 1-bromopinacolone (4.99 ml, 37.1 mmol), and refluxed by heating for 4 hours. The reaction mixture was concentrated, neutralized with saturated aqueous sodium hydrogencarbonate, and extracted with ethyl acetate. The organic layer was dried over sodium sulfate, and then the solvent was evaporated under reduced pressure. The residue was dissolved in anhydrous ethanol (200 ml), added with sodium hydroboride (2.10 g, 55.6 mmol), and stirred at room temperature for 16 hours. The reaction solution was further added with sodium borohydride (500 mg, 13.2 mmol) and stirred at room temperature for 4 hours, and then the solvent was evaporated under reduced pressure. The residue was cooled to -78°C, and the resulting white solid was taken by filtration to obtain the title compound (2.77 g, 44%). ¹H-NMR (CDCl₃) δ : 1.33 (9H, s), 4.92 (2H, s), 6.86 (1H, s)

### (B) 4-(tert-Butyl)-1,3-thiazol-2-carbaldehyde

[4-(tert-Butyl)-1,3-thiazol-2-yl]methanol (2.77 g, 16.2 mmol) obtained in (A) was dissolved in methylene chloride (45 ml), added with pyridinium dichromate (12.2 g, 32.3 mmol), and stirred at room temperature for 13.5 hours. After insoluble solids were removed, the reaction solution was evaporated, and the residue was purified by silica gel column chromatography (dichloromethane) to obtain the title compound (2.71 g, 99%).
¹H-NMR (CDCl₃) δ : 1.40 (9H, s), 7.36 (1H, s), 9.98 (1H, s)

### (C) tert-Butyl N-(4-{2-[4-(tert-butyl)-1,3-thiazol-2-yl]-2-hydroxyethyl}-2-pyridyl)-carbamate

tert-Butyl N-(4-methyl-2-pyridyl)carbamate (3.11 g, 14.9 mmol) was dissolved in anhydrous tetrahydrofuran (50 ml), added with n-butyl lithium (1.59 M in n-hexane, 19.7 ml, 31.3 mmol) at -78°C under argon atmosphere, and stirred at room temperature for 1.5 hours. The reaction mixture was cooled to -78°C again, then added with a solution of the 4-(tert-butyl)-1,3-thiazol-2-carbaldehyde (2.30 g, 13.6 mmol) obtained in (B) in anhydrous tetrahydrofuran (10 ml), and stirred for 30 minutes. The reaction mixture was added with saturated aqueous ammonium chloride, warmed to room temperature and extracted with chloroform. The organic layer was dried over sodium sulfate, and the solvent was evaporated under reduced pressure. The residue was purified by silica gel column chromatography (n-hexane:ethyl acetate = 2:1 → 1:1, v/v) to obtain the title compound (2.29 g, 45%). ¹H-NMR (CDCl₃) δ: 1.32 (9H, s), 1.53 (9H, s), 3.08 (1H, dd, J=8.4, 13.8Hz), 3.29 (1H, dd, J=4.2, 13.8), 5.23 (1H, dd, J=4.2, 8.4Hz), 6.83 (1H, s), 6.85 (1H, dd, J=1.5, 5.1Hz), 7.67 (1H, s), 7.87 (1H, s), 8.13 (1H, d, J=5.1Hz)

### (D) tert-Butyl N-(4-(E)-{2-[4-(tert-butyl)-1,3-thiazol-2-yl)-1-ethenyl}-2-pyridyl)-carbamate

The tert-butyl N-(4-{2-[4-(tert-butyl)-1,3-thiazol-2-yl]-2-hydroxyethyl}-2-pyridyl)carbamate (1.80 g, 4.77 mmol) obtained in (C) was dissolved in anhydrous tetrahydrofuran (20 ml), added with triethylamine (2.33 ml, 16.7 mmol) and methanesulfonyl chloride (738 µl, 9.54 mmol), and stirred at 0°C for 3.5 hours. After insoluble solids were removed, the reaction mixture was washed with tetrahydrofuran, and the solvent was evaporated under reduced pressure. The residue was dissolved in toluene (30 ml), added with 1,8-diazabicyclo[5.4.0]undec-7-ene (1.07 ml, 7.15 mmol), and refluxed by heating for 1 hour. The reaction mixture was concentrated, and the residue was purified by silica gel column chromatography (n-hexane:ethyl acetate = 3:1 → 2:1, v/v) to obtain the title compound (1.23 g, 72%).
¹H-NMR (CDCl₃) δ : 1.37 (9H, s), 1.55 (9H, s), 6.89 (1H, s), 7.08 (1H, dd, J=1.5, 5.4Hz), 7.29 (1H, d, J=16.4Hz), 7.51 (1H, d, J=16.4Hz), 8.12 (1H, s), 8.09 (1H, s), 8.21 (1H, d, J=5.4Hz)

### (E) 4-(E)-2-[4-(tert-Butyl)-1,3-thiazol-2-yl]-1-ethenyl-2-pyridinamine

The tert-butyl N-(4-(E)-2-[4-(tert-butyl)-1,3-thiazol-2-yl]-1-ethenyl-2-pyridyl)-carbamate (585 mg, 1.63 mmol) obtained in (D) was dissolved in trifluoroacetic acid (15 ml) and stirred at room temperature for 1 hour. The reaction mixture was concentrated, neutralized with saturated aqueous sodium hydrogencarbonate, and extracted with chloroform. The organic layer was dried over sodium sulfate, and the solvent was evaporated under reduced pressure to obtain the title compound (422 mg, 100%).
¹H-NMR (CDCl₃) δ : 1.38 (9H, s), 6.62 (1H, s), 6.84 (1H, d, J=6.0Hz), 6.93 (1H, s), 7.17 (1H, d, J=16.2Hz), 7.42 (1H, d, J=16.2Hz), 7.92 (1H, d, J=6.0Hz)

### (F) 8-(E)-2-[4-(tert-Butyl)-1,3-thiazol-2-yl]-1-ethenyl-3-(2H-1,2,3,4-tetrazol-5-yl)-4H-pyrido[1,2-a]pyrimidin-4-one

Ethyl 3-(dimethylamino)-2-[2-(4-methoxybenzyl)-2H-1,2,3,4-tetrazol-5-yl]-2-propenoate (150 mg, 0.453 mmol) was dissolved in propionic acid (2.0 ml), added to the 4-(E)-2-[4-(tert-butyl)-1,3-thiazol-2-yl]-1-ethenyl-2-pyridinamine (98.0 mg, 0.378 mmol) obtained in (E), and refluxed by heating for 3 hours. The reaction mixture was concentrated, and the residue was purified by silica gel column chromatography (chloroform) to obtain the title compound (175 mg, 93%).
¹H-NMR (CDCl₃) δ: 1.40 (9H, s), 3.79 (3H, s), 5.81 (2H, s), 6.89 (2H, d, J=8.3Hz), 7.02 (1H, s), 7.41 (1H, d, J=16.2Hz), 7.42 (2H, d, J=8.3Hz), 7.46 (1H, d, J=7.5Hz), 7.60 (1H, d, J=16.2Hz), 7.74 (1H, s), 9.21 (1H, d, J=7.5Hz), 9.21 (1H, s)

### (G) 8-(E)-2-[4-(tert-Butyl)-1,3-thiazol-2-yl]-1-ethenyl-3-(2H-1,2,3,4-tetrazol-5-yl)-4H-pyrido[1,2-a]pyrimidin-4-one

The 8-(E)-2-[4-(tert-butyl)-1,3-thiazol-2-yl]-1-ethenyl-3-(2H-1,2,3,4-tetrazol-5-yl)-4H-pyrido[1,2-a]pyrimidin-4-one (170 mg, 0.340 mmol) obtained in (F) was added with trifluoroacetic acid (4.0 ml) and stirred at 60°C for 3 hours. After the reaction was completed, the trifluoroacetic acid was evaporated under reduced pressure, and the residue was neutralized with 0.1 N sodium hydroxide and phosphate buffer (pH 7-8) and extracted with chloroform. The organic layer was dried over sodium sulfate, and the solvent was evaporated under reduced pressure. The yellow solid deposited from the residue was collected by filtration to obtain the title compound (20.0 mg, 15%).
¹H-NMR (DMSO-d₆) δ: 1.35 (9H, s), 7.47 (1H, s), 7.67 (1H, d, J=16.1Hz), 8.03 (1H, d, J=16.1Hz), 8.04 (1H, d, J=1.9Hz), 8.13 (1H, d, J=1.9Hz), 9.11 (1H, d, J=7.3Hz), 9.17 (1H, s)
EI-MS; m/z: 380 (M⁺+1)

### Example 112: (E)-3-{8-[2-(4-Isopropyl-1,3-thiazol-2-yl)ethyl]-4-oxo-2-(tetrahydro-3-furanylmethoxy)-4H-pyrido[1,2-a]pyrimidin-3-yl}-2-propenoic acid

### (A) tert-Butyl (E)-3-{8-[2-(4-isopropyl-1,3-thiazol-2-yl)ethyl]-4-oxo-2-(tetrahydro-3-furanylmethoxy)-4H-pyrido[1,2-a]pyrimidin-3-yl}-2-p ropenoate

tert-Butyl (E)-3-(2-hydroxy-8-[2-(4-isopropyl-1,3-thiazol-2-yl)ethyl]-4-oxo-4H-pyrido[1,2-a]pyrimidin-3-yl)-2-propenoate (17 mg) was added with dimethylformamide (3 ml), diisopropylethylamine (500 ml) and 3-(iodomethyl)tetrahydrofuran (50 ml), and then the mixture was stirred at 80°C for 16 hours. The reaction mixture was added with ethyl acetate and saturated brine, and the organic layer was further washed twice with saturated brine and dried over magnesium sulfate. After the solvent was evaporated under reduced pressure, the residue was purified by silica gel column chromatography to obtain the title compound (14 mg).
¹H-NMR (CDCl₃) δ : 1.29 (6H, d, J=7.1Hz), 1.52 (9H, s), 1.75 (1H, m), 2.16 (1H, m), 2.82 (1H, m), 3.07 (1H, m), 3.26 (2H, m), 3.39 (2H, m), 3.71 (1H, m), 3.80 (1H, m), 3.92 (2H, m), 4.42 (2H, m), 6.74 (1H, s), 7.02 (1H, m), 7.13 (1H, d, J=15.8Hz), 7.34 (1H, s), 7.91 (1H, d, J=15.8Hz), 8.99 (1H, d, J=7.3Hz)

### (B) (E)-3-{8-[2-(4-Isopropyl-1,3-thiazol-2-yl)ethyl]-4-oxo-2-(tetrahydro-3-furanylmethoxy)-4H-pyrido[1,2-a]pyrimidin-3-yl}-2-propenoic acid

The tert-butyl (E)-3-{8-[2-(4-isoprogyl-1,3-thiazol-2-yl)ethyl]-4-oxo-2-(tetrahydro-3-furanylmethoxy)-4H-pyrido[1,2-a]pyrimidin-3-yl}-2-propenoate (10 mg) obtained in (A) was dissolved in methylene chloride (1 ml) and formic acid (1 ml), and then the mixture was stirred for 5 hours. After the solvent was evaporated under reduced pressure, the residue was purified by silica gel column chromatography to obtain the title compound (14 mg).
¹H-NMR (CDCl₃) δ : 1.29 (6H, d, J=6.6Hz), 1.79 (1H, m), 2.16 (1H, m), 2.83 (1H, m), 3.07 (1H, m), 3.26 (2H, m), 3.39 (2H, m), 3.73 (2H, m), 3.81 (1H, m), 3.93 (2H, m), 4.42 (2H, m), 6.73 (1H, s), 7.02 (1H, m), 7.15 (1H, d, J=15.8Hz), 7.33 (1H, s), 8.05 (1H, d, J=15:8Hz), 8.99 (1H, d, J=6.5Hz)

### Example 113: 8-[2-(4-Isopropyl-1,3-thiazol-2-yl)ethyl]-3-[(E)-2-(1H-1,2,3,4-tetrazol-5-yl)-1-ethenyl]-2-(tetrahydro-3-furanylmethoxy)-4H-pyrido[1,2-a]pyrimidin-4-one

### (A) (E)-2-{8-[2-(4-Isopropyl-1,3-thiazol-2-yl)ethyl]-4-oxo-2-(tetrahydro-3-furanylmethoxy)-4H-pyrido[1,2-a]pyrimidin-3-yl}-1-ethenylcyanide

(E)-2-{2-Hydroxy-8-[2-(4-isopropyl-1,3-thiazol-2-yl)ethyl]-4-oxo-4H-pyrido[1,2-a]pyrimidin-3-yl}-1-ethenylcyanide (100 mg) was added with dimethylformamide (5 ml), diisopropylethylamine (500 ml) and 3-(iodomethyl)tetrahydrofuran (200 ml), and then the mixture was stirred at 80°C for 2 hours and further stirred overnight at room temperature. After the reaction mixture was distributed between chloroform and water, the organic layer was dried over magnesium sulfate. After the solvent was evaporated under reduced pressure, the residue was purified by silica gel column chromatography to obtain the title compound (51 mg).
¹H-NMR (CDCl₃) δ : 1.29 (6H, d, J=6.8Hz), 1.78 (1H, m), 2.16 (1H, m), 2.79 (1H, m), 3.05 (1H, m), 3.29 (2H, m), 3.38 (2H, m), 3.71 (1H, m), 3.82 (1H, m), 3.93 (2H, m), 4.42 (2H, m), 6.74 (1H, s), 6.68 (1H, d, J=16.3Hz), 7.07 (1H, d, J=7.3Hz), 7.37 (1H, s), 7.64 (1H, d, J=16.3Hz), 8.97 (1H, d, J=7.3Hz)

### (B) 8-[2-(4-Isopropyl-1,3-thiazol-2-yl)ethyl]-3-[(E)-2-(1H-1,2,3,4-tetrazol-5-yl)-1-ethenyl]-2-(tetrahydro-3-furanylmethoxy)-4H-pyrido[1,2-a]pyrimidin-4-one

¹H-NMR (CDCl₃) δ : 1.29 (6H, d, J=6.8Hz), 1.80 (1H, m), 2.18 (1H, m), 2.86 (1H, m), 3.08 (1H, m), 3.27 (2H, m), 3.42 (2H, m), 3.80 (2H, m), 3.96 (2H, m), 4.47 (2H, m), 6.76 (1H, s), 7.04 (1H, m), 7.34 (1H, s), 7.90 (1H, d, J=16.3Hz), 8.04 (1H, d, J=16.3Hz), 8.99 (1H, d, J=7.0Hz)

### Example 114: (E)-3-{8-[2-(4-Isopropyl-1,3-thiazol-2-yl)ethyl]-4-oxo-2-(2-pyridylmethoxy)-4H-pyrido[1,2-a]pyrimidin-3-yl}-2-propenoic acid

¹H-NMR (CD₃OD-CDCl₃) δ : 1.42 (6H, d, J=6.8Hz), 3.29 (1H, m), 3.40 (2H, m), 3.77 (2H, m), 5.96 (2H, s), 7.18 (1H, d, J=15.9Hz), 7.37 (1H, m), 7.44 (1H, s), 7.80 (1H, s), 8.02 (1H, d, J=15.9Hz), 8.07 (1H, m), 8.31 (1H, m), 8.68 (1H, m), 8.92 (1H, m), 9.04 (1H, d, J=7.3Hz)

### Example 115: (E)-3-{8-[2-(4-Isopropyl-1,3-thiazol-2-yl)ethyl]-4-oxo-2-(3-pyridylmethoxy)-4H-pyrido[1,2-a]pyrimidin-3-yl}-2-propenoic acid

¹H-NMR (CD₃OD-CDCl₃) δ : 1.42 (6H, d, J=6.8Hz), 3.29 (1H, m), 3.45 (2H, m), 3.83 (2H, m), 5.85 (2H, s), 7.07 (1H, d, J=15.9Hz), 7.39 (1H, d, J=7.3Hz), 7.46 (1H, s), 7.78 (1H, s), 7.93 (1H, d, J=15.9Hz), 8.18 (1H, m), 8.82 (2H, m), 9.01 (1H, d, J=7.1Hz), 9.24 (1H, s)

### Example 116: (E)-3-{8-[2-(4-Isopropyl-1,3-thiazol-2-yl)ethyl]-4-oxo-2-(4-pyridylmethoxy)-4H-pyrido[1,2-a]pyrimidin-3-yl}-2-propenoic acid

¹H-NMR (CD₃OD) δ : 1.35 (6H, d, J=6.8Hz), 3.29 (1H, m), 3.37 (2H, m), 3.70 (2H, m), 5.97 (2H, s), 7.16 (1H, d, J=15.9Hz), 7.38 (1H, d, J=7.0Hz), 7.52 (2H, s), 8.08 (1H, d, J=15.9Hz), 8.20 (2H, m), 8.88 (2H, m), 9.06 (1H, d, J=7.0Hz)

### Example 117: (E)-3-{8-[(E)-2-(4-Isopropyl-1,3-thiazol-2-yl)-1-ethenyl]-4-oxo-2-(tetrahydro-3-furanylmethoxy)-4H-pyrido[1,2-a]pyrimidin-3-yl}-2-propenoic acid

### (A) 8-[(E)-2-(4-Isopropyl-1,3-thiazol-2-yl)-1-ethenyl]-2-(tetrahydro-3-furanylmethoxy)-4H-pyrido[1,2-a]pyrimidin-4-one

2-Hydroxy-8-[(E)-2-(4-isopropyl-1,3-thiazol-2-yl)-1-ethenyl]-4H-pyrido[1,2-a]-pyrimidin-4-one (210 mg, 0.670 mmol) was suspended in dimethylformamide (6.0 ml), added with diisopropylethylamine (600 µl) and 3-(iodomethyl)tetrahydrofuran (334 µl), and stirred overnight at 80°C. The reaction solution was further added with diisopropylethylamine (600 µl) and 3-(iodomethyl)tetrahydrofuran (334 µl) and stirred overnight at 80°C. The reaction mixture was concentrated, and the residue was purified by silica gel column chromatography (chloroform:methanol = 100:0 → 100:1 → 100:2 → 100:5, v/v) to obtain the title compound (277 mg, quantitative).

### (B) tert-Butyl (E)-3-{8-[(E)-2-(4-isopropyl-1,3-thiazol-2-yl)-1-ethenyl]-4-oxo-2-(tetrahydro-3-furanylmethoxy)-4H-pyrido[1,2-a]pyrimidin-3-yl}-2-propenoate

Dimethylformamide (3.0 ml) was added with phosphorus oxychloride (187 µl, 2.02 mmol) under ice cooling and stirred at room temperature for 30 minutes. The mixture was added to 8-[(E)-2-(4-isopropyl-1,3-thiazol-2-yl)-1-ethenyl]-2-(tetrahydro-3-furanylmethoxy)-4H-pyrido[1,2-a]pyrimidin-4-one (277 mg) obtained in (A) and dissolved in dimethylformamide (3.0 ml) under ice cooling, and then the mixture was stirred at room temperature for 4 hours. The reaction mixture was added with saturated aqueous sodium hydrogencarbonate and extracted with chloroform. The organic layer was dried over magnesium sulfate, and the solvent was evaporated under reduced pressure. The residue was suspended in anhydrous tetrahydrofuran (6.0 ml) and anhydrous N,N-dimethylformamide (3.0 ml), added with (tert-butoxycarbonylmethylene)triphenylphosphorane (505 mg, 1.34 mmol) and stirred at 50°C for 16 hours. The reaction mixture was concentrated, and then the residue was purified by silica gel column chromatography (n-hexane:ethyl acetate, 5:1 → 3:1 → 2:1 → 1:1, v/v) to obtain the title compound (215 mg, 61%).
¹H-NMR (CDCl₃) δ : 1.36 (6H, d, J=6.8Hz), 1.53 (9H, s), 1.80 (1H, dt, J=7.3, 12.8Hz), 2.12-2.21 (1H, m), 2.80-2.87 (1H, m), 3.12-3.19 (1H, m), 3.73 (1H, dd, J=5.4, 8.8Hz), 3.81 (1H, dd, J=7.7, 15.2Hz), 3.91-3.97 (2H, m), 4.44-4.47 (2H, m), 6.99 (1H, s), 7.16 (1H, d, J=16.3Hz), 7.31 (1H, d, J=1.8, 7.5Hz), 7.39 (1H, d, J=16.2Hz), 7.48 (1H, d, J=1.5Hz), 7.54 (1H, d, J=16.2Hz), 7.92 (1H, d, J=16.3Hz), 9.04 (1H, d, J=7.5Hz)
ESI-MS; m/z: 524 (M⁺+1)

### (C) (E)-3-{8-[(E)-2-(4-Isopropyl-1,3-thiazol-2-yl)-1-ethenyl]-4-oxo-2-(tetrahydro-3-furanylmethoxy)-4H-pyrido[1,2-a]pyrimidin-3-yl}-2-propenoic acid

The tert-butyl (E)-3-{8-[(E)-2-(4-isopropyl-1,3-thiazol-2-yl)-1-ethenyl]-4-oxo-2-(tetrahydro-3-furanylmethoxy)-4H-pyrido[1,2-a]pyrimidin-3-yl}-2-propenoate (101 mg, 0.193 mmol) obtained in (B) was dissolved in 1,4-dioxane (1.6 ml), added with 4 N hydrochloric acid in 1,4-dioxane (3.2 ml), and then the mixture was stirred at room temperature for 16.25 hours. The reaction solution was concentrated, added with saturated aqueous sodium hydrogencarbonate, neutralized with phosphate buffer (pH 7-8), and then extracted with a mixture of chloroform/methanol (10:1, v/v). The organic layer was dried over magnesium sulfate, and then the solvent was evaporated under reduced pressure. The residue was purified by thin layer chromatography (chloroform:methanol = 20:1, v/v) to obtain the title compound (77.1 mg, 86%).
¹H-NMR (CDCl₃ + CD₃OD) δ : 1.36 (6H, d, J=6.8Hz), 1.17-1.85 (1H, m), 2.13-2.22 (1H, m), 2.82-2.89 (1H, m), 3.13-3.19 (1H, m), 3.74 (1H, dd, J=5.4, 8.8Hz), 3.82 (1H, dd, J=7.7, 15.5Hz), 3.93-3.98 (2H, m), 4.44-4.52 (2H, m), 7.01 (1H, s), 7.16 (1H, d, J=15.9Hz), 7.36-7.41 (2H, m), 7.50 (1H, s), 7.57 (1H, d, J=16.1Hz), 8.03 (1H, d, J=15.9Hz), 9.03 (1H, d, J=7.3Hz)
ESI-MS; m/z: 468 (M⁺+1)

### Example 118: 8-[2-(4-Isopropyl-1,3-thiazol-2-yl)ethyl]-2-morpholino-3-(1H-1,2,3,4-tetrazol-5-yl)-4H-pyrido[1,2-a]pyrimidin-4-one

### (A) 8-[2-(4-Isopropyl-1,3-thiazol-2-yl)ethyl]-2-hydroxy-3-[(4-methoxybenzyl)tetrazol-5-yl]-4H-pyrido[1,2-a]pyrimidin-4-one

4-[2-(4-Isopropyl-1,3-thiazol-2-yl)ethyl]-2-aminopyridine (50 mg) and diethyl 2-[1-(4-methoxybenzyl)tetrazol-5-yl]malonate (141 mg) were heated to 150° C in bromobenzene with stirring for 4 hours. After the solvent was evaporated, the residue was purified by silica gel column chromatography to obtain the title compound (73 mg) as cream solid.
¹H-NMR (CDCl₃) δ : 1.22 (d, J=6.6Hz, 6H), 3.02 (m, 1H), 3.20-3.40 (m, 4H), 3.70 (s, 3H), 5.80 (s, 2H), 6.70 (s, 1H), 6.80 (bs, 2H), 7.00 (s, 1H), 7.30-7.50 (m, 3H), 9.00 (bs, 1H)
MS(-), m/z, 502 (M-H⁺)

### (B) 8-[2-(4-Isopropyl-1,3-thiazol-2-yl)ethyl]-2-tosyloxy-3-[(4-methoxybenzyl)tetrazol-5-yl]-4H-pyrido[1,2-a]pyrimidin-4-one

The 8-[2-(4-isopropyl-1,3-thiazol-2-yl)ethyl]-2-hydroxy-3-[(4-methoxybenzyl)-tetrazol-5-yl]-4H-pyrido[1,2-a]pyrimidin-4-one(73 mg) obtained in (A) was dissolved in methylene chloride (2 ml), added with dimethylaminopyridine (5 mg), triethylamine(40 ml) and p-toluenesulfonyl chloride (83 mg) at 0°C, and then the mixture was stirred at room temperature for 6 hours. The reaction solution was directly purified by silica gel column chromatography to obtain the title compound (61 mg, 64%) as pale yellow powder.
¹H-NMR (CDCl₃) δ : 1.22 (d, J=6.6Hz, 6H), 2.42 (s, 3H), 3.05 (m, 1H), 3.25-3.32 (m, 4H), 3.80 (s, 3H), 5.75 (s, 2H), 6.75 (s, 1H), 6.85 (d, J=7.2Hz, 2H), 7.15 (d, J=7.5Hz, 1H), 7.24 (d, J=7.2Hz, 2H), 7.35 (d, J=7.2Hz, 2H), 7.42 (s, 1H), 7.90 (d, J=7.2Hz, 2H), 9.01 (d, J=7.5Hz, 1H)

### (C) 8-[2-(4-Isopropyl-1,3-thiazol-2-yl)ethyl]-2-morpholino-3-(1-p-methoxybenzyl-1,2,3,4-tetrazol-5-yl)-4H-pyrido[1, 2-a]pyrimidin-4-one

The 8-[2-(4-isopropyl-1,3-thiazol-2-yl)ethyl]-2-tosyloxy-3-[(4-methoxybenzyl)-tetrazol-5-yl]-4H-pyrido[1,2-a]pyrimidin-4-one (61 mg) obtained in (B) was dissolved in tetrahydrofuran (1 ml), added with morpholine (40 ml) at room temperature, and stirred overnight at the same temperature. After the solvent was evaporated under reduced pressure, the residue was purified by silica gel column chromatography to obtain the title compound (31 mg, 59%).
¹H-NMR (CDCl₃) δ: 1.28 (d, J=6.6Hz, 6H), 3.06 (m, 1H), 3.30-3.70 (m, 12H), 3.78 (s, 3H), 5.76 (s, 2H), 6.72 (s, 1H), 6.77 (d, J=7.5Hz, 1H), 6.86 (d, J=7.2Hz, 2H), 7.14 (s, 1H), 7.36 (d, J=7.2Hz, 2H), 8.88 (m, J=7.5Hz, 1H)

### (D) 8-[2-(4-Isopropyl-1,3-thiazol-2-yl)ethyl]-2-morpholino-3-(1H-1,2,3,4-tetrazol-5-yl)-4H-pyrido[1,2-a]pyrimidin-4-one

The 8-[2-(4-isopropyl-1,3-thiazol-2-yl)ethyl]-2-morpholino-3-(1-p-methoxybenzyl-1,2,3,4-tetrazol-5-yl)-4H-pyrido[1,2-a]pyrimidin-4-one (10 mg) obtained in (C) was dissolved in trifluoroacetic acid (1 ml) and added with anisole (50 ml), and then stirred at room temperature for 4 hours. After the solvent was evaporated, the residue was purified by silica gel column chromatography to obtain the title compound (7 mg) as yellow powder.
¹H-NMR (CDCl₃) δ: 1.25 (d, 6H), 3.04 (m, 1H), 3.22 (t, 2H), 3.38 (t, 2H), 3.66 (m, 4H), 3.82 (m, 4H), 6.73 (s, 1H), 6.89 (m, 1H), 7.22 (m, 1H), 8.83 (d, 1H)
MS (ES-) m/z 451 (M⁺-1)

In the following Examples 119 to 121, synthesis was performed in the same manner as in Example 118.

### Example 119: 8-[2-(4-Isopropyl-1,3-thiazol-2-yl)ethyl]-2-(3-pyridylmethoxy)-3-(1H-1,2,3,4-tetrazol-5-yl)-4H-pyrido[1,2-a]pyrimidin-4-one

¹H-NMR (CDCl₃) δ : 1.21 (d, 6H), 2.98 (m, 1H), 3.25 (t, 2H), 3.36 (t, 2H), 5.73 (s, 2H), 6.71 (s, 1H), 7.16 (d, 1H), 7.31 (m, 1H), 7.42 (s, 1H), 8.10 (d, 1H), 8.41 (d, 1H), 8.69 (s, 1H), 8.99 (d, 1H)
MS (ES+) m/z 475 (M⁺+1); MS (ES-) m/z 473 (M⁺-1)

### Example 120: 8-[2-(4-Isopropyl-1,3-thiazol-2-yl)ethyl]-2-dimethylamino-3-(1H-1,2,3,4-tetrazol-5-yl)-4H-pyrido[1,2-a]pyrimidin-4-one

¹H-NMR (CD₃OD) δ : 1.24 (d, 6H), 2.95 (s, 6H), 3.01 (m, 1H), 3.21 (t, 2H), 3.41 (t, 2H), 6.95 (s, 1H), 7.00 (m, 1H), 7.23 (s, 1H), 8.80 (d, 1H)
MS (ES+) m/z 512 (M⁺+1); MS (ES-) m/z 510 (M⁺-1)

### Example 121: 8-[2-(4-Isopropyl-1,3-thiazol-2-yl)ethyl]-2-(4-cyanopiperidino)-3-(1H-1,2,3,4-tetrazol-5-yl)-4H-pyrido[1,2-a]pyrimidin-4-one

¹H-NMR (CDCl₃) δ : 1.33 (d, 6H), 2.02 (m, 2H), 2.10 (m, 2H), 2.95 (m, 1H), 3.20 (m, 4H), 3.45 (m, 2H), 3.62 (m, 1H), 3.79 (m, 2H), 6.82 (s, 1H), 6.90 (d, 1H), 7.18 (s, 1H), 8.85 (d, 1H)
MS (ES-) m/z 476 (M⁺+1); 474 (M⁺-1)

### Example 122: 4-Isopropyl-2-(2-(3-[([(4-methylphenyl)sulfonyl]aminocarbonyl)amino]-4-oxo-4H-pyrido[1,2-a]pyrimidin-8-yl)ethyl)-1,3-thiazole

### (A) tert-Butyl N-8-[2-(4-isopropyl-1,3-thiazol-2-yl)ethyl]-4-oxo-4H-pyrido[1,2-a]-pyrimidin-3-ylcarbamate

4-[2-(4-Isopropyl-1,3-thiazol-2-yl)ethyl]-2-aminopyridine (300 mg) and methyl 2-(t-butoxycarbonyl)amino-3-dimethylaminopropenoate (445 mg) were added with xylene (2 ml) and heated at 140°C for 6.5 hours with stirring. After the solvent was evaporated, the residue was purified by silica gel column chromatography to obtain the title compound (200 mg) as yellow powder.
¹H-NMR (CDCl₃) δ : 1.21 (d, J=6.9Hz, 6H), 3.01 (m, 1H), 3.22 (t, J=7.8Hz, 2H), 3.37 (t, J=7.8Hz, 2H), 6.72 (s, 1H), 6.96 (d, J=7.2Hz, 1H), 7.25 (s, 1H), 7.47 (s, 1H), 8.83 (d, J=7.5Hz, 1H), 9.14 (s, 1H)
MS(+), m/z, 415 (M+H⁺), 829 (2M+H⁺)

### (B) 3-Amino-8-[2-(4-isopropyl-1,3-thiazol-2-yl)ethyl]-4H-pyrido[1,2-a]pyrimidin-4-one

The tert-butyl N-8-[2-(4-isopropyl-1,3-thiazol-2-yl)ethyl]-4-oxo-4H-pyrido[1,2-a]pyrimidin-3-ylcarbamate (200 mg) obtained in (A) was treated with 2 ml of trifluoroacetic acid at room temperature for 1 hour, and the solvent was evaporated under reduced pressure. The residue was dissolved in ethyl acetate, washed with saturated sodium hydrogencarbonate and dried over sodium sulfate. The solvent was evaporated under reduced pressure, and the residue was purified by silica gel column chromatography to obtain the title compound (quantitative) as yellow powder.
¹H-NMR (CDCl₃) δ : 1.28 (d, J=6.9Hz, 6H), 3.06 (m, 1H), 3.23 (t, J=7.8Hz, 2H), 3.39 (t, J=7.8Hz, 2H), 6.75 (s, 1H), 6.95 (d, J=7.5Hz, 1H), 7.50 (s, 1H), 7.47 (s, 1H), 7.94 (s, 1H), 8.80 (d, J=7.5Hz, 1H)
MS(+), m/z, 315 (M+H+)

### (C) 4-Isopropyl-2-(2-3-[([(4-methylphenyl)sulfonyl]aminocarbonyl)amino]-4-oxo-4H-pyrido[1,2-a]pyrimidin-8-ylethyl)-1,3-thiazole

The 3-amino-8-[2-(4-isopropyl-1,3-thiazol-2-yl)ethyl]-4H-pyrido[1,2-a]-pyrimidin-4-one (44 mg) obtained in (B) and p-toluenesulfonyl isocyanate (42 mg) were dissolved in toluene (0.5 ml) and refluxed by heating for 2.5 hours. After the solvent was evaporated, the residue was purified by silica gel column chromatography to obtain the title compound (33 mg) as white powder.
¹H-NMR (CDCl₃) δ : 1.26 (d, 6H), 2.41 (s, 3H), 3.05 (m, 1H), 3.25 (t, 2H), 3.39 (t, 2H), 6.73 (s, 1H), 7.19 (dd, 1H), 7.31 (d, 1H), 7.56 (s, 1H), 7.96 (d, 1H), 8.71 (s, 1H), 9.04 (d, 1H), 9.39 (s, 1H)
MS (ES+) m/z 411 (M⁺+1)

### Example 123: (E)-3-{8-[2-(4-Isopropyl-1,3-thiazol-2-yl)ethyl]-4-oxo-4H-pyrido[1,2-a]-pyrimidin-3-yl}-2-propenoic acid

### (A) Ethyl (E)-3-{8-[2-(4-isopropyl-1,3-thiazol-2-yl)ethyl]-4-oxo-4H-pyrido[1,2-a]-pyrimidin-3-yl}-2-propenoate

4-[2-(4-Isopropyl-1,3-thiazol-2-yl)ethyl]-2-aminopyridine(200 mg), diethyl 4-dimethylaminomethyleneglutaconate (265 mg) was added with bromobenzene (2 ml) and heated at 120°C for 1 hour and at 160°C for 4 hours with stirring. The residue was purified by silica gel column chromatography to obtain the title compound (20 mg) as white powder.
¹H-NMR (CDCl₃) δ : 1.20-1.40 (m, 9H), 3.05 (m, 1H), 3.32 (t, J=6.6Hz, 2H), 3.40 (t, J=6.6Hz, 2H), 4.27 (q, J=7.2Hz, 2H), 6.73 (s, 1H), 7.11 (d, J=7.2Hz, 1H), 7.18 (d, J=15.9Hz, 1H), 7.51 (s, 1H), 7.66 (d, J=15.9Hz, 1H), 8.45 (s, 1H), 9.08 (s, 1H)
MS(+), m/z, 398 (M+H⁺), 420 (M+Na⁺), MS(-), m/z, 396 (M-H⁺)

### (B) (E)-3-{8-[2-(4-Isopropyl-1,3-thiazol-2-yl)ethyl]-4-oxo-4H-pyrido[1,2-a]pyrimidin-3-yl}-2-propenoic acid

The ethyl (E)-3-{8-[2-(4-isopropyl-1,3-thiazol-2-yl)ethyl]-4-oxo-4H-pyrido[1,2-a]pyrimidin-3-yl}-2-propenoate (15 mg) obtained in (A) was dissolved in tetrahydrofuran (1 ml) and methanol (0.5 ml), added with a solution of 8 mg of lithium hydroxide dissolved in 0.5 ml of water, and stirred at room temperature for 3.5 hours, and the solvent was concentrated under reduced pressure. The residue was distributed between ether and water, and the aqueous layer was separated, made pH 3 with hydrochloric acid, extracted with ethyl acetate, and dried over sodium sulfate. After the solvent was evaporated, the residue was purified by silica gel column chromatography to obtain the title compound.
¹H-NMR (CDCl₃) δ: 1.21 (d, 6H), 2.41 (s, 3H), 3.00 (m, 1H), 3.25 (t, 2H), 3.34 (t, 2H), 6.67 (s, 1H), 7.06-7.19 (m, 2H), 7.48 (s, 1H), 7.68 (d, J=16Hz, 1H), 8.40 (s, 1H), 9.03 (d, J=7.5Hz, 1H)
MS (ES+) m/z 370 (M⁺+1); MS (ES-) m/z 368 (M⁺-1)

### Example 124: 2-{8-[2-(4-Isopropyl-1,3-thiazol-2-yl)ethyl]-4-oxo-4H-pyrido[1,2-a]-pyrimidin-3-yloxy}acetic acid

### (A) Ethyl 2-{8-[2-(4-isopropyl-1,3-thiazol-2-yl)ethyl]-4-oxo-4H-pyrido[1,2-a]-pyrimidin-3-yloxy}acetate

4-[2-(4-Isopropyl-1,3-thiazol-2-yl)ethyl]-2-aminopyridine (100 mg) and ethyl 2-ethoxycarbonylmethoxy-3-methoxy-3-dimethylaminopropenoate (150 mg) were heated at 140°C in xylene (1 ml) for 7 hours with stirring. After the solvent was evaporated under reduced pressure, the residue was purified by silica gel column chromatography to obtain the title compound (41 mg).
¹H-NMR(CDCl₃, 300MHz) δ : 1.20-1.30 (m, 9H), 3.04 (m, 1H), 3.23 (t, J=7.8Hz, 2H), 3.40 (t, J=7.8Hz, 2H), 4.25 (q, J=7.2Hz, 2H), 4.86 (s, 2H), 6.72 (s, 1H), 6.98 (d, J=7.5Hz, 1H), 7.45 (s, 1H), 8.29 (s, 1H), 8.90 (d, J=7.5Hz, 1H)
MS(+), m/z, 401 (M+H⁺)

### (B) 2-(8-[2-(4-Isopropyl-1,3-thiazol-2-yl)ethyl]-4-oxo-4H-pyrido[1,2-a]pyrimidin-3-yloxy)acetic acid

The ethyl 2-{8-[2-(4-isopropyl-1,3-thiazol-2-yl)ethyl]-4-oxo-4H-pyrido[1,2-a]-pyrimidin-3-yloxy}acetate (41 mg) obtained in (A) was dissolved in tetrahydrofuran (1 ml) and methanol (300 ml), added with a solution of lithium hydroxide (5 mg) dissolved in water (300 ml), and then the mixture was stirred at room temperature for 1 hour, and the solvent was concentrated under reduced pressure. The residue was distributed between ether and water, and the aqueous layer was separated and made pH 3 with hydrochloric acid, and then extracted with ethyl acetate. The organic layer was dried over sodium sulfate. After the solvent was evaporated, the residue was purified by silica gel column chromatography to obtain the title compound (quantitative).
¹H-NMR (DMSO-d₆) δ : 1.23 (d, 6H), 3.00 (m, 1H), 3.21 (t, 2H), 3.40 (t, 2H), 4.54 (s, 2H), 6.96 (s, 1H), 7.18 (d, 1H), 7.38 (s, 1H), 8.12 (s, 1H), 8.85 (d, 1H)
MS (ES-) m/z 372 (M⁺-1)

### Example 125: 5-{8-[2-(4-Isopropyl-1,3-thiazol-2-yl)ethyl]-4-oxo-4H-pyrido[1,2-a]pyrimidin-3-yl}-2, 3-dihydro-1,3,4-oxadiazol-2-one

Methyl 8-[2-(4-isopropyl-1,3-thiazol-2-yl)ethyl]-4-oxo-4H-pyrido[1,2-a]-pyrimidin-3-carboxylate (22 mg) and hydrazine (4 ml) were refluxed by heating in methanol (3 ml) for 2.5 hours under nitrogen atmosphere. The reaction mixture was further added with hydrazine (10 ml) and refluxed by heating for 2 days. After the reaction mixture was cooled, insoluble solids were collected by filtration and suspended in methylene chloride (5 ml). The suspension was added with diphosgene (7 ml) and stirred for 30 minutes. Insoluble solids were removed by filtration, and the reaction mixture was concentrated to obtain the title compound (11 mg).
¹H-NMR (CD₃OD) δ : 1.36 (s, 3H), 1.39 (s, 3H), 3.22 (m, 1H), 3.53 (m, 2H), 3.77 (m, 2H); 7.58 (s, 1H); 7.75 (d, 1H), 7.91 (s, 1H), 8, 84 (s, 1H), 9.31 (d, 1H)
MS (ES+) m/z 384 (M⁺+1); MS (ES-) m/z 382 (M⁺-1)

### Example 126: (E)-3-{8-[2-(4-Isopropyl-1,3-thiazol-2-yl)ethyl]-4-oxo-4H-pyrido[1,2-a]-pyrimidin-3-yl}-2-methyl-2-propenoic acid

### (A) tert-Butyl (E)-3-{8-[2-(4-isopropyl-1,3-thiazol-2-yl)ethyl]-4-oxo-4H-pyrido[1,2-a]-pyrimidin-3-yl}-2-methyl-2-propenoate

tert-Butyl 2-(diethylphosphono)propionate (70 mg) was dissolved in tetrahydrofuran(5 ml), added with sodium hydride (60% in oil, 40 mg), and stirred for 10 minutes. The reaction mixture was added with 8-(2-(4-isopropyl-1,3-thiazol-2-yl)-ethyl)-4-oxo-4H-pyrido[1,2-a]pyrimidine-3-carbaldehyde (56 mg), stirred for 10 minutes and added with acetic acid (0.2 ml), and then distributed between ethyl acetate and saturated aqueous sodium hydrogencarbonate. The organic layer was concentrated, and the residue was purified by silica gel column chromatography to obtain the title compound (70 mg) as yellow oil.
¹H-NMR (CDCl₃) δ: 1.27 (d, 6H), 1.51 (s, 9H), 2.07 (s, 3H), 3.05 (m, 1H), 3.3-3.5 (m, 4H), 6.72 (s, 1H), 7.07 (d, 1H), 7.52 (s, 1H), 7.68 (s, 1H), 8.41 (s, 1H), 9.00 (s, 1H)

### (B) (E)-3-{8-[2-(4-Isopropyl-1,3-thiazol-2-yl)ethyl]-4-oxo-4H-pyrido[1,2-a]pyrimidin-3-yl}-2-methyl-2-propenoic acid

The tert-butyl (E)-3-{8-[2-(4-isopropyl-1,3-thiazol-2-yl)ethyl]-4-oxo-4H-pyrido[1,2-a]pyrimidin-3-yl}-2-methyl-2-propenoate (70 mg) obtained in (A) was dissolved in trifluoroacetic acid (1 ml) and stirred at room temperature for 1 hour, and then the solvent was evaporated under reduced pressure. The residue was purified by silica gel column chromatography to obtain the title compound (21 mg).
¹H-NMR (CDCl₃ + CD₃OD) δ: 1.25 (d, 6H), 2.10 (s, 3H), 3.04 (m, 1H), 3.28 (t, 2H), 3.35-3.45 (m, with CD₃OD), 6.74 (s, 1H), 7.15 (d, 1H), 7.59 (s, 1H), 7.79 (s, 1H), 8.45 (s, 1H), 9.01 (d, 1H)

### Example 127: (E)-2-(3-Chloropropyl)-3-{8-[2-(4-isopropyl-1,3-thiazol-2-yl)ethyl]-4-oxo-4H-pyrido[1,2-a]pyrimidin-3-yl}-2-propenoic acid

¹H-NMR (CDCl₃) δ: 1.28 (d, 6H), 2.10 (m, 2H), 2.70 (m, 2H), 3.05 (m, 1H), 3.30 (t, 2H), 3.40 (t, 2H), 3.60 (m, 2H), 6.75 (s, 1H), 7.10 (d, 1H), 7.54 (s, 1H), 7.94 (s, 1H), 8.51 (s, 1H), 9.03 (d, 1H)
MS (ES+) m/z 446 (M⁺+1); MS (ES-) m/z 444 (M⁺-1)

### Example 128: (E)-3-{8-[2-(4-Isopropyl)-1,3-thiazol-2-yl]ethyl}-4-oxo-2-phenyl-4H-pyrido[1,2-a]pyrimidin-3-yl}-2-propenoic acid

### (A) 2-Trifluoromethanesulfonyloxy-8-[2-(4-isopropyl-1,3-thiazol-2-yl)ethyl]-4H-pyrido[1,2-a]pyrimidin-4-one

2-Hydroxy-8-[2-(4-isopropyl-1,3-thiazol-2-yl)ethyl]-4H-pyrido[1,2-a]-pyrimidin-4-one (400 mg) and DMAP (310 mg) were dissolved in methylene chloride (8 ml), added with trifluoromethanesulfonic anhydride (427 ml) at -78°C, and then the mixture was stirred overnight while the reaction temperature was gradually returned to room temperature. The reaction mixture was added with 0.2 M hydrochloric acid (50 ml) and extracted with methylene chloride, and the organic layer was dried over sodium sulfate. After the solvent was evaporated, the residue was purified by silica gel column chromatography to obtain the title compound (499 mg, 88%).
¹H-NMR (CDCl₃) δ: 1.22 (d, J=7.5Hz, 6H), 3.10 (m, 1H), 3.25-3.40 (m, 4H), 6.10 (s, 1H), 6.75 (s, 1H), 7.18 (d, J=7.5Hz, 1H), 7.55 (s, 1H), 8.95 (d, J=7.5Hz, 1H)

### (B) 2-Phenyl-8-[2-(4-isopropyl-1,3-thiazol-2-yl)ethyl]-4H-pyrido[1,2-a]pyrimidin-4-one

The 2-trifluoromethanesulfonyloxy-8-[2-(4-isopropyl-1,3-thiazol-2-yl)ethyl]-4H-pyrido[1,2-a]pyrimidin-4-one (82 mg) obtained in (A), phenylboronic acid (45 mg), palladium tetrakistriphenylphosphine (11 mg), potassium bromide (24 mg) and potassium carbonate (38 mg) were stirred at 85°C overnight in dioxane (4 ml) under a nitrogen flow. The reaction mixture was cooled and added with water (20 ml) and extracted with ethyl acetate, and then the organic layer was dried over sodium sulfate. The solvent was evaporated under reduced pressure, and the residue was purified by silica gel column chromatography to obtain the title compound (65 mg, 94%) as white powder.
¹H-NMR (CDCl₃) δ : 1.22 (d, J=7.5Hz, 6H), 3.00-3.10 (m, 1H), 3.30-3.50 (m, 4H), 6.72 (s, 1H), 6.85 (s, 1H), 6.99 (d, J=7.5Hz, 1H), 7.46-7.56 (m, 5H), 8.20-8.40 (m, 1H), 8.96 (d, J=7.5Hz, 1H)

### (C) tert-Butyl (E)-3-{2-phenyl-4-oxo-8-[2-(4-isopropyl-1,3-thiazol-2-yl)ethyl]-4H-pyrido[1,2-a]pyrimidin-3-yl}-2-propenoate

Dimethylformamide (1 ml) and phosphorus oxychloride (25 ml) were mixed under ice cooling and stirred at room temperature for 30 minutes. The mixture was added with a solution of the 2-phenyl-8-[2-(4-isopropyl-1,3-thiazol-2-yl)ethyl]-4H-pyrido[1,2-a]pyrimidin-4-one (65 mg) obtained in (B) and dissolved in dimethylformamide (1 ml) and stirred at room temperature for 1 hour and at 95°C for 1.5 hours on an outer bath. The reaction mixture was cooled and then added with ethyl acetate and hexane. Then, the organic layer was washed with saturated aqueous sodium hydrogencarbonate and water and dried over magnesium sulfate, and the solvent was evaporated under reduced pressure. The residue was added with tetrahydrofuran (2.5 ml) and dimethylformamide (0.5 ml), further added with (tert-butoxycarbonylmethylidene)triphenylphosphorane (240 mg), and stirred at 80°C for 10 hours. The solvent was evaporated under reduced pressure, and the residue was purified by silica gel column chromatography to obtain the title compound (18 mg). ¹H-NMR (CDCl₃) δ : 1.22 (d, J=7.5Hz, 6H), 3.00-310 (m, 1H), 3.30-3.50 (m, 4H), 6.82 (s, 1H), 6.99 (d, J=7.5Hz, 1H), 7.40-7.66 (m, 7H), 8.05-8.10 (m, 1H), 8.95 (d, J=7.5Hz, 1H)

### (D) (E)-3-{8-[2-(4-Isopropyl)-1,3-thiazol-2-yl]ethyl}-4-oxo-2-phenyl-4H-pyrido[1,2-a]pyrimidin-3-yl}-2-propenoic acid

The tert-butyl (E)-3-{2-phenyl-4-oxo-8-[2-(4-isopropyl-1,3-thiazol-2-yl)ethyl]-4H-pyrido[1,2-a]pyrimidin-3-yl}-2-propenoate (18 mg) obtained in (C) was added with trifluoroacetic acid (1 ml) and stirred at room temperature for 2 hours. The solvent was evaporated under reduced pressure, and the residue was purified by silica gel column chromatography to obtain the title compound (15 mg) as yellow powder.
¹H-NMR (CDCl₃) δ : 1.25 (d, 6H), 3.05 (m, 1H), 3.28 (t, 2H), 3.39 (t, 2H), 6.75 (s, 1H), 7.12 (d, 1H), 7.39 (d, J=16Hz, 1H), 7.50 (m, 3H), 7.58 (m, 3H), 7.72 (d, J=16Hz, 1H), 9.05 (d, 1H)

### Example 129: (E)-3-{8-[2-(4-Isopropyl-1,3-thiazol-2-yl)ethyl]-4-oxo-2-(3-pyridyl)-4H-pyrido[1,2-a]pyrimidin-3-yl}-2-propenoic acid

### (A) 8-[2-(4-Isopropyl-1,3-thiazol-2-yl)ethyl]-2-(3-pyridyl)-4H-pyrido[1,2-a]pyrimidin-4-one

2-Trifluoromethanesulfonyloxy-8-[2-(4-isopropyl-1,3-thiazol-2-yl)ethyl]-4H-pyrido[1,2-a]pyrimidin-4-one(499 mg), pyridine-3-boronic acid 1,3-propanediol cyclic ester (364 mg), palladium tetrakistriphenylphosphine (65 mg), potassium bromide (146 mg) and potassium carbonate (231 mg) were added with dioxane(8 ml) and stirred overnight at 85°C under nitrogen atmosphere. The reaction mixture was cooled and then added with ethyl acetate. Insoluble solids were removed by filtration, and the solvent of the filtrate was evaporated. The residue was purified by silica gel column chromatography to obtain the title compound (400 mg).
¹H-NMR (CDCl₃) δ : 1.18 (d, J=7.2Hz, 6H), 2.98 (m, 1H), 3.05-3.30 (m, 4H), 6.66 (s, 1H), 6.75 (s, 1H), 7.02 (dd, J=7.5, 1.8Hz, 1H), 7.38 (dd, J=7.5, 7.8Hz, 1H), 7.49 (d, J=1.2Hz, 1H), 8.30 (dt, J=7.5, 1.8Hz, 1H), 8.55 (dd, J=7.5, 1.2Hz, 1H), 8.86 (d, J=7.8Hz, 1H), 9.11 (d, J=1.2Hz, 1H).

### (B) Methyl (E)-3-(8-[2-(4-isopropyl-1,3-thiazol-2-yl)ethyl]-4-oxo-2-(3-pyridyl)-4H-pyrido[1,2-a]pyrimidin-3-yl)-2-propenoate

Phosphorus oxychloride (367 ml) was added to dimethylformamide (5 ml) under ice cooling and stirred at the same temperature for 10 minutes. The mixture was added with the 8-[2-(4-isopropyl-1,3-thiazol-2-yl)ethyl]-2-(3-pyridyl)-4H-pyrido[1,2-a]pyrimidin-4-one (336 mg) obtained in (A) and stirred at 95°C for 1.5 hours. The mixture was cooled, then slowly added with sodium carbonate and extracted with ethyl acetate. The organic layer was dried over sodium sulfate, and the solvent was evaporated under reduced pressure. The residue was purified by silica gel column chromatography to obtain yellow powder (58 mg). The resulting powder was dissolved in tetrahydrofuran (2.5 ml) and dimethylformamide (0.5 ml), added with methyl (triphenylphosphoranylidene)acetate (240 mg) and stirred at 90°C for 10 hours. After the solvent was evaporated under reduced pressure, the residue was purified by silica gel column chromatography to obtain the title compound (19 mg) as yellow powder.
¹H-NMR (CDCl₃) δ : 1.22 (d, J=7.5Hz, 6H), 3.05 (m, 1H), 3.30-3.50 (m, 4H), 3.75 (s, 3H), 6.88 (s, 1H), 7.15 (dd, J=7.2, 2.1Hz, 1H), 7.40-7.60 (m, 4H), 7.93 (dt, J=7.8, 1.8Hz, 1H), 8.75 (dd, J=5.1, 1.5Hz, 1H), 8.87 (d, J=1.8Hz, 1H), 9.08 (d, J=7.5Hz, 1H)

### (C) (E)-3-{8-[2-(4-Isopropyl-1,3-thiazol-2-yl)ethyl]-4-oxo-2-(3-pyridyl)-4H-pyrido[1,2-a]pyrimidin-3-yl}-2-propenoic acid

The methyl (E)-3-{8-[2-(4-isopropyl-1,3-thiazol-2-yl)ethyl]-4-oxo-2-(3-pyridyl)-4H-pyrido[1,2-a]pyrimidin-3-yl}-2-propenoate obtained in (B) was dissolved in tetrahydrofuran (2 ml) and methanol (0.5 ml), added with a solution (0.5 ml) containing lithium hydroxide (5.5 mg) and stirred at room temperature for 1 hour. The solvent was evaporated under reduced pressure, and the residue was distributed between water and diethyl ether. The aqueous layer was separated, made pH 4-5 with hydrochloric acid, and then extracted with ethyl acetate. The organic layer was dried over sodium sulfate, and the solvent was evaporated. The residue was purified by silica gel column chromatography to obtain the title compound (6.9 mg) as yellow powder.
¹H-NMR (CDCl₃) δ : 1.18 (d, 6H), 2.95 (m, 1H), 3.21 (t, 2H), 3.32 (t, 2H), 6.67 (s, 1H), 7.15 (d, 1H), 7.23 (d, J=13Hz, 1H), 7.41 (d, J=13Hz, 1H), 7.48 (s, 1H), 7.55 (m, 1H), 8.01 (m, 1H), 8.65 (m, 1H), 8.76 (s, 1H), 8.98 (d, 1H)
MS (ES+) m/z 447 (M⁺+1); MS (ES-) m/z 445 (M⁺-1)

### Example 130: (E)-3-{8-[2-(4-Isopropyl-1,3-thiazol-2-yl)ethyl]-4-oxo-2-(4-pyridyl)-4H-pyrido[1,2-a]pyrimidin-3-yl}-2-propenoic acid

¹H-NMR (CD₃OD) δ : 1.25 (d, 6H), 3.02 (m, 1H), 3.2-3.5 (m, withCHD₂OD), 6.87 (s, 1H), 7.33 (d, J=16Hz, 1H), 7.38 (m, 1H), 7.45 (d, J=16Hz, 1H), 7.59 (m, 2H), 7.72 (s, 1H), 8.72 (m, 2H), 9.13 (d, 1H)
MS (ES+) m/z 447 (M⁺+1); MS (ES-) m/z 445 (M⁺-1)

### Example 131: (E)-3-{8-[2-(4-Isopropyl-1,3-thiazol-2-yl)ethyl]-2-morpholino-4-oxo-4H-pyrido[1,2-a]pyrimidin-3-yl}-2-methyl-2-propenoic acid

### (A) Ethyl (E)-3-{8-[2-(4-isopropyl-1,3-thiazol-2-yl)ethyl]-2-morpholino-4-oxo-4H-pyrido[1,2-a]pyrimidin-3-yl}-2-methyl-2-propenoate

Under nitrogen atmosphere, triethyl 2-phosphonopropionate (155 mg, 0.65 mmol) was dissolved in tetrahydrofuran (2 ml), added with n-butyl lithium (1 M, 0.65 ml) at -78°C, and stirred at the same temperature for 20 minutes. The reaction mixture was added with a solution of 8-[2-(4-isopropyl-1,3-thiazol-2-yl)ethyl]-2-morpholino-4-oxo-4H-pyrido[1,2-a]pyrimidine-3-carbaldehyde (50 mg) dissolved in tetrahydrofuran (1 ml). The cooling bath was removed, and the reaction mixture was warmed to room temperature and stirred overnight at room temperature. The reaction mixture was added with saturated aqueous sodium hydrogencarbonate and extracted with ethyl acetate. The organic layer was dried over magnesium sulfate, and the solvent was evaporated under reduced pressure. The residue was purified by silica gel column chromatography to obtain the title compound (14 mg).
¹H-NMR (CDCl₃) δ: 1.20-1.30 (m, 9H), 1.79 (s, 3H), 2:95 (m, 1H), 3.11 (t, J=7.5Hz, 2H), 3.29 (t, J=7.5Hz, 2H), 3.48 (t, J=4.8Hz, 4H), 3.66 (t, J=4.8Hz, 4H), 4.05-4.15 (m, 2H), 6.66 (s, 1H), 6.75 (d, J=7.5Hz; 1H), 7.12 (s, 1H), 7.45 (s, 1H), 8.75 (d, J=7.5Hz, 1H)

### (B) (E)-3-{8-[2-(4-Isopropyl-1,3-thiazol-2-yl)ethyl]-2-morpholino-4-oxo-4H-pyrido[1,2-a]pyrimidin-3-yl}-2-methyl-2-propenoic acid

The ethyl (E)-3-{8-[2-(4-isopropyl-1,3-thiazol-2-yl)ethyl]-2-morpholino-4-oxo-4H-pyrido[1,2-a]pyrimidin-3-yl}-2-methyl-2-propenoate (14 mg) obtained in (A) was dissolved in tetrahydrofuran (4 ml) and methanol (1 ml), added with a solution of lithium hydroxide (1.4 mg) in water (1 ml) and stirred at room temperature for 1.5 hours. The reaction mixture was added with water and washed with ether, and then the aqueous layer was made acidic with diluted hydrochloric acid and extracted with ethyl acetate. The organic layer was dried over magnesium sulfate, and the solvent was evaporated under reduced pressure. The residue was purified by silica gel column chromatography to obtain the title compound (4.4 mg) as yellow powder.
¹H-NMR (CDCl₃) δ : 1.24 (d, 6H), 1.86 (s, 3H), 3.05 (m, 1H), 3.19 (t, 2H), 3.38 (t, 2H), 3.58 (m, 4H), 3.74 (m, 4H), 6.74 (s, 1H), 6.82 (d, 1H), 7.20 (s, 1H), 7.63 (s, 1H), 8.83 (d, 1H)
MS (ES+) m/z 469 (M⁺+1); MS (ES-) m/z 467 (M⁺-1)

### Example 132: (E)-2-Methyl-3-(8-{2-[4-(1-methylcyclopropyl)-1,3-thiazol-2-yl]ethyl}-2-morpholino-4-oxo-4H-pyrido[1,2-a]pyrimidin-3-yl)-2-propenoic acid

¹H-NMR (CDCl₃) δ : 0.75 (m, 2H), 1.14 (m, 2H), 1.42 (s, 3H), 1.88 (s, 3H), 3.18 (t, 2H), 3.34 (t, 2H), 3.59 (m, 4H), 3.76 (m, 4H), 6.73 (s, 1H), 6.81 (d, 1H), 7.22 (s, 1H), 7.63 (s, 1H), 8.82 (d, 1H)
MS (ES+) m/z 481

### Example 133: (E)-3-{8-[2-(4-tert-Butyl-1,3-thiazol-2-yl)ethyl]-2-morpholino-4-oxo-4H-pyrido[1,2-a]pyrxmidin-3-yl}-2-methyl-2-propenoic acid

¹H-NMR (CDCl₃) δ : 1.35 (s, 9H), 1.85 (s, 3H), 3.20 (t, 2H), 3.36 (t, 2H), 3.58 (m, 4H), 3.75 (m, 4H), 6.73 (s, 1H), 6.81 (d, 1H), 7.20 (s, 1H), 7.61 (s, 1H), 8.81 (d, 1H)
MS (ES+) m/z 483 (M⁺+1); MS (ES-) m/z 481 (M⁺-1)

### Example 134: (E)-3-{8-[2-(4-Isopropyl-1,3-thiazol-2-yl)ethyl]-2-(3-aminopyrrolidino-4-oxo-4H-pyrido[1,2-a]pyrimidin-3-yl)-2-methyl-2-propenoic acid

¹H-NMR (CD₃OD) δ: 1.20 (d, 6H), 1.59 (s, 3H), 1.95 (m, 1H), 2.23 (m, 1H), 2.98 (m, 1H), 3.10 (m, 2H), 3.35 (m, 2H), 3.43 (m, 1H), 3.62 (m, 2H), 3.75 (m, 2H), 6.88 (m, 2H), 7.08 (s, 1H), 7.36 (s, 1H), 8.62 (d, 1H)
MS (ES+) m/z 468 (M⁺+1)

### Example 135: (Z)-2-Fluoro-3-{8-[2-(4-isopropyl-1,3-thiazol-2-yl)ethyl]-2-morpholino-4-oxo-4H-pyrido[1,2-a]pyrimidin-3-yl}-2-propenoic acid

### (A) Ethyl (Z)-2-fluoro-3-{8-[2-(4-isopropyl-1,3-thiazol-2-yl)ethyl]-2-hydroxy-4-oxo-4H-pyrido[1,2-a]pyrimidin-3-yl}-2-propenoate

2-Fluoro-2-phosphonoacetic acid triethyl ester (384 mg) was dissolved in tetrahydrofuran (3 ml) and dimethylformamide (1.5 ml), added with n-butyl lithium (1.6 M, 1 ml) at -78°C under nitrogen atmosphere, and then the mixture was stirred at the same temperature for 20 minutes. The reaction mixture was added with a solution of 8-[2-(4-isopropyl-1,3-thiazol-2-yl)ethyl]-2-hydroxy-4-oxo-4H-pyrido[1,2-a]-pyrimidine-3-carbaldehyde (108 mg) dissolved in tetrahydrofuran (2 ml). The reaction temperature was gradually raised to room temperature, and the reaction mixture was stirred overnight at the same temperature. The reaction mixture was added with water and extracted with methylene chloride, and the organic layer was dried over magnesium sulfate. Then, the solvent was evaporated under reduced pressure. The residue was purified by silica gel column chromatography to obtain the title compound (35 mg) as a mixture of (E)- and (Z)-isomers.
¹H-NMR (CDCl₃) δ : 1.20-1.30 (m, 9H), 3.05 (m, 1H), 3.20-3.30 (m, 4H), 4.05-4.15 (m, 2H), 6.70-6.80 (m, 2H), 7.14 (d, J=7.5Hz, 1H), 7.41 (s, 1H), 9.00-9.05 (m, 1H)
MS(+), m/z, 382 (M+H⁺); MS(-), m/z, 380 (M-H⁺)

### (B) Ethyl (Z)-2-fluoro-3-(8-[2-(4-isopropyl-1,3-thiazol-2-yl)ethyl]-2-morpholino-4-oxo-4H-pyrido[1,2-a]pyrimidin-3-yl)-2-propenoate

The ethyl 2-fluoro-3-(8-[2-(4-isopropyl-1,3-thiazol-2-yl)ethyl]-2-hydroxy-4-oxo-4H-pyrido[1,2-a]pyrimidin-3-yl)-2-propenoate (35 mg) obtained in (A) was dissolved in methylene chloride (2 ml), added with triethylamine(35 ml) and tosyl chloride (46 mg) under ice cooling, and then the mixture was stirred overnight at room temperature. The reaction mixture was added with morpholine (49 ml) and further stirred for 4 hours. After the solvent was evaporated under reduced pressure, the residue was purified by silica gel column chromatography to obtain yellow powder (28 mg). This was dissolved in methylene chloride (1 ml), added with a trace amount of iodine, and then the mixture was stirred at room temperature for 30 minutes, and the solvent was evaporated to obtain the title compound.
¹H-NMR (CDCl₃) δ : 1.20-1.30 (m, 9H), 3.05 (m, 1H), 3.20 (t, J=7.8Hz, 2H), 3.36 (t, J=7.8Hz, 2H), 3.55-3.80 (m, 8H), 4.20-4.35 (m, 2H), 6.70-6.82 (m, 2H), 7.19 (d, J=7.5Hz, 1H), 7.35 (d, J=36.9Hz, 1H), 8.75-8.85 (m, 1H)

### (C) (Z)-2-Fluoro-3-{8-[2-(4-isopropyl-1,3-thiazol-2-yl)ethyl]-2-morpholino-4-oxo-4H-pyrido[1,2-a]pyrimidin-3-yl}-2-propenoic acid

The ethyl (Z)-2-fluoro-3-{8-[2-(4-isopropyl-1,3-thiazol-2-yl)ethyl]-2-morpholino-4-oxo-4H-pyrido[1,2-a]pyrimidin-3-yl}-2-propenoate (28 mg) obtained in (B) was dissolved in tetrahydrofuran (2 ml) and methanol (0.5 ml), added with a solution of lithium hydroxide(5 mg) in water (0.5 ml), and then the mixture was stirred at room temperature for 1 hour. The reaction mixture was added with water and washed with ether. Then, the aqueous layer was made acidic with diluted hydrochloric acid and extracted with ethyl acetate. The organic layer was dried over magnesium sulfate, and the solvent was evaporated under reduced pressure. The residue was purified by silica gel column chromatography to obtain the title compound (26 mg) as yellow powder.
¹H-NMR (CD₃OD) δ : 1.24 (d, 6H), 3.03 (m, 1H), 3.18 (t, 2H), 3.37 (t, 2H), 3.62 (m, 4H), 3.72 (m, 4H), 6.88-7.0 (m, 3H), 7.18 (s, 1H), 8.72 (d, 1H)
MS (ES+) m/z 473 (M⁺+1); MS (ES-) m/z 471 (M⁺-1)

### Example 136: (Z)-2-Fluoro-3-{8-[2-(4-isopropyl-1,3-thiazol-2-yl)ethyl]-2-(3-hydroxypiperidino)-4-oxo-4H-pyrido[1,2-a]pyrimidin-3-yl}-2-propenoic acid

¹H-NMR (CD₃OD) δ : 1.2-1.4 (m, 7H), 1.52 (m, 2H), 1.80 (m, 1H), 2.0 (m, 1H), 3.0 (m, 2H), 3.2 (t, 2H), 3.38 (t, 2H), 3.65 (m, 1H), 3.9 (m, 1H), 4.15 (m, 1H), 6.97 (m, 2H), 7.15 (d, J=36Hz, 1H), 7.15 (s, 1H), 8.66 (d, 1H)

### Example 137: (Z)-2-Fluoro-3-{8-[2-(4-isopropyl-1,3-thiazol-2-yl)ethyl]-2-(3-aminocarbonylmorpholino)-4-oxo-4H-pyrido[1,2-a]pyrimidin-3-yl}-2-propenoic acid

¹H-NMR (CD₃OD) δ : 1.24 (d, 6H), 3.0 (m, 2H), 3.2 (m, 3H), 3.38 (t, 2H), 3.68 (m, 1H), 4.0 (m, 3H), 4.46 (d, 1H), 6.97 (s, 1H), 7.03 (d, 1H), 7.13 (s, 1H), 7.19 (d, J=36Hz, 1H), 7.25 (s, 1H), 8.75 (d, 1H)
MS (ES-) m/z 514 (M⁺-1)

### Example 138: (Z)-2-Fluoro-3-{8-[2-(4-isopropyl-1,3-thiazol-2-yl)ethyl]-2-(3-cyanomorpholino)-4-oxo-4H-pyrido[1,2-a]pyrimidin-3-yl}-2-propenoic acid

¹H-NMR (CDCl₃) δ: 1.22 (d, 6H), 3.0 (m, 1H), 3.15 (m, 2H), 3.25 (m, 4H), 3.70-4.0 (m, 4H), 4.26 (dd, 1H), 6.72 (s, 1H), 6.81 (d, 1H), 6.85 (d, J=36Hz, 1H), 7.16 (s, 1H), 8.72 (d, 1H)
MS (ES-) m/z 496 (M⁺-1)

### Example 139: (Z)-2-Fluoro-3-{8-[2-(4-isopropyl-1,3-thiazol-2-yl)ethyl]-2-(4-aminomethylcarbonylpiperazino)-4-oxo-4H-pyrido[1,2-a]pyrimidin-3-yl}-2-propenoic acid

¹H-NMR (CD₃OD) δ: 1.22 (d, 6H), 3.02 (m, 1H), 3.21 (t, 2H), 3.39 (t, 2H), 3.53 (m, 2H), 3.65 (m, 6H), 3.98 (s, 2H), 6.98 (s, 1H), 7.05 (d, 1H), 7.22 (s, 1H), 7.26 (d, J=37Hz, 1H), 8.77 (d, 1H)
MS (ES+) m/z 529 (M⁺+1); MS (ES-) m/z 527 (M⁺-1)

### Example 140: (Z)-3-{8-[2-(4-Isopropyl-1,3-thiazol-2-yl)ethyl]-2-morpholino-4-oxo-4H-pyrido[1,2-a]pyrimidin-3-yl}-2-methoxy-2-propenoic acid

### (A) Ethyl (Z)-3-{8-[2-(4-isopropyl-1,3-thiazol-2-yl)ethyl]-2-morpholino-4-oxo-4H-pyrido[1,2-a]pyrimidin-3-yl}-2-methoxy-2-propenoate

2-Methoxy-2-phosphonoacetic acid triethyl ester (92 mg) was dissolved in tetrahydrofuran (1 ml), added with n-butyl lithium (1.6 M, 0.23 ml) at -78°C under nitrogen atmosphere, and then the mixture was stirred at the same temperature for 30 minutes. The reaction mixture was added with a solution of 8-[2-(4-isopropyl-1,3-thiazol-2-yl)ethyl]-2-morpholino-4-oxo-4H-pyrido[1,2-a]pyrimidine-3-carbaldehyde (50 mg) dissolved in tetrahydrofuran (1 ml). The reaction temperature was gradually raised to room temperature, and the reaction mixture was stirred overnight at the same temperature. The reaction mixture was added with water and extracted with methylene chloride. The organic layer was dried over magnesium sulfate, and the solvent was evaporated under reduced pressure. The residue was purified by silica gel column chromatography to obtain the title compound.
¹H-NMR (CDCl₃) δ : 1.20-1.30 (m, 9H), 3.05 (m, 1H), 3.15-3.35 (m, 4H), 3.50 (s, 3H), 3.55-3.70 (m, 8H), 4.05-4.15 (m, 2H), 6.70-6.80 (m, 2H), 7.14 (s, 1H), 7.21 (s, 1H), 8.78 (d, J=7.5Hz, 1H)

### (B) (Z)-3-{8-[2-(4-Isopropyl-1,3-thiazol-2-yl)ethyl]-2-morpholino-4-oxo-4H-pyrido[1,2-a]pyrimidin-3-yl}-2-methoxy-2-propenoic acid

The ethyl (Z)-3-{8-[2-(4-isopropyl-1,3-thiazol-2-yl)ethyl]-2-morpholino-4-oxo-4H-pyrido[1,2-a]pyrimidin-3-yl}-2-methoxy-2-propenoate obtained in (A) was dissolved in tetrahydrofuran (2 ml) and methanol (0.5 ml), added with a solution of lithium hydroxide (5 mg) in water (0.5 ml), and then the mixture was stirred at room temperature for 2.5 hours. The reaction mixture was added with water and washed with ether, and then the aqueous layer was made acidic with diluted with hydrochloric acid and extracted with ethyl acetate. The organic layer was dried over magnesium sulfate, and the solvent was evaporated under reduced pressure to obtain the title compound (6 mg, 10% for the two steps) as yellow powder.
¹H-NMR (CD₃OD) δ : 1.23 (d, 6H), 3.02 (m, 1H), 3.19 (t, 2H), 3.38 (t, 2H), 3.55 (s, 3H), 3.62 (m, 4H), 3.70 (m, 4H), 6.95 (s, 1H), 6.99 (m, 2H), 7.18 (s, 1H), 8.74 (d, 1H)
MS (ES+) m/z 485 (M⁺+1); MS (ES-) m/z 483 (M⁺-1)

### Example 141: 5-(1-{8-[2-(4-Isopropyl-1,3-thiazol-2-yl)ethyl]-2-morpholino-4-oxo-4H-pyrido[1,2-a]pyrimidin-3-yl}methylidene)-1,3-thiazolidine-2,4-dione

8-[2-(4-Isopropyl-1,3-thiazol-2-yl)ethyl]-2-morpholino-4-oxo-4H-pyrido[1,2-a]-pyrimidine-3-carbaldehyde (18 mg) and 2,4-thiazolidinedione (53 mg) were added with benzene (10 ml), piperidine (one drop) and acetic acid (two drops) and refluxed by heating for 3 hours in a vessel provided with a Dean-Stark trap. After the solvent was evaporated under reduced pressure, the residue was purified by silica gel column chromatography to obtain the title compound (17 mg) as yellow powder.
¹H-NMR (CDCl₃) δ : 1.26 (d, 6H), 3.01 (m, 1H), 3.22 (t, 2H), 3.38 (t, 2H), 3.63 (m, 4H), 3.80 (m, 4H), 6.73 (s, 1H), 6.87 (d, 1H), 7.23 (s, 1H), 7.78 (s, 1H), 8.80 (d, 1H)
MS (ES-) m/z 510 (M⁺-1)

### Example 142: (E)-3-{8-[2-(4-Isopropyl-1,3-thiazol-2-yl)ethyl]-2-methoxy-4-oxo-4H-pyrido[1,2-a]pyrimidin-3-yl}-2-propenoic acid

### (A) tert-Butyl (E)-3-{8-[2-(4-isopropyl-1,3-thiazol-2-yl)ethyl]-2-methoxy-4-oxo-4H-pyrido[1,2-a]pyrimidin-3-yl}-2-propenoate

tert-Butyl (E)-3-{8-[2-(4-isopropyl-1,3-thiazol-2-yl)ethyl]-2-hydroxy-4-oxo-4H-pyrido[1,2-a]pyrimidin-3-yl}-2-propenoate (20 mg) and triethylamine (63 ml) were dissolved in dimethylformamide (1.5 ml) and stirred at room temperature for 20 minutes under nitrogen atmosphere. The reaction mixture was added with dimethyl sulfate (4.3 ml) and stirred in the dark for 2 days. The reaction mixture was further added with dimethyl sulfate (4.3 ml) and stirred for 2 days in the same manner. The reaction mixture was added with water and extracted with ethyl acetate. The organic layer was washed with 1% aqueous solution of lithium chloride and dried over magnesium sulfate, and the solvent was evaporated under reduced pressure. The residue was purified by silica gel column chromatography to obtain the title compound (6.7 mg) as yellow powder. The starting material (7.6 mg) was also recovered.
¹H-NMR (CDCl₃): 1.28 (s, 3H), 1.29 (s, 3H), 1.51 (s, 9H), 3.07 (m, 1H), 3.21 (m, 2H), 3.42 (m, 2H), 4.06 (s, 3H), 6.74 (s, 1H), 7.01 (d, 1H), 7.11 (d, 1H), 7.37 (s, 1H), 7.94 (d, 1H), 9.00 (d, 1H)

### (B) (E)-3-{8-[2-(4-Isopropyl-1,3-thiazol-2-yl)ethyl]-2-methoxy-4-oxo-4H-pyrido[1,2-a]-pyrimidin-3-yl}-2-propenoic acid

The tert-butyl (E)-3-{8-[2-(4-isopropyl-1,3-thiazol-2-yl)ethyl]-2-methoxy-4-oxo-4H-pyrido(1,2-a]pyrimidin-3-yl}-2-propenoate (6.7 mg) obtained in (A) was treated with trifluoroacetic acid (0.5 ml) in the dark for 30 minutes, and then the solvent was evaporated. The residue was added with methanol and methylene chloride, and insoluble matters were collected by filtration to obtain the title compound (7.2 mg).
¹H-NMR (CDCl₃): 1.30 (s, 3H), 1.32 (s, 3H), 3.11. (m, 1H), 3.31 (m, 2H), 3.45 (m, 2H), 4.07 (s, 3H), 6.77 (s, 1H), 7.05 (d, 1H), 7.18 (d, 1H), 7.39 (s, 1H), 8.10 (d; 1H), 9.02 (d, 1H)
MS (ES-): 398

### Example 143: (Z)-3-{8:[2-(4-Isopropyl-1,3-thiazol-2-yl)ethyl]-2-methoxy-4-oxo-4H-pyrido[1,2-a]pyrimidin-3-yl}-2-propenoic acid

(E)-3-{8-[2-(4-Isopropyl-1,3-thiazol-2-yl)ethyl]-2-methoxy-4-oxo-4H-pyrido[1,2-a]pyrimidin-3-yl}-2-propenoic acid (1 mg) was dissolved in CDCl₃ (0.5 ml) and irradiated with light from a fluorescent lamp for 19 hours. The solvent was evaporated to obtain the title compound (1 mg).
¹H-NMR (CDCl₃) δ : 1.28 (s, 3H), 1.29 (s, 3H), 3.12 (m, 1H), 3.38 (m, 2H), 3.61 (m, 2H), 4.06 (s, 3H), 6.19 (d, 1H), 6.80 (d, 1H), 6.90 (s, 1H), 7.05 (d, 1H), 7.40 (d, 1H), 7.50 (s, 1H), 9.04 (d, 1H)
MS (ES-): 398

### Example 144: (E)-3-{8-[2-(4-Isopropyl-1,3-thiazol-2-yl)ethyl]-2-hydroxy-4-oxo-4H-pyrido[1,2-a]pyrimidin-3-yl}-2-propenoic acid

tert-Butyl (E)-3-{8-[2-(4-isopropyl-1,3-thiazol-2-yl)ethyl]-2-hydroxy-4-oxo-4H-pyrido[1,2-a]pyrimidin-3-yl]-2-propenoate (15 mg) was treated with formic acid (1 ml) in the dark for 1 hour, and added with toluene. The solvent was evaporated under reduced pressure to obtain the title compound (22 mg).
¹H-NMR (DMSO-d₆) δ: 1.18 (s, 3H), 1.21 (s, 3H), 2.99 (m, 1H), 3.23-3.40 (m, 4H), 6.82 (d, 1H), 7.10 (s, 1H), 7.20 (s, 1H), 7.42 (d, 1H), 7.85 (d, 1H), 8.91 (d, 1H)
MS (ES-): 384

### Example 145: (E)-3-{8-[2-(4-Isopropyl-1,3-thiazol-2-yl)ethyl]-4-oxo-9-methoxy-2 -morpholino-4H-pyrido[1,2-a]pyrimidin-3-yl}-2-propenoic acid

### (A) 3-Methoxy-4-methyl-2-nitropyridine

3-Hydroxy-4-methyl-2-nitropyridine (5 g) was dissolved in dimethylformamide (50 ml), added with cesium carbonate (11.6 g) and methyl iodide (13.7 g), and then the mixture was stirred overnight at room temperature. The reaction mixture was added with ethyl acetate and hexane, washed with water and dried over magnesium sulfate, and then the solvent was evaporated under reduced pressure to obtain the title compound (quantitative).
¹H-NMR (CDCl₃) δ : 2.30 (s, 3H), 3.78 (s, 3H), 7.27 (d, J=4.8Hz, 1H), 7.98 (d, J=4.8Hz, 1H)

### (B) 2-Amino-3-methoxy-4-methylpyridine

The 3-methoxy-4-methyl-2-nitropyridine (1 g) obtained in (A) was dissolved in methanol (50 ml), added with 5% Pd/C (200 mg), and stirred at a pressure of 40 psi for 2 hours under hydrogen atmosphere. After the catalyst was removed by filtration, the solvent was evaporated under reduced pressure to obtain the title compound (850 mg) as yellow oil.
¹H-NMR (CDCl₃) δ : 2.20 (s, 3H), 3.70 (s, 3H), 4.6 (s, 2H), 6.45 (d, J=4.8Hz, 1H), 7.85 (d, J=4.8Hz, 1H)

### (C) 2-(tert-Butoxycarbonylamino)-3-methoxy-4-methylpyridine

The 2-amino-3-methoxy-4-methylpyridine (850 mg) obtained in (B) was dissolved in tert-butanol (10 ml), added with di-tert-butyl dicarbonate (2 g), and stirred at room temperature for 72 hours. After the solvent was evaporated under reduced pressure, the residue was purified by silica gel column chromatography to obtain the title compound (1.1 g).
¹H-NMR (CDCl₃) δ: 1.52 (s, 9H), 2.27 (s, 3H), 3.75 (s, 3H), 4.6 (s, 1H), 6.78 (d, J=4.8Hz, 1H), 8.05 (d, J=4.8Hz, 1H)

### (D) tert-Butyl N-4-[2-(4-isopropyl-1,3-thiazol-2-yl)ethyl]-3-methoxy-2-pyridylcarbamate

The 2-(tert-butoxycarbonylamino)-3-methoxy-4-methylpyridine (2.84 g) obtained in (C) was dissolved in anhydrous tetrahydrofuran (50 ml) and added dropwise with n-butyl lithium (1.6 M, 19 ml) at -78°C under nitrogen atmosphere. Then, the reaction temperature was raised to room temperature. The reaction mixture was cooled to -78°C again, added dropwise with a solution of 2-bromomethyl-4-isopropylthiazole (3.94 g) dissolved in tetrahydrofuran (10 ml) and stirred at the same temperature for 1 hour. Then, the reaction mixture was added with water and extracted with ethyl acetate. The organic layer was dried over sodium sulfate, and the solvent was evaporated under reduced pressure. The residue was purified by silica gel column chromatography to obtain the title compound (4.1 g).
¹H-NMR (CDCl₃) δ : 1.20 (d, J=6.9Hz, 6H), 1.45 (s, 9H), 2.95-3.20 (m, 5H), 3.67 (s, 3H), 6.60 (s, 1H), 6.70 (d, J=4.8Hz, 1H), 8.05 (d, J=4.8Hz, 1H)

### (E) 2-Amino-4-[2-(4-isopropyl-1,3-thiazol-2-yl)ethyl]-3-methoxypyridine

The tert-butyl N-4-[2-(4-isopropyl-1,3-thiazol-2-yl)ethyl]-3-methoxy-2-pyridylcarbamate (4.1 g) obtained in (D) was added with trifluoroacetic acid (20 ml) at room temperature and stirred overnight. Then, the reaction mixture was added with 50 ml of water and 5 ml of 6 M hydrochloric acid and washed with ether. The aqueous layer was carefully added with sodium hydrogencarbonate to make pH of the aqueous layer weakly alkaline, and then extracted with ethyl acetate. The organic layer was dried over sodium sulfate, and the solvent was evaporated under reduced pressure to obtain the title compound (2.7 g).
¹H-NMR (CDCl₃) δ : 1.20 (d, J=6.9Hz, 6H), 3.00-3.30 (m, 5H), 3.70 (s, 3H), 4.6 (s, 2H), 6.50 (s, 1H), 6.70 (d, J=4.8Hz, 1H), 7.75 (d, J=4.8Hz, 1H)

### (F) 2-Hydroxy-8-[2-(4-isopropyl-1,3-thiazol-2-yl)ethyl]-9-methoxy-4H-pyrido[1,2-a]-pyrimidin-4-one

The 2-amino-4-[2-(4-isopropyl-1,3-thiazol-2-yl)ethyl]-3-methoxypyridine (2.35 g) obtained in (E) and bis-2,4,6-trichlorophenyl malonate(4.3 g) were refluxed by heating for 1 hour in toluene (25 ml), and then the solvent was evaporated under reduced pressure. The residue was purified by silica gel column chromatography to obtain the title compound (2.8 g).
¹H-NMR (CDCl₃) δ: 1.28 (d, J=6.9Hz, 6H), 2.95-3.05 (m, 1H), 3.30-3.40 (m, 4H), 3.95 (s, 3H), 5.35 (s, 1H), 6.70 (s, 1H), 7.00 (d, J=7.2Hz, 1H), 8.82 (d, J=7.2Hz, 1H)

### (G) 8-[2-(4-Isopropyl-1,3-thiazol-2-yl)ethyl]-9-methoxy-2-morpholino-4H-pyrido[1,2-a]-pyrimidin-4-one

The 2-hydroxy-8-[2-(4-isopropyl-1,3-thiazol-2-yl)ethyl]-9-methoxy-4H-pyrido[1,2-a]pyrimidin-4-one (1.77 g) obtained in (F) was dissolved in methylene chloride (40 ml), added with triethylamine (1.5 ml) and tosyl chloride (1.96 g) under ice cooling, and then the mixture was stirred at room temperature. After disappearance of the starting material was observed, the reaction mixture was added with morpholine (2.2 ml) and stirred overnight. After the solvent was evaporated under reduced pressure, the residue was purified by silica gel column chromatography to obtain the title compound (0.99 g) as yellow powder.
¹H-NMR (CDCl₃) δ : 1.28 (d, J=6.9Hz, 6H), 3.00-3.10 (m, 1H), 3.20 (t, J=7.2Hz, 2H), 3.30 (t, J=7.2Hz, 2H), 3.65-3.80 (m, 8H), 4.00 (s, 3H), 5.60 (s, 1H), 6.65-6.70 (m, 2H), 8.80 (d, J=7.2Hz, 1H)

### (H) 8-[2-(4-Isopropyl-1,3-thiazol-2-yl)ethyl]-9-methoxy-2-morpholino-4-oxo-4H-pyrido[1,2-a]pyrimidine-3-carbaldehyde

Dimethylformamide (2 ml) was added with phosphorus oxychloride (0.6 ml) under ice cooling and stirred at room temperature for 20 minutes. This mixture was cooled again with ice, and added with a solution of the 8-[2-(4-isopropyl-1,3-thiazol-2-yl)ethyl]-9-methoxy-2-morpholino-4H-pyrido[1,2-a]pyrimidin-4-one (0.99 g) obtained in (G) in methylene chloride (10 ml). The reaction mixture was stirred at room temperature for 3 hours, added with water, then added with saturated aqueous sodium hydrogencarbonate and extracted with methylene chloride. After the organic layer was dried over sodium sulfate, the solvent was evaporated and the residue was purified by silica gel column chromatography to obtain the title compound (1.54 g). ¹H-NMR (CDCl₃) δ : 1.28 (d, J=6.9Hz, 6H), 3.00-3.10 (m, 1H), 3.20 (t, J=7.2Hz, 2H), 3.30 (t, J=7.2Hz, 2H), 3.70-3.85 (m, 8H), 3.95 (s, 3H), 6.75 (d, J=7.5Hz, 1H), 8.00 (s, 1H), 8.55 ((d, J=7.5Hz), 10.05 (s, 1H)

### (I) tert-Butyl (E)-3-{8-[2-(4-isopropyl-1,3-thiazol-2-yl)ethyl]-4-oxo-9-methoxy-2-morpholino-4H-pyrido[1,2-a]pyrimidin-3-yl}-2-propenoate

The 8-[2-(4-isopropyl-1,3-thiazol-2-yl)ethyl]-9-methoxy-2-morpholino-4-oxo-4H-pyrido[1,2-a]pyrimidine-3-carbaldehyde (300 mg) obtained in (H) was dissolved in tetrahydrofuran (4 ml) and dimethylformamide (1 ml), added with (tert-butoxycarbonylmethylene)triphenylphosphorane (767 mg), and stirred at 80°C for 15 hours. After the solvent was evaporated, the residue was purified by silica gel column chromatography to obtain the title compound (209 mg) as yellow powder.
¹H-NMR (CDCl₃) δ : 1.25 (d, J=6.9Hz, 6H), 1.5 (s, 9H), 3.00-3.10 (m, 1H), 3.20-3.35 (m, 4H), 3.60-3.85 (m, 8H), 4.00 (s, 3H), 6.70 (s, 1H), 6.80 (d, J=7.5Hz, 1H), 7.05 (d, J=15.3Hz, 1H), 7.50 (d, J=15.3Hz, 1H), 8.65 (d, J=7.5Hz, 1H)

### (J) (E)-3-{8-[2-(4-Isopropyl-1,3-thiazol-2-yl)ethyl]-4-oxo-9-methoxy-2-morpholino-4H-pyrido[1,2-a]pyrimidin-3-yl}-2-propenoic acid

The tert-butyl (E)-3-{8-[2-(4-isopropyl-1,3-thiazol-2-yl)ethyl]-4-oxo-9-methoxy-2-morpholino-4H-pyrido[1,2-a]pyrimidin-3-yl}-2-propenoate (209 mg) obtained in (I) was dissolved in trifluoroacetic acid (2 ml) and stirred at room temperature for 30 minutes. The solvent was evaporated under reduced pressure to obtain the title compound (180 mg) as yellow powder.
¹H-NMR (CDCl₃) δ: 1.22 (d, 6H), 3.00 (m, 1H), 3.16 (m, 2H), 3.25 (t, 2H), 3.55 (m, 4H), 3.78 (m, 4H), 3.98 (s, 3H), 6.68 (s, 1H), 6.94 (d, J=16Hz, 1H), 7.58 (d, J=16Hz, 1H), 8.58 (d, 1H)
MS (ES+) m/z 485 (M⁺+1), MS (ES-) m/z 483 (M⁺-1)

### Example 146: 5-((Z)-1-{8-[2-(4-Isopropyl-1,3-thiazol-2-yl)ethyl]-4-oxo-4H-pyrido[1,2-a]-pyrimidin-3-yl}methylidene)-1,3-thiazolidine-2,4-dione

### (A) Methyl 8-[2-(4-isopropyl-1,3-thiazol-2-yl)ethyl]-4-oxo-4H-pyrido[1,2-a]-pyrimidine-3-carboxylate

4-[2-(4-Isopropyl-1,3-thiazol-2-yl)ethyl)-2-aminopyridine (497 mg) and dimethyl methoxymethylenemalonate (425 mg) were dissolved in methylene chloride, and stirred at 90°C for 2 hours while the solvent was evaporated. The reaction mixture was added with propionic acid (0.5 ml) and heated at 160°C for 10 hours with stirring. The reaction mixture was cooled, then added with ethyl acetate, washed with saturated sodium hydrogencarbonate and dried over sodium over sodium sulfate. The solvent was evaporated under reduced pressure, and the residue was purified by silica gel column chromatography to obtain the title compound (416 mg).
¹H-NMR (CDCl₃) δ : 1.28 (s, 3H), 1.31 (s, 3H), 3.06 (m, 1H), 3.35 (m, 2H), 3.40 (m, 2H), 3.99 (s, 3H), 6.75 (s, 1H), 7.20 (d, 1H), 7.60 (s, 1H), 9.07 (s, 1H), 9.19 (d, 1H)

### (B) 8-[2-(4-Isopropyl-1,3-thiazol-2-yl)ethyl]-4-oxo-4H-pyrido[1,2-a]pyrimidine-3-carbaldehyde

The methyl 8-[2-(4-isopropyl-1,3-thiazol-2-yl)ethyl]-4-oxo-4H-pyrido[1,2-a)-pyrimidin-3-carboxylate (660 mg) obtained in (A) was dissolved in tetrahydrofuran (30 ml), added dropwise with diisopropylaluminum hydride (1 M solution in tetrahydrofuran, 9.2 ml) at -78°C, and then the mixture was stirred for 2 hours at the same temperature. The reaction mixture was added with saturated aqueous ammonium chloride (1 ml), then added with 12% aqueous hydrochloric acid, and stirred at room temperature for 1 hour. After insoluble solids were removed by filtration through a Celite layer, the solvent of the filtrate was evaporated, and the residue was dissolved in methylene chloride. This solution was added with active manganese dioxide (1.2 g) and stirred at room temperature for 16 hours. Then, insoluble matters were removed by filtration to obtain the title compound (390 mg) as yellow powder.
¹H-NMR (CDCl₃) δ : 1.27 (d, 6H), 3.05 (m, 1H), 3.3-3.5 (m, 4H), 6.74 (s, 1H), 7.25 (d, 1H), 7.63 (s, 1H), 8.87 (s, 1H), 9.16 (d, 1H), 10.36 (s, 1H)

### (C) 5-((Z)-1-{8-[2-(4-Isopropyl-1,3-thiazol-2-yl)ethyl]-4-oxo-4H-pyrido[1,2-a]-pyrimidin-3-yl}methylidene)-1,3-thiazolidine-2,4-dione

The 8-[2-(4-isopropyl-1,3-thiazol-2-yl)ethyl]-4-oxo-4H-pyrido[1,2-a]pyrimidine-3-carbaldehyde (70 mg) obtained in (B) and 2,4-thiazolidinedione (360 mg) were added with benzene(10 ml), piperidine (one drop) and acetic acid (two drops) and refluxed by heating for 1 hour in a vessel provided with a Dean-Stark trap. After the solvent was evaporated under reduced pressure, the residue was purified by silica gel column chromatography to obtain the title compound (62 mg) as yellow powder.
¹H-NMR (CDCl₃ + CD₃OD) δ: 1.22 (d, 6H), 3.00 (m, 1H), 3.2-3.4 (m, with CD₃OD), 6.72 (s, 1H), 7.18 (d, 1H), 7.51 (s, 1H), 7.86 (s, 1H), 8.42 (s, 1H), 8.98 (d, 1H)
MS (ES+) m/z 427 (M⁺+1), MS (ES-) m/z 425 (M⁺-1)

### Example 147: 3-{8-[2-(4-Isopropyl-1,3-thiazol-2-yl)ethyl]-4-oxo-4H-pyrido[1,2-a]-pyrimidin-3-yl}-4,5-dihydro-1,2,4-oxadiazol-5-one

### (A) 8-[2-(4-Isopropyl-1,3-thiazol-2-yl)ethyl]-4-oxo-4H-pyrido[1,2-a]pyrimidine-3-carboxamide

A mixture of methyl 8-[2-(4-isopropyl-1,3-thiazol-2-yl)ethyl]-4-oxo-4H-pyrido[1,2-a]pyrimidine-3-carboxylate (400 mg), concentrated aqueous ammonia (6 ml) and methanol (9 ml) was stirred overnight. Insoluble solids were collected by filtration to obtain the title compound (117 mg). The reaction mixture was concentrated and the residue was purified by silica gel column chromatography to further obtain the title compound (69 mg).
¹H-NMR (CDCl₃) δ : 1.29 (s, 3H), 1.31 (s, 3H), 3.09 (m, 1H), 3.39 (m, 2H), 3.48 (m, 2H), 5.73 (brs, 1H), 6.79 (s, 1H), 7.29 (d, 1H), 7.66 (s, 1H), 8.71 (brs, 1H), 9.14 (d, 1H), 9.30 (s, 1H)
MS (ES+) m/z 343 (M⁺+1), MS (ES-) m/z 341 (M⁺-1)

### (B) 8-[2-(4-Isopropyl-1,3-thiazol-2-yl)ethyl]-4-oxo-4H-pyrido[1,2-a]pyrimidine-3-carbonitrile

A mixture of the 8-[2-(4-isopropyl-1,3-thiazol-2-yl)ethyl]-4-oxo-4H-pyrido[1,2-a]pyrimidine-3-carboxamide (186 mg) obtained in (A), p-toluenesulfonyl chloride (207 mg) and pyridine (0.18 ml) was stirred overnight in methylene chloride. The mixture was added with triethylamine (0.2 ml), further stirred for 2 days, and diluted with methylene chloride. This mixture was washed with saturated aqueous sodium hydrogencarbonate and saturated brine, and dried over sodium sulfate, and the solvent was evaporated under reduced pressure. The residue was purified by silica gel column chromatography to obtain the title compound (98 mg). The starting material was also recovered (73 mg).
¹H-NMR (CDCl₃) δ : 1.29 (s, 3H), 1.31 (s, 3H), 3.09 (m, 1H), 3.37 (m, 2H), 3.49 (m, 2H), 6.79 (s, 1H), 7.31 (d, 1H), 7.65 (s, 1H), 8.58 (s, 1H), 9.09 (d, 1H)
MS (ES+) m/z 325 (M⁺+1), MS (ES-) m/z 323 (M⁺-1)

### (C) 3-{8-[2-(4-Isopropyl-1,3-thiazol-2-yl)ethyl]-4-oxo-4H-pyrido[1,2-a]pyrimidin-3-yl}-4,5-dihydro-1,2,4-oxadiazol-5-one

The 8-[2-(4-isopropyl-1,3-thiazol-2-yl)ethyl]-4-oxo-4H-pyrido[1,2-a]pyrimidine-3-carbonitrile (35 mg) obtained in (B), hydroxylamine (9 mg) and triethylamine (28 ml) were refluxed overnight by heating in ethanol. After the reaction mixture was cooled, the solvent was evaporated under reduced pressure, and the residue was purified by silica gel column chromatography to obtain an oxim compound (22 mg). This compound (11 mg), 1,1'-carbodiimidazole (5 mg) and pyridine (2 ml) were dissolved in tetrahydrofuran (0.5 ml), refluxed by heating for 45 minutes, and stirred overnight at room temperature. The reaction mixture was concentrated, and the residue was purified by silica gel column chromatography to obtain the title compound (1.6 mg). ¹H-NMR (CDCl₃) δ : 1.26 (s, 3H), 1.30 (s, 3H), 3.08 (m, 1H), 3.40 (m, 4H), 6.75 (s, 1H), 7.18 (brm, 1H), 7.70 (brs, 1H), 9.08 (brm, 1H)
MS (ES+) m/z 384 (M⁺+1), MS (ES-) m/z 382 (M⁺-1)

### Example 148: 2-Hydroxy-8-[2-(4-isopropyl-1,3-thiazol-2-yl)ethyl]-3-(1H-1,2,3,4-tetrazol-5-yl)-4H-pyrido(1,2-a]pyrimidin-4-one

2-Hydroxy-8-[2-(4-isopropyl-1,3-thiazol-2-yl)ethyl]-3-[2-(4-methoxybenzyl)-2H-1,2,3,4-tetrazol-5-yl]-4H-pyrido[1,2-a]pyrimidin-4-one (30 mg) was added with anisole (0.15 ml) and trifluoroacetic acid (1 ml) and stirred at room temperature for 20 hours. After the solvent was evaporated under reduced pressure, the residue was added with methanol and toluene and the solvents were evaporated again under reduced pressure. The residue was added with methanol and methylene chloride, and the deposited crystals were collected by filtration to obtain the title compound (15 mg). ¹H-NMR (CD₃OD) δ : 1.25 (s, 3H), 1.28 (s, 3H), 3.04 (m, 1H), 3.35 (m, 2H), 3.46 (m, 2H), 7.04 (s, 1H), 7.31 (s, 1H), 7.45 (d, 1H), 9.11 (d, 1H)
MS (ES+) m/z 384 (M⁺+1)

### Example 149: 8-[2-(4-Isopropyl-1,3-thiazol-2-yl)ethyl]-2-methoxy-3-(1H-1,2,3,4-tetrazol-5-yl)-4H-pyrido[1,2-a]pyrimidin-4-one

### (A) 8-[2-(4-Isopropyl-1,3-thiazol-2-yl)ethyl]-2-methoxy-3-[2-(4-methoxybenzyl)-2H-1,2,3,4-tetrazol-5-yl]-4H-pyrido[1,2-a]pyrimidin-4-one

2-Hydroxy-8-[2-(4-isopropyl-1,3-thiazol-2-yl)ethyl]-3-(2-(4-methoxybenzyl)-2H-1,2,3,4-tetrazol-5-yl]-4H-pyrido[1,2-a]pyrimidin-4-one (24 mg) was dissolved in dimethylformamide (1.5 ml), added with triethylamine (1.6 ml) and dimethyl sulfate (22 ml) and stirred overnight at room temperature. The reaction mixture was further added with dimethyl sulfate (5 ml), stirred for 4 hours, and then added with water and extracted with ethyl acetate. After the organic layer was dried over sodium sulfate, the solvent was evaporated under reduced pressure, and the residue was purified by silica gel column chromatography to obtain the title compound (5 mg).
¹H-NMR (CDCl₃) δ : 1.28 (s, 3H), 1.33 (s, 3H), 3.09 (m, 1H), 3.29 (m, 2H), 3.44 (m, 2H), 3.79 (s, 3H), 4.03 (s, 3H), 5.79 (s, 2H), 6.79 (s, 1H), 6.88 (d, 2H), 7.02 (d, 1H), 7.41 (d, 2H), 9.02 (d, 1H)
MS (ES+) m/z 518 (M⁺+1), MS (ES-) m/z 516 (M⁺-1)

### (B) 8-[2-(4-Isopropyl-1,3-thiazol-2-yl)ethyl]-2-methoxy-3-(1H-1,2,3,4-tetrazol-5-yl)-4H-pyrido[1,2-a]pyrimidin-4-one

The 8-[2-(4-isopropyl-1,3-thiazol-2-yl)ethyl]-2-methoxy-3-[2-(4-methoxybenzyl)-2H-1,2,3,4-tetrazol-5-yl]-4H-pyrido[1,2-a]pyrimidin-4-one (5 mg) obtained in (A) was added with trifluoroacetic acid (0.3 ml) and anisole (0.1 ml) and stirred for 2 days. The solvent was evaporated under reduced pressure, and the residue was added with hexane and ethyl acetate. Insoluble solids were collected by filtration and dried to obtain the title compound (1.2 mg).
¹H-NMR (CDCl₃) δ : 1.30 (s, 3H), 1.32 (s, 3H), 3.12 (m, 1H), 3.37 (m, 2H), 3.49 (m, 2H), 4.29 (s, 3H), 6.79,(s, 1H), 7.53 (s, 1H), 9.10 (d, 1H)
MS (ES+) m/z 398 (M⁺+1), MS (ES-) m/z 396 (M⁺-1)

### Example 150: (E)-3-{2-Carboxymethylthio-8-[2-(4-isopropyl-1,3-thiazol-2-yl)ethyl]-4-oxo-4H-pyrido[1,2-a]pyrimidin-3-yl}-2-propenoic acid

### (A) tert-Butyl (E)-3-{2-[(diphenoxyphosphoryl)oxy]-8-[2-(4-isopropyl-1,3-thiazol-2-yl)-ethyl]-4-oxo-4H-pyrido[1,2-a]pyrimidin-3-yl}-2-propenoate

tert-Butyl (E)-3-(8-[2-(4-isopropyl-1,3-thiazol-2-yl)ethyl]-2-hydroxy-4-oxo-4H-pyrido[1,2-a]pyrimidin-3-yl}-2-propenoate (80 mg) was suspended in methylene chloride (40 ml), added with triethylamine (0.13 ml) and diphenylphosphoryl chloride (0.15 ml), and then the mixture was stirred at room temperature for 16 hours. The reaction mixture was washed with saturated aqueous sodium hydrogencarbonate, 5% aqueous hydrochloric acid and saturated brine, and then dried over sodium sulfate and the solvent was evaporated under reduced pressure. The residue was purified by silica gel column chromatography to obtain the title compound (104 mg).
¹H-NMR (CDCl₃) δ : 1.26 (d, 6H), 1.51 (s, 9H), 3.05 (m, 1H), 3.2-3.4 (m, 4H), 6.73 (s, 1H), 7:1-7.5 (m, 13H), 7.75 (d, J=15.8Hz, 1H), 8.96 (d, 1H)

### (B) tert-Butyl (E)-3-{2-(tert-butoxycarbonylmethylthio)-8-[2-(4-isopropyl-1,3-thiazol-2-yl)ethyl]-4-oxo-4H-pyrido[1,2-a]pyrimidin-3-yl}-2-propenoate

The tert-butyl (E)-3-{2-((diphenoxyphosphoryl)oxy]-8-(2-(4-isopropyl-1,3-thiazol-2-yl)ethyl]-4-oxo-4H-pyrido[1,2-a]pyrimidin-3-yl}-2-propenoate (62 mg) obtained in (A) was dissolved in dimethylformamide (1 ml) and added dropwise with a solution of lithium sulfide in ethanol (0.1 g/ml) until the starting material disappeared. Separately, tert-butyl bromoacetate (0.04 ml) was dissolved in dimethylformamide (1 ml), added with sodium iodide (69 mg), and then stirred at room temperature for 40 minutes. These two of solutions were mixed and stirred at room temperature for 2 hours. The reaction mixture was distributed between ethyl acetate and water, and the organic layer was dried. The solvent was evaporated, and the residue was purified by silica gel column chromatography to obtain the title compound (19 mg). ¹H-NMR (CDCl₃) δ : 1.29 (d, 6H), 1.45 (s, 9H), 1.53 (s, 9H), 3.06 (m, 1H), 3.2-3.4 (m, 4H), 3.93 (s, 2H), 6.73 (s, 1H), 6.99 (d, 1H), 7.24 (d, J=15.6Hz, overlapped with CHCl₃, 1H), 7.32 (s, 1H), 7.78 (d, J=15.6Hz, 1H), 8.95 (d, 1H)

### (C) (E)-3-{2-Carboxymethylthio-8-[2-(4-isopropyl-1,3-thiazol-2-yl)ethyl]-4-oxo-4H-pyrido[1,2-a]pyrimidin-3-yl}-2-propenoic acid

The tert-butyl (E)-3-{2-(tert-butoxycarbonylmethylthio)-8-[2-(4-isopropyl-1,3-thiazol-2-yl)ethyl]-4-oxo-4H-pyrido[1,2-a]pyrimidin-3-yl}-2-propenoate (19 mg) obtained in (B) was dissolved in methylene chloride (2 ml), added with triethylsilane (0.5 ml) and trifluoroacetic acid (0.5 ml), and then the mixture was stirred at room temperature for 5 hours. After the solvent was evaporated under reduced pressure, the residue was purified by silica gel column chromatography to obtain the title compound (4 mg) as yellow powder.
¹H-NMR (CDCl₃) δ : 1.18 (d, 6H), 2.96 (m, 1H), 3.15 (m, 2H), 3.25 (m, 1H), 3.91 (d, 2H), 6.69 (s, 1H), 6.99 (d, 1H), 7.15 (d, J=15Hz, 1H), 7.31 (s, 1H), 7.77 (d, J=15Hz, 1H), 8.83 (d, J=7Hz, 1H)
MS (ES+) m/z 460 (M⁺+1), MS (ES-) m/z 458 (M⁺-1)

### Example 151: (E)-3-{2-Methylthio-8-[2-(4-isopropyl-1,3-thiazol-2-yl)ethyl]-4-oxo-4H-pyrido[1,2-a]pyrimidin-3-yl}-2-propenoic acid

¹H-NMR (CDCl₃) δ : 1.21 (d, 6H), 2.56 (s, 3H), 3.00 (m, 1H), 3.19 (m, 2H), 3.31 (m, 2H), 6.69 (s, 1H), 6.99 (d, 1H), 7.19 (d, J=15Hz, 1H), 7.36 (s, 1H), 7.83 (d, J=15Hz, 1H), 8.87 (d, J=7Hz, 1H)

### Example 152: (E)-3-{2-Aminocarbonylmethylthio-8-[2-(4-isopropyl-1,3-thiazol-2-yl)-ethyl]-4-oxo-4H-pyrido[1,2-a]pyrimidin-3-yl}-2-propenoic acid

¹H-NMR (CDCl₃ + CD₃OD) δ : 1.23 (d, 6H), 3.02 (m, 1H), 3.1-3.5 (m, with CD₃OD), 3.88 (d, 2H), 6.74 (s, 1H), 7.06 (d, 1H), 7.15 (d, J=16Hz, 1H), 7.41 (s, 1H), 7.74 (d, J=16Hz, 1H), 8.89 (d, J=8Hz, 1H)
MS (ES+) m/z 458 (M⁺+1)

### Example 153: (E)-3-{2-[2-(Aminoethylthiomethyl)-3-pyridylthio]-8-[2-(4-isopropyl-1,3-thiazol-2-yl)ethyl]-4-oxo-4H-pyrido[1,2-a]pyrimidin-3-yl}-2-propenoic acid

¹H-NMR (CD₃OD) δ: 1.22 (d, 6H), 3.02 (m, 1H), 3.15-3.4 (m, with CHD₂OD) 6.95 (s, 1H), 7.02 (s, 1H), 7.28 (d, 1H), 7.32 (d, J=16Hz, 1H), 7.52 (dd, 1H), 8.02 (d, J=16Hz, 1H), 8.10 (d, 1H), 8.72 (m, 1H), 8.98 (d, 1H)

### Example 154: 1-Ethyl-7-[(E)-2-(4-isopropyl-1,3-thiazol-2-yl)-1-ethenyl]-4-oxo-1,4-dihydro[1,8]naphthylidine-3-carboxylic acid

### (A) Ethyl 1-ethyl-7-[(E)-2-(4-isopropyl-1,3-thiazol-2-yl)-1-ethenyl]-4-oxo-1,4-dihydro[1,8]naphthylidine-3-carboxylate

[(4-Isopropyl-1,3-thiazol-2-yI)methyl](triphenyl)phosphonium bromide (723 mg, Chem. Pharm. Bull., 1977, 25, 349-352) was suspended in tetrahydrofuran (20 ml), added dropwise with n-butyl lithium (1.6 M, 1.2 mmol) at -20°C under nitrogen atmosphere, and stirred at the same temperature for 20 minutes. The reaction mixture was added with a solution of ethyl 1-ethyl-7-formyl-4-oxo-1,4-dihydro[1,8]naphthylidine-3-carboxylate (316 mg) dissolved in tetrahydrofuran, and stirred for 2 hours. The reaction mixture was added with saturated aqueous ammonium chloride and extracted with ethyl acetate. After the organic layer was dried, the solvent was evaporated under reduced pressure. The residue was purified by silica gel column chromatography to obtain the title compound (456 mg).
¹H-NMR (300MHz, CDCl₃) δ : 1.35 (d, 6H), 1.41 (t, 3H), 1.53 (t, 3H), 3.15 (m, 1H), 4.39 (q, 2H), 4.52 (q, 2H), 6.94 (s, 1H), 7.44 (d, J=15.8Hz, 1H), 7.47 (d, 1H), 7.94 (d, J=15.8Hz, 1H), 8.63 (s, 1H), 8.73 (d, 1H)

### (B) 1-Ethyl-7-[(E)-2-(4-isopropyl-1,3-thiazol-2-yl)-1-ethenyl]-4-oxo-1,4-dihydro[1,8]-naphthylidine-3-carboxylic acid

The ethyl 1-ethyl-7-[(E)-2-(4-isopropyl-1,3-thiazol-2-yl)-1-ethenyl]-4-oxo-1,4-dihydro[1,8]naphthylidine-3-carboxylate (120 mg) obtained in (A) and lithium hydroxide (26 mg) were added with methanol (10 ml) and water (5 ml), and then the mixture was stirred at room temperature for 16 hours. After the methanol was evaporated, pH of the residue was made 6 by using 5% hydrochloric acid, and insoluble substance was collected by filtration and dried to obtain the title compound (80 mg). ¹H-NMR (300MHz, CDCl₃) δ : 1.39 (d, 6H), 1.61 (t, 3H), 3.25 (m, 1H), 4.69 (q, 2H), 7.05 (s, 1H), 7.62 (m, 2H), 8.13 (d, J=16Hz, 1H), 8.80 (d, J=8Hz, 1H), 8.94 (s, 1H)
MS (ES+) m/z 370 (M⁺+1), MS (ES-) m/z 368 (M⁺-1)

### Example 155: 1-Ethyl-7-[(E)-2-(4-isopropyl-1,3-thiazol-2-yl)-1-ethenyl]-3-(1H-1,2,3,4-tetrazol-5-yl)-1,4-dihydro[1,8]naphthylidin-4-one

### (A) 1-Ethyl-7-[(E)-2-(4-isopropyl-1,3-thiazol-2-yl)-1-ethenyl]-4-oxo-1,4-dihydro[1,8]-naphthylidine-3-carboxamide

Eethyl 1-ethyl-7-[(E)-2-(4-isopropyl-1,3-thiazol-2-yl)-1-ethenyl]-4-oxo-1,4-dihydro[1,8]naphthylidine-3-carboxylate (160 mg) was added with 25% aqueous ammonia (20 ml) and isopropanol (3 ml) and heated at 100°C for 16 hours in a Parr acid digestion bomb. After the reaction mixture was cooled, the solvent was evaporated to obtain the title compound (148 mg).
¹H-NMR (300MHz, CDCl₃) δ : 1.36 (d, 6H), 1.56 (t, 3H), 3.18 (m, 1H), 4.61 (q, 2H), 5.75 (brs, 1H)6.97 (s, 1H), 7.47 (d, J=15.8Hz, 1H), 7.51 (d, 1H), 7.98 (d, J=15.8Hz, 1H), 8.75 (d, 1H), 8.95 (s, 1H), 9.56 (brs, 1H)

### (B) 1-Ethyl-7-[(E)-2-(4-isopropyl-1,3-thiazol-2-yl)-1-ethenyl]-4-oxo-1,4-dihydro[1,8]-naphthylidine-3-carbonitrile

The 1-ethyl-7-[(E)-2-(4-isopropyl-1,3-thiazol-2-yl)-1-ethenyl]-4-oxo-1,4-dihydro[1,8]naphthylidine-3-carboxamide (32 mg) obtained in (A) was dissolved in 1,2-dichloroethane, added with benzenesulfonyl chloride (0.12 ml), pyridine (0.18 ml) and dimethylaminopyridine (several pieces), and then the mixture was stirred at 40°C for 24 hours. The reaction mixture was diluted with 1,2-dichloroethane, then washed with 5% hydrochloric acid, saturated aqueous sodium hydrogencarbonate and saturated brine, and then dried and the solvent was evaporated under reduced pressure. The residue was purified by silica gel column chromatography to obtain the title compound (19 mg).
¹H-NMR (300MHz, CDCl₃) δ : 1.36 (d, 6H), 1.56 (t, 3H), 3.18 (m, 1H), 4.56 (q, 2H), 6.99 (s, 1H), 7.45 (d, J=15.8Hz, 1H), 7.51 (d, 1H), 7.98 (d, J=15.8Hz, 1H), 8.20 (s, 1H), 8.69 (d, 1H)

### (C) 1-Ethyl-7-[(E)-2-(4-isopropyl-1,3-thiazol-2-yl)-1-ethenyl]-3-(1H-1,2,3,4-tetrazol-5-yl)-1,4-dihydro[1,8]naphthylidin-4-one

The 1-ethyl-7-[(E)-2-(4-isopropyl-1,3-thiazol-2-yl)-1-ethenyl]-4-oxo-1,4-dihydro[1,8]naphthylidine-3-carbonitrile (32 mg) obtained in (B) was dissolved in tetrahydrofuran (20 ml), added with sodium azide (100 mg), ammonium chloride (200 mg) and stirred at 80°C for 2 hours. The reaction mixture was added with water and adjusted to pH 8 with saturated aqueous sodium hydrogencarbonate, and the tetrahydrofuran was evaporated. The residue was added with five drops of 25% aqueous ammonia, and insoluble solids were removed by filtration. The filtrate was adjusted to pH 7 with 12% aqueous hydrochloric acid, loaded on a HP-20 reverse phase column, sufficiently washed with water, and then eluted with water/acetonitrile/aqueous ammonia (80:20:0.2, v/v). After the solvent was evaporated, the residue was added with tert-butyl methyl ketone, and insoluble substance was taken by filtration and dried to obtain the title compound (13 mg) as yellow powder.
¹H-NMR (300MHz, CDCl₃ + CD₃OD) δ: 1.24 (d, 6H), 1.50 (t, 3H), 3.02 (m, 1H), 4.61 (q, 2H), 6.92 (s, 1H), 7.40 (d, J=15.8Hz, 1H), 7.51 (d, J=8Hz, 1H), 7.91 (d, J=15.8Hz, 1H), 8.66 (d, J=8Hz, 1H), 9.04 (s, 1H)
MS (ES+) m/z 394 (M⁺+1), MS (ES-) m/z 392 (M⁺-1)

### Example 156: (E)-3-{7-Fluoro-2-(3-hydroxypiperidino)-8-[2-(4-isopropyl-1,3-thiazol-2-yl)ethyl]-4-oxo-4H-pyrido[1,2-a]pyrimidin-3-yl}-2-propenoic acid

### (A) N¹-(4-Methyl-5-nitro-2-pyridyl)acetamide

4-Methyl-5-nitro-2-pyridinamine (3.69 g, 2.41 mmol) was added with acetic anhydride (10 ml) and stirred at 130°C for 1.5 hours.' The reaction mixture was left stand for cooling, and then added with distilled water (5.4 ml) at 0°C, heated to 130°C, and stirred for 45 minutes. The reaction mixture was left stand for cooling and concentrated, and the deposited crystals were collected by filtration and washed with distilled water to obtain the title compound (4.71 g, quantitative).
¹H-NMR (CDCl₃) δ : 2.26 (3H, s), 2.70 (3H, s), 8.19 (1H, br), 8.23 (1H, s), 8.95 (1H, s) MS; m/z: MH- 194

### (B) N¹-(5-Amino-4-methyl-2-pyridyl)acetamide

The N1-(4-methyl-5-nitro-2-pyridyl)acetamide (4.70 g, 24.1 mmol) obtained in (A) was dissolved in ethanol (150 ml), added with 10% palladium/carbon (0.95 g) and subjected to catalytic reduction overnight at 1 atm under hydrogen atmosphere. The catalyst was removed by filtration and washed with ethanol. The filtrate was concentrated and the resulting residue was purified by silica gel column chromatography (chloroform → chloroform: methanol = 100:5) to obtain the title compound (4.10 g, quantitative) as brown solid.
¹H-NMR (CD₃OD) δ : 2.11 (3H, s), 2.18 (3H, s), 7.61 (1H, s), 7.70 (1H, s)

### (C) 6-(Acetylamino)-4-methyl-3-pyridinediazonium tetrafluoroborate

The N¹-(5-amino-4-methyl-2-pyridyl)acetamide (8.00 g, 48.4 mmol) obtained in (B) was dissolved in tetrafluoroboric acid (160 ml) and slowly added dropwise with an aqueous solution (40 ml) of sodium nitrite (3.51 g, 50.8 mmol) at -20°C under nitrogen atmosphere. The reaction mixture was further stirred at -10°C for 1 hour and added with diethyl ether (800 ml), and the deposited white solid was collected by filtration and washed with diethyl ether to obtain the title compound (14.7 g, quantitative).

### (D) N¹-(5-Fluoro-4-methyl-2-pyridyl)acetamide

Toluene (280 ml) heated at 100°C with stirring was added with the 6-(acetylamino)-4-methyl-3-pyridinediazonium tetrafluoroborate (12.8 g, 48.4 mmol) obtained in (C) and further refluxed by heating for 1 hour. After the reaction mixture was left stand for cooling and the solvent was evaporated, the residue was diluted with chloroform and washed with 1 N aqueous sodium hydroxide. The organic layer was dried over anhydrous magnesium sulfate, then the solvent was evaporated and the resulting residue was purified by silica gel column chromatography (chloroform: methanol = 100:1) to obtain the title compound (3.48 g, 43%) as yellow solid.
¹H-NMR (CDCl₃) δ: 2.19 (3H, s), 2.32 (3H, s), 7.93 (1H, br), 7.98 (1H, s), 8.08 (1H, d, J=5.6Hz)
MS; m/z: (MH⁺) 169

### (E) 5-Fluoro-4-methyl-2-pyridinamine

The N¹-(5-fluoro-4-methyl-2-pyridyl)acetamide (4.25 g, 25.3 mmol) obtained in (D) was dissolved in ethanol (3 ml), added with 6 M aqueous hydrochloric acid (3 ml) and refluxed by heating for 1 hour and 30 minutes. The reaction mixture was left stand for cooling and the solvent was concentrated. The resulting residue was dissolved in distilled water, and the system was made basic with 1 N aqueous sodium hydroxide and extracted twice with chloroform. The organic layer was dried over anhydrous magnesium sulfate and the solvent was evaporated to obtain the title compound (2.82 g, 88%) as solid.
¹H-NMR (CD₃OD) δ : 2.19 (3H, s), 6.45 (1H, m), 7.65 (1H, m)
ES-MS; m/z: (MH⁺) 127

### (F) tert-Butyl N-(5-fluoro-4-methyl-2-pyridyl)carbamate

The 5-fluoro-4-methyl-2-pyridinamine (2.82 g, 22.4 mmol) obtained in (E) was dissolved in tert-butanol (100 ml), slowly added dropwise with a solution of di-tert-butyl dicarbonate (5.12 g, 23.5 mmol) in tetrahydrofuran (10 ml) over 1 hour, and then the mixture was stirred at room temperature. The deposited substance was removed by filtration, and the reaction mixture was concentrated. The resulting residue was purified by silica gel column chromatography (chloroform) to obtain the title compound (3.17 g, 66%) as white solid.
¹H-NMR (CDCl₃) δ : 1.53 (9H, s), 2.30 (3H, s), 7.74 (1H, br), 7.83 (1H, d, J=5.8Hz), 7.99 (1H, d, J=1.2Hz)
MS; m/z: (MH⁺) 227

### (G) tert-Butyl N-{5-fluoro-4-[2-(4-isopropyl-1,3-thiazol-2-yl)ethyl]-2-pyridyl}carbamate

The tert-butyl N-(5-fluoro-4-methyl-2-pyridyl)carbamate (75.6 mg, 0.334 mmol) obtained in (F) was dissolved in tetrahydrofuran (2 ml) and added dropwise with n-butyl lithium (1.5 M solution in hexane, 0.468 ml, 0.702 mmol) at -78°C under argon atmosphere. The reaction mixture was warmed to room temperature, stirred for 1 hour, cooled to -78°C again, added dropwise with a solution of 2-(bromomethyl)-4-isopropyl-1,3-thiazole (84.6 mg, 0.384 mmol) in tetrahydrofuran (2 ml), and then warmed to room temperature. The reaction mixture was added with water and extracted twice with ethyl acetate. The organic layer was dried over anhydrous magnesium sulfate, and the solvent was evaporated. The resulting residue was purified by silica gel column chromatography (chloroform: ethyl acetate = 100:1) to obtain the title compound (40.0 mg, 33%).
¹H-NMR (CDCl₃) δ : 1.28 (6H, d, J=7.1Hz), 1.54 (9H, s), 3.09 (1H, m), 3.15 (2H, m), 3.32 (2H, m), 6.71 (1H, s), 7.90 (1H, d, J=5.6Hz), 8.05 (1H, d, J=1.5Hz)8.23 (1H, br) MS; m/z: (MH⁺) 366

### (H) 5-Fluoro-4-[2-(4-isopropyl-1,3-thiazol-2-yl)ethyl]-2-pyridinamine

The tert-butyl N-{5-fluoro-4-[2-(4-isopropyl-1,3-thiazol-2-yl)ethyl]-2-pyridyl}carbamate (393 mg, 1.08 mmol) obtained in (G) was dissolved in methylene chloride (10 ml), added with trifluoroacetic acid (10 ml) under ice cooling, and stirred overnight at room temperature. The reaction mixture was concentrated, and the resulting residue was dissolved in chloroform and washed with 1 N aqueous sodium hydroxide. The organic layer was dried over anhydrous magnesium sulfate and the solvent was evaporated. The resulting residue was purified by silica gel column chromatography (chloroform:methanol = 100:2) to obtain the title compound (40.0 mg, 33%) as an oil. ¹H-NMR (CDCl₃) δ: 1.29 (6H, d, J=6.8Hz), 3.05 (3H, m), 3.27 (2H, m), 6.32 (1H, m), 6.72 (1H, m), 7.84 (1H, m)

### (I) 7-Fluoro-2-hydroxy-8-[2-(4-isopropyl-1,3-thiazol-2-yl)ethyl]-4-oxo-4H-pyrido[1,2-a]-pyrimidin-4-one

The 5-fluoro-4-[2-(4-isopropyl-1,3-thiazol-2-yl)ethyl]-2-pyridinamine (310 mg, 1.15 mmol) obtained in (H) was dissolved in xylene (2.5 ml), added with di(2,4,6-trichlorophenyl)malonate (568 mg, 1.23 mmol), and refluxed by heating for 30 minutes. The reaction mixture was left stand for cooling and then concentrated, and the resulting residue was purified by silica gel column chromatography (chloroform:methanol = 100:1) to obtain the title compound (325 mg, 84%) as yellow solid.
¹H-NMR (CDCl₃) δ : 1.27 (6H, d, J=6.8Hz), 3.05 (1H, m), 3.40 (4H, m), 5.40 (1H, s), 6.75 (1H, s), 7.40 (1H, d, J=5.9Hz), 9.00 (1H, d, J=4.6Hz)
MS; m/z: (MH⁺) 334, (MH⁻) 332

### (J) 7-Fluoro-2-(3-hydroxypiperidino)-8-[2-(4-isopropyl-1,3-thiazol-2-yl)ethyl]-4-oxo-4H-pyrido[1,2-a]pyrimidin-4-one

The 7-fluoro-2-hydroxy-8-[2-(4-isopropyl-1,3-thiazol-2-yl)ethyl]-4-oxo-4H-pyrido[1,2-a]pyrimidin-4-one (150 mg, 0.450 mmol) obtained in (I) was dissolved in a mixed solvent of tetrahydrofuran (3 ml) and dimethylformamide (1 ml), added with p-toluenesulfonyl chloride (129 mg, 0.675 mmol) and 4-dimethylaminopyridine (60.5 mg, 0.494 mmol), and then the mixture was stirred at room temperature for 2 hours. The reaction mixture was diluted with chloroform, washed with water and dried over anhydrous magnesium sulfate. The solvent was evaporated and the resulting residue was dissolved in dimethylformamide (3 ml), added with 3-hydroxypiperidine (310 mg, 2.25 mmol) and triethylamine (0.3 ml) and stirred at 80°C for 2 hours. The reaction mixture was left stand for cooling, diluted with chloroform, washed with water and dried over anhydrous magnesium sulfate. Then, the solvent was evaporated and the resulting residue was purified by preparative TLC (chloroform:methanol = 100:3) to obtain the title compound (147 mg, 79%) as yellow solid.
¹H-NMR (CDCl₃) δ: 1.29 (6H, d, J=6.8Hz), 1.56 (1H, m), 1.64 (1H, m), 1.86 (1H, m), 1.99 (1H, m), 3.07 (1H, m), 3.21 (2H, m), 3.34 (1H, m), 3.36 (2H, m), 3.38 (1H, m), 3.82 (1H, m), 3.98 (1H, m), 5.66 (1H, s), 6.73 (1H, s), 7.11 (1H, d, J=6.6Hz), 8.70 (1H, d, J=5.4Hz)

### (K) 1-{7-Fluoro-3-formyl-8-[2-(4-isopropyl-1,3-thiazol-2-yl)ethyl]-4-oxo-4H-pyrido[1,2-a]pyrimidin-2-yl}-3-piperidyl formate

Dimethylformamide (3 ml) was added with phosphorus oxychloride (0.083 ml, 0.882 mmol) under ice cooling and stirred for 30 minutes. The mixture was added with a solution of the 7-fluoro-2-(3-hydroxypiperidino)-8-[2-(4-isopropyl-1,3-thiazol-2-yl)ethyl]-4-oxo-4H-pyrido[1,2-a]pyrimidin-4-one (147 mg, 0.353 mmol) obtained in (J) in dimethylformamide (2 ml), warmed to room temperature, and then stirred for 1 hour. The reaction mixture was further added with phosphorus oxychloride (0.083 ml, 0.882 mmol) and stirred for 30 minutes. The reaction mixture was added with saturated aqueous sodium hydrogencarbonate and extracted twice with ethyl acetate. The organic layer was dried over anhydrous magnesium sulfate, and the solvent was evaporated. The resulting residue was purified by silica gel column chromatography (chloroform → chloroform:methanol = 400:1) and further purified by preparative TLC (chloroform:methanol = 100:1) to obtain the title compound (145 mg, 87%) as yellow oil.
¹H-NMR (CDCl₃) δ : 1.28 (6H, d, J=6.8Hz), 1.69 (1H, m), 1.85 (1H, m), 1.89 (1H, m), 2.02 (1H, m), 3.06 (1H, m), 3.25 (2H, m), 3.37 (2H, m), 3.63 (2H, m), 3.78 (1H, dd, J=13.7 and 6.6Hz), 3.88 (1H, dd, J=13.7 and 3.2Hz), 5.06 (1H, m), 6.74 (1H, s), 7.10 (1H, d, J=6.6Hz), 7.98 (1H, s), 8.69 (1H, d, J=5.4Hz), 10.1 (1H, s)

### (L) tert-Butyl (E)-3-{7-fluoro-2-(3-formylpiperidino)-8-[2-(4-isopropyl-1,3-thiazol-2-yl)-ethyl]-4-oxo-4H-pyrido[1,2-a]pyrimidin-3-yl}-2-propenoate

The 1-{7-fluoro-3-formyl-8-[2-(4-isopropyl-1,3-thiazol-2-yl)ethyl]-4-oxo-4H-pyrido[1,2-a]pyrimidin-2-yl}-3-piperidyl formate (145 mg, 0.307 mmol) obtained in (K) was dissolved in tetrahydrofuran (5 ml), added with tert-butoxycarbonylmethylenetriphenylphosphorane (1.38 g, 3.68 mmol) and refluxed by heating for 2 days. The reaction mixture was concentrated, and the resulting residue was purified by preparative TLC (chloroform:methanol =100:4) to obtain the title compound (147 mg, 84%) as yellow solid.
¹H-NMR (CDCl₃) δ: 1.28 (6H, d, J=6.9Hz), 1.51 (9H, s), 1.73 (1H, m), 1.85 (1H, m), 1.99 (2H, m), 3.06 (1H, m), 3.26 (2H, m), 3.38 (2H, m), 3.48 (1H, m), 3.52 (1H, m), 3.65 (1H, m), 3.74 (1H, m), 5.12 (1H, m), 6.73 (1H, s), 7.05 (1H, d, J=15.5Hz), 7.21 (1H, d, J=6.9Hz), 7.48 (1H, d, J=15.5Hz), 8.07 (1H, s), 8.80 (1H, d, J=5.4Hz)

### (M) tert-Butyl (E)-3-{7-fluoro-2-(3-hydroxypiperidino)-8-[2-(4-isopropyl-1,3-thiazol-2-yl)ethyl]-4-oxo-4H-pyrido[1,2-a]pyrimidin-3-yl}-2-propenoate

The tert-butyl (E)-3-{7-fluoro-2-(3-formylpiperidino)-8-[2-(4-isopropyl-1,3-thiazol-2-yl)ethyl]-4-oxo-4H-pyrido[1,2-a]pyrimidin-3-yl}-2-propenoate (147 mg, 0.258 mmol) obtained in (L) was dissolved in methanol (3 ml), added with sodium methoxide (33.6 mg, 0.618 mmol), and then stirred at room temperature for 9 hours. The rearction mixture was added with distilled water and extracted with chloroform (twice). The organic layer was dried over anhydrous magnesium sulfate, and the solvent was evaporated. The resulting residue was purified by preparative TLC (chloroform:methanol = 100:5) to obtain the title compound (91.6 mg, 65%).
¹H-NMR (CDCl₃) δ : 1.27 (6H, d, J=6.8Hz), 1- 51 (9H, s), 1.60 (2H, m), 1.83 (2H, m), 3.05 (1H, m), 3.26 (2H, m), 3.38 (2H, m), 3.53 (1H, m), 3.57 (2H, m), 3.73 (1H, m), 3.87 (1H, m), 4.01 (1H, m), 6.73 (1H, s), 7.03 (1H, d, J=15.6Hz), 7.21 (1H, d, J=6.6Hz), 7.48 (1H, d, J=15.6Hz), 8.81 (1H, d, J=5.6Hz)

### (N) (E)-3-{7-Fluoro-2-(3-hydroxypiperidino)-8-[2-(4-isopropyl-1,3-thiazol-2-yl)ethyl]-4-oxo-4H-pyrido[1,2-a]pyrimidin-3-yl}-2-propenoic acid

The tert-butyl (E)-3-{7-fluoro-2-(3-hydroxypiperidino)-8-[2-(4-isopropyl-1,3-thiazol-2-yl)ethyl)-4-oxo-4H-pyrido[1,2-a]pyrimidin-3-yl}-2-propenoate (43.6 mg, 0.0803 mmol) obtained in (M) was dissolved in 4 N hydrochloric acid/dioxane (3 ml) and stirred at room temperature for 2 hours. The reaction mixture was concentrated and the resulting residue was purified by preparative TLC (chloroform:methanol = 10:1) to obtain the title compound (35.6 mg, 91%) as lyophilized product.
¹H-NMR (CDCl₃) δ : 1.27 (6H, d, J=6.8Hz), 1.54 (1H, m), 1.71 (1H, m), 1.87 (2H, m), 3.06 (1H, m), 3.23 (2H, m), 3.37 (2H, m), 3.45 (1H, m), 3.62 (3H, m), 3.99 (1H, m), 6.73 (1H, s), 7.00 (1H, d, J=15.4Hz), 7.18 (1H, d, J=6.6Hz), 7.56 (1H, d, J=15.4Hz), 8.73 (1H, d, J=4.9Hz)
MS; m/z: (MH⁺) 487, (MH⁻) 485

### Example 157: (E)-3-{2-{3-[(Aminocarbonyl)oxy]piperidino}-7-fuoro-8-[2-(4-isopropyl-1,3-thiazol-2-yl)ethyl]-4-oxo-4H-pyrido[1,2-a]pyrimidin-3-yl}-2-propenoic acid

### (A) tert-Butyl (E)-3-{2-{3-[(aminocarbonyl)oxy]piperidino}-7-fluoro-8-[2-(4-isopropyl-1,3-thiazol-2-yl)ethyl]-4-oxo-4H-pyrido[1,2-a]pyrimidin-3-yl}-2-propenoate

The tert-butyl (E)-3-{7-fluoro-2-(3-hydroxypiperidino)-8-[2-(4-isopropyl-1,3-thiazol-2-yl)ethyl]-4-oxo-4H-pyrido(1,2-a]pyrimidin-3-yl}-2-propenoate (48.0 mg, 0.0885 mmol) obtained in Example 156, (M) was dissolved in ethyl acetate (3 ml), added with trichloroacetyl isocyanate (0.052 ml, 0.442 mmol) and stirred at room temperature for 1 hour. The reaction mixture was added with methanol:chloroform (1:10, 10 ml) and concentrated, and the resulting residue was dissolved in a mixed solvent of methanol (3 ml) and distilled water (1 ml), added with sodium formate (12.0 mg, 0.177 mmol) and stirred overnight at room temperature. The solution was further added with sodium formate (12.0 mg, 0.177 mmol), stirred for 5 hours and then concentrated, and the resulting residue was purified by preparative TLC (chloroform:methanol = 100:5) to obtain the title compound (71.3 mg, quantitative) as lyophilized product.
¹H-NMR (CDCl₃) δ : 1.27 (6H, d, J=6.8Hz), 1- 51 (9H, s), 1.78 (1H, m), 1.84-2.00 (3H, m), 3.06 (1H, m), 3.26 (2H, m), 3.27 (1H, m), 3.39 (2H, m), 3.52 (1H, m), 3.66 (2H, m), 4.81 (1H, m), 4.90 (1H, br), 6.70 (1H, br), 6.74 (1H, s), 7.08 (1H, d, J=15.6Hz), 7.26 (1H, d, J=6.8Hz), 7.68 (1H, d, J=15.6Hz), 8.81 (1H, d, J=5.4Hz)
MS; m/z: (MH⁺) 586, (MH) 584

### (B) (E)-3-{2-{3-[(Aminocarbonyl)oxy]piperidino}-7-fluoro-8-[2-(4-isopropyl-1,3-thiazol-2-yl)ethyl]-4-oxo-4H-pyrido[1,2-a]pyrimidin-3-yl}-2-propenoic acid

The tert-butyl (E)-3-{2-{3-[(aminocarbonyl)oxy]piperidino}-7-fluoro-8-[2-(4-isopropyl-1,3-thiazol-2-yl)ethyl]-4-oxo-4H-pyrido[1,2-a]pyrimidin-3-yl}-2-propenoate (51.8 mg, 0.0884 mmol) obtained in (A) was dissolved in 4 N hydrochloric acid in dioxane (3 ml)and stirred at room temperature. The reaction mixture was concentrated and the resulting residue was purified by preparative TLC (chloroform:methanol = 10:1) to obtain the title compound (26.8 mg, 57%) as lyophilized product.
¹H-NMR (CD₃OD) δ: 1.25 (6H, d, J=6.8Hz), 1.68 (1H, m), 1.82 (1H, m), 1.97 (2H, m), 3.02 (1H, m), 3.25 (2H, m), 3.41 (2H, m), 3.48 (1H, m), 3.57 (1H, m), 3.66 (1H, m), 3.77 (1H, m), 4.73 (1H, m), 6.94 (1H, d, J=15.6Hz), 6.97 (1H, s), 7.29 (1H, d, J=6.8Hz), 7.55 (1H, d, J=15.6Hz), 8.71 (1H, d, J=5.6Hz)
MS; m/z: (MH⁺) 530, (MH⁻) 528

### Example 158: 3-{2-(3-Hydroxypiperidino)-8-[2-(4-isopropyl-1,3-thiazol-2-yl)ethyl]-4-oxo-4H-pyrido[1,2-a]pyrimidin-3-yl}propanoic acid

### (A) Methyl (E)-3-{2-(3-formylpiperidino)-8-[2-(4-isopropyl-1,3-thiazol-2-yl)ethyl]-4-oxo-4H-pyrido[1,2-a]pyrimidin-3-yl}-2-propenoate

1-{3-Formyl-8-[2-(4-isopropyl-1,3-thiazol-2-yl)ethyl]-4-oxo-4H-pyrido[1,2-a]pyrimidin-2-yl}-3-piperidylformate (94.0 mg, 0.207 mmol) and bis(2,2,2-trifluoroethyl)-(methoxycarbonylmethyl)phosphonoate (0.131 ml, 0.620 mmol) were dissolved in tetrahydrofuran (2 ml), added with DBU (0.085 ml, 0.620 mmol) and lithium chloride (26.3 mg, 0.620 mmol), and then the mixture was stirred at room temperature for 1 hour.

The reaction mixture was concentrated and the obtained residue was purified by preparative TLC (chloroform:methanol = 100:2) to obtain the title compound (80.9 mg, 76%).
¹H-NMR (CDCl₃) δ : 1.28 (6H, d, J=6.8Hz), 1.74-2.00 (4H, m), 3.07 (1H, m), 3.20 (2H, t, J=7.8Hz), 3.36 (2H, t, J=7.8Hz), 3.54 (2H, m), 3.67 (1H, m), 3.76 (1H, m), 3.77 (3H, s), 5.14 (1H, m), 6.73 (1H, s), 6.84 (1H, dd, J=7.3 and 1.7Hz), 7.10 (1H, d, J=15.6Hz), 7.20 (1H, s), 7.62 (1H, d, J=15.6Hz), 8.09 (1H, s), 8.85 (1H, d, J=7.3Hz)

### (B) Methyl 3-{2-(3-formylpiperidino)-8-[2-(4-isopropyl-1,3-thiazol-2-yl)ethyl]-4-oxo-4H-pyrido[1,2-a]pyrimidin-3-yl}propanoate

The methyl (E)-3-{2-(3-formylpiperidino)-8-[2-(4-isopropyl-1,3-thiazol-2-yl)-ethyl]-4-oxo-4H-pyrido[1,2-a]pyrimidin-3-yl}-2-propenoate (80.9 mg, 0.158 mmol) obtained in (A) was dissolved in ethanol (3 ml), added with 5% palladium/carbon (30 mg) and stirred at 1 atm for 2 days under hydrogen atmosphere. The catalyst was removed by filtration, and the filtrate was concentrated. The resulting residue was purified by preparative TLC (chloroform:methanol = 100:2) to obtain the title compound (23.1 mg, 28%).
¹H-NMR (CDCl₃) δ :1.29 (6H, d, J=6.8Hz), 1.74-2.03 (4H, m), 3.07 (1H, m), 3.20 (2H, t, J=7.8Hz), 2.73 (2H, m), 2.93 (2H, m), 3.15 (1H, m), 3.20 (2H, m), 3.30 (2H, m), 3.35 (2H, m), 3.37 (2H, m), 3.68 (3H, s), 5.12 (1H, m), 6.72 (1H, s), 6.81 (1H, d, J=7.3Hz), 7.20 (1H, s), 7.26 (1H, s), 8.07 (1H, s), 8.79 (1H, d, J=7.3Hz)
MS; m/z: 513 (MH⁺), 511 (MH⁻)

### (C) 3-{2-(3-Hydroxypiperidino)-8-[2-(4-isopropyl-1,3-thiazol-2-yl)ethyl]-4-oxo-4H-pyrido[1,2-a]pyrimidin-3-yl}propanoic acid

The methyl 3-{2-(3-formylpiperidino)-8-[2-(4-isopropyl-1,3-thiazol-2-yl)ethyl]-4-oxo-4H-pyrido(1,2-a]pyrimidin-3-yl}propanoate (23.1 mg, 0.0451 mmol) obtained in (B) was dissolved in a mixed solution of methanol, tetrahydrofuran and water (1:1:1, 3 ml), added with lithium hydroxide monohydrate (3.8 mg, 0.0901 mmol), and stirred overnight at room temperature. The reaction mixture was added with 1 N hydrochloric acid (0.091 ml) to neutralize the system, and then the solvent was evaporated. The resulting residue was purified by preparative TLC (chloroform: methanol =10:1) to obtain the title compound (15.0 mg, 71%) as lyophilized product.
¹H-NMR (CD₃OD) δ : 1.26 (6H, d, J=6.8Hz), 1.51 (1H, m), 1.67 (1H, m), 1.84 (1H, m), 1.99 (1H, m), 2.64 (2H, m), 2.90 (2H, m), 2.95-3.08 (3H, m), 3.20 (2H, t, J=7.3Hz), 3.40 (2H, t, J=7.3Hz), 3.57 (1H, m), 3.78 (2H, m), 6.96 (1H, s), 7.03 (1H, dd, J=7.3 and 2.0Hz), 7.21 (1H, s), 8.74 (1H, d, J=7.3Hz)
MS; m/z: 471 (MH⁺), 469 (MH-)

### Example 159: 1-(2-Fluoroethyl)-7-[(E)-2-(4-isopropyl-1,3-thiazol-2-yl)-1-ethenyl]-3-(1H-1,2,3,4-tetrazol-5-yl)-1,4-dihydro-4-quinolinone

### (A) Ethyl 1-(2-fluoroethyl)-7-[(E)-2-(4-isopropyl-1,3-thiazol-2-yl)-1-ethenyl]-4-oxo-1,4-dihydro-3-quinolinecarboxylate

Ethyl 7-[(E)-2-(4-isopropyl-1,3-thiazol-2-yl)-1-ethenyl]-4-oxo-1,4-dihydro-3-quinolinecarboxylate (368 mg, 1.00 mmol) was dissolved in dimethylformamide (6 ml), added with potassium carbonate (276 mg, 2.00 mmol) and 1-bromo-2-fluoroethane (0.223 ml, 3.00 mmol), and then the mixture was stirred overnight at 65°C. The reaction mixture was left stand for cooling and then diluted with ethyl acetate. The organic layer was washed with water and dried over anhydrous magnesium sulfate, and the solvent was evaporated. The resulting residue was purified by silica gel column chromatography (chloroform → chloroform:methanol = 100:1) to obtain the title compound (254 mg, 61%) as colorless transparent oil.
¹H-NMR (CDCl₃) δ : 1.34 (6H, d, J=7.1Hz), 1.39 (3H, t, J=7.1Hz), 3.13 (1H, m), 4.36 (2H,q), 4.54 (2H, ddd, J=24.9, 4.6 and 4.4Hz), 4.85 (2H, ddd, J=46.6, 4.6 and 4.4Hz), 6.86 (1H, s), 7.40 (2H, s), 7.43 (1H, s), 7.57 (1H, dd, J=8.5 and 1.2Hz), 8.43 (1H, s), 8.47 (1H, dd, J=8.5 and 2.0Hz)
MS; m/z: (MH⁺) 415

### (B) 1-(2-Fluoroethyl)-7-[(E)-2-(4-isopropyl-1,3-thiazol-2-yl)-1-ethenyl]-4-oxo-1,4-dihydro-3-quinolinecarboxylic acid

The ethyl 1-(2-fluoroethyl)-7-[(E)-2-(4-isopropyl-1,3-thiazol-2-yl)-1-ethenyl]-4-oxo-1,4-dihydro-3-quinolinecarboxylate (254 mg, 0.613 mmol) obtained in (A) was dissolved in a mixed solution of methanol, tetrahydrofuran and water (1:1:1), added with 1 N aqueous sodium hydroxide (1.23 ml, 1.23 mmol), and then the mixture was stirred at room temperature for 8 hours. The reaction mixture was added with 1 N hydrochloric acid (1.23 ml, 1.23 mmol) to neutralize the system, and extracted with chloroform. The organic layer was dried over anhydrous magnesium sulfate, and the solvent was evaporated to obtain the title compound (174 mg, 73%) as yellow solid.

### (C) 1-(2-Fluoroethyl)-7-[(E)-2-(4-isopropyl-1,3-thiazol-2-yl)-1-ethenyl]-4-oxo-1,4-dihydro-3-quinolinecarboxamide

The 1-(2-fluoroethyl)-7-[(E)-2-(4-isopropyl-1,3-thiazol-2-yl)-1-ethenyl]-4-oxo-1,4-dihydro-3-quinolinecarboxylic acid (174 mg, 0.450 mmol) obtained in (B) was dissolved in dimethylformamide (3 ml), added with triethylamine (0.126 ml, 0.900 mmol) and ethyl chloroformate (0.086 ml, 0.900 mmol) under ice cooling, and then the mixture was stirred for 1 hour. The reaction mixture was warmed to room temperature, stirred for 30 minutes and further stirred at 0°C for 1 hour. The reaction mixture was added with concentrated aqueous ammonia (0.15 ml) and stirred overnight at room temperature. The reaction mixture was diluted with ethyl acetate and washed successively with aqueous citric acid, saturated aqueous sodium hydrogencarbonate and saturated brine and dried over anhydrous magnesium sulfate. Then, the solvent was evaporated to obtain the title compound (164 mg, 94%) as pale yellow solid.
¹H-NMR (CDCl₃) δ : 1.36 (6H, d, J=6.8Hz), 3.17 (1H, m), 4.59 (2H, ddd, J=23.9, 4.9 and 4.6Hz), 4.87 (2H, ddd, J=46.4, 4.9 and 4.6Hz), 5.75 (1H,br), 6.92 (1H, s), 7.28 (1H, m), 7.47 (2H, s), 7.69 (1H, d, J=8.6Hz), 8.54 (1H, d, J=8.6Hz), 8.79 (1H, s), 9.66 (1H, br)
MS; m/z: (MH⁺) 386

### (D) 1-(2-Fluoroethyl)-7-[(E)-2-(4-isopropyl-1,3-thiazol-2-yl)-1-ethenyl]-4-oxo-1,4-dihydro-3-quinolinecarbonitrile

A solution of dimethylformamide (0.109 ml, 1.40 mmol) in acetonitrile (2 ml) was added with oxalyl chloride (0.111 ml, 1.28 mmol) under ice cooling, stirred at the same temperature for 15 minutes, and then added with a solution of the 1-(2-fluoroethyl)-7-[(E)-2-(4-isopropyl-1,3-thiazol-2-yl)-1-ethenyl]-4-oxo-1,4-dihydro-3-quinolinecarboxamide (164 mg, 0.425 mmol) obtained in (C) in acetonitrile (3 ml). The mixture was stirred at the same temperature for 10 minutes. This mixture was added with pyridine (0.206 ml, 2.55 mmol) and stirred at the same temperature for 10 minutes and further at room temperature for 2 hours. The reaction mixture was added with a solution of dimethylformamide (0.109 ml, 1.40 mmol) and oxalyl chloride (0.111 ml, 1.28 mmol) in acetonitrile (1 ml) prepared beforehand at 0°C, then added with pyridine (0.206 ml, 2.55 mmol), and stirred at room temperature for 1 hour. The reaction mixture was added with ethyl acetate and washed with aqueous citric acid, saturated aqueous sodium hydrogencarbonate and saturated brine. The organic layer was dried over anhydrous magnesium sulfate, and the solvent was evaporated. The resulting residue was purified by preparative TLC (chloroform:methanol = 100:7) to obtain the title compound (179 mg, quantitative) as pale yellow as solid.
¹H-NMR (CDCl₃) δ: 1.36 (6H, d, J=7.1Hz), 3.15 (1H, m), 4.52 (2H, ddd, J=24.6, 4.6 and 4.4Hz), 4.85 (2H, ddd, J=46.6, 4.6 and 4.4Hz), 6.93 (1H, s), 7.43 (1H, s), 7.46 (2H, s), 7.69 (1H, dd, J=8.5 and 1.2Hz), 8.03 (1H, s), 8.49 (1H, d, J=8.6Hz)

### (E) 1-(2-Fluoroethyl)-7-[(E)-2-(4-isopropyl-1,3-thiazol-2-yl)-1-ethenyl]-3-(1H-1,2,3,4-tetrazol-5-yl)-1,4-dihydro-4-quinolinone

A solution of aluminum chloride (55.7 mg, 0.418 mmol) in dimethylformamide (1 ml) was added with sodium azide (81.5 mg, 1.25 mmol) under ice cooling, and then stirred at room temperature for 15 minutes. Then, the reaction mixture was added with a solution of the 1-(2-fluoroethyl)-7-((E)-2-(4-isopropyl-1,3-thiazol-2-yl)-1-ethenyl]-4-oxo-1,4-dihydro-3-quinolinecarbonitrile (51.2 mg, 0.139 mmol) obtained in (D) in dimethylformamide (1 ml) and stirred overnight at 85-90°C. The reaction mixture was poured into ice water/1 N hydrochloric acid (1 ml) and stirred at room temperature for 1.5 hours. The precipitates were collected by filtration and recrystallized from chloroform/methanol/hexane to obtain the title compound (15.1 mg, 26%) as colorless solid.
¹H-NMR (DMSO-d₆) δ : 1.29 (6H, d, J=7.1Hz), 3.08 (1H, m), 4.82 (1H, m), 4.94 (2H, m), 4.95 (1H, m), 7.34 (1H, s), 7.64 (1H, d, J=16.2Hz), 7.84 (1H, d, J=16.2Hz), 7.96 (1H, d, J=8.6Hz), 8.23 (1H, s), 8.37 (1H, d, J=8.6Hz), 9.05 (1H, s)
LCMS; m/z: 411 (MH⁺)

### Example 160: 1-Cyclopropyl-6-fluoro-7-[(4-isopropyl-1,3-thiazol-2-yl)methyloxy]-2-morpholino-4-oxo-1,4-dihydro-3-quinolinecarboxylic acid

### (A) Ethyl 1-cyclopropyl-6,7-difluoro-2-(methylsulfonyl)-4-oxo-1,4-dihydro-3-quinoline-carboxylate

Ethyl 1-cyclopropyl-6,7-difluoro-2-(methylsulfanyl)-4-oxo-1,4-dihydro-3-quinolinecarboxylate (300 mg, 0.976 mmol), which is a known compound [J. Heterocyclic Chem., 27, 839 (1990)], was dissolved in methylene chloride (6 ml), added with metachloroperformic acid (755 mg, 2.93 mmol) and stirred overnight. The reaction mixture was diluted with chloroform and washed successively with saturated aqueous sodium hydrogencarbonate and sodium hydrogensulfite. The organic layer was dried over anhydrous sodium sulfate, and the solvent was evaporated. The resulting residue was developed by silica gel column chromatography (hexane:ethyl acetate = 2:1) to obtain the title compound (140 mg, 39%) as white solid. The resulting compound was further developed (chloroform:methanol = 100:1) to obtain ethyl 1-cyclopropyl-6,7-difluoro-2-(methylsulfinyl)-4-oxo-1,4-dihydro-3-quinoline-carboxylate (101 mg, 29%) as colorless transparent oil.
Sulfone compound (title compound)
¹H-NMR (CDCl₃) δ : 0.88 (2H, m), 1:39 (3H, t, J=7.1Hz), 1.44 (2H, m), 3.56 (3H, s), 3.92 (1H, m), 4.43 (2H,q, J=7.1Hz), 7.63 (1H, dd, J=11.5 and 6.4Hz), 8.01 (1H, dd, J=9.8 and 8.6Hz)
ES-MS; m/z: 372 (MH⁺)

### Sulfoxide compound

¹H-NMR (CDCl₃) δ : 0.99 (2H, m), 1.39 (3H, t, J=6.9Hz), 1.46 (2H, m), 3.16 (3H, s), 4.42 (3H, m), 7.66 (1H, dd, J=11.3 and 6.4Hz), 8.13 (1H, t, J=9.3Hz)
ES-MS; m/z: 356 (MH⁺)

### (B) Ethyl 1-cyclopropyl-6,7-difluoro-2-morpholino-4-oxo-1,4-dihydro-3-quinoline-carboxylate

The ethyl 1-cyclopropyl-6,7-difluoro-2-(methylsulfonyl)-4-oxo-1,4-dihydro-3-quinolinecarboxylate (140 mg, 0.377 mmol) obtained in (A) was dissolved in tetrahydrofuran (5 ml), added with morpholine (0.0395 ml, 0.452 mmol), N,N-diisopropylethylamine (0.131 ml, 0.754 mmol) and magnesium bromide diethyl etherate (389 mg, 1.51 mmol), and then the mixture was refluxed by heating for 2 hours. The reaction mixture was diluted with ethyl acetate and washed with water. The organic layer was dried over magnesium sulfate, and the solvent was evaporated under reduced pressure. The resulting residue was purified by preparative TLC (chloroform:methanol = 100:5) to obtain the title compound (89.2 mg, 62%).
¹H-NMR (CDCl₃) δ : 0.80 (2H, m), 1.40 (3H, t, J=7.1Hz), 1.41 (2H, m), 3.14 (1H, m), 3.36 (4H, t, J=4.4Hz), 3.84 (4H, t, J=4.4Hz), 4.38 (2H,q, J=7.1Hz), 7.47 (1H, dd, J=11.7 and 6.4Hz), 8.01 (1H, dd, J=10.0 and 9.0Hz)

### (C) Ethyl 1-cyclopropyl-6-fluoro-7-[(4-isopropyl-1,3-thiazol-2-yl)methyloxy]-2-morpholino-4-oxo-1, 4-dihydro-3-quinolinecarboxylate

(4-Isopropyl-1,3-thiazol-2-yl)methanol (37.0 mg, 0.235 mmol) was dissolved in dimethylformamide (2 ml), added with 18-crown-6 (68.4 mg, 0.259 mmol) and sodium hydride (95%, 6.5 mg, 0.259 mmol), and stirred for 10 minutes under argon atmosphere. The reaction mixture was added with a solution of the ethyl 1-cyclopropyl-6,7-difluoro-2-morpholino-4-oxo-1,4-dihydro-3-quinolinecarboxylate (89.0 mg, 0.235 mmol) obtained in (B) in dimethylformamide (1 ml), and stirred at room temperature for 1 hour. Then, the reaction mixture was added with aqueous ammonium chloride and extracted with ethyl acetate. The organic layer was dried over magnesium sulfate, and the solvent was evaporated under reduced pressure. The resulting residue was purified by preparative TLC (chloroform:methanol = 100:5, v/v) to obtain the title compound (102 mg, 84%).
¹H-NMR (CDCl₃) δ : 0.68 (2H, m), 1.29 (2H, m), 1.32 (6H, d, J=6.8Hz), 1.39 (3H, t, J=7.1Hz), 3.05 (1H, m), 3.11 (1H, m), 3.32 (4H, t, J=4.4Hz), 3.82 (4H, t, J=4.4Hz), 4.39 (2H,q, J=7.1Hz), 5.52 (2H, s), 6.97 (1H, s), 7.27 (1H, d, J=7.3Hz), 7.93 (1H, d, J=11.2Hz)
ES-MS; m/z: 516 (MH⁺)

### (D) 1-Cyclopropyl-6-fluoro-7-[(4-isopropyl-1,3-thiazol-2-yl)methyloxy]-2-morpholino-4-oxo-1,4-dihydro-3-quinolinecarboxylic acid

A solution of aluminum chloride (266 mg, 2.00 mmol) in 1,2-dichloroethane (4 ml) was added with dimethyl sulfide (0.293 ml, 4.00 mmol) and stirred at 0°C for 30 minutes. Then, the reaction mixture was added with ethyl 1-cyclopropyl-6-fluoro-7-[(4-isopropyl-1,3-thiazol-2-yl)methyloxy]-2-morpholino-4-oxo-1,4-dihydro-3-quinolin ecarboxylate (51.5 mg, 0.0999 mmol) and refluxed overnight by heating. The reaction mixture was left stand for cooling, diluted with chloroform, washed with 1N hydrochloric acid, and dried over anhydrous magnesium sulfate. The solvent was evaporated and the obtained residue was purified by preparative TLC (chloroform:methanol = 10:1) to obtain the title compound (1.3 mg, 3%).
¹H-NMR (CDCl₃) δ : 0.58 (2H, m), 1.17 (2H, m), 1.33 (6H, d, J=6.8Hz), 3.11 (1H, m), 3.29 (1H, m), 3.52 (4H, m), 4.00 (4H, m), 5.55 (2H, s), 6.97 (1H, s), 7.28 (1H, d, J=6.8Hz), 7.90 (1H, d, J=10.7Hz)
LCMS; m/z: 487 (M)

### Example 161: (Z)-3-(1-Cyclopropyl-6-fluoro-7-[(4-isopropyl-1,3-thiazol-2-yl)methyloxy]-4-oxo-1,4-dihydro-3-quinolyl)-2-propeonic acid

### (A) 1-Cyclopropyl-6,7-difluoro-4-oxo-1,4-dihydro-3-quinolinecarboxamide.

1-Cyclopropyl-6,7-difluoro-4-oxo-1,4-dihydro-3-quinolinecarboxylic acid (1.00 g, 3.77 mmol) was dissolved in dimethylformamide (15 ml), added with triethylamine (0.788 ml, 5.66 mmol) and ethyl chloroformate (0.538 ml, 5.66 mmol) under ice cooling, and stirred for 1 hour. The reaction mixture was warmed to room temperature, stirred for 30 minutes, and further stirred at 0°C for 1 hour. The reaction mixture was added with concentrated aqueous ammonia (0.75 ml) and stirred overnight at room temperature. The reaction mixture was diluted with ethyl acetate and washed successively with aqueous citric acid, saturated aqueous sodium hydrogencarbonate and saturated brine. The organic layer was dried over anhydrous magnesium sulfate, and the solvent was evaporated to obtain the title compound (1.23 g, quantitative) as white solid.
¹H-NMR (CD₃OD) δ : 1.21 (2H, m), 1.42 (2H, m), 3.56 (1H, m), 7.88 (1H, dd, J=11.2 and 6.4Hz), 8.25 (1H, dd, J=10.5 and 8.5Hz), 8.88 (1H, s)

### (B) 1-Cyclopropyl-6,7-difluoro-4-oxo-1,4-dihydro-3-quinolinecarbonitrile

A solution of dimethylformamide (0.712 ml, 9.19 mmol) in acetonitrile (10 ml) was added with oxalyl chloride (0.729 ml, 8.36 mmol) under ice cooling, stirred at the same temperature for 15 minutes, added with a solution of the 1-cyclopropyl-6,7-difluoro-4-oxo-1,4-dihydro-3-quinolinecarboxamide (1.23 g, 4.18 mmol) obtained in (A) in acetonitrile (10 ml) and stirred at the same temperature for 10 minutes. This mixture was added with pyridine (1.35 ml, 16.7 mmol), stirred at the same temperature for 10 minutes, and then stirred overnight at room temperature. The reaction mixture was added with ethyl acetate, washed successively with aqueous citric acid, saturated aqueous sodium hydrogencarbonate and saturated brine, and dried over anhydrous magnesium sulfate to obtain the title compound (714 mg, 77%) as pale yellow solid.
¹H-NMR (CDCl₃) δ : 1.16 (2H, m), 1.42 (2H, m), 3.47 (1H, m), 7.78 (1H, dd, J=11.0 and 6.4Hz), 8.16 (1H, s), 8.21 (1H, dd, J=10.0 and 8.6Hz)
MS; m/z: 247 (MH⁺)

### (C) 1-Cyclopropyl-6,7-difluoro-4-oxo-1,4-dihydro-3-quinolinecarbaldehyde

The 1-cyclopropyl-6,7-difluoro-4-oxo-1,4-dihydro-3-quinolinecarbonitrile (150 mg, 0.609 mmol) obtained in (B) was dissolved in a mixed solution of acetic acid, water and pyridine (1:1:2, 4 ml), added with Raney Nickel (catalytic amount) and sodium phosphinate monohydrate (258 mg, 2.44 mmol), and then the mixture was stirred overnight at 60°C. The reaction mixture was left stand for cooling and the catalyst was removed by filtration through a Celite layer and washed with hot ethanol. The reaction mixture was concentrated, then diluted with chloroform, and washed with aqueous copper sulfate. The organic layer was dried over anhydrous magnesium sulfate to obtain the title compound (97.0 mg, 64%) as pale yellow solid.
¹H-NMR (CDCl₃) δ : 1.18 (2H, m), 1.39 (2H, m), 3.47 (1H, m), 7.78 (1H, dd, J=11.2 and 6.3Hz), 8.27 (1H, dd, J=10.2 and 8.8Hz), 8.42 (1H, s), 10.37 (1H, s)
LCMS; m/z: 250 (MH⁺)

### (D) tert-Butyl (E)- and (Z)-3-(1-cyclopropyl-6, 7-difluoro-4-oxo-1,4-dihydro-3-quinolyl)-2-propenoate

The 1-cyclopropyl-6,7-difluoro-4-oxo-1,4-dihydro-3-quinolinecarbaldehyde (97.0 mg, 0.389 mmol) obtained in (C) was dissolved in a mixed solvent of dimethylformamide and tetrahydrofuran (2:1, 3 ml), added with (tert-butoxycarbonylmethylene)triphenylphosphorane (176 mg, 0.467 mmol), and stirred at 70°C for 11 hours. The reaction mixture was concentrated, 'and the resulting residue was separated and purified by preparative TLC (chloroform:methanol = 100:5) to obtain Z-isomer of the title compound (68.7 mg, 51%) and E-isomer of the title compound (32.9 mg, 29%) as pale yellow solids.
(Z-Isomer) Rf = Higher
¹H-NMR (CDCl₃) δ: 1.23 (2H, m), 1.32 (2H, m), 1.50 (9H, s), 3.43 (1H, m), 5.86 (1H, d, J=13.2Hz), 7.27 (1H, d, J=13.2Hz), 7.71 (1H, dd, J=11.5 and 6.4Hz), 8.22 (1H, dd, J=10.0 and 9.1Hz), 9.36 (1H, s)
MS; m/z: 348 (MH⁺)
(E-Isomer) Rf = Lower ¹H-NMR (CDCl₃) δ : 1.11 (2H, m), 1.33 (2H, m), 1.52 (9H, s), 3.43 (1H, m), 7.14 (1H, d, J=15.8Hz), 7.39 (1H, d, J=15.8Hz), 7.70 (1H, dd, J=11.5 and 6.3Hz), 7.88 (1H, s), 8.24 (1H, dd, J=10.5 and 8.5Hz)
MS; m/z: 348 (MH⁺)

### (E) tert-Butyl (Z)-3-(1-cyclopropyl-6-fluoro-7-[(4-isopropyl-1,3-thiazol-2-yl)methyloxy]-4-oxo-1,4-dihydro-3-quinolyl)-2-propenoate

(4-Isopropyl-1,3-thiazol-2-yl)methanol (31.1 mg, 0.198 mmol) was dissolved in dimethylformamide (1 ml), added with 18-crown-6 (57.5 mg, 0.218 mmol) and sodium hydride (95%, 5.5 mg, 0.218 mmol), and then the mixture was stirred for 15 minutes under argon atmosphere. The reaction mixture was added with a solution of the tert-butyl (Z)-3-(1-cyclopropyl-6,7-difluoro-4-oxo-1,4-dihydro-3-quinolyl)-2-propenoate (68.7 mg, 0.198 mmol) obtained in (D) in dimethylformamide (1 ml) and stirred at room temperature for 2 hours. Then, the reaction mixture was added with aqueous ammonium chloride and extracted with chloroform. The organic layer was dried over magnesium sulfate, and the solvent was evaporated under reduced pressure. The obtained residue was purified by preparative TLC (chloroform:methanol = 100:1) to obtain the title compound (52.4 mg, 55%).
¹H-NMR (CDCl₃) δ : 1.15 (2H, m), 1.29 (2H, m), 1.33 (3H, d, J=6.8Hz), 1.50 (9H, s), 3.11 (1H, m), 3.36 (1H, m), 5.58 (2H, s), 5.82 (1H, d, J=12.9Hz), 6.97 (1H, s), 7.27 (1H, d, J=12.9Hz), 7.55 (1H, d, J=6.8Hz), 8.11 (1H, d, J=11.5Hz), 9.32 (1H, s)

### (F) (Z)-3-(1-Cyclopropyl-6-fluoro-7-[(4-isopropyl-1,3-thiazol-2-yl)methyloxy]-4-oxo-1,4-dihydro-3-quinolyl)-2-propeonic acid

The (Z)-3-(1-cyclopropyl-6-fluoro-7-[(4-isopropyl-1,3-thiazol-2-yl)methyl]-4-oxo-1,4-dihydro-3-quinolyl)-2-propenoate (52.4 mg, 0.108 mmol) obtained in (E) was dissolved in 4 N hydrochloric acid in dioxane (2 ml) and stirred overnight. The reaction mixture was concentrated, and the deposited solid was collected by filtration using ether to obtain the title compound (54.3 mg, quantitative) as pale yellow solid. ¹H-NMR (CD₃OD/CDCl₃) δ: 1.36 (3H, d, J=7.10Hz), 1.53 (2H, m), 1.73 (2H, m), 3.18 (1H, m), 4.38 (1H, m), 5.90 (2H, s), 6.77 (1H, d, J=9.6Hz), 7.26 (1H, s), 8.25 (1H, d, J=9.6Hz), 8.41 (2H, m), 9.79 (1H, s)
ES-MS; m/z: 427 (MH-)

### Example 162: (E)-3-(1-Cyclopropyl-6-fluoro-7-[(4-isopropyl-1,3-thiazol-2-yl)-methyloxy]-4-oxo-1,4-dihydro-3-quinolyl)-2-propeonic acid

### (A) tert-Butyl (E)-3-(1-cyclopropyl-6-fluoro-7-[(4-isopropyl-1,3-thiazol-2-yl)-methyloxy]-4-oxo-1,4-dihydro-3-quinolyl)-2-propenoate

(4-Isopropyl-1,3-thiazol-2-yl)methanol (19.5 mg, 0.124 mmol) was dissolved in dimethylformamide (1 ml), added with 18-crown-6 (35.8 mg, 0.135 mmol) and sodium hydride (95%, 3.4 mg, 0.135 mmol), and stirred for 15 minutes under argon atmosphere. The reaction mixture was added with a solution of tert-butyl (E)-3-(1-cyclopropyl-6,7-difluoro-4-oxo-1,4-dihydro-3-quinolyl)-2-propenoate (39.2 mg, 0.124 mmol) in dimethylformamide (1 ml) and stirred at room temperature for 3 hours. Then, the reaction mixture was added with aqueous ammonium chloride and extracted with chloroform. The organic layer was dried over magnesium sulfate, and the solvent was evaporated under reduced pressure. The resulting residue was purified by preparative TLC (chloroform:methanol = 100:1) to obtain the title compound (44.7 mg, 82%).
¹H-NMR (CDCl₃) δ : 1.03 (2H, m), 1.30 (2H, m), 1.31 (3H, d, J=6.8Hz), 1.51 (9H, s), 3.11 (1H, m), 3.36 (1H, m), 5.57 (2H, s), 6.96 (1H, s), 7.12 (1H, d, J=15.8Hz), 7.37 (1H, d, J=15.8Hz), 7.53 (1H, d, J=6.8Hz), 7.79 (1H, s), 8.10 (1H, d, J=11.2Hz)

### (B) (E)-3-(1-Cyclopropyl-6-fluoro-7-[(4-isopropyl-1,3-thiazol-2-yl)methyloxy]-4-oxo-1,4-dihydro-3-quinolyl)-2-propeonic acid

The (E)-3-(1-cyclopropyl-6-fluoro-7-[(4-isopropyl-1,3-thiazol-2-yl)methyloxy]-4-oxo-1,4-dihydro-3-quinolyl)-2-propenoate (44.7 mg, 0.0992 mmol) obtained in (A) was dissolved in 4 N hydrochloric acid in dioxane (2 ml) and stirred overnight. The reaction mixture was concentrated, and the deposited solid was collected by filtration using ether to obtain the title compound (32.9 mg, 83%) as pale yellow solid.
¹H-NMR (CD₃OD/CDCl₃) δ : 1.14 (2H, m), 1.33 (3H, d, J=5.6Hz), 1.38 (2H, m), 3.15 (1H, m), 3.56 (1H, m), 5.66 (2H, s), 7.13 (1H, d, J=15.6Hz), 7.17 (1H, s), 7.60 (1H, d, J=15.6Hz), 7.75 (1H, d, 6.9Hz), 8.04 (1H, d, J=11.2Hz), 8.25 (1H, s)
ES- MS; m/z: 429 (MH⁺).

### Example 163: (E)-3-[2-[3-(Aminocarbonyl)piperidino]-8-({[4-(tert-butyl)-1,3-thiazol-2-yl]amino}carbonyl)-4-oxo-4H-pyrido[1,2-a]pyrimidin-3-yl]-2-propenoic acid

### (A) tert-Butyl-(E)-3-[8-([4-(tert-butyl)-1,3-thiazol-2-yl]aminocarbonyl)-2-hydroxy-4-oxo -4H-pyrido[1,2-a]pyrimidin-3-yl]-2-propenoate

DMF (28.1 µl, 0.36 mmol) was dissolved in methylene chloride (3 ml), added dropwise with oxalyl chloride (31.7 µl, 0.36 mmol) with ice cooling and stirred for 15 minutes at the same temperature. The reaction solution was added with N⁸-[4-(tert-butyl)-1,3-thiazol-2-yl]-2,4-dioxo-3,4-dihydro-2H-pyrido[1,2-a]pyrimidine-8-carboxamide (50.0 mg, 0.15 mmol) and stirred at room temperature for 1 hour and 30 minutes. The reaction mixture was added with a small amount of saturated aqueous sodium hydrogencarbonate with ice cooling, extracted with chloroform at about pH 7 and dried over anhydrous sodium sulfate, and the solvent was evaporated. The residue was dissolved in THF (10 ml), added with (tert-butoxycarbonylmethylene)-triphenylphosphorane (82.0 mg, 0.22 mmol) and stirred at room temperature for 13 hours. The solvent was evaporated, and the residue was purified by preparative TLC (two plates, chloroform:methanol = 20:1, v/v) to obtain the title compound (34.3 mg, 50.2%).
¹H-NMR (DMSO-d₆) δ: 1.30 (9H, s), 1.45 (9H, s), 6.87 (1H, d, J=15.7Hz), 6.82-6.91 (1H, m), 7.82 (1H, d, J=15.7Hz), 7.87-7.95 (2H, m), 9.07 (1H, d, J=7.6Hz).
MS; m/z: 471 (MH⁺).

### (B) tert-Butyl (E)-3-[2-[3-(aminocarbonyl)piperidino]-8-([4-(tert-butyl)-1,3-thiazol-2-yl]aminocarbonyl)-4-oxo-4H-pyrido[1,2-a]pyrimidin-3-yl]-2-propenoate

tert-Butyl (E)-3-[8-([4-(tert-butyl)-1,3-thiazol-2-yl]aminocarbonyl)-2-hydroxy-4-oxo-4H-pyrido[1,2-a]pyrimidin-3-yl]-2-propenoate (67.3 mg, 0.14 mmol) was dissolved in DMF (20 ml), added with tosyl chloride (40.9 mg, 0.21 mmol) and dimethylaminopyridine (26.2 mg, 0.21 mmol), stirred at room temperature for 3 hours, added with nipecotamide (91.7 mg, 0.72 mmol), and stirred at room temperature for 15 hours. The solvent was evaporated, and the residue was purified by preparative PLC (chloroform:methanol = 30:1, v/v) to obtain the title compound (53.9 mg, 64.9% for the two steps) as an amorphous mixture of pale orange oily substance.
¹H-NMR (CDCl₃) δ : 1.31 (9H, s), 1.51 (9H, s), 1.50-1.85 (3H, m), 1.96-2.03 (1H, m), 2.74-2.95 (2H, m), 3.19-3.28 (1H, m), 4.04 (1H, brd, J=12.7Hz), 4.41 (1H, brd, J=12.7Hz), 6.61 (1H, s), 6.76 (1H, brs), 6.96 (1H, brs), 7.07 (1H, d, J=15.4Hz), 7.44 (1H, dd, J=7.6, 1.5Hz), 7.51 (1H, d, J=15.4Hz), 7.97 (1H, brs), 8.97 (1H, d, J=7.6Hz).

### (C) (E)-3-[2-[3-(Aminocarbonyl)piperidino]-8-({[4-(tert-butyl)-1,3-thiazol-2-yl]amino}carbonyl)-4-oxo-4H-pyrido[1,2-a]pyrimidin-3-yl]-2-propenoic acid (D11-2168)

tert-Butyl (E)-3-[2-[3-(aminocarbonyl)piperidino]-8-([4-(tert-butyl)-1,3-thiazol-2-yl]aminocarbonyl)-4-oxo-4H-pyrido[1,2-a]pyrimidin-3-yl]-2-propenoate (60.6 mg, 0.10 mmol) was dissolved in 4 N hydrochloric acid in dedioxane (6 ml) and stirred at room temperature for 3 hours. The solvent and the excessive reagents were evaporated, and the residue was subjected to azeotropy with ether. The residue was purified by preparative PLC (chloroform:methanol = 10:1, v/v) and lyophilized from dioxane to obtain the title compound (35.8 mg, 65.4%) as orange powder.
m.p.: 206-223°C (decomp.)
¹H-NMR (DMSO-d₆) δ : 1.24 (9H, s), 1.60-1.70 (2H, m), 1.72-1.81 (1H, m), 1.94-2.01 (1H, m), 3.07-3.20 (2H, m), 3.40-3.50 (1H, m), 3.93 (1H, brd, J=12.8Hz), 4.14 (1H, brd, J=12.8Hz), 6.80-6.98 (2H, m), 6.93 (1H, d, J=15.5Hz), 7.31 (1H, brs), 7.44 (1H, d, J=15.5Hz), 7.62 (1H, d, J=6.6Hz), 8.22 (1H, brs), 8.90 (1H, d, J=7.3Hz).
IR (microscopic ATR): 3320, 3191,2960, 2861, 1666, 1592, 1519, 1442 cm⁻¹.
FAB/MS; m/z: 525 (MH⁺).
H-R FAB/MS: Calcd. for C₂₅H₂₈N₆O₅S: 525.1920. Found: 525.1880.

In a similar manner, the following compounds were synthesized.

### Example 164: (E)-3-(8-({[4-(tert-Butyl)-1,3-thiazol-2-yl]amino}carbonyl)-2-{3-[(dimethylamino)carbonyl]piperidino}-4-oxo-4H-pyrido[1,2-a]pyrimidin-3-yl)-2-propenoic acid

m.p.: 175-187°C (decomp.)
¹H-NMR (DMSO-d₆) δ: 1.02 (3H, t, J=7.0Hz), 1.18 (3H, t; J=7.0Hz), 1.31 (9H, s), 1.60-1.79 (3H, m), 1.82-1.89 (1H, m), 2.88-2.97 (1H, m), 3.09-3.21 (2H, m), 3.38-3.55 (4H, m), 3.94 (1H, brd, J=12.9Hz), 4.10 (1H, brd, J=13.2Hz), 6.86 (1H, brs), 6.94 (1H, d, J=15.4Hz), 7.43 (1H, d, J=15.5Hz), 7.65 (1H, d, J=6.1Hz), 8.12 (1H, brs), 8.91 (1H, d, J=7.6Hz).
IR (microscopic ATR): 2962, 2933, 2869, 1677, 1633, 1598, 1546, 1519, 1442 cm⁻¹. FAB/MS; m/z: 581 (MH⁺).
H-R FAB/MS: Calcd. for C₂₉H₃₆N₆O₅S: 581.2546. Found: 581.2526.

### Example 165: (E)-3-[8-({[4-(tert-Butyl)-1,3-thiazol-2-yl]amino}carbonyl)-2-(3-{[(methylamino)carbonyl]amino}piperidino)-4-oxo-4H-pyrido[1,2-a]pyrimidin-3-yl]-2-propenoic acid

m.p.: 220-225°C (decomp.)
¹H-NMR (DMSO-d₆) δ: 1.31 (9H, s), 1.40-1.52 (1H, m), 1.60-1.73 (1H, m), 1.73-1.86 (1H, m), 1.86-1.96 (1H, m), 2.52 (3H, d, J=4.4Hz), 3.05-3.17 (1H, m), 3.18-3.30 (1H, m), 3.53-3.46 (1H, m), 3.69-3.78 (1H, m), 3.95-4.04 (1H, m), 3.66-3.72 (1H, m), 5.90-5.98 (1H, m), 6.80-6.95 (1H, m), 6.92 (1H, d, J=15.5Hz), 7.44 (1H, d, J=15.5Hz), 7.60 (1H, d, J=5.9Hz), 8.19 (1H, brs), 8.89 (1H, d, J=7.6Hz).
IR (microscopic ATR): 3345, 2927, 2854, 1673, 1635, 1592, 1558, 1515, 1436 cm⁻¹. FAB/MS; m/z: 554 (MH⁺).
H-R FAB/MS: Calcd. for C₂₆H₃₁N₇O₅S: 554.2186. Found: 554.2151.

### Example 166: (E)-3-[8-({[4-(tert-Butyl)-1,3-thiazol-2-yl]amino}carbonyl)-2-(3-{[(dimethylamino)carbonyl]amino}piperidino)-4-oxo-4H-pyrido[1,2-a]pyrimidin-3-yl]-2-propenoic acid

m.p.: 194-200°C (decomp.)
¹H-NMR (DMSO-d₆) δ : 1.31 (9H, s), 1.53-1.72 (2H, m), 1.76-1.85 (1H, m), 1.85-1.94 (1H, m), 2.77 (6H, s), 3.00-3.24 (2H, m), 3.57-3.69 (1H, m), 3.81-3.90 (1H, m), 4.04-4.12 (1H, m), 6.09 (1H, d, J=7.1Hz), 6.80-6.98 (1H, m), 6.92 (1H, d, J=15.7Hz), 7.43 (1H, d, J=15.7Hz), 7.60 (1H, d, J=7.3Hz), 8.22 (1H, brs), 8.89 (1H, d, J=7.4Hz).
IR (microscopic ATR): 2927, 2861, 1673, 1633, 1596, 1513, 1440 cm⁻¹.
FAB/MS; m/z: 568 (MH⁺).
H-R FAB/MS: Calcd. for C₂₇H₃₃N₇O₅S: 568.2342. Found: 568.2339.

### Example 167: (E)-3-[2-{3-[(Aminocarbonyl)amino]piperidino}-8-({[4-(tert-butyl)-1,3-thiazol-2-yl]amino}carbonyl)-4-oxo-4H-pyrido[1,2-a]pyrimidin-3-yl]-2-propenoic acid

¹H-NMR (DMSO-d₆) δ : 1.31 (9H, s), 1.39-1.52 (1H, m), 1.60-1.96 (3H, m), 3.08-3.18 (1H, m), 3.40-3.65 (2H, m), 3.70-3.78 (1H, m), 3.97-4.03 (1H, m), 5.43 (2H, brs), 6.03 (1H, d, J=8.3Hz), 6.86 (1H, brs), 6.92 (1H, d, J=15.5Hz), 7.44 (1H, d, J=15.5Hz), 7.60 (1H, d, J=7.3Hz), 8.19 (1H, brs), 8.90 (1H, d, J=7.3Hz).
IR (microscopic ATR): 2958, 2925, 2856, 1668, 1594, 1540, 1519, 1436 cm⁻¹.
FAB/MS; m/z: 540 (MH⁺).
H-R FAB/MS: Calcd. for C₂₅H₂₉N₇O₅S: 540.2029. Found: 540.2033.

### Example 168: (E)-3-[8-({[4-(tert-Butyl)-1,3-thiazol-2-yl]amino}carbonyl)-2-(3-{[(2-methoxyethyl)amino]carbonyl}piperidino)-4-oxo-4H-pyrido[1,2-a]pyrimidin-3-yl]-2-propenoic acid

m.p.: 183-190°C (decomp.)
¹H-NMR (DMSO-d₆) δ : 1.31 (9H, s), 1.58-1.82 (3H, m), 1.88-1.97 (1H, m), 3.05-3.36 (7H, m), 3.23 (3H, s), 3.90-3.99 (1H, m), 4.08-4.15 (1H, m), 6.87 (1H, brs), 6.93 (1H, d, J=15.4Hz), 7.42 (1H, d, J=15.4Hz), 7.60 (1H, d, J=7.1Hz), 7.87-7.94 (1H, m), 8.22 (1H, brs), 8.90 (1H, d, J=7.3Hz).
IR (microscopic ATR): 3320, 2935, 2865, 1668, 1592, 1519, 1442 cm⁻¹.
FAB/MS; m/z: 583 (MH⁺).
H-R FAB/MS: Calcd. for C₂₈H₃₄N₆O₆S: 583.2339. Found: 583.2358.

### Example 169: (E)-3-{8-({[4-(tert-Butyl)-1,3-thiazol-2-yl]amino}carbonyl)-2-[3-(hydroxymethyl)piperidino]-4-oxo-4H-pyrido[1,2-a]pyrimidin-3-yl}-2-propenoic acid m.p.: 177-187°C (decomp.)

¹H-NMR (DMSO-d₆) δ : 1.31 (9H, s), 1.57-1.79 (1H, m), 1.79-1.83 (3H, m), 2.88-2.98 (1H, m), 3.06-3.17 (1H, m), 3.30-3.43 (3H, m), 3.90-3.98 (1H, m), 4.10-4.19 (1H, m), 4.55 (1H, t, J=4.9Hz), 6.87 (1H, brs), 6.92 (1H, d, J=15.4Hz), 7.43 (1H, d, J=15.4Hz), 7.58 (1H, d, J=6.9Hz), 8.18 (1H, brs), 8.88 (1H, d, J=7.6Hz).
IR (microscopic ATR): 2925, 2856, 1673, 1590, 1521, 1440 cm⁻¹.
FAB/MS; m/z: 512 (MH⁺).
H-R FABIMS: Calcd. for C₂₅H₂₉N₅O₅S: 512.1968. Found: 512.1969.

### Example 170: (E)-3-[8-({[4-(tert-Butyl)-1,3-thiazol-2-yl]amino}carbonyl)-2-(3-{[(2-hydroxyethyl)amino]carbonyl}piperidino)-4-oxo-4H-pyrido[1,2-a]pyrimidin-3-yl]-2-propenoic acid

m.p.: 173-190°C (decomp.)
¹H-NMR (DMSO-d₆) δ : 1.31 (9H, s), 1.59-1.84 (3H,m), 1.88-2.00 (1H, m), 3.05-3.25 (3H, m), 3.88-3.98 (1H, m), 4.10-4.19 (1H, m), 4.60-4.72 (1H, m), 6.86 (1H, brs), 6.94 (1H, d, J=15.6Hz), 7.43 (1H, d, J=15.6Hz), 7.62 (1H, d, J=7.1Hz), 7.86 (1H, t, J=4.9Hz), 8.23 (1H, brs), 8.91 (1H, d, J=7.6Hz).
IR (microscopic ATR): 3318, 2935, 2863, 1668, 1641, 1592, 1519, 1442 cm⁻¹.
FAB/MS; m/z: 569 (MH⁺).
H-R FAB/MS: Calcd. for C₂₇H₃₂N₆O₆S: 569.2182. Found: 569.2160.

### Example 171: (E)-3-[2-(3-{[(Aminocarbonyl)oxylmethyl}piperidino)-8-({[4-(tert-butyl)-1,3-thiazol-2-yl]amino}carbonyl)-4-oxo-4H-pyrido[1,2-a]pyrimidin-3-yl]-2-propenoic acid

m.p.: 167-178°C (decomp.)
¹H-NMR (DMSO-d₆) δ : 1.31 (9H, s), 1.58-1.72 (1H, m), 1.72-1.90 (2H, m), 1.91-2.03 (1H, m), 2.92-3.01 (1H, m), 3.07-3.16 (1H, m), 3.30-3.42 (1H, m), 3.80-3.93 (3H, m), 4.08-4.14 (1H, m), 6.48 (2H, brs), 6.87 (1H, brs), 6.94 (1H, d, J=15.5Hz), 7.44 (1H, d, J=15.5Hz), 7.61 (1H, d, J=6.6Hz), 8.19 (1H, brs), 8.91 (1H, d, J=7.6Hz).
IR (microscopic ATR): 3673, 3345, 3181,2967, 2900, 2865, 1673, 1592, 1519, 1442, 1409 cm⁻¹.
FAB/MS; m/z: 555 (MH⁺).
H-R FAB/MS: Calcd. for C₂₆H₃₀N₆O₆S: 555.2026. Found: 555.2026.

### Example 172: N⁸-[4-(tert-Butyl)-1,3-thiazol-2-yl]-2-{3-[(aminocarbonyl)amino]-piperidino}-4-oxo-3-[(E)-2-(2H-1,2,3,4-tetrazol-5-yl)-1-ethenyl]-4H-pyrido[1,2-a]-pyrimidine-8-carboxyamide

m.p.: 260-310°C (decomp.)
¹H-NMR (DMSO-d₆) δ : 1.31 (9H, s), 1.40-1.52 (1H, m), 1.68-1.88 (2H, m), 1.89-1.99 (1H, m), 2.93-3.30 (2H, m), 3.61-3.73 (1H, m), 3.84-3.93 (1H, m), 3.99-4.07 (1H, m), 5.51 (2H, brs), 6.11 (1H, d, J=8.3Hz), 6.88 (1H, brs), 7.44 (1H, d, J=15.9Hz), 7.63 (1H, d, J=6.9Hz), 7.83 (1H, d, J=15.9Hz), 8.22 (1H, brs), 8.94 (1H, d, J=7.3Hz).
IR (microscopic ATR): 3318, 3095, 2925, 2854, 1670, 1614, 1519, 1440 cm⁻¹.
FAB/MS; m/z: 564 (MH⁺).
H-R FAB/MS: Calcd. for C₂₅H₂₉N₁₁O₃S: 564.2254. Found: 564.2285.

### Example 173: (E)-3-{8-({[4-(tert-Butyl)-1,3-thiazol-2-yl]amino}carbonyl)-2-[3-({[2-(dimethylamino)ethyl]amino}carbonyl)piperidino]-4-oxo-4H-pyrido[1,2-a]pyrimidin-3-yl}-2-propenoic acid

¹H-NMR (400MHz, DMSO-d₆) δ : 1.31 (9H, s), 1.59-1.72 (2H, m), 1.72-1.86 (1H, m), 1.86-1.98 (1H, m), 2.22 (6H, s), 2.32-2.45 (2H, m), 3.05-3.35 (5H, m), 4.01 (1H, brd, J=12.4Hz), 4.09 (1H, brd, J=12.4Hz), 6.85 (1H, s), 6.92 (1H, d, J=15.4Hz), 7.42 (1H, d, J=15.4Hz), 7.61 (1H, d, J=7.6Hz), 7.80-7.90 (1H, m), 8.19 (1H, s), 8.89 (1H, d, J=7.3Hz).
IR (microscopic ATR): 2958, 2937, 2861, 1660, 1519, 1440 cm⁻¹.
m.p.: 177-187°C (decomp.)
FAB/MS m/z: 596 (MH⁺).
HR-FAB/MS: 596.2646 (Calcd. for C₂₉H₃₇N₇O₅S: 596.2655).

### Example 174: (E)-3-[8-({[4-(tert-Butyl)-1,3-thiazol-2-yl]amino}carbonyl)-2-(3-{[3-(dimethylamino)propanoyl]amino}piperidino)-4-oxo-4H-pyrido[1,2-a]pyrimidin-3-yl]-2-propenoic acid

¹H-NMR (400MHz, DMSO-d₆) δ : 1.31 (9H, s), 1.43-1.60 (1H, m), 1.60-1.76 (1H, m), 1.78-1.96 (2H, m), 2.27 (6H, s), 2.25-2.38 (1H, m), 2.59-2.71 (1H, m), 3.05-3.25 (3H, m), 3.63-3.75 (2H, m), 3.75-3.87 (1H, m), 3.87-3.98 (1H, m), 6.85 (1H, s), 6.92 (1H, d, J=15.4Hz), 7.44 (1H, d, J=15.4Hz), 7.61 (1H, dd, J=7.3, 2.0Hz), 8.13 (1H, brd, J=7.1Hz), 8.16 (1H, d, J=2.0Hz), 8.90 (1H, d, J=7.3Hz).
IR (microscopic ATR): 2956, 2360, 1660, 1515, 1440 cm⁻¹.
m.p.: 177-197°C (decomp.)
FAB/MS m/z: 596 (MH⁺).
HR-FAB/MS: 596.2646 (Calcd. for C₂₉H₃₇N₇O₅S: 596.2655).

### Example 175: (2-{[(1-{8-({[4-(tert-Butyl)-1,3-thiazol-2-yl]amino}carbonyl)-3-[(E)-2-carboxy-1-ethenyl]-4-oxo-4H-pyrido[1,2-a]pyrimidin-2-yl}-3-piperidyl)carbonyl]-amino}ethyl)(trimethyl)ammonium iodide

(E)-3-{8-({[4-(tert-Butyl)-1,3-thiazol-2-yl]amino}carbonyl)-2-[3-({[2-(dimethylamino)ethyl]amino}carbonyl)piperidino]-4-oxo-4H-pyrido[1,2-a]pyrimidin-3-yl}-2-propenoic acid (D11-4378, 15.4 mg, 0.026 mmol) was dissolved in DMF (1 ml), added dropwise with methyl iodide (100 µl) and left in a refrigerator for 24 hours. The solvent and the excessive reagents were evaporated, and the residue was subjected to azeotropy with toluene and ether. The residue was lyophilized from dioxane/water to obtain 20.6 mg (quantitative) of the title compound as yellow orange powder.
¹H-NMR (400MHz, DMSO-d₆) δ : 1.31 (9H, s), 1.60-1.75 (2H, m), 1.75-1.83 (1H, m), 1.92-2.02 (1H, m), 3.09 (9H, s), 3.05-3.55 (7H, m), 3.90-3.98 (1H, m), 4.07-4.14 (1H, m), 6.88 (1H, brs), 6.94 (1H, d, J=15.4Hz), 7.43 (1H, d, J=15.4Hz), 7.64 (1H, d, J=7.1Hz), 8.18 (1H, s), 8.21 (1H, brt, J=5.4Hz), 8.92 (1H, d, J=7.3Hz).
IR (microscopic ATR) cm⁻¹: 3409, 2956, 2763, 2362, 1664, 1590, 1517, 1444.
m.p.: 132-142°C (decomp.)
FAB/MS m/z: 610 (M⁺).
HR-FAB/MS: 610.2803 (Calcd. for C₃₀H₄₀N₇O₅S: 610.2812).

In a similar manner, the following compounds were synthesized.

### Example 176: {3-[(1-{8-({[4-(tert-Butyl)-1,3-thiazol-2-yl]amino}carbonyl)-3-[(E)-2-carboxy-1-ethenyl]-4-oxo-4H-pyrido[1,2-a]pyrimidin-2-yl}-3-piperidyl)amino]-3-oxopropyl}(trimethyl)ammonium iodide

¹H-NMR (400MHz, DMSO-d₆) δ : 1.31 (9H, s), 1.47-1.60 (1H, m), 1.60-1.77 (1H, m), 1.80-2.00 (2H, m), 2.60-2.78 (2H, m), 3.04 (9H, s), 3.12-3.60 (4H, m), 3.70-3.90 (2H, m), 3.92-4.02 (1H, m), 6.88 (1H, brs), 6.94 (1H, d, J=15.6Hz), 7.45 (1H, d, J=15.6Hz), 7.64 (1H, d, J=7.6Hz), 8.00-8.30 (2H, m), 8.22 (1H, d, J=6.9Hz), 8.92 (1H, d, J=7.6Hz).
IR (microscopic ATR): 3019, 2981,2807, 2773, 1670, 1521, 1457, 1403 cm⁻¹.
m.p.: 117-124°C (decomp.)
FAB/MS m/z: 610 (M⁺).
HR-FAB/MS: 610.2830 (Calcd. for C₃₀H₄₀N₇O₅S: 610.2812).

### Example 177: (E)-3-[8-({[4-(tert-Butyl)-1,3-thiazol-2-yl]amino}carbonyl)-2-(3-{[2-(dimethylamino)acetyl]amino}piperidino)-4-oxo-4H-pyrido[1,2-a]pyrimidin-3-yl]-2-propenoic acid

¹H-NMR (400MHz, CDCl₃) δ : 1:39 (9H, s), 1.72-2.17 (4H, m), 2.08 (6H, s), 2.84 (1H, ABq, J=15.9Hz), 2.91 (1H, ABq, J=15.9Hz), 3.53-3.66 (1H, m), 3.69-4.00 (3H, m), 4.12-4.22 (1H, m), 6.66 (1H, s), 7.32 (1H, d, J=15.6Hz), 7.74 (1H, d, J=15.6Hz), 7.78 (1H, d, J=6.4Hz), 7.83 (1H, d, J=7.1Hz), 8.32 (1H, s), 9.09 (1H, d, J=7.3Hz).
IR (ATR) cm⁻¹: 3050, 2946, 2830, 2778, 1668, 1594, 1511, 1438.
m.p.: 193-203°C (decomp.)
FAB/MS m/z: 582 (MH⁺).
HR-FAB/MS: 582.2492 (Calcd. for C₂₈H₃₅N₇O₅S: 582.2499).

### Example 178: (E)-3-[8-({[4-(tert-Butyl)-1,3-thiazol-2-yl]amino}carbonyl)-4-oxo-2-(3-{[2-(1,1,1-trimethylammonio)acetyl]amino}piperidino)-4H-pyrido[1,2-a]pyrimidin-3-yl]-2-propenoate

¹H-NMR (400MHz, CD₃OD) δ : 1.33 (9H, s), 1.64-1.90 (2H, m), 1.90-2.08 (2H, m), 3.32 (9H, s), 3.37-3.53 (2H, m), 3.60-3.76 (1H, m), 3.80-3.96 (1H, m), 4.02-4.24 (3H, m), 6.75 (1H, s), 7.07 (1H, d, J=15.4Hz), 7.58 (1H, d, J=15.4Hz), 7.63 (1H, d, J=7.6Hz), 8.02 (1H, s), 8.98 (1H, d, J=7.3Hz).
IR (ATR) cm⁻¹: 3193, 2958, 2854, 1662, 1554, 1515, 1440.
m.p.: 213-225°C (decomp.)
FAB/MS m/z: 596 (MH⁺).
Anal. Calcd. for C₂₉H₃₇N₇O₅S·7H₂O: C, 48.25; H, 7.12; N, 13.58. Found: C, 47.85; H, 6.97; N, 13.36.

### Example 179: N⁸-[4-(tert-Butyl)-1,3-thiazol-2-yl]-2-((3S)-3-{[2-(dimethylamino)acetyl]-amino}hexahydro-1-pyridinyl)-4-oxo-3-[(E)-2-(1H-1,2,3,4-tetrazol-5-yl)-1-ethenyl]-4H-pyrido[1,2-a]pyrimidine-8-carboxyamide

### (A) N⁸-[4-(tert-Butyl)-1,3-thiazol-2-yl]-2-((3S)-3-{[2-(dimethylamino)acetyl]amino}hexahydro-1-pyridinyl)-3-{(E)-2-[1-(4-methoxybenzyl)-1H-1,2,3,4-tetrazol-5-yl]-1-ethenyl}-4-oxo-4H-pyrido[1,2-a]pyrimidine-8-carboxyamide

N⁸-[4-(tert-Butyl)-1,3-thiazol-2-yl]-2-hydroxy-3-{(E)-2-[1-(4-methoxybenzyl)-1 H-1,2,3,4-tetrazol-5-yl]-1-ethenyl}-4-oxo-4H-pyrido[1,2-a]pyrimidine-8-carboxamide (106.3 mg, 0.19 mmol) was suspended in acetonitrile (3 ml) and DMF (3 ml), added dropwise with diphenyl chlorophosphate (79.0 µl, 0.38 mmol) and diisopropylethylamine (132.6 µl, 0.76 mmol) at -10°C under argon atmosphere, stirred-at the same temperature for 15 minutes, then added dropwise with a solution of N¹-[(3S)-hexahydro-3-pyridinyl]-2-(dimethylamino)acetamide (70.5 mg, 0.38 mmol) in DMF (1 ml) and diisopropylethylamine (66.3 µl, 0.38 mmol) and heated to 80°C for 1 hour with stirring. The reaction solution was added with a solution of N¹-[(3S)-hexahydro-3-pyridinyl]-2-(dimethylamino)acetamide (70.5 mg, 0.38 mmol) in DMF (1 ml) and diisopropylethylamine (66.3 µl, 0.38 mmol), further heated to 80°C for 1 hour with stirring and then cooled. The reaction mixture was added with saturated aqueous sodium hydrogencarbonate and extracted with chloroform. The resulting organic layer was washed with saturated brine and dried over anhydrous sodium sulfate, and the solvent was evaporated. The residue was purified by preparative TLC (chloroform:methanol = 10:1) to obtain the title compound (111.5 mg, 80.7%) as yellow orange oily substance.
¹H-NMR (400MHz, CDCl₃) δ : 1.37 (9H, s), 1.45-1.94 (4H, m), 2.20 (6H, s), 2.85-3.10 (1H, m), 3.12-3.24 (1H, m), 3.28-3.40 (1H, m), 3.42-3.68 (2H, m), 3.78 (3H, s), 3.80-4.01 (1H, m), 4.07-4.20 (1H, m), 5.52 (2H, s), 6.56 (2H, s), 6.90 (2H, d, J=8.6Hz), 7.36 (2H, d, J=8.6Hz), 7.52 (1H, d, J=7.1Hz), 7.72 (1H, d, J=15.5Hz), 7.90 (1H, d, J=15.5Hz), 8.01 (1H, s), 8.88 (1H, d, J=7.3Hz).

### (B) N⁸-[4-(tert-Butyl)-1,3-thiazol-2-yl]-2-((3S)-3-{[2-(dimethylamino)acetyl]amino}hexahydro-1-pyridinyl)-4-oxo-3-[(E)-2-(1H-1,2,3,4-tetrazol-5-yl)-1-ethenyl]-4H-pyrido[1,2-a]pyrimidine-8-carboxyamide

N⁸-[4-(tert-Butyl)-1,3-thiazol-2-yl]-2-((3S)-3-{[2-(dimethylamino)acetyl]-amino}exahydrol-pyridinyl)-3-{(E)-2-[1-(4-methoxybenzyl)-1H-1,2,3,4-tetrazol-5-yl]-1-ethenyl}-4-oxo-4H-pyrido[1,2-a]pyrimidine-8-carboxamide (92.9 mg, 0.13 mmol) was dissolved in trifluoroacetic acid (20 ml) and stirred at room temperature for 62 hours, and the excessive reagent was evaporated. The residue was subjected to azeotropy with toluene and ether, and the residue was purified by preparative TLC (chloroform:methanol:water = 8:3:0.5). The solvent was evaporated, and the residue was powdered with ether and centrifuged. The supernatant was removed and the residue was dried under reduced pressure to obtain the title compound (28.9 mg, 30.1%) as orange powder.
¹H-NMR (400MHz, CD₃OD) δ : 1.35 (9H, s), 1.72-1.85 (2H, m), 1.90-2.02 (2H, m), 2.65 (6H, s), 3.46-3.75 (3H, m), 3.64 (2H, s), 3.75-3.82 (1H, m), 4.09-4.18 (1H, m), 6.74 (1H, s), 7:46 (1H, d, J=16.2Hz), 7.62 (1H, dd, J=7.6, 1.8Hz), 7.95 (1H, d, J=16.2Hz), 8:04 (1H, s), 9.00 (1H, d, J=7.6Hz).
IR (ATR) cm⁻¹: 3052, 2954, 2859, 1658, 1509, 1425.
m.p.: 201-207°C (decomp.)
FAB/MS m/z: 606 (MH⁺).
HR-FAB/MS: 606.2714 (Calcd. for C₂₈H₃₅N₁₁O₃S: 606.2723).

### Example 180: N⁸-[4-(tert-Butyl)-1,3-thiazol-2-yl]-4-oxo-3-[(E)-2-(1H-1,2,3,4-tetrazol-5-yl)-1-ethenyl]-2-((3S)-3-{[2-(1,1,1-trimethylammonio)acetyl]amino}hexahydro-1-pyridinyl}-4H-pyrido[1,2-a]pyrimidine-8-carboxyamide formate

N⁸-[4-(tert-Butyl)-1,3-thiazol-2-yl)-2-((3S)-3-{(2-(dimethylamino)acetyl]-amino)hexahydro-1-pyridinyl)-4-oxo-3-[(E)-2-(1H-1,2,3,4-tetrazol-5-yl)-1-ethenyl]-4H-pyrido[1,2-a]pyrimidine-8-carboxamide (D11-5061, 26.2 mg, 0.043 mmol) was dissolved in DMF (3 ml), added dropwise with methyl iodide (300 µl) and left in a refrigerator for 62 hours. The solvent and the excessive reagents were evaporated and the residue was subjected to azeotropy with toluene and ether. The residue was powdered by addition of ether, centrifuged and dried after the supernatant was removed. The resulting powder was purified by preparative TLC (chloroform:methanol = 10:1, developed twice) and HPLC and lyophilized from dioxane and water to obtain the title compound (23.9 mg, 30.1%) as orange powder.
HPLC conditions
Column: CAPCELL PAK C18 SG120A, 5 µ m, 30 mm ϕ x 250 mm (SHISEIDO)
Mobile phase: H₂O:CH₃CN = 70:30 (0.1% HCO₂H)
Detection wavelength: UV 254 nm
FR: 8 ml/min
Retention time: 12.0 min
¹H-NMR (400MHz, CD₃OD) δ : 1.35 (9H, s), 1.70-1.87 (2H, m), 1.92-2.10 (2H, m), 3.25 (9H, s), 3.46-3.58 (2H, m), 3.60-3.77 (2H, m), 4.08-4.23 (3H, m), 6.75 (1H, s), 7.49 (1H, d, J=15.6Hz), 7.64 (1H, d, J=7.6Hz), 7.96 (1H, d, J=15.6Hz), 8.06 (1H, s), 9.03 (1H, d, J=7.3Hz).
IR (ATR) cm⁻¹: 2954, 2923, 2854, 1733, 1670, 1548, 1519, 1444.
m.p.: 211-215°C (decomp.)
FAB/MS m/z: 620 (MH⁺).
HR-FAB/MS: 620.2824 (Calcd. for C₂₉H₃₇N₁₁O₃S: 620.2880).

### Example 181: (E)-3-{8-({[4-(tert-Butyl)-1,3-thiazol-2-yl]amino}carbonyl)-4-oxo-2-[(3R)-3-({[(2-tetrahydro-1H-1-pyrrolylethyl)amino]carbonyl}oxy)hexahydro-1-pyridinyl]-4H-pyrido[1,2-a]pyrimidin-3-yl}-2-propenoic acid

### (A) tert-Butyl (E)-3-{8-({[4-(tert-butyl)-1,3-thiazol-2-yl]amino}carbonyl)-4-oxo-2-[(3R)-3-({[(2-tetrahydro-1H-1-pyrrolylethyl)amino]carbonyl}oxy)hexahydro-1-pyridinyl]-4H-pyrido[1,2-a]pyrimidin-3-yl}-2-propenoate

tert-Butyl (E)-3-{8-({[4-(tert-butyl)-1,3-thiazol-2-yl]amino}carbonyl)-2-[(3R)-3-({[(2-chloroethyl)amino]carbonyl}oxy)hexahydro-1-pyridinyl]-4-oxo-4H-pyrido[1,2-a]pyrimidin-3-yl}-2-propenoate (0.51 g, purity: about 58%) was dissolved in DMF (5 ml) and added dropwise with pyrrolidine (375 µl, 4.49 mmol). The reaction solution was stirred under an argon flow at about 80°C for 4 hours, further added with pyrrolidine (375 µl, 4.49 mmol) and stirred at about 80°C for 3 hours. The reaction mixture was further added with pyrrolidine (375 µl, 4.49 mmol), stirred at about 80°C for 8 hours, added with saturated aqueous sodium hydrogencarbonate and extracted with chloroform, the resulting organic layer was washed with saturated brine and dried over anhydrous sodium sulfate, and the solvent was evaporated. The residue was purified by preparative TLC (chloroform:methanol = 20:1) to obtain the title compound (235.8 mg, 75.7%) as a mixture of orange oily substance and solid.
¹H-NMR (400MHz, CDCl₃) δ : 1.33 (9H, s), 1.52 (9H, s), 1.58-1.75 (5H, m), 1.80-2.00 (3H, m), 2.25-2.78 (6H, m), 3.25-3.82 (6H, m), 4.80-4.95 (1H, m), 5.75-5.86 (1H, m), 6.58 (1H, s), 7.07 (1H, d, J=15.4Hz), 7.48 (1H, d, J=7.3Hz), 7.49 (1H, d, J=15.4Hz), 7.87 (1H, s), 8.96 (1H, d, J=7.3Hz).

### (B) (E)-3-{8-({[4-(tert-Butyl)-1,3-thiazol-2-yl]amino}carbonyl)-4-oxo-2-[(3R)-3-({[(2-tetrahydro-1H-1-pyrrolylethyl)amino]carbonyl}oxy)hexahydro-1-pyridinyl]-4H-pyrido[1,2-a]pyrimidin-3-yl}-2-propenoic acid

tert-Butyl (E)-3-{8-({[4-(tert-butyl)-1,3-thiazol-2-yl]amino}carbonyl)-4-oxo-2-[(3R)-3-({[(2-tetrahydro-1H-1-pyrrolylethyl)amino]carbonyl}oxy)hexahydro-1-pyridinyl]-4H-pyrido[1,2-a]pyrimidin-3-yl}-2-propenoate (235.8 mg, 0.34 mmol) was dissolved in 4 N hydrochloric acid solution in dioxane (20 ml) and stirred at room temperature for 1 hour. The excessive reagent was evaporated, and the residue was subjected to azeotropy with toluene and ether. The residue was purified by preparative TLC (chloroform:methanol = 10:1) to obtain the title compound (197.3 mg, 91.0%) as yellow orange solid.
¹H-NMR (400MHz, CD₃OD) δ : 1.35 (9H, s), 1.69-1.87 (2H, m), 1.90-2.18 (6H, m), 3.05-3.45 (9H, m), 3.52-3.63 (1H, m), 3.90-4.00 (1H, m), 4.08-4.19 (1H, m), 4.92-5.01 (1H, m), 6.74 (1H, s), 7.15 (1H, d, J=15.5Hz), 7.60 (1H, d, J=7.3Hz), 7.76 (1H, d, J=15.5Hz), 8.03 (1H, s), 8.97 (1H, d, J=7.3Hz).
IR (ATR) cm⁻¹: 2956, 2859, 1706, 1666, 1594, 1513, 1438.
m.p.: 162-182°C (decomp.)
FAB/MS m/z: 638 (MH⁺).
HR-FAB/MS: 638.2772 (Calcd. for C₃₁H₃₉N₇O₆S: 638.2761).

### Example 182: (E)-3-(8-({[4-(tert-Butyl)-1,3-thiazol-2-yl]amino}carbonyl)-2-{(3R)-3-[({[2-(1-methyltetrahydro-1H-1-pyrrolyl)ethyl]amino}carbonyl)oxy]hexahydro-1-pyridinyl}-4-oxo-4H-pyrido[1,2-a]pyrimidin-3-yl)-2-propenoic acid iodide

(E)-3-{8-({[4-(tert-Butyl)-1,3-thiazol-2-yl]amino}carbonyl)-4-oxo-2-[(3R)-3-({[(2-tetrahydro-1H-1-pyrrolylethyl)amino]carbonyl}oxy)hexahydrol-pyridinyl]-4H-pyrido[1,2-a]pyrimidin-3-yl}-2-propenoic acid (D11-5127, 99.2 mg, 0.16 mmol) was dissolved in DMF (10 ml), added dropwise with methyl iodide (1 ml) and left in a refrigerator for 12 hours. The solvent and the excessive reagents were evaporated, and the residue was subjected to azeotropy with toluene and ether. The residue was powdered by addition of ether and centrifuged. The supernatant was removed and the residue was dried to obtain 124.9 mg of pale orange powder.

This compound (70 mg) was added with 4 N hydrochloric acid solution in dioxane (4 ml) and stirred, and the excessive reagent was evaporated. This operation was further repeated twice, and the residue was subjected to azeotropy with toluene and ether. The residue was purified by preparative TLC (chloroform:methanol:water = 8:3:0.5). The residue was powdered by addition of ether and centrifuged. The supernatant was removed, and the residue was dried to obtain the title compound (52.3 mg, 76.9%) as yellow orange powder.
¹H-NMR (400MHz, CD₃OD) δ : 1.35 (9H, s), 1.60-1.73 (1H, m), 1.85-2.08 (3H, m), 2.10-2.28 (4H, m), 3.13 (3H, s), 3.30-3.68 (11H, m), 3.83-4.07 (2H, m), 6.73 (1H, s), 7.03 (1H, d, J=15.6Hz), 7.55 (1H, d, J=15.6Hz), 7.57 (1H, dd, J=7.3, 2.0Hz), 7.96 (1H, s), 8.92 (1H, d, J=7.3Hz).
IR (ATR) cm⁻¹: 3384, 2958, 2861, 1700, 1662, 1596, 1508, 1438.
m.p.: 182-202°C (decomp.)
FAB/MS m/z: 652 (MH⁺).
HR-FAB/MS: 652.2962 (Calcd. for C₃₂H₄₁N₇O₆S: 652.2917).
Anal. Calcd. for C₃₂H₄₁N₇O₆S·HI·4.8H₂O: C, 44.37; H, 6.00; N, 11.32. Found: C, 43.95; H, 5.51; N, 11.07.

### Example 183: N⁸-[4-(tert-Butyl)-1,3-thiazol-2-yl]-2-[(3S)-3-({2-[1-(2-amino-2-oxoethyl)-1,1-dimethylammonio]acetyl}amino)hexahydro-1-pyridinyl]-4-oxo-3-[(E)-2-(1H-1,2,3,4-tetrazol-5-yl)-1-ethenyl]-4H-pyrido[1,2-a]pyrimidine-8-carboxyamide trifluoroacetate

### (A) N⁸-[4-(tert-Butyl)-1,3-thiazol-2-yl]-2-[(3S)-3-({2-[1-(2-amino-2-oxoethyl)-1,1-dimethylammonio]acetyl}amino)hexahydro-1-pyridinyl]-3-{(E)-2-[1-(4-methoxybenzyl)-1H-1,2,3,4-tetrazol-5-yl]-1-ethenyl}-4-oxo-4H-pyrido[1,2-a]pyrimidine-8-carboxyamideiodide

N⁸-[4-(tert-Butyl)-1,3-thiazol-2-yl]-2-((3S)-3-{[2-(dimethylamino)acetyl]-amino}hexahydro-1-pyridinyl)-3-{(E)-2-[1-(4-methoxybenzyl)-1H-1,2,3,4-tetrazol-5-yl]-1-ethenyl}-4-oxo-4H-pyrido[1,2-a]pyrimidine-8-carboxamide (468.3 mg, 0.65 mmol) was dissolved in DMF (30 ml), added with iodoacetamide (155.1 mg, 0.84 mmol) and stirred at room temperature for 16 hours. The solvent was evaporated, and the residue was subjected to azeotropy with toluene and ether. The residue was purified by preparative TLC (chloroform:methanol:water = 8:3:0.1) to obtain the title compound (406.0 mg, 69.1%) as a mixture of orange oily substance and foamy substance.
¹H-NMR (400MHz, CD₃OD) δ : 1.35 (9H, s), 1.65-1.80 (2H, m), 1.84-2.04 (2H, m), 3.42 (3H, s), 3.44 (3H, s), 3.40-3.58 (3H, m), 3.77 (3H, s), 3.68-3.88 (3H, m), 4.03-4.12 (1H, m), 3.35-3.48 (3H, m), 4.50-4.62 (1H, m), 5.61 (1H, ABq, J=15.4Hz), 5.66 (1H, ABq, J=15.4Hz), 6.76 (1H, s), 6.93 (2H, d, J=8.8Hz), 7.35 (2H, d, J=8.8Hz), 7.62-7.70 (1H, m), 7.69 (1H, d, J=15.6Hz), 7.88 (1H, d, J=15.6Hz), 7.90 (1H, s), 8.06 (1H, s), 9.01 (1H, d, J=7.3Hz).

### (B) N⁸-[4-(tert-Butyl)-1,3-thiazol-2-yl]-2-[(3S)-3-({2-[1-(2-amino-2-oxoethyl)-1,1-dimethylammonio]acetyl}amino)hexahydro-1-pyridinyl]-4-oxo-3-[(E)-2-(1H-1,2,3,4-tetrazol-5-yl)-1-ethenyl]-4H-pyrido[1,2-a]pyrimidine-8-carboxyamide trifluoroacetate

N⁸-[4-(tert-Butyl)-1,3-thiazol-2-yl]-2-[(3S)-3-({2-[1-(2-amino-2-oxoethyl)-1,1-dimethylammonio]acetyl}amino)hexahydro-1-pyridinyl]-3-{(E)-2-[1-(4-methoxybenzyl)-1H-1,2,3,4-tetrazol-5-yl]-1-ethenyl}-4-oxo-4H-pyrido[1,2-a]pyrimidine-8-carboxamide iodide (401.3 mg, 4.41mmol) was dissolved in TFA (80 ml) and stirred at room temperature for 89 hours. The solvent was evaporated, and the residue was subjected to azeotropy with toluene and ether. The residue was purified by preparative TLC (chloroform:methanol water = 8:3:0.5). The solvent was evaporated, and the residue was powdered by addition of ether and centrifuged. The supernatant was removed, and the residue was dried under reduced pressure to obtain the title compound (172.6 mg, 50.4%) as orange powder.
¹H-NMR (400MHz, CD₃OD) δ : 1.35 (9H, s), 1.65-1.84 (2H, m), 1.88-2.04 (2H, m), 3.35 (3H, s), 3.39 (3H, s), 3.30-3.53 (2H, m), 3.68-3.85 (2H, m), 4.05-4.16 (1H, m), 4.30 (1H, ABq, J=15.4Hz), 4.37 (1H, ABq, J=15.4Hz), 4.43 (2H, s), 6.75 (1H, s), 7.44 (1H, d, J=16.1Hz), 7.61 (1H, dd, J=7.5, 1.7Hz), 7.91 (1H, d, J=16.1Hz), 8.03 (1H, d, J=1.7Hz), 8.99 (1H, d, J=7.8Hz).
IR (ATR) cm⁻¹: 3181,3050, 2960, 2865, 1664, 1544, 1513, 1423.
m.p.: 180-203°C (decomp.)
FAB/MS m/z: 663 (MH⁺).
HR-FAB/MS: 663.2910 (Calcd. for C₃₀H₃₉N₁₂O₄S: 663.2938).
Anal. Calcd. for C₃₀H₃₉N₁₂O₄S·C₂F₃O₂·2.6H₂O: C, 46.66; H, 5.41; N, 20.41. Found: C, 47.23; H, 5.44; N, 19.77.

### Example 184: (E)-3-[2-((3R)-3-{[({2-[1-(2-Amino-2-oxoethyl)tetrahydro-1H-1-pyrrolyl]ethyl}amino)carbonyl]oxy}hexahydro-1-pyridinyl)-8-({[4-(tert-butyl)-1,3-thiazol-2-yl]amino}carbonyl)-4-oxo-4H-pyrido[1,2-a]pyrimidin-3-yl]-2-propenoate

¹H-NMR (400MHz, CD₃OD) δ: 1.35 (9H, s), 1.63-1.76 (1H, m), 1.83-1.96 (1H, m), 1.96-2.27 (6H, m), 3.20-3.40 (2H, m), 3.44-3.99 (10H, m), 4.18-4.27 (2H, m), 4.53-4.61 (1H, m), 6.75 (1H, s), 7.12 (1H, d, J=15.6Hz), 7.55-7.62 (1H, m), 7.59 (1H, d, J=15.6Hz), 8.02 (1H, s), 8.97 (1H, d, J=7.6Hz).
IR (ATR) cm⁻¹: 2954, 2859, 1660, 1637, 1598, 1511, 1432.
m.p.: 188-199°C (decomp.)
FAB/MS m/z: 695 (MH⁺).
HR-FAB/MS: 695:2957 (Calcd. for C₃₃H₄₂N₈O₇S: 695.2975).

### Example 185: (E)-3-[8-({[4-(tert-Butyl)-1,3-thiazol-2-yl]amino}carbonyl)-4-oxo-2-((3S)-3-{[2-(1,1,1-trimethylammonio)acetyl]amino}hexahydro-1-pyridinyl)-4H-pyrido[1,2-a]pyrimidin-3-yl]-2-propenoate hydrochloride

¹H-NMR (400MHz, CD₃OD) δ : 1.34 (9H, s), 1.67-1.85 (2H, m), 1.85-2.05 (2H, m), 3.33 (9H, s), 3.40-3.52 (2H, m), 3.52-3.68 (1H, m), 3.75-3.86 (1H, m), 4.05-4.15 (1H, m), 4.14 (1H, ABq, J=15.1Hz), 4.23 (1H, d, J=15.1Hz), 6.72 (1H, s), 7.04 (1H, d, J=15.6Hz), 7.45 (1H, d, J=15.6Hz), 7.56 (1H, d, J=7.6Hz), 7.94 (1H, s), 8.90 (1H, d, J=7.6Hz).
IR (ATR) cm⁻¹: 3345, 3189, 3046, 2956, 2861, 1664, 1596, 1540, 1513, 1438.
m.p.: 207-217°C (decomp.)
FAB/MS m/z: 596 (MH⁺).
HR-FAB/MS: 596.2672 (Calcd. for C₂₉H₃₇N₇O₅S: 596.2655).
Anal. Calcd. for C₂₉H₃₇N₇O₅S·HCl·3.9H₂O: C, 49.59; H, 6.57; N, 13.96. Found: C, 49.56; H, 6.05; N, 13.45.

### Example 186: (E)-3-[2-[(3S)-3-({2-[1-(2-Amino-2-oxoethyl)-1,1-dimethylammonio]-acetyl}amino)hexahydro-1-pyridinyl]-8-({[4-(tert-butyl)-1,3-thiazol-2-yl]amino}carbonyl)-4-oxo-4H-pyrido[1,2-a]pyrimidin-3-yl]-2-propenoate hydrochloride

¹H-NMR (400MHz, CD₃OD) δ : 1.35 (9H, s), 1.68-1.83 (2H, m), 1.88-2.01 (2H, m), 3.43 (3H, s), 3.46 (3H, s), 3.40-3.80 (4H, m), 4.05-4.14 (1H, m), 4.36-4.52 (4H, m), 6.76 (1H, s), 7.08 (1H, d, J=15.6Hz), 7.44 (1Hd, J=15.6Hz), 7.61 (1H, dd, J=7.4, 1.5Hz), 8.03 (1H, d, J=1.5Hz), 8.97 (1H, d, J=7.4Hz).
IR (ATR) cm⁻¹: 3189, 2958, 2859, 1660, 1542, 1515, 1438.
m.p.: 185-197°C (decomp.)
FAB/MS m/z: 639 (MH⁺).
HR-FAB/MS: 639.2697 (Calcd. for C₃₀H₃₈N₈O₆S: 639.2713).
Anal. Calcd. for C₃₀H₃₈N₈O₆S•2HCl•5.4H₂O: C, 44.54; H, 6.33; N, 13.85; S, 3.96. Found: C, 45.09; H, 6.00; N, 13.00; S, 3.90.

### Example 187: (E)-3-[8-({[4-(tert-Butyl)-1,3-thiazol-2-yl]amino}carbonyl)-4-oxo-2-((3R)-3-{[2-(1,1,1-trimethylammonio)acetyl]amino]hexahydro-1-pyridinyl)-4H-pyrido[1,2-a]pyrimidin-3-yl]-2-propenoate hydrochloride

¹H-NMR (400MHz, CD₃OD) δ : 1.35 (9H, s), 1.68-1.85 (2H, m), 1.90-2.02 (2H, m), 3:32 (9H, s), 3.45-3.57 (2H, m), 3.57-3.68 (1H, m), 3.72-3.80 (1H, m), 4.08-4.17 (1H, m), 4.11 (1H, ABq, J=15.1Hz), 4.23 (1H, ABq, J=15.1Hz), 6.75 (1H, s), 7.10 (1H, d, J=15.7Hz), 7.49 (1Hd, J=15.7Hz), 7.62 (1H, d, J=7.3Hz), 8.02 (1H, s), 8.97 (1H, d, J=7.3Hz).
IR (ATR) cm⁻¹: 3199, 3050, 2954, 2929, 2857, 1664, 1596, 1540, 1513, 1438.
m.p.: 210-220°C (decomp.)
FAB/MS m/z: 596 (MH⁺).
HR-FAB/MS: 596.2660 (Calcd. for C₂₉H₃₇N₇O₅S: 596.2655).

### Example 188: 2-(1-{2-[((3S)-1-{8-({[4-(tert-Butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}hexahydro-3-pyridinyl)amino]-2-oxoethyl}-1,1-dimethylammonio)acetate trifluoroacetate

¹H-NMR (400MHz, CD₃OD) δ : 1.35 (9H, s), 1.68-1.82 (2H, m), 1.90-2.03 (2H, m), 3.36 (6H, s), 3.25-2.50 (2H, m), 3.70-3.80 (1H, m), 3.83-3.92 (1H, m), 3.97-4.09 (2H, m), 4.09-4.18 (1H, m), 4.39 (1H, ABq, J=14.6Hz), 4.54 (1H, ABq, J=14.6Hz), 6.74 (1H, s), 7.53 (1H, d, J=16.1Hz), 7.63 (1H, d, J=7.3Hz), 7.92 (1H, d, J=16.1Hz), 8.10 (1H, s), 9.01 (1H, d, J=7.3Hz).
IR (ATR) cm⁻¹: 3226, 3062, 2967, 2869, 1668, 1635, 1544, 1509, 1438, 1403.
m.p.: 184-238°C (decomp.)
FAB/MS m/z: 664 (MH⁺).
HR-FAB/MS: 664.2796 (Calcd. for C₃₀H₃₇N₁₁O₅S: 664.2778).
Anal. Calcd. for C₃₀H₃₇N₁₁O₅S•3.6C₂HF₃O₂•5.8H₂O: C, 37.91; H, 4.46; N, 13.07; F, 17.41. Found: C, 37.46; H, 3.63; N, 12.39; F, 16.93.

### Example 189: (E)-3-{8-({[4-(tert-Butyl)-1,3-thiazol-2-yl]amino}carbonyl)-2-[(3R)-3-({[(2-hydroxyethyl)amino]carbonyl}oxy)hexahydro-1-pyridinyl]-4-oxo-4H-pyrido[1,2-a]pyrimidin-3-yl}-2-propenoic acid

### (A) tert-Butyl (E)-3-{8-({[4-(tert-butyl)-1,3-thiazol-2-yl]amino}carbonyl)-2-[(3R)-3-({[(2-hydroxyethyl)amino]carbonyl}oxy)hexahydro-1-pyridinyl]-4-oxo-4H-pyrido[1,2-a]pyrimidin-3-yl}-2-propenoate

tert-Butyl (E)-3-{8-({[4-(tert-butyl)-1,3-thiazol-2-yl]amino}carbonyl) -2-hydroxy-4-oxo-4H-pyrido[1,2-a]pyrimidin-3-yl}-2-propenoate (204.7 mg, 0.37 mmol) was dissolved in methylene chloride (20 ml), added with N,N'-carbonyldiimidazole (240.0 mg, 1.48 mmol) and stirred at room temperature for 1 hour and 30 minutes. The reaction mixture was washed with water and saturated brine and dried over anhydrous sodium sulfate, and the solvent was evaporated to obtain 443.3 mg of a crude product.

The crude product was dissolved in THF (15 ml), and the atmosphere in the system was replaced with nitrogen. The solution was added dropwise with triethylamine (154.7 µl, 1.11 mmol) and 2-aminoethanol (44.6 µl, 0.74 mmol) and stirred at room temperature for 14 hours. The solvent and the excessive reagents were evaporated. Then the residue was subjected to azeotropy with ether, added with chloroform, washed successively with 1 N aqueous hydrochloric acid, saturated aqueous sodium hydrogencarbonate and saturated brine and dried over anhydrous magnesium sulfate, and the solvent was evaporated. The residue was purified by preparative TLC (hexane:ethyl acetate = 1:2) to obtain the title compound (182.4 mg, 77.0%) as a mixture of orange yellow oily substance and foamy substance.
¹H-NMR (400MHz, CDCl₃) δ: 1.33 (9H, s), 1.51 (9H, s), 1.60-1.68 (1H, m), 1.78-2.07 (4H, m), 3.20-3.61 (5H, m), 3.61-3.95 (3H, m), 4.03-4.17 (1H, m), 4.90-4.99 (1H, m), 5.67-5.77 (1H, m), 6.59 (1H, s), 7.08 (1H, d, J=15.6Hz), 7.47 (1H, d, J=7.3Hz), 7.54 (1H, d, J=15.6Hz), 7.91 (1H, s), 8.96 (1H, d, J=7.3Hz).

### (B) (E)-3-{8-({[4-(tert-Butyl)-1,3-thiazol-2-yl]amino}carbonyl)-2-[(3R)-3-({[(2-hydroxyethyl)amino]carbonyl}oxy)hexahydro-1-pyridinyl]-4-oxo-4H-pyrido[1,2-a]-pyrimidin-3-yl}-2-propenoic acid

tert-Butyl (E)-3-{8-({[4-(tert-butyl)-1,3-thiazol-2-yl]amino}carbonyl)-2-[(3R)-3-({[(2-hydroxyethyl)amino]carbonyl}oxy)hexahydro-1-pyridinyl]-4-oxo-4H-pyrido[1,2-a]-pyrimidin-3-yl]-2-propenoate (182.4 mg, 0.28 mmol) was dissolved in 4 N hydrochloric acid solution in dioxane (20 ml) and stirred at room temperature for 3 hours. The solvent and the excessive reagent were evaporated under reduced pressure, and the residue was subjected to azeotropy with toluene and ether. The residue was purified by preparative TLC (chloroform:methanol = 10:1) to obtain 114.2 mg of a compound. This compound (112.0 mg) was powdered by addition of ether and centrifuged. The supernatant was removed, and the residue was dried under reduced pressure to obtain the title compound (97.2 mg, 58.4%) as yellow orange powder.
¹H-NMR (400MHz, CD₃OD) δ : 1.35 (9H, s), 1.63-1.76 (1H, m), 1.80-1.92 (1H, m), 1.92-2.10 (2H, m), 3.15-3.24 (2H, m), 3.30-3.38 (1H, m), 3.46-3.60 (3H, m), 3.72-3.90 (3H, m), 6.74 (1H, s), 7.02 (1H, d, J=15.6Hz), 7.57 (1H, dd, J=7.3, 1.7Hz), 7.63 (1H, d, J=15.6Hz), 8.01 (1H, d, J=1.7Hz), 8.93 (1H, d, J=7.3Hz).
IR (ATR) cm⁻¹: 3444, 3369, 2954, 2871, 1710, 1675, 1585, 1515, 1432.
m.p.: 210-220°C (decomp.)
FAB/MS m/z: 585 (MH⁺).
HR-FAB/MS: 585.2118 (Calcd. for C₂₇H₃₂N₆O₇S: 585.2131).
Anal. Calcd. for C₂₇H₃₂N₆O₇S•H₂O: C, 53.81; H, 5.69; N, 13.94. Found: C, 54.13; H, 5.57; N, 13.84.

### Example 190: (E)-3-{8-({[4-(tert-Butyl)-1,3-thiazol-2-yl]amino}carbonyl)-2-[(3S)-3-(glycoloylamino)hexahydro-1-pyridinyl]-4-oxo-4H-pyrido[1,2-a]pyrimidin-3-yl}-2-propenoic acid

### (A) tert-Butyl (E)-3-[2-((3S)-3-{[2-(acetyloxy)acetyl]aminothexahydro-1-pyridinyl)-8-({[4-(tert-butyl)-1,3-thiazol-2-yl]amino}carbonyl)-4-oxo-4H-pyrido[1,2-a]pyrimidin-3-yl]-2-propenoate

tert-Butyl (E)-3-{8-({[4-(tert-butyl)-1,3-thiazol-2-yl]amino}carbonyl)-2-hydroxy-4-oxo-4H-pyrido[1,2-a]pyrimidin-3-yl}-2-propenoate (305.6 mg, 0.65 mmol) was substantially dissolved in DMF (50 ml), added with tosyl chloride (185.7 mg, 0.97 mmol) and dimethylaminopyridine (158.7 mg, 1.29 mmol) and stirred at room temperature for 4 hours. The reaction solution was added with triethylamine (271.8 µl, 1.95 mmol) and a solution of 2-[(3S)-hexahydro-3-pyridinylamino]-2-oxoethylacetate (390 mg, 1.95mmol) in DMF (1 ml) and stirred at room temperature for 15 hours. The solvent was evaporated, and the residue was subjected to azeotropy with toluene. The residue was purified by preparative TLC (chloroform:methanol = 30:1, developed twice) and preparative TLC (hexane:ethyl acetate = 1:2) to obtain the title compound (62.7mg, 14.8%) as a mixture of yellow orange oily substance and foamy substance.
¹H-NMR (400MHz, CDCl₃) δ : 1.34 (9H, s), 1.52 (9H, s), 1.60-2.00 (4H, m), 2.16 (3H, s), 3.50-3.73 (3H, m), 3.85-3.97 (1H, m), 4.20-4.30 (1H, m), 4.51 (1H, ABq, J=14.5Hz), 4.72 (1H, ABq, J=14.5Hz), 6.56 (1H, s), 7.10 (1H, d, J=15.6Hz), 7.44 (1H, d, J=15.6Hz), 7.58 (1H, d, J=7.3Hz), 8.07 (1H, s), 9.01 (1H, d, J=7.3Hz).

### (B) tert-Butyl (E)-3-{8-({[4-(tert-butyl)-1,3-thiazol-2-yl]amino}carbonyl)-2-[(3S)-3-(glycoloylamino)hexahydro-1-pyridinyl]-4-oxo-4H-pyrido[1,2-a]pyrimidin-3-yl}-2-propenoate

tert-Butyl (E)-3-(2-((3S)-3-{[2-(acetyloxy)acetyl]amino}hexahydro-1-pyridinyl)-8-({[4-(tert-butyl)-1,3-thiazol-2-yl]amino]carbonyl)-4-oxo-4H-pyrido[1,2-a]pyrimidin-3-yl]-2-propenoate (62.7 mg, 0.096 mmol) was dissolved in THF (6 ml), added dropwise with 1 N aqueous sodium hydroxide (192 µ l, 0.19 mmol) and stirred for 17 hours. The solvent was evaporated, and then the residue was added with saturated aqueous sodium hydrogencarbonate, and extracted with chloroform. The organic layer was washed with saturated brine and dried over anhydrous magnesium sulfate. The solvent was evaporated, and the residue was purified by preparative TLC (chloroform:methanol = 20:1) to obtain the title compound (49.7 mg, 84.7%) as a mixture of orange yellow oily substance and an amorphous substance.
¹H-NMR (400MHz, CDCl₃) δ : 1.32 (9H, s), 1.51 (9H, s), 1.55-1.82 (5H, m), 1.97-2.07. (1H, m), 3.25-3.40 (2H, m), 3.77-3.95 (2H, m), 4.15 (1H, ABq, J=16.1Hz), 4.28 (1H, ABq, J=16.1Hz), 4.28-4.38 (1H, m), 6.60 (1H, s), 7.14 (1H, d, J=15.6Hz), 7.48 (1H, d, J=15.6Hz), 7.60 (1H, dd, J=7.3, 1.7Hz), 8.07 (1H, d, J. 7.6Hz), 8.13 (1H, d, J=1.7Hz), 9.02 (1H, d, J=7.3Hz).

### (C) (E)-3-{8-({[4-(tert-Butyl)-1,3-thiazol-2-yl]amino}carbonyl)-2-[(3S)-3-(glycoloylamino)hexahydro-1-pyridinyl]-4-oxo-4H-pyrido[1,2-a]pyrimidin-3-yl}-2-propenoic acid

tert-Butyl (E)-3-{8-({[4-(tert-butyl)-1,3-thiazol-2-yl]amino}carbonyl)-2-[(3S)-3-(glycoloylamino)hexahydro-1-pyridinyl]-4-oxo-4H-pyrido[1,2-a]pyrimidin-3-yl}-2-propenoate (49.7 mg, 0.081 mmol) was dissolved in 4 N hydrochloric acid solution in dioxane (5 ml) and stirred at room temperature for 19 hours. The solvent and excessive reagents were evaporated, and the residue was subjected to azeotropy with toluene and ether. The residue was purified by preparative TLC (chloroform:methanol = 10:1). The solvent was evaporated, and the residue was powdered by addition of ether and centrifuged. The supernatant was removed, and the residue was dried under reduced pressure to obtain the title compound (45.0 mg, 99.7%) as yellow orange powder.
¹H-NMR (400MHz, CD₃OD) δ: 1.35 (9H, s), 1.65-2.02 (4H, m), 3.45-3.56 (1H, m), 3.61-3.82 (3H, m), 3.90-3.97 (1H, m), 3.97 (2H, d, J=3.2Hz), 4.02-4.10 (1H, m), 6.75 (1H, s), 7.03 (1H, d, J=15.6Hz), 7.58 (1H, d, J=15.6Hz), 7.59 (1H, dd, J=7.3, 1.4Hz), 8.06 (1H, d, J=1.4Hz), 8.96 (1H, d, J=7.3Hz).
IR (ATR) cm⁻¹: 2952, 2859, 1664, 1511, 1438.
m.p.: 187-195°C (decomp.)
FAB/MS m/z: 555 (MH⁺).
HR-FAB/MS: 555.2032 (Calcd. for C₂₆H₃₀N₆O₆S: 555.2026).
Anal. Calcd. for C₂₆H₃₀N₆O₆S•3.5H₂O: C, 50.56; H, 6.04; N, 13.61. Found: C, 50.66; H, 5.22; N, 12.78.

### Example 191:2-{1-[2-(4-{8-({[4-(tert-Butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}piperazino)-2-oxoethyl]-1,1-dimethylammonio}acetate

### (A) [2-(tert-Butoxy)-2-oxoethyl](2-[4-(8-({[4-(tert-butyl)-1,3-thiazol-2-yl)amino}carbonyl)-3-{(E)-2-[1-(4-methoxybenzyl)-1H-1,2,3,4-tetrazol-5-yl]-1-ethenyl}-4-oxo-4H-pyrido[1,2-a]pyrimidin-2-yl)piperazino]-2-oxoethyl}dimethylammonium bromide

N⁸-[4-(tert-Butyl)-1,3-thiazol-2-yl]-2-{4-[2-(dimethylamino)acetyl]piperazino}-3-{(E)-2-[1-(4-methoxybenzyl)-1H-1,2,3,4-tetrazol-5-yl]-l-ethenyl}-4-oxo-4H-pyrido[1,2-a]pyrimidine-8-carboxamide (192.7 mg, 0.27 mmol) was dissolved in DMF (10 ml), added dropwise with tert-butyl bromoacetate (80.0 µ l, 0.54 mmol) and stirred at room temperature for 63 hours. The solvent was evaporated, and the residue was subjected to azeotropy with toluene and ether and purified by preparative TLC (chloroform:methanol:water = 8:3:0.5) to obtain the title compound (213.2 mg, 86.8%) as a mixture of yellow orange oily substance and an amorphous substance.
¹H-NMR (400MHz, CD₃OD) δ: 1.33 (9H, s), 1.52 (9H, s), 3.50 (6H, s), 3.50-3.78 (10H, m), 3.74 (3H, s), 4.65 (2H, s), 5.57 (2H, s), 6.66 (1H, s), 6.89 (2H, d, J=8.7Hz), 7.30 (2H, d, J=8.7Hz), 7.55 (1H, d, J=7.3Hz), 7.56 (1H, d, J=15.5Hz), 7.75 (1H, d, J=15.5Hz), 7.89 (1H, s), 8.86 (1H, d, J=7.3Hz).

### (B) 2-{1-[2-(4-{8-({[4-(tert-Butyl)-1,3-thiazol-2-yl]amino}carbonyl)-4-oxo-3-[(E)-2-(1H-1,2, 3,4-tetrazol-5-yl)-1-ethenyl]-4H-pyrido[1,2-a]pyrimidin-2-yl}piperazino)-2-oxoethyl]-1,1-dimethylammonio}acetate

[2-(tert-Butoxy)-2-oxoethyl]{2-[4-(8-({[4-(tert-butyl)-1,3-thiazol-2-yl]amino}carbonyl)-3-{(E)-2-[1-(4-methoxybenzyl)-1H-1,2,3,4-tetrazol-5-yl]-1-ethenyl}-4-oxo-4H-pyirido[1,2-a]pyrimidin-2-yl)piperazino]-2-oxoethyl}dimethylammonium bromide (213.2 mg, 0.24 mmol) was dissolved in 4 N hydrochloric acid solution in dioxane (21 ml) and stirred at room temperature for 17 hours. The solvent and excessive reagents were evaporated, and the residue was subjected to azeotropy with toluene and ether. The residue was purified by preparative TLC (chloroform:methanol:water = 8:3:0.5) to obtain 170.4 mg of carboxylic acid compound as a mixture of yellow orange oily substance and an amorphous substance.

This compound was dissolved in TFA (50 ml), heated at 60°C with stirring for 3 hours and cooled. Then TFA was evaporated, and the residue was subjected to azeotropy with toluene and ether. The residue was purified by preparative TLC (chloroform:methanol:water = 8:3:0.5). The purified product was suspended in methanol and water, neutralized by addition of 1 N aqueous sodium hydroxide and loaded on HP-20 (washed with water and then eluted with methanol:water = 7:3) for desalting. The product was further purified by HPLC and preparative TLC (chloroform:methanol:water = 8:3:0.5), powdered by addition of ether and centrifuged. The supernatant was removed, and the residue was dried under reduced pressure to obtain the title compound (47.1mg, 30.8% for the two steps) as orange powder.
HPLC conditions
Column: CAPCELL PAK C18 SG 120A, 5 µ m, 30 mm ϕ x 250 mm (SHISEIDO)
Mobile phase: MeOH:H₂O = 50:50
Detection wavelength: UV 254 nm
FR: 12ml/min
Retention time: 14.0 min
¹H-NMR (400MHz, CD₃OD) δ : 1.35 (9H, s), 3.44 (6H, s), 3.38-3.47 (2H, m), 3.57-3.70 (4H, m), 3.65 (2H, s), 3.72-3.80 (2H, m), 4.20 (2H, s), 6.70 (1H, s), 7.44 (1H, d, J=16.1Hz), 7.59 (1H, dd, J=7.4, 1.7Hz), 7.91 (1H, d, J=16.1Hz), 7.95 (1H, s), 8.93 (1H, d, J=7.4Hz).
IR (ATR) cm⁻¹: 2964, 2867, 1916, 1652, 1621, 1548, 1509, 1465, 1448, 1434.
m.p.: 230-245°C (decomp.)
FAB/MS m/z: 650 (MH⁺).
HR-FAB/MS: 650.2629 (Calcd. for C₂₉H₃₅N₁₁O₅S: 650.2622).
Anal. Calcd. for C₂₉H₃₅N₁₁O₅S•3.6H₂O: C, 48.74; H, 5.95; N, 21.56. Found: C, 49.31; H, 5.49; N, 21.01.

### Example 192: 2-{1-[2-(4-{8-({[4-(tert-Butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}-1,4-diazepan-1-yl)-2-oxoethyl]-1,1-dimethylammonio}acetate

[2-(tert-Butoxy)-2-oxoethyl](2-[4-(8-({[4-(tert-butyl)-1,3-thiazol-2-yl]amino}carbonyl)-3-{(E)-2-[1-(4-methoxybenzyl)-1H-1,2,3,4-tetrazol-5-yl]-1-ethenyl}-4-oxo-4H-pyrido[1,2-a]pyrimidin-2-yl)-1,4-diazepan-1-yl]-2-oxoethyl]dimethylammonium bromide (528.9 mg, 0.57 mmol) was dissolved in 4 N hydrochloric acid solution in dioxane (53 ml) and stirred at room temperature for 33 hours. The solvent and excessive reagents were evaporated, and the residue was subjected to azeotropy with toluene and ether. The residue was purified by preparative TLC (chloroform:methanol:water = 8:3:0.1) to obtain 481.6 mg of carboxylic acid compound as a mixture of orange oily substance and foamy substance.

This compound was dissolved in TFA (100 ml), heated to 60°C for 2 hours with stirring and cooled. Then TFA was evaporated, and the residue was subjected to azeotropy with toluene and ether and purified by preparative TLC (chloroform:methanol:water = 8:3:0.5). The product was substantially dissolved in water, neutralized by adding 1 N aqueous sodium hydroxide, loaded on HP-20 (washed with water and then eluted with methanol:water = 7:3) for desalting. The residue was purified by HPLC, powdered by addition of ether and centrifuged. The supernatant was removed, and the residue was dried under reduced pressure to obtain the title compound (96.0 mg, 30.8% for the two steps) as orange powder.
HPLC conditions
Column: Develosil Combi-PR-5, 5 µ m, 20 x 100 mm (NOMURA CHEMICAL CO., LTD.)
Mobile phase: H₂O (0.1% HCO₂H):CH₃CN (0.1% HCO₂H) = 69:31 → 40:60 (4 min) Detection wavelength: UV 254 nm
FR: 25 ml/min
Retention time: 3.3 min
¹H-NMR (400MHz, CD₃OD) δ : 1.35, 1.35 (total 9H, s), 1.92-2.02 (0.8H, m), 2.12-2.22 (1.2H, m), 3.82,3.84 (total 6H, s), 3.60-3.72 (2H, m), 3.75-3.89 (4H, m), 3.97-4.10 (2H, m), 4.15, 4.22 (total 2H, s), 4.90, 4.93-(total 2H, s), 6.73, 6.74 (total 1H, s),-7.49 (1H, d, J=16.1Hz), 7.55, 7.59 (total 1H, dd, J=7.5, 1.7Hz), 7.81, 7.83 (total 1H, d, J=16.1Hz), 8.01, 8.07 (total 1H, d, J=1.7Hz), 8.11 (1H, s), 8.95, 8.97 (total 1H, d, J=7.5Hz).
IR (ATR) cm⁻¹: 2958, 2867, 1652, 1515, 1425.
m.p.: 219-225°C (decomp.)
FAB/MS m/z: 664 (MH⁺).
HR-FAB/MS: 664.2764 (Calcd. for C₃₀H₃₇N₁₁O₅S: 664.2778).
Anal. Calcd. for C₃₀H₃₇N₁₁O₅S•HCO₂H•0.3H₂O: C, 52.06; H, 5.58; N, 21.54. Found: C, 52.46; H, 5.93; N, 21.18.

### Example 193: 2-{1-[2-(4-{8-({[4-(tert-Butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}-cis-2,6-dimethylhexahydro-1-pyrazinyl)-2-oxoethyl]-1,1-dimethylammonio}acetate

### (A) N⁸-[4-(tert-Butyl)-1,3-thiazol-2-yl]-2-{4-[2-(dimethylamino)acetyl]-cis-3,5-dimethylhexahydro-1-pyrazinyl}-3-{(E)-2-[1-(4-methoxybenzyl)-1H-1,2,3,4-tetrazol-5-yl]-1-ethenyl}-4-oxo-4H-pyrido[1,2-a]pyrimidine-8-carboxyamide

N⁸-[4-(tert-Butyl)-1,3-thiazol-2-yl]-2-hydroxy-3-{(E)-2-[1-(4-methoxybenzyl)-1H-1,2,3,4-tetrazol-5-yl]-1-ethenyl}-4-oxo-4H-pyrido[1,2-a]pyrimidine-8-carboxamide (1.00 g, 1.79 mmol) was suspended in acetonitrile (20 ml) and DMF (20 ml), added dropwise with diisopropyl ethylamine (1.25 ml, 7.16 mmol) and diphenyl chlorophosphate (742.0 µl, 3.58 mmol) at -10°C under argon atmosphere, stirred at the same temperature for 15 minutes, added dropwise with cis-2,6-dimethylpiperazine (409.0 mg, 3.58 mmol) and diisopropylethylamine (624.0 µl, 3.58 mmol), heated at 80°C for 1 hour and 30 minutes with stirring and cooled. Then the reaction mixture was diluted with chloroform, washed with saturated aqueous sodium hydrogencarbonate and saturated brine and dried over anhydrous sodium sulfate, and the solvent was evaporated. The residue was purified by silica gel column chromatography (chloroform → chloroform:methanol = 30:1 → 10:1) to obtain 1.27 g of the 2-substituted compound containing impurities as a mixture of orange oily substance and solid.

This compound (1.18 g) was suspended in THF (100 ml), added with N,N-dimethylglycine (0.93 g, 9.01 mmol), di-2-pyridylcarbonate (2.73 g, 12.6 mmol) and dimethylaminopyridine (1.10 g, 9.01 mmol) under argon atmosphere, and heated to 60°C for 7 hours with stirring. The solvent was evaporated, and the residue was added with saturated aqueous sodium hydrogencarbonate, and extracted with ethyl acetate and chloroform/methanol (10:1). The organic layer was washed with saturated brine and dried over anhydrous sodium sulfate. The solvent was evaporated, and the residue was purified by silica gel column chromatography (chloroform → chloroform:methanol = 30:1 → 20:1) and preparative TLC (chloroform:methanol = 10:1, developed twice) and (chloroform:ethyl acetate = 2:1, developed seven times) to obtain the title compound (76.7 mg, 6.2% for the three 3 steps) as yellow orange powder.
¹H-NMR (400MHz, CDCl₃) δ : 1.15-1.45 (6H, m), 1.37 (9H, s), 2.28 (6H, s), 2.90-3.40 (6H, m), 3.50-3.63 (2H, m), 3.79 (3H, s), 5.54 (2H, s), 6.58 (1H, s), 6:91 (2H, d, J=8.5Hz), 7.38 (2H, d, J=8.5Hz), 7.52 (1H, d, J=7.5Hz), 7.86 (1H, d, J=15.4Hz), 7.97 (1H, d, J=15.4Hz), 8.03 (1H, s), 8.94 (1H, d, J=7.5Hz).

### (B) [2-(tert-Butoxy)-2-oxoethyl]{2-[4-(8-({[4-(tert-butyl)-1,3-thiazol-2-yl]amino}carbonyl)-3-{(E)-2-[1-(4-methoxybenzyl)-1H-1,2,3,4-tetrazol-5-yl]-1-ethenyl}-4-oxo-4H-pyrido[1,2-a]pyrimidin-2-yl)]-cis-2,6-dimethylhexahydro-1-pyrazinyl}-2-oxoethyl}dimethylammonium bromide

N⁸-[4-(tert-Butyl)-1,3-thiazol-2-yl]-2-{4-[2-(dimethylamino)acetyl]-cis-3,5-dimethylhexahydro-1-pyrazinyl}-3-{(E)-2-[1-(4-methoxybenzyl)-1H-1,2,3,4-tetrazol-5-yl]-1-ethenyl}-4-oxo-4H-pyrido[1,2-a]pyrimidine-8-carboxamide (76.7 mg, 0.10 mmol) was dissolved in DMF (5 ml), added dropwise with tert-butyl bromoacetate (30.6 µ 1, 0.21 mmol) and stirred at room temperature for 20 hours. The solvent was evaporated, and the residue was subjected to azeotropy with toluene and ether, and the residue was purified by preparative TLC (chloroform:methanol:water = 8:3:0.1) to obtain the title compound (51.9 mg, 53.5%) as a mixture of orange yellow oily substance and an amorphous substance.
¹H-NMR (400MHz, CD₃OD) δ : 1.35 (9H, s), 1.37-1.45 (3H, m), 1.50-1.58 (3H, m), 1.51 (9H, s), 3.43 (2H, s), 3.47 (6H, s), 3.76 (3H, s), 3.96-4.15 (3H, m), 4.50-4.60 (1H, m), 4.60-5.00 (4H, m), 5.62 (2H, s), 6.72 (1H, s), 6.92 (2H, d, J=8.8Hz), 7.34 (2H, d, J=8.8Hz), 7.65 (1H, d, J=7.4Hz), 7.77 (1H, d, J=15.4Hz), 7.89 (1H, d, J=15.4Hz), 8.03 (1H, s), 8.99 (1H, d, J=7.6Hz).

### (C) 2-{1-[2-(4-{8-({[4-(tert-Butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}cis-2,6-dimethylhexahydro-1-pyrazinyl)-2-oxoethyl]-1,1-dimethylammonio}acetate

[2-(tert-Butoxy)-2-oxoethyl]{2-[4-(8-({[4-(tert-butyl)-1,3-thiazol-2-yl]amino}carbonyl)-3-{(E)-2-[1-(4-methoxybenzyl)-1H-1,2,3,4-tetrazol-5-yl]-1-ethenyl}-4-oxo-4H-pyrido[1,2-a]pyrimidin-2-yl)]-cis-2,6-dimethylhexahydro-1-pyrazinyl}-2-oxoethyl}dimethylammonium bromide (51.9 mg, 0.056 mmol) was dissolved in 4 N hydrochloric acid solution in dioxane (5 ml), stirred at room temperature for 18 hours, added with 4 N hydrochloric acid solution in dioxane (5 ml) and stirred at room temperature for 24 hours. The solvent and excessive reagents were evaporated, and the residue was subjected to azeotropy with toluene and ether. The residue was purified by preparative TLC (chloroform:methanol:water = 8:3:0.1) to obtain 29.4 mg of a carboxylic acid compound as yellow orange solid and recover 6.1 mg of the starting material. The recovered starting material was treated in the same manner as above to obtain 4.7 mg of the carboxylic acid compound as yellow orange solid.

The carboxylic acid compound (34.1 mg) was dissolved in TFA (10 ml), heated to 60°C with stirring for 2 hours and cooled. Then TFA was evaporated, and the residue was subjected to azeotropy with toluene and ether, and the residue was purified by preparative TLC (chloroform:methanol:water = 8:3:0.5) and HPLC. The product was powdered by addition of ether and centrifuged. The supernatant was removed, and the residue was dried under reduced pressure to obtain the title compound (9.7 mg, 25.8% for the two steps) as orange powder.
HPLC conditions
Column: Develosil Combi-PR-5, 5 µm, 20 x 100 mm (NOMURA CHEMICAL CO.,LTD) Mobile phase: H₂O (0.1% HCO₂H): CH₃CN (0.1% HCO₂H) = 67:33 → 37:63 (4 min) Detection wavelength: UV 254 nm
FR: 25 ml/min
Retention time: 3.35 min
¹H-NMR (400MHz, CD₃OD) δ : 1.35 (9H, s), 1.35-1.62 (6H, m), 3.43 (6H, s), 3.35-3.52 (4H, m), 4.04-4.18 (3H, m), 4.24-4.39 (1H, m), 4.85-4.95 (2H, m), 6.73 (1H, s), 7.64 (1H, dd, J=7.3, 2.0Hz), 7.68 (1H, d, J=16.0Hz), 7.89 (1H, d, J=16.0Hz), 8.07 (1H, d, J=2.0Hz), 8.10 (1H, s), 9.00 (1H, d, J=7.3Hz).
IR (ATR) cm⁻¹: 3666, 2964, 2863, 2387, 2325, 2167, 2098, 1930, 1635, 1546, 1511, 1502, 1432.
m.p.: 239-253°C (decomp.)
FAB/MS m/z: 678 (MH⁺).
Anal. Calcd. for C₃₁H₃₉N₁₁O₅S•HCO₂H•2H₂O: C, 50.58; H, 5.97; N, 20.28. Found: C, 50.36; H, 5.96; N, 19.96.

### Example 194: (E)-3-[2-[4-((2S)-2-Amino-5-{[amino(imino)methyl]amino}pentanoyl)-piperazino]-8-({[4-(t-butyl)-1,3-thiazol-2-yl]amino}carbonyl)-4-oxo-4H-pyrido[1,2-a]pyrimidin-3-yl]-2-propenoic acid

### (A) (E)-3-[8-({[4-(t-Butyl)-1,3-thiazol-2-yl]amino}carbonyl)-2-hydroxy-4-oxo-4H-pyrido[1,2-a]pyrimidin-3-yl]-2-propenoic acid t-butyl ester

A solution of N,N-dimethylformamide (561 µl, 7.25 mmol) dissolved in methylene chloride (60.0 ml) was added with oxalyl chloride (632 µl, 7.25 mmol) with ice cooling and stirred at room temperature for 15 minutes. This suspension was added with N⁸-[4-(t-butyl)-1,3-thiazol-2-yl]-2,4-dioxo-3,4-dihydro-2H-pyrido[1,2-a]pyrimidine-8-carboxyamide (1.00 g, 2.90 mmol) and stirred at room temperature for 1 hour. The reaction was terminated with saturated aqueous sodium hydrogencarbonate, and the solution was adjusted to pH 5 with 1 N hydrochloric acid. This solution was extracted with chloroform (five times), and the combined organic layer was concentrated under reduced pressure. The resulting residue was dissolved in a mixed solvent of tetrahydrofuran (500 ml) and N,N-dimethylformamide (20 ml), added with (t-butoxycarbonylmethylene) triphenylphosphorane (1.64 g, 4.34 mmol) and stirred overnight. The reaction mixture was concentrated under reduced pressure. The resulting residue was purified by column chromatography (silica gel, methylene chloride/methanol = 20/1 → 10/1), and the resulting orange solid was washed with methanol to obtain the title compound (E)-3-[8-({[4-(t-butyl)-1,3-thiazol-2-yl]amino}carbonyl)-2-hydroxy-4-oxo-4H-pyrido[1,2-a]pyrimidin-3-yl)-2-propenoic acid t-butyl ester (742 mg, 54%) as pale yellow solid.
m.p.: 254-257°C (decomp.)
¹H-NMR (DMSO-d₆) δ : 1.32 (9H, s), 1.46 (9H, s), 6.85 (1H, s), (6.87 (1H, d, J=15.7Hz), 7.82 (1H, d, J=15.9Hz), 7.90-7.93 (2H, m), 9.09 (1H, d, J=7.10Hz)
LRMS-FAB; m/z: 471 (MH⁺).
IR (cm⁻¹): 1678, 1603, 1513, 1308, 1349, 1250; 1146, 727, 440

### (B) (E)-3-{2-[4-(2-t-Butoxycarbonylamino-5-[(t-butoxycarbonyl)amino]-{[(t-butoxycarbonyl)imino]methyl}aminopentanoyl)piperazin-1-yl]-8-({[4-(t-butyl)-1,3-thiazol-2-yl]amino}carbonyl)-4-oxo-4H-pyrido[1,2-a]pyrimidin-3-yl}-2-propenoic acid t-butyl ester

A solution of (E)-3-[8-({[4-(t-Butyl)-1,3-thiazol-2-yl]amino}carbonyl)-2-hydroxy-4-oxo-4H-pyrido[1,2-a]gyrimidin3-yl]-2-propenoic acid t-butyl ester (117 mg, 0.957 mmol) in N,N-dimethylformamide (100 ml) was added with a solution of tosyl chloride (182 mg, 0.957 mmol) in N,N-dimethylformamide (5.0 ml) and stirred at room temperature for 3 hours. This reaction mixture was added with piperazine (275 mg, 3.19 mmol) and further stirred overnight at room temperature. The reaction mixture was concentrated under reduced pressure to obtain an orange residue.

This residue and Boc-Arg(Boc)₂OH (908 mg, 1.91 mmol) were dissolved in a mixed solvent of N,N-dimethylformamide (15 ml) and methylene chloride (25 ml), added with EDC•HCl (489 mg, 2.55 mmol) with ice cooling and stirred overnight at room temperature. The reaction mixture was concentrated under reduced pressure, and the resulting residue was purified by column chromatography (silica gel, methylene chloride:methanol = 30:1). The resulting crude product was further purified by column chromatography (silica gel, n-hexane:ethyl acetate = 2:1) to obtain the title compound (E)-3-{2-[4-(2-t-butoxycarbonylamino-5-[(t-butoxycarbonyl)-amino] {[(t-butoxycarbonyl)imino]methyl}aminopentanoyl)piperazin-1-yl]-8-({[4-(t-butyl)-1,3-thiazol-2-yl]amino}carbonyl)-4-oxo-4H-pyrido[1,2-a]pyrimidin-3-yl}-2-propenoic acid t-butyl ester (294 mg, 46%) as yellow amorphous solid.
¹H-NMR (CDCl₃) δ : 1.26-1.71 (49H, m), 3.50-3.94 (10H, m), 4.63 (1H, brs), 5.59 (1H, d, J=8.33Hz), 6.61 (1H, s), 7.11 (1H, d, J=15.4Hz), 7.44-7.51 (2H, m), 7.97 (1H, s), 9.03 (1H, d, J=7.35Hz)

### (C) (E)-3-[2-[4-((2S)-2-Amino-5-{[amino(imino)methyl]amino}pentanoyl)piperazino]-8-({[4-(t-butyl)-1,3-thiazol-2-yl]amino}carbonyl)-4-oxo-4H-pyrido[1,2-a]pyrimidin-3-yl]-2-propenoic acid

A solution of (E)-3-{2-[4-(2-t-butoxycarbonylamino5-[(t-butoxycarbonyl)-amino] {[(t-butoxycarbonyl)-imino]methyl}aminopentanoyl)piperazin-1-yl]-8-({[4-(t-butyl)-1,3-thiazol-2-yl]amino}carbonyl)-4-oxo-4H-pyrido[1,2-a]pyrimidin-3-yl}-2-propenoic acid t-butyl ester (294 mg, 0.296 mmol) in trifluoroacetic acid (30 ml) was stirred at room temperature for 2 hours, and the reaction mixture was concentrated under reduced pressure. The mixture was subjected to azeotropy with toluene three times, and the resulting residue was washed with diethyl ether. Further, a procedure in which the resulting residue was added with formic acid and concentrated under reduced pressure was repeated three times, and the residue was washed with diethyl ether to obtain the title compound (E) 3-[2-[4-((2S)-2-amino-5-{[amino(imino)methyl]amino]pentanoyl)piperazino]-8-({[4-(t-butyl)-1,3-thiazol-2-yl]amino}carbonyl)-4-oxo-4H-pyrido[1,2-a]pyrimidin-3-yl]-2-propenoic acid (191 mg, 86%) as orange powder.
m.p.: 240°C (decomp.)
¹H-NMR (CD₃OD) δ : 1.35 (9H, s), 1.61-1.86 (4H, m), 3.25-3.88 (10H, m), 4.34 (1H, brs), 6.70 (1H, s), 7.08 (1H, d, J=16.1Hz), 7.45 (1H, d, J=15.6Hz), 7.59-7.67 (1H, m), 7.91 (1H, s), 8.48 (2H, s), 8.91 (1H, d, J=7.32Hz)
LRMS-FAB; m/z: 639 (MH⁺)
IR (cm⁻¹): 1631, 1514, 1435, 1365, 1225, 702, 646

| Anal.(for C₂₉H₃₈N₁₀O₅S • 2.0 formic acid • 1.0 H₂O): | | | |
|---|---|---|---|
| Calcd. | C, 49.72; | H, 5.92; | N, 18.71. |
| Found | C, 50.19; | H, 6.35; | N, 18.24. |

### Example 195: (E)-3-[2-[4-(5-Aminopentanoyl]piperazino]-8-({[4-(t-butyl)-1,3-thiazol-2-yl]amino}carbonyl)-4-oxo-4H-pyrido[1,2-a]pyrimidin-3-yl]-2-propenoic acid

### (A) (E)-3-[2-(4-5-(t-Butoxycarbonyl)aminopentanoylpiperazino)-8-({[4-(t-butyl)-1,3-thiazol-2-yl]amino}carbonyl)-4-oxo-4H-pyrido[1,2-a]pyrimidin-3-yl]-2-gropenoic acid t-butyl ester

A solution of (E)-3-[8-({[4-(t-butyl)-1,3-thiazol-2-yl]amino}carbonyl)-2-hydroxy-4-oxo-4H-pyrido[1,2-a]pyrimidin3-yl]-2-propenoic acid t-butyl ester (50.0 mg, 0.106 mmol) in N,N-dimethylformamide (15 ml) was added with 4-dimethylaminopyridine (19.4 mg, 0.159 mmol) and slowly added dropwise with a solution of tosyl chloride (30.3 mg, 0.159 mmol) in N,N-dimethylformamide (3.0 ml) at room temperature. The reaction mixture was stirred at room temperature for 4 hours and 30 minutes, added with piperazine (45.7 mg, 0.530 mmol) and further stirred overnight at room temperature. The reaction mixture was concentrated under reduced pressure, and then the resulting residue was dissolved in a mixture solution of N,N-dimethylformamide (3.0 ml) and methylene chloride (10 ml) and added with 5-(t-butoxycarbonylamino)valeric acid (46.1 mg, 0.212 mmol). The mixture was added with EDC•HCl (61.0 mg, 0.318 mmol) with ice cooling and the reaction mixture was stirred overnight at room temperature. The reaction mixture was concentrated under reduced pressure, and the resulting residue was purified by thin layer chromatography (silica gel, methylene chloride:methanol = 30:1, developed twice) to obtain the title compound (E)-3-[2-(4-5-[(t-butoxycarbonyl)amino] pentanoylpiperazino)-8-({[4-(t-butyl)-1,3-thiazol-2-yl]amino}carbonyl)-4-oxo-4H-pyrido[1,2-a]-pyrimidin-3-yl]-2-propenoic acid t-butyl ester (31.3 mg, 41%) as yellow amorphous solid.
¹H-NMR (CD₃OD) δ : 1.26-1.71 (31H, m), 2.48 (2H, t, J=7.47Hz), 3.05―3.08 (2H, m), 3.64-3.76 (8H, m), 6.73 (1H, s), 7.00 (1H, dd, J=4.65, 15.7Hz), 7.51 (1H, dd, J=4.41, 15.7Hz), 7.61-7.66 (1H, m), 8.02 (1H, s), 8.96 (1H, t, J=6.86Hz)

### (B) (E)-3-[2-[4-(5-Aminopentanoyl)piperazino]-8-({[4-(t-butyl)-1,3-thiazol-2-yl]amino}carbonyl)-4-oxo-4H-pyrido[1,2-a]pyrimidin-3-yl]-2-propenoic acid

((E)-3-[2-(4-5-[(t-Butoxycarbonyl)amino] pentanoylpiperazino)-8-({[4-(t-butyl)-1,3-thiazol-2-yl]amino}carbonyl)-4-oxo-4H-pyrido[1,2-a]pyrimidin-3-yl]-2-pro penoic acid t-butyl ester (31.3 mg, 0.0432 mmol) was dissolved in trifluoroacetic acid (5.0 ml) and stirred at room temperature for 1 hour. The reaction mixture was concentrated, and the resulting residue was purified by high performance liquid chromatography (MeCN/H₂O system, containing 0.1% formic acid). The resulting amorphous solid was washed with methanol and diethyl ether to obtain the title compound (E)-3-[2-[4-(5-aminopentanoyl)piperazino]-8-({[4-(t-butyl)-1,3-thiazol-2-yl]amino}carbonyl)-4-oxo-4H-pyrido[1,2-a]pyrimidin-3-yl]-2-propenoic acid (14 mg, 48%) as yellow powder.
m.p.: 194-198°C
¹H-NMR (CD₃OD) δ: 1.35 (9H, s), 1.72 (4H, brs), 2.53 (2H, brs), 2.96 (2H, brs), 3.64-3.76 (8H, m), 6.73 (1H, s), 7.06 (1H, d, J=15.4Hz), 7.56 (1H, d, J=15.6Hz), 7.61 (1H, d, J=7.57Hz), 7.99 (1H, s), 8.43 (1H, s), 8.95 (1H, d, J=7.32Hz)
LRMS-FAB; m/z: 582 (MH⁺)
IR (cm⁻¹): 2964, 1666, 1633, 1599, 1514, 1435, 1365, 1319, 1201, 1132, 1014, 985, 741, 702

| Anal. (for C₂₈H₃₅N₇O₅S • 2.0 formic acid • 1.75H₂O): | | | |
|---|---|---|---|
| Calcd. | C, 51.09; | H, 6.07; | N, 13.90. |
| Found | C, 50.75; | H, 5.70; | N, 13.86. |

### Example 196: (E)-3-(8-({[4-(t-Butyl)-1,3-thiazol-2-yl]amino}carbonyl)-4-oxo-2-{4-[5-(2-pyrimidinylamino)pentanoyl]piperazino}-4H-pyrido[1,2-a]pyrimidin-3-yl)-2-propenoic acid

### (A) 2-(5-Hydroxypentyl)-1,3-isoindolinedione

A solution of 3-aminol-pentanol (25.0 g, 242 mmol) and 1,3-dihydro-1,3-isobenzofurandione (35.9 g, 242 mmol) in toluene (300 ml) was refluxed overnight by heating. The reaction mixture was concentrated under reduced pressure, and the residue was dissolved in methylene chloride, washed with 1 N hydrochloric acid, saturated aqueous sodium hydrogencarbonate and saturated brine and further dried over anhydrous sodium sulfate. After filtration, the solvent was evaporated under reduced pressure to obtain the title compound 2-(5-hydroxypentyl)-1,3-isoindolinedione (55.5 g, 98%) as pale yellow syrup.
¹H-NMR (CDCl₃) δ : 1.38-1.46 (2H, m), 1.59-1.76 (4H, m), 3.64-3.72 (4H, m), 7.69-7.72 (2H, m), 7.82-7.85 (2H, m)

### (B) 5-(1,3-Dioxo-2,3-dihydro-1H-2-isoindolyl)pentanoic acid

A mixed solution of 2-(5-hydroxypentyl)-1,3-isoindolinedione (10.0 g, 42.9 mmol) and sodium periodate (36.7 g, 171.6 mmol) in carbon tetrachloride (140 ml), acetonitrile (140 ml) and water (220 ml) was added with ruthenium chloride n-hydrate (178 mg, 0.858 mmol) with ice cooling, and the reaction mixture was stirred for 3 hours while maintained at 5-18°C. The organic layer was separated, and the aqueous layer was extracted twice with methylene chloride. The organic layers were combined and washed with saturated brine. The organic layer was dried over anhydrous sodium sulfate, and the solvent was evaporated under reduced pressure to obtain the title compound 5-(1,3-dioxo-2,3-dihydro-1H-2-isoindolyl)pentanoic acid (10.7 g, 100%) as gray solid.
¹H-NMR (CDCl₃) δ : 1.65-1.79 (4H, m), 2.41 (2H, t, J=7.20Hz), 3.72 (2H, t, 6.71Hz), 7.70-7.72 (2H, m), 7.83-7.85 (2H, m)

### (C) 5-(1,3-Dioxo-2,3-dihydro-1H-2-isoindolyl)pentanoic acid t-butyl ester

A solution of 5-(1,3-dioxo-2,3-dihydro-1H-2-isoindolyl)pentanoic acid (10.7 g, 43.3 mmol) and 4-dimethylaminopyridine (529 mg, 4.33 mmol) in t-butyl alcohol (100 ml) was added with a solution of di-t-butyl dicarbonate (14.2 g, 65.0 mmol) in t-butyl alcohol (20 ml) and stirred at 60°C for 1 hour. The reaction mixture was concentrated under reduced pressure, and the residue was dissolved in methylene chloride, washed with 1 N hydrochloric acid, saturated aqueous sodium hydrogencarbonate and saturated brine and further dried over anhydrous sodium sulfate. After filtration, the solvent was evaporated under reduced pressure to obtain the title compound 5-(1,3-dioxo-2,3-dihydro-1H-2-isoindolyl)pentanoic acid t-butyl ester (6.48 g, 49%) as colorless syrup.
¹H-NMR (CDCl₃) δ : 1.43 (9H, s), 1.59-1.76 (4H, m), 2.26 (2H, t, J=7.23Hz), 3.70 (2H, t, J=6.89Hz), 7.68-7.73 (2H, m), 7.81-7.86 (2H, m)

### (D) 5-Aminopentanoic acid t-butyl ester

A solution of 5-(1,3-dioxo-2,3-dihydro-1H-2-isoindolyl)pentanoic acid t-butyl ester (6.48 g, 21.4 mmol) in ethanol (500 ml) was added with hydrazine monohydrate and stirred at room temperature for 18 hours. The produced white precipitates were removed to obtain the title compound 5-aminopentanoic acid t-butyl ester (3.30 g, 82%) as pale yellow oily substance.
¹H-NMR (CDCl₃) δ : 1.41-1.50 (11H, m), 1.58-1.66 (2H, m), 2.23 (2H, t, 7.46Hz), 2.70 (2H, t, J=6.97Hz)

### (E) 5-(2-Pyrimidinylamino)pentanoic acid t-butyl ester

A solution of 5-aminopentanoic acid t-butyl ester (100 mg, 0.577 mmol) and ethyldiisopropylamine (224 mg, 1.73 mmol) in dimethyl sulfoxide (10 ml) was added with 2-bromopyrimidine (91.7 mg, 0.577 mmol) and stirred overnight at 120°C. The reaction mixture was added with water and extracted with methylene chloride (three times), and the organic layer was washed with saturated brine and dried over anhydrous sodium sulfate. The solvent was evaporated under reduced pressure, and the crude product was purified by thin layer chromatography (methylene chloride:methanol = 9:1) to obtain the-title compound 5-(2-pyrimidinylamino) pentanoic acid t-butyl ester (92.9 mg, 64%) as pale yellow oily substance.
¹H-NMR (CDCl₃) δ : 1.44 (9H, s), 1.59-1.73 (4H, m), 2.27 (2H, t, J=7.09Hz), 3.38-3.44 (2H, m), 5.22 (1H, brs), 6.50 (1H, t, J=4.77Hz), 8.26 (2H, d, J=4.65Hz)

### (F) 5-(2-Pyrimidinylamino)pentanoic acid trifluoroacetate

A solution of 5-(2-pyrimidinylamino)pentanoic acid t-butyl ester (92.9 mg, 0.370 mmol) in methylene chloride (5.0 ml) was added with trifluoroacetic acid (5.0 ml) and stirred at room temperature for 1 hour. The reaction mixture was concentrated under reduced pressure to obtain the title compound 5-(2-pyrimidinylamino)pentanoic acid trifluoroacetate (122 mg, >100%) as a brown amorphous solid.
¹H-NMR (CD₃OD) δ : 1.68 (4H, brs), 2.33-2.36 (2H, m), 3.46-3.49 (2H, m), 6.86 (1H, t, J=5.27Hz), 8.47 (2H, brs).

### (G) (E)-3-(8-({[4-(t-Butyl)-1,3-thiazol-2-yl]amino}carbonyl)-4-oxo-2-{4-[5-(2-pyrimidinylamino)pentanol]-4H-pyrido[1,2-a]pyrimidin-3-yl}-2-propenoic acid t-butyl ester

A solution of (E)-3-(8-({[4-(t-butyl)-1,3-thiazol-2-yl]amino]carbonyl)-2-hydroxy-4-oxo-4H-pyrido(1,2-a]pyrimidin-3-yl]-2-propenoic acid t-butyl ester (100 mg, 0.213 mmol) in dimethylformamide (30 ml) was successively added with 4-dimethylaminopyridine (39.1 mg, 0.320 mmol) and p-toluenesulfonyl chloride (61.0 mg, 0.320 mmol) and stirred at room temperature for 4 hours. The reaction mixture was further added with piperazine (92.2 mg, 1.07 mmol) and stirred overnight, and the solvent was evaporated under reduced pressure to obtain an orange residue.

The residue was dissolved in dimethylformamide (10 ml)/methylene chloride (30 ml), added with 5-(2-pyrimidinylamino)pentanoic acid trifluoroacetate (123 mg, 0.398 mmol), triethylamine (59.1 µl, 0.426 mmol) and further EDC•HCl (123 mg, 0.639 mmol), and stirred overnight at room temperature. The reaction mixture was concentrated, and the residue was purified by thin layer chromatography (methylene chloride:methanol = 9:1). The target fraction was extracted with methylene chloride/methanol (9:1), and further the resulting crude product was purified by thin layer chromatography (ethyl acetate) again to obtain the title compound (E)-3-(8-({[4-(t-butyl)-1,3-thiazol-2-yl]amino}carbonyl-4-oxo-2-{4-[5-(2-pyrimidinylamino)pentanol]-4H-pyrido[1,2-a]pyrimidin-3-yl}-2-propenoic acid t-butyl ester (46.3 mg, 30%) as yellow amorphous solid.
¹H-NMR (CDCl₃) δ : 1.34 (9H, s), 1.53 (9H, s), 1.56-1.81 (4H, m), 2.41-2.45 (2H, m), 3.42-3.76 (10H, m), 5.30 (1H, s), 6.51 (1H, t, J=4.77Hz), 6.60 (1H, s), 7.11 (1H, d, J=15.7Hz), 7.44-7.50 (2H, m), 7.93 (1H, d, J=1.22Hz), 8.26 (2H, d, J=4.65Hz), 9.00 (1H, d, J=7.58Hz)
ESI-MS; m/z: 717 (MH⁺)

### (H) (E)-3-(8-({[4-(t-Butyl)-1,3-thiazol-2-yl]amino}carbonyl)-4-oxo-2-{4-[5-(2-pyrimidinylamino)pentanoyl]piperazino}-4H-pyrido[1,2-a]pyrimidin-3-yl)-2-propenoic acid 1.0 trifluoroacetate

A solution of (E)-3-(8-({[4-(t-butyl)-1,3-thiazol-2-yl]amino}carbonyl)-4-oxo-2-{4-[5-(2-pyrimidinylamino)pentanol]-4H-pyrido[1,2-a]pyrimidin-3-yl}-2-propenoic acid t-butyl ester (46.3 mg, 0.0647 mmol) dissolved in methylene chloride (10 ml) was added with trifluoroacetic acid (10 ml) and stirred at room temperature for 1 hour and 30 minutes. The reaction mixture was concentrated under reduced pressure, and the resulting solid was washed with diethyl ether to obtain the title compound (E)-3-(8-({[4-(t-butyl)-1,3-thiazol-2-yl]amino}carbonyl)-4-oxo-2-{4-[5-(2-pyrimidinylamino)pentanoyl]piperazino}-4H-pyrido[1,2-a]pyrimidin-3-yl)-2-propenoic acid 1.0 trifluoroacetate (32.5 mg, 65%).
m.p. 234-238°C

| Anal. (for C₃₃H₃₇N₉O₅S G • 1.0TFA): | | | |
|---|---|---|---|
| Calcd. | C, 52.78; | H, 4.95; | N, 16.29. |
| Found | C, 52.65; | H, 5.19; | N, 16.10. |

¹H-NMR (DMSO-d₆) δ : 1.31 (9H, s), 1.56 (4H, brs), 2.39 (2H, brs), 3.28-3.62 (10H, m), 6.57 (1H, t, J=4.88Hz), 6.86 (1H, s), 6.96 (1H, d, J=15.6Hz), 7.38 (1H, brs), 7.47 (1H, d, J=15.6Hz), 7.66 (1H, d, J=7.32Hz), 8.22 (1H, s), 8.29 (2H, d, J=7.32Hz)
FAB-MS; m/z: 660 (MH⁺)
IR (cm⁻¹): 1672, 1518, 1442, 1286, 1200, 1140, 984, 721

### Example 197: (E)-3-({[4-(t-Butyl)-1,3-thiazol-2-yl]amino}carbonyl)-4-oxo-2-{4-[5-(1,4,5,6-tetrahydro-2-pyrimidinylamino)pentanoyl]piperazino}-4H-pyrido[1,2-a]pyrimidin-3-yl)-2-propenoic acid

### (A) 2-(Methylsulfanyl)-1,4,5,6-tetrahydropyrimidine hydroiodide

3,4,5,6-Tetrahydro-2-pyrimidinethiol (5.0 g, 43.0 mmol) was dissolved in acetone (60 ml), added with methyl iodide (2.68ml, 43.0 mmol) and refluxed by heating for 10 minutes. This reaction solution was added with ethanol and n-hexane and cooled with ice. The deposited crystals were collected by filtration to obtain the title compound (10.8g) as white powder.
¹H-NMR (CD₃OD) δ : 2.02-2.08 (2H, m), 2.63 (3H, d, J=3.42Hz), 3.48-3.52 (4H, m)

### (B) 5-(1,4,5,6-Tetrahydro-2-pyrimidinylamino)pentanoic acid benzyl ester

2-(Methylsulfanyl)-1,4,5,6-tetrahydropyrimidine hydroiodide (745 mg, 2.89 mmol), 5-aminopentanoic acid t-butyl ester (500 mg, 2.89 mmol) and triethylamine (400 µ 1) were dissolved in dimethylformamide (5.0 ml) and stirred at 100°C for 26 hours. The solution was cooled to room temperature and concentrated under reduced pressure, and then the residue was purified by column chromatography (silica gel, chloroform, chloroform:acetone = 9:1, chloroform:methanol = 9:1) to obtain the title compound (410 mg) as pale yellow oily substance.
¹H-NMR (CDCl₃) δ : 1.45 (9H, s), 1.65-1.68 (4H, m), 1.96-2.02 (2H, m), 2.30 (2H, t, J=6.59Hz), 3.17-3.22 (2H, m), 3.40-3.43 (4H, m), 7.13 (1H, brs), 7.63 (1H, brs)

### (C) 5-(1,4,5,6-Tetrahydro-2-pyrimidinylamino)pentanoic acid

5-(1,4,5,6-Tetrahydro-2-pyrimidinylamino)pentanoic acid benzyl ester (109 mg, 0.426 mmol) was dissolved in methylene chloride (5.0 ml), added with trifluoroacetic acid (5.0 ml) and stirred at room temperature for 1 hour 30 minutes. The reaction solution was concentrated under reduced pressure to obtain the title compound (85 mg) as yellow oily substance.
¹H-NMR (CD₃OD) δ : 1.56-1.69 (4H, m), 1.82-1.97 (2H, m), 2.34 (2H, t, J=6.97Hz), 3.12 (2H, t, J=6.72Hz), 3.30-3.36 (6H, m)

### (D) (E)-3-({[4-(t-Butyl)-1,3-thiazol-2-yl]amino}carbonyl)-4-oxo-2-{4-[5-(1,4,5,6-tetrahydro-2-pyrimidinylamino)pentanoyl]piperazino}-4H-pyrido[1,2-a]pyrimidin-3-yl)-2-propenoic acid t-butyl ester

(E)-3-[8-({[4-(t-Butyl)-1,3-thiazol-2-yl]amino}carbonyl)-2-hydroxy-4-oxo-4H-pyrido[1,2-a]pyrimidin3-yl]-2-propenoic acid t-butyl ester (100 mg, 0.213 mmol), 4-dimethylaminopyridine (39.1 mg, 0.320 mmol) and tosyl chloride (61.0 mg, 0.320 mmol) were dissolved in dimethylformamide (30 ml) and stirred at room temperature for 3 hours. This solution was added with piperazine (92.2 mg, 1.07 mmol), stirred overnight at room temperature and concentrated under reduced pressure.

The resulting residue and 5-(1,4,5,6-tetrahydro-2-pyrimidinylamino)valeric acid (85 mg, 0.426 mmol) were dissolved in a mixed solution of dimethylformamide (10 ml) and methylene chloride (30 ml), added with triethylamine (59.1 µ l, 0.426 mmol) and EDC•HCl (123 mg, 0.639 mmol) and stirred at room temperature for 2 days. The reaction solution was concentrated under reduced pressure, and the resulting residue was purified by thin layer chromatography (methylene chloride: methanol = 9:1, developed twice) to obtain the title compound (32.1 mg) as yellow amorphous solid.
¹H-NMR (CDCl₃) δ : 1.23-1.90 (24H, m), 2.47-2.48 (2H, m), 3.17-3.18 (2H, m), 3.34 (4H, brs), 3.56-3.73 (8H, m), 6.57 (1H, s), 7.08-7.13 (2H, m), 7.45 (1H, d, J=15.7Hz), 7.56 (1H, d, J=7.59Hz), 7.74 (1H, d, J=8.08Hz), 7.96 (1H, brs), 8.02 (1H, s), 9.00 (1H, d, J=7.35Hz)
LRMS-ESI; m/z: 721 (MH⁺)

### (E) (E)-3-({[4-(t-Butyl)-1,3-thiazol-2-yl]amino}carbonyl)-4-oxo-2-{4-[5-(1,4,5,6-tetrahydro-2-pyrimidinylamino)pentanoyl]piperazino}-4H-pyrido[1,2-a]pyrimidin-3-yl)-2-propenoic acid

(E)-3-({[4-(t-Butyl)-1,3-thiazol-2-yl]amino}carbonyl)-4-oxo-2-{4-[5-(1,4,5,6-tetrahydro-2-pyrimidinylamino)pentanoyl]piperazino}-4H-pyrido[1,2-a]pyrimidin-3-yl) -2-propenoic acid t-butyl ester (32.1 mg, 0.0446 mmol) was dissolved in methylene chloride (10 ml), added with trifluoroacetic acid (10 ml) and stirred at room temperature for 3 hours. The reaction solution was concentrated under reduced pressure, and then the resulting residue was washed with diethyl ether and dried under reduced pressure to obtain the title compound (26.2 mg) as orange amorphous solid.
¹H-NMR (DMSO-d₆) δ : 1.31 (9H, s), 1.51 (4H, m), 1.81 (2H, t, J=5.13Hz), 2.39 (2H, t, J=6.74Hz), 3.06-3.07 (2H, m), 3.24-3.62 (12H, m), 6.86 (1H, s), 6.96 (1H, d, J=15.4Hz), 7.23 (1H, brs), 7.45-7.49 (2H, m), 7.64-7.68 (2H, m), 8.19 (1H, s), 8.94 (1H, d, J=7.35Hz) LRMS-FAB; m/z: 664 (MH⁺)
IR (cm⁻¹): 2968, 1641, 1520, 1439, 1367, 1284, 1173, 1122, 1034, 1011, 683, 567

| HRMS-FAB(C₃₂H₄₂O₅N₉S); m/z: | |
|---|---|
| Calcd. (m/z) | 664.3030 |
| Found (m/z) | 664.3019 |

### Example 198: (E)-3-({[4-t-Butyl]-1,3-thiazol-2-yl}amino)carbonyl)-2-{4-[5-(dimethylamino)pentanoyl]piperazino}-4-oxo-4H-pyrido[1,2-a]pyrimidin-3-yl)-2-propenoic acid

### (A) 5-Hydroxypentanoic acid benzyl ester

A solution of δ-valerolactone (16.0 g, 160 mmol) in ethanol (160 ml) was slowly added with a solution of sodium hydroxide (6.72 g, 168 mmol) in water (42 ml) with ice cooling and stirred overnight at room temperature. The reaction mixture was concentrated under reduced pressure, and the residue was subjected to azeotropy with toluene (twice) to removed moisture. The resulting residue was dissolved in N,N-dimethylformamide (200 ml). This solution was added with benzyl bromide (19.0 ml, 160 ml) and stirred at room temperature for a whole day and night. The reaction mixture was concentrated under reduced pressure, and the residue was added with water and extracted twice with ethyl acetate. The combined organic layer was washed with water and saturated brine and further dried over anhydrous sodium sulfate. After sodium sulfate was removed by filtration, the filtrate was concentrated under reduced pressure, and the resulting residue was purified by column chromatography (silica gel, n-hexane:ethyl acetate = 1:1) to obtain the title compound 5-hydroxypentanoic acid benzyl ester (18.8 g, 56%) as colorless syrup.
¹H-NMR (CDCl₃) δ : 1.56-1.78 (4H, m), 2.41 (2H, t, J=7.35Hz), 3.63 (2H, t, J=6.25Hz), 5.12 (2H, s), 7.29-7.38 (5H, m)
LRMS-FAB; m/z: 209 (MH⁺)
IR (ATR) cm⁻¹: 3444, 2939, 1730, 1456, 1151, 1057, 980, 737, 696, 579, 496

### (B) 5-Bromopentanoic acid benzyl ester

A solution of 5-hydroxypentanoic acid benzyl ester (3.0 g, 14.4 mmol) and triphenylphosphine (4.53 g, 17.3 mmol) in methylene chloride (100 ml) was added with carbon tetrabromide (7.16 g, 21.6 mmol) and stirred at room temperature for 30 minutes. The reaction mixture was concentrated under reduced pressure, and the resulting residue was purified by column chromatography (silica gel, n-hexane:ethyl acetate =10:1) to obtain the title compound 5-bromopentanoic acid benzyl ester (2.80 g, 72%) as colorless oily substance.
¹H-NMR (CDCl₃) δ : 1.77-1.94 (4H, m), 2.40 (2H, t, J=7.20Hz), 3.40 (2H, t, J=6.47Hz), 5.12 (2H, s), 7.30-7.40 (5H, m)
LRMS-FAB; m/z: 271 (MH⁺)
IR (ATR) cm⁻¹: 2958, 1732, 1454, 1255, 1163, 737, 696, 561

| HRMS-FAB (C₁₂H₁₆O₂Br); m/z: | |
|---|---|
| Calcd. (m/z) | 271.0334 |
| Found (m/z) | 271.0325 |

### (C) 5-(Trimethylamino)pentanoic acid benzyl ester

A solution of 5-bromopentanoic acid benzyl ester (1.00 g, 3.69 mmol) in tetrahydrofuran (20 ml) was added with a solution of dimethylamine in tetrahydrofuran (2.0 M, 3.69 ml) and stirred at room temperature for 4 days. The reaction mixture was concentrated under reduced pressure, and the resulting residue was dissolved in methylene chloride, washed with saturated brine and further dried over anhydrous sodium sulfate. After sodium sulfate was removed by filtration, the filtrate was concentrated under reduced pressure, and the resulting residue was purified by column chromatography (silica gel, methylene chloride:methanol = 5:1) to obtain the title compound 5-(trimethylamino)pentanoic acid benzyl ester (672 mg, 77%).
¹H-NMR (CDCl₃) δ : 1.43-1.56 (2H, m), 1.64-1.71 (2H, m), 2.23 (6H, s), 2.30 (2H, t, J=7.45Hz), 2.39 (2H, t, J=7.32Hz), 5.12 (2H, s), 7.30-7.39 (5H, m)

### (D) 5-(Dimethylamino)pentanoic acid

5-(Trimethylamino)pentanoic acid benzyl ester (672 mg, 2.86 mmol) in methanol (30 ml) was added with a 10% palladium carbon catalyst (M) (containing water, 200 mg) and stirred overnight under hydrogen atmosphere at room temperature. The catalyst was removed by filtration, and then the filtrate was concentrated under reduced pressure to obtain the title compound 5-(dimethylamino)pentanoic acid (432 mg, 100%) as colorless syrup.
¹H-NMR (DMSO-d₆) δ : 1.46-1.51 (4H, m), 2.15-2.21 (2H, m), 2.32 (6H, s), 2.47-2.52 (2H, m)

### (E) (E)-3-({[4-t-Butyl]-1,3-thiazol-2-yl}amino)carbonyl)-2-{4-[5-(dimethylamino)-pentanoyl]piperazino]-4-oxo-4H-pyrido[1,2-a]pyrimidin-3-yl)-2-propenoic acid t-butyl ester

A solution of (E)-3-[8-(4-t-butylthiazol-2-ylcarbamoyl)-2-hydroxy-4-oxo-4H-pyrido[1,2-a]pyrimidin-3-yl]propenoic acid t-butyl ester (90.6 mg, 0.193 mmol) in N,N-dimethylformamide (30 ml) was added with 4-dimethylaminopyridine (35.4 mg, 0.290 mmol) and tosyl chloride (55.3 mg, 0.290 mmol) and stirred overnight at room temperature. The reaction mixture was concentrated under reduced pressure, and the resulting residue and 5-(dimethylamino)pentanoic acid (70.1 mg, 0.483 mmol) were dissolved in methylene chloride (30 ml) and N,N-dimethylformamide (10 ml). This mixture was added with EDC•HCl (111 mg, 0.579 mmol), stirred overnight at room temperature and concentrated under reduced pressure. The resulting residue was purified by thin layer chromatography (methylene chloride:methanol = 9:1, developed twice), and the target fraction was extracted with methylene chloride/methanol (9:1) to obtain the title compound (E)-3-{8-(4-t-butylthiazol-2-ylcarbamoyl)-2-[4-(5-dimethylaminopentanoyl)piperazine-1-yl]-4-oxo-4H-pyrido[1,2-a]pyrimidin-3-yl}propenoic acid t-butyl ester (70.8 mg, 55%) as orange solid.
¹H-NMR (CDCl₃) δ : 1.34 (9H, s), 1.53 (9H; s), 1.72-1.74 (4H, m), 2.44 (2H, t, J=6.59Hz), 2.64 (6H, s), 2.79-2.82 (2H, m), 3.51-3.73 (8H, m), 6.59 (1H, s), 7.10 (1H, d, J=15.9Hz), 7.46 (1H, d, J=15.6Hz), 7.52 (1H, d, J=7.32Hz), 7.75 (1H, d, J=8.06Hz), 7.96 (1H, s), 8.99 (1H, d, J=7.32Hz)
LRMS-ESI; m/z: 666 (MH⁺)

### (F) (E)-3-({[4-t-Butyl]-1,3-thiazol-2-yl}amino)carbonyl)-2-{4-[5-(dimethylamino)-pentanoyl]piperazino}-4-oxo-4H-pyrido[1,2-a]pyrimidin-3-yl)-2-propenoic acid

A solution of (E)-3-({[4-t-butyl]-1,3-thiazol-2-yl}amino)carbonyl)-2-{4-[5-(dimethylamino)pentanoyl]piperazino}-4-oxo-4H-pyrido[1,2-a]pyrimidin-3-yl)-2-propenoic acid t-butyl ester (70.8 mg, 0.106 mmol) in methylene chloride (10 ml) was added with trifluoroacetic acid (10 ml) and stirred for 2 hours at room temperature. The reaction mixture was concentrated under reduced pressure, and the resulting residue was purified by high performance liquid chromatography (MeCN/H₂O system, containing 0.1% formic acid). The resulting residue was lyophilized to obtain the title compound (33.3 mg) as yellow amorphous solid.
¹H-NMR (CD₃OD) δ : 1.35 (9H, s), 1.69-1.78 (4H, m), 2.54 (2H, t, J=6.59Hz), 2.87 (6H, s), 3.11-3.15 (2H, m), 3.63-3.78 (8H, m), 6.74 (1H, s), 7.08 (1H, d, J=15.4Hz), 7.57 (1H, d, J=15.6Hz), 7.63 (1H, d, J=7.57Hz), 8.01 (1H, s), 8.97 (1H, d, J=7.33Hz)
LRMS-FAB; m/z: 610 (MH⁺)
IR (ATR) cm⁻¹: 1637, 1516, 1435, 1365, 1290, 1225, 1011, 874, 683, 567

| HRMS-FAB (C₃₀H₄₀O₅N₇S); m/z: | |
|---|---|
| Calcd. (m/z) | 610.2812 |
| Found (m/z) | 610.2789 |

### Example 199: [5-(4-{8-({[4-(t-Butyl)-1,3-thiazol-2-yl)amino}carbonyl)-3-[(E)-2-carboxy-1-ethenyl]-4-oxo-4H-pyrido[1,2-a]pyrimidin-2-yl}piperazino)-5-oxopentyl]-(trimethyl)ammonium iodide

(E)-3-({[4-t-Butyl]-1,3-thiazol-2-yl}amino)carbonyl)-2-{4-[5-(dimethylamino)-pentanoyl]piperazino}-4-oxo-4H-pyrido[1,2-a]pyrimidin-3-yl)-2-propenoic acid (15 mg, 0.0246 mmol) was dissolved in dimethylformamide (10 ml), added with methyl iodide (15 µl, 0.246 mmol) and left overnight in a refrigerator (3°C). The reaction solution was concentrated under reduced pressure, and the resulting residue was washed with diethyl ether and dried under reduced pressure to obtain the title compound (15.5 mg) as orange powder.
m.p.: 176-182°C
¹H-NMR (CD₃OD) δ : 1.35 (9H, s), 1.69 (2H, brs), 1.86 (2H, brs), 2.57 (2H, t, J=6.96Hz), 2.88-2.90 (2H, m), 3.14 (9H, s), 3.49-3.77 (8H, m), 6.76 (1H, s), 7.09 (1H, d, J=15.4Hz), 7.23 (1H, d, J=7.57Hz), 7.62-7.71 (2H, m), 8.05 (1H, s), 9.01 (1H, d, J=7.32Hz)

| HRMS-FAB (C₃₁H₄₂O₅N₇S); m/z: | |
|---|---|
| Calcd. (m/z) | 624.2968 |
| Found (m/z) | 624.2982 |

IR (ATR) cm⁻¹: 2956, 1670, 1637, 1597, 1519, 1439, 1225, 1011, 744, 683, 631

### Example 200: (E)-3-(8-({[4-(t-Butyl)-1,3-thiazol-2-yl]amino}carbonyl)-2-{(3R)-3-[(hydroxysulfonyl)oxy]hexahydro-1-pyridinyl}-4-oxo-4H-pyrido[1,2-a]pyrimidin-3-yl)-2-propenoic acid

### (A) (E)-3-(8-({[4-(t-Butyl)-1,3-thiazol-2-yl]amino}carbonyl)-2-{(3R)-3-[(hydroxysulfonyl)oxy]hexahydro-1-pyridinyl}-4-oxo-4H-pyrido[1,2-a]pyrimidin-3-yl)-2-propenaic acid t-butyl ester

(E)-3-{8-({[4-(t-Butyl)-1,3-thiazol-2-yl]amino}carbonyl)-2-[(3R)-3-hydroxyhexahydro-1-pyridinyl]-4-oxo-4H-pyrido[1,2-a]pyrimidin-3-yl}-2-propenoic acid t-butyl ester (166 mg, 0.299 mmol) and dicyclohexylcarbodiimide (309 mg, 1.50 mmol) were dissolved in dimethylformamide (11 ml) and added with a solution of concentrated sulfuric acid (44.0 mg, 0.449 mmol) in dimethylformamide (2.0 ml) with ice cooling. The reaction solution was stirred for 20 minutes and added with triethylamine (2.0 ml). The produced precipitates were removed, and the reaction solution was concentrated under reduced pressure. The resulting residue was purified by thin layer chromatography (methylene chloride:methanol = 10:1) and then purified by thin layer chromatography (ethyl acetate:methanol = 9:1, developed twice) again to obtain the title compound (72.5 mg) as orange amorphous solid.
¹H-NMR (CD₃OD) δ : 1.34 (9H, s), 1.53 (9H, s), 1.69-1.75 (1H, m), 1.84-1.89 (1H, m), 1.98 (1H, brs), 2.15 (1H, brs), 3.46-3.66 (3H, m), 4.27 (1H, dd, J=3.29, 13.0Hz), 4.51-4.55 (1H, m), 6.73 (1H, s), 6.95 (1H, d, J=15.6Hz), 7.49 (1H, d, J=15.6Hz), 7.54 (1H, d, J=7.31Hz), 8.05 (1H, s), 8.92 (1H, d, J=7.43Hz)
LRMS-ESI; m/z: 634 (MH⁺)

### (B) (E)-3-(8-({[4-(t-Butyl)-1,3-thiazol-2-yl]amino}carbonyl)-2-{(3R)-3-[(hydroxysulfonyl)-oxy]hexahydro-1-pyridinyl}-4-oxo-4H-pyrido[1,2-a]pyrimidin-3-yl)-2-propenoic acid

(E)-3-(8-({[4-(t-Butyl)-1,3-thiazol-2-yl]amino}carbonyl)-2-{(3R)-3-[(hydroxysulfonyl)oxy]hexahydro-1-pyridinyl}-4-oxo-4H-pyrido[1,2-a]pyrimidin-3-yl)-2-propenoic acid t-butyl ester (72.5 mg, 0.114 mmol) was dissolved in methylene chloride (10 ml), added with trifluoroacetic acid (10 ml) and stirred at room temperature for 4 hours. The reaction solution was concentrated under reduced pressure, and then the resulting residue was washed with diethyl ether and methanol and dried under reduced pressure to obtain the title compound (60 mg) as orange powder.
m.p.: 196°C (decomp.)
¹H-NMR (DMSO-d₆) δ : 1.31 (9H, s), 1.65 (2H, m), 1.87 (1H, brs), 2.01 (1H, brs), 3.17-3.48 (3H, m), 4.09-4.17 (2H, m), 6.84 (1H, s), 6.92 (1H, d, J=15.4Hz), 7.42 (1H, d, J=15.4Hz), 7.58 (1H, dd, J=1.71, 7.39Hz), 8.20 (1H, s), 8.89 (1H, d, J=7.35Hz)
LRMS-FAB; m/z: 578 (MH⁺)
IR (ATR) cm⁻¹: 1684, 1591, 1522, 1437, 1236, 1184, 1005, 960, 862, 798, 746, 582

| Anal. (as C₂₄H₂₇N₅O₈S₂•0.25TFA•1.75H₂O) | | | |
|---|---|---|---|
| Calcd. | C, 46.15; | H, 4.86; | N, 10.98 |
| Found | C, 46.45; | H, 4.96; | N, 10.74 |

### Example 201: (E)-3-[8-({[4-(t-Butyl)-1,3-thiazol-2-yl]amino}carbonyl)-2-(4-{5-[(hydroxysulfonyl)oxy]pentanoyl}piperazino)-4-oxo-4H-pyrido[1,2-a]pyrimidin-3-yl]-2-propenoic acid

### (A) 5-[1-(t-Butyl)-1,1-diphenylsilyl]oxypentanoic acid benzyl ester

A solution of 5-hydroxypentanoic acid benzyl ester (2.0 g, 9.60 mmol) and imidazole (1.96 g, 28.8 mmol) in dimethylformamide (10 ml) was added with a solution oft-butyldiphenylsilyl chloride (3.17 g, 11.5 mmol) in dimethylformamide (10 ml) and stirred overnight at room temperature. The reaction mixture was concentrated under reduced pressure, added with ethyl acetate and water and separated, and the organic layer was washed with water (three times) and saturated brine and dried over anhydrous sodium sulfate. The solvent was evaporated under reduced pressure, and then the resulting residue was purified by column chromatography (silica gel, n-hexane:ethyl acetate = 15:1) to obtain the title compound (3.25 g) as colorless oily substance.
¹H-NMR (CDCl₃) δ : 1.04 (s, 9H), 1.56-1.62 (m, 2H), 1.71-1.79 (m, 2H), 2.36 (t, J=7.47Hz, 2H), 3.65 (t, J=6.25Hz, 2H), 5.10 (s, 2H), 7.34-7.43 (m, 6H), 7.63-7.66 (m, 4H)

### (B) 5-[1-t-Butyl]-1,1-diphenylsilyl]oxypentanoic acid

A solution of 5-[1-(t-butyl)-1,1-diphenylsilyl]oxypentanoic acid benzyl ester (3.25 g, 7.28 mmol) in tetrahydrofuran (100 ml) was added with a solution of lithium hydroxide 1.0 hydrate (914 mg, 21.8 mmol) in water (100 ml) and stirred at room temperature for 6 hours. The reaction solution was made acidic by addition of 1 N hydrochloric acid and extracted with methylene chloride three times, and then the combined organic layer was washed with saturated brine and dried over anhydrous sodium sulfate. The solvent was evaporated under reduced pressure to obtain the title compound (2.50 g) as colorless oily substance.
¹H-NMR (CDCl₃) δ : 1.05 (9H, s), 1.57-1.76 (4H, m), 2.36 (2H, t, J=7.35Hz), 3.66-3.68 (2H, m), 7.35-7.42 (6H, m), 7.64-7.67 (4H, m)
IR (ATR) cm⁻¹: 2931,2858, 1707, 1427, 1105, 822, 739, 698, 613, 503
LRMS-FAB; m/z: 357 (MH⁺)

| HRMS-FAB (C₂₁H₂₉O₃Si); m/z: | |
|---|---|
| Calcd. (m/z) | 357.1886 |
| Found (m/z) | 357.1928 |

### (C) (E)-3-[8-({[4-(t-Butyl)-1,3-thiazol-2-yl]amino}carbonyl)-2-hydroxy-4H-pyrido[1,2-a]pyrimidin-3-yl]-2-propenoic acid t-butyl ester

A mixed solution of dimethylformamide (1.12 ml) and methylene chloride (120 ml) was added with oxalyl chloride (1.26 ml, 14.5 mmol) with ice cooling and stirred for 15 minutes. This solution was added with N⁸-[4-(t-butyl)-1,3-thiazol-2-yl]-2,4-dioxo-3,4-dihydro-2H-pyrido[1,2-a]pyrimidine-8-carboxamide (2.00 g, 5.81 mmol), stirred at room temperature for 1 hour and 30 minutes and added with saturated aqueous sodium hydrogencarbonate. This solution was concentrated under reduced pressure, and then this residue was added with 1 N aqueous hydrochloric acid. The mixture was adjusted to about pH 4.5 and extracted with chloroform five times, and the organic layer was concentrated under reduced pressure.

The resulting residue was dissolved in a mixed solution of tetrahydrofuran (1000 ml) and dimethylformamide (50 ml), added with (t-butoxycarbonylmethylene)triphenylphosphorane (3.28 g, 8.72 mmol) and stirred overnight at room temperature. The reaction mixture was concentrated under reduced pressure, and the resulting residue was washed with methanol and dried over under reduced pressure to obtain the title compound (1.31 g) as yellow solid.
¹H-NMR (DMSO-d₆) δ : 1.32 (9H, s), 1.47 (9H, s), 6.85-6.90 (2H, m), 7.82-7.92 (3H, m), 9.07 (1H, d, J=7.08Hz)
LRMS-FAB; m/z: 470 (MH⁺)
IR (ATR) cm⁻¹: 3435, 2972, 1678, 1585, 1514, 1308, 1250, 1146, 987, 854, 727, 538, 438

| Anal. (for C₂₃H₂₆N₄O₅S • 1.75H₂O) | | | |
|---|---|---|---|
| Calcd. | C, 55.02; | H, 5.92; | N, 11.16 |
| Found | C, 55.51; | H, 5.91; | N, 10.74 |

### (D) (E)-3-{8-({[4-(t-Butyl)-1,3-thiazol-2-yl]amino}carbonyl)-2-[4-(5-hydroxypentanoyl)-piperazino]-4-oxo-4H-pyrido[1,2-a]pyrimidin-3-yl)-2-propenoic acid t-butyl ester

A solution of (E)-3-[8-({[4-(t-butyl)-1,3-thiazol-2-yl]amino}carbonyl)-2-hydroxy-4H-pyrido[1,2-a]pyrimidin-3-yl]-2-propenoic acid t-butyl ester (200 mg, 0.425 mmol) in dimethylformamide (30 ml) was added with 4-dimethylaminopyridine (78 mg, 0.638 mmol) and tosyl chloride (122 mg, 0.638 mmol) and stirred at room temperature for 4 hours. This reaction solution was added with piperazine (183 mg, 2.13 mmol), stirred overnight at room temperature and concentrated under reduced pressure.

The resulting residue was dissolved in a mixed solution of methylene chloride (30 ml) and dimethylformamide (10 ml), added with 5-{[1-(t-butyl)-1,1-diphenylsilyl]-oxy}valeric acid (455 mg, 1.28 mmol) and EDC•HCl (326 mg, 1.70 mmol) and stirred overnight at room temperature. The reaction mixture was concentrated under reduced pressure, and the resulting residue was purified by thin layer chromatography (silica gel, methylene chloride:methanol = 9:1) and then thin layer chromatography (silica gel, n-hexane:ethyl acetate = 2:1) again to obtain yellow amorphous solid.

The obtained solid was dissolved in tetrahydrofuran (30 ml), added with tetra-n-butyl ammonium fluoride THF solution (1:0 M solution, 474 µl, 0.474 mmol) and stirred at room temperature for 3 hours and 30 minutes. The reaction mixture was concentrated under reduced pressure, and the resulting residue was purified by column chromatography (silica gel, methylene chloride:methanol = 20:1). The resulting crude product was purified by thin layer chromatography (silica gel, ethyl acetate:methanol = 9:1), and the resulting solid was washed with diethyl ether to obtain the title compound (116 mg) as yellow powder.
¹H-NMR (CD₃OD) δ: 1.25-1.70 (22H, m), 2.44-2.51 (2H, m), 3.57-3.76 (10H, m), 6.74 (1H, s), 7.01 (1H, d, J=15.6Hz), 7.52 (1H, d, J=15.6Hz), 7.64 (1H, m), 8.03 (1H, s), 8.97 (1H, d, J=7.32Hz)
LRMS-FAB; m/z: 639 (MH⁺)

| HRMS-FAB (C₃₂H₄₃O₆N₆S); m/z: | |
|---|---|
| Calcd. (m/z) | 639.2965 |
| Found (m/z) | 639.2975 |

### (E) (E)-3-[8-({[4-(t-Butyl)-1,3-thiazol-2-yl]amino}carbonyl)-2-(4-{5-[(hydroxysulfonyl)-oxy]pentanoyl}piperazino)-4-oxo-4H-pyrido[1,2-a]pyrimidin-3-yl]-2-propenoic acid t-butyl ester

A solution of (E)-3-{8-({[4-(t-butyl)-1,3-thiazol-2-yl)amino}carbonyl)-2-[4-(5-hydroxypentanoyl)piperazino]-4-oxo-4H-pyrido[1,2-a]pyrimidin-3-yl}-2-propenoic acid t-butyl ester (101 mg, 0.159 mmol) in dimethylformamide (9 ml) was added with dicyclohexylcarbodiimide (164 mg, 0.795 mmol) and added with a solution of concentrated sulfuric acid (12.7 µ l, 0.239 mmol) in dimethylformamide (1.0 ml) with ice cooling. The reaction solution was stirred for 40 minutes, then added with triethylamine (2.0 ml) and concentrated under reduced pressure, and the resulting residue was purified by thin layer chromatography (silica gel, methylene chloride:methanol = 9:1). The resulting solid was purified by thin layer chromatography (silica gel, separated with ethyl acetate:methanol = 9:1, then separated with acetate:methanol = 5:1). The resulting solid was washed with diethyl ether and dried under reduced pressure to obtain the title compound (60.8 mg) as orange amorphous solid.
¹H-NMR (CD₃OD) δ : 1.35 (9H, s), 1.53 (9H, s), 1.75-1.77 (4H, m), 2.53 (2H, t, J=7.32Hz), 3.59-3.76 (8H, m), 4.04 (2H, t, J=5.98Hz), 6.74 (1H, s), 7.00 (1H, d, J=15.6Hz), 7.53 (1H, d, J=15.6Hz), 7.62 (1H, d, J=6.10Hz), 8.06 (1H, s), 8.97 (1H, d, J=7.32Hz)
LRMS-FAB; m/z; 719 (MH⁺)

| HRMS-FAB (C₃₂H₄₃O₉N₆S₂); m/z: | |
|---|---|
| Calcd. | 719.2533 |
| Found | 719.2578 |

### (F) (E)-3-[8-({[4-(t-Butyl)-1,3-thiazol-2-yl]amino}carbonyl)-2-(4-{5-[(hydroxysulfonyl)oxy]pentanoyl}piperazino)-4-oxo-4H-pyrido[1,2-a]pyrimidin-3-yl]-2-propenoic acid

A solution of (E)-3-[8-({[4-(t-butyl)-1,3-thiazol-2-yl]amino}carbonyl)-2-(4-{5-[(hydroxysulfonyl)oxy]pentanoyl}piperazino)-4-oxo-4H-pyrido[1,2-a]pyrimidin-3-yl]-2-propenoic acid t-butyl ester (60 mg, 0.0835 mmol) in methylene chloride (10 ml) was added with trifluoroacetic acid (10 ml) and stirred at room temperature for 2 hours. The reaction mixture was concentrated under reduced pressure, and the resulting solid was washed with diethyl ether and dried under reduced pressure to obtain the title compound (54.6 mg) as orange powder.
m.p.: 230°C (decomp.)
¹H-NMR (DMSO-d₆) δ : 1.32 (9H, s), 1.55 (4H, brs), 2.33-2.38 (2H, m), 3.58-3.72 (10H, m), 6.85 (1H, s), 6.95 (1H, d, J=15.4Hz), 7.48 (1H, d, J=15.4Hz), 7.63 (1H, d, J=7.34Hz), 8.25 (1H, s), 8.93 (1H, d, J=7.58Hz)
IR (ATR) cm⁻¹: 2962, 1676, 1593, 1518, 1433, 1200, 984, 742, 582
LRMS-FAB; m/z: 663 (MH⁺)

| HRMS-FAB (C₂₈H₃₅O₉N₆S₂); m/z: | |
|---|---|
| Calcd. | 663.1907 |
| Found | 663.1907 |

| Anal. (for C₂₈H₃₄N₆O₉S₂ • 1.0TFA • 2.0H₂O) | | | |
|---|---|---|---|
| Calcd. | C, 44.33; | H, 4.84; | N, 10.34 |
| Found | C, 44.18; | H, 5.01; | N, 10.36 |

### Example 202: (E)-3-[8-({[4-(t-Butyl)-1,3-thiazol-2-yl]amino}carbonyl)-2-(4-(5-[(carboxymethyl)(methyl)amino]pentanoyl}piperazino)-4-oxo-4H-pyrido[1,2-a]-pyrimidin-3-yl]-2-propenoic acid

### (A) 5-[[2-(t-Butoxy)-2-oxoethyl](methyl)amino]pentanoic acid benzyl ester

A solution of 5-bromopentanoic acid benzyl ester (4.05 g, 14.9 mmol), sarcosine t-butyl ester hydrochloride (5.41 g, 29.8 mmol) and triethylamine (4.53 g, 44.7 mmol) in methylene chloride (160 ml) was refluxed overnight by heating and concentrated under reduced pressure. The resulting residue was dissolved in methylene chloride, successively washed with water and saturated brine and dried over anhydrous sodium sulfate. The solvent was evaporated under reduced pressure, and the resulting residue was purified by column chromatography (silica gel, n-hexane:ethyl acetate = 3:1) to obtain the title compound (3.48 g) as colorless oily substance.
¹H-NMR (CDCl₃) δ : 1.46-1.54 (11H, m), 1.61-1.70 (2H, m), 2.33 (3H, s), 2.37-2.40 (2H, m), 2.45-2.49 (2H, m), 3.12 (2H, s), 5.11 (2H, s), 7.29-7.38 (5H, m)
LRMS-ESI; m/z: 336 (MH⁺)

### (B) 5-[[2-(t-Butoxy)-2-oxoethyl](methyl)amino]pentanoic acid

A suspension of 5-[[2-(t-butoxy)-2-oxoethyl](methyl)amino]pentanoic acid benzyl ester (500 mg, 1.49 mmol) and 10% palladium carbon catalyst in methanol (20 ml) was stirred overnight at room temperature under hydrogen atmosphere. The catalyst was removed by filtration, and then the filtrate was concentrated under reduced pressure to obtain the title compound (353 mg) as colorless oily substance.
¹H-NMR (CDCl₃) δ : 1.46-1.59 (13H, m), 2.28-2.31 (2H, m), 2.46 (3H, s), 2.63-2.67 (2H, m), 3.29 (2H, s)
LRMS-FAB; m/z: 246 (MH⁺)

| HRMS-FAB (C₁₂H₂₄O₄N); m/z: | |
|---|---|
| Calcd. | 246.1705 |
| Found | 246.1697 |

IR (ATR) cm⁻¹: 1728, 1367, 1221, 1151, 1059, 839, 735

### (C) (E)-3-[2-(4-{5-[[2-(t-Butoxy)-2-oxoethyl](methyl)amino]pentanoyl)piperazino)-8-({[4-(t-butyl)-1,3-thiazol-2-yl]amino}carbonyl)-4-oxo-4H-pyrido[1,2-a]pyrimidin-3-yl]-2-propenoic acid t-butyl ester

A solution of (E)-3-[8-({[4-(t-butyl)-1,3-thiazol-2-yl]amino}carbonyl)-2-hydroxy-4H-pyrido[1,2-a]pyrimidin-3-yl]-2-propenoic acid t-butyl ester (150 mg, 0.319 mmol) in dimethylformamide (30 ml) was added with 4-dimethylaminopyridine (58.5 mg, 0.479 mmol) and tosyl chloride (91.3 mg, 0.479 mmol) and stirred at room temperature for 4 hours. This solution was added with piperazine (137 mg, 1.60 mmol) and further stirred overnight at room temperature.

The reaction mixture was concentrated under reduced pressure, and the resulting orange solid was dissolved in a mixed solution of dimethylformamide (10 ml) and methylene chloride (30 ml). This solution was added with 5-[[2-(t-butoxy)-2-oxoethyl](methyl)amino]pentanoic acid (196 mg, 0.798 mmol) and EDC•HCl (183 mg, 0.957 mmol), stirred overnight at room temperature and concentrated under reduced pressure. The resulting residue was purified by thin layer chromatography (silica gel, methylene chloride:methanol = 9:1) to obtain a crude product. This product was purified by thin layer chromatography (silica gel, ethyl acetate) again to obtain the title compound (107 mg) as orange solid.
¹H-NMR (CD₃OD) δ : 1.28-1.69 (31H, m), 2.34 (3H, s), 2.48-2.54 (4H, m), 3.18 (2H, s), 3.63-3.76 (8H, m), 6.73 (1H, s), 6.98 (1H, d, J=15.9Hz), 7.49 (1H, d, J=15.9Hz), 7.60 (1H, d, J=7.08Hz), 7.99 (1H, s), 8.93 (1H, d, J=7.08Hz)
LRMS-FAB; m/z: 766 (MH⁺)

| HRMS-FAB (C₃₉H₅₆O₇N₇S); m/z: | |
|---|---|
| Calcd. | 766.3962 |
| Found | 766.3965 |

IR (ATR) cm⁻¹: 2968, 1671, 1549, 1516, 1433, 1365, 1292, 1223, 1149, 1061, 1016, 984, 854, 733, 702

### (D) (E)-3-[8-({[4-(t-Butyl)-1,3-thiazol-2-yl]amino}carbonyl)-2-(4-{5-[(carboxymethyl)-(methyl)amino]pentanoyl}piperazino)-4-oxo-4H-pyrido[1,2-a]pyrimidin-3-yl]-2-propenoic acid

A solution of (E)-3-[8-({[4-(t-butyl)-1,3-thiazol-2-yl]amino}carbonyl)-2-(4-{5-[(carboxymethyl)(methyl)amino]pentanoyl}piperazino)-4-oxo-4H-pyrido[1,2-a]pyrimidin-3-yl]-2-propenoic acid t-butyl ester (57.1 mg, 0.0745 mmol) in methylene chloride (10 ml) was added with trifluoroacetic acid (10 ml) and stirred at room temperature for 2 hours. The reaction solution was concentrated under reduced pressure, and the residue was subjected to azeotropy with toluene. The resulting solid was washed with diethyl ether and dried under reduced pressure to obtain the title compound (49.6 mg) as orange powder.
m.p.: 169-172°C
¹H-NMR (CD₃OD) δ : 1.35 (9H, s), 1.69-1.84 (4H, m), 2.56 (2H, t, J=7.08Hz), 2.96 (3H, s), 3.24 (2H, brs), 3.67-3.79 (8H, m), 4.08 (2H, brs), 6.75 (1H, s), 7.08 (1H, d, J=15.6Hz), 7.61-7.66 (2H, m), 8.04 (1H, d, J=1.22Hz), 9.00 (1H, d, J=7.32Hz)
LRMS-FAB; m/z: 654 (MH⁺)

| HRMS-FAB (C₃₁H₄₀O₇N₇S); m/z: | |
|---|---|
| Calcd. | 654.2710 |
| Found | 654.2708 |

IR (ATR) cm⁻¹: 1672, 1520, 1437, 1284, 1180, 1132, 984, 742, 719

| Anal. (for C₃₁H₃₉N₇O₇S • 1.5TFA • 1.25H₂O) | | | |
|---|---|---|---|
| Calcd. | C, 48.20; | H, 5.12; | N, 11.57 |
| Found | C, 48.38; | H, 4.95; | N, 11.20 |

### Example 203: [5-(4-{8-({[4-(t- Butyl-1,3-thiazol-2-yl]amino}carbonyl)-3-[(E)-2-carboxy-1-ethenyl]-4-oxo-4H-pyrido(1,2-a]pyrimidin-2-yl}piperazino)-5-oxopentyl]-(carboxymethyl)dimethylammonium

A solution of (E)-3-[8-({[4-(t-butyl]-1,3-thiazol-2-yl]amino}carbonyl-2-(4-{5-[(carboxymethyl)(methyl)amino]pentanoyl}piperazino)-4-oxo-4H-pyrido[1,2-a]pyrimidin-3-yl]-2-propenoic acid t-butyl ester (50.0 mg, 0.0653 mmol) in dimethylformamide (10 ml) was added with methyl iodide (162 µl, 2.61 mmol) and left stand at -5°C for 3 days. The reaction solution was concentrated under reduced pressure, and then the residue was dissolved in methylene chloride (10 ml), added with trifluoroacetic acid (10 ml) and stirred at room temperature for 2 hours. This solution was concentrated under reduced pressure, and the residue was further subjected to azeotropy with toluene. Then the residue was purified by high performance liquid chromatography (containing 0.1% formic acid, acetonitrile:water = 3:7) and lyophilized to obtain the title compound (33.8 mg) as orange solid.
m.p.: 220-228°C
¹H-NMR (CD₃OD) δ : 1.35 (9H, s), 1.65-1.72 (2h, m), 1.81-1.89 (2H, m), 2.57 (2H, t, J=7.09Hz), 3.29 (6H, s), 3.58-3.78 (10H, m), 4.26 (2H, s), 6.75 (1H, s), 7.07 (1H, d, J=15.7Hz), 7.61-7.66 (2H, m), 8.04 (1H, d, J=1.22Hz), 8.98 (1H, d, J=7.34Hz)
LRMS-FAB; m/z: 668 (MH⁺)
HRMS-FAB (C₃₂H₄₂O₇N₇S); m/z:
Calcd.: 668.2866.
Found: 668.2866
IR (ATR) cm⁻¹: 1631, 1516, 1435, 1379, 1308, 1225, 1103, 1012, 984, 872, 735, 702

| Anal. (for C₃₂H₄₁N₇O₇S • 1.5 formic acid • 4.25H₂O) | | | |
|---|---|---|---|
| Calcd. | C, 49.41; | H, 6.62; | N, 12.04 |
| Found | C, 49.48; | H, 6.36; | N, 11.70 |

### Example 204: (2-Amino-2-oxoethyl)(2-{[({(3R)-1-(t-butoxy)-3-oxo-1-propenyl}-8-({[4-(t-butyl)-1,3-thiazol-2-yl]amino}carbonyl)-4-oxo-4H-pyrido[1,2-a]pyrimidin-2-yl)hexahydro-3-pyridinyl}oxy}carbonyl}amino}ethyl}dimethylammonium

### (A) N⁸-[4-(t-Butyl)-1,3-thiazol-2-yl]-2-[(3R)-3-hydroxyhexahydro-1-pyridinyl]-4-oxo-4H-pyrido[1,2-a]pyrimidine-8-carboxyamide

Under argon atmosphere, N⁸-[4-(t-butyl)-1,3-thiazol--2-yl]-2,4-dioxo-3,4-dihydro-2H-pyrido[1,2-a]pyrimidine-8-carboxyamide (5.0 g, 14.5 mmol) in a mixed solvent of dimethylformamide (120 ml) and acetonitrile (60 ml) was added with diphenyl chlorophosphate (6.10 ml, 29.4 mmol) and diisopropylethylamine (10 ml) with ice cooling and stirred for 40 minutes. This solution was added with (R)-(+)-3-hydroxypiperidine hydrochloride (3.0 g, 21.8 mmol) and diisopropylethylamine (5.0 ml) and stirred at 90-100°C for 2 hours. The reaction solution was cooled to room temperature and concentrated under reduced pressure, and the residue was dissolved in ethyl acetate, washed with water (three times) and saturated brine and dried over anhydrous sodium sulfate. When the solvent was evaporated under reduced pressure, and the residue was added with methylene chloride and left stand overnight at room temperature, solid precipitated. This solid was taken by filtration with washing with methanol to obtain the title compound (3.75 g) as pale yellow powder.
¹H-NMR (CDCl₃) δ : 1.34 (9H, s), 1.60-1.76 (2H, m), 1.97-1.98 (2H, m), 3.61 (3H, brs), 3.96 (2H, brs), 5.72 (1H, s), 6.62 (1H, s), 7.31 (1H, d, J=1.71Hz), 7.79 (1H, d, J=1.47Hz), 8.91 (1H, d, J=7.32Hz)
LRMS-FAB; m/z: 428 (MH⁺)

| HRMS-FAB (C₂₁H₂₆O₃N₅S); m/z: | |
|---|---|
| Calcd. | 428.1756 |
| Found | 428.1724 |

IR (ATR) cm⁻¹: 2962, 1660, 1637, 1531, 1496, 1410, 1331, 1223, 1063, 856, 758, 490

### (B) (E)-3-{8-({[4-(t-Butyl)-1,3-thiazol-2-yl]amino}carbonyl)-2-[(3R)-3-formyloxyhexahydro-1-pyridinyl]-4-oxo-4H-pyrido[1,2-a]pyrimidin-3-yl}-2-propenoic acid t-butyl ester

Under argon atmosphere, dimethylformamide (16 ml) was cooled with ice, added with phosphorus oxychloride (1.58 ml, 17.0 mmol) and stirred at room temperature for 30 minutes. This solution was added with a solution of N⁸-[4-(t-butyl)-1,3-thiazol-2-yl]-2-[(3R)-3-hydroxyhexahydro-1-pyridinyl]-4-oxo-4H-pyrido[1,2-a]pyrimidine-8-carboxamide (3.64 g, 8.51 mmol) in dimethylformamide (16 ml) with ice cooling and stirred for 2 hours. This solution was added with saturated aqueous sodium hydrogencarbonate to terminate the reaction and extracted with chloroform three times. The combined organic layer was washed with saturated brine and dried over anhydrous sodium sulfate. The solvent was evaporated under reduced pressure, and then the resulting residue was dissolved in tetrahydrofuran (100 ml), added with (t-butoxycarbonylmethylene)triphenylphosphorane (9.64 g, 25.6 mmol) and refluxed by heating for 11 hours. The reaction mixture was cooled to room temperature and concentrated under reduced pressure, and the residue was purified by column chromatography (silica gel, n-hexane:ethyl acetate = 3:1-2:1) to obtain the title compound (2.30 g) as orange solid.
¹H-NMR (CDCl₃) δ : 1.34 (9H, s), 1.52 (9H, s), 1.68-2.02 (4H, m), 3.56-3.88 (4H, m), 5.16 (1H, brs), 6.62 (1H, s), 7.09 (1H, d, J=15.6Hz), 7.45-7.49 (2H, m), 7.90 (1H, d, J=1.22Hz), 8.10 (1H, s), 8.98-9.00 (1H, m)
LRMS-FAB; m/z: 582 (MH⁺)

### (C) (E)-3-(8-({[4-(t-Butyl)-1,3-thiazol-2-yl]amino}carbonyl)-2-[(3R)-3-hydroxyhexahydro-1-pyridinyl]-4-oxo-4H-pyrido[1,2-a]pyrimidin-3-yl}-2-propenoic acid t-butyl ester

A solution of (E)-3-{8-({(4-(t-butyl)-1,3-thiazol-2-yl]amino}carbonyl)-2-[(3R)-3-formyloxyhexahydro-1-pyridinyl]-4-oxo-4H-pyrido[1,2-a]pyrimidin-3-yl}-2-propenoic acid t-butyl ester (2.30 g, 3.95 mmol) in methanol (40 ml) was added with sodium methoxide (962 mg, 17.8 mmol) with ice cooling and stirred for 10 minutes. This solution was added with saturated brine and extracted with chloroform, and the aqueous layer was further extracted twice with a mixed solution of chloroform and methanol (9:1). The combined organic layer was dried over anhydrous sodium sulfate. The solvent was evaporated under reduced pressure, and the residue was purified by column chromatography (silica gel, methylene chloride:methanol = 9:1) to obtain the title compound (1.91 g) as yellow powder.
¹H-NMR (CDCl₃) δ : 1.37 (9H, s), 1.40-1.91 (13H, m), 3.58-3.68 (3H, m), 3.86 (1H, brs), 4.06 (1H, brs), 6.56 (1H, s), 7.06 (1H, d, J=15.7Hz), 7.44-7.48 (2H, m), 7.98 (1H, brs), 8.95 (1H, d, J=7.34Hz)
LRMS-FAB; m/z: 554 (MH⁺)

| HRMS-FAB (C₂₈H₃₆O₅N₅S); m/z: | |
|---|---|
| Calcd. | 554.2437 |
| Found | 554.2464 |

### (D) (E)-3-{8-(4-t-Butylthiazol-2-ylcarbamoyl)-2-[(3R)-3-(2-dimethylaminoethylcarbamoyloxy)piperidin-1-yl]-4-oxo-4H-pyrido[1,2-a]pyrimidin-3-yl}-2-propenoic acid t-butyl ester

Under argon atmosphere, a solution of (E)-3-{8-({[4-(t-butyl)-1,3-thiazol-2-yl]amino}carbonyl)-2-[(3R)-3-hydroxyhexahydro-1-pyridinyl]-4-oxo-4H-pyrido[1,2-a]pyrimidin-3-yl}-2-propenoic acid t-butyl ester (1.00 g, 1.81 mmol) and pyridine (227 µl, 2.82 mmol) in tetrahydrofuran (20 ml) was added with 2-chloroethyl isocyanate (311 µl, 3.62 mmol) and stirred at 80°C for 7 hours. The solution was added with 2-chloroethyl isocyanate (933 µl, 10.9 mmol) again, stirred for 14 hours, further added with 2-chloroethyl isocyanate (622 µl) and stirred for 3 hours. The reaction mixture was cooled to room temperature, added with saturated aqueous sodium hydrogencarbonate and extracted with chloroform three times. The combined organic layer was dried over anhydrous sodium sulfate. The solvent was evaporated under reduced pressure, and the resulting residue was purified by column chromatography (silica gel, methylene chloride:methanol = 40:1) to obtain the title compound (2.06 g) as crude product of yellow amorphous solid.

This solid (300 mg) was dissolved in dimethylformamide (30 ml), added with a solution of dimethylamine in tetrahydrofuran (2.0 M, 23 ml) and sodium iodide (4.0 mg) and stirred overnight at 60°C. The reaction mixture was cooled to room temperature and concentrated under reduced pressure, and the resulting residue was purified by column chromatography (silica gel, methylene chloride:methanol = 9:1) to obtain the title compound (159 mg) as yellow amorphous solid.
¹H-NMR (CDCl₃) δ : 1.33 (9H, s), 1.52 (9H, s), 165-2.13 (4H, m), 2.24 (6H, s), 2.47 (2H, brs), 3.31 (2H, brs), 3.49-3.83 (4H, m), 4.89 (1H, brs), 5.54 (1H, brs), 6.60 (1H, s), 7.10 (1H, d, J=15.7Hz), 7.46 (1H, dd, J=1.84, 7.47Hz), 7.54 (1H, d, J=15.4Hz), 7.93 (1H, d, J=1.23Hz), 8.98 (1H, d, J=7.35Hz)

### (E) (E)-3-{8-t-Butylthiazol-2-ylcarbamoyl}-2-[(3R)-3-(2-dimethylaminoethylcarbamoyloxy)piperidin-1-yl]-4-oxo-4H-pyrido[1,2-a]pyrimidin-3-yl}-2-propenoic acid

A solution of (E)-3-{8-(4-t-butylthiazol-2-yl-carbamoyl)-2-[(3R)-3-(2-dimethylaminoethylcarbamoyloxy)piperidin-1-yl]-4-oxo-4H-pyrido[1,2-a]pyrimidin-3-yl}-2-propenoic acid t-butyl ester (159 mg, 0.239 mmol) in methylene chloride (10 ml) was added with trifluoroacetic acid (10 ml) and stirred at room temperature for 30 minutes. The reaction mixture was concentrated under reduced pressure, and the resulting residue was purified by thin layer chromatography (silica gel, methylene chloride:methanol = 9:1, thereafter 5:1). The resulting solid was washed with diethylether to obtain the title compound (118 mg) as orange powder.
¹H-NMR (CDCl₃) δ : 3.34 (9H, s), 1.65-1.72 (2H, m), 1.94-1.97 (1H, m), 2.13-2.17 (1H, m), 2.56 (6H, s), 2.64-2.68 (1H, m), 2.85-2.90 (1H, m), 2.94-3.00 (1H, m), 3.03-3.12 (1H, m), 3.18-3.21 (1H, m), 3.70-3.73 (1H, m), 3.86 (1H, d, J=13.5Hz), 4.14 (1H, d, J=12.7Hz), 4.98 (1H, brs), 6.17 (1H, d, J=6.37Hz), 6.61 (1H, s), 6.97 (1H, d, J=15.4Hz), 7.47 (1H, dd, J=7.84, 7.47Hz), 7.81 (1H, d, J=15.7Hz), 7.87 (1H, s), 9.03 (1H, d, J=7.35Hz)
LRMS-FAB; m/z: 612 (MH⁺)

| HRMS-FAB (C₂₉H₃₈O₆N₇S); m/z: | |
|---|---|
| Calcd. | 612.2604 |
| Found | 612.2103 |

### (F) (2-Amino-2-oxoethy)(2-{[({(3R)-1-(t-butoxy)-3-oxo-1-propenyl}-8-({[4-(t-butyl)-1,3-thiazol-2-yl]amino]carbonyl)-4-oxo-4H-pyrido[1,2-a]pyrimidin-2-yl)hexahydro-3-pyridinyl]oxy}carbonyl)amino}ethyl)dimethylammonium

A solution of (E)-3-{8-t-butylthiazol-2-ylcarbamoyl}-2-[(3R)-3-(2-dimethylaminoethylcarbamoyloxy)piperidin-1-yl]-4-oxo-4H-pyrido[1,2-a]pyrimidin-3-yl}-2-propenoic acid (116 mg, 0.189 mmol) in dimethylformamide (20 ml) was added with iodoacetamide (52.5 mg, 0.284 mmol) and stirred overnight at room temperature. The reaction mixture was concentrated under reduced pressure, and the residue was purified by thin layer chromatography (silica gel, lower layer of chloroform: methanol:water = 7:3:1, developed twice). The resulting solid was washed with diethyl ether to obtain the title compound (107 mg) as orange powder.
m.p.: 191-194°C
¹H-NMR (CD₃OD) δ : 1.34 (9H, s), 1.70 (1H, brs), 1.88-1.91 (1H, m), 1.91-2.01 (2H, m), 3.30-3.34 (10H, m), 3.46-3.74 (3H, m), 3.91-3.94 (2H, m), 4.21 (2H, s), 6.74 (1H, s), 7.10 (1H, d, J=15.7Hz), 7.52-7.59 (2H, m), 8.00 (1H, d, J=1.23Hz), 8.95 (1H, d, J=7.35Hz) LRMS-FAB; m/z: 669 (MH⁺)

| HRMS-FAB (C₃₁H₄₁O₇N₈S); m/z: | |
|---|---|
| Calcd. | 669.2819 |
| Found | 669.2801 |

IR (ATR) cm⁻¹: 1658, 1512, 1431, 1362, 1227, 1101, 862, 737, 700

| Anal. (for C₃₁H₄₀N₈O₇S•0.25TFA•3.0H₂O) | | | |
|---|---|---|---|
| Calcd. | C, 50.36; | H, 6.20; | N, 14.91 |
| Found | C, 50.68; | H, 6.36; | N, 14.77 |

### Example 205: [2-({[((3R)-1-{8-({[4-(t-Butyl)-1,3-thiazol-2-yl]amino}carbonyl)-3-[(E)-2-carboxy-1-ethenyl]-4-oxo-4H-pyrido[1,2-a]pyrimidin-2-yl}hexahydro-3-pyridinyl)-oxy]carbonyl}amino)ethyl](dimethyl)[2-(methylamino)-2-oxoethyl]ammonium

A solution of (E)-3-}8-t-butylthiazol-2-ylcarbamoyl}-2-[(3R)-3-(2-dimethylaminoethylcarbamoyloxy)piperidin-1-yl]-4-oxo-4H-pyrido[1,2-alpyrimidin-3-yl}-2-propenoic acid (56.9 mg, 0.0930 mmol) in dimethylformamide (10 ml) was added with N¹-methyl-2-iodoacetamide (27.9 mg, 0.140 mmol) and stirred overnight at room temperature. The reaction mixture was concentrated under reduced pressure, and the residue was purified by thin layer chromatography (silica gel, chloroform:methanol:water = 8:3:0.5, developed twice). The resulting solid was washed with diethyl ether to obtain the title compound (46 mg) as orange powder. m.p.: 185-190°C
¹H-NMR (CD₃OD) δ: 1.35 (9H, s), 1.70 (1H, brs), 1.88-1.99 (3H, m), 2.78 (3H, s), 3.30-3.69 (12H, m), 3.90-3.93 (2H, m), 4.16 (2H, s), 6.74 (1H, s), 7.12 (1H, d, 15.7Hz), 7.50-7.60 (2H, m), 8.00 (1H, d, J=1.96Hz), 8.95 (1H, d, J=7.35Hz)
LRMS-FAB; m/z: 683 (MH⁺)
IR (ATR) cm⁻¹: 1662, 1510, 1433, 1360, 1227, 735, 700

| Anal. (for C₃₂H₄₂N₈O₇S•0.25TFA•2.OH₂O) | | | | |
|---|---|---|---|---|
| Calcd. | C, 52.23; | H, 6.24; | N, 14.99; | S, 4:29 |
| Found | C, 52.26; | H, 6.06; | N, 15.17; | S, 4.37 |

### Example 206: 1-{8-({[4-(t-Butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}-4-piperidylcarbamate

### (A) N⁸-[4-(t-Butyl)-1,3-thiazol-2-yl]-2-hydroxy-3-{(E)-2-(1-(4-methoxybenzyl)-1H-1,2,3,4-tetrazol-5-yl]-1-ethenyl}-4-oxo-4H-pyrido[1,2-a]pyridopyrimidine-8-carboxyamide

A mixed solution of dimethylformamide (1.67 ml, 21.8 mmol) and methylene chloride (50 ml) was added with oxalyl chloride (1.86 ml, 21.8 mmol) with ice cooling and stirred for 25 minutes. This solution was added with N⁸-[4-(t-butyl)-1,3-thiazol-2-yl]-2,4-dioxo-3,4-dihydro-2H-pyrido[1,2-a]pyrimidine-8-carboxamide (3.00 g, 8.71 mmol) and methylene chloride (20 ml), stirred at room temperature for 1 hour and added with saturated aqueous sodium hydrogencarbonate to terminate the reaction. The resulting solution was adjusted to about pH 3 with 1 N aqueous hydrochloric acid. This solution was extracted with chloroform (10 times), and the combined organic layer was concentrated under reduced pressure.

The resulting residue, 2-[1-(4-methoxybenzyl)-1H-1,2,3,4 tetrazol-5-yl]acetic acid (3.25 g, 13.1 mmol), was dissolved in a mixed solution of piperidine (30 ml) and pyridine (200 ml) and refluxed by heating for 3 hours. The reaction mixture was cooled to room temperature, concentrated under reduced pressure and added with 1 N aqueous hydrochloric acid. The precipitated solid was collected by filtration and washed with ethyl acetate. To remove the remaining piperidine, this solid was suspended in 1 N aqueous hydrochloric acid, stirred, then collected by filtration and washed with ethyl acetate to obtain the title compound (2.00 g, 41%) as orange powder. ¹H-NMR (DMSO-d₆) δ : 1.30 (9H, s), 3.71 (3H, s), 5.57 (2H, s), 6.86 (1H, d, J=12.0Hz), 6.92 (1H, d, J=8.56Hz), 7.22 (1H, d, J=8.56Hz), 7.70 (1H, d, J=15.9Hz), 7.93 (1H, d, J=7.34Hz), 7.95 (1H, s), 8.05 (1H, d, J=15.7Hz), 9.14 (1H, d, J=7.09Hz)

### (B) 1-(8-({[4-(t-Butyl)-1,3-thiazol-2-yl]amino}carbonyl)-3-{(E)-2-[1-(4-methoxybenzyl)-1H-1,2,3,4-tetrazol-5-yl]-1-ethenyl}-4-oxo-4H-pyrido[1,2-a]pyrimidin-2-yl)-4-piperidylcarbamate

N⁸-[4-(t-Butyl)-1,3-thiazol-2-yl]-2-hydroxy-3-{(E)-2-[1-(4-methoxybenzyl)-1H-1,2,3,4-tetrazol-5-yl]-1-ethenyl}-4-oxo-4H-pyrido[1,2-a]pyrimidine-8-carboxamide (200 mg, 0.358 mmol) in a mixed solvent of dimethylformamide (20 ml) and acetonitrile (10 ml) was added with diphenyl chlorophosphate (148 µ l, 0.756 mmol) and diisopropylethylamine (245 µ l, 1.43 mmol) with ice cooling and stirred for 20 minutes. This solution was added with 4-hydroxypiperidine (543 mg, 0.537 mmol) and stirred at 80-90°C for 2 hours. The reaction mixture was cooled to room temperature and concentrated under reduced pressure, and the residue was added with ethyl acetate, successively washed three times with water and once with saturated brine. The solvent was evaporated under reduced pressure, and the residue was purified by thin layer chromatography (silica gel, chloroform:methanol = 9:1) and further purified by thin layer chromatography (silica gel, n-hexane:ethyl acetate = 1:3) to obtain the crude product as orange solid. This solid was dissolved in ethyl acetate (5.0 ml), added with trichloroethyl isocyanate (130 *µ*l, 1.10 mmol) and stirred at room temperature for 85 minutes. This solution was added with a mixed solution of chloroform and methanol (10:1, 10 ml) and concentrated under reduced pressure. The resulting residue was added with tetrahydrofuran (40 ml), water (20 ml) and sodium formate (74.8 mg, 1.10 mmol) and stirred at room temperature for 3 hours and 30 minutes. The reaction mixture was concentrated under reduced pressure and extracted with chloroform three times. The combined organic layer was washed with saturated brine and dried over anhydrous magnesium sulfate. Further, the solvent was evaporated under reduced pressure, and the resulting residue was purified by thin layer chromatography (silica gel, methylene chloride:methanol = 9:1) to obtain the title compound (157 mg) as yellow solid.
¹H-NMR (CDCl₃) δ : 1.32 (9H, s), 1.81-2.04 (4H, m), 3.08-3.16 (1H, m), 3.44-3.59 (3H, m), 3.78-3.82 (1H, m), 4.63-4.77 (1H, m), 4.97-4.99 (1H, m), 5.51 (2H, s), 6.64 (1H, s), 6.87 (1H, d, J=8.82Hz), 7.15-7.18 (1H, m), 7.25-7.36 (3H, m), 7.55 (1H, dd, J=1.84, 7.47Hz), 7.71 (1H, d, J=15.4Hz), 7.88 (1H, d, J=15.7Hz), 8.14 (1H, s), 9.01 (1H, d, J=7.58Hz)
LRMS-ESI; m/z: 685 (MH⁺)

### (C) 1-{8-({[4-(t-Butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}-4-piperidylcarbamate

1-(8-({[4-(t-Butyl)-1,3-thiazol-2-yl]amino}carbonyl)-3-{(E)-2-[1-(4-methoxybenzyl)-1H-1,2,3,4-tetrazol-5-yl]-1-ethenyl}-4-oxo-4H-pyrido[1,2-a]pyrimidin-2-yl)-4-piperidylcarbamate (157 mg, 0.229 mmol) was dissolved in trifluoroacetic acid (30 ml) and stirred at 60°C for 3 hours. The reaction mixture was cooled to room temperature and concentrated under reduced pressure, and the residue was purified by thin layer chromatography (silica gel, lower layer of chloroform:methanol:water = 7:3:1) to obtain the title compound (21.6 mg) as orange solid.
m.p.: 270-273°C (decomp.)
¹H-NMR (DMSO-d₆) δ : 1.30 (9H, s), 1.70-1.72 (2H, m), 1.99 (2H, brs), 3.32-3.40 (2H, m), 3.86-3.89 (2H, m), 4.77 (1H, brs), 6.51 (2H, brs), 6.86 (1H, s), 7.47 (1H, d, J=16.1Hz), 7.63 (1H, d, J=8.54Hz), 7.82 (1H, d, J=15.8Hz), 8.23 (1H, s), 8.94 (1H, d, J=7.32Hz) LRMS-FAB; m/z: 565 (MH⁺)
IR (ATR) cm⁻¹: 1716, 1637, 1601, 1502, 1442, 1406, 1333, 1227, 1065, 754

| Anal. (for C₂₅H₂₈N₁₀O₄S•0.50TFA) | | | | |
|---|---|---|---|---|
| Calcd. | C, 50.24; | H, 4.62; | N, 22.53; | S, 5.16 |
| Found | C, 50.21; | H, 4.77; | N, 22.45; | S, 5.22 |

### Example 207: (3R)-1-{8-({[4-(t-Butyl)-1,3-thiazol-2-yl]amino]carbonyl)-4-oxo-3-[(E)-2-(1H-1,2,3,4-tetrazol-5-yl)-1-ethenyl]-4H-pyrido[1,2-a]pyrimidin-2-yl}hexahydro-3-pyridinylsulfamate

### (A) Sulfamoyl chloride

Under argon atmosphere, a solution of chlorosulfonyl isocyanate (3.08 ml, 35.3 mmol) in methylene chloride (15 ml) was added with a solution of formic acid (1.38 ml, 36.4 mmol) in methylene chloride (15 ml) little by little over 1 hour and 30 minutes. The reaction mixture was stirred overnight, refluxed by heating for 1 hour and further stirred at room temperature for 24 hours. This reaction solution was used as 1.18 M methylene chloride solution in the following steps (Ref. Tetrahedron Vol.50, No.23, pp.6825-6838, 1994).

### (B) N⁸-[4-(t-Butyl)-1,3-thiazol-2-yl]-2-[(3R)-3-hydroxyhexahydro-1-pyridinyl]-3-{(E)-2-[1-(4-methoxybenzyl)-1H-1,2,3,4-tetrazol-5-yl]-1-ethenyl}-4-oxo-4H-pyrido[1,2-a]-pyrimidine-8-carboxyamide

A solution of N⁸-[4-(t-butyl)-1,3-thiazol-2-yl]-2-hydroxy-3-{(E)-2-[1-(4-methoxybenzyl)-1H-1,2,3,4-tetrazol-5-yl]-1-ethenyl]-4-oxo-4H-pyrido[1,2-a]pyrimidine-8-carboxamide (1.00 g, 1.79 mmol) in dimethylformamide (50 ml) was added with diphenyl chlorophosphate (962 mg, 3.58 mmol) and diisopropylethylamine (1.22 ml, 7.16 mmol) with ice cooling and stirred for 40 minutes. This solution was added with (R)-(+)-3-hydroxypiperidine hydrochloride (739 mg, 5.37 mmol) and diisopropylethylamine (1.84 ml, 10.7 mmol) and stirred at 84-90°C for 2 hours. The reaction solution was cooled to room temperature and concentrated under reduced pressure, and the residue was purified by column chromatography (silica gel, chloroform:ethyl acetate = 1:1, chloroform:methanol = 100:1) to obtain the title compound (793 mg) as orange solid.
¹H-NMR (CDCl₃) δ : 1.38 (9H, s)1.54-1.90 (4H, m), 3.53 (1H, brs), 3.64-3.67 (2H, m), 3.78-3.86 (4H, m), 4.08 (1H, brs), 5.52 (2H, s), 6.52 (1H, s), 6.87-6.92 (2H, m), 7.14-7.36 (4H, m), 7.46 (1H, d, J=7.32Hz), 7.81 (1H, s), 8.00 (1H, brs), 8.88 (1H, d, J=7.57Hz)

### (C) (3R)-1-(8-({[4-(t-Butyl)-1,3-thiazol-2-yl]amino}carbonyl)-3-{(E)-2-[1-(4-methoxybenzyl)-1H-1,2,3,4-tetrazol-5-yl]-1-ethenyl}-4-oxo-4H-pyrido[1,2-a]-pyrimidin-2-yl)hexahydro-3-pyridinylsulfamate

A solution of N⁸-[4-(t-butyl)-1,3-thiazol-2-yl]-2-[(3R)-3-hydroxyhexahydro-1-pyridinyl]-3-{(E)-2-[1-(4-methoxybenzyl)-1H-1,2,3,4-tetrazol-5-yl]-1-ethenyl}-4-oxo-4H-pyrido[1,2-a]pyrimidine-8-carboxamide (793 mg, 1.24 mmol) and triethylamine (2.06 ml, 14.9 mmol) in methylene chloride (100 ml) with ice cooling was added with sulfamoyl chloride (1.18 mmol/ml. methylene chloride solution, 6.30 ml, 5.33 mmol) and 4-dimethylaminopyridine (75.7 mg, 0.620 mmol) and stirred overnight at room temperature. The reaction solution was concentrated under reduced pressure, added with water and extracted with methylene chloride three times. The combined organic layer was concentrated under reduced pressure. When the resulting residue was added with a mixed solution of n-hexane and ethyl acetate (1:1), solid precipitated. The solid was collected by filtration to obtain the title compound (404 mg) as yellow powder.
m.p.: 196-201°C
¹H-NMR (CDCl₃) δ : 1.37 (9H, s), 2.05 (4H, brs), 3.50-3.79 (7H, m), 4.85 (1H, brs), 5.53 (2H, s), 6.59 (1H, s), 6.91 (2H, d, J=8.79Hz), 7.37 (2H, d, J=8.55Hz), 7.45 (1H, brs), 7.73 (1H, d, J=18.6Hz), 7.91 (1H, s), 8.03 (1H, d, J=14.9Hz), 8.96 (1H, s)
LRMS-ESI; m/z: 721 (MH⁺)

### (D) (3R)-1-{8-({[4-(t-Butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}hexahydro-3-pyridinylsulfamate

(3R)-1-(8-({[4-(t-Butyl)-1,3-thiazol-2-yl]amino}carbonyl)-3-{(E)-2-[1-(4-methoxybenzyl)-1H-1,2,3,4-tetrazol-5-yl]-1-ethenyl}-4-oxo-4H-pyrido[1,2-a]pyrimidin-2-yl)hexahydro-3-pyridinylsulfamate (840 mg, 1.17 mmol) was dissolved in trifluoroacetic acid (50 ml) and anisole (5.0 ml), stirred overnight at room temperature and then stirred at 50°C for 7 hours and 30 minutes. The solution was concentrated under reduced pressure, and the residue was purified by column chromatography (silica gel, chloroform:methanol = 20:1·9:1). The resulting solid was dissolved in a lower layer of chloroform:methanol:water = 7:3:1 and filtered through a cotton plug to obtain the title compound (454 mg) as pale yellow powder.
m.p.: 238°C (decomp.)
¹H-NMR (CD₃OD) δ: 1.35 (9H, s), 1.77 (1H, brs), 1.94-2.03 (2H, m), 2.14-2.15 (1H, m), 3.63-3.68 (3H, m), 4.11 (1H, d, J=13.0Hz), 4.73-4.77 (1H, m), 6.73 (1H, s), 7.56-7.62 (2H, m), 7.89 (1H, d, J=16.1Hz), 8.04 (1H, s), 8.97-8.99 (1H, m)
LRMS-FAB; m/z: 601 (MH⁺)
IR (ATR) cm⁻¹: 2962, 1606, 1496, 1442, 1385, 1333, 1240, 1176, 1099, 962, 862, 804, 762, 735,704,542

| Anal. (for C₂₄H₂₈N₁₁O₅S₂•1.0H₂O) | | | |
|---|---|---|---|
| Calcd. | C, 46.59; | H, 4.89; | N, 22.64 |
| Found | C, 46.70; | H, 5.11; | N, 22.24 |

### Example 208: [2-({[(1-{8-({[4-(t-Butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}-4-piperidyl)oxy]carbonyl}amino)ethyl] (trimethyl)ammonium

### (A) 1-(8-({[4-(t-Butyl)-1,3-thiazol-2-yl]amino}carbonyl)-3-{(E)-2-[1-(4-methoxybenzyl)-1H-1,2,3,4-tetrazol-5-yl]-1-ethenyl}-4-oxo-4H-pyrido[1,2-a]pyrimidin-2-yl)-4-piperidyl-N-[2-(dimethylamino)ethyl]carbamate

A solution of N⁸-[4-(t-Butyl)-1,3-thiazol-2-yl]-2-hydroxy-3-{(E)-2-[1-(4-methoxybenzyl)-1H-1,2,3,4-tetrazol-5-yl]-1-ethenyl}-4-oxo-4H-pyrido[1,2-a]pyrimidine-8-carboxamide (400 mg, 0.716 mmol) dissolved in a mixture of dimethylformamide (50 ml) and acetonitrile (25 ml) was added with diphenyl chlorophosphate (384 mg, 1.43 mmol) and diisopropylethylamine (489 µl, 2.86 mmol) with ice cooling and stirred for 40 minutes. This solution was added with 4-hydroxypiperidine (108 mg, 1.07 mmol) and stirred at 80-90°C for 2 hours. The reaction solution was cooled to room temperature and concentrated under reduced pressure, and the reside was purified by thin layer chromatography (silica gel, methylene chloride:methanol = 9:1) to obtain yellow solid.

This solid was dissolved in tetrahydrofuran (10 ml), added with pyridine (39.7 µ l, 0.493 mmol) and added with 2-chloroethyl isocyanate (510 µl, 5.94 mmol) as 7 divided portions at 80°C with stirring. After stirred for 5 days, the solution was added with saturated aqueous sodium hydrogencarbonate and extracted with chloroform three times. The combined organic layer was washed with saturated brine and dried over anhydrous sodium sulfate. The solvent was evaporated under reduced pressure, and the residue was purified by thin layer chromatography (silica gel, methylene chloride:methanol = 9:1) to obtain yellow solid.

This solid was dissolved in dimethylformamide (20 ml), added with sodium iodide (30 mg) and a solution of dimethylamine in tetrahydrofuran (2.0 M, 20 ml) and stirred at 60°C for 3 hours. This solution was further added with sodium iodide (30 mg) and a solution of dimethylamine in tetrahydrofuran (2.0 M, 20 ml), stirred for 5 hours and concentrated under reduced pressure. The resulting residue was purified by thin layer chromatography (silica gel, methylene chloride:methanol = 9:1) to obtain the title compound (184.2 mg) as yellow solid.
LRMS-ESI; m/z: 756 (MH⁺)

### (B) {2-[({[1-(8-({[4-(t-Butyl)-1,3-thiazol-2-yl]amino}carbonyl)-3-{(E)-2-[1-(4-methoxybenzyl)-1H-1,2,3,4-tetrazol-5-yl]-1-ethenyl}-4-oxo-4H-pyrido[1,2-a]-pyrimidin-2-yl)-4-piperidyl]oxy}carbonyl)amino]ethyl}(trimethyl)ammonium

1-(8-({[4-(t-Butyl)-1, 3-thiazol-2-yl] amino}carbonyl)-3-{(E)-2-[1-(4-methoxybenzyl)-1H-1,2,3,4-tetrazol-5-yl]-1-ethenyl}-4-oxo-4H-pyrido[1,2-a]pyrimidin-2-yl)-4-piperidyl-N-[2-(dimethylamino)ethyl]carbamate (86.2 mg, 0.114 mmol) was dissolved in dimethylformamide (10 ml), added with methyl iodide (328 µl, 5.28 mmol) and left stand overnight at -5°C. The reaction mixture was further added with methyl iodide (656 µl, 10.6 mmol) and left stand overnight at -5°C. Then the solution was concentrated under reduced pressure, and the residue was purified by thin layer chromatography (silica gel, lower layer of chloroform:methanol:water = 7:3:1) to obtain the title compound (69.3 mg, 79%) as orange solid.
LRMS-ESI; m/z: 771 (MH⁺)

### (C) [2-({[(1-{8-({[4-(t-Butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}-4-piperidyl)oxy]-carbonyl}amino)ethyl] (trimethyl)ammonium

{2-[({[1-(8-({[4-(t-Butyl)-1,3-thiazol-2-yl]amino}carbonyl)-3-{(E)-2-[1-(4-methoxybenzyl)-1H-1,2,3,4-tetrazol-5-yl]-1-ethenyl}-4-oxo-4H-pyrido[1,2-a]pyrimidin-2-yl)-4-piperidyl]oxy}carbonyl)amino]ethyl}(trimethyl)ammonium was dissolved in trifluoroacetic acid (20 ml) and stirred at 60°C for 4 hours. The reaction mixture was cooled to room temperature and concentrated under reduced pressure, and the residue was purified by thin layer chromatography (silica gel, chloroform: methanol: water = 8:3:0.5). The resulting solid was washed with diethyl ether to obtain the title compound (44.8 mg, 78%) as orange powder.
m.p.: 210-215°C
¹H-NMR (CD₃OD) δ : 1.35 (9H, s), 1.86 (2H, brs), 2.06 (2H, brs), 3.19 (9H, s), 3.46-3.63 (6H, m), 3.88 (2H, brs), 4.90 (1H, brs), 6.74 (1H, s), 7.39 (1H, d, J=15.6Hz), 7.58 (1H, d, J=7.08Hz), 7.89-8.01 (2H, m), 8.98 (1H, d, J=7.33Hz)
LRMS-ESI; m/z: 650 (MH⁺)
IR (ATR) cm⁻¹: 1676, 1512, 1425, 1203, 1176, 1124, 841, 800, 723, 619, 517

| Anal. (for C₃₀H₁₀N₁₀O₄S•0.50Et₂O•3.25H₂O) | | | |
|---|---|---|---|
| Calcd. | C, 51.56; | H, 6.83; | N, 20.67 |
| Found | C, 51.89; | H, 6.48; | N, 20.33 |

### Example 209: (2-Amino-2-oxoethyl)[{[(1-{8-({[4-(t-butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]-pyrimidin-2-yl}-4-piperidyl)oxy]carbonyl}amino]ethyl]dimethylammonium

### (A) (2-Amino-2-oxoethyl)(2-{[({1-[3-[(E)-2-{1-(4-methoxybenzyl)-1H-1,2,3,4-tetrazol-5-yl}-1-ethenyl]-8-({[4-(t-butyl)-1,3-thiazol-2-yl]amino}carbonyl)-4-oxo-4H-pyrido[1,2-a]pyrimidin-2-yl]-4-piperidyl}oxy)carbonyl]amino}ethyl)dimethylammonium

1-(8-({[4-(t-Butyl)-1,3-thiazol-2-yl]amino}carbonyl)-3-{(E)-2-[1-(4-methoxybenzyl)-1H-1,2,3,4-tetrazol-5-yl]-1-ethenyl}-4-oxo-4H-pyrido[1,2-a]pyrimidin-2-yl)-4-piperidyl-N-[2-(dimethylamino)ethyl]carbamate (97.0 mg, 0.128 mmol) and iodoacetamide (35.5 mg, 0.192 mmol) were dissolved in dimethylformamide (15 ml) and stirred overnight at room temperature. The reaction mixture was concentrated under reduced pressure, and the residue was purified by thin layer chromatography (silica gel, chloroform:methanol:water = 8:3:0.5). The resulting solid was washed with diethyl ether to obtain the title compound (78.1 mg, 75%) as yellow solid.
LRMS-ESI; m/z: 813 (MH⁺)

### (B) (2-Amino-2-oxoethyl)[{[(1-{8-({[4-(t-butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}-4-piperidyl)oxy]carbonyl}amino]ethyl]dimethylammonium

(2-Amino-2-oxoethyl)(2-{[(1-[3-[(E)-2-{1-(4-methoxybenzy)-1H-1,2,3,4-tetrazol-5-yl}-1-ethenyl]-8-({[4-(t-butyl)-1,3-thiazol-2-yl]amino}carbonyl)-4-oxo-4H-pyrido[1,2-a]pyrimidin-2-yl]-4-piperidyl}oxy)carbonyl]amino}ethyl)-dimethylammonium (78.1 mg, 0.0960 mmol) was dissolved in trifluoroacetic acid (20 ml), added with anisole (5.0 ml) and stirred at 60°C for 5 hours. The reaction mixture was cooled to room temperature and concentrated under reduced pressure, and the residue was purified by thin layer chromatography (silica gel, chloroform:methanol:water = 8:3:0.5) to obtain the title compound (78.1 mg) as orange solid.
m.p.: 213-217°C
¹H-NMR (CD₃OD) δ : 1.34 (9H, s), 1.88 (2H, brs), 2.08 (2H, brs), 3.30-3.34 (8H, m), 3.51 (2H, brs), 3.60 (2H, brs), 3.74 (2H, brs), 3.86 (2H, brs), 4.87 (1H, brs), 6.56 (1H, s), 7.43 (1H, d, J=16.4Hz), 7.87-7.94 (2H, m), 8.10 (1H, s), 8.92 (1H, d, J=7.10Hz)
LRMS-FAB; m/z: 693 (MH⁺)

| HRMS-FAB (C₃₁H₄₁O₅N₁₂S); m/z: | |
|---|---|
| Calcd. | 693.3044 |
| Found | 693.3020 |

IR (ATR) cm⁻¹: 1693, 1655, 1633, 1510, 1425, 1377, 1236, 1101, 1026

### Example 210: (E)-3-[8-({[4-(t-Butyl)-1,3-thiazol-2-yl]amino}carbonyl)-2-(4-hydroxypiperidino)-4-oxo-4H-pyrido[1,2-a]pyrimidin-3-yl]-2-propenoic acid

### (A) (E)-3-[8-({[4-(t-Butyl)-1,3-thiazol-2-yl]amino}carbonyl)-2-(4-hydroxypiperidino)-4-oxo-4H-pyrido[1,2-a]pyrimidin-3-yl]-2-propenoic acid t-butyl ester

A solution of (E)-3-[8-({[4-(t-butyl)-1,3-thiazol-2-yl]amino}carbonyl)-2-hydroxy-4-oxo-4H-pyrido[1,2-a]pyrimidin-3-yl]2-propenoic acid t-butyl ester (500 mg, 1.06 mmol) in a mixture of dimethylformamide (40 ml) and acetonitrile (20 ml) was added with diphenyl chlorophosphate (570 mg, 2.12 mmol) and diisopropylethylamine (725 µl, 4.24 mmol) with ice cooling and stirred for 50 minutes. This solution was added with 4-hydroxypiperidine (322 mg, 3.18 mmol) at 80-90°C, stirred for 90 minutes, added with diisopropylethylamine (725 µl, 4.24 mmol) and further stirred for 2 hours. The reaction solution was cooled to room temperature and concentrated under reduced pressure, and the residue was purified by column chromatography (silica gel, chloroform:ethyl acetate = 3:1-1:1) to obtain the title compound as yellow powder.
LRMS-ESI: 554 (MH⁺)

### (B) (E)-3-[8-({[4-(t-Butyl)-1,3-thiazol-2-yl]amino}carbonyl)-2-(4-hydroxypiperidino)-4-oxo-4H-pyrido[1,2-a]pyrimidin-3-yl]-2-propenoic acid

(E)-3-[8-({[4-(t-Butyl)-1,3-thiazol-2-yl]amino}carbonyl)-2-(4-hydroxypiperidino )-4-oxo-4H-pyrido[1,2-a]pyrimidin-3-yl]-2-propenoic acid t-butyl ester (80.0 mg, 0.144 mmol) was dissolved in a solution of hydrochloric acid in dioxane (4 N, 30 ml) and stirred at room temperature for 5 hours. The reaction mixture was concentrated under reduced pressure, and the residue was purified by thin layer chromatography (silica gel, lower layer of methylene chloride:methanol:water = 7:3:1) to obtain the title compound (26.3 mg) as yellow powder.
m.p.: 215-218°C
¹H-NMR (DMSO-d₆) δ: 1.30 (9H, s), 1.45-1.55 (2H, m), 1.86-1.89 (2H, m), 3.32-3.50 (2H, m), 3.76-3.77 (1H, m), 3.84-3.68 (2H, m), 4.80 (1H, d, J=3.91Hz), 6.84 (1H, s), 6.91 (1H, d, J=15.4Hz), 7.43 (1H, d, J=15.6Hz), 7.57-7.60 (1H, m), 8.16 (1H, d, J=1.22Hz), 8.88 (1H, d, J=7.32Hz)
LRMS-FAB; m/z: 498 (MH⁺)

| HRMS-FAB (C₂₄H₂₈O₅S); m/z: | |
|---|---|
| Calcd. | 498.1811 |
| Found | 498.1835 |

IR (ATR) cm⁻¹: 1666, 1520, 1441, 1365, 1292, 1217, 1196, 1103, 1068, 739, 688, 476

| Anal. (for C₂₄H₂₇N₅O₅S · 0.75H₂O) | | | |
|---|---|---|---|
| Calcd. | C, 56.40; | H, 5.62; | N, 13.70 |
| Found | C, 56.86; | H, 5.48; | N, 13.23 |

### Example 211: (E)-3-[2-{4-[(Aminocarbonyl)oxy]piperidino}-8-({4-(t-butyl)-1,3-thiazol-2-yl}amino)carbonyl]-4-oxo-4H-pyrido[1,2-a]pyrimidin-3-yl]-2-propenoic acid

### (A) (E)-3-[2-{4-[(Aminocarbonyl)oxy]piperidino}-8-({4-(t-butyl)-1,3-thiazol-2-yl}amino)carbonyl]-4-oxo-4H-pyrido[1,2-a]pyrimidin-3-yl]-2-propenoic acid t-butyl ester

(E)-3-[8-({[4-(t-Butyl)-1,3-thiazol-2-yl)amino}carbonyl)-2-(4-hydroxypiperidino)-4-oxo-4H-pyrido[1,2-a]pyrimidin-3-yl)-2-propenoic acid t-butyl ester (150 mg, 0.271 mmol) was suspended in ethyl acetate, added with trichloroacetyl isocyanate (161 µl, 1.36 mmol) and stirred at room temperature for 2 hours and 30 minutes. The reaction mixture was added with a mixed solution of chloroform and methanol (10:1, 11 ml) to terminate the reaction and concentrated under reduced pressure to obtain orange amorphous solid. This solid was dissolved in a mixed solvent of tetrahydrofuran (40 ml) and water (20 ml), added with sodium formate (92.5 mg) and stirred at room temperature for 2 hours. The reaction mixture was concentrated under reduced pressure, and the residue was purified by thin layer chromatography (silica gel, chloroform:methanol = 9:1) to obtain the title compound (145 mg, 90%) as yellow amorphous solid.
LRMS-ESI; m/z: 597 (MH⁺)

### (B) (E)-3-[2-{4-[(Aminocarbonyl)oxy]piperidino}-8-({4-(t-butyl)-1,3-thiazol-2-yl}amino)carbonyl]-4-oxo-4H-pyrido[1,2-a]pyrimidin-3-yl]-2-propenoic acid

(E)-3-[2-{4-[(Aminocarbonyl)oxy]piperidino}-8-({4-(t-butyl)-1,3-thiazol-2-yl}amino)carbonyl]-4-oxo-4H-pyrido[1,2-a]pyrimidin-3-yl]-2-propenoic acid t-butyl ester (145 mg, 0.243 mmol) was dissolved in a solution of hydrochloric acid in dioxane (4 N, 50 ml) and stirred at room temperature for 4 hours. The reaction mixture was concentrated under reduced pressure, and the residue was purified by thin layer chromatography (silica gel, lower layer of methylene chloride:methanol:water = 7:3:1) to obtain the title compound (60.6 mg) as yellow powder.
m.p.: 227-230°C
1H-NMR (DMSO-d₆) δ : 1.30 (9H, s), 1.65-1.67 (2H, m), 1.98 (2H, brs), 3.34-3.43 (2H, m), 3.83-3.86 (2H, m), 4.77 (1H, m), 6.50 (2H, brs), 6.85 (1H, s), 6.92 (1H, d, J=15.4Hz), 7.44 (1H, d, J=15.6Hz), 7.61 (1H, d, J=7.08Hz), 8.19 (1H, s), 8.90 (1H, d, J=7.81Hz) LRMS-FAB; m/z: 541 (MH⁺)
IR (ATR) cm⁻¹: 1741, 1664, 1601, 1562, 1522, 1442, 1308, 1221, 1036, 858, 742, 681, 633

| Anal. (for C₂₅H₂₈N₆S•0.5H₂O) | | | | |
|---|---|---|---|---|
| Calcd. | C, 54.63; | H, 5.32; | N, 15.29, | S, 5.83 |
| Found | C, 54.71; | H, 5.26; | N, 15.14, | S, 5.88 |

### Example 212: (E)-3-{8-({[4-(t-Butyl)-1,3-thiazol-2-yl]amino}carbonyl)-2-[4-({[(2-hydroxyethyl)amino]carbonyl}oxy)piperidino]-4-oxo-4H-pyrido[1,2-a]pyrimidin-3-yl}-2-propenoic acid

### (A) (E)-3-[8-({[4-(t-Butyl)-1,3-thiazol-2-yl]amino}carbonyl)-2-(4-hydroxypiperidino)-4-oxo-4H-pyrido[1,2-a]pyrimidin-3-yl]-2-propenoic acid t-butyl ester

A solution of (E)-3-[8-({[4-(t-butyl)-1,3-thiazol-2-yl]amino}carbonyl)-2-hydroxy-4-oxo-4H-pyrido[1,2-a]pyrimidin-3-yl]-2-propenoic acid t-butyl ester (500 mg, 1.06 mmol) in a mixture of dimethylformamide (40 ml) and acetonitrile (20 ml) was added with diphenyl chlorophosphate (570 mg, 2.12 mmol) and diisopropylethylamine (548 mg, 4.24 mmol) with ice cooling and stirred for 50 minutes. This solution was added with 4-hydroxypiperidine (322 mg, 3.18 mmol) and diisopropylethylamine (548 mg, 4.24 mmol) and stirred at 80-90°C for 2 hours. The reaction solution was concentrated under reduced pressure, and the resulting residue was purified by column chromatography (silica gel, chloroform:ethyl acetate = 3:1-1:1) to obtain the title compound (380 mg) as yellow powder.
LRMS-ESI; m/z: 554

### (B) (E)-3-{8-({[4-(t-Butyl)-1,3-thiazol-2-yl]amino}carbonyl)-2-[4-({[(2-hydroxyethyl)-amino]carbonyl}oxy)piperidino]-4-oxo-4H-pyrido[1,2-a]pyrimidin-3-yl}-2-propenoic acid t-butyl ester

A solution of (E)-3-[8-({[4-(t-butyl)-1,3-thiazol-2-yl]amino}carbonyl)-2-(4-hydroxypiperidino)-4-oxo-4H-pyrido[1,2-a]pyrimidin-3-yl]-2-propenoic acid t-butyl ester (148 mg, 0.267 mmol) dissolved in a mixture of methylene chloride (30 ml) and tetrahydrofuran (10 ml) was added with a solution of N,N'-carbonyldiimidazole (261 mg, 1.60 mmol) in methylene chloride (20 ml) and stirred overnight at room temperature. The reaction mixture was washed with water and saturated brine and dried over anhydrous sodium sulfate, and the solvent was evaporated under reduced pressure.

The obtained yellow amorphous solid was dissolved in tetrahydrofuran (100 ml), added with triethylamine (81.1 mg, 0.801 mmol) and 2-aminoethanol (32.6 mg, 0.534 mmol) and stirred overnight at room temperature. The reaction mixture was concentrated under reduced pressure, and the resulting residue was dissolved in chloroform, successively washed with 1 N aqueous hydrochloric acid, saturated aqueous sodium hydrogencarbonate and saturated brine and dried over anhydrous sodium sulfate. The solvent was evaporated under reduced pressure, and the resulting residue was purified by thin layer chromatography (silica gel, methylene chloride:methanol = 9:1) to obtain the title compound (130 mg) as yellow amorphous solid.
LRMS-ESI; m/z: 641

### (C) (E)-3-{8-({[4-(t-Butyl)-1,3-thiazol-2-yl]amino}carbonyl)-2-[4-({[(2-hydroxyethyl)-amino]carbonyl}oxy)piperidino]-4-oxo-4H-pyrido[1,2-a]pyrimidin-3-yl}-2-propenoic acid

(E)-3-{8-({[4-(t-Butyl)-1,3-thiazol-2-yl]amino}carbonyl)-2-[4-({[(2-hydroxyethyl) amino]carbonyl}oxy)piperidino]-4-oxo-4H-pyrido[1,2-a]pyrimidin-3-yl}-2-propenoic acid t-butyl ester (130 mg, 0.203 mmol) was dissolved in a solution of hydrochloric acid in dioxane (4 N, 30 ml) and stirred overnight at room temperature. The reaction mixture was concentrated under reduced pressure, and the resulting residue was purified by thin layer chromatography (silica gel, lower layer of chloroform:methanol:water = 7:3:1). The resulting solid was washed with methanol to obtain the title compound (48.6 mg, 41%) as yellow powder.
m.p.: 231-234°C
¹H-NMR (DMSO-d₆) δ : 1.30 (9H, s), 1.65-1.67 (2H, m), 1.97 (2H, brs), 3.04 (2H, dd, J=6.10, 12.2Hz), 3.32-3.44 (6H, m), 3.81 (2H, brs), 4.61 (1H, t, J=5.49Hz), 4.81 (1H, brs), 6.86 (1H, s), 6.92 (1H, d, J=15.6Hz), 7.06 (1H, t, J=5.86Hz), 7.44 (1H, d, J=15.6Hz), 7.61 (1H, dd, J=1.83, 7.45Hz), 8.19 (1H, s), 8.90 (1H, d, J=7.33Hz)
LRMS-ESI; m/z: 585 (MH⁺)
IR (ATR) cm-¹: 2962, 1662, 1520, 1427, 1311, 1221, 1138, 1066, 1014, 860, 733,471

| Anal. (for C₂₇H₃₂N₆O₇S•0.75H₂O) | | | | |
|---|---|---|---|---|
| Calcd. | C, 54.22; | H, 5.64; | N, 14.05; | S, 5.36 |
| Found | C, 54.35; | H, 5.42; | N, 13.99; | S, 5.34 |

### Example 213: (E)-3-[2-{(3R)-3-[(Aminosulfonyl)oxy]hexahydro-1-pyridinyl}-8-({[4-(t-butyl)-1,3-thiazol-2-yl]amino}carbonyl)-4-oxo-4H-pyrido[1,2-a]pyrimidin-3-yl]-2-propenoic acid

### (A) (E)-3-[2-{(3R)-3-[(Aminosulfonyl)oxy]hexahydro-1-pyridinyl}-8-({[4-(t-butyl)-1,3-thiazol-2-yl]amino}carbonyl)-4-oxo-4H-pyrido[1,2-a]pyrimidin-3-yl]-2-propenoic acid t-butyl ester

A solution of (E)-3-{8-({[4-(t-butyl)-1,3-thiazol-2-yl]amino}carbonyl)-2-[(3R)-3-hydroxyhexahydro-1-pyridinyl]-4-oxo-4H-pyrido[1,2-a]pyrimidin-3-yl}-2-propenoic acid t-butyl ester (178 mg, 0.321 mmol), triethylamine (260 mg, 2.57 mmol) and 4-dimethylaminopyridine (19.7 mg, 0.161 mmol) in methylene chloride (50 ml) was added with a solution of sulfamoyl chloride in methylene chloride (1.18 mmol/ml, 1.08 ml) with ice cooling and stirred at room temperature for 24 hours. The reaction solution was washed with water, and the aqueous layer was extracted with. chloroform three times. The combined organic layer was concentrated under reduced pressure. The resulting residue was purified by thin layer chromatography (n-hexane:ethyl acetate = 1:3) to obtain the title compound (161 mg, 79%) as yellow solid.
¹H-NMR (CDCl₃) δ: 1.36 (9H, s), 1.45 (9H, s), 1.63 (2H, brs), 2.00 (2H, brs), 3.49 (2H, brs), 2.72-3.75 (2H, m), 4.81 (1H, brs), 6.54 (1H, s), 7.00-7.04 (1H, m), 7.36 (1H, brs), 7.54 (1H, d, J=7.57Hz), 7.90 (1H, d, J=1.22Hz), 9.02 (1H, d, J=7.33Hz)
LRMS-ESI; m/z: 633 (MH⁺)

### (B) (E)-3-[2-{(3R)-3-[(Aminosulfonyl)oxy]hexahydro-1-pyridinyl}-8-({[4-(t-butyl)-1,3-thiazol-2-yl]amino}carbonyl)-4-oxo-4H-pyrido[1,2-a]pyrimidin-3-yl]-2-propenoic acid

(E)-3-[2-{(3R)-3-[(Aminosulfonyl)oxy]hexahydro-1-pyridinyl}-8-({[4-(t-butyl)-1,3-thiazol-2-yl]amino}carbonyl)-4-oxo-4H-pyrido[1,2-a]pyrimidin-3-yl]-2-propenoic acid t-butyl ester (733 mg, 1.16 mmol) was dissolved in a solution of hydrochloric acid in dioxane (4 N, 100 ml) and stirred overnight at room temperature. The reaction mixture was concentrated under reduced pressure, and the residue was purified by column chromatography (silica gel, methylene chloride:methanol = 9:1, chloroform:methanol:water = 8:3:0.5). The obtained yellow solid was washed with a mixed solvent of diethyl ether, n-hexane and methanol to obtain the title compound (369 mg, 55%) as yellow powder.
m.p.: 222°C (decomp.)
¹H-NMR (CD₃OD) δ : 1.35 (9H, s), 1.76-1.77 (1H, m), 1.95-2.03 (2H, m), 2.13-2.17 (1H, m), 3.61 (2H, t, J=5.27Hz), 3.65-3.70 (1H, m), 4.04-4.08 (1H, m), 4.71-4.79 (1H, m), 6.74 (1H, s), 7.07 (1H, d, J=15.7Hz), 7.59-7.63 (2H, m), 8.02-8.08 (1H, m), 8.95-8.97 (1H, m) IR (ATR) cm⁻¹: 3332,3103, 2960, 1668, 1512, 1441, 1365, 1271, 1173, 1099, 930, 901, 864,829,741,687,552
LRMS-FAB; m/z: 577 (MH⁺)

| Anal. (for C₂₄H₂₈N₆O₇S₂•1.75H₂O) | | | |
|---|---|---|---|
| Calcd. | C, 47.40; | H, 5.22; | N, 13.82 |
| Found | C, 47.21; | H, 5.04; | N, 13.47 |

### Example 231: (E)-3-[8-({[4-(tert-Butyl)-1,3-thiazol-2-yl]amino}carbonyl)-2-(3,4-dihydroxypiperidino)-4-oxo-4H-pyrido[1,2-a]pyrimidin-3-yl}-2-propenoic acid

### (A) N⁸-[4-(tert-Butyl)-1,3-thiazol-2-yl]-3-formyl-2-hydroxy-4-oxo-4H-pyrido[1,2-a]-pyrimidine-8-carboxyamide

Dimethylformamide (5 ml) was added dropwise with phosphorus oxychloride (54 µ l, 0.58 mmol, 2 eq) with ice cooling and stirred at room temperature for 20 minutes. This mixture was added with N⁸-[4-(tert-butyl)-1,3-thiazol-2-yl]-2,4-dioxo-3,4-dihydro-2H-pyrido[1,2-a]pyrimidine-8-carboxamide (100 mg, 0.29 mmol) at 0°C, stirred at room temperature for 30 minutes and then stirred at 80°C for 20 minutes. After completion of the reaction, the reaction solution returned to room temperature was added with water and saturated aqueous sodium hydrogencarbonate (pH 5-6) and extracted with chloroform. The collected organic layer was dried over magnesium sulfate, and the solvent was concentrated under reduced pressure. The obtained oily title compound was used in the following reaction without being purified.
¹H-NMR (CD₃OD/CDCl₃) δ : 1.31 (9H, s), 6.63 (1H, s), 8.03 (2H, m), 9.16 (1H, brd), 10.16 (1H, brd).
EI-MS; m/s: 373 (M⁺+1).

### (B) tert-Butyl (E)-3-[8-({[4-(tert-butyl)-1,3-thiazol-2-yl]amino}carbonyl)-2-hydroxy-4-oxo-4H-pyrido[1,2-a]pyrimidin-3-yl}-2-propenoate

N⁸-[4-(tert-Butyl)-1,3-thiazol-2-yl]-3-formyl-2-hydroxy-4-oxo-4H-pyrido[1,2-a]-pyrimidine-8-carboxamide (108 mg, 0.29 mmol) was dissolved in tetrahydrofuran (5 ml), added with (tert-butoxycarbonylmethylene)triphenylphosphorane (165 mg, 0.44 mmol) at room temperature and stirred at the same temperature for 2 hours. After completion of the reaction, the solvent was concentrated under reduced pressure, and the obtained oily substance was purified by thin layer silica gel column chromatography (lower layer of chloroform:methanol:water = 8:6:1) to obtain the title compound (63 mg, 46% for the two steps) as a yellow substance.
¹H-NMR (CD₃OD/CDCl₃) δ : 1.38 (9H, s), 1.53 (9H, s), 6.63 (1H, s), 7.05 (1H, d, J=15.87Hz), 8.00 (1H, d, J=15.87Hz), 8.01 (1H, d, J=7.08Hz), 8.06 (1H, s), 9.18 (1H, d, J=7.08Hz):
EI-MS; m/s: 471 (M⁺+1).

### (C) tert-Butyl (E)-3-[8-({[4-(tert-butyl)-1,3-thiazol-2-yl]amino}carbonyl)-2-(3,4-dihydroxypiperidino)-4-oxo-4H-pyrido[1,2-a]pyrimidin-3-yl]-2-propenoate

tert-Butyl (E)-3-[8-({[4-(tert-butyl)-1,3-thiazol-2-yl]amino}carbonyl)-2-hydroxy-4-oxo-4H-pyrido[1,2-a]pyrimidin-3-yl]-2-propenoate (63 mg, 0.13 mmol) was added with 1 ml each of dimethylformamide and acetonitrile, cooled to -10°C with ice and then added dropwise with diphenylphosphonyl chloride (56 µl, 0.26 mmol, 2 eq) and diisopropylethylamine (93 µl, 0.52 mmol, 4 eq). The mixture was stirred at the same temperature for 10 minutes, added with 3,4-dihydroxypiperidine hydrochloride (31 mg, 0.20 mmol, 1.5 eq) and diisopropylethylamine (50 µl, 0.26 mmol, 2 eq), stirred for 15 minutes and further stirred at room temperature for 20 minutes. Then the mixture was heated to 90°C, stirred for 1 hour and 30 minutes, added with 3,4-dihydroxypiperidine hydrochloride (20 mg) and diisopropylethylamine (25 µl) and further stirred for 40 minutes. After completion of the reaction, the reaction solution was added with water and extracted with chloroform. The collected organic layer was dried over magnesium sulfate, and then the solvent was concentrated under reduced pressure. The obtained oily substance was purified by thin layer silica gel column chromatography (chloroform:methanol = 30:1) to obtain the yellow title compound (35 mg, 45%).
¹H-NMR (CDCl₃) δ :1.35 (9H, s), 1.52 (9H, s), 1.69 (1H, m), 1.88 (1H, m), 3.22 (1H, m), 3.45 (3H, m), 3.82 (1H, m), 3.90 (1H, m), 6.58 (1H, s), 7.06 (1H, d, J=15.87Hz), 7.43 (1H, d, J=15.87Hz), 7.53 (1H, dd, J=7.32, 1.47Hz), 8.03 (1H, s), 8.95 (1H, d, J=7.32Hz).
EI-MS; m/s: 570 (M⁺+1).

### (D) (E)-3-(8-({(4-(tert-Butyl)-1,3-thiazol-2-yl]amino)carbonyl)-2-(3,4-dihydroypiperidino)-4-oxo-4H-pyrido(1,2-a]pyrimidin-3-yl]-2-propenoic acid

tert-Butyl (E)-3-[8-({[4-(tert-butyl)-1,3-thiazol-2-yl]amino}carbonyl)-2-(3,4-dihydroxypiperidino)-4-oxo-4H-pyrido(1,2-a]pyrimidin-3-yl)-2-propenoate (35 mg, 0.06 mmol) was added with 4 N solution of hydrochloric acid in dioxane (3 ml) at room temperature and stirred at the same temperature for 4 hours. After completion of the reaction, the solvent was concentrated under reduced pressure, and the obtained oily substance was purified by thin layer silica gel column chromatography (chloroform:methanol = 10:1, developed 4 times) to obtain the title compound (18 mg, 26% for the two steps) as yellow crystals.
¹H-NMR (CD₃OD/CDCl₃) δ: 1.36 (9H, s), 1.87 (1H, m), 1.99 (1H, m), 3.60 (1H, m), 3.77 (3H, m), 3.88 (1H, m), 3.96 (1H, m), 6.64 (1H, s), 7.05 (1H, d, J=15.68Hz), 7.56 (1H, d, J=7.35Hz), 7.62 (1H, d, J=15.68Hz), 8.06 (1H, s), 8.95 (1H, d, J=7.35Hz).
m.p.: 182-189°C
ES-MS; m/s: 514 (M⁺+1).

### Example 232: (E)-3-(8-({[4-(tert-Butyl)-1,3-thiazol-2-yl]amino}carbonyl)-2-{3,4-di[(aminocarbonyl)oxy]piperidino}-4-oxo-4H-pyrido[1,2-a]pyrimidin-3-yl}-2-propenoic acid

### (A) N⁸-[4-(tert Butyl)-1,3-thiazol-2-yl]-3-formyl-2-hydroxy-4-oxo-4H-pyrido[1,2-a]-pyrimidine-8-carboxyamide

Dimethylamide (15 ml) was added dropwise with phosphorus oxychloride (97 µ l, 1.04 mmol, 1.2 eq) with ice cooling and stirred at room temperature for 30 minutes. The reaction solution was cooled with ice again, then added with N⁸-[4-(tert-butyl)-1,3-thiazol-2-yl]-2,4-dioxo-3,4-dihydro-2H-pyrido[1,2-a]pyrimidine-8-carboxamide (300 mg, 0.87mmol) at 80-90°C and stirred for 45 minutes. The reaction solution was returned to room temperature, then neutralized by adding saturated aqueous sodium hydrogencarbonate and extracted with chloroform. The resulting organic layer was dried over magnesium sulfate, and the solvent was further concentrated under reduced pressure to obtain a crude product (360 mg) as a yellow oily substance.
¹H-NMR (CD₃OD/CDCl₃) δ : 1.31 (9H, s), 6.63 (1H, s), 8.03 (2H, m), 9.16 (1H, brd), 10.16 (1H, brd).
EI-MS; m/s: 373 (M⁺+1).

### (B) tert-Butyl (E)-3-[8-({[4-(tert-butyl)-1,3-thiazol-2-yl]amino}carbonyl)-2-hydroxy-4-oxo-4H-pyrido[1,2-a]pyrimidin-3-yl}-2-propenoate

N⁸-[4-(tert-Butyl)-1,3-thiazol-2-yl]-3-formyl-2-hydroxy-4-oxo-4H-pyrido[1,2-a]pyrimidine-8-carboxamide (324 mg, 0.87 mmol) was dissolved in tetrahydrofuran (20 ml) and dimethylformamide (2 ml), added with (tert-butoxycarbonylmethylene)-triphenylphosphorane (490 mg, 1.31 mmol, 1.5 eq) and stirred at room temprerature for 3 hours. Then the mixture was further added with 250 mg of (tert-butoxycarbonylmethylene)triphenylphosphorane, and 1 hour after that, further added with 300 mg of (tert-butoxycarbonylmethylene)triphenylphosphorane. After the reaction mixture was stirred for 10 hours, the solvent was concentrated under reduced pressure, and the obtained oily substance was purified by silica gel column chromatography (chloroform:methanol = 100:1 → 20:1) to obtain the title compound (368 mg) as a mixture with triphenylphosphine oxide.
¹H-NMR (CD₃OD/CDCl₃) δ : 1.38 (9H, s), 1.53 (9H, s), 6.63 (1H, s), 7.05 (1H, d, J=15.87Hz), 8.00 (1H, d, J=15.87Hz), 8.01 (1H, d, J=7.08Hz), 8.06 (1H, s), 9.18 (1H, d, J=7.08Hz).
EI-MS; m/s: 471 (M⁺+1).

### (C) tert-Butyl (E)-3-[8-({[4-(tert-butyl)-1,3-thiazol-2-yl]amino}carbonyl)-2-(3,4-dihydroxypiperidino)-4-oxo-4H-pyrido[1,2-a]pyrimidin-3-yl]-2-propenoate

tert-Butyl (E)-3-[8-({(4-(tert-butyl)-1,3-thiazol-2-yl]amino}carbonyl)-2-hydroxy-4-oxo-4H-pyrido[1,2-a]pyrimidin-3-yl]-2-propenoate (367 mg, 0.78 mmol) was dissolved in dimethylformamide and acetonitrile (8 ml each), cooled to -10°C with ice and added dropwise with phosphonyl chloride (0.3 ml, 1.56 mmol, 2 eq) and diisopropylamine (0.5 ml, 3.12 mmol, 4 eq). The reaction mixture was stirred at the same temperature for 10 minutes, added with 3,4-dihydroxypiperidine hydrochloride (180 mg) and diisopropylamine (0.25 ml), stirred at room temperature for 5 minutes and then stirred at 90°C for 1 hour. Then the reaction mixture was added with 3,4-dihydroxypiperidine hydrochloride (80 mg) and diisopropylamine (0.25 ml) and further stirred at 90°C for 1 hour. The reaction solution was returned to room temperature, added with water, extracted with chloroform, and the organic solvent was dried over magnesium sulfate. The solvent was concentrated under reduced pressure, and the resulting crude product was purified by silica gel column chromatography (chloroform:methanol = 100:1 → 20:1) to obtain the title compound (190 mg) with contained impurities.
¹H-NMR (CDCl₃) δ: 1.35 (9H, s), 1.52 (9H, s), 1.69 (1H, m), 1.88 (1H, m), 3.45 (2H, m), 3.82 (1H, m), 3.90 (1H, m), 4.11 (1H, s), 4.18 (1H, d, J=13.92Hz), 6.58 (1H, s), 7.06 (1H, d, J=15.87Hz), 7.43 (1H, d, J=15.87Hz), 7.53 (1H, dd, J=7.32, 1.47Hz), 8.03 (1H, s), 8.95 (1H, d, J=7.32Hz).
EI-MS; m/s: 570 (M⁺+1).

### (D) tert-Butyl (E)-3-(8-({[4-(tert-butyl)-1,3-thiazol-2-yl]amino}carbonyl)-2-{3,4-di[(aminocarbonyl)oxy]piperidino}-4-oxo-4H-pyrido[1,2-a]pyrimidin-3-yl)-2-propenoate

tert-Butyl (E)-3-[8-({[4-(tert-butyl)-1,3-thiazol-2-yl]amino}carbonyl)-2-(3,4-dihydroxypiperidino)-4-oxo-4H-pyrido(1,2-a]pyrimidin-3-yl)-2-propenoate (213 mg, 0.37 mmol) dissolved in ethyl acetate (5 ml) was added with trichloroacetyl isocyanate (230 µl, 1.85 mmol, 5 eq) with ice cooling and stirred at the same temperature for 30 minutes. Then the mixture was further added with 0.5 ml of the isocyanate and after 30 minutes, further added with 0.5 ml of the same. After the reaction mixture was stirred for 30 minutes, the solvent was concentrated under reduced pressure, and the obtained oily substance was added with methanol (5 ml), water (1 ml) and sodium formate (330 mg) and stirred at room temperature. After 40 minutes, the reaction mixture was further added with sodium formate (500 mg), and after 20 minutes, further added with sodium formate (500 mg). After 30 minutes, the reaction mixture was further added with sodium formate (500 mg), water (1 ml) and methanol (0.5 ml) and stirred for 30 minutes. Then the reaction solution was added with water and extracted with chloroform. The collected organic layer was dried over magnesium sulfate, and the solvent was concentrated under reduced pressure. The obtained oily substance was purified by silica gel column chromatography to obtain the title compound (77 mg).
¹H-NMR (CDCl₃) δ : 1.31 (9H, s), 1.48 (9H, s), 1.94 (1H, m), 2.16 (1H, m), 3.40 (1H, m), 3.61 (1H, m), 3.83 (1H, m), 4.01 (1H, m), 5.09 (2H, s), 6.62 (1H, s), 7.11 (1H, d, J=15.87Hz), 7.57 (1H, m), 7.58 (1H, d, J=15.87Hz), 8.21 (1H, s), 9.00 (1H, d, J=7.32Hz).
EI-MS; m/s: 656 (M⁺+1).

### (E) (E)-3-(8-({[4-(tert-Butyl)-1,3-thiazol-2-yl]amino}carbonyl)-2-{3,4-di[(aminocarbonyl)oxy]piperidino}-4-oxo-4H-pyrido[1,2-a]pyrimidin-3-yl)-2-propenoic acid

tert-Butyl (E)-3-(8-({[4-(tert-butyl)-1,3-thiazol-2-yl]amino}carbonyl)-2-{3,4-di[(aminocarbonyl)oxy]piperidino}-4-oxo-4H-pyrido[1,2-a]pyrimidin-3-yl)-2-propenoate (76.9 mg, 0.1173 mmol) was added with 4 N solution of hydrochloric acid in dioxane (6 ml) at room temperature and stirred at the same temperature for 3 hours. After completion of the reaction, the solvent was concentrated under reduced pressure, and the obtained oily substance was purified by thin layer silica gel column chromatography (lower layer of chloroform:methanol:water = 8:3:1) to obtain the title compound (30 mg, 6% for the six steps) as yellow crystals.
¹H-NMR (CD₃OD) δ : 1.36 (9H, s), 1.97 (1H, m), 2.12 (1H, m), 3.55 (1H, m), 3.71 (1H, d, J=13.92Hz), 3.89 (1H, m), 4.21 (1H, m), 5.05 (2H, s), 6.65 (1H, s), 7.06 (1H, d, J=15.63Hz), 7.61 (1H, m), 7.62 (1H, d, J=15.63Hz), 8.10 (1H, s), 8.96 (1H, d, J=7.32Hz). m.p.: 193-199°C (decomp.)
FAB-MS; m/s: 600 (M⁺+1).

### Example 233: (3R)-1-{8-({[4-(tert-Butyl)-1,3-thiazol-2-yl]amino}carbonyl)-4-oxo-3-[(E)-2-(2H-1,2,3,4-tetrazol-5-yl)-1-ethenyl]-4H-pyrido[1,2-a]pyrimidin-2-yl}hexahydro-3-pyridinylcarbamate

### (A) N⁸-[4-(tert-Butyl)-1,3-thiazol-2-yl]-2-[(3R)-3-hydroxyhexahydro-1-pyridinyl]-3-{(E)-2-[2-(4-methoxybenzyl)-2H-1,2,3,4-tetrazol-5-yl]-1-ethenyl}-4-oxo-4H-pyrido[1,2-a]pyrimidine-8-carboxyamide

N⁸-[4-(tert-Butyl)-1,3-thiazol-2-yl]-2-hydroxy-3-{(E)-2-[2-(4-methoxybenzyl)-2H-1,2,3,4-tetrazol-5-yl]-1-ethenyl}-4-oxo-4H-pyrido[1,2-a]pyrimidine-8-carboxamide (100 mg, 0.18 mmol) was dissolved in dimethylformamide (2 ml) and acetonitrile (2 ml) and cooled to -10°C with ice. The reaction mixture was added dropwise with diphenylphosphonyl chloride (74 µl, 0.36 mmol, 2 eq) and diisopropylamine (125 µl, 0.72 mmol, 4 eq) and stirred at the same temperature for 20 minutes. Then the reaction mixture was added with (R)-(+)-3-hydroxypiperidine hydrochloride (40 mg, 0.2685 mmol, 1.5 eq) and diisopropylamine (63 µ l, 0.36 mmol, 2 eq) at the same temperature, warmed to room temperature and then to 80°C, and after 1 hour, further added with piperidine (45 mg) and diisopropylamine (70 µl). Further, after stirred for 1 hour, the reaction solution was returned to room temperature, added with water and extracted with chloroform, and the organic layer was dried over magnesium sulfate. The solvent was concentrated under reduced pressure, and the obtained oily substance was purified by silica gel column chromatography (chloroform → chloroform:methanol = 100:1 → 80:1 → 50:1) to obtain the title compound (143 mg, almost quantitative) as a yellow oily substance with contained dimethylformamide.
¹H-NMR (CDCl₃) δ : 1.37 (9H, s), 1.48 (2H, m), 1.84 (2H, m), 3.32 (1H, m), 3.54 (2H, m), 3.64 (1H, m), 3.78 (3H, s), 4.07 (1H,s), 5.51 (2H, s), 6.54 (1H, s), 6.88 (2H, d, J=8.82Hz), 7.34 (2H, d, J=8.82Hz), 7.47 (1H, dd, J=7.59, 1.71Hz), 8.00 (1H, s), 8.03 (2H, s), 8.91 (1H, d, J=7.59Hz).
ES-MS; m/s: 642 (M⁺+1)

### (B) (3R)-1-(8-({[4-(tert-Butyl)-1,3-thiazol-2-yl]amino}carbonyl)-3-{[(E)-2-[2-(4-methoxybenzyl)-2H-1,2,3,4-tetrazol-5-yl]-1-ethenyl}-4-oxo-4H-pyrido[1,2-a]-pyrimidin-2-yl)hexahydro-3-pyridinylcarbamate

N⁸-[4-(tert-Butyl)-1,3-thiazol-2-yl]-2-[(3R)-3-hydroxyhexahydro-1-pyridinyl]-3-{(E)-2-[2-(4-methoxybenzyl)-2H-1,2,3,4-tetrazol-5-yl]-1-ethenyl}-4-oxo-4H-pyrido[1,2-a ]pyrimidine-8-carboxamide (143 mg, 0.22 mmol) was dissolved in ethyl acetate (3 ml), cooled to 0°C with ice, added with trichloro-isocyanate (45 µl, 0.36 mmol, 1.6 eq), stirred at the same temperature for 20 minutes, further added with trichloro-isocyanate (45 µl) and stirred for 15 minutes. After completion of the reaction, the solvent was concentrated under reduced pressure, and the obtained oily substance was added with methanol (4 ml), water (0.6 ml) and sodium formate (145 mg) at room temperature and stirred at the same temperature for 30 minutes. Then the reaction mixture was added with sodium formate (170 mg), after 30 minutes, further added with sodium formate (170 mg), water (0.5 ml) and methanol (1 ml) and stirred for 10 hours. The reaction mixture was added with water and extracted with chloroform, and the organic layer was dried over magnesium sulfate. The solvent was concentrated under reduced pressure, and the obtained oily substance was purified by thin layer silica gel column chromatography (chloroform:methanol = 15:1) to obtain the yellow title compound (144 mg) with contained impurities.
¹H-NMR (CDCl₃) δ: 1.32 (9H, s), 1.49 (1H, m), 1.84 (3H, m), 3.42 (2H, m), 3.78 (3H, s), 3.86 (1H, d, J=14.40Hz), 3.96 (1H, d, J=10.99Hz), 4.90 (1H, s), 5.53 (2H, s), 6.62 (1H, s), 6.89 (2H, d; J=8.79Hz), 7.34 (2H, d, J=8.79Hz), 7.59 (1H, d, J=7.32Hz), 7.98 (2H, s), 8.12 (1H, s), 8.99 (1H, d, J=7.32Hz).
EI-MS; m/s: 685 (M⁺+1)

### (C) (3R)-1-{8-({[4-(tert-Butyl)-1,3-thiazol-2-yl]amino}carbonyl)-4-oxo-3-[(E)-2-(2H-1,2,3,4-tetrazol-5-yl)-1-ethenyl]-4H-pyrido[1,2-a]pyrimidin-2-yl}hexahydro-3-pyridinylcarbamate

(3R)-1-({[4-(tert-Butyl)-1,3-thiazol-2-yl]amino]carbonyl)-3-{(E)-2-[2-(4-methoxybenzyl)-2H-1,2,3,4-tetrazol-5-yl]-1-ethenyl}-4-oxo-4H-pyrido[1,2-a]pyrimidin-2-yl)hexahydro-3-pyridinylcarbamate (144 mg, 0.21 mmol) was added with trifluoroacetic acid (10 ml) and stirred at room temperature for 17 hours. The solvent was concentrated under reduced pressure, and the obtained oily substance was purified by thin layer silica gel column chromatography (lower layer of chloroform:methanol:water = 8:3:1) to obtain the title compound (39 mg, 39% for the three steps) as yellow crystals.
¹H-NMR (CD₃OD/CDCl₃) δ : 1.36 (9H, s), 1.71 (1H, m), 1.97 (3H, m), 3.60 (1H, m), 3.76 (2H, m), 3.89 (1H, m), 4.87 (1H, s), 6.64 (1H, s), 7.62 (1H, d, J=7.31Hz), 7.68 (1H, d, J=15.60Hz), 7.86 (1H, d, J=15.60Hz), 8.12 (1H, s), 8.98 (1H, d, J=7.31Hz).
ES-MS; m/s: 565 (M⁺+1)
m.p.: 206-217.8°C
FAB-MS; m/s: 565 (MH+)

### Example 234: N⁸-[4-(tert-Butyl)-1,3-thiazol-2-yl]-2-{3,4-di[(aminocarbonyl)oxy]-piperidino}-4-oxo-3-[(E)-2-(2H-1,2,3,4-tetrazol-5-yl)-1-ethenyl]-4H-pyrido[1,2-a]-pyrimidine-8-carboxyamide

### (A) tert-Butyl 3,4-di[(aminocarbonyl)oxy]-1-piperidincarboxylate

tert-Butyl 3,4-dihydroxy-1-piperidinecarboxylate (200 mg, 0.92 mmol) was dissolved in ethyl acetate (10 ml) and cooled to 0°C with ice. The solution was added dropwise with trichloro-isocyanate (0.2 ml, 1.94 mmol, 2 eq) and stirred at the same temperature for 30 minutes. Then the reaction mixture was added with trichloro-isocyanate (0.2 ml), and after 40 minutes, further added with 0.25 ml of trichloro-isocyanate. After stirred for 30 minutes, the reaction solvent was concentrated under reduced pressure, and the obtained oily substance was added with methanol (9 ml), water (1.8 ml) and sodium formate (750 mg) at room temperature and stirred at the same temperature for 1 hour. Then the reaction mixture was added with sodium formate (750 mg) and further stirred for 1 hour. After completion of the reaction, the reaction solution was added with water and extracted with chloroform, and the organic layer was dried over magnesium sulfate. The solvent was evaporated under reduced pressure, and the obtained oily substance was purified by silica gel column chromatography (chloroform → chloroform:methanol = 80:1 → 50:1 → 20:1) to obtain the title compound (277.7 mg, 100%).
¹H-NMR (CD₃OD) δ : 1.46 (9H, s), 1.78 (1H, m), 1.93 (1H, m), 3.09 (1H, m), 3.28 (1H, m), 3.89 (1H, m), 4.07 (1H, m), 4.85 (2H, m).
EI-MS; m/s: 304 (M⁺+1)

### (B) 3,4-Di[(aminocarbonyl)oxy]piperidine

tert-Butyl 3,4-di[(aminocarbonyl)oxy]-1-piperidinecarboxylate (278 mg, 0.92 mmol) was added with 4 N solution of hydrochloric acid in dioxane (25 ml) at room temperature and stirred at the same temperature for 11 hours. The reaction solution was concentrated under reduced pressure to obtain the title compound (248 mg, 100%) as white crystals.
¹H-NMR (CD₃OD) δ : 2.07 (1H, m), 2.21 (1H, m), 3.17 (1H, m), 3.36 (3H, m), 4.98 (1H, m), 5.18 (1H, m).
EI-MS; m/s: 204 (M⁺+1)

### (C) N⁸-[4-(tert-Butyl)-1,3-thiazol-2-yl]-2-{3,4-di[(aminocarbonyl)oxy]piperidino}-3-(E)-2-[2-(4-methoxybenzyl)-2H-1,2,3,4-tetrazol-5-yl]-1-ethenyl-4-oxo-4H-pyrido[1,2-a]pyrimidine-8-carboxyamide

N⁸-[4-(tert-Butyl)-1,3-thiazol-2-yl]-2-hydroxy-3-(E)-2-(2-(4-methoxybenzyl)-2H - 1,2,3,4-tetrazol-5-yl]-1-ethenyl-4-oxo-4H-pyrido[1,2-a]pyrimidine-8-carboxamide (150 mg, 0.27 mmol) was dissolved in acetonitrile (3 ml) and dimethylformamide (3 ml) and cooled to -10°C with ice. The solution was added with diphenylphosphonyl chloride (111 µl, 0.54 mmol, 2 eq) and then added with diisopropylamine (185 µl, 1.08 mmol, 4 eq). The mixture was stirred at the same temperature for 10 minutes, then further added with diphenylphosphonyl chloride (60 µl) and diisopropylamine (90 µl) and further stirred for 10 minutes. Then the mixture was added with 3,4-di[(aminocarbonyl)oxy]piperidine hydrochloride (95 mg, 0.41 mmol, 1.5 eq) and diisopropylamine (95 µl, 0.54 mmol, 2 eq), heated to 80°C and stirred for 40 minutes. The mixture was added with 3,4-di[(aminocarbonyl)oxy]piperidine hydrochloride (60 mg) and diisopropylamine (40 µl) and after 30 minutes, further added with 3,4-di[(aminocarbonyl)oxy]piperidine hydrochloride (70 mg) and diisopropylamine (80 µl). After stirred for 1 hour, the reaction solution was added with water and extracted with chloroform, and the organic layer was dried over magnesium sulfate. The solvent was concentrated under reduced pressure, and the obtained oily compound was purified by thin layer silica gel column chromatography (chloroform:methanol = 10:1) to obtain the title compound (67 mg, 34%) as yellow crystals.
¹H-NMR (CD₃OD/CDCl₃) δ: 1.36 (9H, s), 1.93 (1H, m), 2.10 (1H, m), 3.52 (1H, m), 3.65 (1H, m), 3.80 (3H, s), 3.97 (1H, m), 4.19 (1H, m), 5.05 (2H, m), 5.56 (2H, s), 6.62 (1H, s), 6.91 (2H, d, J=8.78Hz), 7.36 (2H, d, J=8.78Hz), 7.62 (1H, d, J=7.56Hz), 7.73 (1H, d, J=15.36Hz), 7.90 (1H, d, J=15.36Hz), 8.11 (1H, s), 9.01 (1H, d, J=7.56Hz).
EL-MS; m/s: 744 (M⁺+1)

### (D) N⁸-[4-(tert-Butyl)-1,3-thiazol-2-yl]-2-{3,4-di[(aminocarbonyl)oxy]piperidino}-4-oxo-3-[(E)-2-(2H-1,2,3,4-tetrazol-5-yl)-1-ethenyl]-4H-pyrido[1,2-a]pyrimidine-8-carboxyamide

N⁸-[4-(tert-Butyl)-1,3-thiazol-2-yl]-2-{3,4-di[(aminocarbonyl)oxy]piperidino}-3-(E)-2-[2-(4-methoxybenzyl)-2H-1,2,3,4-tetrazol-5-yl]-1-ethenyl-4-oxo-4H-pyrido[1,2-a]pyrimidine-8-carboxamide (67 mg, 0.09 mmol) was added with trifluoroacetic acid (10 ml) at room temperature and stirred at the same temperature for 15 hours. Then the solvent of the reaction solution was concentrated under reduced pressure, and the obtained oily substance was purified by thin layer silica gel column chromatography (lower layer of chloroform:methanol:water = 8:3:1) to obtain the title compound (30 mg, 53%) as orange crystals.
¹H-NMR (CD₃OD) δ : 1.35 (9H, s), 1.95 (1H, m), 2.15 (1H, m), 3.57 (1H, m), 3.72 (1H, m), 4.02 (1H, m), 4.25 (1H, m), 5.02 (2H, m), 6.75 (1H, s), 7.52 (2H, d, J=16.36Hz), 7.59 (1H, d, J=7.32Hz), 7.89 (1H, d, J=16.36Hz), 7.08 (1H, s), 8.98 (1H, d, J=7.32Hz).
m.p.: 184-214°C (decomp.)
EI-MS; m/s: 624 (M⁺+1)
FAB-MS; m/s: 624 (MH+)

### Example 235: (E)-3-[8-{[(4-Cyclobutyl-1,3-thiazol-2-yl)amino]carbonyl}-2-{3,4-di[(aminocarbonyl)oxy]piperidino}-4-oxo-4H-pyrido[1,2-a]pyrimidin-3-yl]-2-propenoic acid

### (A) tert-Butyl (E)-3-{8-{[(4-cyclobutyl-1,3-thiazol-2-yl)amino]carbonyl}-2-{3,4-di[(aminocarbonyl)oxy]piperidino}-4-oxo-4H-pyrido[1,2-a]pyrimidin-3-yl}-2-propenoate

tert-Butyl (E)-3-(8-{[(4-cyclobutyl-1,3-thiazol-2-yl)amino]carbonyl}-2-hydroxy-4-oxo-4H-pyrido[1,2-a]pyrimidin-3-yl)-2-propenoate (50 mg, 0.11 mmol) was dissolved in acetonitrile (1 ml) and dimethylformamide (1 ml), cooled to -10°C with ice, added with diphenylphosphonyl chloride (46 µl, 0.22 mmol, 2 eq) and diisopropylamine (80 µl, 0.44 mmol, 4 eq) and stirred at the same temperature for 10 minutes. Then the reaction mixture was added with diphenylphosphonyl chloride (40 µl) and diisopropylamine (60 µl) and further stirred for 10 minutes. The reaction mixture was added with 3,4-di[(aminocarbonyl)oxy]piperidine hydrochloride (40 mg, 0.17 mmol, 1.5 eq) and diisopropylamine (40 µl) and heated to 90°C. After stirred for 30 minutes, the mixture was added with 3,4-di[(aminocarbonyl)oxy]piperidine hydrochloride (100 mg) and diisopropylamine (100 µl) and further stirred for 30 minutes. The reaction solution was returned to room temperature, added with water and extracted with chloroform, and the organic layer was dried over magnesium sulfate. The solvent was concentrated under reduced pressure, and the obtained oily compound was purified by thin layer silica gel column chromatography (chloroform:methanol = 10:1) to obtain the yellow title compound (23 mg, 32%).
¹H-NMR (CD₃OD/CDCl₃) δ : 1.52 (9H, s), 1.95-2.39 (8H, m), 3.45-3.70 (4H, m), 3.86 (1H, m), 5.03 (1H, m), 5.09 (1H, m), 6.63 (1H, s), 7.08 (1H, d, J=15.63Hz), 7.51 (1H, d, J=15.63Hz), 7.56 (1H, d, J=7.56Hz), 8.08 (1H, s), 8.98 (1H, d, J=7.56Hz).
EI-MS; m/s: 654 (M⁺+1)

### (B) (E)-3-[8-{[(4-Cyclobutyl-1,3-thiazol-2-yl)amino]carbonyl]-2-{3,4-di[(aminocarbonyl)oxy]piperidino}-4-oxo-4H-pyrido[1,2-a]pyrimidin-3-yl]-2-propenoic acid

tert-Butyl (E)-3-{8-{[(4-cyclobutyl-1,3-thiazol-2-yl)amino]carbonyl)-2-{3,4-di[(aminocarbonyl)oxy]piperidino}-4-oxo-4H-pyrido[1,2-a]pyrimidin-3-yl}-2-propenoate (23 mg, 0.04 mmol) was added with 4 N solution of hydrochloric acid in dioxane (3 ml) and stirred at room temperature for 3 hours. The solvent was concentrated under reduced pressure, and the obtained oily compound was purified by thin layer silica gel column chromatography (lower layer of chloroform:methanol:water = 8:3:1) to obtain the title compound (11 mg, 52%) as yellow crystals.
¹H-NMR (CD₃OD/CDCl₃) δ : 1.94-2.37 (8H, m), 3.40-3.60 (4H, m), 3.86 (1H, m), 5.08 (2H, m), 6.63 (1H, s), 7.10 (1H, m), 7.63 (2H, m), 8.12 (1H, s), 8.98 (1H, m).
m.p.: 202-210°C (decomp.)
EI-MS; m/s: 598 (M⁺+1)
FAB-MS; m/s: 598 (MH+)

### Example 236: [2-({[((3R)-1-{8-({[4-(tert-Butyl)-1,3-thiazol-2-yl]amino}carbonyl)-3-[(E)-2-carboxy-1-ethenyl]-4-oxo-4H-pyrido[1,2-a]pyrimidin-2-yl}hexahydro-3-pyridinyl)-oxy]carbonyl}amino)ethyl](trimethyl)ammonium

### (A) tert-Butyl (E)-3-{8-({[4-(tert-butyl)-1,3-thiazol-2-yl]amino}carbonyl)-2-[(3R)-3-({[(2-chloroethyl)amino]carbonyl}oxy)hexahydro-1-pyridinyl]-4-oxo-4H-pyrido[1,2-a]pyrimidin-3-yl}-2-propenoate

tert-Butyl (E)-3-{8-({[4-(tert-butyl)-1,3-thiazol-2-yl]amino}carbonyl)-2-[(3R)-3-hydroxyhexahydro-1-pyridinyl]-4-oxo-4H-pyrido[1,2-a]pyrimidin-3-yl}-2-propenoate (237 mg, 0.43 mmol) was added with dichloromethane (4 ml) and pyridine (50 µl), added with 2-chloroethyl isocyanate (73 µl, 0.86 mmol) for 40 minutes, stirred at room temperature and refluxed by heating at 60°C for 1 hour. The mixture was added with 2-chloroethyl isocyanate (73 µl, 0.86 mmol) and further refluxed by heating for 24 hours. Then the reaction mixture was returned to room temperature and added with water to terminate the reaction. The reaction solution was extracted with chloroform, and the collected organic layer was dried over magnesium sulfate. The solvent was concentrated under reduced pressure, and the resulting crude product was purified by thin layer silica gel chromatography (chloroform:methanol = 3:1) to obtain the target compound (287 mg).
¹H-NMR (CDCl₃) δ : 1.34 (9H, s), 1.52 (9H, s), 1.74-1.92 (6H, m), 3.20-3.69 (6H, m), 4.87 (1H, s), 6.61 (1H, s), 7.15 (1H, d, J=14.67Hz), 7.48 (1H, d, J=7.34Hz), 7.55 (1H, d, J=14.67Hz), 7.95 (1H, s), 8.99 (1H, d, J=7.34Hz).
EI-MS; m/s: 660 (M⁺+1)

### (B) tert-Butyl (E)-3-(8-({[4-(tert-butyl)-1,3-thiazol-2-yl]amino}carbonyl)-2-{(3R)-3-[({[2-(dimethylamino)ethyl]amino}carbonyl)oxy]hexahydro-1-pyridinyl}-4-oxo-4H-pyrido[1,2-a]pyrimidin-3-yl)-2-propenoate

tert-Butyl (E)-3-{8-({[4-(tert-butyl)-1,3-thiazol-2-yl]amino}carbonyl)-2-[(3R)-3-({[(2-chloroethyl)amino]carbonyl}oxy)hexahydro-1-pyridinyl]-4-oxo-4H-pyrido[1,2-a]pyrimidin-3-yl}-2-propenoate (196 mg, 0.30 mmol) was added with dimethylformamide (2 ml), then added with a dimethylamine solution in tetrahydrofuran (2.0 M, 0.75 ml, 1.50 mmol, 5 eq) and heated to 80°C. Then a dimethylamine solution in tetrahydrofuran was occasionally added until completion of the reaction, and 9.25 ml was used in total. The reaction was performed for 30 hours. The reaction solution was added with saturated brine and extracted with chloroform, and the resulting organic layer was dried over magnesium sulfate. The solvent was concentrated under reduced pressure, and the resulting crude product was purified by thin layer silica gel chromatography (chloroform:methanol = 20:1) to obtain the title compound (55 mg).
¹H-NMR (CDCl₃) δ: 1.34 (9H, s), 1.52 (9H, s), 1.65 (1H, m), 1.94 (3H, m), 2.22 (6H, s), 2.34 (1H, m), 2.47 (1H, m), 3.20 (1H, m), 3.31 (1H, m), 3.49 (1H, m), 3.70 (2H, m), 3.85 (1H, d, J=12.72Hz), 4.96 (1H, s), 6.61 (1H, s), 7.10 (1H, d, J=15.41Hz), 7.45 (1H, d, J=7.34Hz), 7.55 (1H, d, J=15.41Hz), 7.94 (1H, s), 8.98 (1H, d, J=7.34Hz).
EI-MS; m/s: 668 (M⁺+1).

### (C) (E)-3-(8-({[4-(tert-Butyl)-1,3-thiazol-2-yl]amino}carbonyl)-2-{(3R)-3-[({[2-(dimethylamino)ethyl]amino}carbonyl)oxy]hexahydro-1-pyridinyl}-4-oxo-4H-pyrido[1,2-a]pyrimidin-3-yl)-2-propenoic acid

tert-Butyl (E)-3-(8-({[4-(tert-butyl)-1,3-thiazol-2-yl]amino}carbonyl)-2-{(3R)-3-[({[2-(dimethylamino)ethyl]amino}carbonyl)oxy]hexahydro-1-pyridinyl]-4-oxo-4H-pyrido[1,2-a]pyrimidin-3-yl)-2-propenoate (55 mg, 0.08 mmol) was added with 4 N hydrochloric acid in dioxane (3 ml) at room temperature and stirred at the same temperature for 1 hour 30 minutes. After completion of the reaction, the reaction mixture was concentrated under reduced pressure, and the obtained oily crude product was purified by thin layer silica gel chromatography (lower layer of chloroform:methanol:water = 8:3:1) to obtain the title compound (21 mg, 8% for the three steps).
¹H-NMR (CDCl₃) δ : 1.34 (9H, s), 1.71 (2H, m), 1.95 (1H, d, J=12.74Hz), 2.11 (1H, d, J=12.74Hz), 2.57 (6H, s), 2.66 (1H, m), 2.88 (1H, m), 2.98 (1H, t, J=11.27Hz), 3.10 (1H, d, J=13.72Hz), 3.21 (1H, m), 3.70 (1H, m), 3.84 (1H, d, J=13.72Hz), 4.13 (1H, d, J=11.27Hz), 4.98 (1H, s), 6.19 (1H, d, J=6.12Hz), 6.62 (1H, s), 6.95 (1H, d, J=15.43Hz), 7.46 (1H, dd, J=7.35, 1.96Hz), 7.80 (1H, d, J=15.43Hz), 7.87 (1H, s), 9.03 (1H, d, J=7.35Hz).
EI-MS; m/s: 612 (M⁺+1).

### (D) [2-({[((3R)-1-{8-({(4-(tert-Butyl)-1,3-thiazol-2-yl]amino}carbonyl)-3-[(E)-2-carboxy-1-ethenyl]-4-oxo-4H-pyrido[1,2-a]pyrimidin-2-yl}hexahydro-3-pyridinyl)oxy]-carbonyl}amino)ethyl](trimethyl)ammonium

(E)-3-(8-({[4-(tert-Butyl)-1,3-thiazol-2-yl]amino}carbonyl)-2-{(3R)-3-[({[2-(dimethylamino)ethyl]amino}carbonyl)oxy]hexahydro-1-pyridinyl}-4-oxo-4H-pyrido[1,2-a]pyrimidin-3-yl)-2-propenoic acid (21 mg, 0.03 mmol) was added with N,N-dimethylformamide (4 ml) and methyl iodide (43 µl, 0.68 mmol, 20 eq) and left stand in the refrigerator for 20 hours. Then the mixture was further added with methyl iodide (50 µl, 0.79 mmol, 23 eq) and left stand in the refrigerator for 24 hours. After completion of the reaction was confirmed, the solvent was concentrated under reduced pressure, and the resulting crude product was purified by liquid chromatography (acetonitrile:water = 30:70, 0.1% formic acid solution, flow rate 10 cc/min) to obtain the title compound (17 mg, 79%).
(¹H-NMR (CD₃OD) δ : 1.35 (9H, s), 1.67 (1H, brd), 2.01 (3H, brd), 3.19 (9H, s), 3.41-3.71 (6H, m), 3.92 (1H, m), 4.04 (1H, m), 4.85 (1H, brd), 6.75 (1H, s), 7.08 (1H, brd), 7.62 (2H, m), 8.00 (1H, s), 8.95 (1H, d, J=7.34Hz).
ES-MS; m/s: 626 (M⁺).

### Example 237:[3-({[((3R)-1-{8-({[4-(tert-Butyl)-1,3-thiazol-2-yl]amino}carbonyl)-3-[(E)-2-carboxy-1-ethenyl]-4-oxo-4H-pyrido[1,2-a]pyrimidin-2-yl}hexahydro-3-pyridinyl)oxy]carbonyl}amino)propyl](trimethyl)ammonium

### (A) tert-Butyl (E)-3-{8-({[4-(tert-butyl)-1,3-thiazol-2-yl]amino]carbonyl)-2-((3R)-3-({[(3-chloropropyl)amino]carbonyl}oxy)hexahydro-1-pyridinyl]-4-oxo-4H-pyrido[1,2-a]pyrimidin-3-yl}-2-propenoate

tert-Butyl (E)-3-{8-({[4-(tert-butyl)-1,3-thiazol-2-yl]amino}carbonyl)-2-[(3R)-3-hydroxyhexahydro-1-pyridinyl]-4-oxo-4H-pyrido [1,2-a]pyrimidin-3-yl]-2-propenoate (682 mg, 1.23 mmol) was added with tetrahydrofuran (20 ml) and pyridine (155 µl), then added with 3-chloropropyl isocyanate (265 µ l, 2.46 mmol) and refluxed by heating at 80°C for 3 hours. Then 3-chloropropyl isocyanate was added until completion of the reaction, and 2.1 ml (17.2 mmol, 14 eq) was used in total. The reaction mixture was stirred for 3 days. The reaction mixture was returned to room temperature and added with saturated aqueous sodium hydrogencarbonate to terminate the reaction. The reaction mixture was extracted with chloroform, and the collected organic layer was dried over magnesium sulfate. The solvent was concentrated under reduced pressure, and the resulting crude product was purified by silica gel chromatography (chloroform → chloroform:methanol = 100:1) to obtain the target compound (522.5 mg, 63%).
¹H-NMR (CDCl₃) δ: 1.33 (9H, s), 1.52 (9H, s), 1.69-2.04 (6H, m), 3.19-3.94 (8H, m), 4.86 (1H, s), 5.80 (1H, brd), 6.59 (1H, s), 7.11 (1H, d, J=15.36Hz), 7.50 (1H, d, J=7.31Hz), 7.68 (1H, d, J=15.36Hz), 7.97 (1H, s), 8.98 (1H, d, J=7.31Hz).
ES-MS; m/s: 675 (M⁺+1).

### (B) tert-Butyl (E)-3-(8-({[4-(tert-butyl)-1,3-thiazol-2-yl]amino}carbonyl)-2-{(3R)-3-[({[3-(dimethylamino)propyl]amino}carbonyl)oxy]hexahydro-1-pyridinyl}-4-oxo-4H-pyrido[1,2-a]pyrimidin-3-yl)-2-propenoate

tert-Butyl (E)-3-{8-({[4-(tert-butyl)-1,3-thiazol-2-yl]amino}carbonyl)-2-[(3R)-3-({[(3-chloropropyl)amino]carbonyl}oxy)hexahydro-1-pyridinyl]-4-oxo-4H-pyrido[1,2-a]pyrimidin-3-yl}-2-propenoate (258 mg, 0.4 mmol) was added with dimethylformamide (4 ml), then added with a dimethylamine solution in tetrahydrofuran (2.0 M, 0.75 ml, 1.50 mmol, 5 eq) and heated to 80°C. Then the dimethylamine solution in tetrahydrofuran was occasionally added until completion of the reaction, and 17 ml (34 mmol, 85 eq) was used in total. The reaction was performed for 48 hours. The reaction solution was added with saturated brine and extracted with chloroform. The collected organic layer was dried over magnesium sulfate. The solvent was concentrated under reduced pressure, and the resulting crude product was purified by thin layer silica gel chromatography (chloroform:methanol = 10:1) to obtain the title compound (46 mg, 18%).
¹H-NMR (CDCl₃) δ: 1.33 (9H, s), 1.52 (9H, s), 1.72 (3H, brd), 1.92 (3H, brd), 2.26 (6H, s), 2.41 (2H, m), 3.26 (2H, m), 3.49 (1H, m), 3.73 (2H, m), 3.85 (1H, m), 4.90 (1H, s), 5.84 (1H, brd), 6.60 (1H, s), 7.11 (1H, d, J=15.36Hz), 7.47 (1H, d, J=7.56Hz), 7.57 (1H, d, J=15.36Hz), 7.96 (1H, s), 8.98 (1H, d, J=7.56Hz).
EI-MS; m/s: 682 (M⁺+1).

### (C) (E)-3-(8-({[4-(tert-Butyl)-1,3-thiazol-2-yl]amino}carbonyl)-2-{(3R)-3-[({[3-(dimethylamino)propyl]amino}carbonyl)oxy]hexahydro-1-pyridinyl}-4-oxo-4H-pyrido[1,2-a]pyrimidin-3-yl)-2-propenoic acid

tert-Butyl (E)-3-(8-({[4-(tert-butyl)-1,3-thiazol-2-yl]amino}carbonyl)-2-{(3R)-3-[({[3-(dimethylamino)propyl]amino}carbonyl)oxy]hexahydro-1-pyridinyl}-4-oxo-4H-pyrido[1,2-a]pyrimidin-3-yl)-2-propenoate (46 mg, 0.07 mmol) was added with 4 N hydrochloric acid in dioxane (5 ml) at room temperature and stirred for at the same temperature 1 hour and 30 minutes. After completion of the reaction, the reaction mixture was concentrated under reduced pressure. The obtained oily crude product was purified by thin layer silica gel chromatography (lower layer of chloroform:methanol:water = 8:3:1 → chloroform:methanol = 10:1, develop 4 times) to obtain the title compound (20 mg, 49%).
¹H-NMR (CD₃OD/CDCl₃) δ: 1.34 (9H, s), 1.73 (2H, brd), 1.90 (3H, m), 2.15 (1H, m), 2.70 (6H, s), 3.31-3.44 (6H, m), 3.81 (1H, d, J=14.65Hz), 3.99 (1H, m), 4.89 (1H, s), 6.62 (1H, s), 7.23 (1H, d, J=15.38Hz), 7.56 (1H, d, J=6.84Hz), 7.82 (1H, d, J=15.38Hz), 8.06 (1H, s), 9.03 (1H, d, J=6.84Hz).
EI-MS; m/s: 626 (M⁺+1).

### (D) [3-({[((3R)-1-{8-({[4-(tert-Butyl)-1,3-thiazol-2-yl]amino}carbonyl)-3-[(E)-2-carboxy-1-ethenyl]-4-oxo-4H-pyrido[1,2-a]pyrimidin-2-yl}hexahydro-3-pyridinyl)-oxy]carbonyl}amino)propyl](trimethyl)ammonium

(E)-3-(8-({[4-(tert-Butyl)-1,3-thiazol-2-yl]amino}carbonyl)-2-{(3R)-3-[({[3-(dimethylamino)propyl]amino}carbonyl)oxy]hexahydro-1-pyridinyl}-4-oxo-4H-pyrido[1,2-a]pyrimidin-3-yl)-2-propenoic acid (20 mg, 0.03 mmol) was added with N,N-dimethylformamide (2.5 ml) and methyl iodide (100 µl, 1.2 mmol, 40 eq) and left stand in the refrigerator for 64 hours. After completion of the reaction was confirmed, the solvent was concentrated under reduced pressure, and the resulting crude product was purified by liquid chromatography (acetonitrile:water = 30:70, 0.1% formic acid solution, flow rate: 9 cc/min) to obtain the title compound (17 mg, 83%).
¹H-NMR (CD₃OD) δ: 1.35 (9H, s), 1.69 (1H, brd), 1.93 (5H, m), 3.15 (9H, s), 3.16 (2H, m), 3.31 (1H, brd), 3.44 (1H, brd), 3.71 (2H, m), 3.93 (2H, m), 4.86 (1H, brd), 6.75 (1H, s), 7.03 (1H, brd), 7.59 (2H, brd), 7.99 (1H, brd), 8.94 (1H, s).
ES-MS; m/s: 640 (M⁺).

### Example 238: (E)-3-(8-({[4-(tert-Butyl)-1,3-thiazol-2-yl]amino}carbonyl)-4-oxo-2-{(3R)-3-[({[2-(1-pyridinyl)ethyl]amino}carbonyl)oxy]hexahydro-1-pyridinyl}-4H-pyrido[1,2-a]pyrimidin-3-yl)-2-propenoic acid

### (A) (E)-3-{8-({[4-(tert-Butyl)-1,3-thiazol-2-yl]amino}carbonyl)-2-[(3R)-3-({[(2-chloroethyl)amino]carbonyl}oxy)hexahydro-1-pyridinyl]-4-oxo-4H-pyrido[1,2-a]-pyrimidin-3-yl}-2-propenoic acid

A crude compound of tert-butyl (E)-3-{8-({[4-(tert-butyl)-1,3-thiazol-2-yl]amino}carbonyl)-2-[(3R)-3-({[(2-chloroethyl)amino]carbonyl}oxy)hexahydro-1-pyridinyl]-4-oxo-4H-pyrido[1,2-a]pyrimidin-3-yl)-2-propenoate (620 mg, 0.94 mmol) was added with 4 N hydrochloric acid in dioxane (30 ml) at room temperature and stirred at the same temperature for 11 hours. The reaction solution was concentrated under reduced pressure, and the resulting crude product was purified by silica gel chromatography (chloroform → chloroform:methanol = 100:1 → 70:1 → 50:1 → 20:1) and further purified by HPLC to obtain the title compound (57 mg).
¹H-NMR (CDCl₃) δ : 1.39 (9H, s), 1.80-2.04 (4H, m), 3.43 (2H, m), 3.51 (2H, m), 3.70 (3H, m), 4.03 (1H, brd), 4.90 (1H, brd), 5.29 (1H, brd), 6.65 (1H, s), 7.28 (1H, d, J=17.31Hz), 7.76 (2H, m), 8.25 (1H, s), d), 9.07 (1H, d, J=7.31Hz).
EI-MS; m/s: 603 (M⁺).

### (B) (E)-3-(8-({[4-(tert-Butyl)-1,3-thiazol-2-yl]amino}carbonyl)-4-oxo-2-{(3R)-3-[({[2-(1-pyridinyl)ethyl]amino}carbonyl)oxy]hexahydro-1-pyridinyl}-4H-pyrido[1,2-a]-pyrimidin-3-yl)-2-propenoic acid

(E)-3-{8-({[4-(tert-Butyl)-1,3-thiazol-2-yl]amino}carbonyl)-2-[(3R)-3-({[(2-chloroethyl)amino]carbonyl}oxy)hexahydro-1-pyridinyl]-4-oxo-4H-pyrido[1,2-a]pyrimidin-3-yl}-2-propenoic acid (57 mg, 0.10 mmol) was added with pyridine (3 ml) and stirred at 100°C for 17 hours. After completion of the reaction, the solvent was concentrated under reduced pressure, and the resulting crude product was purified by HPLC to obtain the title compound (25 mg, 41%).
¹H-NMR (CD₃OD) δ : 1.35 (9H, s), 1.69 (1H, m), 1.80 (1H, m), 1.90 (2H, m), 3.46 (1H, m), 3.65 (5H, m), 4.66 (3H, m), 6.75 (1H, s), 7.03 (1H, d, J=15.38Hz), 7.61 (2H, m), 7.96 (1H, s), 8.11 (2H, m), 8.37 (1H, brd), 8.61 (1H, t, J=7.81Hz), 8.95 (3H, m).
ES-MS; m/s: 646 (M⁺).

### Example 239: 1-[2-({[((3R)-1-{8-({[4-(tert-Butyl)-1,3-thiazol-2-yl]amino}carbonyl)-4-oxo-3-[(E)-2-(2H-1,2,3,4-tetrazol-5-yl)-1-ethenyl]-4H-pyrido[1,2-a]pyrimidin-2-yl}hexahydro-3-pyridinyl}oxy}carbonyl}amino}ethyl}pyridinium

### (A) N⁸-[4-(tert-Butyl)-1,3-thiazol-2-yl]-2-[(3R)-3-hydroxyhexahydro-1-pyridinyl]-3-{(E)-2-[2-(4-methoxybenzyl)-2H-1,2,3,4-tetrazol-5-yl]-1-ethenyl}-4-oxo-4H-pyrido[1,2-a]pyrimidine-8-carboxyamide

Reactions were performed by using N⁸-[4-(tert-butyl)-1,3-thiazol-2-yl]-2-hydroxy-3-{(E)-2-[2-(4-methoxybenzyl)-2H-1,2,3,4-tetrazol-5-yl]-1-ethenyl}-4-oxo-4H-pyrido[1,2-a]pyrimidine-8-carboxamide (400 mg, 0.72 mmol) in the same manner as in Example 3, (A) to obtain the title compound (500 mg, 100%).
¹H-NMR (CD₃OD/CDCl₃) δ: 1.36 (9H, s), 1.66 (2H, m), 1.93 (2H, m), 3.49 (1H, m), 3.64 (1H, m), 3.79 (3H, m), 3.90 (1H, m), 4.03 (1H, m), 4.59 (1H, brd), 5.59 (2H, s), 6.64 (1H, s), 6.93 (2H, d, J=8.53Hz), 7.37 (2H, d, J=8.53Hz), 7.62 (1H, brd), 7.70 (1H, d, J=15.60Hz), 7.87 (1H, d, J=15.60Hz), 8.08 (1H, brd), 9.02 (1H, d, J=7.32Hz).
EI-MS; m/s: 642 (M⁺+1).

### (B) (3R)-1-(8-({[4-(tert-Butyl)-1,3-thiazol-2-yl]amino}carbonyl)-3-{(E)-2-[2-(4-methoxybenzyl)-2H-1,2,3,4-tetrazol-5-yl]-1-ethenyl}-4-oxo-4H-pyrido[1,2-a]-pyrimidin-2-yl)hexahydro-3-pyridinyl N-(2-chloroethyl)carbamate

N⁸-[4-(tert-Butyl)-1,3-thiazol-2-yl]-2-[(3R)-3-hydroxyhexahydro-1-pyridinyl] -3-{(E)-2-[2-(4-methoxybenzyl)-2H-1,2,3,4-tetrazol-5-yl]-1-ethenyl}-4-oxo-4H-pyrido[1,2-a]pyrimidine-8-carboxamide (500 mg, 0.8 mmol) was added with tetrahydrofuran (8 ml) and pyridine (100 µl, 1.3 mmol, 1.6 eq), then added with 2-chloroethyl isocyanate (150 µl, 1.6 mmol, 2 eq) and stirred at 80°C for 2 hours. Then the mixture was added with 2-chloroethyl isocyanate (550 µl, 5.9 mmol, 7.3 eq) and further stirred at 80°C for 4 hours. The reaction mixture was returned to room temperature, added with saturated aqueous sodium hydrogencarbonate to terminate the reaction and extracted with chloroform. The collected organic layer was dried over magnesium sulfate, and the solvent was concentrated under reduced pressure. The resulting crude product was purified by silica gel chromatography (chloroform → chloroform:methanol = 80:1 → 50:1) to obtain the target compound (570 mg) with contained impurities.
¹H-NMR (CD₃OD/CDCl₃) δ : 1.36 (9H, s), 1.73 (1H, m), 1.93 (3H, m), 3.35 (3H, m), 3.50 (2H, m), 3.62 (3H, m), 3.80 (3H, s), 4.85 (1H, brd), 5.59 (2H, s), 6.63 (1H, s), 6.93(2H, d, J=8.78Hz), 7.37 (2H, d, J=8.78Hz), 7.63 (1H, m), 7.91 (2H, q, J=15.38Hz), 8.12 (1H, brd), 9.02 (2H, d, J=7.57Hz).
EI-MS; m/s: 747 (M⁺).

### (C) 1-{2-[({(3R)-1-(8-({[4-(tert-Butyl)-1,3-thiazol-2-yl]amino}carbonyl)-3-{(E)-2-[2-(4-methoxybenzyl)-2H-1,2,3, 4-tetrazol-5-yl]-1-ethenyl}-4-oxo-4H-pyrido[1,2-a]-pyrimidin-2-yl)hexahydro-3-pyridinyl]oxy}carbonyl)amino]ethyl}pyridinium

(3R)-1-(8-({[4-(tert-Butyl)-1,3-thiazol-2-yl]amino}carbonyl)-3-{(E)-2-[2-(4-methoxybenzyl)-2H-1,2,3,4-tetrazol-5-yl]-1-ethenyl}-4-oxo-4H-pyrido[1,2-a]pyrimidin-2-yl)hexahydro-3-pyridinyl N-(2-chloroethyl)carbamate (137 mg, 0.18 mmol) was added with pyridine (8 ml) and stirred at 100°C for 16 hours and 30 minutes. After completion of the reaction, the solvent was concentrated under reduced pressure, and the resulting crude product was purified by thin layer silica gel column chromatography (chloroform:methanol:water = 8:3:1) to obtain the title compound (122 mg, 84%).
¹H-NMR (CD₃OD) δ: 1.35 (9H, s), 1.67 (1H, m), 1.84 (3H, m), 3.37-3.80 (6H, m), 3.75 (3H, s), 4.64 (1H, brd), 4.73 (2H, m), 5.57 (2H, d, J=6.12Hz), 6.70 (1H, s), 6.91 (2H, d, J=8.82Hz), 7.32 (2H, d, J=8.82Hz), 7.59 (1H, m), 7.78 (2H, m), 7.93 (1H, brd), 8.07 (2H, t, J=7.10Hz), 8.53 (1H, t, J=7.10Hz), 8.94 (3H, m).
EI-MS; m/s: 790 (M⁺).

### (D) 1-[2-({[((3R)-1-{8-({[4-(tert-Butyl)-1,3-thiazol-2-yl]amino}carbonyl)-4-oxo-3-[(E)-2-(2H-1,2,3,4-tetrazol-5-yl)-1-ethenyl]-4H-pyrido[1,2-a]pyrimidin-2-yl}hexahydro-3-pyridinyl)oxy]carbonyl}amino)ethyl]pyridinium

1-{2-[({[(3R)-1-(8-({[4-(tert-Butyl)-1,3-thiazol-2-yl]amino}carbonyl)-3-{(E)-2-[2-(4-methoxybenzyl)-2H-1,2,3,4-tetrazol-5-yl]-1-ethenyl]-4-oxo-4H-pyrido[1,2-a]pyrimidin-2-yl)hexahydro-3-pyridinyl]oxy}carbonyl)amino]ethyl}pyridinium (122 mg, 0.15 mmol) was added with trifluoroacetic acid (8 ml) at room temperature and stirred at the same temperature for 3 hours and 30 minutes. As the reaction did not proceed, the reaction mixture was heated to 60°C and further stirred for 2 hours and 30 minutes. After completion of the reaction, the solvent was concentrated under reduced pressure, and the resulting crude product was purified by thin layer silica gel column - chromatography (lower layer of chloroform:methanol:water = 8:3:1) to obtain the title compound (74 mg, 72%).
¹H-NMR (CD₃OD) δ: 1.35 (9H, s), 1.70 (1H, m), 1.83 (2H, m), 1.97 (1H, m), 3.37-3.50 (2H, m), 3.71 (4H, m), 4.69 (3H, m), 6.75 (1H, s), 7.56 (1H, d, J=15.41Hz), 7.61 (1H, d, J=7.35Hz), 8.00 (4H, m), 8.48 (1H, t, J=7.84Hz), 8.83 (2H, d, J=5.63Hz), 9.01 (1H, d, J=7.35Hz).
ES-MS; m/s: 670 (M⁺).

### Example 240:(2-Amino-2-oxoethyl)[3-({[((3R)-1-{8-({[4-(tert-butyl)-1,3-thiazol-2-yl]-amino}carbonyl)-3-[(E)-2-carboxy-1-ethenyl]4-oxo-4H-pyrido[1,2-a]pyrimidin-2-yl}hexahydro-3-pyridinyl)oxy]carbonyl}amino)propyl]dimethylammonium

### (A) tert-Butyl (E)-3-(8-({[4-(tert-butyl)-1,3-thiazol-2-yl]amino}carbonyl)-2-{(3R)-3-[({[3-(dimethylamino)propyl]amino}carbonyl)oxy]hexahydro-1-pyridinyl}-4-oxo-4H-pyrido[1,2-a]pyrimidin-3-yl)-2-propenoate

tert-Butyl (E)-3-{8-({[4-(tert-butyl)-1,3-thiazol-2-yl]amino}carbonyl)-2-[(3R)-3-({[(3-chloropropyl)amino]carbonyl}oxy)hexahydro-1-pyridinyl]-4-oxo-4H-pyrido[1,2-a]pyrimidin-3-yl}-2-propenoate (198 mg, 0.29 mmol) was added with a dimethylamine solution in tetrahydrofuran (2.0 M, 5 ml, 10 mmol, 34 eq) and heated to 65°C. Then the dimethylamine solution in tetrahydrofuran was occasionally added until completion of the reaction, 15 ml (30 mmol, 103 eq) was used in total and the reaction was performed for 30 hours. The reaction solution was added with saturated brine and extracted with chloroform. The collected organic layer was dried over magnesium sulfate. The solvent was concentrated under reduced pressure, and the resulting crude product was purified by thin layer silica gel chromatography (chloroform:methanol = 15:1) to obtain the title compound (32 mg, 16%).
¹H-NMR (CDCl₃) δ : 1.33 (9H, s), 1.52 (9H, s), 1.72 (3H, brd), 1.92 (3H, m), 2.25 (6H, s), 2.41 (2H, m), 3.26 (2H, m), 3.49 (1H, m), 3.67 (2H, m), 3.83 (1H, m), 4.90 (1H, s), 5.84 (1H, brd), 6.60 (1H, s), 7.10 (1H, d, J=15.36Hz), 7.47 (1H, d, J=7.56Hz), 7.57 (1H, d, J=15.36Hz), 7.96 (1H, s), 8.98 (1H, d, J=7.56Hz).
EI-MS; m/s: 682 (M⁺+1).

### (B) (2-Amino-2-oxoethyl)(3-{[({(3R)-1-[3-[(E)-3-(tert-butoxy)-3-oxo-1-propenyl]-8-({[4-(tert-butyl)-1,3-thiazol-2-yl]amino}carbonyl)-4-oxo-4H-pyrido[1,2-a]pyrimidin-2-yl]-hexahydro-31-pyridinyl}oxy)carbonyl]amino}propyl)dimethylammonium

tert-Butyl (E)-3-(8-({[4-(tert-butyl)-1,3-thiazol-2-yl]amino}carbonyl)-2-{(3R)-3-[({[3-(dimethylamino)propyl]amino}carbonyl)oxy]hexahydro-1-pyridinyl}-4-oxo-4H-pyrido[1,2-a]pyrimidin-3-yl)-2-propenoate (32 mg, 0.05 mmol) was added with iodoacetamide (15 mg, 0.09 mmol, 1.8 eq) and N,N-dimethylformamide (4 ml) and stirred at room temperature for 14 hours. After completion of the reaction, the solvent was concentrated under reduced pressure and the resulting crude product was purified by thin layer silica gel column chromatography (chloroform:methanol:water = 6:4:1) to obtain the title compound (48 mg, ~quantitative).
¹H-NMR (CD₃OD) δ: 1.36 (9H, s), 1.54 (9H, s), 1.73 (1H, brd), 2.01 (5H, m), 3.19 (2H, m), 3.62 (2H, m), 3.69 (6H, s), 3.82 (2H, m), 4.14 (2H, m), 4.60 (2H, m), 4.83 (1H, s), 6.66 (1H, d, J=14.67Hz), 6.97 (1H, d, J=15.16Hz), 7.53 (1H, d, J=15.16Hz), 7.62 (1H, s), 8.06 (1H, s), 8.94 (1H, s).
EI-MS; m/s: 739 (M⁺).

### (C) (2-Amino-2-oxoethyl)[3-({[((3R)-1-{8-({[4-(tert-butyl)-1,3-thiazol-2-yl]amino}carbonyl)-3-[(E)-2-carboxy-1-ethenyl]-4-oxo-4H-pyrido[1,2-a]pyrimidin-2-yl}hexahydro-3-pyridinyl)oxy]carbonyl}amino)propyl]dimethylammonium

Reactions were performed in the same manner as in Example 238, (A) by using (2-amino-2-oxoethyl)(3-{[({(3R)-1-(3-[(E)-3-(tert-butoxy)-3-oxo-1-propenyl]-8-([(4-(tert-butyl)-1,3-thiazol-2-yl]amino}carbonyl)-4-oxo-4H-pyrido(1,2-a]pyrimidin-2-yl]hexahydro-3-pyridinyl}oxy)carbonyl]amino}propyl)dimethylammonium (48 mg, 0.06 mmol) and 4 N hydrochloric acid in dioxane (6 ml), and the resulting crude product was purified by thin layer silica gel column chromatography (lower layer of chloroform:methanol:water = 8:3:1) to obtain the title compound (18.5 mg, 49%).
¹H-NMR (CD₃OD/CDCl₃) δ : 1.36 (9H, s), 1.78 (2H, m), 2.03 (4H, m), 3.15 (1H, m), 3.25 (1H, m), 3.35 (6H, s), 3.39 (2H, m), 3.68 (2H, m), 3.86 (1H, m), 3.95 (1H, m), 4.32 (2H, m), 4.85 (1H, s), 6.64 (1H, s), 7.18 (1H, d, J=15.11Hz), 7.61 (1H, d, J=7.56Hz), 7.69 (1H, d, J=15.11Hz), 8.08 (1H, s), 8.98 (1H, d, J=7.56Hz).
ES-MS; m/s: 683 (M⁺).

### Example 241: (2-Amino-2-oxoethyl)[2-({[((3R)-1-{8-({[4-(tert-butyl)-1,3-thiazol-2-yl]-amino}-carbonyl)-4-oxo-3-[(E)-2-(2H-1,2,3,4-tetrazol-5-yl)-1-ethenyl]-4H-pyrido[1,2-a]pyrimidin-2yl}hexahydro-3-pyridinyl)oxy]carbonyl}amino}ethyl}dimethylammonium

### (A) N⁸-[4-(tert-Butyl)-1,3-thiazol-2-yl]-2-[(3R)-3-hydroxyhexahydro-1-pyridinyl]-3-{(E)-2-[2-(4-methoxybenzyl)-2H-1,2,3,4-tetrazol-5-yl]-1-ethenyl}-4-oxo-4H-pyrido[1,2-a]pyrimidine-8-carboxyamide

N⁸-[4-(tert-Butyl)-1,3-thiazol-2-yl]-2-hydroxy-3-{(E)-2-[2-(4-methoxybenzyl)-2H-1,2,3,4-tetrazol-5-yl]-1-ethenyl}-4-oxo-4H-pyrido[1,2-a]pyrimidine-8-carboxamide (7.1 g, 12.7 mmol) was dissolved in dimethylformamide (50 ml) and acetonitrile (200 ml) and cooled to 0°C with ice, The mixture was added dropwise with diphenylphosphonyl chloride (5.3 ml, 25.4 mmol, 2 eq) and diisopropylamine (8.8 ml, 50.8 mmol, 4 eq) and stirred at the same temperature for 15 minutes. Then the mixture was added with diphenylphosphonyl chloride (1.3 ml, 6.4 mmol, 0.5 eq) and diisopropylamine (2.5 ml, 12.7 mmol, 1 eq) and further stirred at 0°C for 10 minutes. The reaction solution was heated to 80°C, added with (R)-(+)-3-hydroxypiperidine hydrochloride (2.6 g, 19 mmol, 1.5 eq) and diisopropylamine (4.4 ml, 25.4 mmol, 2 eq) and after 30 minutes, added with (R)-(+)-3-hydroxypiperidine hydrochloride (3.3 g, 25.4 mmol, 2 eq) and diisopropylamine (9 ml, 50.8 mmol, 4 eq). After further stirred for 40 minutes, the reaction solution was returned to room temperature, added with 1 N hydrochloric acid and extracted with chloroform. The organic layer was neutralized by adding saturated aqueous sodium hydrogencarbonate, and the collected organic layer was dried over magnesium sulfate. The solvent was concentrated under reduced pressure, and the obtained oily substance was purified by silica gel column chromatography (chloroform → chloroform:ethyl acetate = 3:1 → 1:1 → chloroform:methanol = 50:1 → 30:1 → 10:1) to obtain the title compound (5.92 g, 73%) as a yellow oily substance with contained dimethylformamide.
¹H-NMR (CDCl₃) δ : 1.38 (9H, s), 1.52 (1H, m), 1.87 (3H, m), 3.50-3.83 (4H, m), 3.77 (3H, s), 4.07 (1H, s), 5.50 (2H, s), 6.49 (1H, s), 6.89 (2H, d, J=8.53Hz), 7.30 (2H, d, J=8.53Hz), 7.44 (1H, d, J=5.83Hz), 7.78 (2H, s), 8.05 (1H, s), 8.83 (1H, s).
EI-MS; m/s: 642 (M⁺+1)

### (B) (3R)-1-(8-({[4-(tert-Butyl)-1,3-thiazol-2-yl]amino]carbonyl)-3-{(E)-2-[2-(4-methoxybenzyl)-2H-1,2,3,4-tetrazol-5-yl]-1-ethenyl}-4-oxo-4H-pyrido[1,2-a]pyrimidin-2-yl)hexahydro-3-pyridinyl N-(2-chloroethyl)carbamate

Reactions were performed in the same manner as in Example 239, (B) by using N⁸-[4-(tert-butyl)-1,3-thiazol-2-yl]-2-[(3R)-3-hydroxyhexahydro-1-pyridinyl]-3-{(E)-2-[2-(4-methoxybenzyl)-2H-1,2,3,4-tetrazol-5-yl]-1-ethenyl}-4-oxo-4H-pyrido[1,2-a]pyrimidine-8-carboxamide (6 g, 9.4 mmol), tetrahydrofuran (70 ml), pyridine (0.75 ml, 9.4 mmol, 1 eq) and 2-chloroethyl isocyanate (19 ml, 179 mmol, 19 eq), and the resulting crude product was purified by silica gel chromatography (chloroform → chloroform:ethyl acetate = 2:1 → 1:1) to obtain the title compound (7.97 g) with contained impurities.
¹H-NMR (CDCl₃) δ: 1.36 (9H, s), 1.73 (1H, brd), 1.93 (3H, m), 3.35 (2H, m), 3.50 (2H, m), 3.62 (2H, m), 3.80 (3H, s), 3.81 (2H, m), 4.85 (1H, s), 5.59 (2H, s), 6.63 (1H, brd), 6.92 (2H, d, J=8.55Hz), 7.37 (2H, d, J=8.55Hz), 7.63 (1H, brd), 7.91 (2H, dd, J=17.59, 15.38Hz), 8.12 (1H, brd), 9.02 (1H, d, J=7.56Hz).
EI-MS; m/s: 748 (M⁺+1).

### (C) (3R)-1-(8-({[4-(tert-Butyl)-1,3-thiazol-2-yl]amino}carbonyl)-3-{(E)-2-[2-(4-methoxybenzyl)-2H-1,2,3,4-tetrazol-5-yl]-1-ethenyl}-4-oxo-4H-pyrido[1,2-a]-pyrimidin-2-yl)hexahydro-3-pyridinyl N-[2-(dimethylamino)ethyl]carbamate

(3R)-1-(8-({[4-(tert-Butyl)-1,3-thiazol-2-yl]amino}carbonyl)-3-{(E)-2-(2-(4-methoxybenzyl]-2H-1,2,3,4-tetrazol-5-yl]-1-ethenyl}-4-oxo-4H-pyrido[1,2-a]pyrimidin-2-yl)hexahydro-3-pyridinyl N-(2-chloroethyl)carbamate (7.97 g, 10.7 mmol) was added with 50% aqueous solution of N,N-dimethylamine (30 ml, 0.53 mol, 50 eq) and ethanol (20 ml) and stirred at 80°C for 5 hours. The reaction solution was returned to room temperature, added with saturated brine and extracted with chloroform, and the organic layer was dried over magnesium sulfate. The solvent was concentrated under reduced pressure, and the resulting crude product was purified by silica gel column chromatography (chloroform → chloroform:methanol = 100:1 → 70:1 → 50:1 → 30:1 → 10:1) to obtain the title compound (3.6 g, 37% for the four steps).
¹H-NMR (CDCl₃) δ : 1.35 (9H, s), 1.68 (1H, brd), 1.96 (3H, m), 2.20 (6H, s), 2.50 (2H, s), 3.33 (3H, m), 3.62 (1H, m), 3.72 (2H, s), 3.77 (3H, s), 4.85 (1H, s), 5.49 (2H, s), 6.11 (1H, brd), 6.55 (1H, s), 6.88 (2H, d, J=8.55Hz), 7.35 (2H, d, J=8.55Hz), 7.46 (1H, d, J=7.32Hz), 7.84 (3H, m), 8.90 (1H, d, J=7.32Hz).
EI-MS; m/s: 756 (M⁺+1).

### (D) (2-Amino-2-oxoethyl)[2-({[((3R)-1-{8-({[4-(tert-butyl)-1,3-thiazol-2-yl]amino}carbonyl)-4-oxo-3-{(E)-2-[2-(4-methoxybenzyl)-2H-1,2,3,4-tetrazol-5-yl]-1-ethenyl}-4H-pyrido[1,2-a]pyrimidin-2-yl}hexahydro-3-pyridinyl)oxy]carbonyl}amino)ethyl]-dimethylammonium

(3R)-1-(8-({[4-(tert-Butyl)-1,3-thiazol-2-yl]amino}carbonyl)-3-{(E)-2-[2-(4-methoxybenzyl)-2H-1,2,3,4-tetrazol-5-yl]-1-ethenyl}-4-oxo-4H-pyrido[1,2-a]pyrimidin-2-yl)hexahydro-3-pyridinyl N-[2-(dimethylamino)ethyl]carbamate (3.6 g, 4.8 mmol) was added with iodoacetamide (1 g, 5.3 mmol, 1.1 eq) and dimethylformamide (30 ml) and stirred at room temperature for 17 hours and 30 minutes. After completion of the reaction, the solvent was concentrated under reduced pressure and used in the following reaction without being purified.
¹H-NMR (CDCl₃) δ : 1.34 (9H, s), 1.74-2.17 (4H, m), 3.20 (1H, m), 3.35 (1H, m), 3.42 (3H, s), 3.46 (3H, s), 3.67 (3H, s), 3.69-4.04 (6H, m), 4.61 (2H, s), 4.82 (1H, s), 5.54 (2H, s), 6.62 (1H, s), 6.91 (2H, d, J=8.82Hz), 7.36 (2H, d, J=8.82Hz), 7.55 (1H, d, J=7.35Hz), 7.97 (3H, m), 9.00 (1H, d, J=7.35Hz).
EI-MS; m/s: 813 (M⁺).

### (E) (2-Amino-2-oxoethyl)[2-({[((3R)-1-{8-({[4-(tert-butyl)-1,3-thiazol-2-yl]amino}carbonyl)-4-oxo-3-[(E)-2-(2H-1,2,3,4-tetrazol-5-yl)-1-ethenyl]-4H-pyrido[1,2-a]-pyrimidin-2-yl}hexahydro-3-pyridinyl)oxy]carbonyl}amino)ethyl]dimethylammonium

(2-Amino-2-oxoethyl)[2-({[((3R)-1-{8-({[4-(tert-butyl)-1,3-thiazol-2-yl]amino}carbonyl)-4-oxo-3-{(E)-2-[2-(4-methoxybenzyl)-2H-1,2,3,4-tetrazol-5-yl]-1-ethenyl}-4H-pyrido[1,2-a]pyrimidin-2-yl}hexahydro-3-pyridinyl)oxy]carbonyl}amino)ethyl]-dimethylammonium (4.48 g, 4.8 mmol) was added with anisole (5 ml) and trifluoroacetic acid (160 ml) and stirred at 60°C for 3 hours. After completion of the reaction, the solvent was concentrated under reduced pressure, and the crude product was added with diisopropyl ether. The precipitated solid was taken by filtration and washed with diisopropylether to remove most of anisole. The resulting solid was purified by liquid chromatography (methanol:0.1% acetic acid aqueous solution = 60:40, flow rate: 25 cc/min) to obtain the title compound (825 mg, 25%).
¹H-NMR (CD₃OD) δ : 1.35 (9H, s), 1.71 (1H, m), 1.98 (3H, m), 3.32 (6H, s), 3.63 (6H, m), 3.89 (2H, m), 4.17 (2H, m), 4.80 (1H, m), 6.73 (1H, s), 7.52 (1H, d, J=16.14Hz), 7.57 (1H, d, J=7.34Hz), 7.94 (1H, d, J=16.14Hz), 8.00 (1H, s), 8.96 (1H, d, J=7.34Hz).
FAB-MS; m/s: 693 (M⁺).

### Example 242: (E)-3-[2-{3-[(Aminocarbonyl)oxy]piperidino}-8-({[4-(tert-butyl)-1,3-thiazol-2-yl]amino)carbonyl)-4-oxo-4H-pyrido[1,2-a]pyrimidin-3-yl]-2-propenoic acid

### (A) tert-Butyl (E)-3-[8-({[4-(tert-butyl)-1,3-thiazol-2-yl]amino}carbonyl)-2-(3-hydroxypiperidino)-4-oxo-4H-pyrido[1,2-a]pyrimidin-3-yl]-2-propenoate

Reactions were performed in the same manner as in Example 231, (C) by using tert-butyl (E)-3-{8-({[4-(tert-butyl)-1,3-thiazol-2-yl]amino}carbonyl)-2-hydroxy-4-oxo-4H-pyrido[1,2-a]pyrimidin-3-yl}-2-propenoate (695 mg, 1.5 mmol), and the resulting crude product was purified by silica gel column chromatography (chloroform → chloroform: methanol = 100:1 → 80:1 → 60:1) to obtain the title compound (709 mg) with contained impurities.
¹H-NMR (CDCl₃) δ : 1.30 (9H, s), 1.38 (9H, s), 1.72 (2H, m), 1.82 (2H, m), 2.91 (2H, m), 3.53 (2H, m), 3.60 (1H, m), 6.57 (1H, s), 7.94 (1H, s), 8.90 (1H, m).
EI-MS; m/s: 554 (M⁺+1).

### (B) tert-Butyl (E)-3-[2-{3-[(aminocarbonyl)oxy]piperidino}-8-({[4-(tert-butyl)-1,3-thiazol-2-yl]amino}carbonyl)-4-oxo-4H-pyrido[1,2-a]pyrimidin-3-yl]-2-propenoate

Reactions were performed in the same manner as in Example 232, (D) by using tert-Butyl (E)-3-[8-({[4-(tert-butyl)-1,3-thiazol-2-yl]amino}carbonyl)-2-(3-hydroxypiperidino)-4-oxo-4H-pyrido[1,2-a]pyrimidin-3-yl]-2-propenoate (709 mg, 1.28 mmol), and the resulting crude product was purified by silica gel column chromatography (chloroform → chloroform:methanol = 100:1) to obtain the title compound (670 mg) with contained impurities.
¹H-NMR (CDCl₃) δ: 1.30 (9H, s), 1.51 (9H, s), 1.48 (1H, m), 1.75 (3H, m), 3.24 (3H, m), 3.42 (1H, m), 4.51 (1H, brd), 6.60 (1H, s), 7.11 (1H, d, J=13.16Hz), 7.45 (1H, dd, J=7.56, 4.84Hz), 7.53 (1H, d, J=13.16Hz), 8.07 (1H, s), 8.95 (1H, d, J=7.56Hz).

### (C) (E)-3-[2-{3-[(Aminocarbonyl)oxy]piperidino}-8-({[4-(tert-butyl)-1,3-thiazol-2-yl]amino}carbonyl)-4-oxo-4H-pyrido[1,2-a]pyrimidin-3-yl]-2-propenoic acid

Reactions were performed in the same manner as in Example 232, (E) by using tert-Butyl (E)-3-[2-{3-[(aminocarbonyl)oxy]piperidino}-8-({[4-(tert-butyl)-1,3-thiazol-2-yl]amino}carbonyl)-4-oxo-4H-pyrido[1,2-a]pyrimidin-3-yl]-2-propenoate (670 mg, 1.12 mmol), and the resulting crude product was purified by silica gel column chromatography (chloroform → chloroform:methanol = 100:1 → 80:1 → 50:1 → 10:1 → chloroform:methanol:water = 6:4:1) to obtain the title compound (40 mg, 5% for the three steps).
¹H-NMR (CD₃OD) δ: 1.35 (9H, s), 1.70 (1H, m), 1.85 (1H, m), 2.00 (2H, m), 3.56 (1H, m), 3.71 (1H, m), 3.84 (2H, m), 4.86 (1H, m), 6.74 (1H, s), 7.00 (1H, d, J=15.63Hz), 7.56 (1H, dd, J=7.32, 1.71Hz), 7.61 (1H, d, J=15.63Hz), 8.01 (1H, s), 8.92 (1H, d, J=7.32Hz).
ES-MS; m/s: 541 (M⁺+1).

### Example 243: N⁸-[4-(tert-Butyl)-1,3-thiazol-2-yl]-2-[4-({2-[1-(2-amino-2-oxoethyl)-1,1-dimethylammonio]acetyl)amino)piperidino]-4-oxo-3-[(E)-2-(2H-1,2,3,4-tetrazol-5-yl)-1-ethenyl]-4H-pyrido[1,2a]pyrimidine-8-carboxyamide

### (A) N-(1-Benzyl-piperidin-4-yl)-2-(dimethylamino)acetamide

1-Benzyl-4-piperidinamine (3 g, 16 mmol) was added with dichloromethane (30 ml), dimethylaminoacetic acid (1.95 g, 19 mmol), 1-hydroxybenzotriazole (2.6 g, 19.2 mmol, 1.2 eq) and triethylamine (3.3 ml, 24 mmol, 1.5 eq) at room temperature, then added with 1-ethyl-3-(3-dimethylaminopropyl)carbodiimide hydrochloride (4.5 g, 24 mmol, 1.5 eq) at room temperature and stirred at the same temperature for 16 hours and 30 minutes. After completion of the reaction, the mixture was added with saturated aqueous sodium hydrogencarbonate and extracted with chloroform. The organic layer was washed with saturated brine, and the collected organic layer was dried over magnesium sulfate. The solvent was concentrated under reduced pressure, and the resulting crude product was purified by silica gel column chromatography (chloroform → chloroform:methanol = 100:1 → 80:1 → 50:1 → 30:1 → 10:1) to obtain the title compound (4.6 g, 100%).
¹H-NMR (CDCl₃) δ: 1.50 (2H, dq, J=11.23, 3.91Hz), 1.89 (2H, dd, J=12.45, 3.91Hz), 2.13 (2H, d, J=11.23Hz), 2.27 (6H, s), 2.80 (2H, d, J=12.45Hz), 2.91 (2H, s), 3.49 (2H, s), 3.82 (1H, m), 7.04 (1H, d, J=7.81Hz), 7.27 (5H, m).
EI/MS; m/z: 276 (M⁺+1).

### (B) N¹-(4-Piperidyl)-2-(dimethylamino)acetamide

N-(1-Benzyl-piperidin-4-yl)-2-(dimethylamino)acetamide (4.6 g, 17 mmol) was added with methanol (16 ml) and 20% palladium hydroxide carbon (500 mg), attached with a balloon containing hydrogen, and stirred at room temperature for 16 hours. After completion of the reaction, palladium was removed by filtration through a Celite layer, and the solvent was concentrated under reduced pressure to obtain the title compound (3.4 g, 100%) without purification.
¹H-NMR (CD₃OD) δ: 1.77 (2H, q, J=11.94Hz), 2.06 (2H, d, J=11.94Hz), 2.34 (6H, s), 3.07 (4H, m), 3.34 (2H, m), 3.97 (1H, m).
EI/MS; m/z: 186 (M⁺+1).

### (C) N⁸-[4-(tert-Butyl)-1,3-thiazol-2-yl]-2-(4-{[2-(dimethylamino)acetyl]amino}piperidino)-3-{(E)-2-[2-(4-methoxybenzyl)-2H-1,2,3,4-tetrazol-5-yl]-1-ethenyl}-4-oxo-4H-pyrido[1,2-a]pyrimidine-8-carboxyamide

N⁸-[4-(tert-Butyl)-1,3-thiazol-2-yl]-2-hydroxy-3-[(E)-2-[2-(4-methoxybenzyl)-2 H-1,2,3,4-tetrazol-5-yl]-1-ethenyl}-4-oxo-4H-pyrido[1,2-a]pyrimidine-8-carboxamide (310 mg, 0.6 mmol) was dissolved in dimethylformamide (6.5 ml) and acetonitrile (11 ml) and cooled to -10°C with ice. The mixture was added dropwise with diphenylphosphonyl chloride (0.23 ml, 1.2 mmol, 2 eq) and diisopropylamine (0.38 ml, 2.4 mmol, 4 eq) and stirred at the same temperature for 5 minutes. Then the mixture was added with diphenylphosphonyl chloride (0.23 ml, 1.2 mmol, 2 eq) and diisopropylamine (0.38 ml, 2.4 mmol, 4 eq) and further stirred at -10°C for 15 minutes. The reaction mixture was added with 2-dimethylamino-N-piperidin-4-yl-acetamide (155 mg, 0.9 mmol, 1.5 eq), heated to 80°C, stirred for 1 hour and then added with 2-dimethylamino-N-piperidin-4-yl-acetamide (200 mg, 1.2 mmol, 2 eq) and diisopropylamine (0.3 ml, 1.8 mmol, 3 eq). After further stirred for 40 minutes, the reaction solution was returned to room temperature, added with water and extracted with chloroform, and the collected organic layer was dried over magnesium sulfate. The solvent was concentrated under reduced pressure, and the obtained oily substance was purified by silica gel column chromatography (chloroform → chloroform:methanol = 100:1 → 70:1 → 40:1 → 10:1) and further purified by thin layer silica gel column chromatography (chloroform:methanol = 20:1) to obtain the title compound (182 mg, 45%).
¹H-NMR (CDCl₃) δ : 1.37 (9H, s), 1.59 (1H, m), 1.86 (2H, d, J=13.23Hz), 2.00 (1H, m), 2.26 (6H, s), 2.92 (2H, s), 3.08 (1H, m), 3.11 (1H, t, J=12.23Hz), 3.73 (2H, m), 3.78 (3H, s), 3.89 (1H, m), 4.04 (1H, m), 5.53 (2H, s), 6.57 (1H, s), 6.90 (2H, d, J=7.56Hz), 7.16 (2H, d, J=7.56Hz), 7.45 (1H, d, J=7.09Hz), 7.75 (1H, d, J=15.41Hz), 7.92 (1H, d, J=15.41Hz), 7.96 (1H, s), 8.88 (1H, d, J=7.09Hz).
EI-MS; m/s: 726 (M⁺+1).

### (D) N⁸-[4-(tert-Butyl)-1,3-thiazol-2-yl]-2-[4-({2-[1-(2-amino-2-oxoethyl)-1, 1-dimethylammonio]acetyl}amino)piperidino]-3-{(E)-2-[2-(4-methoxybenzyl)-2H-1,2,3,4-tetrazol-5-yl]-1-ethenyl}-4-oxo-4H-pyrido[1,2-a]pyrimidine-8-carboxyamide

Reactions were performed in the same manner as in Example 11, (D) by using N⁸-[4-(tert-butyl)-1,3-thiazol-2-yl]-2-(4-{[2-(dimethylamino)acetyl]amino}piperidino)-3-{(E)-2-[2-(4-methoxybenzyl)-2H-1,2,3,4-tetrazol-5-yl]-1-ethenyl}-4-oxo-4H-pyrido[1,2-a]pyrimidine-8-carboxamide (182 mg, 0.25 mmol), and the resulting crude product was purified by thin layer silica gel column chromatography (lower layer of chloroform:methanol:water = 8:3:1) to obtain the title compound (129 mg, 66%).
¹H-NMR (CDCl₃) δ : 1.30 (9H, s), 1.82 (1H, m), 2.06 (3H, m), 2.90 (2H, m), 3.28 (2H, m), 3.67 (3H, s), 3.73 (6H, s), 4.70 (5H, m), 5.48 (2H, s), 6.56 (1H, s), 6.85 (2H, d, J=8.79Hz), 7.18 (2H, d, J=8.79Hz), 7.47 (1H, s), 7.54 (1H, d, J=15.87Hz), 7.78 (1H, d, J=15.87Hz), 7.87 (1H, s), 8.85 (1H, s).
EI-MS; m/s: 783 (M⁺).

### (E) N⁸-[4-(tert-Butyl)-1,3-thiazol-2-yl]-2-[4-({2-[1-(2-amino-2-oxoethyl)-1,1-dimethylammonio]acetyl}amino)piperidino]-4-oxo-3-[(E)-2-(2H-1,2,3,4-tetrazol-5-yl)-1-ethenyl]-4H-pyrido[1,2a]pyrimidine-8-carboxyamide

Reactions were performed in the same manner as in Example 9, (D) by using N⁸-[4-(tert-butyl)-1,3-thiazol-2-yl]-2-[4-({2-[1-(2-amino-2-oxoethyl)-1,1-dimethylammonio]acetyl}amino)piperidino]-3-{(E)-2-[2-(4-methoxybenzyl)-2H-1,2,3,4-tetrazol-5-yl]-1-ethenyl}-4-oxo-4H-pyrido[1,2-a]pyrimidine-8-carboxamide (129 mg, 0.16 mmol), and the resulting crude product was purified by thin layer silica gel column chromatography (lower layer of chloroform:methanol:water = 8:3:1) to obtain the title compound (28 mg, 26%).
¹H-NMR (CD₃OD/CDCl₃) δ: 1.35 (9H, s), 1.70 (2H, m), 1.97 (2H, m), 3.09 (2H, t, J=12.21Hz), 3.41 (2H, m), 3.50 (6H, s), 4.40 (5H, m), 6.60 (1H, s), 7.36 (1H, d, J=16.11Hz), 7.53 (1H, d, J=7.57Hz), 7.89 (1H, d, J=16.11Hz), 7.99 (1H, s), 8.94 (1H, d, J=7.57Hz).
FAB-MS; m/s: 663 (M⁺).

### Example 244: [3-({[((3R)-1-{8-({[4-(tert-Butyl)-1,3-thiazol-2-yl]amino}carbonyl)-4-oxo-3-[(E)-2-(2H-1,2,3,4-tetrazol-5-yl)-1-ethenyl]-4H-pyrido[1,2-a]pyrimidin-2-yl}hexahydro-3-pyridinyl)oxy]carbonyl}amino)propyl](trimethyl)ammonium

### (A) tert-Butyl (3R)-3-hydroxyhexahydro-1-pyridinecarboxylate

(3R)-(+)-Hydroxypiperidine hydrochloride (1 g, 7.3 mmol) was added with dichloromethane (15 ml) and methanol (10 ml) and cooled to 0°C with ice. The reaction mixture was added with triethylamine (2.5 ml, 18.3 mmol, 2.5 eq) and di-tert-butyl dicarbonate (2.4 g, 11 mmol, 1.5 eq) and stirred at the same temperature for 1 hour. The mixture was added with water to terminate the reaction and extracted with chloroform, and the collected organic layer was dried over magnesium sulfate. The solvent was concentrated under reduced pressure, and the resulting crude product was purified by silica gel column chromatography to obtain the title compound (1.3 g, 89%).
¹H-NMR (CDCl₃) δ : 1.45 (9H, s), 1.46 (2H, m), 1.75 (1H, m), 1.89 (1H, brd), 3.02 (2H, m), 3.57 (1H, brd), 3.71 (1H, brd), 3.78 (1H, dd, J=12.70, 3.91Hz).

### (B) tert-Butyl (3R)-3-({[(3-chloropropyl)amino]carbonyl}oxy)hexahydro-1-pyridinecarboxylate

Reactions were performed in the same manner as in Example 7, (A) by using tert-butyl (3R)-3-hydroxyhexahydro-1-pyridinecarboxylate (730 mg, 3.6 mmol), and the resulting crude product was purified by silica gel column chromatography (chloroform → chloroform:methanol = 100:1 → 80:1 → 50:1) to obtain the title compound (1.14 g, 98%).
¹H-NMR (CDCl₃) δ: 1.45 (9H, s), 1.69-1.99 (6H, m), 3.34-3.67 (8H, m), 4.67 (1H, brd).

### (C) tert-Butyl (3R)-3-[({[3-(dimethylamino)propyl]amino}carbonyl)oxy]hexahydro-1-pyridinecarboxylate

tert-Butyl (3R)-3-({[(3-chloropropyl)amino]carbonyl}oxy)hexahydro-1-pyridinecarboxylate (100 mg, 0.3 mmol) was added with 50% aqueous solution of dimethylamine (2 ml) and ethanol (1 ml) and stirred in a sealed tube at 130°C for 4 hours. After completion of the reaction, the reaction mixture was extracted with chloroform, and the collected organic layer was dried over magnesium sulfate. The solvent was concentrated under reduced pressure to obtain the title compound.
¹H-NMR (CDCl₃) δ: 1.44 (9H, s), 1.73 (2H, brd), 1.85 (3H, brd), 2.08 (1H, brd), 2.65 (4H, brd), 3.29 (6H, s), 3.48 (4H, brd), 4.66 (1H, brd).
EI-MS; m/s: 330 (M⁺+1).

### (D) (3R)-Hexahydro-3-pyridinyl N-[3-(dimethylamino)propyl]carbamate

tert-Butyl (3R)-3-[({[3-(dimethylamino)propyl]amino}carbonyl)oxy]hexahydro-1-pyridinecarboxylate (885 mg, 2.69 mmol) was added with 4 N hydrochloric acid in dioxane (30 ml) at room temperature and stirred at the same temperature for 2 hours. After completion of the reaction, the solvent was concentrated under reduced pressure to obtain the title compound (730 mg) without purifying the obtained product.
¹H-NMR (CD₃OD) δ: 1.97 (6H, brd), 2.90 (3H, s), 2.91 (3H, s), 2.97 (1H, m), 3.07 (1H, m), 3.30 (4H, m), 3.66 (2H, m), 5.01 (1H, brd).
EI-MS; m/s: 230 (M⁺+1).

### (E) (3R)-1-(8-({[4-(tert-Butyl)-1,3-thiazol-2-yl]amino}carbonyl)-3-{(E)-2-[2-(4-methoxybenzyl)-2H-1,2,3,4-tetrazol-5-yl]-1-ethenyl}-4-oxo-4H-pyrido[1,2-a]-pyrimidin-2-yl)hexahydro-3-pyridinyl N-[3-(dimethylamino)propyl]carbamate

Reactions were performed in the same manner as in Example 3, (A) by using N⁸-[4-(tert-butyl)-1,3-thiazol-2-yl]-2-hydroxy-3-{(E)-2-[2-(4-methoxybenzyl)-2H-1,2,3,4 -tetrazol-5-yl]-1-ethenyl]-4-oxo-4H-pyrido[1,2-a]pyrimidine-8-carboxamide (250 mg, 0.45 mmol) and (3R)-hexahydro-3-pyridinyl N-[3-(dimethylamino)propyl]carbamate (540 mg, 1.83 mmol, 4 eq), and the resulting crude product was purified by silica gel column chromatography (chloroform:methanol = 10:1) to obtain the title compound (357 mg, 100%) with contained impurities.
¹H-NMR (CDCl₃) δ : 1.34 (9H, s), 1.65 (1H, brd), 1.75 (1H, brd), 1.97 (3H, brd), 2.11 (1H, brd), 2.58 (6H, s), 2.90 (2H, m), 3.28 (2H, m), 3.72 (4H, m), 3.77 (3H, m), 4.80 (1H, brd), 5.50 (2H, s), 6.58 (1H, s), 6.88 (2H, d, J=8.53Hz), 7.34 (2H, d, J=8.53Hz), 7.49 (1H, d, J=7.56Hz), 7.96 (3H, d, J=11.94Hz), 8.96 (2H, d, J=7.56Hz).
EI-MS; m/s: 770 (M⁺+1).

### (F) [3-({[((3R)-1-{8-({[4-(tert-Butyl)-1,3-thiazol-2-yl]amino}carbonyl)-4-oxo-3-{(E)-2-[2-(4-methoxybenzyl)-2H-1,2,3,4-tetrazol-5-yl]-1-ethenyl}-4H-pyrido[1,2a]pyrimidin-2-yl}hexahydro-3-pyridinyl)oxy]carbonyl}amino)propyl](trimethyl)ammonium

Reactions were performed in the same manner as in Example 6, (D) by using (3R)-1-(8-({[4-(tert-butyl)-1,3-thiazol-2-yl]amino)carbonyl)-3-{(E)-2-[2-(4-methoxybenzyl)-2H-1,2,3,4-tetrazol-5-yl]-1-ethenyl}-4-oxo-4H-pyrido[1,2-a]pyrimidin-2-yl)hexahydro-3-pyridinyl N-[3-(dimethylamino)propyl]carbamate (253 mg, 0.33 mmol), and the resulting crude product was purified by thin layer silica gel column chromatography (lower layer of chloroform:methanol:water = 8:3:1) to obtain the title compound (71 mg).
¹H-NMR (CD₃OD/CDCl₃) δ : 1.36 (9H, s), 1.71 (1H, brd), 1.99 (5H, brd), 3.13 (9H, s), 3.19-3.38 (6H, m), 3.70 (2H, m), 3.78 (3H, s), 4.91 (1H, brd), 5.61 (2H, s), 6.72 (1H, s), 6.92 (2H, m), 7.34 (2H, m), 7.87 (3H, m), 8.06 (1H, s), 9.01 (1H, s).
EI-MS; m/s: 784 (M⁺).

### (G) [3-({[((3R)-1-{8-({[4-(tert-Butyl)-1,3-thiazol-2-yl]amino}carbonyl)-4-oxo-3-((E)-2-(2H-1,2,3,4-tetrazol-5-yl)-1-ethenyl]-4H-pyrido[1,2-a]pyrimidin-2-yl}hexahydro-3-pyridinyl)oxy]carbonyl}amino)propyl](trimethyl)ammonium

Reactions were performed in the same manner as in Example 239, (D) by using [3-({[((3R)-1-{8-({[4-(tert-butyl)-1,3-thiazol-2-yl]amino}carbonyl)-4-oxo-3-{(E)-2-[2-(4-methoxybenzyl)-2H-1,2,3,4-tetrazol-5-yl]-1-ethenyl}-4H-pyrido[1,2a]pyrimidin-2-yl}hexahydro-3-pyridinyl)oxy]carbonyl}amino)propyl](trimethyl)ammonium (71 mg, 0.09 mmol), and the resulting crude product was purified by thin layer silica gel column chromatography (chloroform:methanol:water = 6:4:1) to obtain the title compound (19 mg, 32%).
¹H-NMR (CD₃OD) δ: 1.35 (9H, s), 1.69 (1H, brd), 1.92 (4H, brd), 2.05 (1H, brd), 3.11 (9H, s), 3.12-3.34 (5H, m), 3.53 (1H, d, J=13.92Hz), 3.94 (2H, m), 4.86 (1H, brd), 6.75 (1H, s), 7.93 (2H, m), 7.95 (1H, d, J=15.38Hz), 8.04 (1H, s), 8.98 (1H, d, J=7.08Hz).
FAB-MS; m/s: 664 (M⁺).

### Example 245: (2-Amino-2-oxoethyl)[3-({[((3R)-1-{8-({[4-(tert-butyl)-1,3-thiazol-2-yl]-amino}carbonyl)-4-oxo-3-[(E)-2-(2H-1,2,3,4-tetrazol-5-yl)-1-ethenyl]-4H-pyrido[1,2-a]pyrimidin-2-yl}hexahydro-3-pyridinyl)oxy]carbonyl}amino)propyl]-dimethylammonium

### (A)(2-Amino-2-oxoethyl)[3-({[((3R)-1-{8-({[4-(tert-butyl)-1,3-thiazol-2-yl]amino}carbonyl)-4-oxo-3-{(E)-2-[2-(4-methoxybenzyl)-2H-1,2,3,4-tetrazol-5-yl]-1-ethenyl}-4H-pyrido[1,2-a]pyrimidin-2-yl}hexahydro-3-pyridinyl)oxy]carbonyl}amino)-propyl]dimethylammonium

Reactions were performed in the same manner as in Example 240, (B) by using (3R)-1-(8-({[4-(tert-butyl)-1,3-thiazol-2-yl]amino}carbonyl)-3-{(E)-2-[2-(4-methoxybenzyl)-2H-1,2,3,4-tetrazol-5-yl]-1-ethenyl}-4-oxo-4H-pyrido[1,2-a]pyrimidin-2-yl)hexahydro-3-pyridinyl N-[3-(dimethylamino)propyl]carbamate (166 mg, 0.22 mmol), and the resulting crude product was purified by thin layer silica gel column chromatography (lower layer of chloroform:methanol:water = 8:3:1) to obtain the title compound (137 mg) with contained impurities.
¹H-NMR (CD₃OD/CDCl₃) δ: 1.40 (9H, s), 1.75 (2H, brd), 2.10 (4H, brd), 3.14 (4H, m), 3.30 (3H, s), 3.32 (3H, s), 3.45-3.90 (4H, m), 3.77 (3H, s), 4.26 (2H, s), 4.83 (1H, s), 5.56 (2H, s), 6.63 (1H, s), 6.92 (2H, d, J=8.79Hz), 7.27 (2H, d, J=8.79Hz), 7.64 (1H, d, J=7.32Hz), 7.99 (2H, s), 8.11 (1H, s), 9.04 (1H, d, J=7.32Hz).
EI-MS; m/s: 827 (M⁺).

### (B) (2-Amino-2-oxoethyl)[3-({[((3R)-1-{8-({[4-(tert-butyl)-1,3-thiazol-2-yl]amino}carbonyl)-4-oxo-3-[(E)-2-(2H-1,2,3,4-tetrazol-5-yl)-1-ethenyl]-4H-pyrido[1,2-a]-pyrimidin-2-yl}hexahydro-3-pyridinyl)oxy]carbonyl}amino)propyl]dimethylammonium

Reactions were performed in the same manner as in Example 239, (D) by using (2-amino-2-oxoethyl) [3-([((3R)-1-{8-([4-(tert-butyl)-1,3-thiazol-2-yl]-amino}carbonyl)-4-oxo-3-{(E)-2-[2-(4-methoxybenzyl)-2H-1,2,3,4-tetrazol-5-yl]-1-ethenyl]-4H-pyrido[1,2-a]pyrimidin-2-yl-hexahydro-3-pyridinyl)oxy]carbonylamino)-propyl]dimethylammonium (137 mg, 0.17 mmol), and the resulting crude product was purified by thin layer silica gel column chromatography (lower layer of chloroform:methanol:water = 8:3:1) to obtain the title compound (12 mg, 10%).
¹H-NMR (CD₃OD) δ : 1.35 (9H, s), 1.74 (2H, brd), 1.99 (3H, brd), 2.17 (1H, brd), 3.14 (3H, d, J=12.70Hz), 3.22 (3H, s), 3.32 (3H, s), 3.37 (1H, m), 3.50 (2H, dd, J=5.75, 6.84Hz), 3.95 (2H, m), 4.15 (2H, dd, J=43.46, 15.63Hz), 4.82 (1H, s), 6.63 (1H, s), 7.59 (1H, d, J=7.81Hz), 7.75 (1H, d, J=16.11Hz), 8.08 (1H, d, J=16.11Hz), 8.09 (1H, s), 9.05 (1H, d, J=7.81Hz).
FAB-MS; m/s: 707 (M⁺).

### Example 246: [2-({[((3R)-1-{8-({[4-(tert-Butyl)-1,3-thiazol-2-yl]amino}carbonyl)-4-oxo-3-[(E)-2-(2H-1,2,3,4-tetrazol-5-yl)-1-ethenyl]-4H-pyrido[1,2-a]pyrimidin-2-yl}hexahydro-3-pyridinyl)oxy]carbonyl}amino)ethyl](trimethyl)ammonium

### (A) (3R)-1-(8-({[4-(tert-Butyl)-1,3-thiazol-2-yl]amino}carbonyl)-3-{(E)-2-[2-(4-methoxybenzyl)-2H-1,2,3,4-tetrazol-5-yl]-1-ethenyl}-4-oxo-4H-pyrido[1,2-a]-pyrimidin-2-yl)hexahydro-3-pyridinyl N-[2-(dimethylamino)ethyl]carbamate

(3R)-1-(8-({[4-(tert-Butyl)-1,3-thiazol-2-yl]amino}carbonyl)-3-{(E)-2-[2-(4-methoxybenzyl)-2H-1,2,3,4-tetrazol-5-yl]-1-ethenyl}-4-oxo-4H-pyrido[1,2-a]pyrimidin-2-yl)hexahydro-3-pyridinyl N-(2-chloroethyl)carbamate (400 mg, 0.54 mmol) was added with N,N-dimethylamine solution in tetrahydrofuran (2.0 M, 60 ml) and heated to 100°C in a sealed tube with stirring for 12 hours. After completion of the reaction, the reaction solution was returned to room temperature. The solvent was concentrated under reduced pressure, and the resulting crude product was purified by thin layer silica gel column chromatography (chloroform:methanol = 10:1) to obtain the title compound (190 mg, 47%).
¹H-NMR (CDCl₃) δ : 1.34 (9H, s), 1.65 (1H, brd), 1.92 (3H, brd), 2.25 (6H, s), 2.52 (2H, t, J=6.59Hz), 3.33 (2H, brd), 3.73 (4H, q, J=7.08Hz), 3.78 (3H, s), 4.86 (1H, s), 5.51 (2H, s), 6.04 (1H, brd), 6.59 (1H, s), 6.89 (2H, d, J=8.79Hz), 7.35 (2H, d, J=8.79Hz), 7.49 (1H, d, J=7.32Hz), 7.88 (2H, q, J=11.74Hz), 7.92 (1H, s), 8.96 (1H, d, J=7.32Hz).
EI-MS; m/s: 756 (M⁺+1).

### (B) [2-({[((3R)-1-{8-({[4-(tert-Butyl)-1,3-thiazol-2-yl]amino}carbonyl)-4-oxo-3-{(E)-2-[2-(4-methoxybenzyl)-2H-1,2,3,4-tetrazol-5-yl]-1-ethenyl}-4H-pyrido[1,2-a]pyrimidin-2-yl}hexahydro-3-pyridinyl)oxy)carbonyl}amino)ethyl](trimethyl)ammonium

Reactions were performed in the same manner as in Example 237, (D) by using (3R)-1-(8-({[4-(tert-butyl)-1,3-thiazol-2-yl]amino}carbonyl)-3-{(E)-2-[2-(4-methoxybenzyl)-2H- 1,2,3,4-tetrazol-5-yl]-1-ethenyl}-4-oxo-4H-pyrido[1,2-a]pyrimidin-2-yl)hexahydro-3-pyridinyl N-[2-(dimethylamino)ethyl]carbamate (90 mg, 0.12 mmol), and the resulting crude product was purified by thin layer silica gel column chromatography (lower layer of chloroform:methanol:water = 8:3:1) to obtain the title compound (87 mg, 95%).
¹H-NMR (CDCl₃) δ : 1.36 (9H, s), 1.67 (1H, brd), 1.81 (2H, brd), 2.14 (1H, brd), 3.15 (1H, brd), 3.33 (1H, d, J=12.94Hz), 3.50 (9H, s), 3.73 (4H, q, J=7.08Hz), 3.78 (3H, s), 3.89 (2H, brd), 4.78 (1H, s), 5.51 (2H, s), 6.60 (1H, s), 6.91 (2H, d, J=8.55Hz), 7.36 (2H, d, J=8.55Hz), 7.47 (1H, brd), 7.89 (1H, s), 7.99 (2H, s), 8.95 (1H, brd).
EI-MS; m/s: 770 (M⁺).

### (C) [2-({[((3R)-1-{8-({[4-(tert-Butyl)-1,3-thiazol-2-yl]amino}carbonyl)-4-oxo-3-[(E)-2-(2H-1,2,3,4-tetrazol-5-yl)-1-ethenyl]-4H-pyrido[1,2-a]pyrimidin-2-yl}hexahydro-3-pyridinyl)oxy]carbonyl}amino)ethyl](trimethyl)ammonium

Reactions were performed in the same manner as in Example 239, (D) by using [2-({[((3R)-1-{8-({[4-(tert-butyl)-1,3-thiazol-2-yl]amino}carbonyl)-4-oxo-3-{(E)-2-[2-(4-methoxybenzyl)-2H-1,2,3,4-tetrazol-5-yl]-1-ethenyl}-4H-pyrido[1,2-a]pyrimidin-2-yl}hexahydro-3-pyridinyl)oxy]carbonyl}amino)ethyl](trimethyl)ammonium (87 mg, 0.11 mmol), and the resulting crude product was purified by thin layer silica gel column chromatography (lower layer of chloroform:methanol:water = 8:3:1) to obtain the title compound (52 mg, 71%).
¹H-NMR (CD₃OD) δ : 1.35 (9H, s), 1.70 (1H, brd), 1.97 (3H, brd), 3.16 (9H, s), 3.42 (3H, m), 3.55 (3H, m), 3.93 (2H, m), 4.89 (1H, s), 6.75 (1H, s), 7.56 (1H, d, J=16.14Hz), 7.60 (1H, dd, J=7.34, 1.22Hz), 7.96 (1H, d, J=16.14Hz), 8.03 (1H, s), 8.99 (1H, d, J=7.34Hz). EI-MS; m/s: 650 (M⁺).

### Example 247: [2-({[((3S)-1-{8-({[4-(tert-Butyl)-1,3-thiazol-2-yl]amino}carbonyl)-4-oxo-3-[(E)-2-(2H-1,2,3,4-tetrazol-5-yl)-1-ethenyl]-4H-pyrido[1,2-a]pyrimidin-2-yl}hexahydro-3-pyridinyl}oxy}carbonyl}amino}ethyl}(trimethyl)ammonium

### (A) N⁸-[4-(tert-Butyl)-1,3-thiazol-2-yl]-2-[(3S)-3-hydroxyhexahydro-1-pyridinyl]-3-{(E)-2-[2-(4-methoxybenzyl)-2H-1,2,3,4-tetrazol-5-yl]-1-ethenyl}-4-oxo-4H-pyrido[1,2-a]pyrimidine-8-carboxyamide

Reactions were performed in the same manner as in Example 243, (C) by using N⁸-[4-(tert-butyl)-1,3-thiazol-2-yl]-2-hydroxy-3-{(E)-2-[2-(4-methoxybenzyl)-2H-1,2,3,4-tetrazol-5-yl]-1-ethenyl}-4-oxo-4H-pyrido[1,2-a]pyrimidine-8-carboxamide (1 g, 1.8 mmol) and (S)-(-)-3-hydroxypiperidine hydrochloride (1.1 g, 8.1 mmol, 4.5 eq), and the resulting crude product was purified by silica gel column chromatography (chloroform → chloroform:methanol = 100:1 → 80:1 → 50:1) to obtain the title compound (1.3 g, 100%) with contained dimethylformamide.
¹H-NMR (CDCl₃) δ : 1.37 (9H, s), 1.487 (2H, m), 1.84 (2H, m), 3.32 (1H, m), 3.54 (2H, m), 3.64 (1H, m), 3.78 (3H, s), 4.07 (1H, s), 5.51 (2H, s), 6.59 (1H, s), 6.88 (2H, d, J=8.80Hz), 7.33 (2H, d, J=8:80Hz), 7.47 (1H, d, J=7.20Hz), 7.78 (2H, s), 8.00 (1H, brd), 8.86 (1H, d, J=7.20Hz).
EI-MS; m/s: 642 (M⁺+1)

### (B) (3S)-1-(8-({[4-(tert-Butyl)-1,3-thiazol-2-yl]amino}carbonyl)-3-{(E)-2-[2-(4-methoxybenzyl)-2H-1,2,3,4-tetrazol-5-yl]-1-ethenyl}-4-oxo-4H-pyrido[1,2-a]-pyrimidin-2-yl}hexahydro-3-pyridinyl N-(2-chloroethyl)carbamate

Reactions were performed in the same manner as in Example 9, (B) by using N⁸-[4-(tert-butyl)-1,3-thiazol-2-yl]-2-[(3S)-3-hydroxyhexahydro-1-pyridinyl]-3-{(E)-2-[2-(4-methoxybenzyl)-2H-1,2,3,4-tetrazol-5-yl]-1-ethenyl}-4-oxo-4H-pyrido[1,2-a]pyrimidine-8-carboxamide (1.3 g, 2.0 mmol), and the resulting crude product was purified by silica gel chromatography to obtain the title compound (1.03 g, 68%).
¹H-NMR (CDCl₃) δ : 1.35 (9H, s), 1.73 (1H, brd), 1.93 (3H, m), 3.35 (2H, m), 3.50 (2H, m), 3.60 (2H, m), 3.62 (2H, m), 3.78 (3H, s), 4.83 (1H, s), 5.51 (2H, s), 6.60 (1H, s), 6.88 (2H, d, J=8.57Hz), 7.34 (2H, d, J=8.57Hz), 7.52 (1H, d, J=7.35Hz), 7.96 (1H, s), 7.99 (2H, d, J=7.10Hz), 9.00 (1H, d, J=7.35Hz).
EI-MS; m/s: 747 (M⁺).

### (C) (3S)-1-(8-({[4-(tert-Butyl)-1,3-thiazol-2-yl]amino}carbonyl)-3-{(E)-2-[2-(4-methoxybenzyl)-2H-1,2,3,4-tetrazol-5-yl]-1-ethenyl}-4-oxo-4H-pyrido[1,2-a]-pyrimidin-2-yl)hexahydro-3-pyridinyl N-[2-(dimethylamino)ethyl]carbamate

Reactions were performed in the same manner as in Example 246, (A) by using (3S)-1-(8-({[4-(tert-butyl)-1,3-thiazol-2-yl]amino}carbonyl)-3-{(E)-2-[2-(4-methoxybenzyl)-2H-1,2,3,4-tetrazol-5-yl]-1-ethenyl}-4-oxo-4H-pyrido[1,2-a]pyrimidin-2-yl)hexahydro-3-pyridinyl N-(2-chloroethyl)carbamate (1.03 g, 1.4 mmol), and the resulting crude product was purified by silica gel column chromatography to obtain the title compound (580 mg, 43% for the three steps).
¹H-NMR (CDCl₃) δ: 1.35 (9H, s), 1.65 (1H, m), 1.89 (3H, m), 2.21 (6H, s), 2.50 (2H, m), 3.32 (2H, m), 3.66 (4H, m), 3.77 (3H, s), 4.85 (1H, brd), 5.50 (2H, s), 6.10 (1H, brd), 6.88 (1H, s), 7.28 (2H, d, J=8.79Hz), 7.34 (2H, d, J=8.79Hz), 7.47 (1H, d, J=7.32Hz), 7.85 (3H, m), 8.92 (1H, d, J=7.32Hz).
EI-MS; m/s: 756 (M⁺+1).

### (D) [2-({[((3S)-1-{(8-({[4-(tert-Butyl)-1,3-thiazol-2-yl]amino}carbonyl)-4-oxo-3-{(E)-2-[2-(4-methoxybenzyl)-2H-1,2,3,4-tetrazol-5-yl]-1-ethenyl}-4H-pyrido[1,2-a]pyrimidin-2-yl}hexahydro-3-pyridinyl)oxy]carbonyl}amino)ethyl](trimethyl)ammonium

Reactions were performed in the same manner as in Example 237, (D) by using (3S)-1-(8-({[4-(tert-butyl)-1,3-thiazol-2-yl]amino}carbonyl)-3-{(E)-2-[2-(4-methoxybenzyl)-2H-1,2,3,4-tetrazol-5-yl]-1-ethenyl}-4-oxo-4H-pyrido[1,2-a]pyrimidin-2-yl)hexahydro-3-pyridinyl N-[2-(dimethylamino)ethyl]carbamate (261 mg, 0.35 mmol), and the resulting crude product was purified by thin layer silica gel column chromatography (lower layer of chloroform:methanol:water = 8:3:1) to obtain the title compound (236 mg, 89%).
¹H-NMR (CD₃OD/CDCl₃) δ: 1.35 (9H, s), 1.74-2.18 (4H, m), 3.23-3.47 (2H, brd), 3.47 (9H, s), 3.68-3.92 (6H, m), 3.43 (3H, s), 4.81 (1H, brd), 5.54 (2H, s), 6.61 (1H, s), 6.91 (2H, d, J=8.82Hz), 7.36 (2H, d, J=8.82Hz), 7.56 (1H, dd, J=7.35, 1.96Hz), 8.00 (3H, brd), 9.02 (1H, d, J=7.35Hz).
EI-MS; m/s: 770 (M⁺).

### (E) [2-({[((3S)-1-{8-({[4-(tert-Butyl)-1,3-thiazol-2-yl]amino}carbonyl)-4-oxo-3-[(E)-2-(2H-1,2,3,4-tetrazol-5-yl)-1-ethenyl]-4H-pyrido[1,2-a]pyrimidin-2-yl}hexahydro-3-pyridinyl)oxy]carbonyl}amino)ethyl](trimethyl)ammonium

Reactions were performed in the same manner as in Example 239, (D) by using [2-({[((3S)-1-{8-({[4-(tert-butyl)-1,3-thiazol-2-yl]amino}carbonyl)-4-oxo-3-{(E)-2-[2-(4-methoxybenzyl)-2H-1,2,3,4-tetrazol-5-yl]-1-ethenyl]-4H-pyrido[1,2-a]pyrimidin-2-yl}hexahydro-3-pyridinyl)oxy]carbonyl}amino)ethyl] (trimethyl)-ammonium (236 mg, 0.31 mmol), and the resulting crude product was purified by thin layer silica gel column chromatography (lower layer of chloroform:methanol:water = 8:3:1) to obtain the title compound (196 mg, 98%).
¹H-NMR (CD₃OD) δ : 1.35 (9H, s), 1.69 (1H, brd), 1.92 (3H, brd), 3.18 (9H, s), 3.31 (2H, brd), 3.43 (2H, brd), 3.56 (2H, brd), 3.85 (2H, brd), 4.80 (1H, brd), 6.71 (1H, s), 7.49 (1H, d, J=16.36Hz), 7.55 (1H, brd), 7.90 (1H, d, J=16.36Hz), 7.95 (1H, brd), 8.92 (1H, brd).
ES-MS; m/s: 649 (M⁺).

### Example 248: (2-Amino-2-oxoethyll)[2-({[((3S)-1-{8-({[4-(tert-butyl)-1,3-thiazol-2-yl]-amino}-carbonyl)-4-oxo-3-[(E)-2-(2H-1,2,3,4-tetrazol-5-yl)-1-ethenyl]-4H-pyrido[1,2-a]pyrimidin-2-yl}hexahydro-3-pyridinyl)oxy]carbonyl}amino)ethyl]-dimethylammonium

### (A) (2-Amino-2-oxoethyl)(2-({[((3S)-1-{8-({[4-(tert-butyl)-1,3-thiazol-2-yl]amino}carbonyl)-4-oxo-3-{(E)-2-(2-(4-methoxybenzyl)-2H-1,2,3,4-tetrazol-5-yl]-1-ethenyl}-4H-pyrido[1,2-a]pyrimidin-2-yl}hexahydro-3-pyridinyl)oxy]carbonyl}amino)ethyl]-dimethylammonium

Reactions were performed in the same manner as in Example 241, (D) by using (3S)-1-(8-({[4-(tert-butyl)-1,3-thiazol-2-yl]amino}carbonyl)-3-{(E)-2-[2-(4-methoxybenzyl)-2H-1,2,3,4-tetrazol-5-yl]-1-ethenyl}-4-oxo-4H-pyrido[1,2-a]pyrimidin-2-yl)hexahydro-3-pyridinyl N-[2-(dimethylamino)ethyl]carbamate (320 mg, 0.42 mmol), and the resultant was used in the following reaction without being purified.
¹H-NMR (CD₃OD/CDCl₃) δ: 1.34 (9H, s), 1.76 (1H, brd), 2.09 (3H, brd), 3.20-3.35 (2H, m), 3.41 (3H, s), 3.46 (3H, s), 3.69-3.93 (6H, m), 3.79 (3H, s), 4.61 (2H, s), 4.84 (1H, brd), 5.54 (2H, s), 6.62 (1H, s), 6.90 (1H, d, J=8.57Hz), 7.36 (1H, d, J=8.57Hz), 7.56 (1H, dd, J=7.35, 1.72Hz), 8.01 (3H, m), 9.02 (1H, d, J=7.35Hz).
EI-MS; m/s: 813 (M⁺).

### (B) (2-Amino-2-oxoethyll)[2-({[((3S)-1-{8-({[4-(tert-butyl)-1,3-thiazol-2-yl]amino}carbonyl)-4-oxo-3-[(E)-2-(2H-1,2,3,4-tetrazol-5-yl)-1-ethenyl]-4H-pyrido[1,2-a)-pyrimidin-2-yl}hexahydro-3-pyridinyl)oxy]carbonyl}amino)ethyl]dimethylammonium

Reactions were performed in the same manner as in Example 239, (D) by using (2-amino-2-oxoethyl)[2-({[((3S)-1-{8-({[4-(tert-butyl)-1,3-thiazol-2-yl]amino}carbonyl)-4-oxo-3-{(E)-2-[2-(4-methoxybenzyl)-2H-1,2,3,4-tetrazol-5-yl]-1-ethenyl}-4H-pyrido[1,2-a]pyrimidin-2-yl}hexahydro-3-pyridinyl)oxy]carbonyl}amino)-ethyl]dimethylammonium (316 mg, 0.4 mmol), and the resulting crude product was purified by thin layer silica gel column chromatography (lower layer of chloroform:methanol:water = 8:3:1) to obtain the title compound (195 mg, 66% for the two steps).
¹H-NMR (CD₃OD) δ : 1.34 (9H, s), 1.68 (1H, brd), 1.92 (3H, brd), 3.34 (6H, s), 3.41 (1H, m), 3.55 (3H, m), 3.66 (2H, m), 3.86 (2H, m), 4.16 (2H, s), 4.86 (1H, brd), 6.72 (1H, s), 7.51 (1H, d, J=16.16Hz), 7.56 (1H, d, J=7.35Hz), 7.92 (1H, d, J=16.16Hz), 8.99 (1H, s), 8.95 (1H, d, J=7.35Hz).
ES-MS; m/s: 693 (M⁺)

### Example 249: 2-{1-[2-({[(1-{8-({[4-(tert-Butyl)-1,3-thiazol-2-yl]amino}carbonyl)-4-oxo-3-[(E)-2-(2H-1,2,3,4-tetrazol-5-yl)-1-ethenyl]-4H-pyrido[1,2-a]pyrimidin-2-yl}-4-piperidyl}oxy}carbonyl}amino}ethyl}-1,1-dimethylammonio}acetate

### (A) N⁸-[4-(tert-Butyl)-1,3-thiazol-2-yl]-2-(4-hydroxypiperidino)-3-{(E)-2-[2-(4-methoxybenzyl)-2H-1,2,3,4-tetrazol-5-yl]-1-ethenyl}-4-oxo-4H-pyrido[1,2-a]-pyrimidine-8-carboxyamide

Reactions were performed in the same manner as in Example 241, (A) by using N⁸-[4-(tert-butyl)-1,3-thiazol-2-yl]-2-hydroxy-3-{(E)-2-[2-(4-methoxybenzyl)-2H-1,2,3,4-tetrazol-5-yl]-1-ethenyl}-4-oxo-4H-pyrido[1,2-a]pyrimidine-8-carboxamide (500 mg, 0.9 mmol) and 4-hydroxypiperidine (5 mmol, 5.5 eq), and the obtained oily substance was purified by silica gel column chromatography (chloroform → chloroform:ethyl acetate = 3:1 → 1:1 → chloroform:methanol = 80:1 → 50:1 → 30:1 → 10:1) to obtain the title compound (578 mg, 100%) as a yellow oily substance with contained dimethylformamide.
¹H-NMR (CDCl₃) δ: 1.34 (9H, s), 1.41 (2H, brd), 1.81 (2H, brd), 3.00 (1H, brd), 3.49 (1H, m), 3.63 (1H, m), 3.78 (3H, s), 4.00 (1H, brd), 4.16 (1H, brd), 5.53 (2H, s), 6.59 (1H, s), 6.89 (2H, d, J=8.55Hz), 7.37 (2H, d, J=8.55Hz), 7.50 (1H, d, J=7.32Hz), 7.74 (1H, d, J=15.38Hz), 7.89 (1H, s), 7.91 (1H, d, J=15.38Hz), 8.95 (1H, d, J=7.32Hz).
EI-MS; m/s: 642 (M⁺+1).

### (B) 1-(8-({[4-(tert-Butyl)-1,3-thiazol-2-yl]amino}carbonyl)-3-{(E)-2-[2-(4-methoxybenzyl)-2H-1,2,3,4-tetrazol-5-yl]-1-ethenyl}-4-oxo-4H-pyrido[1,2-a]-pyrimidin-2-yl)-4-piperidyl-N-[2-(dimethylamino)ethyl]carbamate

N⁸-[4-(tert-Butyl)-1,3-thiazol-2-yl]-2-(4-hydroxypiperidino)-3-{(E)-2-[2-(4-methoxybenzyl)-2H-1,2,3,4-tetrazol-5-yl]-1-ethenyl}-4-oxo-4H-pyrido[1,2-a]pyrimidine-8-carboxamide (578 mg, 0.9 mmol) was added with dichloromethane (9 ml), N,N-dimethylethylenediamine (0.4 ml, 3.6 mmol, 4 eq) and 1,1'-carbonyl-bis-1H-imidazole (220 mg, 1.4 mmol, 1.5 eq) at room temperature and stirred at the same temperature for 1 hour. The reaction mixture was added with 1,1'-carbonyl-bis-1H-imidazole (100 mg, 0.7 mmol, 0.75 eq), after 30 minutes, further added with N,N-dimethylethylenediamine (0.4 ml, 3.6 mmol, 4 eq) and stirred for 15 hours. After completion of the reaction, the reaction mixture was added with 1 N hydrochloric acid and extracted with chloroform:methanol = 10:1. The organic layer was neutralized by adding saturated aqueous sodium hydrogencarbonate, and the collected organic layer was dried over magnesium sulfate. The solvent was concentrated under reduced pressure, and the resulting crude product was purified by silica gel column chromatography (chloroform → chloroform:methanol = 60:1 → 30:1 → 10:1) to obtain the title compound (354 mg, 52% for the two steps).
¹H-NMR (CDCl₃) δ : 1.40 (9H, s), 1.53 (1H, brd), 1.69 (1H, brd), 1.78 (1H, brd), 1.91 (1H, brd), 2.22 (6H, s), 2.42 (2H, m), 3.14 (1H, brd), 3.26 (2H, m), 3.39 (1H, brd), 3.51 (1H, brd), 3.74 (1H, m), 3.79 (3H, s), 4.85 (1H, brd), 5.35 (1H, brd), 5.53 (2H, s), 6.57 (1H, s), 6.92 (2H, d, J=8.56Hz), 7.23 (2H, d, J=8.56Hz), 7.38 (1H, d, J=8.56Hz), 7.76 (1H, d, J=15.41Hz), 7.82 (1H, s), 7.91 (1H, d, J=15.41Hz), 8.82 (1H, brd).
EI-MS; m/s: 756 (M⁺+1).

### (C) 2-(1-{2-[({[1-(8-({[4-(tert-Butyl)-1,3-thiazol-2-yl]amino}carbonyl)-3-{(E)-2-[2-(4-methoxybenzyl)-2H-1,2,3,4-tetrazol-5-yl]-1-ethenyl}-4-oxo-4H-pyrido[1,2-a]-pyrimidin-2-yl)-4-piperidyl]oxy}carbonyl)amino]ethyl}-1,1-dimethylammonio)acetate

1-(8-({[4-(tert-Butyl)-1,3-thiazol-2-yl]amino}carbonyl)-3-{(E)-2-[2-(4-methoxybenzyl)-2H-1,2,3,4-tetrazol-5-yl]-1-ethenyl}-4-oxo-4H-pyrido[1,2-a]pyrimidin-2-yl)-4-piperidyl-N-[2-(dimethylamino)ethyl]carbamate (495 mg, 0.7 mmol) was added with N,N-dimethylformamide (10 ml) and bromoacetic acid tert-butyl ester (106 µl, 0.8 mmol, 1.1 eq), stirred at room temperature for 2 hours, further added with bromoacetic acid tert-butyl ester (50 µl, 0.4 mmol, 0.5 eq) and further stirred at the same temperature for 1 hour. After completion of the reaction, the solvent was evaporated under reduced pressure, and the residue was added with 4 N hydrochloric acid in dioxane (10 ml) and stirred at room temperature for 14 hours and 30 minutes. After the solvent was concentrated under reduced pressure, the reaction mixture was used in the following reaction without being purified.
ES-MS; m/s: 814 (M⁺).

### (D) 2-{1-[2-({[(1-{8-({[4-(tert-Butyl)-1,3-thiazol-2-yl]amino}carbonyl)-4-oxo-3-[(E)-2-(2H-1,2,3,4-tetrazol-5-yl)-1-ethenyl]-4H-pyrido[1,2-a]pyrimidin-2-yl}-4-piperidyl)-oxy]carbonyl}amino)ethyl]-1,1-dimethylammonio}acetate

2-(1-{2-[({[1-(8-({[4-(tert-Butyl)-1,3-thiazol-2-yl]amino}carbonyl)-3-{(E)-2-[2-(4-methoxybenzyl)-2H-1,2,3,4-tetrazol-5-yl]-1-ethenyl}-4-oxo-4H-pyrido[1,2-a]pyrimidin-2-yl)-4-piperidyl]oxy}carbonyl)amino]ethyl}-1,1-dimethylammonio)acetate (557 mg, 0.7 mmol) was added with trifluoroacetic acid (25 ml) and anisole (50 µl) and heated at 60°C with stirring for 3 hours. After completion of the reaction, the solvent was concentrated under reduced pressure, and the resulting crude product was purified by liquid chromatography (methanol:water = 60:40, flow rate: 10 cc/min) to obtain the title compound (122 mg, 27% for the three steps).
¹H-NMR (CD₃OD) δ : 1.35 (9H, s), 1.87 (2H, brd), 2.07 (2H, brd), 3.29 (6H, s), 3.57 (4H, m), 3.73 (2H, m), 3.87 (4H, m), 4.91 (1H, brd), 6.70 (1H, s), 7.40 (1H, d, J=16.11Hz), 7.64 (1H, dd, J=7.32, 1.71Hz), 7.93 (1H, d, J=16.11Hz), 8.04 (1H, s), 8.96 (1H, d, J=7.32Hz).
ES-MS; m/s: 694 (M⁺+1).

### Example 250: (E)-3-[2-{4-{2-(2-Aminothiazol-5-yl)-3-pyridin-4-yl-acryloyl]piperazin-1-yl}-8-({[4-(tert-butyl)-1,3-thiazol-2-yl]amino}carbonyl)-4-oxo-4H-pyrido[1,2-a]-pyrimidin-3-yl]-2-propenoic acid

### (A) (E)-2-(2-tert-Butoxycarbonylaminothiazol-5-yl)-3-pyridin-4-yl-2-propenoic acid

A solution of (E)-2-(2-tert-butoxycarbonylaminothiazol-5-yl)-3-pyridin-4-yl-2-propenoic acid ethyl ester (1.13 g, 3.02 mmol) in tetrahydrofuran (6 ml), methanol (2 mL) and water (2 mL) was added with lithium hydroxide monohydrate (190 mg, 4.53 mmol) with ice cooling and stirred at room temperature for 21 hours, at 50°C for 8 hours and at 80°C for 16 hours. The solvent was concentrated under reduced pressure and the residue was diluted with 10% aqueous citric acid and extracted with ethyl acetate. The organic layer was washed with saturated brine and dried over anhydrous magnesium sulfate. Unpurified title compound (330 mg) was obtained as yellow oily substance and used in the following reaction without being purified.

### (B) (E)-3-[2-{4-[2-(2-tert-Butoxycarbonylaminothiazol-5-yl)-3-pyridin4-yl-acryloyl]-piperazin-1-yl}-8-({[4-(tert-butyl)-1,3-thiazol-2-yl]amino}carbonyl)-4-oxo-4H-pyrido[1,2-a]pyrimidin-3-yl}-2-propenoic acid tert-butyl ester

A solution of N⁸-[4-(tert-butyl)-1,3-thiazol-2-yl]-3-{(E)-2-[1-(4-methoxybenzyl)-1H-1,2,3,4-tetrazol-5-yl]-1-ethenyl}-4-oxo-2-piperazino-4H-pyrido[1,2-a]pyrimidine-8-carboxamide (297 mg) and (E)-2-(2-tert-butoxycarbonyl aminothiazole-5-yl)-3-pyridin-4-yl-2-propenoic acid (180 mg) in N,N-dimethylformamide (2 ml) and methylenechloride (2 ml) was added with a solution of WSCI·HCl (150 mg, 0.780 mmol) in methylene chloride (6 ml) with ice cooling. The reaction mixture was stirred at room temperature for 20 hours, and then the solvent was concentrated under reduced pressure. The residue was purified by preparative TLC (4 plates, dichloromethane:methanol = 9:1), preparative TLC (3 plates, ethyl acetate) and preparative TLC (1 plate, dichloromethane:methanol = 9:1) to obtain the title compound (27.1 mg) as orange foamy syrup.
¹H-NMR (CDCl₃) δ : 1.33 (9H, s), 1.49 (9H, s), 1.56 (9H, s), 2.96-3.10 (1H, m), 3.24-4.01 (7H, m), 6.58 (1H, s), 6.94 (1H, s), 7.08 (1H, d, J=15.7Hz), 7.22-7.40 (5H, m), 7.49 (1H, d, J=7.3Hz), 7.85 (1H, s), 8.60-8.82 (3H, m), 8.94 (1H, d, J=6.8Hz).

### (C) (E)-3-[2-{4-[2-(2-Aminothiazol-5-yl)-3-pyridin-4-yl-acryloyl]piperazin-1-yl}-8-({[4-(tert-butyl)-1,3-thiazol-2-yl]amino}carbonyl)-4-oxo-4H-pyrido[1,2-a]pyrimidin-3-yl]-2-propenoic acid

(E)-3-[2-{4-[2-(2-tert-Butoxycarbonylaminothiazol-5-yl)-3-pyridin-4-yl-acryloyl]piperazin-1-yl}-8-({[4-(tert-butyl)-1,3-thiazol-2-yl]amino}carbonyl)-4-oxo-4H-pyrido[1,2-a]pyrimidin-3-yl]-2-propenoic acid tert-butyl ester (27.1 mg, 31.2 µmol) was dissolved in 4 N solution of hydrochloric acid in dioxane (1 ml) and stirred at room temperature for 4 hours. The reaction mixture was subjected to azeotropy with toluene and concentrated, and the residue was dissolved in methylene chloride (2 ml) again, added with trifluoroacetic acid (2 ml) and stirred at room temperature for 8 hours. The reaction mixture was subjected to azeotropy with toluene and dried under reduced pressure, and the residue was added with ether. The precipitated solid was collected by filtration and dried at 40°C under reduced pressure to obtain the title compound (23.4 mg) as orange solid.
¹H-NMR (DMSO-d₆) δ: 1.31 (9H, s), 3.27-4.00 (8H, m), 6.88 (1H, s), 6.95 (1H, d, J=15.7Hz), 7.01 (1H, s), 7.34 (1H, s), 7.42 (1H, d, J=15.7Hz), 7.66 (1H, d, J=7.6Hz), 7.87 (2H, d, J=6.4Hz), 8.17 (1H, s), 8.78 (2H, d, J=6.4Hz), 8.94 (1H, d, J=7.3Hz).
IR (ATR) cm⁻¹: 2968, 1678, 1633, 1593, 1520, 1439, 1365, 1284, 1232, 1201, 1141, 1099, 1065, 1003.
ES-MS: m/z: 712 (MH⁺).
Anal. Calcd. for C₃₄H₃₃N₉O₅S₂·4HCl·2H₂O: C, 45.44, ; H, 4.77, ; N, 14.10.
Found: C, 46.11, ; H, 4.85, ; N, 12.46.

### Example 251: (E)-3-[2-{4-[2-(2-Amino-1-methyl-1H-imidazol4-yl)-2-oxo-acetyl]-piperazin-1-yl}-8-({[4-(tert-butyl)-1,3-thiazol-2-yl]amino}carbonyl)-4-oxo-4H-pyrido[1,2-a]pyrimidin-3-yl]-2-propenoic acid

### (A) (E)-3-[8-({[4-(tert-Butyl)-1,3-thiazol-2-yl]amino}carbonyl)-2-(4-{2-[1-methyl-2-(tritylamino)-1H-imidazol-4-yl]-2-oxo-acetyl}piperazin-1-yl)-4-oxo-4H-pyrido[1,2-a]-pyrimidin-3-yl]-2-propenoic acid tert-butyl ester

A solution of N⁸-[4-(tert-butyl)-1,3-thiazol-2-yl]-3-((E)-2-[1-(4-methoxybenzyl)-1H-1,2,3,4-tetrazol-5-yl]-1-ethenyl}-4-oxo-2-piperazino-4H-pyrido[1,2 a]pyrimidine-8-carboxamide (456 mg) and [1-methyl-2-(tritylamino)-1H-imidazol-4-yl]-oxo-acetic acid (329 mg, 0.798 mmol) in N,N-dimethylformamide (2 ml) and methylene chloride (2 ml) was added with a solution of WSCI•HCI (230 mg, 1.20 mmol) in methylene chloride (6 ml) with ice cooling and stirred at room temperature for 20 hours, and the solvent was concentrated under reduced pressure. The residue was purified by silica gel column chromatography (20 g, chloroform:methanol = 99:1 → 98:2) to obtain the title compound (96.5 mg) as orange foamy syrup.
¹H-NMR (CDCl₃) δ : 1.32 (9H, s), 1.51 (9H, s), 3.15 (5H, s), 3.27 (2H, s), 3.52 (2H, s), 3.65 (2H, s), 4.97 (1H, br), 6.60 (1H, s), 7.09-7.44 (19Hm), 7.91 (1H, s), 8.91-8.95 (1H, m).

### (B) (E)-3-[2-{4-[2-(2-Amino-1-methyl-1H-imidazol-4-yl)-2-oxo-acetyl]piperazin-1-yl}-8-({[4-(tert-butyl)-1,3-thiazol-2-yl]amino}carbonyl)-4-oxo-4H-pyrido[1,2-a]pyrimidin-3-yl]-2-propenoic acid

A solution of (E)-3-[8-({[4-(tert-butyl)-1,3-thiazol-2-yl]amino}carbonyl)-2-(4-{2-[1-methyl-2-(tritylamino)-1H-imidazol-4-yl]-2-oxo-acetyl}piperazin-1-yl)-4-oxo-4 H-pyrido[1,2-a]pyrimidin-3-yl]-2-propenoic acid tert-butyl ester (96.5 mg) in dichloromethane (2 ml) was added with trifluoroacetic acid (2 ml) with ice cooling and stirred at room temperature for 4 hours. The reaction mixture was concentrated under reduced pressure and subjected to azeotropy with 4 N solution of hydrochloric acid in dioxane and toluene. The residue was added with ether, and the precipitated solid was collected by filtration and dried at 40°C under reduced pressure to obtain the title compound (70.4 mg) as orange solid.
¹H-NMR (DMSO-d₆) δ : 1.30 (9H, s); 3.15-3.80 (11H, m), 6.87 (1H, br), 6.96 (1H, d, J=15.6Hz), 7.46 (1H, d, J=15.4Hz), 7.67 (1H, d, J=7.3Hz), 8.10-8.28 (4H, m), 8.95 (1H, d, J=7.3Hz).
IR (ATR) cm⁻¹: 2966, 1678, 1639, 1593, 1516, 1440, 1363, 1282, 1234, 1173, 1099, 1065, 1007.
ES-MS m/z: 634 (MH⁺).
m.p.: 210-216°C.
Anal. Calcd. for C₂₉H₃₁N₉O₆S·2HCl·1.5H₂O: C, 47.48, ; H, 4.95; N, 17.18.
Found: C, 47.66; H, 5.15; N, 16.35.

### Example 252: Carbamic acid 1-{8-({[4-(tert-butyl)-1,3-thiazol-2-yl]amino}carbonyl)-3-[(E)-2-(2-dimethylaminoethylaminocarbonyl)vinyl]-4-oxo-4H-pyrido[1,2-a]pyrimidin-2-yl}-(R)-piperidin-3-yl-ester

### (A) (R)-N-tert-Butoxycarbonyl-3-hydroxypiperidine

A solution of (R)-3-hydroxypiperidine (5.00 g) in 1,4-dioxane (100 ml) and saturated aqueous sodium hydrogencarbonate (100 ml) was added with (Boc)₂O (7.93 g) with ice cooling and stirred at room temperature for 15 hours. The solvent was concentrated under reduced pressure, and the residue was diluted with 10% aqueous citric acid and extracted with methylene chloride. The organic layer was dried over anhydrous magnesium sulfate, and the solvent was concentrated under reduced pressure to obtain unpurified title compound (7.58 g). The compound was used in the following reaction without being further purified.
¹H-NMR (CDCl₃) δ: 1.46 (9H, s), 1.49-1.93 (5H, m), 3.04-3.17 (2H, m), 3.51 (1H, br), 3.68-3.76 (2H, m).

### (B) (R)-N-tert-Butoxycarbonyl-3-aminocarbonyloxypiperidine

A solution of (R)-N-tert-butoxycarbonyl-3-hydroxypiperidine (2.00 g) in ethyl acetate (40 ml) was added with trichloroacetyl isocyanate (1.18 ml) with ice cooling and stirred with ice cooling for 30 minutes. The reaction mixture was further added with trichloroacetyl isocyanate (0.592 ml) with ice cooling and stirred with ice cooling for 30 minutes. The solvent was concentrated under reduced pressure, and a suspension of the residue in methanol (40 ml) and water (8 ml) was added with sodium formate (2.03 g) and stirred at room temperature for 15 hours. The reaction mixture was further added with sodium formate (2.03 g), stirred at room temperature for 4 hours, added with sodium formate (2.03 g), stirred at room temperature for 2 hours, added with sodium formate (2.03 g), and stirred at room temperature for 18 hours. The solvent was concentrated under reduced pressure, and the residue was diluted with ethyl acetate. The organic layer was washed with 1 N hydrochloric acid, saturated aqueous sodium hydrogencarbonate and saturated brine and dried over anhydrous magnesium sulfate. The solvent was concentrated under reduced pressure, and the residue was purified by silica gel column chromatography (100 g, hexane:ethyl acetate = 2:1 → 1:1) to obtain the title compound (2.34 g)
¹H-NMR (CDCl₃) δ : 1.46 (9H, s), 1.47-1.92 (4H, m), 3.25-3.60 (4H, m), 4.53-4.71 (3H, m).

### (C) (R)-3-Aminocarbonyloxypiperidine hydrochloride

(R)-N-tert-Butoxycarbonyl-3-aminocarbonyloxypiperidine (2.34 g) was dissolved in 4 N solution of hydrochloric acid in dioxane (10 ml) with ice cooling and stirred at room temperature for 2 hours. The solvent was removed by azeotropy with toluene, and the residue was dried over under reduced pressure to obtain unpurified title compound (2.27 g). This compound was used in the following reaction without being further purified.
¹H-NMR (400MHz, DMSO-d₆) δ : 1.51-1.94 (4H, m), 2.81-3.27 (4H, m), 4.69-4.78 (1H, m), 6.59 (2H, s), 9.11-9.58 (2H, m).

### (D) (E)-3-[8-({[4-(tert-Butyl)-1,3-thiazol-2-yl]amino}carbonyl)-2-((R)-3-aminocarbonyloxypiperidin-1-yl)-4-oxo-4H-pyrido[1,2-a]pyrimidin-3-yl] -2-propenoic acid tert-butyl ester

A solution of tert-Butyl (E)-3-[B-({[4-(tert-butyl)-1,3-thiazol-2-yl]amino}carbonyl)-2-hydroxy-4-oxo-4H-pyrido[1,2-a]pyrimidin-3-yl]-2-propenoate (1.50 g) in N,N-dimethylformamide (100 ml) was added with dimethylaminopyridine (585 mg) and p-toluenesulfonyl chloride (913 mg) and stirred at room temperature for 2 hours. The reaction mixture was further added with triethylamine (2.22 ml) and (R)-3-aminocarbonyloxypiperidine hydrochloride (2.27 g) and stirred at room temperature for 22 hours. The solvent was concentrated under produced pressure, and the residue was purified by silica gel column chromatography (250 g, chloroform → chloroform:methanol = 89:1 → 98:2 → 97:3 → 96:4, and 250 g, chloroform → chloroform:methanol = 99:1 → 95: 5) to obtain the title compound (1.69 g) as orange solid.
¹H-NMR (CDCl₃) δ : 1.29 (9H, d, J=3.9Hz), 1.46 (9H, d, J=3.7Hz), 1.66 (2H, m), 1.83-2.05 (2H, m), 3.41-3.62 (2H, m), 3.84-3.95 (1H, m), 4.49-4.66 (2H, m), 6.47 (2H, br), 6.83-6.95 (1H, m), 7.33-7.47 (1H, m), 7.54-7.63 (1H, m), 7.94 (1H, s), 8.19 (1H, s), 8.82-8.91 (1H, m), 13.03 (1H, br).

### (E) (E)-3-[8-({[4-(tert-Butyl)-1,3-thiazol-2-yl]amino}carbonyl)-2-((R)-3-aminocarbonyloxypiperidin-1-yl)-4-oxo-4H-pyrido[1,2-a]pyrimidin-3-yl]-2-propenoic acid hydrochloride

A solution of (E)-3-[8-({[4-(tert-butyl)-1,3-thiazol-2-yl]amino}carbonyl)-2-((R)-3-aminocarbonyloxypiperidin-1-yl)-4-oxo-4H-pyrido[1,2-a]pyrimidin-3-yl]-2-propenoic acid tert-butyl ester (1.69 g) in methylene chloride (10 ml) was added with TFA (10 ml) with ice cooling and stirred at room temperature for 2 hours. The solvent was concentrated under reduced pressure and the reaction mixture was subjected to azeotropy with 4 N solution of hydrochloric acid in dioxane. The residue was added with ether, and the precipitated solid was collected by filtration, washed with ether and dried at 40°C over under reduced pressure to obtain the title compound (1.26 g) as orange solid.
¹H-NMR (DMSO-d₆) δ : 1.31 (9H, s), 1.58-2.05 (4H, m), 2.72-2.93 (1H, m), 3.49-3.96 (3H, m), 4.52-4.68 (1H, m), 6.44 (2H, br), 6.87 (1H, s), 6.93 (1H, d, J=15.4Hz), 7.44 (1H, s), 7.47 (1H, d, J=8.1Hz), 7.59-7.63 (2H, m), 8.19 (1H, s), 8.90 (1H, d, J=7.3Hz).

### (F) Carbamic acid 1-{8-({[4-(tert-butyl)-1,3-thiazol-2-yl]amino}carbonyl)-3-[(E)-2-(2-dimethylaminoethylaminocarbonyl)vinyl]-4-oxo-4H-pyrido[1,2-a]pyrimidin-2-yl}-(R)-piperidin-3-yl-ester

A solution of (E)-3-[8-({[4-(tert-butyl)-1,3-thiazol-2-yl]amino}carbonyl)-2-((R)-3-aminocarbonyloxypiperidin-1-yl)-4-oxo-4H-pyrido[1,2-a]pyrimidin-3-yl]-2-propenoic acid hydrochloride (100 mg) and N,N-dimethylethylenediamine (20.9 µl) in methylene chloride (2 ml) and DMF (1 ml) was added with diisopropylethylamine (75.5 *µ*l), HOBt (25.8 mg) and WSCD • HCl (36.5 mg) with ice cooling and stirred at room temperature for 26 hours. The solvent was concentrated under reduced pressure, and the residue was purified by preparative TLC (3 plates, lower layer of chloroform:methanol:water = 8:3:1) and preparative TLC (2 plates, lower layer of chloroform:methanol:water = 8:3:1) to obtain the title compound (75.3 mg) as orange solid.
¹H-NMR (DMSO-d₆) δ : 1.31 (9H, s), 1.61-1.76 (2H, m), 1.83-2.03 (2H, m), 2.24 (6H, s), 2.39-2.48 (2H, m), 3.15-3.58 (6H, m), 3.83-3.91 (1H, m), 4.57-4.67 (1H, m), 6.48 (1H, br), 6.85 (1H, s), 7.17 (1H, d, J=15.4Hz), 7.22-7.30 (1H, m), 7.34 (1H, d, J=15.2Hz), 7.54 (0.5H, d, J=7.8Hz), 7.61 (1H, dd, J=1.7, 7.3Hz), 7.79 (0.5H, d, J=7.8Hz), 8.06 (1H, t, J=5.6Hz), 8.20 (1H, s), 8.89 (1H, d, J=7.3Hz).
ES-MS: m/z: 612 (MH⁺).
Anal. Calcd. for C₂₉H₃₈N₈O₅S•1.7H₂O: C, 54.31; H, 6.51; N, 17.47.
Found: C, 54.12; H, 6.34; N, 17.86.

### Example 253: 2-[4-(3-Aminopropionyl)-piperazin-1-yl]-4-oxo-3-[(E)-2-(1-tetrazol-5-yl)vinyl]-4H-pyrido[1,2-a]pyrimidine-8-carboxylic acid (4-tert-butylthiazol-2-yl)amide

### (A) 3-{(E)-2-[1-(4-methoxybenzyl)-1H-tetrazol-5-yl]vinyl}-4-oxo-2-piperazin-1-yl-4H-pyrido[1,2-a]pyrimidine-8-carboxylic acid (4-tert-butylthiazol-2-yl)amide

tert-Butyl 4-(8-({[4-(tert-butyl)-1,3-thiazol-2-yl]amino}carbonyl)-3-{(E)-2- [1-(4-methoxybenzyl)-1H-1,2,3,4-tetrazol-5-yl]-1-ethenyl}-4-oxo-4H-pyrido[1,2-a]pyrimidin-2-yl)-1-piperazine carboxylate (100 mg) was dissolved in formic acid (2 ml) and stirred at room temperature for 1 hour and 30 minutes. The solvent was concentrated under reduced pressure by azeotropy with chloroform, and the residue was diluted with saturated aqueous sodium hydrogencarbonate and extracted with chloroform. The organic layer was dried over anhydrous magnesium sulfate, and the solvent was concentrated under reduced pressure to obtain unpurified title compound (76.6 mg). This compound was used in the following reaction without being further purified.
¹H-NMR (DMSO-d₆) δ : 1.29 (9H, s), 2.91 (4H, m), 3.54 (4H, br), 5.63 (2H, s), 6.77 (1H, s), 6.94 (1H, d, J=8.5Hz), 7.26 (1H, d, J=8.8Hz), 7.64 (1H, d, J=15.6Hz), 7.66-7.68 (1H, m), 7.76 (1H, d, J=16.6Hz), 8.16 (1H, m), 8.93 (1H, d, J=7.3Hz).

### (B) {3-[4-(8-({[4-(tert-Butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)piperazin-1-yl]-3-oxopropyl}carbamic acid tert-butyl ester

A solution of 3-{(E)-2-[1-(4-methoxybenzyl)-1H-tetrazol-5-yl]vinyl}-4-oxo-2-piperazin-1-yl-4H-pyrido[1,2-a]pyrimidine-8-carboxylic acid (4-tert-butylthiazol-2-yl)amide (76.6 mg) and Boc- β -alanine (25.4 mg) in methylene chloride (2 ml) and N,N-dimethylformamide (2 ml) was added with WSCD · HCl (25.8 mg) with ice cooling and stirred at room temperature for 17 hours. The solvent was concentrated under reduced pressure, and the residue was diluted with chloroform and washed with 10% aqueous citric acid, saturated aqueous sodium hydrogencarbonate and saturated brine. The organic layer was dried over anhydrous magnesium sulfate and concentrated under reduced pressure. The residue was purified by preparative TLC (chloroform:methanol = 40:1, developed twice) to obtain the title compound (64.7 mg). ¹H-NMR (CDCl₃) δ : 1.42 (9H, s), 1.44 (9H, s), 2.47-2.59 (2H, m), 3.36-3.69 (10H, m), 3.80 (3H, s), 5.28 (1H, br), 5.57 (2H, s), 6.56 (1H, s), 6.94 (2H, d, J=7.3Hz), 7.40 (3H, d, J=7.1Hz), 7.78 (1H, d, J=15.4Hz), 7.89 (1H, s), 7.95 (1H, d, J=15.4Hz), 8.86 (1H, br).

### (C) 2-[4-(3-Aminopropionyl)piperazin-1-yl]-3-{(E)-2-[1-(4-methoxybenzyl)-1H-tetrazol-5-yl]vinyl}-4-oxo-4H-pyrido[1,2-a]pyrimidine-8-carboxylic acid (4-tert-butylthiazol-2-yl)amide

{3-[4-(8-({[4-(tert-Butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)piperazine-1-yl]-3-oxopropyl}carbamic acid tert-butyl ester (64.7 mg) was dissolved in 4 N solution of hydrochloric acid in dioxane (5 ml) and stirred at room temperature for 30 minutes. The solvent was concentrated under reduced pressure by azeotropy with chloroform to obtain unpurified title compound (69.6 mg). This compound was used in the following reaction without being further purified.

### (D) 2-[4-(3-Aminopropionyl)-piperazin-1-yl]-4-oxo-3-[(E)-2-(1-tetrazol-5-yl)vinyl]-4H-pyrido[1,2-a]pyrimidine-8-carboxylic acid (4-tert-butylthiazol-2-yl)amide

2-[4-(3-Aminopropionyl)piperazin-1-yl]-3-{(E)-2-[1-(4-methoxybenzyl)-1H-tetrazol-5-yl]vinyl}-4-oxo-4H-pyrido[1,2-a]pyrimidine-8-carboxylic acid (4-tert-butyl-thiazol-2-yl)amide (69.6 mg) was dissolved in trifluoroacetic acid (2 ml) and stirred at 60°C for four hours. The solvent was concentrated under reduced pressure by azeotropy with chloroform. The residue was added with methanol, and the insoluble matters were removed by filtration. The residue was purified by HPLC to obtain the title compound (19.2 mg) as orange solid.'
¹H-NMR (DMSO-d₆) δ : 1.31 (9H, s), 2.72 (2H, t, J=5.9Hz), 3.04 (2H, t, J=5.9Hz), 3.50-3.77 (8H, m), 6.79 (1H, s), 7.28 (1H, d, J=15.9Hz), 7.68 (1H, d, J=7.3Hz), 7.86 (1H, d, J=15.9Hz), 8.18 (1H, s), 8.97 (1H, d, J=7.3Hz).
IR (ATR) cm⁻¹: 2964, 2866, 1657, 1626, 1514, 1431, 1371, 1313, 1227, 1144, 1103, 1068, 1018.
ES-MS: m/z: 578 (MH⁺).
Anal. Calcd. for C₂₆H₃₁N₁₁O₃S•3HCOOH•4H₂O: C, 44.21; H, 5.76; N, 19.56.
Found: C, 43.87; H, 5.18; N, 20.01.

In a similar manner, the following compounds were synthesized.

### Example 254: 2-[4-(4-Aminobutyryl)piperazin-1-yl]-4-oxo-3-[(E)-2-(1H-tetrazol-5-yl)vinyl]-4H-pyrido[1,2-a]pyrimidine-8-carboxylic acid (4-tert-butylthiazol-2-yl)amide

¹H-NMR (DMSO-d₆) δ: 1.31 (9H, s), 1.73-1.87 (2H, m), 2.45-2.58 (2H, m), 2.80-2.90 (2H, m), 3.49-3.75 (8H, m), 6.81 (1H, s), 7.28 (1H, d, J=15.9Hz), 7.67 (1H, d, J=7.3Hz), 7.86 (1H, d, J=15.9Hz), 8.19 (1H, s), 8.97 (1H, d, J=7.3Hz).
IR (ATR) cm⁻¹: 2962, 1658, 1620, 1516, 1431, 1365, 1311, 1225, 1132, 1103, 1066, 1016. ES-MS: m/z: 592 (MH⁺).
Anal. Calcd. for C₂₇H₃₃N₁₁O₃S•3HCOOH•2.5H₂O: C, 46.50; H, 5.72; N, 19.89.
Found: C, 46.41; H, 5.69; N, 19.46.

### Example 255: 2-[4-(5-Aminopentanoyl)piperazin-1-yl]-4-oxo-3-[(E)-2-(1H-tetrazol-5-yl)vinyl]-4H-pyrido[1,2-a]pyrimidine-8-carboxylic acid (4-tert-butylthiazol-2-yl)amide

¹H-NMR (DMSO-d₆) δ: 1.31 (9H, s), 1.59 (4H, br), 2.41 (2H, br), 2.81 (2H, br), 3.54 (4H, br), 3.67 (4H, br), 6.78 (1H, s), 7.27 (1H, d, J=16.3Hz), 7.68 (1H, d, J=7.6Hz), 7.86 (1H, d, J=16.3Hz), 8.18 (1H, s), 8.97 (1H, d, J=7.3Hz).
IR (ATR) cm⁻¹: 2964, 1658, 1618, 1516, 1429, 1365, 1311, 1254, 1225, 1206, 1132, 1103, 1066, 1016.
ES-MS: m/z: 606 (MH⁺).
Anal. Calcd. for C₂₈H₃₅N₁₁O₃S•3HCOOH•4H₂O: C, 45.63; H, 6.05; N, 18.89.
Found: C, 45.86; H, 5.95; N, 18.32.

### Example 256: 2-[4-(6-Aminohexanoyl)-piperazin-1-yl]-4-oxo-3-[(E)-2-(1H-tetrazol-5-yl)vinyl]-4H-pyrido[1,2-a]pyrimidine-8-carboxylic acid (4-tert-butylthiazol-2-yl)amide

¹H-NMR (400 MHz, DMSO-d₆) δ: 1.25-1.40 (m, 2H), 1.31 (9H, s), 1.50-1.61 (4H, m), 2.31-2.42 (2H, m), 2.76-2.85 (2H, m), 3.54 (4H, br), 3.66 (4H, br), 6.79 (1H, s), 7.27 (1H, d, J=16.2Hz), 7.67 (1H, dd, J=1.7, 7.3Hz), 7.85 (1H, d, J=16.2Hz), 8.19 (1H, s), 8.97 (1H, d, J=7.3Hz).
IR (ATR) cm⁻¹: 2958, 2864, 1658, 1618, 1516, 1427, 1365, 1311, 1250, 1225, 1134, 1103, 1068, 1018.
ES-MS: m/z: 620 (MH⁺).
Anal. Calcd. for C₂₉H₃₇N₁₁O₃S•3HCOOH•2.5H₂O: C, 47.86; H, 6.03; N, 19.19. Found: C, 47.76; H, 6.05; N, 19.12.

### Example 257: 2-[4-(2-Aminoacetyl)piperazin-1-yl]-4-oxo-3-[(E)-2-(1H-tetrazol-5-yl)vinyl]-4H-pyrido[1,2-a]pyrimidine-8-carboxylic acid (4-tert-butylthiazol-2-yl)amide

¹H-NMR (400 MHz, DMSO-d₆) δ : 1.31 (9H, s), 3.16-3.77 (8H, m), 3.93 (2H, s), 6.85 (1H, s), 7.32 (1H, d, J=16.0Hz), 7.68 (1H, d, J=7.3Hz), 7.89 (1H, d, J=16.0Hz), 8.20 (1H, s), 8.98 (1H, d, J=7.3Hz).
IR (ATR) cm⁻¹: 2960, 2359, 1658, 1514, 1431, 1371, 1309, 1244, 1203, 1134, 1103, 1065, 1013.
ES-MS: m/z: 564 (MH⁺).
Anal. Calcd. for C₂₅H₂₉N₁₁O₃S•3HCOOH•3H₂O: C44.50, H5.47, N20.39. Found: C44.99, H5.47, N20.75.

### Example 258: 2-{4-[2-(2-Aminoacetylamino)acetyl]piperazin-1-yl}-4-oxo-3-[(E)-2-(1H-tetrazol-5-yl)vinyl]-4H-pyrido[1,2-a]pyrimidine-8-carboxylic acid (4-tert-butylthiazol-2-yl)amide

¹H-NMR (DMSO-d₆) δ: 1.31 (9H, s), 3.07-3.72 (10H, m), 4.12 (2H, s), 6.86 (1H, s), 7.34 (1H, d, J=16.0Hz), 7.67 (1H, d, J=6.9Hz), 7.87 (1H, d, J=16.0Hz), 8.22 (1H, s), 8.50 (1H, br), 8.99 (1H, d, J=7.6Hz).
IR (ATR) cm⁻¹: 2962, 2866, 1657, 1637, 1516, 1431, 1365, 1227, 1205, 1103, 1066, 1016. ES-MS: m/z: 621 (MH⁺).
Anal. Calcd. for C₂₇H₃₂N₁₂O₄S•3HCOOH•2H₂O: C45.34, H5.33, N21.15. Found: C45.49, H5.51, N21.32.

### Example 259: {[2-(4-{8-({[4-(tert-Butyl)-1,3-thiazol-2-yl]amino}carbonyl)-4-oxo-3-[(E)-2-(1H-tetrazol-5-yl)vinyl]-4H-pyrido[1,2-a]pyrimidin-2-yl}piperazin-1-yl)-2-oxo-ethylaminocarbonyl]methyl}trimethylammonium

### (A) (2-Dimethylaminoacetylamino)acetic acid tert-butyl ester

A solution of glycine tert-butyl ester hydrochloride (1.63 g) and sarcosine (1.00 g) in methylene chloride (20 ml) and N,N-dimethylformamide (20 ml) was added with triethylamine (2.03 ml), HOBt (1.44 g) and WSCD•HCl (2.04 g) with ice cooling and stirred at room temperature for 14 hours. The solvent was concentrated under reduced pressure, and the residue was diluted with ethyl acetate and washed with 10% aqueous citric acid, saturated aqueous sodium hydrogencarbonate and saturated brine. The organic layer was dried over anhydrous magnesium sulfate and concentrated under reduced pressure. The residue was purified by silica gel column chromatography (chloroform → chloroform:methanol = 95:5) to obtain the title compound (2.08 g) as pale yellow oily substance.
¹H-NMR (CDCl₃) δ : 1.48 (9H, s), 2.33 (6H, s), 2.99 (2H, s), 3.97 (2H, d, J=5.6Hz), 7.57 (1H, br).

### (B) (2-Dimethylaminoacetylamino)acetic acid

(2-Dimethylaminoacetylamino)acetic acid tert-butyl ester (2.08 g) was dissolved in trifluoroacetic acid (10 ml) and stirred at room temperature for 4 hours. The solvent was concentrated under reduced pressure by azeotropy with chloroform and ethanol to obtain unpurified title compound (2.93 g). This compound was used in the following reaction without being further purified.
¹H-NMR (CDCl₃) δ : 2.94 (6H, d, J=2.2Hz), 4.00 (4H, d, J=2.0Hz).

### (C) 2-{4-[2-(2-Dimethylaminoacetylamino)acetyl]piperazin-1-yl}-3-{(E)-2-[1-(4-methoxybenzyl)-1H-tetrazol-5-yl]vinyl}-4-oxo-4H-pyrido[1,2-a]pyrimidine-8-carboxylic acid (4-tert-butylthiazol-2-yl)amide

A solution of N⁸-[4-(tert-butyl)-1,3-thiazol-2-yl]-3-{(E)-2-[1-(4-methoxybenzyl)-1H-1,2,3,4-tetrazol-5-yl]-1-ethenyl]-4-oxo-2-piperazino-4H-pyrido[1,2-a]pyrimidine-8-carboxamide (92.9 mg) and (2-dimethylaminoacetylamino)acetic acid (41.5 mg) in methylene chloride (4 ml) and N,N-dimethylformamide (1 ml) was added with triethylamine (48.0 µl), HOBt (20.5 mg) and WSCD•HCl ·(29.0 mg) with ice cooling and stirred at room temperature for 19 hours. The solvent was concentrated under reduced pressure, and the residue was purified by silica gel column chromatography (10 g, chloroform → chloroform:methanol = 99:1 → 98:2 → 95:5 → 90:10) and preparative TLC (chloroform: methanol = 10:1) to obtain the title compound (76.7 mg) as orange foamy syrup.
¹H-NMR (CDCl₃) δ : 1.39 (9H, s), 2.30 (6H, s), 3.00 (2H, s), 3.50-3.81 (8H, m), 3.80 (3H, d, J=0.5Hz), 4.16 (2H, d, J=4.6Hz), 5.56 (2H, s), 6.58 (1H, s), 6.92 (2H, d, J=8.7Hz), 7.38 (2H, d, J=8.7Hz), 7.49 (1H, d, J=7.6Hz), 7.77 (1H, d, J=15.5Hz), 7.96 (1H, d, J=15.5Hz), 7.95-8.03 (2H, m), 8.94 (1H, d, J=7.3Hz).

### (D) 2-{4-[2-(2-Dimethylaminoacetylamino)acetyl]piperazin-1-yl}-4-oxo-3-[(E)-2-(1H-tetrazol-5-yl)vinyl]-4H-pyrido[1,2-a]pyrimidine-8-carboxylic acid (4-tert-butylthiazol-2-yl)amide

2-{4-[2-(2-Dimethylaminoacetylamino)acetyl]piperazin-1-yl}-3-{(E)-2-[1-(4-methoxybenzyl)-1H-tetrazol-5-yl]vinyl}-4-oxo-4H-pyrido[1,2-a]pyrimidine-8-carboxylic acid (4-tert-butylthiazol-2-yl)amide (76.7 mg) was dissolved in trifluoroacetic acid (2 ml) and stirred at room temperature for 4 hours. The solvent was concentrated under reduced pressure by azeotropy with ethanol and formic acid. The residue was added with methanol, and the insoluble matters were removed by filtration to obtain the unpurified compound (80.9 mg). This compound (70.9 mg) was purified by HPLC to obtain the title compound (22.8 mg) as orange solid.
¹H-NMR (CD₃OD) δ : 1.35 (9H, s), 2.77 (6H, s), 3.56-3.81 (10H, m), 4.20 (2H, s), 6.72 (1H, s), 7.47 (1H, d, J=16.4Hz), 7.61 (1H, d, J=7.1Hz), 7.95 (1H, d, J=16.4Hz), 7.97-8.04 (2H, m), 8.97 (1H, d, J=7.6Hz).

### (E) {[2-(4-{8-({[4-(tert-Butyl)-1,3-thiazol-2-yl]amino}carbonyl)-4-oxo-3-[(E)-2-(1H-tetrazol-5-yl)vinyl]-4H-pyrido[1,2-a]pyrimidin-2-yl}piperazin-1-yl)-2-oxo-ethylaminocarbonyl]methyl}trimethylammonium

A solution of 2-{4-[2-(2-dimethylaminoacetylamino)acetyl]piperazin-1-yl}-4-oxo-3-[(E)-2-(1H-tetrazol-5-yl)vinyl]-4H-pyrido[1,2-a]pyrimidine-8-carboxylic acid (4-tert-butylthiazol-2-yl)amide (22.8 mg) in N,N-dimethylformamide (1 ml) was added with methyl iodide (100 µl) and left stand at 5°C for 21 hours. The reaction mixture was further added with methyl iodide (100 µl), left stand at 5°C for 16 hours, and then the solvent was concentrated under reduced pressure by azeotropy with ethanol. The residue was added with ether, and the precipitated solid was collected by filtration, washed with ether and dried at 40°C under reduced pressure to obtain the title compound (19.9 mg) as orange solid.
¹H-NMR (400MHz, DMSO-d₆-CD₃OD) δ : 1.33 (9H, s), 2.56 (9H, s), 3.60-3.76 (8H, m), 4.15 (4H, s), 6.86 (1H, br), 7.56 (1H, d, J=16.0Hz), 7.71 (1H, d, J=8.3Hz), 7.87 (1H, d, J=16.0Hz), 8.25 (1H, br), 8.74 (1H, br), 9.00 (1H, d, J=7.3Hz).
IR (ATR) cm⁻¹: 2962, 2866, 2754, 1655, 1637, 1541, 1512, 1437, 1363, 1333, 1281, 1227, 1165, 1101, 1051, 1016.
ES-MS: m/z: 663 (MH⁺).

In a similar manner, the following compound was synthesized.

### Example 260: (2-(4-{8-({[4-(tert-Butyl)-1,3-thiazol-2-yl]amino}carbonyl)-4-oxo-3-[(E)-2-(1H-tetrazol-5-yl)vinyl]-4H-pyrido[1,2-a]pyrimidin-2-yl}piperazin-1-yl)-2-oxoethyl]trimethylammonium

¹H-NMR (CDCl₃) δ : 1.33 (9H, s), 3.38 (9H, s), 3.54 (6H, br), 3.69 (2H, br), 4.50 (2H, s), 6.66 (1H, s), 7.36 (1H, d, J=15.9Hz), 7.54 (1H, br), 7.80-7.94 (2H, m), 8.85 (1H, br). Anal. Calcd. for C₂₈H₃₆N₁₁O₃SI•4.5TFA•1.5H₂O: C, 34.86; H, 3.44; N, 12.09; S, 2.52; F, 20.12. Found: C.35.25; H, 3.11; N, 12.04; S, 2.34; F, 19.76.
IR (ATR) cm⁻¹: 3415, 2972, 1672, 1541, 1512, 1439, 1367, 1254, 1203, 1176, 1124.
ES-MS: m/z: 606 (M⁺).

### Example 261: 2-[2-(1-{8-({[4-(tert-Butyl)-1,3-thiazol-2-yl]amino}carbonyl)-4-oxo-3-[(E)-2-(1H-tetrazol-5-yl)vinyl]-4H-pyrido[1,2-a]pyrimidin-2-yl}piperidin-3-yl)-acetylamino]ethyl}trimethylammonium

### (A) [1-(8-({[4-(tert-Butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)piperidin-3-yl]acetic acid

A solution of [1-(8-({[4-(tert-butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)piperidin-3-yl]acetic acid ethyl ester (164 mg) in THF (3 ml) was added with 1 N aqueous sodium hydroxide (346 µl) and stirred at room temperature for 1 hour and at 50°C for 1 hour. The reaction mixture was further added with 1 N aqueous sodium hydroxide (346 µl), stirred at 50°C for 16 hours, added with 1 N aqueous sodium hydroxide (115 µl) and stirred at 50°C for 1 hour. The reaction mixture was neutralized by adding 1 N hydrochloric acid, diluted with saturated brine and extracted with chloroform. The organic layers were combined and dried over anhydrous sodium sulfate to obtain the title compound (130 mg, 82%). This compound was used in the following reaction without being purified.
¹H-NMR (CDCl₃-CD₃OD) δ: 1.36 (9H, s), 1.30-1.41 (1H, m), 1.63-1.81 (2H, m), 1.93-2.03 (1H, m), 2.15-2.33 (3H, m), 2.96-3.06 (1H, m), 3.16-3.27 (1H, m), 3.78 (3H, m), 4.00 (1H, d, J=11.7Hz), 4.23 (1H, d, J=12.7Hz), 5.60 (2H, s), 6.67 (1H, s), 6.92 (2H, d, J=8.6Hz), 7.35 (2H, d, J=8.6Hz), 7.58 (1H, dd, J=1.8, 7.3Hz), 7.65 (1H, d, J=15.6Hz), 7.80 (1H, d, J=15.6Hz), 8.03 (1H, d, J=1.2Hz), 8.93 (1H, d, J=7.3Hz).

### (B) 2-{3-[(2-Dimethylaminoethylaminocarbonyl)methyl]piperidin-1-yl}-3-{(E)-2-[1-(4-methoxybenzyl)-1H-tetrazol-5-yl]vinyl}-4-oxo-4H-pyrido[1,2-a]pyrimidine-8-carboxylic acid (4-tert-butylthiazol-2-yl)amide

A solution of [1-(8-({[4-(tert-butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)piperidin-3-yl]acetic acid (130 mg) and N,N-dimethylethylenediamine (20.8 µg) in methylene chloride (2 ml) and N,N-dimethylformamide (2 ml) was added with triethylamine (39.7 µl), HOBt (28.2 mg) and WSCD•HCl (40.0 mg) with ice cooling and stirred at room temperature for 16 hours. The solvent was concentrated under reduced pressure, and the residue was purified by silica gel column chromatography (10 g, chloroform → chloroform:methanol = 98:2 → 96:4 → 94:6 → 98:2 → 90:10) and preparative TLC (3 plates, chloroform:methanol = 10:1, developed twice) to obtain the title compound (129 mg, 90%) as orange foamy syrup.
¹H-NMR (CDCl₃) δ : 1.27-1.41 (1H, m), 1.35 (9H, s), 1.49-1.77 (2H, m), 1.88-1.99 (1H, m), 2.12-2.32 (3H, m), 2.26 (6H, s), 2.50-2.61 (2H, m), 2.86-2.99 (1H, m), 3.11-3.23 (1H, m), 3.39-3.44 (2H, m), 3.77 (3H, s), 3.90-4.11 (1.H, m),4.09-4.20 (1H, m), 5.51 (2H, s), 6.56 (1H, s), 6.88 (2H, d, J=8.7Hz), 6.93 (1H, br), 7.35 (2H, d, J=8.7Hz), 7.49 (1H, dd, J=1.7, 7.3Hz), 7.66 (1H, d, J=15.4Hz), 7.83 (1H, d, J= 15.4Hz), 7.90 (1H, s), 8.90 (1H, d, J=7.6Hz).

### (C) (2-{2-[1-(8-({[4-(tert-Butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)-piperidin-3-yl]acetylamino}ethyl)trimethylammonium

A solution of 2-{3-[(2-dimethylaminoethylaminocarbonyl)methyl]piperidin-1-yl}-3-{(E)-2-[1-(4-methoxybenzyl)-1H-tetrazol-5-yl]vinyl}-4-oxo-4H-pyrido[1,2-a]pyrimidine-8-carboxylic acid (4-tert-butylthiazol-2-yl)amide (129 mg) in N,N-dimethylformamide (4 ml) was added with methyl iodide (1 ml) and left stand at 5°C for 16 hours. The solvent was evaporated by azeotropy with toluene, and the residue was purified by preparative TLC (2 plates, chloroform:methanol:water = 8:3:0.5, developed twice) to obtain the title compound (132 mg, 86%) as orange solid. ¹H-NMR (CD₃OD-CDCl₃) δ : 1.23-1.41 (1H, m), 1.34 (9H, s), 1.60-1.84 (2H, m), 1.89-2.00 (1H, m), 2.10-2.28 (3H, m), 2.85-2.99 (1H, m), 3.20 (9H, s), 3.25-3.33 (1H, m), 3.40-3.52 (2H, m), 3.55-3.67 (2H, m), 3.75 (3H, s), 3.95-4.10 (2H, m), 5.58 (2H, s), 6.71 (1H, s), 6.92 (2H, d, J=8.8Hz), 7.33 (2H, d, J=8.8Hz), 7.54 (1H, dd, J=1.7, 7.3Hz), 7.59 (1H, d, J=15.6Hz), 7.72 (1H, d, J=15.6Hz), 7.83-7.92 (1H, m), 8.88 (1H, d, J=7.3Hz).

### (D) 2-[2-(1-{8-({[4-(tert-Butyl)-1,3-thiazol-2-yl]amino}carbonyl)-4-oxo-3-[(E)-2-(1H-tetrazol-5-yl)vinyl]-4H-pyrido[1,2-a]pyrimidin-2-yl}piperidin-3-yl)acetylamino]ethyl}trimethylammonium

A solution of (2-{2-[1-(8-({[4-(tert-butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)piperidin-3-yl]acetylamino}ethyl)trimethylammonium (132 mg) in trifluoroacetic acid (3 ml) and stirred at room temperature for 1 hour and at 80°C for 5 hours. The solvent was evaporated by azeotropy with toluene, and residue was purified by preparative TLC (2 plates, chloroform:methanol:water = 8:3:0.5) to obtain the title compound (73.7 mg) as red solid.
¹H-NMR (400 MHz, DMSO-d₆-CD₃OD) δ: 1.25-1.40 (1H, m), 1.35 (9H, s), 1.70-1.98 (3H, m), 2.03-2.28 (3H, m), 2.90-2.99 (1H, m), 3.12 (9H, s), 3.08-3.57 (5H, m), 3.92-4.01 (1H, m), 4.15-4.24 (1H, m), 6.73 (1H, s), 7.36 (1H, d, J=16.1Hz), 7.55 (1H, dd, J=2.0, 7.6Hz), 7.93 (1H, d, J=16.1Hz), 7.96-7.99 (1H, m), 8.94 (1H, d, J=7.6Hz).
IR (ATR) cm⁻¹: 3037, 2956, 2929, 2858, 1655, 1514, 1421, 1375, 1309, 1254, 1201, 1173, 1124, 1103.
ES-MS: m/z: 648 (M⁺).
Anal. Calcd. for C₃₁H₄₂N₁₁O₃S•-0.5I•-0.5TFA-0.75H₂O: C, 49.07; H, 5.66; N, 16.97; S, 4.09; F, 3.64. Found: C, 49.44; H, 5.99; N, 19.25; S, 4.18; F, 3.21.
m.p.: 201-205°C.

### Example 262: {2-[2-(1-{8-({[4-(tert-Butyl)-1,3-thiazol-2-yl]amino}carbonyl)-4-oxo-3-[(E)-2-(1H-tetrazol-5-yl)vinyl]-4H-pyrido[1,2-a]pyrimidin-2-yl}piperidin-3-yl)acetylamino]ethyl}aminocarbonylmethyldimethylammonium

### (A) (2-{2-[1-(8-({[4-(tert-Butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)piperidin-3-yl]acetylamino}ethyl)aminocarbonylmethyldimethylammonium

A solution of 2-{3-[(2-dimethylaminoethylaminocarbonyl)methyl]piperidin-1-yl}-3-{(E)-2-(1-(4-methoxybenzyl)-1H-tetrazol-5-yl]vinyl}-4-oxo-4H-pyrido(1,2-a]pyrimidine-8-carboxylic acid (4-tert-butylthiazol-2-yl)amide (123 mg) in N,N-dimethylformamide (2 ml) was added with iodoacetamide (33.3 mg) and left stand at 5°C for 16 hours. The reaction mixture was further added with iodoacetamide (27.2 mg), stirred at room temperature for 24 hours. The solvent was evaporated by azeotropy with toluene, and then the residue was purified by preparative TLC (3 plates, chloroform:methanol:water = 8:3:0.5) to obtain the title compound (158 mg, quantitative) as orange solid.
¹H-NMR (CD₃OD) : δ 1.27-1.40 (1H, m), 1.34 (9H, s), 1.56-1.83 (2H, m), 1.90-1.99 (1H, m), 2.12-2.27 (3H, m), 2.87-2.99 (1H, m), 3.08-3.19 (1H, m), 3.36 (6H, s), 3.57-3.80 (4H, m), 3.76 (3H, s), 3.88-4.08 (2H, m), 4.21 (2H, s), 5.59 (2H, s), 6.71 (1H, s), 6.92 (2H, d, J=8.9Hz), 7.33 (2H, d, J=8.9Hz), 7.54 (1H, dd, J=1.8, 7.6Hz), 7.58 (1H, d, J=15.5Hz), 7.73 (1H, d, J=15.5Hz), 7.85-7.95 (1H, m), 8.89 (1H, d, J=7.6Hz).

### (B) {2-[2-(1-{8-({[4-(tert-Butyl)-1,3-thiazol-2-yl]amino}carbonyl)-4-oxo-3-[(E)-2-(1H-tetrazol-5-yl)vinyl]-4H-pyrido[1,2-a]pyrimidin-2-yl}piperidin-3-yl)acetylamino]-ethyl}aminocarbonylmethyldimethylammonium

(2-{2-[1-(8-({[4-(tert-Butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)piperidin-3-yl]acetylamino}ethyl)aminocarbonylmethyldimethylammonium (158 mg) was dissolved in trifluoroacetic acid (2 ml) and stirred at 80°C for 5 hours. The solvent was evaporated by azeotropy with toluene, and the residue was purified by preparative TLC (2 plates, chloroform:methanol:water = 8:3:0.5) to obtain the title compound (116 mg) as red solid.
¹H-NMR (400 MHz, CD₃OD-CDCl₃) δ : 1.28-1.41 (1H, m), 1.35 (9H, s), 1.70-1.99 (3H, m), 2.01-2.29 (3H, m), 2.86-2.97 (1H, m), 3.09-3.17 (1H, m), 3.20-3.78 (4H, m), 3.97-4.06 (1H, m), 4.11 (2H, s), 4.14-4.23 (1H, m), 6.73 (1H, s), 7.36 (1H, d, J=16.4Hz), 7.56 (1H, d, J=7.6Hz), 7.91 (1H, d, J=16.4Hz), 7.99 (1H, s), 8.95 (1H, d, J=7.6Hz).
IR (ATR) cm⁻¹: 3319, 3195, 2964, 1671, 1518, 1425, 1363, 1311, 1257, 1201, 1176, 1126. ES-MS: m/z: 691 (M⁺).
Anal. Calcd. for C₃₂H₄₃N₁₂O₄S·0.5I•2TFA•2.5H₂O: C, 42.03; H, 4.90; N, 16.34; S, 3.12; F, 11.10. Found: C, 41.96; H, 4.51; N, 15.76; S, 3.04; F, 11.90.
m.p.: 175-185°C.

### Example 263: (2-{[2-(1-{8-({[4-(tert-Butyl)-1,3-thiazol-2-yl]amino}carbonyl)-4-oxo-3-[(E)-2-(1H-tetrazol-5-yl)vinyl]-4H-pyrido[1,2-a]pyrimidin-2-yl}piperidin-3-yl)-acetyl]methylamino}ethyl)trimethylammonium

### (A) 2-(3-{[(2-Dimethylaminoethyl)methylaminocarbonyl]methyl}piperidin-1-yl)-3-{(E)-2-[1-(4-methoxybenzyl)-1H-tetrazol-5-yl]vinyl}-4-oxo-4H-pyrido[1,2-a]pyrimidine-8-carboxylic acid (4-tert-butylthiazol-2-yl)amide

A solution of [1-(8-({[4-(tert-butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)piperidin-3-yl]acetic acid (140 mg) and N,N,N'-trimethylethylenediamine (26.7 µg) in methylene chloride (2 ml) and N,N-dimethylformamide (2 ml) was added with triethylamine (42.9 *µ*l), HOBt (30.5 mg) and WSCD•HCl (43.2 mg) with ice cooling and stirred at room temperature for 14 hours. The solvent was concentrated under reduced pressure, and the residue was purified by preparative TLC (3 plates, chloroform:methanol = 10:1) to obtain the title compound (145 mg, 92%) as orange solid.
¹H-NMR (CDCl₃) δ : 1.28-1.42 (1H, m), 1.34 (9H, s), 1.57-1.79 (2H, m), 1.90-2.00 (1H, m), 2.20-2.60.(4H, m), 2.25 (3H, s), 2.29 (3H, s), 2.92 (1H, s), 2.95-3.10 (1H, m), 3.06 (3H, s), 3.18-3.31 (1H, m), 3.40-3.63 (2H, m), 3.77 (3H, s), 3.90-4.00 (1H, m), 4.09-4.26 (1H, m), 5.51 (2H, s), 6.53-6.60 (1H, m), 6.89 (2H, d, J=8.5Hz), 7.35 (2H, d, J=8.5Hz), 7.45-7.54 (1H, m), 7.35 (1H, d, J=8.5Hz), 7.71 (1H, d, J=8.5Hz), 7.90-7.96 (1H, m), 8.88-8.98 (1H, m).

### (B) [2-({2-[1-(8-({[4-(tert-Butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)-piperidin-3-yl]acetyl}methylamino)ethyl]trimethylammonium

A solution of 2-(3-{[(2-dimethylaminoethyl)methylaminocarbonyl]methyl}piperidin-1-yl)-3-{(E)-2-[1-(4-methoxybenzyl)-1H-tetrazol-5-yl]vinyl}-4-oxo-4H-pyrido[1,2-a]pyrimidine-8-carboxylic acid (4-tert-butylthiazol-2-yl)amide (145 mg) in N,N-dimethylformamide (2 ml) was added with methyl iodide (1 ml) and left stand at 5°C for 12 hours. The solvent was evaporated by azeotropy with toluene, and the residue was purified by preparative TLC (2 plates, chloroform:methanol:water = 8:3:0.5) to obtain the title compound (159 mg, 92%) as red solid.
¹H-NMR (CD₃OD) δ: 1.25-1.41 (1H, m), 1.35 (9H, s), 1.61-1.84 (2H, m), 1.90-2.00 (1H, m), 2.18-2.46 (3H, m), 2.98-3.03 (1H, m), 3.09 (3H, s), 3.11-3.22 (1H, m), 3.19 (9H, s), 3.45-3.55 (2H, m), 3.70-3.80 (2H, m), 3.76 (3H, s), 3.96-4.08 (2H, m), 5.60 (2H, s), 6.72 (1H, s), 6.92 (2H, d, J=8.8Hz), 7.33 (2H, d, J=8.8Hz), 7.56 (1H, dd, J=1.7, 7.3Hz), 7.62 (1H, d, J=15.6Hz), 7.75 (1H, d, J=15.6Hz), 7.84-7.93 (1H, m), 8.90 (1H, d, J=7.6Hz).

### (C) (2-{[2-(1-{8-({[4-(tert-Butyl)-1,3-thiazol-2-yl]amino}carbonyl)-4-oxo-3-[(E)-2-(1H-tetrazol-5-yl)vinyl]-4H-pyrido(1,2-a]pyrimidin-2-yl}piperidin-3-yl)acetyl]methylamino}ethyl)trimethylammonium

A solution of [2-({2-[1-(8-({[4-(tert-butyl)-1,3-thiazol-2-yl]amino}carbonyl)-3-{(E)-2-[1-(4-methoxybenzyl)-1H-tetrazol-5 yl]vinyl}-4-oxo-4H pyrido[1,2-a]pyrimidin-2-yl)piperidin-3-yl]acetyl}methylamino)ethyl]trimethylammonium (159 mg) in trifluoroacetic acid (2 ml) was stirred at room temperature for 1 hour and at 80°C for 4 hours. The solvent was evaporated by azeotropy with toluene, and the residue was purified by preparative TLC (2 plates, chloroform:methanol:water = 8:3:0.5) to obtain the title compound (139 mg) as red solid.
¹H-NMR (CD₃OD-CDCl₃) δ: 1.27-1.38 (1H, m), 1.34 (9H, s), 1.69-1.86 (2H, m), 1.90-1.99 (1H, m), 2.13-2.25 (2H, m), 2.33-2.42 (1H, m), 2.79-2.89 (1H, m), 2.99 (3H, s), 3.00-3.38 (5H, m), 3.11 (9H, s), 3.94-4.03 (1H, m), 4.12-4.21 (1H, m), 6.72 (1H, s), 7.35 (1H, d, J=16.4Hz), 7.54 (1H, dd, J=1.7, 7.3Hz), 7.88-7.97 (1H, m), 7.92 (1H, d, J=16.4Hz), 8.92 (1H, d, J=7.3Hz).
IR (ATR) cm⁻¹: 2962, 2937, 2864, 1676, 1635, 1514, 1423, 1379, 1309, 1255, 1203, 1174, 1124.
ES-MS: m/z: 662 (M⁺).
Anal. Calcd. for C₃₂H₄₄N₁₁O₃S•0.5I•2.5TFA•1.5H₂O: C, 42.78; H, 4.80; N, 14.83; S, 3.09; F, 13.72. Found: C, 42.80; H, 4.56; N, 14.57; S, 3.11; F, 13.01.
m.p.: 163-168°C.

### Example 264: {3-[2-(1-{8-({[4-(tert-Buty)-1,3-thiazol-2-yl]amino}carbonyl)-4-oxo-3-[(E)-2-(1H-tetrazol-5-yl)vinyl]-4H-pyrido[1,2-a]pyrimidin-2-yl}piperidin-3-yl)acetylamino]propyl}aminocarbonylmethyldimethylammonium

### (A) 2-{3-[(3-Dimethylaminopropylaminocarbonyl)methyl]piperidin-1-yl}-3-{(E)-2-[1-(4-methoxybenzyl)-1H-tetrazol-5-yl]vinyl}-4-oxo-4H-pyrido[1,2-a]pyrimidine-8-carboxylic acid (4-tert-butylthiazol-2-yl)amide

A solution of [1-(8-({[4-(tert-butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)piperidin-3-yl]acetic acid (250 mg) and N,N-dimethyl-1,3-propanediamine (46.0 µl) in methylene chloride (2 ml) and N,N-dimethylformamide (2 ml) was added with triethylamine (76.5 µl), HOBt (54.3 mg) and WSCD·HCl (77.1 mg) with ice cooling and stirred at room temperature for 16 hours. The solvent was concentrated under reduced pressure, and the residue was purified by silica gel column chromatography (25g, chloroform → chloroform:methanol = 15:1 → 10:1 → 4:1) and preparative TLC (3 plates, chloroform:methanol = 4:1) to obtain the title compound (283 mg, 90%) as orange foamy syrup.
¹H-NMR (CD₃OD-CDCl₃) δ : 1.28-1.41 (1H, m), 1.35 (9H, s), 1.65-2.00 (5H, m), 2.14-2.22 (3H, m), 2.68 (6H, s), 2.83-2.89 (2H, m), 2.92-2.99 (1H, m), 3.12-3.20 (3H, m), 3.76 (3H, s), 4.01-4.10 (2H, m), 5.61 (s, 2H), 6.73 (1H, s), 6.93 (2H, d, J=8.8Hz), 7.35 (2H, d, J=8.8Hz), 7.58 (1H, dd, J=1.2, 7.3Hz), 7.64 (1H, d, J=15.6Hz), 7.79 (1H, d, J=15.6Hz), 7.87-7.90 (1H, m), 8.94 (1H, d, J=7.3Hz).

### (B) (3-{2-[1-(8-({[4-(tert-Butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)-piperidin-3-yl]acetylamino}pnopyl)aminocarbonylmethyldimethylammonium

A solution of 2-{3-[(3-dimethylaminopropylaminocarbonyl)methyl]piperidin-1-yl}-3-1{(E)-2-[1-(4-methoxybenzyl)-1H-tetrazol-5-yl]vinyl}-4-oxo-4H-pyrido[1,2-a]pyrimidine-8-carboxylic acid (4-tert-butylthiazol-2-yl)amide (142 mg) in N,N-dimethylformamide (3 ml) was added with iodoacetamide (37.2 mg) and stirred at room temperature for 12 hours. The reaction mixture was added with iodoacetamide (30.4 mg) and stirred at room temperature for 12 hours and 30 minutes. The reaction mixture was further added with iodoacetamide (34.1 mg), stirred at room temperature for 13 hours and the solvent was evaporated by azeotropy with toluene. The residue was diluted with saturated aqueous sodium hydrogencarbonate and extracted with chloroform/methanol, and the organic layer was dried over anhydrous magnesium sulfate. The solvent was concentrated under reduced pressure, and the residue was dissolved in N,N-dimethylformamide (3 ml), added with iodoacetamide (34.1mg) and stirred at room temperature for 1 hour. The solvent was evaporated by azeotropy with toluene, and the residue was purified by preparative TLC (2 plates, chloroform:methanol:water = 8:3:0.5) to obtain the title compound (113 mg, 64%) as orange solid.
¹H-NMR (CD₃OD-CDCl₃) δ : 1.26-1.41 (1H, m), 1.34 (9H, s), 1.65-1.84 (2H, m), 1.91-2.04 (3H, m), 2.12-2.28 (3H, m), 2.88-2.97 (1H, m), 3.09-3.24 (3H, m), 3.29 (6H, s), 3.59-3.67 (2H, m), 3.76 (3H, s), 3.99-4.09 (2H, m), 4.12 (2H, s), 5.59 (2H, s), 6.71 (1H, s), 6.92 (2H, d, J=8.6Hz), 7.33 (2H, d, J=8.6Hz), 7.52-7.57 (1H, m), 7.61 (1H, d, J=15.4Hz), 7.73 (1H, d, J=15.4Hz), 7.91-7.94 (1H, m), 8.89 (1H, d, J=7.3Hz).

### (C) {3-[2-(1-{8-({[4-(tert-Butyl)-1,3-thiazol-2-yl]amino}carbonyl)-4-oxo-3-[(E)-2-(1H-tetrazol-5-yl)vinyl]-4H-pyrido[1,2-a]pyrimidin-2-yl}piperidin-3-yl)acetylamino]-propyl}aminocarbonylmethyldimethylammonium

A solution of (3-{2-[1-(8-({[4-(tert-butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)piperidin-3-yl]acetylamino]propyl)aminocarbonylmethyldimethylammonium (113 mg) in trifluoroacetic acid (2 ml) was stirred at 80°C for 4 hours and 30 minutes. The solvent was evaporated by azeotropy with toluene, and the residue was purified by preparative TLC (2 plates, chloroform:methanol:water = 8:3:0.5). The resulting fraction was subjected to azeotropy with formic acid, and the residue was purified by HPLC to obtain the title compound (33.2 mg) as orange solid.
¹H-NMR (400 MHz, CD₃OD-CDCl₃) δ: 1.27-1.41 (1H, m), 1.35 (9H, s), 1.67-1.99 (5H, m), 2.05-2.25 (3H, m), 2.90-3.21 (4H, m), 3.28 (6H, s), 3.47-3.56 (2H, m), 3.92-4.02 (1H, m), 4.03-4.18 (1H, m), 4.09 (2H, s), 6.72 (1H, s), 7.39 (1H, d, J=15.8Hz), 7.51-7.58 (1H, m), 7.84-7.95 (1H, m), 7.95 (1H, d, J=15.8Hz), 8.90 (1H, d, J=7.3Hz).
IR (ATR) cm⁻¹: 3049, 2960, 2929, 2854, 1653, 1514, 1421, 1375, 1308, 1252, 1227, 1203, 1155, 1101, 1066.
ES-MS: m/z: 705 (M⁺)
Anal. Calcd. for C₃₃H₄₄N₁₂O₄S•HCOOH•1.5H₂O: C, 52.50; H, 6.35; N, 21.61. Found: C, 52.38; H, 6.50; N, 21.51.
m.p.: 186-193°C.

### Example 265: (E)-3-(8-({[4-(tert-Butyl)-1,3-thiazol-2-yl]amino)carbonyl)-2-{3-[(2-hydroxyethylaminocarbonyl)methyl]piperidin-1-yl}-4-oxo-4H-pyrido[1,2-a]pyrimidin-3-yl)-2-propenoic acid

### (A) 3-[(2-Hydroxyethylaminocarbonyl)methyl]piperidine-1-carboxylic acid tert-butyl ester

A solution of 3-carboxymethylpiperidine-1-carboxylic acid tert-butyl ester (583 mg) and ethanolamine (159 µl) in dichloromethane (5 ml) was added with triethylamine (502 µl), HOBt (389 mg) and WSCD·HCl (552 mg) at 0°C. Since insoluble matters were precipitated, the reaction mixture was further added with N,N-dimethylformamide (5 ml) and stirred at room temperature for 12 hours. The reaction mixture was concentrated under reduced pressure by azeotropy with toluene, and the residue was diluted with ethyl acetate and washed with 10% aqueous citric acid, saturated aqueous sodium hydrogencarbonate and saturated brine. The organic layer was dried over anhydrous magnesium sulfate and concentrated under reduced pressure. The residue was purified by silica gel column chromatography (50 g, chloroform:ethyl acetate = 1:1 → chloroform:methanol = 10:1) to obtain the title compound (643 mg, 94%) as colorless oily substance.
¹H-NMR (CD₃OD) δ : 1.05-1.48 (1H, m), 1.44 (9H, s), 1.53-2.18 (6H, m), 2.43-2.93 (2H, m), 3.13-3.35 (2H, m), 3.43-3.62 (2H, m), 3.68-3.94 (2H, m).

### (B) N-(2-Hydroxyethyl)-2-piperidin-3-yl-acetamide hydrochloride

3-[(2-Hydroxyethylaminocarbonyl)methyl]piperidine-1-carboxylic acid tert-butyl ester (643 mg) was dissolved in 4 N hydrochloric acid in dioxane (5 ml) and stirred at room temperature for 1 hour and 30 minutes. The reaction mixture was subjected concentrated under reduced pressure by azeotropy with toluene to obtain the title compound (471 mg, 94%) as colorless oily substance.
¹H-NMR (400 MHz, CDCl₃) δ : 1.22-1.38 (1H, m), 1.66-1.81 (1H, m), 1.84-1.99 (2H, m), 2.12-2.27 (3H, m), 2.67-2.78 (1H, m), 2.85-2.97 (1H, m), 3.26-3.40 (4H, m), 3.59 (2H, t, J=5.7Hz).

### (C) (E)-3-(8-({[4-(tert-Butyl)-1,3-thiazol-2-yl]amino}carbonyl)-2-{3-[(2-hydroxyethylaminocarbonyl)methyl]piperidin-1-yl}-4-oxo-4H-pyrido[1,2-a]pyrimidin-3-yl)-2-propenoic acid tert-butyl ester

The following reaction was performed under argon atmosphere. A solution of (E)-3-[8-({[4-(tert-butyl)-1,3-thiazol-2-yl]amino}carbonyl)-2-hydroxy-4-oxo-4H-pyrido[1,2-a]pyrimidin-3-yl]-2-propenoic acid tert-butyl ester (250 mg) in N,N-dimethylformamide (2 ml) was added with diphenylphosphoryl chloride (220 *µ*l) and diisopropylethylamine (370 µl) at 0°C and stirred at 0°C for 30 minutes. The reaction mixture was added with N-(2-hydroxyethyl)-2-piperidin-3-yl-acetamide hydrochloride (237 mg) and diisopropylethylamine (370 µl) at the same temperature and stirred at 80°C for 2 hours. The reaction mixture was returned to room temperature, then diluted with water and extracted with chloroform. The organic layer was dried over anhydrous magnesium sulfate, and the solvent was concentrated under reduced pressure by azeotropy with toluene. The residue was purified by silica gel column chromatography (50 g, ethyl acetate) and preparative TLC (3 plates, ethyl acetate:methanol = 99:1, developed twice) to obtain the title compound (106 mg, 31%) as orange foamy syrup.
¹H-NMR (CDCl₃) δ : 1.26-1.57 (1H, m), 1.35 (9H, s), 1.53 (9H, s), 1.67-1.86 (2H, m), 1.91-2.02 (1H, m), 2.13-2.27 (3H, m), 2.93-3.04 (1H, m), 3.17-3.37 (3H, m), 3.62 (2H, t, J=5.9Hz), 3.93-4.02 (1H, m), 4.12-4.20 (1H, m), 6.74 (1H, s), 6.94 (1H, d, J=15.6Hz), 7.47 (1H, d, J=15.6Hz), 7.55 (1H, dd, J=1.7, 7.3Hz), 7.95-8.00 (1H, m), 8.91 (1H, d, J=7.3Hz).

### (D) (E)-3-(8-({[4-(tert-Butyl)-1,3-thiazol-2-yl]amino}carbonyl)-2-{3-[(2-hydroxyethylaminocarbonyl)methyl]piperidin-1-yl}-4-oxo-4H-pyrido[1,2-a]pyrimidin-3-yl)-2-propenoic acid

(E)-3-(8-({[4-(tert-Butyl)-1,3-thiazol-2-yl]amino}carbonyl)-2-{3-[(2-hydroxyethylaminocarbonyl)methyl]piperidin-1-yl}-4-oxo-4H-pyrido[1,2-a]pyrimidin-3-yl)-2-propenoic acid tert-butyl ester (106 mg) was dissolved in 4 N hydrochloric acid in dioxane (2 ml) and stirred at room temperature for 4 hours 30 minutes. Since solid was precipitated, the reaction mixture was added with methanol. The solvent was concentrated under reduced pressure, and the residue was purified by preparative TLC (2 plates, chloroform:methanol = 10:1). Since a part of the product became methyl ester, this fraction was dissolved in tetrahydrofuran (3ml), methanol (1ml) and water (1ml), added with lithium hydroxide monohydrate (10 mg) and stirred at room temperature for 1 hour and at 50°C for 1 hour and 30 minutes. The reaction mixture was further added with lithium hydroxide monohydrate (total amount: 20 mg) with stirring at 50°C for 4 hours and 30 minutes and at 80°C for 30 minutes. The reaction mixture was adjusted to pH 3 by adding 1 N hydrochloric acid, and the solvent was concentrated under reduced pressure. The residue and the aforementioned fraction were purified by preparative TLC (3 plates, chloroform:methanol = 4:1) to obtain the title compound (39.7 mg) as yellow solid.
¹H-NMR (CD₃OD-CDCl₃) δ: 1.26-1.44 (1H, m), 1.35 (9H, s), 1.66-1.85 (2H, m), 1.91-1.99 (1H, m), 2.11-2.29 (3H, m), 2.95-3.04 (1H, m), 3.21-3.38 (3H, m), 3.62 (2H, d, J=5.8Hz), 3.98-4.07 (1H, m), 4.13-4.20 (1H, m), 6.74 (1H, s), 7.01 (1H, d, J=15.4Hz), 7.56 (1H, dd, J=2.0, 7.3Hz), 7.58 (1H, d, J=15.4Hz), 7.99-8.02 (1H, m), 8.93 (1H, dd, J=0.7, 7.3Hz).
IR (ATR) cm⁻¹: 3321,2929, 2854, 1664, 1639, 1516, 1439, 1365, 1311, 1282, 1234, 1101, 1061, 1003.
m.p.: 167-175°C
ES-MS: m/z: 583 (MH⁺).

### Example 266: {2-[2-(1-{8-({[4-(tert-Butyl)-1,3-thiazol-2-yl]amino}carbonyl)-4-oxo-3-[(E)-2-(1H-tetrazol-5-yl)vinyl]-4H-pyrido[1,2-a]pyrimidin-2-yl}piperidin-3-yl)-acetylamino]ethyl}-(2-hydroxyethyl)dimethylammonium

### (A) (2-{2-[1-(8-({[4-(tert-Butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)-piperidin-3-yl]acetylamino}ethyl)-(2-hydroxyethyl)dimethylammonium

A solution of 2-{3-[(2-dimethylaminoethylaminocarbonyl)methyl]piperidin-1-yl}-3-{(E)-2-[1-(4-methoxybenzyl)-1H-tetrazol-5-yl]vinyl}-4-oxo-4H-pyrido[1,2-a]pyrimidine-8-carboxylic acid (4-tert-butylthiazol-2-yl)amide (113 mg) in N,N-dimethylformamide (2 ml) was added with iodoethanol (12.9 µl) and stirred at room temperature for 19 hours. The reaction mixture was added with iodoethanol (12.9 µl) and stirred at room temperature 10 hours and 30 minutes. The reaction mixture was added with iodoethanol (58.5 µl) and stirred at room temperature for 20 hours. The reaction mixture was added with iodoethanol (58.5 µl) and stirred at room temperature for 25 hours. The reaction mixture was added with saturated aqueous sodium hydrogencarbonate (1 ml), stirred at room temperature for 30 minutes, then added with iodoethanol (1 ml) and stirred at room temperature for 20 hours. After further stirred for 22 hours and 30 minutes, the reaction mixture was subjected concentrated under reduced pressure by azeotropy with N,N-dimethylformamide and toluene, and the residue was purified by preparative TLC (4 plates, chloroform:methanol:water = 8:3:0.5) to obtain the title compound (141 mg, quantitative) as orange solid.
¹H-NMR (CD₃OD-CDCl₃) δ : 1.31-1.37 (1H, m), 1.35 (9H, s), 1.63-1.85 (2H, m), 1.91-1.99 (1H, m), 2.13-2.26 (3H, m), 2.91-3.01 (1H, m), 3.11-3.17 (1H, m), 3.23 (6H, s), 3.51-3.57 (4H, m), 3.59-3.65 (2H, m), 3.76 (3H, s), 3.98-4.09 (4H, m), 5.60 (2H, s), 6.73 (1H, m), 6.78 (2H, d, J=8.8Hz), 7.33 (2H, d, J=8.8Hz), 7.55-7.59 (1H, m), 7.63 (1H, d, J=15.6Hz), 7.76 (1H, d, J=15.6Hz), 7.94 (1H, d, J=1.5Hz), 8.93 (1H, d, J=7.8Hz).

### (B) {2-[2-(1-{8-({[4-(tert-Butyl)-1,3-thiazol-2-yl]amino}carbonyl)-4-oxo-3-[(E)-2-(1H-tetrazol-5-yl)vinyl]-4H-pyrido[1,2-a]pyrimidin-2-yl}piperidin-3-yl)acetylamino]-ethyl}-(2-hydroxyethyl)dimethylammonium

(2-(2-[l-(8-(([4-(tert-Butyl)-1,3-thiazol-2-yllamino)carbonyl)-3-((E)-2-fl-(4-methoxybenzyl)-1H-tetrazol-5-yl]vinyl}-4-oxo-4H-pyrido[1,2-a]pyrimidin-2-yl)piperidin-3-yl]acetylamino}ethyl)-(2-hydroxyethyl)dimethylammonium (141 mg) was dissolved in anisole (1 ml) and trifluoroacetic acid (2 ml) and stirred at 80°C for 4 hours. The reaction mixture was concentrated under reduced pressure by azeotropy with toluene, and the residue was purified by preparative TLC (3 plates, chloroform:methanol:water = 8:3:0.5) and further purified by HPLC to obtain the title compound (21.9 mg) as orange solid.
¹H-NMR (CD₃OD) δ : 1.22-1.39 (1H, m), 1,34 (9H, s), 1.64-1.95 (3H, m), 2.02-2.24 (3H, m), 2.81-2.93 (1H, m), 3.01-3.15 (1H, m), 3.17 (6H, s), 3.32-3.51 (6H, m), 3.88-4.03 (3H, m), 4.07-4.19 (1H, m), 6.70 (1H, s), 7.33 (1H, d, J=16.4Hz), 7.45-7.56 (1H, m), 7.83-7.94 (2H, m), 8.88 (1H, d, J=7.3Hz).
IR (ATR) cm⁻¹: 3219, 2958, 2927, 2852, 1655, 1512, 1421, 1373, 1308, 1252, 1227, 1101, 1066.
ESI-MSm/z: 678 (M⁺).
m.p.: 207-217°C.

### Example 267: {2-[2-(1-{8-({[4-(tert-Butyl)-1,3-thiazol-2-yl]amino}carbonyl)-4-oxo-3-[(E)-2-(1H-tetrazol-5-yl)vinyl]-4H-pyrido[1,2-a]pyrimidin-2-yl}piperidin-4-yl)acetylamino]ethyl}aminocarbonylmethyldimethylammonium

### (A) 4-Ethoxycarbonylmethylene-piperidine-1-tert-butyl ester

A solution of ethyl diethylphosphonoacetate (11.1 ml) in tetrahydrofuran (100 ml) was added with sodium hydride (95%, 1.32 g) at 0°C and stirred at room temperature for 15 minutes. The reaction mixture was added with a solution of 4-oxo-piperidine-1-tert-butyl ester (10.0 g) in tetrahydrofuran (100 ml) at 0°C and stirred at room temperature for 14 hours. The reaction mixture was diluted with water and extracted with ethyl acetate, and the organic layer was dried over anhydrous magnesium sulfate. The solvent was concentrated under reduced pressure, and the residue was purified by silica gel column chromatography (500 g, hexane:ethyl acetate = 7:1) to obtain the title compound (12.2 g, 90%) as white solid.
¹H-NMR (CDCl₃) δ : 1.28 (3H, t, J=7.1Hz), 1.47 (9H, s), 2.28 (2H, t, J=5.7Hz), 2.93 (2H, t, J=5.7Hz), 4.15 (2H, t, J=5.7Hz), 4.16 (2H, t, J=5.7Hz), 5.71 (1H, s).

### (B) 4-Ethoxycarbonylmethylpiperidine-1-tert-butyl ester

A solution of 4-ethoxycarbonylmethylenepiperidine-1-tert-butyl ester (7.17 g) in ethanol (100 ml) was added with 5% Pd-C (wet, 1.5 g) and stirred at room temperature for 17 hours under hydrogen atmosphere. The catalyst was removed by filtration through a Celite layer and washed with ethanol, and the filtrate and the washing solution were combined and concentrated. The residue was removed by filtration through a silica gel pad and eluted with ethyl acetate. The solvent was concentrated under reduced pressure to obtain the title compound (7.31 g, quantitative) as colorless oily substance.
¹H-NMR (CDCl₃) δ : 1.16 (2H, dq, J=3.7, 12.5Hz), 1.26 (3H, t, J=7.1Hz), 1.45 (9H, s), 1.69 (2H, br.d, J=11.0Hz), 1.87-1.99 (1H, m), 2.23 (2H, d, J=7.1Hz), 2.72 (2H, br.t, J=12.5Hz), 4.00-4.17 (2H, m), 4.13 (2H, q, J=7.1Hz).

### (C) Piperidin-4-yl-acetic acid ethyl ester hydrochloride

4-Ethoxycarbonylmethylpiperidine-1-tert-butyl ester (7.31 g) was dissolved in 4 N solution of hydrochloric acid in dioxane (70 ml) and stirred at room temperature for 2 hours. The solvent was evaporated by azeotropy with toluene to obtain the title compound (6.16 g, quantitative) as a mixture of colorless solid and oily substance.
¹H-NMR (CDCl₃) δ: 1.26 (3H, t, J=7.1Hz), 1.64-1.80 (2H, m), 1.85-2.11 (3H, m), 2.31 (2H, d, J=7.1Hz), 2.81-2.97 (2H, m), 3.72 (2H, d, J=7.1Hz), 4.14 (2H, q, J=7.1Hz).

### (D) [1-(8-({[4-(tert-Butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)piperidin-4-yl]acetic acid ethyl ester

The following reaction was performed under argon atmosphere. A solution of 2-hydroxy-3-{(E)-2-[1-(4-methoxybenzyl)-1H-tetrazol-5-yl]vinyl}-4-oxo-4H-pyrido[1,2-a]pyrimidine-8-(4-tert-butylthiazol-2-yl)amide (500 mg) in N,N-dimethylformamide (4 ml) was added with diisopropylethylamine (624 µl) and diphenylphosphoryl chloride (371 µl) at 0°C and stirred at 0°C for 30 minutes. The reaction mixture was added with piperidin-4-yl-acetic acid ethyl ester hydrochloride (372 mg) and diisopropylethylamine (312 µl) at the same temperature and stirred at 80°C for 1 hour. The reaction mixture was further added with piperidin-4-yl-acetic acid ethyl ester hydrochloride (372 mg) and diisopropylethylamine (312 µl) at the same temperature and stirred at 80°C for 2 hours. The reaction mixture was returned to room temperature, diluted with water and extracted with ethyl acetate. The organic layer was dried over anhydrous magnesium sulfate, and the solvent was concentrated under reduced pressure. The residue was purified by silica gel column chromatography (50 g, chloroform:ethyl acetate = 4:1) and silica gel column chromatography (40 g, hexane:ethyl acetate = 2:1 → 3:2 → 1:1) to obtain the title compound (233 mg) as a hardly separable mixture.
¹H-NMR (CDCl₃) δ : 1.26 (3H; t, J=7.1Hz), 1.37 (9H, s), 1.39-1.50 (2H, m), 1.83 (2H, br.d, J=12.5Hz), 2.03-2.16 (1H, m), 2.31 (2H, d, J=7.1Hz), 3.07-3.19 (2H, m), 3.80 (3H, s), 4.08-4.13 (2H, m), 4.15 (2H, q, J=7.1Hz), 5.59 (3H, s), 6.65 (1H, s), 7.22 (2H, d, J=8.8Hz), 7.36 (2H, d, J=8.8Hz), 7.58 (1H, dd, J=1.8, 7.3Hz), 7.66 (1H, d, J=15.6Hz), 7.85 (1H, d, J=15.6Hz), 8.00-8.04 (1H, m), 8.94 (1H, d, J=7.3Hz).

### (E) [1-(8-({[4-(tert-Butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)piperidin-4-yl]acetic acid

A solution of [1-(8-({[4-(tert-butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)piperidin-4-yl]acetic acid ethyl ester (233 mg) in tetrahydrofuran (2 ml) was added with 1 N aqueous sodium hydroxide (655 µl) and stirred at 50°C for 1 hour. Then the reaction mixture was added with 1 N aqueous sodium hydroxide (327 µl) four times with stirring at 50°C over 6 hours and 30 minutes. The reaction mixture was returned to room temperature and adjusted to about pH 4 by adding 1 N hydrochloric acid. The reaction mixture was diluted with saturated brine and extracted with chloroform. The organic layer was dried over anhydrous magnesium sulfate. The solvent was concentrated under reduced pressure to obtain the title compound (223 mg, 36% for the two steps) as orange foamy syrup.
¹H-NMR (CDCl₃) δ : 1.35 (9H, s), 1.55-1.70 (2H, m), 1.91-2.01 (2H, m), 2.21-2.35 (1H, m), 2.51-2.60 (2H, m), 3.00-3.13 (2H, m), 3.76 (3H, s), 3.95-4.04 (2H, m), 5.49 (2H, s), 6.64 (1H, s), 6.85 (2H, d, J=8.8Hz), 7.31 (2H, d, J=8.8Hz), 7.55 (1H, dd, J=1.5, 7.3Hz), 7.70 (1H, d, J=15.5Hz), 7.89 (1H, d, J=15.5Hz), 8.28 (1H, d, J1.5Hz), 8.98 (1H, d, J=7.3Hz).

### (F) 2-{4-[(2-Dimethylaminoethylaminocarbonyl)methyl]piperidin-1-yl}-3-{(E)-2-[1-(4-methoxybenzyl)-1H-tetrazol-5-yl]vinyl}-4-oxo-4H-pyrido[1,2-a]pyrimidin-8-(4-tert-butylthiazol-2-yl)amide

A solution of [1-(8-({[4-(tert-butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)piperidin-4-yl]acetic acid (223 mg) and N,N-dimethylethylenediamine (35.8 µl) in methylene chloride (3 ml) and N,N-dimethylformamide (1 ml) was added with triethylamine (114 µl), HOBt (48.5 mg) and WSCD•HCl (68.8 mg) with ice cooling and stirred at room temperature for 24 hours. The solvent was subjected to azeotropy with toluene, and the residue was diluted with saturated aqueous sodium hydrogencarbonate and extracted with chloroform. The organic layer was dried over anhydrous magnesium sulfate, and the solvent was concentrated under reduced pressure. The residue was purified by silica gel column chromatography (15 g, chloroform → chloroform:methanol = 10:1) to obtain the title compound (185 mg, 75%) as orange foamy syrup.
¹H-NMR (CDCl₃) δ : 1.33 (9H, s), 1.34-1.48 (2H, m), 1.70-1.80 (2H, m), 1.97-2.09 (1H, m), 2.15-2.21 (2H, m), 2.30 (6H, s), 2.44-2.53 (2H, m), 2.99 (2H, s), 2.95-3.08 (2H, m), 3.28-3.38 (2H, m), 3.72 (3H, s), 4.00-4.11 (2H, m), 5.53 (2H, s), 6.64 (1H, s), 6.87 (2H, d, J=8.7Hz), 7.29 (2H, d, J=8.7Hz), 7.45 (1H, dd, J=1.8, 7.3Hz), 7.52 (1H, d, J=15.6Hz), 7.71 (1H, d, J=15.6Hz), 7.80-7.84 (1H, m), 8.78 (1H, d, J=7.3Hz).

### (G) (2-{2-[1-(8-({[4-(tert-Butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)-piperidin-4-yl]acetylamino}ethyl)aminocarbonylmethyldimethylammonium

A solution of 2-{4-[(2-dimethylaminoethylaminocarbonyl)methyl]piperidin-1-yl}-3-{(E)-2-[1-(4-methoxybenzyl)-1H-tetrazol-5-yl]vinyl}-4-oxo-4H-pyrido[1,2-a]pyrimidin-8-(4-tert-butylthiazol-2-yl)amide (83.3 mg) in N,N-dimethylformamide (2 ml) was added with iodoacetamide (22.5 mg) and stirred for 16 hours with light shielding. The reaction mixture was further added with iodoacetamide (11.0 mg) and stirred for 24 hours with light shielding. The solvent was evaporated by azeotropy with toluene, and the residue was purified by preparative TLC (2 plates, chloroform:methanol:water = 8:3:0.5) to obtain the title compound (102 mg, 98%) as orange solid.
¹H-NMR (CD₃OD) δ : 1.34 (9H, s), 1.40-1.54 (2H, m), 1.75-1.86 (2H, m), 2.00-2.29 (3H, m), 3.02-3.15 (2H, m), 3.37 (6H, s), 3.67-3.82 (4H, m), 3.76 (3H, s), 4.09-4.23 (4H, m), 5.59 (2H, s), 6.72 (1H, s), 6.91 (2H, d, J=8.8Hz), 7.33 (2H, d, J=8.8Hz), 7.51-7.58 (1H, m), 7.61 (1H, d, J=15.6Hz), 7.80 (1H, d, J=15.6Hz), 7.91-7.96 (1H, m), 8.88-8.95 (1H, m).

### (H) {2-[2-(1-{8-({[4-(tert-Butyl)-1,3-thiazol-2-yl]amino}carbonyl)-4-oxo-3-[(E)-2-(1H-tetrazol-5-yl)vinyl]-4H-pyrido[1,2-a]pyrimidin-2-yl}piperidin-4-yl)acetylamino]-ethyl}aminocarbonylmethyldimethylammonium

A solution of (2-{2-[1-(8-({[4-(tert-butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)piperidin-4-yl]acetylamino}ethyl)aminocarbonylmethyldimethylammonium (102 mg) in trifluoroacetic acid (2 ml) was stirred at 50°C for 4 hours and at 80°C for 4 hours. The solvent was evaporated by azeotropy with toluene, and the residue was purified by preparative TLC (2 plates, chloroform:methanol:water = 8:3:0.5, developed twice) to obtain the title compound (62.7 mg) as red solid.
Anal. Calcd. for C₃₂H₄₂N₁₂O₄S · 3H₂O · 3.5TFA: C, 40.95; H, 4.54; N, 14.69; S, 2.80. Found: C, 40.83; H, 4.35; N, 14.91; S, 2.91.
¹H-NMR (400 MHz, CD₃OD) δ : 1.25 (9H, s), 1.35-1.49 (2H, m), 1.64-1.75 (2H, m), 1.90-2.02 (1H, m), 2.10-2.18 (2H, m), 2.90-3.01 (2H, m), 3.23 (6H, s), 3.53-3.68 (4H, m), 4.05 (2H, s), 4.04-4.16 (2H, m), 6.65 (1H, s), 7.31 (1H, d, J=16.4Hz), 7.49 (1H, dd, J=2.0, 7.6Hz), 7.83 (1H, d, J=16.4Hz), 7.89-7.91 (1H, m), 8.89 (1H, d, J=7.6Hz).
IR (ATR) cm⁻¹: 3317, 2964, 2862, 1670, 1518, 1425, 1373, 1311, 1203, 1176, 1126, 1072, 1030.
ES-MS: m/z: 691 (M⁺).
Anal. Calcd. for C₃₂H₄₂N₁₂O₄S · 3H₂O · 3.5TFA: C, 40.95; H, 4.54; N, 14.69; S, 2.80.found: C, 40.83; H, 4.35; N, 14.91; S, 2.91.
m.p.: 151-158°C.

### Example 268: {2-[2-(1-{8-({[4-(tert-Butyl)-1,3-thiazol-2-yl]amino}carbonyl)-4-oxo-3-[(E)-2-(1H-tetrazol-5-yl)vinyl]-4H-pyrido[1,2-a]pyrimidin-2-yl}piperidin-4-yl)acetylamino]ethyl}carboxymethyldimethylammonium

### (A) tert-Butoxycarbonylmethyl-(2-{2-[1-(8-({[4-(tert-butyl)-1,3-thiazol-2-yl]-amino}carbonyl)-3-{(E)-2-[1-(4-methoxybenzyl)-1H-tetrazol-5-yl]vinyl}-4-oxo-4H-pyrido[1,2-a]pyrimidin-2-yl)piperidin-4-yl]acetylamino}ethyl)dimethylammonium

A solution of 2-{3-[(2-dimethylaminoethylaminocarbonyl)methyl]piperidin-1-yl}-3-{(E)-2-[1-(4-methoxybenzyl)-1H-tetrazol-5-yl]vinyl}-4-oxo-4H-pyrido[1,2-a]pyrimidine-8-carboxylic acid (4-tert-butylthiazol-2-yl)amide (87.5 mg) in N,N-dimethylformamide (2 ml) was added with bromoacetic acid tert butyl ester (18.9 µl) and stirred for 16 hours with light shielding. The reaction mixture was added with bromoacetic acid tert-butyl ester (18.9 µl), stirred for 31 hours with light shielding, further added with bromoacetic acid tert-butyl ester (18.9 µl) and stirred for 3.5 days with light shielding. The solvent was evaporated by azeotropy with toluene, and the residue was purified by preparative TLC (2 plates, chloroform:methanol:water = 8:3:0.5) to obtain the title compound (121 mg) as orange solid.
¹H-NMR (CDCl₃) δ : 1.30-1.55 (2H, m), 1.37 (9H, s), 1.47 (9H, s), 1.70-1.83 (2H, m), 2.06-2.20 (1H, m), 2.25-2.40 (2H, m), 2.87-3.04 (2H, m), 3.66 (6H, s), 3.72-3.87 (2H, s), 3.77 (3H, s), 4.00-4.15 (4H, m), 4.67 (2H, s), 5.53 (2H, s), 6.66 (1H, s), 6.90 (2H, d, J=8.4Hz), 7.25-7.41 (1H, m), 7.36 (2H, d, J=8.4Hz), 7.36 (1H, d, J=15.4Hz), 7.79 (1H, br), 7.90 (1H, d, J=15.4Hz), 8.72-8.86 (2H, m).

### (B) {2-[2-(1-{8-({[4-(tert-Butyl)-1,3-thiazol-2-yl]amino}carbonyl)-4-oxo-3-[(E)-2-(1H-tetrazol-5-yl)vinyl]-4H-pyrido[1,2-a]pyrimidin-2-yl}piperidin-4-yl)acetylamino]ethyl}carboxymethyldimethylammonium

tert-Butoxycarbonylmethyl-(2-{2-[1-(8-({[4-(tert-butyl)-1,3-thiazol-2-yl]amino}carbonyl)-3-{(E)-2-[1-(4-methoxybenzyl)-1H-tetrazol-5-yl]vinyl}-4-oxo-4H-pyrido[1,2-a]pyrimidin-2-yl)piperidin-4-yl]acetylamino}ethyl)dimethylammonium (121 mg) was dissolved in 4 N hydrochloric acid in dioxane (2 ml) and stirred at room temperature 13 hours and 30 minutes. The solvent was evaporated by azeotropy with chloroform, and the residue was dissolved in trifluoroacetic acid (2 ml) and stirred at 80°C for 3 hours. The solvent was evaporated by azeotropy with toluene, and the residue was purified by preparative TLC (3 plates, chloroform:methanol:water = 8:3:0.5) to obtain the title compound (121 mg) as red solid.
¹H-NMR (CD₃OD) δ : 1.25 (9H, s), 1.32-1.45 (2H, m), 1.65-1.75 (2H, m), 1.91-2.04 (1H, m), 2.08-2.16 (2H, m), 2.97-3.07 (2H, m), 3.19 (6H, s), 3.50-3.57 (2H, m), 3.60-3.68 (2H, m), 3.76 (2H, s), 4.08-4.17 (2H, m), 6.64 (1H, s), 7.38 (1H, d, J=16.1Hz), 7.48 (1H, dd, J=1.8, 7.6Hz), 7.81 (1H, dd, J=16.1Hz), 7.89-7.92 (1H, m), 8.88 (1H, d, J=7.6Hz).
IR (ATR) cm⁻¹: 3332, 2964, 2866, 1657, 1635, 1516, 1439, 1404, 1373, 1348, 1203, 1178, 1128, 1070.
ES-MS m/z: 692 (MH)⁺.
Anal. Calcd. for C₃₂H₁₄N₁₁O₅S•3.5TFA•5H₂O:C, 39.66; H, 4.65; N, 13.05; S, 2.72. Found: C, 39.27; H, 4.24; N, 13.33; S, 2.72.
m.p.: 148-158°C.

### Example 269: (E)-3-(8-({[4-(tert-Butyl)-1,3-thiazol-2-yl]amino}carbonyl)-2-{4-[(2-hydroxyethylaminocarbonyl)methyl]piperidin-1-yl}-4-oxo-4H-pyrido[1,2-a]-pyrimidin-3-yl)-2-propenoic acid

### (A) (E)-3-[8-({[4-(tert-Butyl)-1,3-thiazol-2-yl]amino}carbonyl)-2-(4-ethoxycarbonylmethylpiperidin-1-yl)-4-oxo-4H-pyrido[1,2-a]pyrimidin-3-yl]-2-propenoic acid tert-butyl ester

The following reaction was performed under argon atmosphere. A solution of (E)-3-[8-({[4-(tert-butyl)-1,3-thiazol-2-yl]amino}carbonyl)-2-hydroxy-4-oxo-4H-pyrido[1,2-a]pyrimidin-3-yl]-2-propenoic acid tert-butyl ester (500 mg) in N,N-dimethylformamide (4 ml) was added with diisopropylethylamine (740 µl) and diphenylphosphoryl chloride (441 µl) at 0°C and stirred at 0°C for 30 minutes. The reaction mixture was added with piperidin-4-yl-acetic acid ethyl ester hydrochloride (441 mg) and diisopropylethylamine (740 µl) at the same temperature and stirred at 80°C for 1 hour. The reaction mixture was returned to room temperature, diluted with water and extracted with chloroform. The organic layer was dried over anhydrous magnesium sulfate, and the solvent was concentrated under reduced pressure. The residue was purified by silica gel column chromatography (35 g, chloroform → chloroform:ethyl acetate = 7:1, and 25g, hexane:ethyl acetate = 3:1) to obtain the title compound (494 mg) as a hardly separable mixture.

### (B) (E)-3-[8-({[4-(tert-Butyl)-1,3-thiazol-2-yl]amino}carbonyl)-2-(4-carboxymethylpiperidin-1-yl)-4-oxo-4H-pyrido[1,2-a]pyrimidin-3-yl]-2-propenoic acid tert-butyl ester

A solution of (E)-3-[8-({[4-(tert-butyl)-1,3-thiazol-2-yl]amino}carbonyl)-2-(4-ethoxycarbonylmethylpiperidin-1-yl)-4-oxo-4H-pyrido[1,2-a]pyrimidin-3-yl]-2-propenoic acid tert-butyl ester (494 mg) in tetrahydrofuran (2 ml) was added with 1 N aqueous sodium hydroxide (2.38 ml) and stirred at 50°C for 1 hour. Then the reaction mixture was added with 1 N aqueous sodium hydroxide (2.38 ml) four times with stirring at 50°C over 5 hours. The reaction mixture was returned to room temperature, then added with 1 N hydrochloric acid and adjusted to about pH 4. The reaction mixture was diluted with saturated brine and extracted with chloroform. The organic layer was dried over anhydrous magnesium sulfate, and the solvent was concentrated under reduced pressure. The residue was purified by silica gel column chromatography (20 g, hexane:ethyl acetate = 1:1 → ethyl acetate) to obtain the title compound (241 mg, 38% for the two steps) as orange solid.
¹H-NMR (CDCl₃) δ: 1.35 (9H, s), 1.48 (9H, s), 1.49-1.63 (2H, m), 1.94-2.02 (2H, m), 2.23-2.35 (1H, m), 2.51-2.58 (2H, m), 3.01-3.11 (2H, m), 3.95-4.04 (2H, m), 6.65 (1H, s), 7.13 (1H, d, J=15.7Hz), 7.44 (1H, d, J=15.7Hz), 7.54 (1H, dd, J=1.7, 7.3Hz), 8.25 (1H, d, J=1.7Hz), 9.01 (1H, d, J=7.3Hz).

### (C) (E)-3-(8-({[4-(tert-Butyl)-1,3-thiazol-2-yl]amino}carbonyl)-2-{4-[(2-hydroxyethylaminocarbonyl)methyl]piperidin-1-yl}-4-oxo-4H-pyrido[1,2-a]pyrimidin-3-yl)-2-propenoic acid tert-butyl ester

A solution of (E)-3-[8-({[4-(tert-butyl)-1,3-thiazol-2-yl] amino}carbonyl)-2-(4-carboxymethylpiperidin-1-yl)-4-oxo-4H-pyrido[1,2-a]pyrimidin-3-yl]-2-propenoic acid tert-butyl ester (120 mg) and ethanolamine (12.2 µl) in methylene chloride (2 ml) and N,N-dimethylformamide (2 ml) was added with triethylamine (70.2 µl), HOBt (29.9 mg) and WSCD · HCl (42.5 mg) with ice cooling and stirred at room temperature for 18 hours. The solvent was subjected to azeotropy with toluene, and the residue was diluted with chloroform and washed with 10% aqueous citric acid and saturated aqueous sodium hydrogencarbonate. The organic layer was dried over anhydrous magnesium sulfate, and the solvent was concentrated under reduced pressure. The residue was purified by preparative TLC (3 plates, chloroform:methanol = 10:1) to obtain the title compound (98.3 mg, 76%) as orange foamy syrup.
¹H-NMR (CDCl₃) δ : 1.28-1.44 (2H, m), 1.34 (9H, s), 1.51 (9H, s), 1.76-2.00 (3H, m), 2.07-2.24 (3H, m), 2.93-3.04 (2H, m), 3.44-3.54 (2H, m), 3.79 (2H, t, J=4.9Hz), 4.04-4.12 (2H, m), 6.52-6.60 (1H, m), 6.58 (1H, s), 7.01 (1H, d, J=15.6Hz), 7.35 (1H, d, J=15.6Hz), 7.43 (1H, dd, J=1.7, 7.4Hz), 7.86-7.91 (1H, m), 8.92 (1H, d, J=7.4Hz).

### (D) (E)-3-(8-({[4-(tert-Butyl)-1,3-thiazol-2-yl]amino}carbonyl)-2-{4-[(2-hydroxyethylaminocarbonyl)methyl]piperidin-1-yl}-4-oxo-4H-pyrido[1,2-a]pyrimidin-3-yl)-2-propenoic acid

(E)-3-(8-({[4-(tert-Butyl)-1,3-thiazol-2-yl]amino}carbonyl)-2-{4-[(2-hydroxyethylaminocarbonyl)methyl]piperidin-1-yl}-4-oxo-4H-pyrido[1,2-a]pyrimidin-3-yl)-2-propenoic acid tert-butyl ester (98.3 mg) was dissolved in 4 N hydrochloric acid in dioxane (2 ml) and stirred at room temperature for 12 hours and 30 minutes. As the reaction was not completed, the solvent was concentrated by azeotropy with toluene. The residue was dissolved in 4 N hydrochloric acid in dioxane (4 ml) and stirred at room temperature for 1 hour. As the reaction did not proceed, the solvent was concentrated by azeotropy with toluene, and the residue was dissolved in trifluoroacetic acid (2 ml) and stirred at room temperature for 1 hour and 30 minutes. The solvent was concentrated by azeotropy with toluene, and the residue was purified by preparative TLC (3 plates, chloroform:methanol:water = 8:3:0.5) to obtain the title compound (67.5 mg) as orange solid.
¹H-NMR (CD₃OD) δ : 1.35 (9H, s), 1.38-1.52 (2H, m), 1.78-1.89 (2H, m), 2.03-2.25 (3H, m), 3.08-3.20 (2H, m), 3.28-3.33 (2H, m), 3.60 (2H, t, J=5.9Hz), 4.16-4.24 (2H, m), 6.74 (1H, s), 7.03 (1H, d, J=15.5Hz), 7.56 (1H, dd, J=1.8, 7.3Hz), 7.58 (1H, d, J=15.5Hz), 7.99-8.00 (1H, m), 8.94 (1H, dd, J=0.7, 7.3Hz).
IR (ATR) cm⁻¹: 3323, 2929, 2868, 2848, 1658, 1514, 1442, 1427, 1365, 1313, 1282, 1225, 1190, 1101, 1063, 1005.
ES-MS: m/z: 583 (MH⁺).
Anal. Calcd. for C₂₈H₃₄N₆O₆S•1.75H₂O: C, 54.75; H, 6.15; N, 13.68; S, 5.22. Found: C, 54.54; H, 5.66; N, 13.43; S, 5.16.
m.p.: 173-183°C.

### Example 270: [2-([((3R)-1-{8-([4-(tert-Butyl)-1,3-thiazol-2-yl]aminocarbonyl)-4-oxo-3-[(E)-2-(1H-1,2,3,4-tetrazol-5-yl)-1-ethenyl]-4H-pyrido[1,2-a]pyrimidin-2-yl-hexahydro-3-pyridinyl}oxy)carbamoyl]amino)ethyl](carboxymethyl)dimethylammonium

### (A) [2-([((3R)-1-{8-([4-(tert-Butyl)-1,3-thiazol-2-yl]aminocarbonyl)-4-oxo-3-[(E)-2-{1-(4-methoxybenzyl)-1H-1,2,3,4-tetrazol-5-yl}-1-ethenyl]-4H-pyrido[1,2-a]pyrimidin-2-ylhexahydro-3-pyridinyl}oxy)carbamoyl]amino)ethyl](carboxymethyl)dimethylammonium

(3R)-1-(8-([4-(tert-Butyl)-1,3-thiazol-2-yl]aminocarbonyl)-3-(E)-2-[1-(4-methoxycarbonyl)-1H-1,2,3,4-tetrazol-5-yl]-1-ethenyl-4-oxo-4H-pyrido[1,2-a]pyrimidin-2-yl)hexahydro-3-pyridinyl N-[2-(dimethylamino)ethyl]carbamate (405 mg, 0.536 mmol) was dissolved in N,N-dimethylformamide (8 ml), added with t-butyl bromoacetate (0.396 ml, 2.67 mmol) and stirred overnight at room temperature. The reaction mixture was concentrated under reduced pressure. After completion of the reaction was confirmed, the resulting residue was dissolved in 4 N hydrochloric acid in dioxane (10 ml) and stirred at room temperature for 9 hours. The reaction mixture was concentrated under reduced pressure, and the resulting residue was purified by preparative TLC (chloroform:methanol:water = 8:3:1) to obtain the title compound (329 mg, 72%).
¹H-NMR (CD₃OD) δ : 1.32 (9H, s), 1.60 (1H, m), 1.88 (3H, m), 2.03 (1H, m), 3.23-3.26 (8H, m), 3.52-3.60 (4H, m), 3.67-3.74 (6H, m), 3.82-3.85 (2H, m), 5.52 (2H, m), 6.64 (1H, m), 6.86 (2H, m), 7.27 (2H, m), 7.47 (1H, m), 7.65 (2H, m), 7.82 (1H, m), 8.78 (1H, m).
ES-MS: m/z: 815 (M⁺)

### (B) [2-([((3R)-1-{8-([4-(tert-Butyl)-1,3-thiazol-2-yl]aminocarbonyl)-4-oxo-3-[(E)-2-(1H-1,2,3,4-tetrazol-5-yl)-1-ethenyl)-4H-pyrido[1,2-a]pyrimidin-2-ylhexahydro-3-pyridinyl}oxy)carbamoyl]amino)ethyl](carboxymethyl)dimethylammonium

[2-([((3R)-1-{8-([4-(tert-Butyl)-1,3-thiazol-2-yl)aminocarbonyl)-4-oxo-3-[(E)-2-{1-(4-methoxybenzyl)-1H-1,2,3,4-tetrazol-5-yl}-1-ethenyl]-4H-pyrido[1,2-a]pyrimidin-2-yl-hexahydro-3-pyridinyl}oxy)carbamoyl]amino)ethyl](carboxymethyl)dimethylammonium (329 mg, 0.387 mmol) was dissolved in trifluoroacetic acid (10 ml), added with anisole (three drops) and stirred at 60°C for 2 hours. The reaction mixture was left stand for cooling, concentrated under reduced pressure and subjected to azeotropy with toluene several times. The resulting residue was purified by preparative TLC (chloroform:methanol:water = 8:3:1, developed three times) and eluted from silica gel with chloroform:methanol:water = 6:4:1 to obtain trifluoroacetic acid salt of the title compound (328 mg). This compound was dissolved in methanol/water, and the solution was adjusted to pH = 8 with 0.1 N aqueous sodium hydroxide, purified by HPLC and lyophilized to obtain the title compound (103 mg, 38%).
HPLC
Column: SHISEIDO CAP CELL PAK C18, SG-120A, 30 x 250 mm
Elute: methanol:water = 60:40
FR: 8 ml/min
Retention time: 23 min.
¹H-NMR (DMSO-d₆) δ : 1.30 (9H, s), 1.69 (1H, m), 1.85 (2H, m), 2.00 (1H, m), 3.20 (6H, s), 3.45 (2H, m), 3.61-3.69 (8H, m), 4.69 (1H, br), 6.71 (1H, brs), 7.55 (1H, d, J=15.9Hz), 7.70 (1H, d, J=7.1Hz), 7.99 (1H, d, J=15.9Hz), 8.17 (1H, m), 8.78 (1H, br), 8.96 (1H, d, J=7.6Hz).
¹H-NMR ((CD₃OD) δ: 1.29 (9H, s), 1.69 (1H, m), 1.94 (2H, m), 2.03 (1H, m), 3.26 (6H, s), 3.44-3.71 (6H, m), 3.84 (2H, s), 3.92 (1H, m), 4.06 (1H, m), 6.71 (1H, s), 7.45 (1H, d, J=15.9Hz), 7.64 (1H, dd, J=1.7and7.6Hz), 7.94 (1H, d, J=15.9Hz), 8.04 (1H, d, J=1.7Hz), 8.96 (1H, d, J=7.6Hz)
IR (ATR) cm⁻¹: 2958, 1700, 1650, 1625, 1511, 1423, 1247, 1103.
m.p.: 220-233°C (decomp.)
ES-MS: m/z: 694 (M⁺)
Anal. Calcd. for C₃₁H₃₉N₁₁O₆S•6H₂O: C, 46.43; H, 6.41; N, 19.21. Found: C, 46.48; H, 5.86; N, 18.66.

### Example 271: [2-({[((3R)-1-{8-({[4-(tert-Butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}hexahydro-3-pyridinyl)oxy]carbonyl}amino)ethyl](2-fluoroethyl)dimethylammonium

### (A) [2-({[((3R)-1-{8-({[4-(tert-Butyl)-1,3-thiazol-2-yl]amino}carbonyl)-4-oxo-3-[(E)-2-{1-(4-methoxycarbonyl)-1H-1,2,3,4-tetrazol-5-yl}-1-ethenyl]-4H-pyrido[1,2-a]-pyrimidin-2-yl}hexahydro-3-pyridinyl)oxy]carbonyl}amino)ethyl](2-fluoroethyl)-dimethylammonium

(3R)-1-(8-([4-(tert-Butyl)-1,3-thiazol-2-yl]aminocarbonyl)-3-(E)-2-[1-(4-methoxycarbonyl)-1H-1,2,3,4-tetrazol-5-yl]-1-ethenyl-4-oxo-4H-pyrido[1,2-a]pyrimidin-2-yl)hexahydro-3-pyridinyl N-[2-(dimethylamino)ethyl]carbamate (323 mg, 0.427 mmol) was dissolved in N,N-dimethylformimidazole-4-yl (6 ml), added with 1-bromo-2-fluoroethane (0.159 ml, 2.14 mmol) and stirred overnight at room temperature. The reaction mixture was further added with 1-bromo-2-fluoroethane (0.159 ml, 2.14 mmol) and stirred at 80°C for 2 days. The reaction mixture was concentrated under reduced pressure, and the resulting residue was purified by preparative TLC (chloroform:methanol:water = 8:3:1) to obtain the title compound (322 mg, 85%).
¹H-NMR (CD₃OD) δ : 1.22 (9H, s), 1.53 (1H, m), 1.79 (3H, m), 3.16 (6H, s), 3.11-3.24 (4H, m), 3.42-3.66 (2H, m), 3.62 (3H, s), 3.78 (2H, m), 4.87 (2H, d, J=49.3Hz), 5.41 (2H, s), 6.54 (1H, s), 6.77 (2H, d, J=8.5Hz), 7.18 (2H, d, J=8.5Hz), 7.39 (1H, m), 7.55 (2H, m), 7.69 (1H, m), 8.55 (1H, m).
ES-MS: m/z: 802 (M+)

### (B) [2-({[((3R)-1-{8-({[4-(tert-Butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}hexahydro-3-pyridinyl)oxy]carbonyl}amino)ethyl](2-fluoroethyl)dimethylammonium

[2-({[((3R)-1-{8-({[4-(tert-Butyl)-1,3-thiazol-2-yl]amino}carbonyl)-4-oxo-3-[(E)-2-{1-(4-methoxycarbonyl)-1H-1,2,3,4-tetrazol-5-yl}-1-ethenyl]-4H-pyrido[1,2-a]pyrimidin-2-yl}hexahydro-3-pyridinyl)oxy]carbonyl}amino)ethyl](2-fluoroethyl)-dimethylammonium (322 mg, 0.365 mmol) was dissolved in trifluoroacetic acid (10 ml), added with anisole (5 drops) and stirred at 60°C for 5 hours. The reaction mixture was left stand for cooling, concentrated under reduced pressure and subjected to azeotropy with toluene several times. The resulting residue was purified by preparative TLC (chloroform:methanol:water = 8:3:1, developed three times) and eluted from silica gel with chloroform:methanol:water = 6:4:1 to obtain trifluoroacetic acid salt of the title compound (145 mg). This compound was dissolved in methanol/water, and the solution was adjusted to pH = 8 with 0.1 N aqueous sodium hydroxide, purified by HPLC and lyophilized to obtain the title compound (98.0 mg, 39%).
HPLC
Column: SHISEIDO CAPCELL PAK C18, SG-120A, 30 × 250 mm
Elute: methanol:water = 70:30
FR: 12 ml/min
Retention time: 32 min.
¹H-NMR (CD₃OD) δ: 1.35 (9H, s), 1.71 (1H, m), 1.92-2.01 (3H, m), 3.22 (6H, s), 3.40 (1H, m), 3.51-3.60 (5H, m), 3.79 (1H, m), 3.88-3.95 (3H, m), 4.95 (2H, d, J=47.0Hz), 6.74 (1H, s), 7.56 (1H, d, J=16.1Hz), 7.60 (1H, dd, J=1.7 and 7.6Hz), 7.96 (1H, d, J=16.1Hz), 8.01 (1H, d, J=1.7Hz), 8.98 (1H, d, J=7.6Hz)
IR (ATR) cm⁻¹: 2958, 1658, 1513, 1425, 1249, 1103.
ES-MS: m/z: 682 (MH⁺), 680 (MH⁻)
Anal. Calcd. for C₃₁H₄₀N₁₁O₄FS·3H₂O: C, 50.60; H, 6.30; N, 20.94; F, 2.58. Found: C, 51.13; H, 6.24; N, 20.21; F, 2.62.

### Example 272: [2-({[((3R)-1-{8-({[4-(tert-Butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}hexahydro-3-pyridinyl)oxy]carbonyl}amino)ethyl](2-hydroxyethyl)dimethylammonium

### (A) [2-({[((3R)-1-{8-({[4-(tert-Butyl)-1,3-thiazol-2-yl]amino}carbonyl)-4-oxo-3-[(E)-2-{1-(4-methoxycarbonyl)-1H-1,2,3,4-tetrazol-5-yl]-1-ethenyl]-4H-pyrido[1,2-a)pyrimidin-2-yl}hexahydro-3-pyridinyl)oxy]carbonyl}amino)ethyl](2-hydroxyethyl)dimethylammonium

(3R)-1-(8-([4-(tert-Butyl)-1,3-thiazol-2-yl)aminocarbonyl)-3-(E)-2-[1-(4-methoxycarbonyl)-1H-1,2,3,4-tetrazol-5-yl]-1-ethenyl-4-oxo-4H-pyrido[1,2-a]pyrimidin-2-yl)hexahydro-3-pyridinyl N-[2-(dimethylamino)ethyl]carbamate (331 mg, 0.438 mmol) was dissolved in N,N-dimethylformamide (3 ml), added with 2-iodoethanol (0.171 ml, 2.19 mmol) and stirred overnight at room temperature. The reaction mixture was further added with 2-iodoethanol (0.171 ml, 2.19 mmol) and stirred at 80°C for 2 days. The reaction mixture was concentrated under reduced pressure, and the resulting residue was purified by preparative TLC (chloroform:methanol:water = 8:3:1) to obtain the title compound (465 mg, quantitative).
¹H-NMR (CD₃OD) δ: 1.35 (9H, s), 1.70 (1H, m), 1.95 (3H, m), 3.23 (6H, s), 3.54-3.71 (10H, m), 3.62 (3H, s), 5.41 (2H, s), 6.72 (1H, s), 6.92 (2H, d, J=8.7Hz), 7.32 (2H, d, J=8.7Hz), 7.61 (1H, m), 7.80 (2H, m), 7.97 (1H, m), 8.95 (1H, m).
ES-MS: m/z: 800 (M⁺)

### (B) [2-({[((3R)-1-[8-({[4-(tert-Butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}hexahydro-3-pyridinyl)oxy]carbonyl}amino)ethyl](2-hydroxyethyl)dimethylammonium

[2-({[((3R)-1-{8-({[4-(-Butyl)-1,3-thiazol-2-yl]amino}carbonyl)-4-oxo-3-[(E)-2-{1-(4-methoxycarbonyl)-1H-1,2,3,4-tetrazol-5-yl}-1-ethenyl]-4H-pyrido[1,2-a]pyrimidin-2-yl}hexahydro-3-pyridinyl)oxy]carbonyl}amino)ethyl](2-hydroxyethyl)dimethylammonium (465 mg, 0.501 mmol) was dissolved in trifluoroacetic acid (10 ml), added with anisole (three drops) and stirred at 60°C for 5 hours. The reaction mixture was left stand for cooling, concentrated under reduced pressure and subjected to azeotropy with toluene several times. The resulting residue was purified by preparative TLC (chloroform:methanol:water = 8:3:1, developed three times) and eluted from silica gel with chloroform:methanol:water = 6:4:1 to obtain trifluoroacetic acid salt of the title compound (190 mg). This compound was dissolved in methanol/water, and the solution was adjusted to pH = 8 with 0.1 N aqueous sodium hydroxide, purified by HPLC and lyophilized to obtain the title compound (107 mg, 31%).
HPLC
Column: SHISEIDO CAPCELL PAK C18, SG-120A, 30 x 250 mm
Elute: methanol:water = 70:30
FR: 12 ml/min
Retention time: 28 min.
¹H-NMR (CD₃OD) δ : 1.35 (9H, s), 1.70 (1H, m), 1.90-2.01 (3H, m), 3.20 (6H, s), 3.41-3.63 (8H, m), 3.90 (2H, m), 4.00 (2H, m), 6.73 (1H, s), 7.55 (1H, d, J=16.1Hz), 7.59 (1H, dd, J=1.5 and 7.6Hz), 7.93 (1H, d, J=16.1Hz), 8.00 (1H, d, J=1.5Hz), 8.97 (1H, d, J=7.6Hz)
IR (ATR) cm⁻¹: 3218, 2958, 1704, 1658, 1540, 1513, 1425, 1247, 1226
ES-MS: m/z: 680 (M⁺), 679 (MH⁻)
Anal. Calcd. for C₃₁H₄₁N₁₁O₅S · 3.25H20: C, 50.43; H, 6.48; N, 20.87. Found: C, 51.18; H, 6.28; N, 19.96.

### Example 273: [2-({[((3R)-1-{8-({[4-(tert-Butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}hexahydro-3-pyridinyl)oxy]carbonyl}amino)ethyl](2,2-difluoroethyl)-dimethylammonium

### (A) [2-({[((3R)-1-{8-({[4-(tert-Butyl)-1,3-thiazol-2-yl]amino}carbonyl)-4-oxo-3-[(E)-2-{1-(4-methoxycarbonyl)-1H-1,2,3,4-tetrazol-5-yl}-1-ethenyl]-4H-pyrido[1,2-a]pyrimidin-2-yl}hexahydro-3-pyridinyl)oxy]carbonyl}amino)ethyl](2,2-difluoroethyl)-dimethylammonium

(3R)-1-(8-([4-(tert-Butyl)-1,3-thiazol-2-yl]aminocarbonyl)-3-(E)-2-[1-(4-methoxycarbonyl)-1H-1,2,3,4-tetrazol-5-yl]-1-ethenyl-4-oxo-4H-pyrido[1,2-a]pyrimidin-2-yl)hexahydro-3-pyridinyl N-[2-(dimethylamino)ethyl]carbamate (343 mg, 0.454 mmol) was dissolved in N,N-dimethylformamide (3 ml), added with 2-bromo-1,1-difluoroethane (0.180 ml, 2.27 mmol) and stirred overnight at room temperature. The reaction mixture was added with 2-bromo-1,1-difluoroethane (0.180 ml, 2.27 mmol) and stirred at 80°C for 9 days. The reaction mixture was concentrated under reduced pressure, and the resulting residue was purified by preparative TLC (chloroform:methanol:water = 8:3:1) to obtain the title compound (210 mg, 51%).
¹H-NMR (CDCl₃) δ : 1.37 (9H, s), 1.57 (1H, m), 1.82 (3H, m), 3.18 (2H, m), 3.48 (3H, s), 3.72 (2H, m), 3.83 (2H, m), 4.04 (1H, m), 4.47 (1H, m), 4.73 (1H, m), 5.49 (2H, s), 6.56 (1H, s), 6.74 (1H, m), 6.90 (2H, d, J=8.3Hz), 7.35 (2H, d, J=8.3Hz), 7.41 (1H, m), 7.88 (3H, m), 8.84 (1H, m).
ES-MS: m/z: 820 (M⁺)

### (B) [2-({[((3R)-1-{8-({[4-(tert-Butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}hexahydro-3-pyridinyl)oxy]carbonyl}amino)ethyl](2, 2-difluoroethyl)dimethylammonium

[2-({[((3R)-1-{8-({[4-(tert-Butyl)-1,3-thiazol-2-yl]amino}carbonyl)-4-oxo-3-[(E)-2-{1-(4-methoxycarbonyl)-1H-1,2,3,4-tetrazol-5-yl}-1-ethenyl]-4H-pyrido[1,2-a]pyrimidin-2-yl}hexahydro-3-pyridinyl)oxy]carbonyl}amino)ethyl] (2,2-difluoroethyl)-dimethylammonium (210 mg, 0.501 mmol) was dissolved in trifluoroacetic acid (5 ml), added with anisole (5 drops) and stirred at 60°C for 5 hours. The reaction mixture was left stand for cooling, concentrated under reduced pressure and subjected to azeotropy with toluene several times. The resulting residue was purified by preparative TLC (chloroform:methanol:water = 8:3:1, developed three times) and eluted from silica gel with chloroform:methanol:water = 6:4:1 to obtain trifluoroacetic acid salt of the title compound (134 mg). This compound was dissolved in methanol/water, and the solution was adjusted to pH = 8 with 0.1 N aqueous sodium hydroxide, purified by HPLC and lyophilized to obtain the title compound (48.3 mg, 30%).
HPLC
Column: SHISEIDO CAPCELL PAK C18, SG-120A, 30 x 250 mm
Elute: methanol:water = 70:30
FR: 12 ml/min
Retention time: 35 min.
¹H-NMR (CD₃OD) δ : 1.35 (9H, s), 1.71 (1H, m), 1.86 (1H, m), 2.02 (1H, m), 3.30 (6H, s), 3.45 (1H, m), 3.60 (5H, m), 3.95-4.15 (4H, m), 6.60 (1H, t, J=49.5Hz), 6.74 (1H, s), 7.56 (1H, d, J=16.1Hz), 7.61 (1H, d, J=7.8Hz), 7.98 (1H, d, J=16.1Hz), 8.03 (1H, s), 8.99 (1H, d, J=7.8Hz)
IR (ATR) cm⁻¹: 2960, 1700, 1654, 1517, 1425, 1249
ES-MS: m/z: 700 (M⁺)
Anal. Calcd. for C₃₁H₃₉F₂N₁₁O₄S·1C₂H₅OH·2.75H₂O: C, 49.83; H, 6.40; N, 19.37; F, 4.78. Found: C, 50.17; H, 5.98; N, 18.93; F, 4.74.

### Example 274: 2-{2-[({[((3R)-1-{8-({[4-(tert-Butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}hexahydro-3-pyridinyl)oxy]carbonyl}amino)methyl]-1-pyridinium-yl}acetate

### (A) (3R)-1-(8-([4-(tert-Butyl)-1,3-thiazol-2-yl]aminocarbonyl)-3-(E)-2-[1-(4-methoxybenzyl)-1H-1,2,3,4-tetrazol-5-yl]-1-ethenyl-4-oxo-4H-pyrido[1,2-a]pyrimidin-2-yl)hexahydro-3-pyridinyl N-(2-pyridylmethyl)carbamate

N⁸-[4-(tert-Butyl)-1,3-thiazol-2-yl]-2-[(3R)-3-hydroxyhexahydro-1-pyridinyl]-3-{(E)-2-[1-(4-methoxybenzyl)-1H-1,2,3,4-tetrazol-5-yl]-1-ethenyl}-4-oxo-4H-pyrido(1,2 -a]pyrimidine-8-carboxamide (808 mg, 1.26 mmol) was dissolved in methylene chloride (16 ml), added with 1,1'-carbonyldiimidazole (306 mg, 1.89 mmol) and stirred at room temperature for 1 hour. The reaction mixture was added with 2-(aminomethyl)pyridine (0.260 ml, 2.52 mmol) and further stirred overnight. The reaction mixture was diluted with chloroform and washed with 1 N hydrochloric acid, saturated aqueous sodium hydrogencarbonate and saturated brine, and the organic layer was dried over anhydrous magnesium sulfate. The solvent was evaporated and concentrated, and the resulting residue was purified by silica gel column chromatography (chloroform:methanol = 100:1) to obtain the title compound (813 mg, 83%).
¹H-NMR (CDCl₃) δ : 1.33 (9H, s), 1.97 (3H, m), 3.79 (3H, s), 4.42 (1H, m), 4.58 (2H, m), 4.92 (1H, m), 5.40 (1H, m), 5.53 (2H, m), 6.60 (1H, m), 6.89 (2H, m), 7.45 (1H, m), 7.52 (1H, m), 7.90 (3H, m), 8.50 (2H, m), 8.97 (1H, m).
ES-MS: m/z: 776 (MH⁺)

### (B) 2-{2-[({[((3R)-1-{8-({[4-(tert-Butyl)-1,3-thiazol-2-yl]amino}carbonyl)-4-oxo-3-[(E)-2-[1-(4-methoxybenzyl)-1H-1,2,3,4-tetrazol-5-yl]-1-ethenyl]-4H-pyrido[1,2-a]-pyrimidin-2-yl}hexahydro-3-pyridinyl)oxy]carbonyl}amino)methyl]-1-pyridinium-yl}acetate

(3R)-1-(8-([4-(tert-Butyl)-1,3-thiazol-2-yl]aminocarbonyl)-3-(E)-2-[1-(4-methoxybenzyl)-1H-1,2,3,4-tetrazol-5-yl]-1-ethenyl-4-oxo-4H-pyrido[1,2-a]pyrimidin-2-yl)hexahydro-3-pyridinyl N-(2-pyridylmethyl)carbamate (813 mg, 1.05 mmol) was dissolved in N,N-dimethylformamide (10 ml), added with t-butyl bromoacetate (2.32 ml, 15.7 mmol) and stirred overnight at room temperature. The reaction mixture was concentrated under reduced pressure, and the resulting residue was dissolved in 4 N hydrochloric acid in dioxane (15 ml) and stirred overnight at room temperature. The reaction mixture was concentrated under reduced pressure, and the resulting residue was purified by preparative TLC (chloroform:methanol:water = 8:3:1) to obtain the title compound (515 mg, 59%).
ES-MS: m/z: 834 (M⁺)

### (C)2-{2-[({[((3R)-1-{8-({[4-(tert-Butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}hexahydro-3-pyridinyl)oxy]carbonyl}amino)methyl]-1-pyridinium-yl}acetate

2-{2-[({[((3R)-1-{8-({[4-(tert-Butyl)-1,3-thiazol-2-yl]amino}carbonyl)-4-oxo-3-[(E)-2-[1-(4-methoxybenzyl)-1H-1,2,3,4-tetrazol-5-yl]-1-ethenyl]-4H-pyrido[1,2-a]pyrimidin-2-yl)hexahydro-3-pyridinyl)oxy]carbonyl}amino)methyl]-1-pyridiniumyl}acetate (515 mg, 0.618 mmol) was dissolved in trifluoroacetic acid (13 ml), added with anisole (0.2 ml) and stirred at 60°C for 4 hours. The reaction mixture was left stand for cooling, concentrated under reduced pressure and subjected to azeotropy with toluene several times. The resulting residue was purified by preparative TLC (chloroform:methanol:water = 8:3:1, develop three times) and eluted from silica gel with chloroform:methanol:water = 6:4:1 to obtain trifluoroacetic acid salt of the title compound (413 mg). This compound was dissolved in methanol/water, and the solution was adjusted to pH = 8 with 0.1 N aqueous sodium hydroxide, purified by
HPLC and lyophilized to obtain the title compound (10.2 mg, 2%).
HPLC
Column: SHISEIDO CAPCELL PAK C18, SG-120A, 30 x 250 mm
Elute: methanol:water = 60:40
FR: 12 ml/min
Retention time: 17 min.
¹H-NMR (CD₃OD) δ : 1.35 (9H, s), 1.94.(2H, m), 2.03 (2H, m), 3.45 (1H, m), 3.72 (1H,. m), 3.92 (2H, m), 4.55 (1H, d, J=17.1Hz), 4.67 (1H, d, J=17.1Hz), 5.18 (1H, d, J=16.6Hz), 5.24 (1H, d, J=16.6Hz), 6.73 (1H; s), 7.43 (1H, d, J=16.1Hz), 7.56 (1H, d, J=5.6Hz), 7.86-7.91 (3H, m), 7.99 (1H, s), 8.46 (1H, m), 8.78 (1H, d, J=6.1Hz), 8.95 (1H, d, J=7.6Hz)
IR (ATR) cm⁻¹: 2925, 1708, 1654, 1511, 1427, 1365, 1249, 1222
ES-MS: m/z: 714 (M⁺)
Anal. Calcd. for C₃₃H₃₅N₁₁O₆S•2C₂H₅OH•4H₂O: C, 50.61; H, 6.31; N, 17.55. Found: C, 50.68; H, 5.76; N, 17.19.

### Example 275: 2-{2-(2S)-[({[((3R)-1-{8-({[4-(tert-Butyl)-1,3-thiazol-2-yl]amino)carbonyl)-4-oxo-3-[(E)-2-(2H-1,2,3,4-tetrazol-5-yl)-1-ethenyl]-4H-pyrido[1,2-a]pyrimidin-2-yl}hexahydro-3-pyridinyl)oxy]carbonyl}amino)methyl]-1-ethyltetrahydro-1H-1-pyrrolium-yl}acetate

### (A) (3R)-1-8-([4-(tert-Butyl)-1,3-thiazol-2-yl]aminocarbonyl)-4-oxo-3-[(E)-2-{1-(4-methoxybenzyl)-1H-1,2,3,4-tetrazol-5-yl)-1-ethenyl]-4H-pyrido[1,2-a]pyrimidin-2-yl-hexahydro-3-pyridinyl N-[(2S)-1-ethyltetrahydro-1H-2-pyrrol-yl]methylcarbamate

N⁸-[4-(tert-Butyl)-1,3 thiazol-2-yl]-2-[(3R)-3-hydroxyhexahydro-1-pyridinyl]-3-{(E)-2-[1-(4-methoxybenzyl)-1H-1,2,3,4-tetrazol-5-yl]-1-ethenyl}-4-oxo-4H-pyrido[1,2-a]pyrimidine-8-carboxamide (524 mg, 0.817 mmol) was dissolved in methylene chloride (10 ml), added with 1,1'-carbonyldiimidazole (199 mg, 1.22 mmol) and stirred at room temperature for 1 hour and 30 minutes. The reaction mixture was added with a solution of (S)-(-)-2-aminomethyl-1-ethylpyrrolidine (209 mg, 1.63 mmol) in methylene chloride (1 ml) and further stirred overnight. The reaction mixture was diluted with chloroform and washed with 1 N hydrochloric acid, saturated aqueous sodium hydrogencarbonate and saturated brine, and the organic layer was dried over anhydrous magnesium sulfate. The solvent was evaporated and concentrated, and the resulting residue was purified by silica gel column chromatography (chloroform:methanol = 100:3 → 100:5 → 10:1) to obtain the title compound (353 mg, 54%).
¹H-NMR (CDCl₃) δ : 1.10 (3H, m), 1.37 (9H, s), 1.64 (4H, m), 1.90 ((4H, m), 2.65 (1H, m), 2.84 (1H, m), 3.15 (3H, m), 3.31 (2H, m), 3.62 (2H, m), 3.77 (3H, s), 4.81 (1H, m), 5.48 (2H, s), 5.84 (1H, m), 6.56 (1H, s), 6.88 (1H, d, J=8.5Hz), 7.45 (1H, m), 7.76-7.90 (3H, m), 8.88 (1H, d, J=7.1Hz)
ES-MS: m/z: 796 (MH⁺)

### (B) 2-{2-(2S)-(({[((3R)-1-{8-({[4-(tert-Butyl)-1,3-thiazol-2-yl]amino}carbonyl)-4-oxo-3-[(E)-2-{1-(4-methoxybenzyl)-1H-1,2,3,4-tetrazol-5-yl}-1-ethenyl]-4H-pyrido[1,2-a]-pyrimidin-2-yl}hexahydro-3-pyridinyl)oxy]carbamoyl}amino)methyl]-1-ethyltetrahydro-1H-1-pyrrolium-yl}acetate

(3R)-1-{8-([4-(tert-Butyl)-1,3-thiazol-2-yl]amino}carbonyl}-4-oxo-3-[(E)-2-{1-(4-methoxybenzyl)-1H-1,2,3,4-tetrazol-5-yl}-1-ethenyl]-4H-pyrido[1,2-a]pyrimidin-2-yl-hexahydro-3-pyridinyl N-[(2S)-1-ethyltetrahydro-1H-2-pyrrolyl]methylcarbamate (353 mg, 0.443 mmol) was dissolved in N,N-dimethylformamide (6 ml), added with t-butyl bromoacetate (0.327 ml, 2.22 mmol) and stirred overnight at room temperature. The reaction mixture was further added with t-butyl bromoacetate (0.981 ml) and stirred for 2 days. The reaction mixture was further added with t-butyl bromoacetate (0.981 ml) and stirred at 80°C for 8 hours. The reaction mixture was concentrated under reduced pressure, and the resulting residue was dissolved in 4 N hydrochloric acid in dioxane (10 ml) and stirred overnight at room temperature. The reaction mixture was concentrated under reduced pressure, and the resulting residue was purified by preparative TLC (chloroform: methanol = 10:1) to obtain the title compound (109 mg, 29%) and the starting substance (180 mg).
ES-MS: m/z: 854 (M⁺)

### (C) 2-{2-(2S)-[({[((3R)-1-{8-({[4-(tert-Butyl)-1,3-thiazol-2-yl]amino}carbonyl)-4-oxo-3-[(E)-2-(2H-1,2,3,4-tetrazol-5-yl)-1-ethenyl]-4H-pyrido[1,2-a]pyrimidin-2-yl}hexahydro-3-pyridinyl)oxy]carbonyl}amino)methyl]-1-ethyltetrahydro-1H-1-pyrrolium-yl}acetate

2-{2-(2S)-[({[((3R)-1-{8-({[4-(tert-Butyl)-1,3-thiazol-2-yl]amino}carbonyl)-4-oxo-3-[(E)-2-{1-(4-methoxybenzyl)-1H-1,2,3,4-tetrazol-5-yl}-1-ethenyl]-4H-pyrido[1,2-a]pyrimidin-2-yl}hexahydro-3-pyridinyl)oxy]carbamoyl}amino)methyl]-1-ethyltetrahydro-1H-1-pyrrolium-yl}acetate (54.2 mg, 0.0635 mmol) was dissolved in trifluoroacetic acid (2 ml), added with anisole (one drop) and stirred at 60°C for 4 hours. The reaction mixture was left stand for cooling, concentrated under reduced pressure and subjected to azeotropy with toluene several times. The resulting residue was purified by preparative TLC (chloroform:methanol:water = 8:3:1, developed three times) and eluted from silica gel with chloroform:methanol:water = 6:4:1 to obtain trifluoroacetic acid salt of the title compound (21.1 mg, 43%) as a lyophilized product. ¹H-NMR (CD₃OD) δ : 1.34 (3H, m), 1.35 (9H, s), 1.65 (1H, m), 1.92-1.99 (6H, m), 2.24 (1H, m), 3.49-4.06 (13H, m), 4.30 (1H, m), 6.73 (1H, s), 7.45 (1H, m), 7.56 (1H, m), 7.89-7.99 (2H, m), 8.95 (1H, m).
IR (ATR) cm⁻¹: 2962, 1668, 1633, 1513, 1432, 1249, 1203.
ES-MS: m/z: 734 (M⁺)
Anal. Calcd. for C₃₄H₄₃N₁₁O₆S•1C₂H₅OH•4.5H₂O•1CF₃COOH: C, 46.81; H, 6.10; N, 15.80. Found: C, 47.33; H, 5.56; N, 15.30.

### Example 276: 2-[1,1-Dimethyl-1-(2-{[(5-{4-oxo-3-[(E)-2-(2H-1,2,3,4-tetrazol-5-yl)-1-ethenyl]-8-[(1,3-thiazol-2-ylamino)carbonyl]-4H-pyrido[1,2-a]pyrimidin-2-yl}-4,5,6,7-tetrahydropyrido[4,3-d] [1,3]oxazol-2-yl)carbonyl]amino}ethyl)ammonio]acetate

### (A) Methyl 4, 5, 6, 7-tetrahydropyrido[4, 3-d][1,3]oxazole-2-carboxylate

5-(tert-Butyl[4,3-d][1,3]oxazole-2,5-dicarboxylate (622 mg, 2.20 mmol) was dissolved in methylene chloride (5 ml), added with trifluoroacetic acid (5 ml) and stirred at room temperature for 3 hour and 30 minutes. The solvent was concentrated, subjected to azeotropy with chloroform several times, diluted with chloroform and washed with saturated aqueous sodium hydrogencarbonate. The reaction mixture was extracted with chloroform again, and then the organic layer was dried over magnesium sulfate. The solvent was concentrated under reduced pressure to obtain the title compound (312 mg, 78%).
¹H-NMR (CDCl₃) δ : 2.69 (2H, m), 3.15 (2H, m), 3.98 (2H, m), 4.00 (3H, s).
MS: m/z: 183 (MH⁺)

### (B) Methyl 5-(8-([4-(tert-butyl)-1,3-thiazol-2-yl]aminocarbonyl)-3-(E)-2-[1-(4-methoxybenzyl)-1H-1,2,3,4-tetrazol-5-yl]-1-ethenyl-4-oxo-4H-pyrido[1,2-a]pyrimidin-2-yl)-4,5,6,7-tetrahydropyrido[4,3-d] [1,3]oxazole-2-carboxylate

N⁸-[4-(tert-Butyl)-1,3-thiazol-2-yl]-2-hydroxy-3-[(E)-2-[1-(4-methoxybenzyl)-1H-1,2,3,4-tetrazol-5-yl]-1-ethenyl}-4-oxo-4H-pyrido[1,2-a]pyrimidine-8-carboxamide (153 mg, 0.274 mmol) was suspended in N,N-dimethylformamide (4 ml), added with diphenylphosphoryl chloride (0.114 ml, 0.548 mmol) and N,N-diisopropylethylamine (0.191 ml, 1.09 mmol) at -10°C and then stirred for 15 minutes. After disappearance of the starting materials was confirmed by TLC, the reaction mixture was added with methyl 4,5,6,7-tetrahydropyrido[4,3-d][1,3]oxazole-2-carboxylate (100 mg, 0.549 mmol) and N,N-diisopropylethylamine (0.0956 ml, 0.549 mmol), heated to 80°C and stirred with heating for 1 hour. Then the reaction mixture was further added with methyl 4,5,6,7-tetrahydropyrido[4,3-d][1,3]oxazole-2-carboxylate (100 mg, 0.549 mmol) and stirred with heating for 1 hour. The reaction mixture was left stand for cooling, then diluted with chloroform and washed with 5% aqueous citric acid, saturated aqueous sodium hydrogencarbonate and saturated brine, respectively. After the chloroform layer was dried, the solvent was evaporated, and the resulting residue was purified by preparative TLC (chloroform:ethyl acetate = 1:1) to obtain the title compound (132 mg, 67%).
¹H-NMR (CDCl₃) δ : 1.51 (9H, s), 2.72 (2H, m), 3.58 (2H, m), 3.83 (3H, s), 4.03 (3H, s), 4.51 (2H, m), 5.64 (2H, s), 6.27 (1H, m), 6.98 (2H, m), 7.25 (1H, m), 7.47 (2H, m), 7.84 (1H, m), 7.90 (1H, m), 8.03 (1H, m), 8.61 (1H, m).
ES-MS: m/z: 723 (MH⁺), 721 (MH-)

### (C) 5-(8-([4-(tert-Butyl)-1,3-thiazol-2-yl]aminocarbonyl)-3-(E)-2-[1-(4-methoxybenzyl)-1H-1,2,3,4-tetrazol-5-yl]-1-ethenyl-4-oxo-4H-pyrido[1,2-a]pyrimidin-2-yl)-4, 5, 6, 7-tetrahydropyrido[4, 3-d][1,3]oxazole-2-carboxylic acid lithium salt

Methyl 5-(8-([4-(tert-butyl)-1,3-thiazol-2-yl]aminocarbonyl)-3-(E)-2-[1-(4-methoxybenzyl)-1H-1,2,3,4-tetrazol-5-yl]-1-ethenyl-4-oxo-4H-pyrido[1,2-a]pyrimidin-2-yl)-4,5,6,7-tetrahydropyrido[4,3-d][1,3]oxazole-2-carboxylate (282 mg, 0.390 mmol) was dissolved in tetrahydrofuran (8 ml) and water (2 ml), added with lithium hydroxide monohydrate (29.5 mg, 0.702 mmol) and stirred for 20 minutes. The reaction mixture was concentrated to obtain crude title compound (318 mg, quantitative) as a lithium salt. This compound was used in the following reaction without being further purified.

### (D) N²-[2-(Dimethylamino)ethyl]-5-(8-([4-(tert-butyl)-1,3-thiazol-2-yl]aminocarbonyl)-3-(E)-2-[1-(4-methoxybenzyl)-1H-1,2,3,4-tetrazol-5-yl]-1-ethenyl-4-oxo-4H-pyrido[1,2-a]pyrimidin-2-yl)-4,5,6,7-tetrahydropyrido[4,3-d][1,3]oxazole-2-carboxyamide

5-(8-([4-(tert-Butyl)-1,3-thiazol-2-yl]aminocarbonyl)-3-(E)-2-[1-(4-methoxybenzyl)-1H-1,2,3,4-tetrazol-5-yl]-1-ethenyl-4-oxo-4H-pyrido[1,2-a]pyrimidin-2-yl)-4,5,6,7-tetrahydropyrido[4,3-d][1,3]oxazole-2-carboxylic acid lithium salt (279 mg, 0.390 mmol) and N,N-dimethylethylenediamine (0.0643 ml, 0.586 mmol) were dissolved in methylene chloride (4 ml) and N,N-dimethylformamide (2 ml), added with 1-hydroxybenzotriazole (15.8 mg, 0.117 mmol) and 1-(dimethylaminopropyl)-3-ethylcarbodiimide (112 mg, 0.586 mmol) and stirred overnight at room temperature. The reaction mixture was further added with amine (0.129 ml), 1-hydroxybenzotriazole (31.6 mg) and 1-(dimethylaminopropyl)-3-ethylcarbodiimide hydrochloride (224 mg) and stirred at 50°C for 13 hours. The reaction mixture was diluted with chloroform and washed with water. The organic layer was dried over anhydrous sodium sulfate. The solvent was concentrated under reduced pressure, and the resulting residue was purified by preparative TLC (chloroform:methanol:water = 8:3:1) to obtain the title compound (119 mg, 39%).
¹H-NMR (CDCl₃) δ : 1.50 (9H, s), 2.30 (6H, s), 2.56 (2H, m), 2.73 (2H, m), 3.57 (2H, m), 3.62 (2H, m), 3.82 (3H, s), 4.50 (2H, m), 5.63 (2H, s), 6.36 (1H, s), 6.97 (2H, d, J=8.6Hz), 7.46 (2H, d, J=8.6Hz), 7.84 (1H, d, J=15.4Hz), 7.89 (1H, s), 8.01 (1H, d, J=15.4Hz), 8.64 (1H, m).
MS: m/z: 779 (MH⁺)

### (E) 2-[1,1-Dimethyl-1-(2-{[(5-{4-oxo-3-[(E)-2-{1-(4-methoxybenzyl)-1H-1,2,3,4-tetrazol-5-yl}-1-ethenyl]-8-[(1,3-thiazol-2-ylamino)carbonyl]-4H-pyrido[1,2-a]pyrimidin-2-yl}-4,5,6,7-tetrahydropyrido[4, 3-d] [1,3]oxazol2-yl)carbonyl]amino}ethyl)ammonio]acetate

N²-[2-(Dimethylamino)ethyl]-5-(8-([4-(tert-butyl)-1,3-thiazol-2-yl]aminocarbonyl)-3-(E)-2-[1-(4-methoxybenzyl)-1H-1,2,3,4-tetrazol-5-yl)-1-ethenyl-4-oxo-4H-pyrido[1,2-a]pyrimidin-2-yl)-4,5,6,7-tetrahydropyrido[4,3-d][1,3]oxazole-2-carboxamide (119 mg, 0.153 mmol) was dissolved in N,N-dimethylformamide (3 ml), added with t-butyl bromoacetate (0.113 ml, 0.764 mmol) and stirred overnight at room temperature. The reaction mixture was concentrated under reduced pressure, and the resulting residue was dissolved in 4 N hydrochloric acid in dioxane (10 ml) and stirred at room temperature for 4 hours. The reaction mixture was concentrated under reduced pressure, and the resulting residue was purified by preparative TLC (chloroform:methanol:water = 8:3:1) to obtain the title compound (106 mg, 83%).
¹H-NMR (GD₃OD/CDCl₃) δ : 1.38 (9H, s), 2.98 (2H, m), 3.34 (6H, s), 3.79 (3H, s), 3.87 (8H, m), 4.77 (2H, m), 5.62 (2H, s), 6.63 (1H, s), 6.92 (2H, d, J=8.5Hz), 7.37 (2H, d, J=8.5Hz), 7.68 (1H, d, J=7.3Hz), 7.74 (1H, d, J=15.6Hz), 7:93-(1H, d, J=15.6Hz), 8.12 (1H, s), 9.00 (1H, d, J=7.3Hz).
ES-MS: m/z: 837 (MH⁺)

### (F) 2-[1,1-Dimethyl-1-(2-{[(5-{4-oxo-3-[(E)-2-(2H-1,2,3,4-tetrazol-5-yl)-1-ethenyl]-8-[(1,3-thiazol-2-ylamino)carbonyl]-4H-pyrido[1,2-a)pyrimidin-2-yl}-4,5,6,7-tetrahydropyrido[4, 3-d][1,3]oxazol-2-yl)carbonyl]amino}ethyl)ammonio]acetate

2-[1,1-Dimethyl-1-(2-{[(5-{4-oxo-3-[(E)-2-{1-(4-methoxybenzyl)-1H-1,2,3,4-tetrazol-5-yl}-1-ethenyl]-8-[(1,3-thiazol-2-ylamino)carbonyl)-4H-pyrido[1,2-a]pyrimidin-2-yl}-4,5,6,7-tetrahydropyrido[4,3-d][1,3]oxazol-2-yl)carbonyl]amino}ethyl)ammonio]acetate (106 mg, 0.127 mmol) was dissolved in trifluoroacetic acid (3 ml), added with anisole (three drops) and stirred at 60°C for 6 hours. The reaction mixture was left stand for cooling, concentrated under reduced pressure and subjected to azeotropy with toluene several times. The resulting residue was purified by preparative TLC (chloroform:methanol:water = 8:3:1, develop four times) and eluted from silica gel with chloroform:methanol:water = 6:4:1 to obtain trifluoroacetic acid salt of the title compound (197 mg). This compound was dissolved in methanol/water, and the solution was adjusted to pH = 8 with 0.1 N aqueous sodium hydroxide, purified by HPLC and lyophilized to obtain the title compound (25.7 mg, 28%).
HPLC
Column: SHISEIDO CAPCELLPAK C18, SG-120A, 30 x 250 mm
Elute: methanol:water = 70: 30
FR: 8 ml/min
Retention time: 12 min.
¹H-NMR (CD₃OD) δ : 1.35 (9H, s), 3.31 (6H, s), 3.82 (4H, m), 3.89 (2H, s), 3.95 (2H, m), 6.73 (1H, s), 7.45 (1H, d, J=16.3Hz), 7.62 (1H, dd, J=1.7 and 7.6Hz), 8.02 (1H, d, J=16.3Hz), 8.09 (1H, d, J=1.7Hz), 9.03 (1H, d, J=7.6Hz).
IR (ATR) cm⁻¹: 2960, 1658, 1631, 1508, 1423, 1371, 1338, 1207
ES-MS: m/z: 717 (MH⁺), 715 (MH⁻)
Anal. Calcd. for C₃₂H₃₆N₁₂O₆S•6.5H₂O: C, 46.09; H, 5.92; N, 20.15; S, 3.85. Found: C, 46.27; H, 5.30; N, 19.62; S, 3.81.

### Example 277: N⁸-[4-(tert-Butyl)-1,3-thiazol-2-yl]-4-oxo-3-[(E)-2-(2H-1,2,3,4-tetrazol-5-yl)-1-ethenyl]-2-3-[(1,1,1-trimethylammonio)methyl]piperidino-4H-pyrido[1,2-a]-pyrimidine-8-carboxyamide

### (A) N⁸-[4-(tert-Butyl)-1,3-thiazol-2-yl]-2-{3-[(dimethylamino)methyl]piperidino}-3-{(E)-2-[1-(4-methoxybenzyl)-1H-1,2,3,4-tetrazol-5-yl]-1-ethenyl}-4-oxo-4H-pyrido[1,2-a]pyrimidine-8-carboxyamide

N⁸-[4-(tert-Butyl)-1,3-thiazol-2-yl]-2-hydroxy-3-{(E)-2-[1-(4-methoxybenzyl)-1H-1,2,3,4-tetrazol-5-yl]-1-ethenyl}-4-oxo-4H-pyrido[1,2-a]pyrimidine-8-carboxamide (281 mg, 0.503 mmol) was suspended in N,N-dimethylformamide (5 ml) and acetonitrile (5 ml), added with diphenylphosphoryl chloride (0.209 ml, 1.01 mmol) and N,N-diisopropylethylamine (0.350 ml, 2.01 mmol) at -10°C and then stirred for 15 minutes. After disappearance of the starting materials was confirmed by TLC, the reaction mixture was added with N,N-dimethyl-N-(3-piperidylmethyl)amine (216 mg, 1.01 mmol) and N,N-diisopropylethylamine (0.700 ml, 0.549 mmol), heated to 80°C and stirred with heating for 1 hour. Then the reaction mixture was further added with N,N-dimethyl-N-(3-piperidylmethyl)amine (108 mg, 0.505 mmol) and N,N-diisopropylethylamine (0.350 ml, 2.01 mmol) and stirred with heating for 1 hour. The reaction mixture was left stand for cooling, then diluted with chloroform and washed with saturated aqueous sodium hydrogencarbonate and saturated brine, respectively. After the chloroform layer was dried, the solvent was evaporated, and the resulting residue was purified by silica gel column chromatography (chloroform:methanol = 100:1 → 100:2) to obtain the title compound (319 mg, 92%). ¹H-NMR (CD₃OD/CDCl₃) δ : 1.35 (9H, s), 2.15 (1H, m), 2.20 (6H, s), 2.32 (1H, m), 2.82 (1H, m), 2.85 (2H, m), 3.19 (1H, m), 3.66 (2H, m), 3.79 (3H, s), 3.98 (1H, m), 4.24 (1H, m), 5.54 (1H, s), 6.62 (1H, s), 6.89 (2H, d, J=8.8Hz), 7.36 (2H, d, J=8.8Hz), 7.55 (1H, d, J=7.3Hz), 7.66 (1H, d, J=15.6Hz), 7.83 (1H, d, J=15.6Hz), 8.00 (1H, s), 8.95 (1H, d, J=7.3Hz).
ES-MS: m/z: 683 (MH⁺), 682 (MH⁻)

### (B) N⁸-[4-(tert-Butyl)-1,3-thiazol-2-yl)-3-{(E)-2-[1-(4-methoxybenzyl)-1H-1,2,3,4-tetrazol-5-yl]-1-ethenyl}-4-oxo-2-{3-[(1,1,1-trimethylammonio)methyl]piperidino}-4H-pyrido[1,2-a]pyrimidine-8-carboxyamide

N⁸-[4-(tert-Butyl)-1,3-thiazol-2-yl]-2-{3-[(dimethylamino)methyl]piperidino}-3-{(E)-2-[1-(4-methoxybenzyl)-1H-1,2,3,4-tetrazol-5-yl]-1-ethenyl}-4-oxo-4H-pyrido[1,2-a]pyrimidine-8-carboxamide (155 mg, 0.227 mmol) was dissolved in N,N-dimethylformamide (4 ml), added with methyl iodide (0.0707 ml, 1.13 mmol) and stirred at room temperature for 3 days. The reaction mixture was concentrated under reduced pressure, and the resulting residue was purified twice by preparative TLC (chloroform:methanol:water = 8:3:1) to obtain the title compound (103 mg, 55%).
¹H-NMR (CD₃OD) δ : 1.36 (9H, s), 1.56 (1H, m), 1.79 (2H, m), 2.05 (1H, m), 2.49 (1H, m), 3.05 (1H, m), 3.24 (9H, s), 3.36 (3H, m), 3.77 (3H, s), 3.91 (1H, m), 4.21 (1H, m), 5.63 (2H, s), 6.76 (1H, s), 6.93 (2H, d, J=8.8Hz), 7.35 (2H, d, J=8.8Hz), 7.69 (2H, m), 7.92 (1H, d, J=15.6Hz), 7.98 (1H, s), 9.05 (1H, d, J=7.6Hz)
ES-MS: m/z: 697 (MH⁺)

### (C) N⁸-[4-(tert-Butyl)-1,3-thiazol-2-yl]-2-(3-[1-(2-amino-2-oxoethyl)-1, 1-dimethylammonio]methylpiperidino)-3-{(E)-2-[1-(4-methoxybenzyl)-1H-1,2,3,4-tetrazol-5-yl]-1-ethenyl}-4-oxo-4H-pyrido[1,2-a]pyrimidine-8-carboxyamide

N⁸-[4-(tert-Butyl)-1,3-thiazol-2-yl]-3-{(E)-2-[1-(4-methoxybenzyl)-1H-1,2,3,4-tetrazol-5-yl]-1-ethenyl}-4-oxo-2-{3-[(1,1,1-trimethylammonio)methyl]piperidino}-4H-pyrido[1,2-a]pyrimidine-8-carboxamide (155 mg, 0.227 mmol) was dissolved in N,N-dimethylformamide (4 ml), added with iodoacetamide (207 mg, 1.12 mmol) and stirred at room temperature for 3 days. The reaction mixture was concentrated under reduced pressure, and the resulting residue was purified twice by preparative TLC (chloroform:methanol:water = 8:3:1) to obtain the title compound (252 mg, quantitative).
¹H-NMR (CD₃OD) δ : 1.36 (9H, s), 1.54 (1H, m), 1.79 (2H, m), 2.06 (1H, m), 2.50 (1H, m), 3.10 (1H, m), 3.29 (6H, s), 3.49 (1H, m), 3.63 (3H, m), 3.77 (3H, s), 3.90 (1H, m), 4.21 (1H, m), 5.63 (2H, s), 6.77 (1H, s), 6.93 (2H, d, J=8.8Hz), 7.35 (2H, d, J=8.8Hz), 7.68 (2H, m), 7.90 (1H, m), 8.01 (1H, m), 9.04 (1H, m).

### (D) N⁸-[4-(tert-Butyl)-1,3-thiazol-2-yl]-4-oxo-3-[(E)-2-(2H-1,2,3,4-tetrazol-5-yl)-1-ethenyl]-2-3-[(1,1,1-trimethylammonio)methyl]piperidino-4H-pyrido[1,2-a]pyrimidin-8-carboxyamide

N⁸-[4-(tert-Butyl)-1,3-thiazol-2-yl]-2-(3-[1-(2-amino-2-oxoethyl)-1,1-dimethylammonio]methylpiperidino)-3-{(E)-2-[1-(4-methoxybenzyl)-1H-1,2,3,4-tetrazol-5-yl]-1-ethenyl}-4-oxo-4H-pyrido[1,2-a]pyrimidine-8-carboxamide (103 mg, 0.125 mmol) was dissolved in trifluoroacetic acid (3 ml), added with anisole (one drop) and stirred at 60°C for 3 hours. The reaction mixture was left stand for cooling, concentrated under reduced pressure and subjected to azeotropy with toluene several times. The resulting residue was purified by preparative TLC (chloroform:methanol:water = 8:3:1, developed three times) and eluted from silica gel with chloroform:methanol:water = 6:4:1 and lyophilized to obtain trifluoroacetic acid salt of the title compound (68.8 mg, 80%).
¹H-NMR (CD₃OD) δ : 1.35 (9H, s), 1.40 (1H, m), 1.76 (2H, m), 1.97 (1H, m), 2.43 (1H, m), 2.83 (1H, m), 3.13 (9H, s), 3.24 (4H, m), 3.91 (1H, m), 4.01 (1H, m), 6.71 (1H, s), 7.32 (1H, d, J=15.8Hz), 7.52 (1H, m), 7.88 (2H, m), 8.85 (1H, m)
ES-MS: m/z: 577 (MH⁺), 575 (MH⁻)
m.p.: 188-197°C.

### Example 278: N⁸-[4-(tert-Butyl)-1,3-thiazol-2-yl]-2-(3-[1-(2-amino-2-oxoethyl)-1,1-dimethylammonio]methylpiperidino)-4-oxo-3-[(E)-2-(2H-1,2,3,4-tetrazol-5-yl)-1-ethenyl]-4H-pyrido[1,2-a]pyrimidine-8-carboxyamide

This compound was synthesized in the same manner as above.
¹H-NMR (CD₃OD) δ : 1.33 (9H, s), 1.36 (1H, m), 1.58 (1H, m), 1.71 (1H, m), 1.94 (1H, m), 2.38 (1H, m), 2.78 (1H, m), 2.98 (1H, m), 3.29 (6H, s), 3.51 (2H, m), 3.81 (1H, m), 3.98 (1H, m), 4.22 (2H, m), 6.65 (1H, s), 7.24 (1H, d, J=16.1Hz), 7.44 (1H, dd, J=1.7 and 7.6Hz), 7.71 (1H, d, J=1.7Hz), 7.80 (1H, d, J=16.1Hz), 8.73 (1H, d, J=7.6Hz)
ES-MS: m/z: 620 (M⁺)
m.p.: 164-175°C.

### Example 279: N⁸-[4-(tert-Butyl)-1,3-thiazol-2-yl]-4-oxo-3-[(E)-2-(1H-1,2,3,4-tetrazol-5-yl)-1-ethenyl]-2-[3-([2-(1,1,1-trimethylammonio)acetyl]aminomethyl)piperidino]-4H-pyrido[1,2-a]pyrimidine-8-carboxyamide

### (A) N⁸-[4-(tert-Butyl)-1,3-thiazol-2-yl]-2-[3-([2-(dimethylamino)acetyl]aminomethyl)-piperidino]-3-{(E)-2-[1-(4-methoxybenzyl)-1H-1,2,3,4-tetrazol-5-yl]-1-ethenyl]-4-oxo-4H-pyrido[1,2-a]pyrimidine-8-carboxyamide

N⁸-[4-(tert-Butyl)-1,3-thiazol-2-yl]-2-hydroxy-3-{(E)-2-[1-(4-methoxybenzyl)-1H-1,2,3,4-tetrazol-5-yl]-1-ethenyl}-4-oxo-4H-pyrido[1,2-a]pyrimidine-8-carboxamide (250 mg, 0.448 mmol) was suspended in N,N-dimethylformamide (5 ml) and acetonitrile (5 ml), added with diphenylphosphoryl chloride (0.186 ml, 0.895 mmol) and N,N-diisopropylethylamine (0.311 ml, 1.79 mmol) at -10°C and then stirred for 15 minutes. After disappearance of the starting materials was confirmed by TLC, the reaction mixture was added with N¹-(3-piperidylmethyl)-2-(dimethylamino)acetamide (244 mg, 0.895 mmol) and N,N-diisopropylethylamine (0.624 ml, 3.58 mmol), heated to 80°C and stirred with heating for 1 hour. Then the reaction mixture was further added with N¹-(3-piperidylmethyl)-2-(dimethylamino)acetamide (122 mg, 0.448 mmol) and N,N-diisopropylethylamine (0.624 ml, 3.58 mmol) and stirred with heating for 1 hour. The reaction mixture was left stand for cooling, diluted with chloroform and washed with saturated aqueous sodium hydrogencarbonate and saturated brine, respectively. After the chloroform layer was dried, the solvent was evaporated, and the resulting residue was purified by silica gel column chromatography (chloroform:methanol = 100:1 → 100:2) to obtain the title compound (153 mg, 46%).
¹H-NMR (CDCl₃) δ : 1.35 (9H, s), 1.62 (1H, m), 1.75 (1H, m), 2.18 (6H, s), 2.63 (1H, m), 2.85 (1H, m), 3.08-3.17 (3H, m), 3.32 (1H, m), 3.61 (1H, m), 3.78 (3H, s), 4.02 (1H, m), 5.52 (2H, s), 6.59 (1H, s), 6.89 (2H, d, J=8.5Hz), 7.36 (2H, m), 7.45 (1H, m), 7.72 (1H, d, J=15.4Hz), 7.90 (2H, m), 8.94 (1H, d, J=7.3Hz).
ES-MS: m/z: 740 (MH⁺)

### (B) N⁸-[4-(tert-Butyl)-1,3-thiazol-2-yl]-3{(E)-2-[1-(4-methoxybenzyl)-1H-1,2,3,4-tetrazol-5-yl]-1-ethenyl}-4-oxo-2-(3-([2-(1,1,1-trimethylammonio)acetyl]-aminomethyl)piperidino]-4H-pyrido[1,2-a]pyrimidine-8-carboxyamide

N⁸-[4-(tert-Butyl)-1,3-thiazol-2-yl]-2-[3-([2-(dimethylamino)acetyl]aminomethyl)piperidinol-3-{(E)-2-[1-(4-methoxybenzyl)-1H-1,2,3,4-tetrazol-5-yl]-1-ethenyl}-4-oxo-4H-pyrido[1,2-a]pyrimidine-8-carboxamide (77.0 mg, 0.227 mmol) was dissolved in N,N-dimethylformamide (2 ml), added with methyl iodide (0.032 ml, 0.520 mmol) and stirred at room temperature for 1 day. The reaction mixture was concentrated under reduced pressure, and the resulting residue was purified by preparative TLC (chloroform:methanol:water = 8:3:1) to obtain the title compound (51.2 mg, 56%).
¹H-NMR (CD₃OD) δ : 1.35 (9H, s), 1.68 (2H, m), 1.81 (1H, m), 1.96 (2H, m), 3.22 (2H, m), 3.30 (9H, s), 3.63 (2H, m), 3.76 (3H, s), 4.00 (2H, m), 4.11 (2H, s), 5.61 (2H, s), 6.73 (1H, s), 693 (2H, d, J=8.8Hz), 7.24 (2H, d, J=8.8Hz), 7.57 (2H, m), 7.78 (1H, d, J=15.6Hz), 7.93 (1H, s), 8.92 (1H, d, J=7.6Hz).
ES-MS: m/z: 754 (M⁺)

### (C) N⁸-[4-(tert-Butyl)-1,3-thiazol-2-yl]-4-oxo-3-[(E)-2-(1H-1,2,3,4-tetrazol-5-yl)-1-ethenyl]-2-[3-([2-(1,1,1-trimethylammonio)acetyl]aminomethyl)piperidino]-4H-pyrido[1,2-a]pyrimidine-8-carboxyamide

N⁸-[4-(tert-Butyl)-1,3-thiazol-2-yl]-3-{(E)-2-[1-(4-methoxybenzyl)-1H-1,2,3,4-tetrazol-5-yl]-1-ethenyl}-4-oxo-2-[3-([2-(1,1,1-trimethylammonio)acetyl]aminomethyl)-piperidino]-4H-pyrido[1,2-a]pyrimidine-8-carboxamide (51.2 mg, 0.0581 mmol) was dissolved in trifluoroacetic acid (3 ml), added with anisole (one drop) and stirred at 60°C for 3 hours. The reaction mixture was left stand for cooling, concentrated under reduced pressure and subjected to azeotropy with toluene several times. The resulting residue was purified twice by preparative TLC (chloroform:methanol:water = 8:3:1, developed three times), eluted from silica gel with chloroform:methanol:water = 6:4:1 and lyophilized to obtain the title compound (23.1 mg, 53%) as trifluoroacetic acid salt.
¹H-NMR (CD₃OD) δ : 1.34 (9H, s), 1.35 (1H, m), 1.75 (1H, m), 1.85 (2H, m), 2.08 (1H, m), 2.85 (1H, m), 3.03 (1H, m), 3.18 (9H, s), 3.20 (1H, m), 3.31 (2H, m), 3.43 (2H, m), 4.02 (1H, m), 4.18 (1H, m), 6.69 (1H, s), 7.28 (2H, d, J=16.1Hz), 7.46 (1H, m), 7.88 (2H, m), 8.80 (1H, d, J=7.6Hz).
ES-MS: m/z: 634 (M⁺) m.p.: 191-195°C
Anal. Calcd. for C₃₀H₄₀N₁₁O₃S•1CF₃COOH₆•5H₂O: C, 44.44; H, 6.18; N, 17.81. Found: C, 45.30; H, 5.70; N, 16.93.

### Example 280: [3-({[((3R)-1-{8-({[4-(tert-Butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}hexahydro-3-pyridinyl)oxy]carbonyl}amino)propyl](carboxymethyl)-dimethylammonium

### (A) [3-({[((3R)-1-{8-({[4-(tert-butyl)-1,3-thiazol-2-yl]amino}carbonyl)-4-oxo-3-{(E)-2-[1-(4-methoxybenzyl)-1H-1,2,3,4-tetrazol-5-yl]-l-ethenyl}-4H-pyrido[1,2-a]pyrimidin-2-yl}hexahydro-3-pyridinyl)oxy]carbonyl}amino)propyl](carboxymethyl)-dimethylammonium

(3R)-1-(8-({[4-(tert-Butyl)-1,3-thiazol-2-yl]amino}carbonyl)-3-{(E)-2-[1-(4-methoxybenzyl)-1H-1,2,3,4-tetrazol-5-yl]-1-ethenyl}-4-oxo-4H-pyrido[1,2-a]pyrimidin-2-yl)hexahydro-3-pyridinyl N-[3-(dimethylamino)propyl]carbamate (277 mg, 0.360 mmol) was dissolved in N,N-dimethylformamide (8 ml), added with t-butyl bromoacetate (0.266 ml,1.80 mmol) and stirred overnight at room temperature. The reaction mixture was further added with t-butyl bromoacetate (0.266 ml, 1.80 mmol) and stirred for 6 hours. The reaction mixture was concentrated under reduced pressure, and the resulting residue was dissolved in 4 N hydrochloric acid in dioxane (10 ml) and stirred overnight at room temperature. The reaction mixture was concentrated under reduced pressure, and the resulting residue was purified by preparative TLC (chloroform:methanol:water = 8:3:1) to obtain the title compound (274 mg, 88%).
¹H-NMR (CD₃OD) δ : 1.33 (9H, s), 1.65 (1H, m), 1.89 (5H, m), 3.15 (2H, m), 3.21 (6H, s), 3.30 (2H, m), 3.56 (3H, m), 3.70 (1H, m), 3.72 (3H, s), 3.80 (2H, s), 4.73 (1H, m), 5.53 (2H, s), 6.65 (1H, s), 6.88 (2H, d, J=8.8Hz), 7.28 (2H, d, J=8.8Hz), 7.48 (1H, dd, J=1.7and7.3Hz), 7.65 (1H, d, J=15.4Hz), 7.68 (1H, d, J=15.4Hz), 7.84 (1H, d, J=1.7Hz), 8.79 (1H, d, J=7.3Hz).
ES-MS: m/z: 828 (M⁺)

### (B) [3-({[((3R)-1-{8-({[4-(tert-Butyl)-1,3-thiazol-2-yl]amino}carbonyl)-4-oxo-3-[(E)-2-(1H-1,2,3,4-tetrazol-5-yl)-1-ethenyl]-4H-pyrido[1,2-a]pyrimidin-2-yl}hexahydro-3-pyridinyl)oxy]carbonyl}amino)propyl](carboxymethyl)dimethylammonium

[3-({[((3R)-1-{8-({[4-(tert-Butyl)-1,3-thiazol-2-yl]amino}carbonyl)-4-oxo-3-{(E)-2-[1-(4-methoxybenzyl)-1H-1,2,3,4-tetrazol-5-yl]-1-ethenyl}-4H-pyrido[1,2-a]pyrimidin-2-yl}hexahydro-3-pyridinyl)oxy]carbonyl]amino)propyl](carboxymethyl)dimethylammonium (274 mg, 0.317 mmol) was dissolved in trifluoroacetic acid (10 ml), added with anisole (three drops) and stirred at 60°C for 2 hours and 30 minutes. The reaction mixture was left stand for cooling, concentrated under reduced pressure and subjected to azeotropy with toluene several times. The resulting residue was purified by preparative TLC (chloroform:methanol:water = 8:3:1, developed three times) and eluted from silica gel with chloroform:methanol:water = 6:4:1 to obtain trifluoroacetic acid salt of the title compound (232 mg, quantitative) as lyophilized product.
¹H-NMR (DMSO-d₆) δ : 1.31 (9H, s), 1.66-1.93 (6H, m), 2.94-3.74 (16H, m), 4.65 (1H, m), 6.85 (1H, s), 7.47 (1H, d, J=16.3Hz), 7.62 (1H, d, J=7.3Hz), 7.76 (1H, br), 7.87 (1H, d, J=16.3Hz), 8.24 (1H, s), 8.96 (1H, d, J=7.3Hz).
m.p.: 157-163°C (dec.)
ES-MS: m/z: 707 (M⁺)
Anal. Calcd. for C₃₂H₄₁N₁₁O₆S•6H₂O•3CF₃COOH: C, 39.41; H, 4.87; N, 13.31. Found: C, 39.12; H, 4.43; N, 13.51.

### Test Example 1: Effect of combined use with antimicrobial agent on multidrug resistant Pseudomonas aeruginosa

As multidrug resistant Pseudomonas aeruginosa, Pseudomonas aeruginosa PAM 1723 strain highly expressing the drug efflux pump was used. As antimicrobial drugs for the combinational uses, levofloxacin (LVFX) as a quinolone antibacterial agent and aztreonam (AZT) as a monobactam antibiotic were used. Each of the compounds shown in Table 1 with Example No., which falls within the compounds of the present invention, was subjected to the measurement of its minimum concentration (µ g/ml) that was required to enhance the antibacterial activity of levofloxacin when the test compound was used in combination with levofloxacin applied at a concentration of 1/4, or 1/8 or less of the minimum inhibitory concentration against the PAM 1723 strain. As for aztreonam, a minimum concentration (µ g/ml) of each text compound was measured that was required to enhance its antibacterial activity when the test compound was used in combination with azthreonam at a concentration of 1/8 or less of the MIC. Effects as combinational uses for 18 hours was indicated for each of the drugs. Effects were determined by visually observing the turbidity of the medium. The Müller-Hinton broth (MHB, Difco) was used as a medium, and the inoculum of the bacteria was 1 × 10⁶ CFU/ml. As clearly shown in Table 1, the compounds of the present invention exhibited effect on the drug resistant Pseudomonas aeruginosa in the combinational uses mainly by inhibiting resistance due to the drug efflux pump, and accordingly, it can be concluded that the class of compounds are expected to be useful clinically.

**Table 1**

| Example No. | MPC4 LVFX | MPC8 LVFX | MPC8 AZT |
|---|---|---|---|
| 5 | 0.5 | 8 | 8 |
| 10 | 2 | 16 | 4 |
| 18 | ≦ 0.25 | 1 | 0.5 |
| 26 | 0.25 | 1 | 1 |
| 78 | 0.5 | 1 | 1 |
| 94 | 4 | 16 | 16 |
| 98 | 1 | 4 | 4 |
| 104 | 0.24 | 1 | 0.5 |
| 117 | 1 | 4 | 4 |
| 130 | 2 | 8 | 8 |
| 135 | 4 | 16 | 8 |
| 141 | 0.25 | 16 | 2 |
| 152 | 2 | 8 | 4 |
| 155 | 0.25 | 2 | 2 |
| 189 | 0.5 | 2 | 1 |
| 274 | 1 | 4 | 2 |
| 277 | 1 | 4 | 2 |

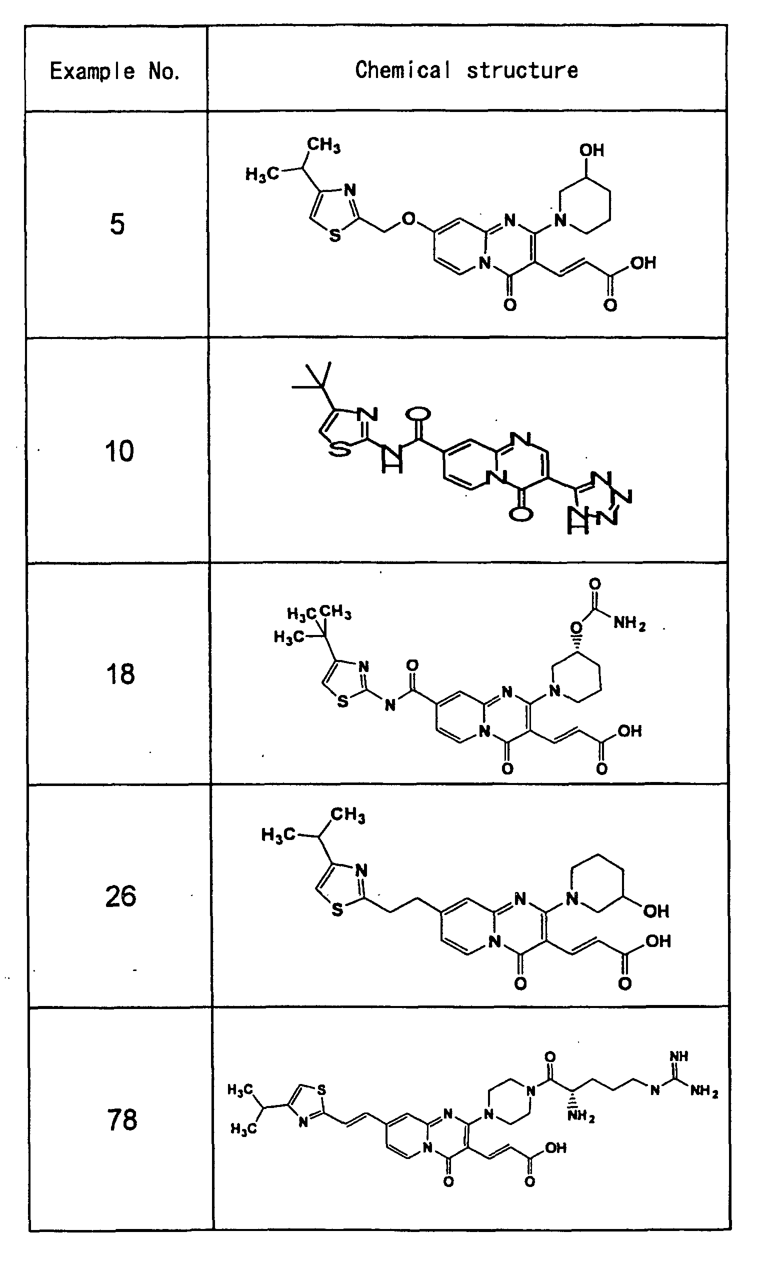

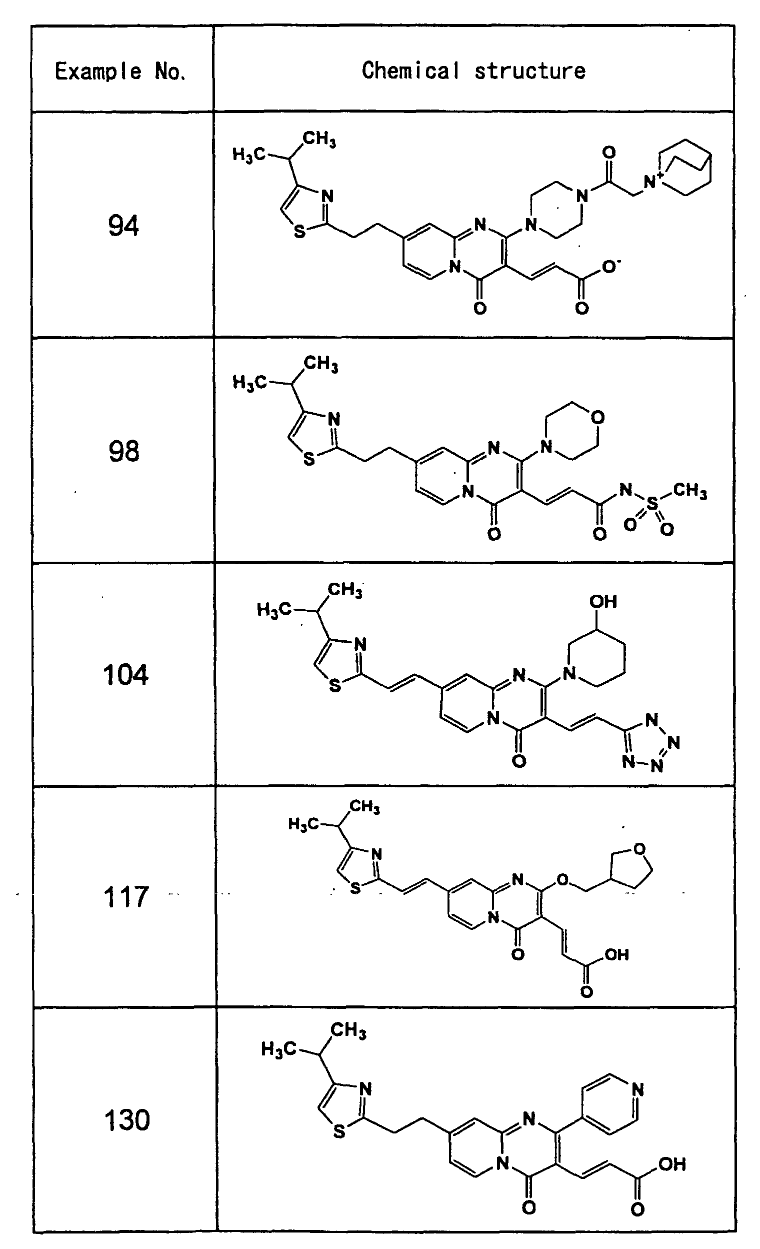

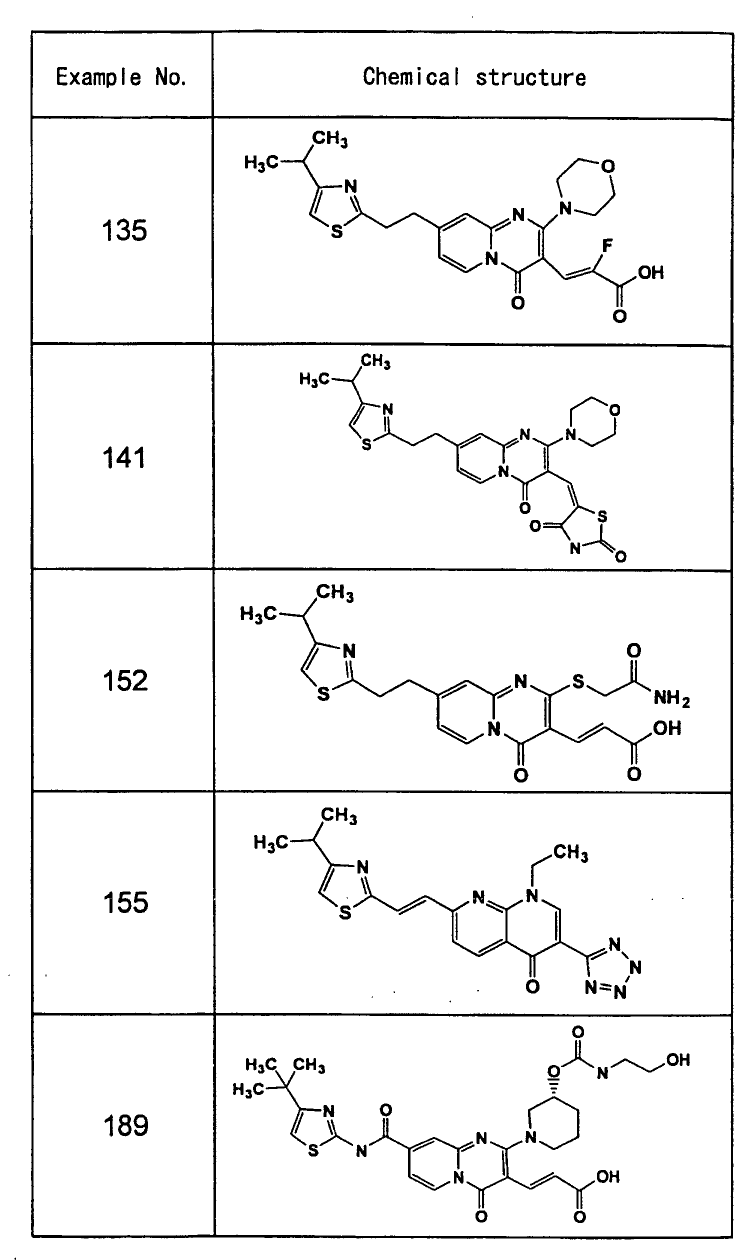

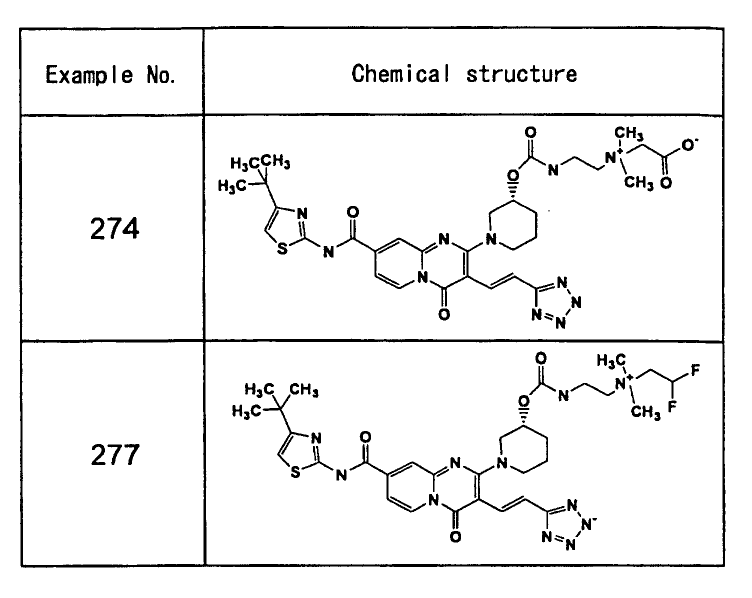

### Test Example 2: Synergistic effect of combined use of a drug efflux pump inhibitor and plural antibacterial agents.

By using a drug efflux pump inhibitor in combination with two or more types of antibacterial agents that can be substrates for the drug efflux pump, strong synergistic effect can be obtained and thus an effective therapeutic method can be provided for treatment of infectious diseases caused by *Pseudomonas aeruginosa.* Antibacterial agents for combination include antibacterial agents excreted by Mex type drug efflux pump of *Pseudomonas aeruginosa,* such as quinolones, *β*-lactams, tetracyclines, macrolides, chloramphenicol, sulfonamides, trimethoprim, *β*-lactamase inhibitors and so forth.

### (1) Effect of combined use of a pump inhibitor, levofloxacin (LVFX), and meropenem (MEPM) evaluated by the three-dimensional checker board method.

The combination effect was measured by the three-dimensional checker board method by using wild type strain of *Pseudomonas aeruginosa* PAM1020. The test strain was inoculated into Mueller-Hinton broth containing various combinations of LVFX (from 0.25 to 0.004 µ g/ml with 2 fold dilutions, and 0 µ g/ml), MEPM (from 0.5 to 0.008 µ g/ml with 2 fold dilutions, and 0 µ g/ml), and the compound of Example 26 (from 40 to 0.625 µ g/ml with 2 fold dilutions, and 0 µ g/ml) at the inoculum size of 10⁶ CFU/ml and incubated for 18 hours at 37° C. MICs of LVFX and MEPM were measured in each combination according to checkerboard method, then FIC index was calculated with the following equation.
FIC index = a/a0 + b/b0
Wherein a0: MIC for Agent A (LVFX) used alone, a: MIC for Agent A when Agent A and B were used in combination, b0: MIC for Agent B (MEPM) used alone, b: MIC for Agent B when Agent A and Agent B were used in combination.

It was determined that there was synergistic effect when FIC index was 0.5 or less, additive effect of weak synergistic effect when the index more than 0.5 but not more than 0.75, no effect of combined use when the index was more than 0.75 but not more than 2, and antagonistic effect when the index was more than 2.0.

The results are shown in the following table. The table shows minimum FIC index determined by usual checker board method. Synergistic effects of the compound of Example 26 were clearly observed in every concentration tested (from 40 to 0.625 µ g/ml with 2 fold dilutions). Further, the effects were observed dose-dependently. The MICs of each compounds for *Pseudomonas aeruginosa* PAM1020 were, 0.25 µg/ml for LVFX, 0.5 µg/ml for MEPM, and > 128 µg/ml for the compound of Example 26.

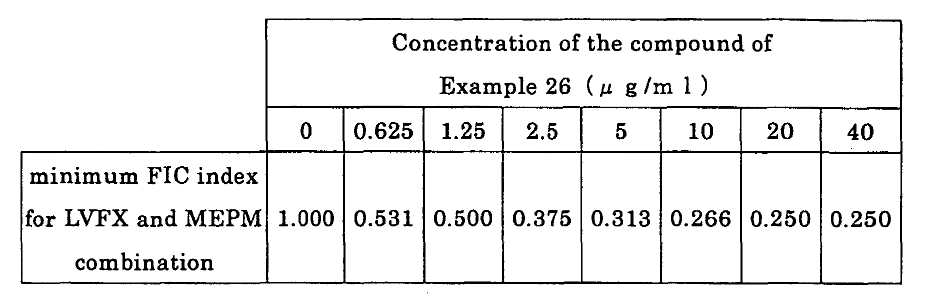

### Test Example 3: Effect of combined use of drug efflux pump inhibitor and disinfectant

By using a drug efflux pump inhibitor in combination with a disinfectant that can be a substrate of the drug efflux pump, effect of combined use can be achieved and an effective disinfection method can be provided.

Effects of a combined use of a drug efflux pump inhibitor and alkyldiaminoethylglycine hydrochloride (AEG, trade name: Tego 51) or chlorhexidine gluconate (CHG, trade name: Hibiten) on *Pseudomonas aeruginosa* PAM 1723 (a strain highly expressing MexAB-OprM drug efflux pump) was determined by the broth dilution method. The compound of Example 26 was added at a final concentration of 10 µ g/ml to Mueller-Hinton broth (MHB) containing a disinfectant at a concentration after 2-fold serial dilution. The amount of the test bacteria inoculated was 1 × 10⁶ CFU/ml, and after standing cultivation at 37°C for 18 hours, the culture was observed by visual inspection. The minimum concentration that gave no turbidity as in the control of MHB was determined as MIC.

The results are shown in the following table. Evaluation of the effect of the combined use of the disinfectant and the compound of Example 26 revealed that the activities of AEG and CHG on PAM 1723 were enhanced by two times and four times, respectively, by the combined use with 10 µ g/ml of the compound of Example 26.

| | MIC (µg/ml) | | | | | |
|---|---|---|---|---|---|---|
| Strain | AEG | | | CHG | | |
| | Single use | Combined use | Ratio* | Single use | Combined use | Ratio* |
| PAM 1723 | 64 | 32 | 2 | 16 | 4 | 4 |

| | | | | | | |
|---|---|---|---|---|---|---|
| ^{*}Ratio of MIC values for single use of the disinfectant and combined use with the compound of Example 26 | | | | | | |

### Example 281

The following three clinically isolated *Pseudomonas aeruginosa* strains: (1) DNS 5494 strain (MexAB-OprM pump highly expressed strain), (2) DNS 5626 strain (MexAB-OprM pump highly expressed and MexXY pump highly expressed strain) and (3) DNS 5512 strain (MexAB-OprM pump low expressed and MexXY pump highly expressed strain), which were collected in 1998, were used as test strains to perform the following test. A cell suspension of each test strain adjusted to about 10⁷ CFU/mL in 33 mM phosphate buffer (pH 7.0) was spread with a swab for antimicrobial susceptibility test. The swab was pressed on a wall of test tube to remove excess cell suspension, and then the cell suspension was uniformly spread on an antibiotic susceptibility test agar medium (Mueller-Hinton agar medium). Aztreonam was used as a β -lactam antibiotic and Levofloxacin as a quinolone antibiotic, and 25 µ L of a solution of each antibacterial agent was impregnated into an antibiotic susceptibility test disk so that the content was 30 µ g for Aztreonam and 5 µ g for Levofloxacin. Further, Compound A as an MexAB-OprM pump inhibitor was dissolved in 5% (v/v) of DMSO (dimethyl sulfoxide), and 64 µ L (50 µ g of Compound A) of the solution was impregnated into a thick disk. Furthermore, a disk impregnated with 64 µ L of a 5% (v/v) of DMSO solution instead of the solution of Compound A was also prepared as a negative control.

On the agar medium, the MexAB-OprM pump inhibitor containing disk was placed at the center, and the Aztreonam containing disk and the MexAB-OprM pump inhibitor containing disk were placed with a space of 2.0 cm. The Levofloxacin containing disk was placed on the 180 degrees opposite side of the Aztreonam disk with a space of 2.0 cm relative to the MexAB-OprM pump inhibitor containing disk as a center. The agar medium was incubated at 37°C for 18 hours, and shape and size of the growth inhibition zones formed around the disks containing the antibacterial agents were investigated and compared with those of the negative control. The results are shown in Fig. 1 (in the figure, "with efflux pump inhibitor" represents the result obtained by using Compound A as a drug efflux pump inhibitor in combination).

When the test strain expressed the MexAB-OprM pump (DNS 5494 strain, MexAB-OprM pump highly expressed strain), inhibition zones were observed around the *β*-lactam antibiotic containing disk and around the quinolone antibiotic containing disk each of which was placed at a position that gave overlap of the diffusion area with that of the MexAB-OprM pump inhibitor (DNS 5494 strain, right column). Furthermore, under excess expression of the MexAB-OprM pump, a further expansion of the inhibition zone was observed. On the other hand, it was found that only small concentric inhibition zones were formed (DNS 5494 strain, left column) around the β -lactam antibiotic containing disk and around the quinolone antibiotic containing disk each of which was placed at a position that gave no overlap of the diffusion area of the β -lactam antibiotic or the quinolone antibacterial agent with that of the MexAB-OprM pump inhibitor. Based on comparison of the sizes and shapes of these inhibition zones, it was concluded that the strain tested expressed the MexAB-OprM pump, and at the same time, a relative expression amount was also clearly recognized.

Further, when the strain expressed the MexAB-OprM pump and simultaneously expressed another drug efflux pump (DNS 5626 strain, MexAB-OprM pump highly expressed and MexXY pump highly expressed strain), an inhibition zone was observed around the *β*-lactam antibiotic containing disk in a region where the diffusion area of the antibiotic overlapped that of the MexAB-OprM pump inhibitor (DNS 5626, right column), whereas no inhibition zone or only a small inhibition zone was observed in a region where the diffusion area of the quinolone antibacterial agent overlapped that of the MexAB-OprM pump inhibitor (DNS 5626, right column). Furthermore, only small concentric inhibition zones (DNS 5626 strain, left column) were formed around the *β*-lactam antibiotic containing disk and around the quinolone containing disk each of which was placed at a position that gave no overlap of the diffusion areas of the *β*-lactam or the quinolone antibacterial agent with that of the MexAB-OprM pump inhibitor. Expansion of the inhibition zone around the *β*-lactam antibiotic containing disk was thus observed due to the diffusion of the MexAB-OprM pump inhibitor (where existence of the MexAB-OprM pump was verified) and no expansion of the inhibition zone by the quinolone antibacterial agent was observed. As a result, it was concluded that the test strain expressed a drug efflux pump other than the MexAB-OprM pump.

Further, when the strain had a low expression level of the MexAB-OprM pump and resistance to quinolone antibacterial agent relatively attributable to expression of another Mex pump (DNS 5512 strain, MexAB-OprM pump low expressed and MexXY pump highly expressed strain), an inhibition zone was formed around the *β*-lactam antibiotic containing disk placed at a position that gave overlap of the diffusion area of the antibiotic with that of the MexAB-OprM pump inhibitor. Whilst around the quinolone containing disk, almost no expansion of the inhibition zone due to the diffusion of the MexAB-OprM pump inhibitor was observed. Upon consideration of the pattern of antimicrobial susceptibility, it was concluded that, when the test strain was assumed to have low level of expression of the MexAB-OprM pump, a Mex pump other than the MexAB-OprM pump was expressed in a relatively higher level in the strain.

### Example 282

As strains to be tested and method for spreading the test strains on the agar medium, the same strains and method as those used in Example 281 were applied. A solution of Compound A at a concentration of 200 µ g/mL (5% (v/v) in DMSO) was prepared, and 400 µ L of the solution was added to the antibiotic susceptibility test agar medium (20 mL) and mixed sufficiently before gelation to prepare an agar medium containing a MexAB-OprM pump inhibitor (final concentration of Compound A: 4 µ g/mL), and then each test bacterium was spread on the agar. The same agar medium not containing Compound A was prepared as a negative control, and the test bacterial suspension was spread on the agar. Then, one strip of Etest Aztreonam AT (registered trademark, AB BIODISK, Sweden) was placed at the center of the agar medium containing Compound A. Further, one strip of Etest Levofloxacin LE (registered trademark, AB BIODISK, Sweden) was placed on the medium in parallel to the Etest Aztreonam AT with a apace of 3.0 cm. In the same manner, the aforementioned two kinds of Etest were placed on the agar medium not containing Compound A as a negative control. After incubation at 37°C for 18 hours, the minimum inhibitory concentration (MIC) was determined based on the inhibition zones formed around the Etest Aztreonam AT and Etest Levofloxacin LE on the agar medium containing Compound A and compared with MIC obtained in the negative control. The results are shown in Fig. 2. As shown in the figure, more accurate measurement of activity of combinational application and estimation of expression amount of a pump was achieved by using Etest in compared to a disk.

### Industrial Applicability.

The medicament of the present invention has an inhibitory activity against the drug efflux pumps of microorganisms, and the medicament has preventing action on the acquisition of resistance to an antimicrobial drug by a microorganism, as well as resistant-eliminating action on a microorganism with acquired resistance. Therefore, the medicament of the present invention can achieve excellent effect in preventive and/or therapeutic treatment of a microbial infection when used, for example, in combination with the administration of an antimicrobial drug.

## Claims

1. A medicament for preventive and/or therapeutic treatment of a microbial infection, which comprises as an active ingredient a compound represented by the following general formula (I) or a physiologically acceptable salt thereof, or a hydrate thereof: 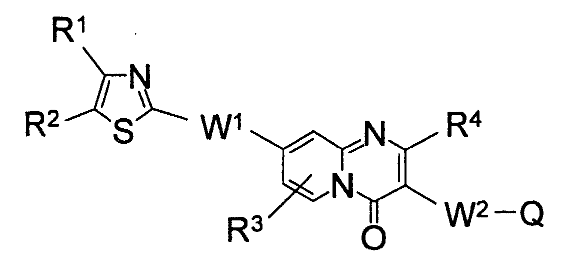 wherein, R¹ and R² each independently represent hydrogen atom, a halogen atom, hydroxyl group, a group of OZ₁₋₆ (the group of OZ₁₋₆ represents an alkyl group having 1-6 carbon atoms or a fluoroalkyl group having 1-6 carbon atoms, which bonds via the oxygen atom), a group of S(O)ₙZ₁₋₄ (Z₁₋₄ represents an alkyl group having 1-4 carbon atoms or a fluoroalkyl group having 1-4 carbon atoms or an alkylene group derived therefrom), a group of N(R¹²)(R¹³) (R¹² and R¹³ each independently represent hydrogen atom, an alkyl group having 1-4 carbon atoms or a fluoroalkyl group having 1-4 carbon atoms), a group of Z₁₋₈ which may be substituted (Z₁₋₈ represents an alkyl group having 1-8 carbon atoms or a fluoroalkyl group having 1-8 carbon atoms), a 5- to 7-membered cyclic alkyl group, an aryl group, a heteroaryl group, or a 4- to 7-membered saturated or partially saturated heterocyclic group (the cyclic alkyl group, aryl group, heteroaryl group and heterocyclic group may have one to three substituents selected from the group consisting of a halogen atom, hydroxyl group, a group of OZ₁₋₄, a group of S(O)ₙZ₁₋₄, a group of N(R¹²)(R¹³), a group of Z₁₋₄, carboxyl group, a group of CO₂Z₁₋₄, group of CONH₂, a group of CONH(Z₁₋₄) and a group of CON(Z₁₋₄)(Z₁₋₄)); W¹ represents a group selected from the group consisting of -CH=CH-, -N(R¹²)CO-, -CON(R¹²)-, -CH₂O- and -CH₂CH₂- (each of the aforementioned groups binds to the thiazole ring at the left end);
R³ represents hydrogen atom, a halogen atom, hydroxyl group or an amino group;
R⁴ represents a group selected from the group consisting of hydrogen atom, a group of -OZ₀₋₄R⁵ (Z₀₋₄ represents an alkylene group having 1-4 carbon atoms, a fluorine-substituted alkylene group having 1-4 carbon atoms or a single bond, and R⁵ represents a 5- to 7-membered cyclic alkyl group, an aryl group, a heteroaryl group or a 4- to 7-membered saturated or partially saturated heterocyclic group (the cyclic alkyl group, aryl group, heteroaryl group and heterocyclic group may have one to three substituents selected from the group consisting of a halogen atom, hydroxyl group, a group of OZ₁₋₄, a group of S(O)ₙZ₁₋₄, a group of N(R¹²)(R¹³), a group of Z₁₋₄, carboxyl group, a group of CO₂Z₁₋₄, group of CONH₂, a group of CONH(Z₁₋₄) and a group of CON(Z₁₋₄)(Z₁₋₄)), a group of -S(O)ₙZ₀₋₄R⁵, a group of -N(R⁶)(R⁷) {R⁶ and R⁷ each independently represent hydrogen atom or Z₁₋₄, or they may bind to each other to form a saturated or unsaturated 5- to 7-membered ring (the ring may contain one or two hetero atoms as ring constituting atoms), and R⁶ and R⁷ may have one to three substituents selected from the group consisting of a halogen atom, hydroxyl group, a group of OCON(R¹⁵)(R¹⁶), a group of CON(R¹⁵)(R¹⁶), a group of N(R¹²)CON(R¹⁵)(R¹⁶), a group of Z₁₋₄, a group of OZ₁₋₄, a group S(O)ₙZ₁₋₄, group of CH₂OH, a group of (CH₂)ₘN(R¹²)(R¹³), a group of Z₁₋₄CON(R¹⁵)(R¹⁶), a group of SO₂N(R¹²)(R¹³), a group of OSO₂N(R¹²)(R¹³), a group of OSO₂R¹², a group of NCOZ₁₋₄R¹⁵ (in the formula, R¹⁵ and R¹⁶ independently represent hydrogen atom, a group of Z₁₋₆R¹¹, a group of Z₁₋₄N(R¹²)(R¹³), a group of Z₁₋₄OH, and a group of Z₁₋₄OZ₁₋₄), carboxyl group, cyano group, a group of COZ₁₋₄R¹⁰, a group of CO-Z₁₋₄(R¹⁰)-N(R¹²)(R¹³) (R¹⁰ is a substituent corresponding to a side chain on an amino acid carbon or a group of -Z₁₋₄-R¹¹ (R¹¹ represents a substituent which forms a quaternary salt)) and a group of 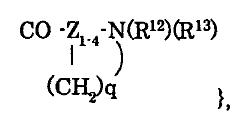 a 5- or 6-membered aryl group which may be substituted and a 5-or 6-membered unsaturated heterocyclic group which may be substituted;
W² represents a single bond or -C(R⁸)=C(R⁹)- (R⁸ and R⁹ each independently represent hydrogen atom, a halogen atom, a lower alkyl group, an alkoxy group, cyano group, carboxyl group, hydroxymethyl group, cyanomethyl group, vinyl group or a group of N(R¹²)(R¹³)), Q represents an acidic group, and W² and Q may bind together to form vinylidenethiazolidinedione in *E*- or *Z*-configuration or an equivalent heterocyclic ring; m and n each independently represent an integer of 0 to 2, and q represents an integer of 0 to 3.

2. A medicament for eliminating resistance of a microorganism with acquired drug resistance, which comprises the compound represented by the aforementioned general formula (I) according to claim 1 or a physiologically acceptable salt thereof as an active ingredient.

3. A medicament for enhancing effect of an antimicrobial agent, which comprises a compound represented by the aforementioned general formula (I) according to claim 1 or a physiologically acceptable salt thereof as an active ingredient.

4. A pharmaceutical composition for preventive and/or therapeutic treatment of a microbial infection, which comprises a compound represented by the aforementioned general formula (I) according to claim 1 or a physiologically acceptable salt thereof together with an antimicrobial agent.

5. A medicament for preventive and/or therapeutic treatment of a microbial infection, which comprises as an active ingredient a compound represented by the following general formula (I) or a physiologically acceptable salt thereof, or hydrates thereof 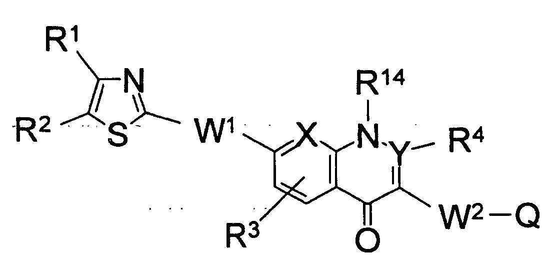 wherein, R¹, R², R³, R⁴, W¹, W² and Q have the same meanings as those defined above; R¹⁴ represents hydrogen atom, Z₁₋₄, Z₁₋₄R⁵ or Z₁₋₄OR⁵; and X and Y each independently represent C-H or nitrogen atom.

6. A method for judging effectiveness of a drug efflux pump inhibitor against a microorganism, which comprises the steps of:
(A1) spreading a microorganism to be tested on a surface of an agar medium, then providing an antibacterial agent as a spot on the surface of the agar medium and culturing the microorganism;
(A2) determining a growth degree of the microorganism in a region of the agar medium into which the antibacterial agent has diffused during the culture period;
(A3) determining a growth degree of the microorganism in a region of the agar medium in which the antibacterial agent that has diffused during the culture period and a drug efflux pump inhibitor contained in the agar medium coexist; and
(A4) judging that the drug efflux pump inhibitor is effective against the microorganism when the growth degree of the microorganism determined in the step (A2) is significantly higher than the growth degree of the microorganism determined in the step (A3).

7. The method according to claim 6, wherein the antibacterial agent is provided as a spot on the agar medium surface by means of a disk.

8. The method according to claim 6 or claim 7, wherein the drug efflux pump inhibitor contained in the agar medium is the drug efflux pump inhibitor diffused from a disk provided as a spot on the agar medium surface.

9. The method according to claim 6 or 7, wherein the drug efflux pump inhibitor contained in the agar medium is the drug efflux pump inhibitor added beforehand to the agar medium during preparation of the agar medium.

10. The method according to any one of claims 6 to 9, wherein the microorganism is *Pseudomonas aeruginosa.*

11. A method for identifying a drug efflux pump expressed in a microorganism, which comprises the steps of:
(B1) spreading a microorganism to be tested on a surface of an agar medium, then providing an antibacterial agent that can be excreted by a particular drug efflux pump as a spot on the surface of the agar medium and culturing the microorganism;
(B2) determining a growth degree of the microorganism in a region of the agar medium into which the antibacterial agent has diffused during culture period;
(B3) determining a growth degree of the microorganism in a region of the agar medium in which the antibacterial agent that has diffused during the culture period and a drug efflux pump inhibitor contained in the agar medium coexist (provided that said drug efflux pump inhibitor is a specific inhibiter for the particular drug efflux pump); and
(B4) judging that the microorganism expresses the drug efflux pump of the particular type when the growth degree of the microorganism measured in the step (B2) is significantly higher than the growth degree of the microorganism determined in the step (B3).

12. The method according to claim 11, wherein the antibacterial agent is provided as a spot on the agar medium surface by using a disk.

13. The method according to claim 11 or claim 12, wherein the drug efflux pump inhibitor contained in the agar medium is the drug efflux pump inhibitor diffused from a disk provided as a spot on the agar medium surface.

14. The method according to claim 11 or claim 12, wherein the drug efflux pump inhibitor contained in the agar medium is the drug efflux pump inhibitor added beforehand to the agar medium during preparation of the agar medium.

15. The method according to any one of claims 11 to 14, wherein the microorganism is *Pseudomonas aeruginosa.*

16. The method according to any one of claims 11 to 15, wherein the particular drug efflux pump is a MexAB-OprM pump.

17. The method according to any one of Claims 11 to 16, wherein the antibacterial agent is a *β*-lactam antibiotic.

18. The method according to claim 17, wherein the antibacterial agent is Aztreonam.

19. A method for verifying expression of two or more kinds of drug efflux pumps in a microorganism, which comprises the steps of:
(C1) spreading a microorganism to be tested on a surface of an agar medium, then providing two or more kinds of antibacterial agents (provided that each of the two or more kinds of the antibacterial agents has different effluxing specificity by the two or more kinds of drug efflux pumps, and one of the two or more kinds of the antibacterial agents (hereinafter referred to as "Antibacterial agent (1)") has a property of being excreted by only one of the two or more kinds of the drug efflux pumps (hereinafter referred to as "Drug efflux pump (1)"), whilst the other antibacterial agent or agents have a property of being excreted by Drug efflux pump (1) and the other drug efflux pump or pumps);
(C2) determining a growth degree of the microorganism in a region of the agar medium into which each antibacterial agent has solely diffused during culture period;
(C3) determining a growth degree of the microorganism in a region of the agar medium in which each antibacterial agent that has solely diffused during the culture period and a drug efflux pump inhibitor contained in the agar medium coexist (provided that the drug efflux pump inhibitor is a specific inhibiter for Drug efflux pump (1)); and
(C4) judging that the microorganism expresses Drug efflux pump (1) and one or more kinds of other drug efflux pumps when the growth degree of the microorganism determined in the step (C2) is significantly higher than the growth degree of the microorganism determined in the step (C3) for Antibacterial agent (1) and the growth degree of the microorganism determined in the step (C2) is significantly lower than the growth degree of the microorganism determined in the step (C3) for the other antibacterial agent or agents.

20. The method according to claim 19, wherein each of the antibacterial agents is provided as a spot on the agar medium surface each by using a disk.

21. The method according to claim 19 or 20, wherein the drug efflux pump inhibitor contained in the agar medium is the drug efflux pump inhibitor diffused from a disk provided as a spot on the agar medium surface.

22. The method according to claim 19 or 20, wherein the drug efflux pump inhibitor contained in the agar medium is the drug efflux pump inhibitor added beforehand to the agar medium during preparation of the agar medium.

23. The method according to any one of claims 19 to 22, wherein the microorganism is *Pseudomonas aeruginosa.*

24. The method according to any one of claims 19 to 23, wherein one of the two or more kinds of drug efflux pumps is a MexAB-OprM pump.

25. The method according to any one of claims 19 to 24, wherein the two or more kinds of antibacterial agents include a combination of a *β*-lactam antibiotic and a quinolone antibacterial agent.

26. The method according to claim 25, the antibacterial agents are Aztreonam and Levofloxacin.

27. The method according to any one of claim 19 to 26, wherein the drug efflux pump inhibitor is a specific inhibitor for a MexAB-OprM pump.

28. The method according to any one of claims 19 to 27, wherein the drug efflux pump inhibitor is a compound represented by the following formula.
